# EUROPEAN PATENT APPLICATION

(11) **EP 4 653 438 A1**
(43) Date of publication of application: **26.11.2025**
(21) Application number: 24744378.1
(22) Date of filing: 18.01.2024
(51) Int. Cl.: C07D 471/22, C07D 487/12, C07D 513/22, C07D 519/00, A61K 31/429, A61K 31/5025, A61P 35/00

(54) **MACROCYCLIC COMPOUNDS, PREPARATION METHOD THEREFOR, AND USE THEREOF**

(30) Priority: 19.01.2023 CN 202310091121; 15.03.2023 CN 202310251933; 22.03.2023 CN 202310293450; 04.04.2023 CN 202310356097; 26.04.2023 CN 202310465755; 17.05.2023 CN 202310560886; 14.06.2023 CN 202310708457; 19.07.2023 CN 202310892369; 23.08.2023 CN 202311070014; 06.09.2023 CN 202311148034; 13.10.2023 CN 202311332607; 18.10.2023 CN 202311355859; 29.11.2023 CN 202311621685; 13.12.2023 CN 202311714943; 20.12.2023 CN 202311781874; 03.01.2024 CN 202410009341; 16.01.2024 CN 202410074142
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: ZHAO, Jichen, Shanghai 201203 (CN); ZHOU, Fusheng, Shanghai 201203 (CN); JIANG, Tao, Shanghai 201203 (CN); LIN, Chonglan, Shanghai 201203 (CN); LIANG, Tao, Shanghai 201203 (CN); MA, Kai, Shanghai 201203 (CN); PENG, Ling, Shanghai 201203 (CN); HE, Wan, Shanghai 201203 (CN); ZHANG, Leitao, Shanghai 201203 (CN); YAN, Feng, Shanghai 201203 (CN); LAN, Jiong, Shanghai 201203 (CN); LU, Qiang, Shanghai 201203 (CN)
(74) Representative: Elzaburu S.L.P.
(86) International application number: PCT/CN2024/073135
(87) International publication number: WO 2024/153208

(57) **Abstract**

Compounds represented by formula (I) or stereoisomers thereof, or pharmaceutically acceptable salts, solvates or prodrugs thereof, each group in the formula being defined in the description, a pharmaceutical composition comprising the compounds and the use thereof in the preparation of drugs used for preventing and/or treating diseases or disorders (such as cancer) related to RAS protein activity.

## Description

### TECHNICAL FIELD

The present disclosure pertains to the field of pharmaceuticals, and particularly relates to a macrocyclic compound capable of inhibiting RAS protein, a preparation method therefor, and use thereof.

### BACKGROUND

The RAS gene (rat sarcoma viral oncogene homolog) encodes a class of small GTPase family proteins with a molecular weight of about 21 kDa. The mammalian RAS family comprises three members: HRAS, KRAS, and NRAS, which serve as molecular switches in a variety of cell pathway signaling processes. RAS is in an inactivated state (dormant or turned off) when bound to GDP. When cells are exposed to certain growth-promoting stimuli, RAS is induced to exchange its bound GDP for GTP, thereby becoming activated. Subsequently, it recruits and activates other downstream target proteins (e.g., Raf and PI3K), thereby promoting cell proliferation. The RAS protein endogenously possesses the activity of hydrolyzing GTP to GDP (thereby converting itself into an inactivated state), but the conversion rate is low. However, once it binds to a GTPase-activating protein (GAP), the interaction between the two greatly accelerates the rate at which the RAS protein converts GTP to GDP, so that RAS can revert to an inactivated state rapidly upon cessation of cell stimulation, thereby shutting down the signaling pathway transduction.

Approximately 30% of human tumors harbor RAS gene mutations, in which KRAS mutations are the most common and account for about 85%, while NRAS and HRAS mutations account for 12% and 3%, respectively. KRAS mutations are predominantly found in pancreatic cancer (86%), colorectal cancer (41%), and lung cancer (32%); NRAS mutations are common in melanoma and acute myeloid leukemia; and HRAS mutations are common in bladder cancer and head and neck cancer. In tumors, RAS gene mutations occur primarily via point mutations. More than 150 distinct RAS point mutations have been identified, with mutations at positions G12, G13, and Q61 being the most common. Generally, these pathogenic mutations affect the interaction between RAS and GAPs and the endogenous GTPase activity of RAS. Consequently, the mutated RAS protein exists mainly in a GTP-bound activated form within cells, such that the signaling pathway mediated by this protein is in a continuously activated state, which in turn gives rise to uncontrolled cell proliferation and ultimately promotes tumor formation.

Currently, for KRAS G12C mutation, targeted drugs AMG510 and MRTX849 have been approved for marketing for the treatment of non-small cell lung cancer with KRAS G12C mutation. However, for KRAS G12D and KRAS G12V mutations that commonly occur in pancreatic cancer and colorectal cancer, as well as NRAS and HRAS mutations commonly found in melanoma and head and neck cancer, no corresponding targeted drugs have been approved. Therefore, the research and development of highly effective inhibitors targeting various RAS mutations is of great clinical significance, and also represents both a focus and a challenge in the field of new drug research and development.

### SUMMARY

The present disclosure provides a class of RAS inhibitors. The RAS inhibitors of the present disclosure have the advantages of high activity, low toxic and side effects, and the like, and possess good physicochemical properties and druggability.

A first aspect of the present disclosure provides a compound represented by formula (18), or a stable deuterated derivative thereof, or a stereoisomer thereof, or a pharmaceutically acceptable salt thereof, a solvate thereof, or a prodrug thereof: 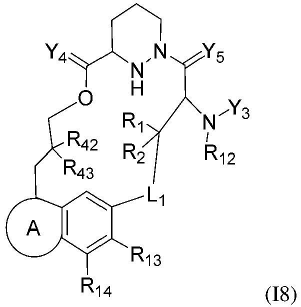 wherein in the formula, 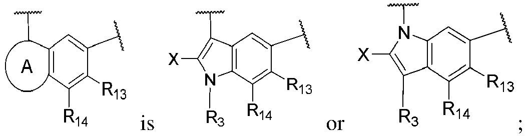
X is 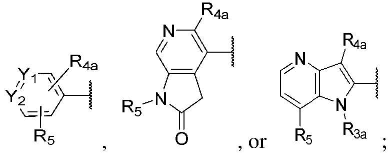
Y₁ is N, CH, CR₄ₐ, CR_{4b}, or CR₅, wherein N may be oxidized (N⁺-O⁻);
Y₂ is N, CH, CR₄ₐ, CR₄₈, or CR₅, wherein N may be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl or substituted or unsubstituted 8- to 10-membered bicyclic heteroaryl, or -P(=O)-(C₁₋₆ alkyl)₂;
R₄ₐ is substituted or unsubstituted C₁₋₆ alkyl, substituted or unsubstituted C₃₋₆ cycloalkyl, or substituted or unsubstituted 3- to 8-membered heterocyclyl;
R_{4b} is deuterium, halogen, substituted or unsubstituted C₁₋₆ alkyl, or substituted or unsubstituted C₁₋₆ heteroalkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁-₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), - S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), - S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 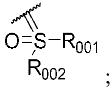 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), -C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form substituted or unsubstituted 3- to 8-membered heterocyclyl;
Y₅ is O or S;
Y₄ is O or S;
R₁₃ is hydrogen, halogen, substituted or unsubstituted C₁₋₆ alkyl, or -P(=O)-(C₁₋₆ alkyl)₂; R₁₄ is hydrogen, halogen, substituted or unsubstituted C₁₋₆ alkyl, or -P(=O)-(C₁₋₆ alkyl)₂; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, - C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁-₆ alkyl, -C₁₋₄ alkyl-C₁-₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, - C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁-₆ alkyl, -O-C₁₋₄ alkyl-C₁-₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, - C(=O)-C₃₋₂₀ cycloalkyl, -C(=O)-C₆₋₁₄ aryl, -NR^{a1}R^{b1}, -C(=O)-NR^{a1}R^{b1}, -C(-O)-NR^{d1}-C₁₋₄ alkyl-R^{c1}, -OR^{c1}, -C₁₋₄ alkylS(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, - C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C=C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, - NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, -N=S(=O)(R^{g1})(R^{h1}), and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁-₆ alkoxy, -C₁₋₄ alkyl-C₁-₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, - (C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

In the present disclosure, when R₆ is dimethylcyclopropyl, Y₄ is O, Y₅ is O, R₁ and R₂ are hydrogen, L₁ is thiazole, R₄₂ and R₄₃ are methyl, and R₁₃ and R₁₄ are hydrogen, and when R₃ is ethyl, R₅ is not methylpiperazinyl.

In one embodiment,

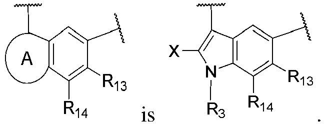

In one embodiment, 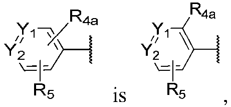 further 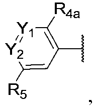 and even further

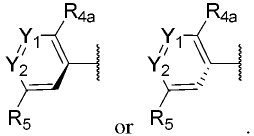

In one embodiment, Y₁ is N, CH, CR₄ₐ, CR_{4b}, or CR₅, wherein N may be oxidized (N⁺-O⁻).

In one embodiment, Y₁ is N.

In one embodiment, Y₁ is N or CH, wherein N may be oxidized (N⁺-O⁻).

In one embodiment, Y₂ is N, CH, CR₄ₐ, CR_{4b}, or CR₅, wherein N may be oxidized (N⁺-O⁻).

In one embodiment, Y₂ is CH or CR_{4b}, wherein R_{4b} is deuterium, halogen, substituted or unsubstituted C₁₋₆ alkyl, or substituted or unsubstituted C₁₋₆ heteroalkyl.

In one embodiment, Y₂ is CH.

In one embodiment, R₄₂ is methyl; R₄₃ is methyl.

In one embodiment, R₄₂ and R₄₃, together with the carbon atom to which they are attached, form substituted or unsubstituted cyclopropyl.

In one embodiment, L₁ is thiazole.

In one embodiment, R₁ is hydrogen; R₂ is hydrogen.

In one embodiment, R₃ is substituted or unsubstituted C₁₋₆ alkyl.

In one embodiment, R₃ is unsubstituted C₁₋₆ alkyl or C₁₋₆ haloalkyl.

In one embodiment, R₄ₐ is substituted or unsubstituted C₁₋₆ alkyl.

In one embodiment, R₄ₐ is C₁₋₆ alkyl substituted with hydroxy or methoxy.

In one embodiment, R₅ is -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, or -C₁₋₄ alkyl-C₂₋₄ alkynyl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 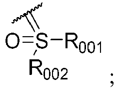 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl.

In one embodiment, R₅ is -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, or -C₁₋₄ alkyl-C₂₋₄ alkynyl-C₁₋₄ alkyl-NR₀₁R₀₂;
R₀₁ and R₀₂ are each independently selected from hydrogen and substituted or unsubstituted C₁₋₆ alkyl; or,
R₀₁ and R₀₂, together with the nitrogen atom to which they are attached, form substituted or unsubstituted azetidinyl, substituted or unsubstituted oxazepanyl, substituted or unsubstituted dioxepanyl, substituted or unsubstituted morpholinyl, substituted or unsubstituted pyrrolidinyl, substituted or unsubstituted piperidinyl, substituted or unsubstituted piperazinyl, substituted or unsubstituted 3-oxa-8-azabicyclo[3.2.1]octanyl, substituted or unsubstituted tetrahydro-1H-pyrrolizinyl, substituted or unsubstituted thiomorpholinyl, substituted or unsubstituted tetrahydro-5H-pyrazolo[4,3-c]pyridinyl, substituted or unsubstituted 1,2,5-thiadiazepanyl, substituted or unsubstituted 1,4-thiazepanyl, substituted or unsubstituted 3,8-diazabicyclo[3.2.1]octanyl, or substituted or unsubstituted 4-oxa-7-azaspiro[2.5]octanyl;
R₀₃ and R₀₄, together with the carbon atom to which they are attached, form substituted or unsubstituted azetidinyl, substituted or unsubstituted oxazepanyl, substituted or unsubstituted dioxepanyl, substituted or unsubstituted morpholinyl, substituted or unsubstituted pyrrolidinyl, substituted or unsubstituted piperidinyl, substituted or unsubstituted piperazinyl, substituted or unsubstituted 3-oxa-8-azabicyclo[3.2.1]octanyl, substituted or unsubstituted tetrahydro-1H-pyrrolizinyl, substituted or unsubstituted thiomorpholinyl, substituted or unsubstituted tetrahydro-5H-pyrazolo[4,3-c]pyridinyl, substituted or unsubstituted 1,2,5-thiadiazepanyl, substituted or unsubstituted 1,4-thiazepanyl, substituted or unsubstituted 3,8-diazabicyclo[3.2.1]octanyl, substituted or unsubstituted 4-oxa-7-azaspiro[2.5]octanyl, or substituted or unsubstituted cyclobutyl;
R₀₅ is hydrogen, deuterium, halogen, deuterated C₁₋₆ alkyl, or C₁₋₆ alkyl.

In one embodiment, R₅ is -C₂₋₄ alkynyl-NR₀₁R₀₂, -C₁₋₄ alkyl-C₂₋₄ alkynyl-NR₀₁R₀₂, -C₂₋₄ alkynyl-C₁₋₄ alkyl-NR₀₁R₀₂, -C₂₋₄ alkynyl-C(=O)-NR₀₁R₀₂, -C₁₋₄ alkyl-C₂₋₄ alkynyl-C(R₀₅)R₀₃R₀₄, -C₂₋₄ alkynyl-C₁₋₄ alkyl-C(R₀₅)R₀₃R₀₄, or -C₂₋₄ alkynyl-C(R₀₅)R₀₃R₀₄; R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -C₂₋₄ alkynyl-NR₀₁R₀₂, -C₁₋₄ alkyl-C₂₋₄ alkynyl-NR₀₁R₀₂, -C₂₋₄ alkynyl-C₁₋₄ alkyl-NR₀₁R₀₂, or -C₁₋₄ alkyl-C₂₋₄ alkynyl-C₁₋₄ alkyl-NR₀₁R₀₂; R₀₁ and R₀₂, together with the nitrogen atom to which they are attached, form substituted or unsubstituted 5- to 15-membered tricyclic heterocyclyl.

In one embodiment, R₅ is -C₂₋₄ alkynyl-NR₀₁R₀₂, -C₁₋₄ alkyl-C₂₋₄ alkynyl-NR₀₁R₀₂, -C₂₋₄ alkynyl-C₁₋₄ alkyl-NR₀₁R₀₂, or -C₁₋₄ alkyl-C₂₋₄ alkynyl-C₁₋₄ alkyl-NR₀₁R₀₂; R₀₁ and R₀₂, together with the nitrogen atom to which they are attached, form substituted or unsubstituted 7- to 12-membered bicyclic heterocyclyl.

In one embodiment, R₅ is -C₂₋₄ alkynyl-NR₀₁R₀₂, -C₁₋₄ alkyl-C₂₋₄ alkynyl-NR₀₁R₀₂, -C₂₋₄ alkynyl-C₁₋₄ alkyl-NR₀₁R₀₂, or -C₁₋₄ alkyl-C₂₋₄ alkynyl-C₁₋₄ alkyl-NR₀₁R₀₂; R₀₁ and R₀₂, together with the nitrogen atom to which they are attached, form substituted or unsubstituted 3- to 8-membered monocyclic heterocyclyl.

In one embodiment, R₅ is -C₂₋₄ alkynyl-C(R₀₅)R₀₃R₀₄; R₀₃ and R₀₄, together with the carbon atom to which they are attached, form substituted or unsubstituted 7- to 12-membered bicyclic heterocyclyl; R₀₅ is hydrogen, deuterium, halogen, deuterated C₁₋₆ alkyl, or C₁₋₆ alkyl.

In one embodiment, R₅ is -C₂₋₄ alkynyl-C(R₀₅)R₀₃R₀₄; R₀₃ and R₀₄, together with the carbon atom to which they are attached, form substituted or unsubstituted 5- to 15-membered tricyclic heterocyclyl; R₀₅ is hydrogen, deuterium, halogen, deuterated C₁₋₆ alkyl, or C₁₋₆ alkyl.

In one embodiment, R₅ is -C₂₋₄ alkynyl-C(R₀₅)R₀₃R₀₄; R₀₃ and R₀₄, together with the carbon atom to which they are attached, form substituted or unsubstituted 3- to 8-membered monocyclic heterocyclyl; R₀₅ is hydrogen, deuterium, halogen, deuterated C₁₋₆ alkyl, or C₁₋₆ alkyl.

In one embodiment, R₅ is -C₂₋₄ alkynyl-C(R₀₅)R₀₃R₀₄; R₀₃ and R₀₄, together with the carbon atom to which they are attached, form substituted or unsubstituted 5- or 6-membered monocyclic heteroaryl or substituted or unsubstituted 8- to 10-membered bicyclic heteroaryl; R₀₅ is hydrogen, deuterium, halogen, deuterated C₁₋₆ alkyl, or C₁₋₆ alkyl.

In one embodiment, R₅ is -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, -O-C(R₀₅)R₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, or -O-substituted or unsubstituted 7- to 12-membered bicyclic heterocyclyl;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 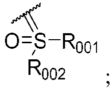 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -O-C₁₋₄ alkyl-NR₀₁R₀₂; R₀₁ and R₀₂, together with the nitrogen atom to which they are attached, form substituted or unsubstituted 5- to 15-membered tricyclic heterocyclyl.

In one embodiment, R₅ is -O-C₁₋₄ alkyl-NR₀₁R₀₂; R₀₁ and R₀₂, together with the nitrogen atom to which they are attached, form substituted or unsubstituted 7- to 12-membered bicyclic heterocyclyl.

In one embodiment, R₅ is -O-C₁₋₄ alkyl-NR₀₁R₀₂; R₀₁ and R₀₂, together with the nitrogen atom to which they are attached, form substituted or unsubstituted 3- to 8-membered monocyclic heterocyclyl.

In one embodiment, R₅ is -O-C₁₋₄ alkyl-substituted or unsubstituted 3- to 8-membered monocyclic heterocyclyl.

In one embodiment, R₅ is -O-C₁₋₄ alkyl-substituted or unsubstituted 5- to 15-membered tricyclic heterocyclyl.

In one embodiment, R₅ is -O-C₁₋₄ alkyl-substituted or unsubstituted 7- to 12-membered bicyclic heterocyclyl.

In one embodiment, R₅ is -C₁₋₄ alkyl-NR₀₁R₀₂, -C₁₋₄ alkyl-C(R₀₅)R₀₃R₀₄, -C_{1-A} alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -C₁₋₄ alkyl-NR₀₁R₀₂, -C₁₋₄ alkyl-C(R₀₅)R₀₃R₀₄, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C_{1-A} alkyl-NR₀₁R₀₂, -C=CR₀₃R₀₄, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 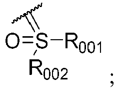 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -NR₀₁R₀₂, -C₁₋₄ alkyl-NR₀₁R₀₂, -C₁₋₄ alkyl-C(=O)-NR₀₁R₀₂, -C₁₋₄ alkyl-N(R₀₆)C(=O)-C₁₋₄ alkyl-NR₀₁R₀₂, -O-C₁₋₄ alkyl-NR₀₁R₀₂, -O-C₁₋₄ alkyl-C(R₀₅)R₀₃R₀₄, -O-C(R₀₅)R₀₃R₀₄, -C₁₋₄ alkyl-NR₀₁R₀₂, -N(R₀₆)C(=O)-C(R₀₅)R₀₃R₀₄, or -CH=CR₀₁R₀₂; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₀₁ and R₀₂, together with the nitrogen atom to which they are attached, form substituted or unsubstituted 5- to 15-membered tricyclic heterocyclyl.

In one embodiment, R₀₁ and R₀₂, together with the nitrogen atom to which they are attached, form substituted or unsubstituted 7- to 12-membered bicyclic heterocyclyl.

In one embodiment, R₀₁ and R₀₂, together with the nitrogen atom to which they are attached, form substituted or unsubstituted 3- to 8-membered monocyclic heterocyclyl.

In one embodiment, Y₃ is -C(=O)R₆; R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl.

In one embodiment, Y₃ is R₁₁; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl.

In one embodiment, R₁₂ is hydrogen.

In one embodiment, Y₅ is O; Y₄ is O.

In one embodiment, R₁₃ is hydrogen; R₁₄ is hydrogen.

In one embodiment, in the groups described herein, the substitutions each independently refer to that the groups described above are each independently substituted with 1, 2, 3, 4, 5, or 6 groups selected from group S1.

In one embodiment, the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₈ cycloalkyl, 3- to 8-membered heterocyclyl, phenyl, 5- or 6-membered monocyclic heteroaryl, 8- to 10-membered bicyclic heteroaryl, -O-C₃₋₈ cycloalkyl, -O-3- to 8-membered heterocyclyl, -O-phenyl, -O-5- or 6-membered monocyclic heteroaryl, - O-8- to 10-membered bicyclic heteroaryl, -C≡C-C₃₋₈ cycloalkyl, -C≡C-3- to 8-membered heterocyclyl, -C≡C-phenyl, -C≡C-5- or 6-membered monocyclic heteroaryl, -C≡C-8- to 10-membered bicyclic heteroaryl, -C≡C-C₁₋₄ alkyl-C₃₋₈ cycloalkyl, - C≡C-C₁₋₄ alkyl-3- to 8-membered heterocyclyl, -C≡C-C₁₋₄ alkyl-phenyl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₈ cycloalkyl, -C₁₋₄ alkyl-O-C₃₋₈ cycloalkyl, -C₁₋₄ alkyl-3- to 8-membered heterocyclyl, -C₁₋₄ alkyl-O-3- to 8-membered heterocyclyl, -C₁₋₄ alkyl-phenyl, -C₁₋₄ alkyl-O-phenyl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₈ cycloalkyl, -O-C₁₋₄ alkyl-O-C₃₋₈ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-phenyl, -O-C₁₋₄ alkyl-O-phenyl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₈ cycloalkyl, -S(=O)₂-3- to 20-membered heterocyclyl, -C(=O)O-C₁₋₆ alkyl, -C(=O)O-C₃₋₈ cycloalkyl, -C(=O)-C₁₋₆ alkyl, -C(=O)-C₃₋₈ cycloalkyl, -C(=O)-phenyl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)2-C₁₋₆ alkyl, -C₁₋₄ alkyl-S(=O)₂-C₃₋₈ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₈ cycloalkyl, -C₁₋₄ alkyl-C(=O)-C₁₋₆ alkyl, -C₁₋₄ alkyl-C(=O)-C₃₋₈ cycloalkyl, -C₁₋₄ alkyl-C(=O)-phenyl, -C(=O)-5- or 6-membered monocyclic heteroaryl, -C(=O)-8- to 10-membered bicyclic heteroaryl, -C(=O)-C₁₋₆ alkyl-C₃₋₈ cycloalkyl, -C(=O)-C₁₋₆ alkyl-3- to 8-membered heterocyclyl, -C(=O)-C₁₋₆ alkyl-phenyl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkylS(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, - NR^{d1}-S(=O)₂-R^{c1}, -S(=O)2-NR^{a1}R^{b1}, -NR^{d1}-S(=O)₂-NR^{a1}R^{b1}, -N=S(=O)(R^{g1})(R^{h1}), and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₈ cycloalkyl, the 3- to 8-membered heterocyclyl, the phenyl, the 5- or 6-membered monocyclic heteroaryl, and the 8- to 10-membered bicyclic heteroaryl are each independently and optionally substituted with 1, 2, 3, or 4 groups selected from group S2; the nitrogen atom(s) in the 5- or 6-membered monocyclic heteroaryl may optionally be oxidized (N⁺-O⁻).

In one embodiment, the groups of group S1 are each independently selected from the group consisting of: oxo (=O), thio (=S), =CR^{e}R^{f}, =NR^{e}, halogen, cyano, hydroxy, C₁₋₆ alkyl, C₁₋₆ alkoxy, phenyl, 5- or 6-membered monocyclic heteroaryl, -C₁₋₄ alkyl-hydroxy, -C₁₋₄ alkyl-cyano, -C₁₋₄ alkyl-5- or 6-membered monocyclic heteroaryl, -S(=O)₂-C₁₋₆ alkyl, -C(=O)O-C₁₋₆ alkyl, - C(=O)-C₁₋₆ alkyl, -C(=O)-NR^{a1}R^{bl}, -N=S(=O)(R^{g1})(R^{h1}), -P(=O)-(C₁₋₆ alkyl)₂, -C(=O)-C₁₋₄ alkyl-hydroxy, and -C(=O)-C₁₋₄ alkyl-cyano, wherein the C₁₋₆ alkyl is optionally substituted with 1, 2, or 3 groups selected from halogen, deuterium, cyano, and hydroxy; the phenyl and the 5- or 6-membered monocyclic heteroaryl are optionally substituted with 1, 2, 3, or 4 groups selected from group S2; the nitrogen atom(s) in the 5- or 6-membered monocyclic heteroaryl may optionally be oxidized (N⁺-O⁻).

In one embodiment, in the groups described herein, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂.

In one embodiment, in the groups described herein, each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, - C₁₋₄ alkyl-P(=O)-(C₁₋₆ alkyl)₂, and -P(=O)-(C₁₋₆ alkyl)₂.

In one embodiment, in the groups described herein, each R^{d1} is independently H, C₁₋₆ alkyl, or deuterated C₁₋₆ alkyl.

In one embodiment, in the groups described herein, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3-to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂.

In one embodiment, in the groups described herein, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl.

In one embodiment, in the groups described herein, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, - (C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅. In one embodiment, in the groups described above, the -C₁₋₄ alkyl- is unsubstituted; or 1, 2, 3, or 4 hydrogen atoms on the - C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f}.

In one embodiment, in the groups described above, the -C₁₋₄ alkyl- is unsubstituted.

In one embodiment, in the groups described above, 1, 2, 3, or 4 hydrogen atoms on the -C₁₋₄ alkyl- are each independently substituted by a group selected from C₁₋₄ alkyl, C₁₋₄ haloalkyl, and deuterated C₁₋₄ alkyl.

In one embodiment, in the groups described above, 2 hydrogen atoms on the same carbon atom of the C₁₋₄ alkyl are both substituted by -(CH₂)ⱼ- to form cycloalkyl, wherein j is 2, 3, 4, 5, or 6.

In one embodiment, in the groups described above, 2 hydrogen atoms on the same carbon atom of the C₁₋₄ alkyl are both substituted by =CR^{e}R^{f}.

In one embodiment, in the groups described herein, each R^{e} is independently H, halogen, C₁₋₆ alkyl, C₁₋₆ haloalkyl, or deuterated C₁₋₆ alkyl. Further, each R^{e} is independently H or halogen.

In one embodiment, in the groups described herein, each R^{f} is independently H, halogen, C₁₋₆ alkyl, C₁₋₆ haloalkyl, or deuterated C₁₋₆ alkyl. Further, each R^{f} is independently H or halogen.

In one embodiment, in the groups described herein, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus.

In one embodiment, in the groups described herein, one or more (e.g., 1, 2, 3, or 4) atoms of the C₁₋₆ heteroalkyl are heteroatoms selected from nitrogen, oxygen, sulfur, and phosphorus.

In one embodiment, in the groups described herein, one or more (e.g., 1, 2, 3, 4, or 5) ring atoms of the 7- to 12-membered bicyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 3- to 20-membered heterocyclyl are heteroatoms selected from nitrogen, oxygen, sulfur, and phosphorus.

In one embodiment, in the groups described herein, one or more (e.g., 1, 2, 3, 4, or 5) ring atoms of the 5- to 20-membered heteroaryl are heteroatoms selected from nitrogen, oxygen, sulfur, and phosphorus.

In one embodiment, in the groups described herein, one or more (e.g., 1, 2, 3, or 4) ring atoms of the 5- or 6-membered monocyclic heteroaryl are heteroatoms selected from nitrogen, oxygen, sulfur, and phosphorus.

In one embodiment, in the groups described herein, one or more (e.g., 1, 2, 3, 4, or 5) ring atoms of the 8- to 10-membered bicyclic heteroaryl are heteroatoms selected from nitrogen, oxygen, sulfur, and phosphorus.

In one embodiment, in the groups described herein, one or more (e.g., 1, 2, 3, or 4) ring atoms of the 3- to 6-membered monocyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 5- to 15-membered tricyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 6- to 12-membered heteroaryl-fused cycloalkyl are heteroatoms selected from nitrogen, oxygen, sulfur, and phosphorus.

In one embodiment, in the groups described herein, one or more (e.g., 1, 2, 3, 4, or 5) ring atoms of the 6- to 12-membered heteroaryl-fused heterocyclyl are heteroatoms selected from nitrogen, oxygen, sulfur, and phosphorus.

In one embodiment, in the groups described herein, when the ring atom is a sulfur atom, the sulfur atom is optionally substituted with one or two groups selected from oxo and =NR^{e}; R^{e} is as defined above.

In one embodiment, in the groups described herein, when the ring atom is a phosphorus atom, the sulfur atom is optionally substituted with one or two groups selected from oxo and =NR^{e}; R^{e} is as defined above.

A first aspect of the present disclosure provides a compound represented by formula (I7), or a stable deuterated derivative thereof, or a stereoisomer thereof, or a pharmaceutically acceptable salt thereof, a solvate thereof, or a prodrug thereof: 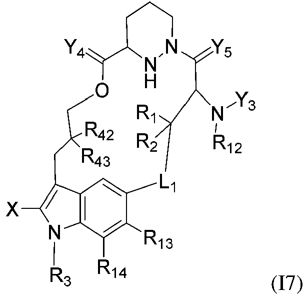 wherein in the formula,
X is 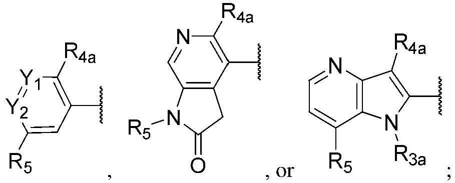
Y₁ is N or CH, wherein N may be oxidized (N⁺-O⁻);
Y₂ is N or CH, wherein N may be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), - S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), - S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), -C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, - C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, - C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, - C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkylS(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, - C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, - NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C_{1 6} alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

Alternatively, provided is a compound represented by formula (16), or a stable deuterated derivative thereof, or a stereoisomer thereof, or a pharmaceutically acceptable salt thereof, a solvate thereof, or a prodrug thereof: 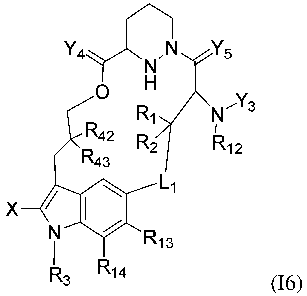 wherein in the formula,
X is 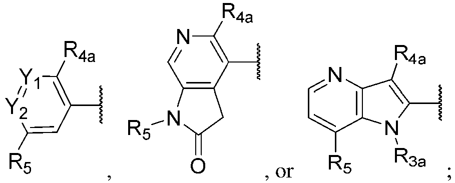
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, or substituted or unsubstituted C₃₋₆ cycloalkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), - S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), - S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, - C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{bl}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, - C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, - NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8-to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C ₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

Alternatively, provided is a compound represented by formula (I5), or a stable deuterated derivative thereof, or a stereoisomer thereof, or a pharmaceutically acceptable salt thereof, a solvate thereof, or a prodrug thereof: 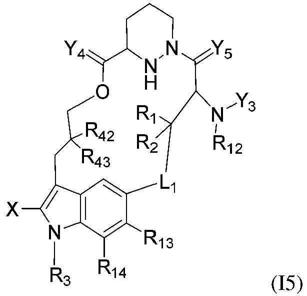 wherein in the formula,
X is 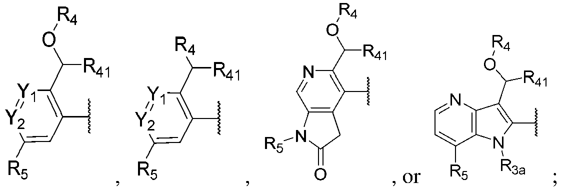
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), - S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₇), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, - C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, - C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, - NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C_{2-\6} alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

Alternatively, provided is a compound represented by formula (14), or a stable deuterated derivative thereof, or a stereoisomer thereof, or a pharmaceutically acceptable salt thereof, a solvate thereof, or a prodrug thereof: 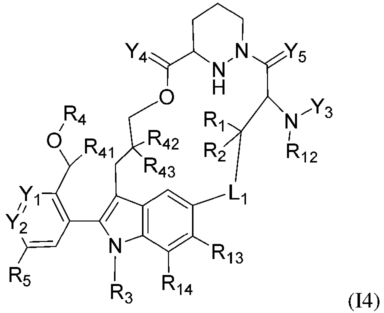 wherein in the formula,
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, - C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆ or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, and sulfur;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, - C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, - C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, - NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 7- to 12-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur; one or more (e.g., 1, 2, 3, 4, or 5) ring atoms of the 5- to 15-membered tricyclic heterocyclyl are heteroatoms selected from nitrogen, oxygen, and sulfur; one or more (e.g., 1, 2, 3, 4, or 5) ring atoms of the 6- to 12-membered heteroaryl-fused cycloalkyl are heteroatoms selected from nitrogen, oxygen, and sulfur; one or more (e.g., 1, 2, 3, 4, or 5) ring atoms of the 6- to 12-membered heteroaryl-fused heterocyclyl are heteroatoms selected from nitrogen, oxygen, and sulfur;
wherein when the ring atom is a sulfur atom, the sulfur atom is optionally substituted with one or two groups selected from oxo and =NR^{e}; R^{e} is as defined above.

Alternatively, provided is a compound represented by formula (I3), or a stable deuterated derivative thereof, or a stereoisomer thereof, or a pharmaceutically acceptable salt thereof, a solvate thereof, or a prodrug thereof: 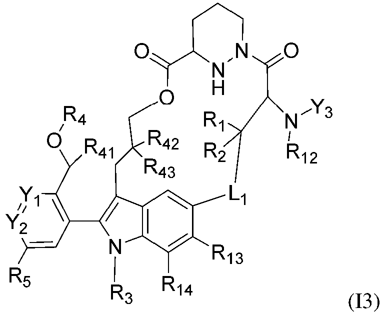 wherein in the formula,
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, - C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆ or R₁₁;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₁₂ is hydrogen, C₁₋₆ alkyl, or deuterated C₁₋₆ alkyl;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, - C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, - C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, - NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 7- to 12-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur; one or more (e.g., 1, 2, 3, 4, or 5) ring atoms of the 5- to 15-membered tricyclic heterocyclyl are heteroatoms selected from nitrogen, oxygen, and sulfur; one or more (e.g., 1, 2, 3, 4, or 5) ring atoms of the 6- to 12-membered heteroaryl-fused cycloalkyl are heteroatoms selected from nitrogen, oxygen, and sulfur; one or more (e.g., 1, 2, 3, 4, or 5) ring atoms of the 6- to 12-membered heteroaryl-fused heterocyclyl are heteroatoms selected from nitrogen, oxygen, and sulfur; wherein when the ring atom is a sulfur atom, the sulfur atom is optionally substituted with one or two groups selected from oxo and =NR^{e}; R^{e} is as defined above.

Alternatively, provided is a compound represented by formula (12), or a stable deuterated derivative thereof, or a stereoisomer thereof, or a pharmaceutically acceptable salt thereof, a solvate thereof, or a prodrug thereof: 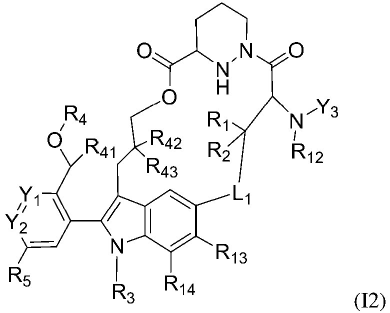 wherein in the formula,
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆ or R₁₁;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, or substituted or unsubstituted 6- to 12-membered heteroaryl-fused heterocyclyl;
R₁₂ is hydrogen, C₁₋₆ alkyl, or deuterated C₁₋₆ alkyl;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, - C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, - C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d i}-S(=O)₂-NR^{a1}R^{b1}, - NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 7- to 12-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur; one or more (e.g., 1, 2, 3, 4, or 5) ring atoms of the 5- to 15-membered tricyclic heterocyclyl are heteroatoms selected from nitrogen, oxygen, and sulfur; one or more (e.g., 1, 2, 3, 4, or 5) ring atoms of the 6- to 12-membered heteroaryl-fused cycloalkyl are heteroatoms selected from nitrogen, oxygen, and sulfur; one or more (e.g., 1, 2, 3, 4, or 5) ring atoms of the 6- to 12-membered heteroaryl-fused heterocyclyl are heteroatoms selected from nitrogen, oxygen, and sulfur; wherein when the ring atom is a sulfur atom, the sulfur atom is optionally substituted with one or two groups selected from oxo and =NR^{e} (for example, when the ring atom is a sulfur atom, the sulfur atom may be substituted to form S(=O), S(=O)₂, or S(=O)(=NR^{e}).); R^{e} is as defined above.

Alternatively, provided is a compound represented by formula (I1), or a stable deuterated derivative thereof, or a stereoisomer thereof, or a pharmaceutically acceptable salt thereof, a solvate thereof, or a prodrug thereof: 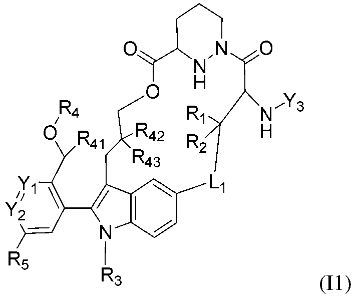 wherein in the formula,
Y₁ is N or CH;
Y₂ is N or CH;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R0₆)(-C₁₋₄ alkyl-NR₀₁R₀₂), -N(R₀₆)-S(=O)(-C₁₋₄ alkyl-NR₀₁R₀₂), - S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), - S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, or C₁₋₆ alkyl;
R₀₆ is hydrogen, deuterium, or C₁₋₆ alkyl;
Y₃ is -C(=O)R₆ or R₁₁;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, or substituted or unsubstituted 6- to 12-membered heteroaryl-fused heterocyclyl;
the substitutions each independently refer to that 1, 2, 3, 4, 5, or 6 hydrogen atoms on the group described above are substituted by a group selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, - C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, - C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, - C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl₋NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)2-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, - NR^{d1}_C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, - C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 7- to 12-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur; one or more (e.g., 1, 2, 3, 4, or 5) ring atoms of the 5- to 15-membered tricyclic heterocyclyl are heteroatoms selected from nitrogen, oxygen, and sulfur.

In one embodiment, R^{4a} is

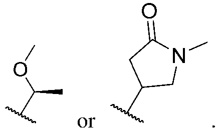

In one embodiment, R^{4a} is

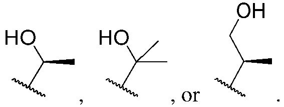

In one embodiment, R^{4a} is C₁₋₆ alkyl, preferably isopropyl.

In one embodiment, R_{4b} is deuterium, halogen, C₁₋₆ alkyl, or C₁₋₆ heteroalkyl.

In one embodiment, R_{4b} is deuterium, halogen, C₁₋₆ alkyl, or C₁₋₆ alkoxy.

In one embodiment, R_{4b} is chlorine, methyl, or methoxy.

In one embodiment, the compound of the present disclosure (including but not limited to the compound of formula (I), the compound of formula (I1), the compound of formula (I2), the compound of formula (I3), the compound of formula (14), the compound of formula (I5), the compound of formula (16), the compound of formula (I7), and the compound of formula (I8)) is represented by formula (I-1) or formula (I-2): 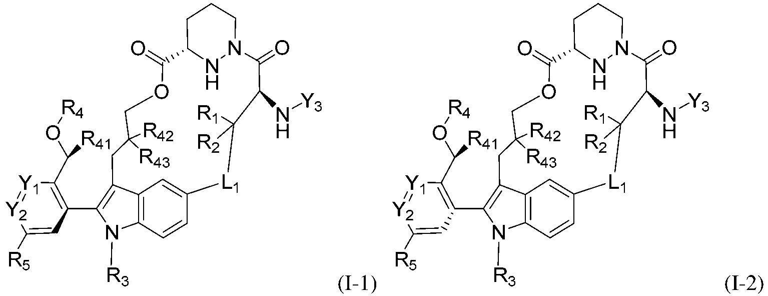 wherein in the formula, R₁, R₂, R₃, R₄, R₅, R₄₁, R₄₂, R₄₃, Y₁, Y₂, Y₃, and L₁ are each as defined herein.

In one embodiment, the compound of the present disclosure (including but not limited to the compound of formula (I), the compound of formula (I1), the compound of formula (I2), the compound of formula (I3), the compound of formula (14), the compound of formula (I5), the compound of formula (16), the compound of formula (I7), and the compound of formula (I8)) is represented by formula (1-11) or formula (I-12): 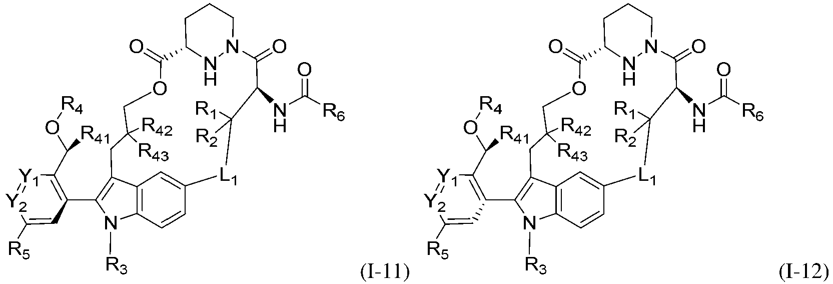 wherein in the formula, R₁, R₂, R₃, R₄, R₅, R₄₁, R₄₂, R₄₃, Y₁, Y₂, R₆, and L₁ are each as defined herein.

In one embodiment, the compound of the present disclosure (including but not limited to the compound of formula (I), the compound of formula (I1), the compound of formula (I2), the compound of formula (I3), the compound of formula (14), the compound of formula (I5), the compound of formula (16), the compound of formula (I7), and the compound of formula (I8)) is represented by formula (I-111), formula (I-121), formula (I'-111), or formula (I'-121): 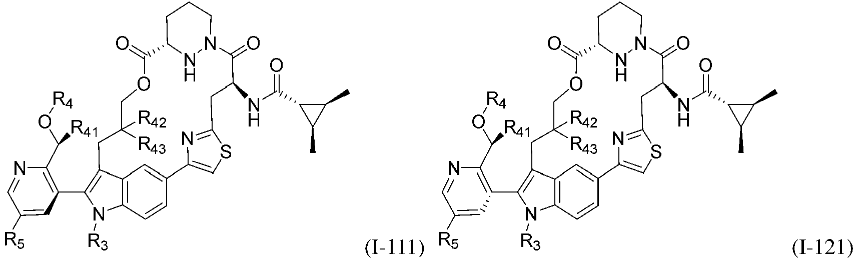 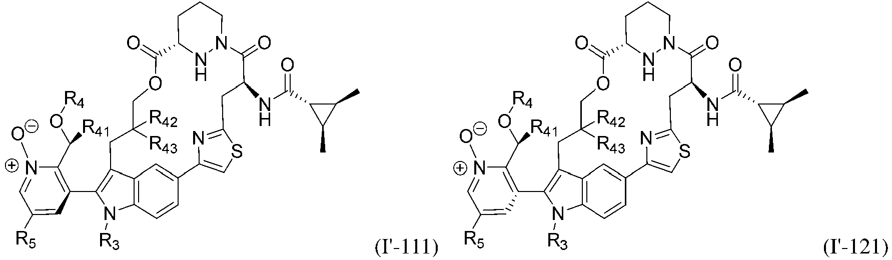 wherein in the formula, R₃, R₄, R₅, R₄₁, R₄₂, and R₄₃ are each as defined herein.

In one embodiment, the compound of the present disclosure (including but not limited to the compound of formula (I), the compound of formula (I1), the compound of formula (I2), the compound of formula (I3), the compound of formula (14), the compound of formula (I5), the compound of formula (16), the compound of formula (I7), and the compound of formula (I8)) is represented by formula (I-112), formula (I-122), formula (I'-112), or formula (I'-122): 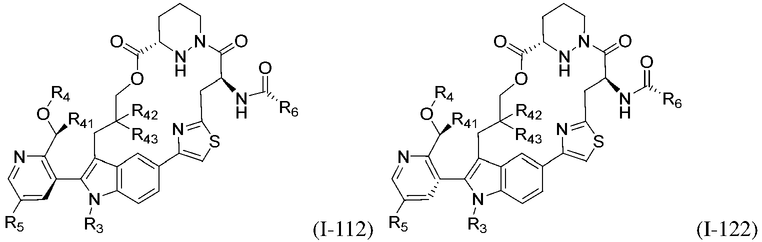 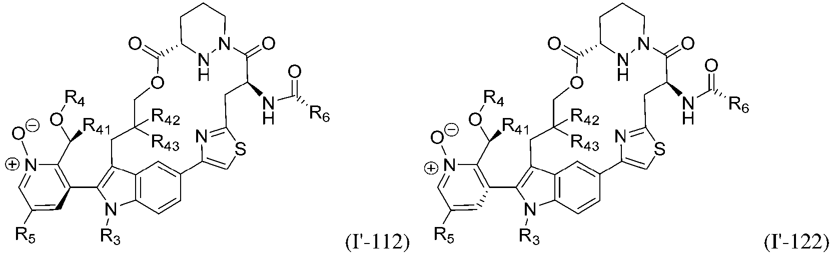 wherein in the formula, R₃, R₄, R₅, R₄₁, R₄₂, R₄₃, and R₄₆ are each as defined herein.

In one embodiment, the compound of the present disclosure (including but not limited to the compound of formula (I), the compound of formula (I1), the compound of formula (I2), the compound of formula (I3), the compound of formula (14), the compound of formula (I5), the compound of formula (16), the compound of formula (I7), and the compound of formula (I8)) is represented by formula (I-21) or formula (I-22): 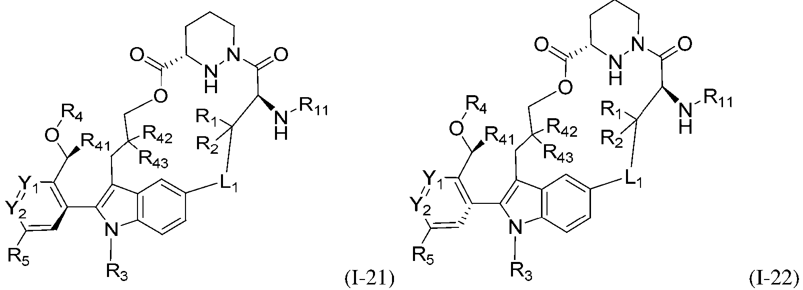 wherein in the formula, R₁, R₂, R₃, R₄, R₅, R₄₁, R₄₂, R₄₃, Y₁, Y₂, R₁₁, and L₁ are each as defined herein.

In one embodiment, the compound described above is represented by formula (I-3) or formula (I-4): 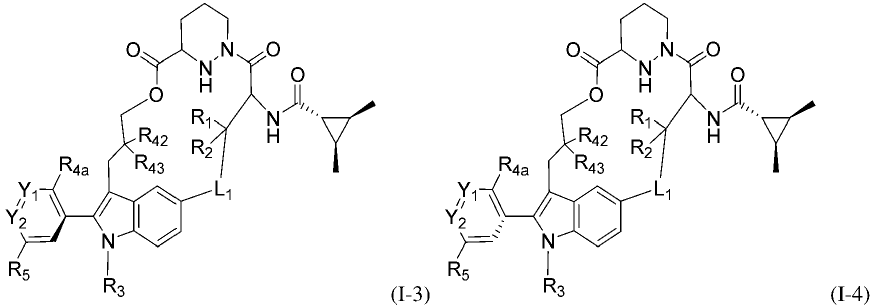 wherein in the formula, Y₁, Y₂, R₁, R₂, R₃, R₄ₐ, R₅, R₄₂, R₄₃, and L₁ are each as defined above.

In one embodiment, Y₁ is N, wherein N may be oxidized (N⁺-O⁻).

In one embodiment, Y₂ is CR_{4b}; R_{4b} is deuterium, halogen, C₁₋₆ alkyl, or substituted or unsubstituted C₁₋₆ heteroalkyl. In one embodiment, the compound of formula (I-3) is represented by formula (I-31) or formula (I-32): 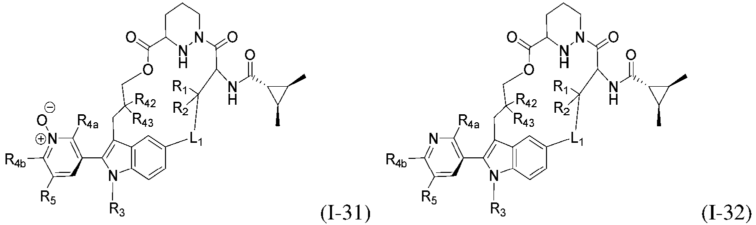 wherein in the formula, R₁, R₂, R₃, R₄ₐ, R₅, R₄₂, R₄₃, and L₁ are each as defined above.

In one embodiment, R_{4b} is halogen, C₁₋₆ alkyl, or C₁₋₆ alkoxy.

In one embodiment, R_{4b} is chlorine, methyl, or methoxy.

In one embodiment, R₄ₐ is C₁₋₆ alkyl, preferably isopropyl.

In one embodiment, R₃ is ethyl.

In one embodiment, L₁ is thiazolyl.

In one embodiment, R₄₃ and R₄₂ are methyl.

In one embodiment, R₁ and R₂ are hydrogen.

In one embodiment, R₅ is -NR₀₁R₀₂; R₀₁ and R₀₂, together with the nitrogen atom to which they are attached, form substituted or unsubstituted 7- to 12-membered bicyclic heterocyclyl; one or more (e.g., 1, 2, 3, 4, or 5) ring atoms of the 7- to 12-membered bicyclic heterocyclyl are heteroatoms selected from nitrogen, oxygen, and sulfur; the substitutions each independently refer to that 1, 2, 3, 4, 5, or 6 hydrogen atoms on the group described above are substituted by a group selected from group S1; the groups of group S1 are selected from =CR^{e}R^{f}; each R^{e} is independently H, halogen, C₁₋₆ alkyl, C₁₋₆ haloalkyl, or deuterated C₁₋₆ alkyl; each R^{f} is independently H, halogen, C₁₋₆ alkyl, C₁₋₆ haloalkyl, or deuterated C₁₋₆ alkyl. In one embodiment, R₅ is -O-C₁₋₄ alkyl-NR₀₁R₀₂; R₀₁ and R₀₂, together with the nitrogen atom to which they are attached, form substituted or unsubstituted 3- to 8-membered monocyclic heterocyclyl; one or more (e.g., 1, 2, 3, or 4) ring atoms of the 3- to 8-membered monocyclic heterocyclyl are heteroatoms selected from nitrogen, oxygen, and sulfur; the substitutions each independently refer to that 1, 2, 3, 4, 5, or 6 hydrogen atoms on the group described above are substituted by a group selected from group S1; wherein when the ring atom is a sulfur atom, the sulfur atom is optionally substituted with one or two groups selected from oxo and =NR^{e}; each R^{e} is independently H, halogen, C₁₋₆ alkyl, C₁₋₆ haloalkyl, or deuterated C₁₋₆ alkyl. In one embodiment, R₅ is -C₂₋₄ alkynyl-C₁₋₄ alkyl-NR₀₁R₀₂; R₀₁ and R₀₂, together with the nitrogen atom to which they are attached, form substituted or unsubstituted 3- to 8-membered monocyclic heterocyclyl; one or more (e.g., 1, 2, 3 or, 4) ring atoms of the 3- to 8-membered monocyclic heterocyclyl are heteroatoms selected from nitrogen, oxygen, and sulfur; the substitutions each independently refer to that 1, 2, 3, 4, 5, or 6 hydrogen atoms on the group described above are substituted by a group selected from group S1; wherein when the ring atom is a sulfur atom, the sulfur atom is optionally substituted with one or two groups selected from oxo and =NR^{e}; each R^{e} is independently H, halogen, C₁₋₆ alkyl, C₁₋₆ haloalkyl, or deuterated C₁₋₆ alkyl.

In one embodiment, the 7- to 12-membered bicyclic heterocyclyl is selected from the group consisting of:

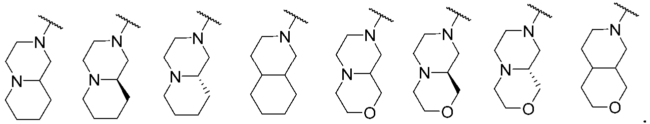

In one embodiment, the 3- to 8-membered monocyclic heterocyclyl is selected from the group consisting of:

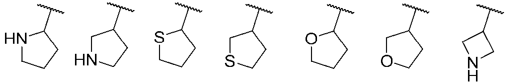

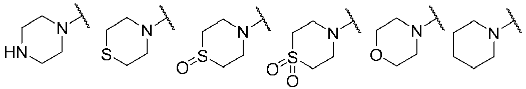

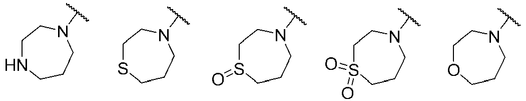

In one embodiment, the 3- to 8-membered monocyclic heterocyclyl is selected from the group consisting of:

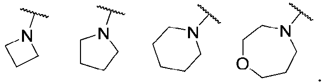

In one embodiment, the 3- to 8-membered monocyclic heterocyclyl is optionally substituted with a substituent selected from cyano, hydroxy, 5- or 6-membered monocyclic heteroaryl, -NR^{a1}R^{b1}, and -N=S(=O)(R^{g1})(R^{h1}).

In one embodiment, the 5- or 6-membered monocyclic heteroaryl is pyridine, and the nitrogen atom in the pyridine may optionally be oxidized (N⁺-O⁻).

In one embodiment, R^{a1} and R^{b1} are each independently H or phenyl; the phenyl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and - P(=O)-(C₁₋₆ alkyl)₂.

In one embodiment, R^{a1} and R^{b1} are each independently H or phenyl; the phenyl is optionally substituted with -P(=O)-(CH₃)₂. In one embodiment, R^{g1} and R^{h1} are each independently H, C₁₋₆ alkyl, or deuterated C₁₋₆ alkyl, preferably C₁₋₆ alkyl.

In one embodiment, R₅ is

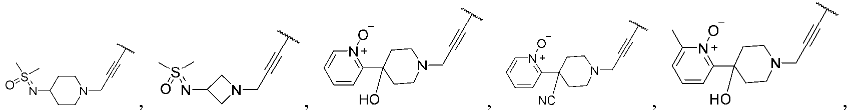

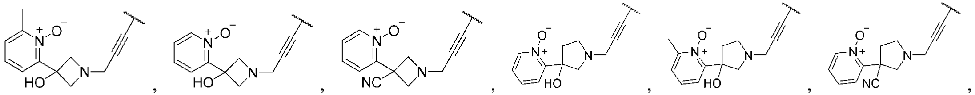

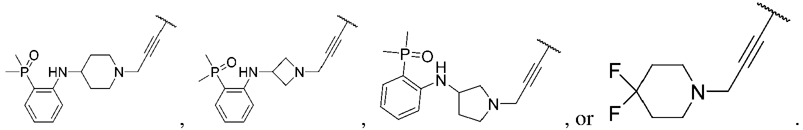

The first aspect of the present disclosure also provides a compound represented by formula (A) or formula (A'), or a stable deuterated derivative thereof, or a stereoisomer thereof, or a pharmaceutically acceptable salt thereof, a solvate thereof, or a prodrug thereof: 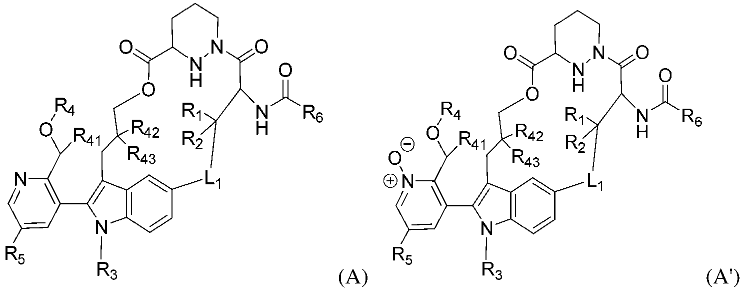 wherein in the formula,
R₄₂ is hydrogen, halogen, or substituted or unsubstituted C₁₋₆ alkyl; R₄₃ is hydrogen, halogen, or substituted or unsubstituted C₁₋₆ alkyl; and R₄₂ and R₄₃ are not both methyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is -NR₀₁R₀₂ or -C(R₀₅)R₀₃R₀₄;
R₀₁ and R₀₂, together with the nitrogen atom to which they are attached, form substituted or unsubstituted 5- to 15-membered tricyclic heterocyclyl; the 5- to 15-membered tricyclic heterocyclyl has, in addition to the existing nitrogen atom, 0 or more (e.g., 0, 1, 2, 3, 4, or 5) ring atoms that are heteroatoms selected from nitrogen, oxygen, and sulfur;
R₀₃ and R₀₄, together with the carbon atom to which they are attached, form substituted or unsubstituted 9- to 11-membered bicyclic heterocyclyl, substituted or unsubstituted 5- to 15-membered tricyclic heterocyclyl, substituted or unsubstituted 5- or 6-membered monocyclic heteroaryl, or substituted or unsubstituted 8- to 10-membered bicyclic heteroaryl; one or more (e.g., 1, 2, 3, 4, or 5) ring atoms of the 5- to 15-membered tricyclic heterocyclyl are heteroatoms selected from nitrogen, oxygen, and sulfur;
R₀₅ is hydrogen, deuterium, or C₁₋₆ alkyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; the substitutions each independently refer to that 1, 2, 3, or 4 hydrogen atoms on the group described above are substituted by a group selected from group S1; the groups of group S1 are each independently selected from the group consisting of: deuterium, oxo (=O), thio (=S), =CR^{e}R^{f}, halogen, cyano, hydroxy, carboxyl, nitro, C₁₋₆ alkyl, C₂₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, - C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, - C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, - C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkylS(=O)₂-NR^{a1}R^{b1}, -C₁₋₄ alkyl-NR^{d1} -S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8-to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C ₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6;
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 9- to 11-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur.

In one embodiment, the compound is represented by formula (A-1), formula (A-2), formula (A'-1), or formula (A'-2): 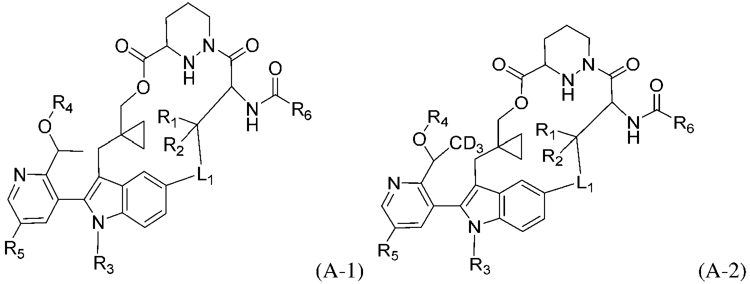 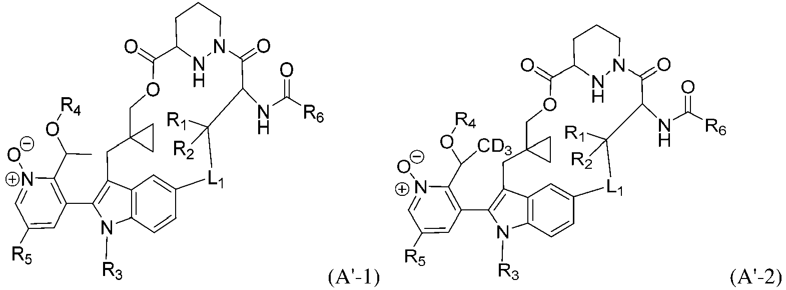 wherein in the formula, R₁, R₂, R₃, R₄, R₅, R₆, and L₁ are each as defined herein.

In one embodiment, the compound is represented by formula (A-3) or formula (A'-3): 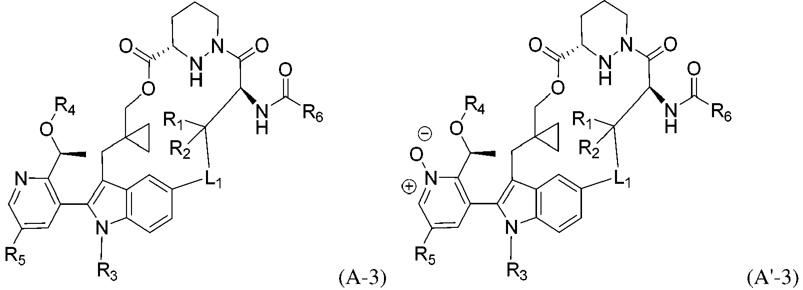 wherein in the formula, R₁, R₂, R₃, R₄, R₅, R₆, and L₁ are each as defined herein.

In one embodiment, the compound is represented by formula (A-4) or formula (A'-4): 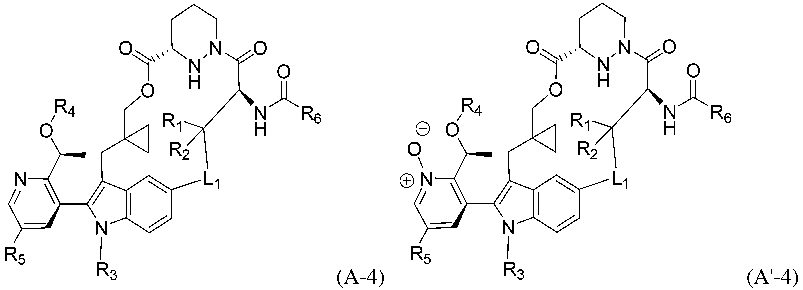 wherein in the formula, R₁, R₂, R₃, R₄, R₅, R₆, and L₁ are each as defined herein.

In one embodiment, R₆ is substituted or unsubstituted C₃₋₆ cycloalkyl; the substitution is as defined herein.

In one embodiment, R₆ is cyclopropyl substituted with C₁₋₆ alkyl. Preferably, R₆ is cyclopropyl substituted with methyl.

Preferably, R₆ is cyclopropyl substituted with two methyl groups. For example, R₆ is

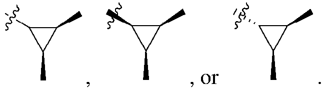

In one embodiment, R₄ is methyl or deuterated methyl (e.g., CD₃).

In one embodiment, R₄₁ is methyl or deuterated methyl (e.g., CD₃).

In one embodiment, R₅ is 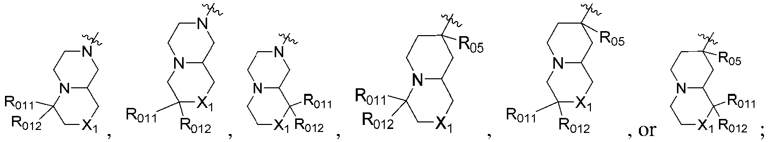
R₀₁₁ and R₀₁₂ are each independently hydrogen, C₁₋₆ alkyl, C₁₋₆ haloalkyl, or deuterated C₁₋₆ alkyl; or R₀₁₁ and R₀₁₂, together with the carbon atom to which they are attached, form substituted or unsubstituted C₃₋₆ cycloalkyl; the substitution is as defined herein;
X₁ is a bond, S, O, or N(R₀₁₃); R₀₁₃ is hydrogen, C₁₋₆ alkyl, or deuterated C₁₋₆ alkyl;
R₀₅ is hydrogen, deuterium, or C₁₋₆ alkyl.

In one embodiment, R₅ is

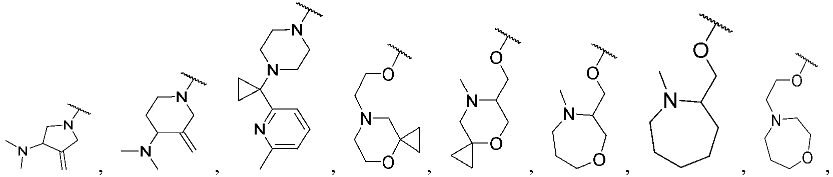

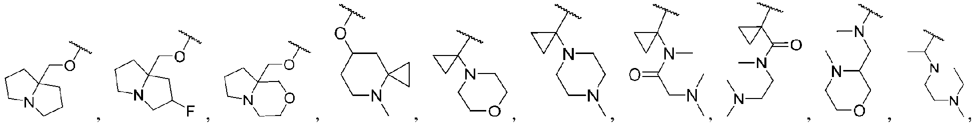

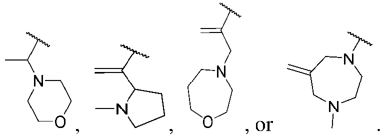

In one embodiment, R₅ is

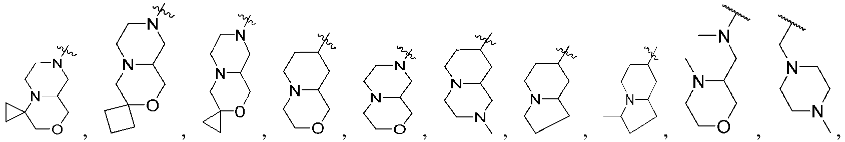

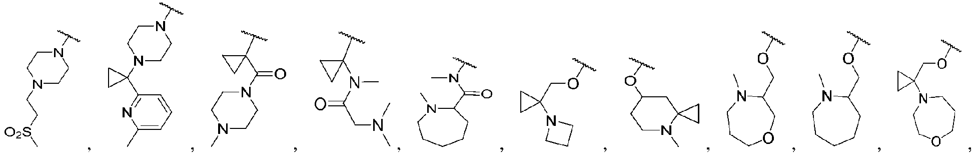

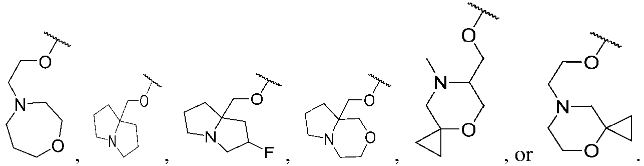

In one embodiment, R₅ is 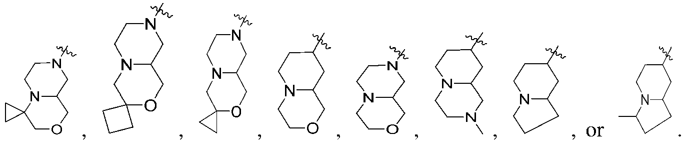 Preferably, R₅ is

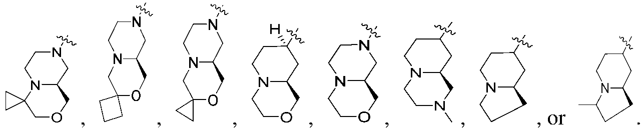

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₃ is hydrogen, C₁₋₆ alkyl, or C₁₋₆ haloalkyl.

In one embodiment, R₃ is trifluoromethyl, trifluoroethyl, or trifluoropropyl.

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, R₁ is hydrogen; R₂ is hydrogen.

In one embodiment, R₁ is ethoxy; R₂ is hydrogen.

In one embodiment, L₁ is 5- or 6-membered monocyclic heteroaryl.

In one embodiment, L₁ is thiazolyl.

In one embodiment, R₄₂ is methyl, and R₄₃ is trifluoromethyl.

In one embodiment, R₄₂ is hydrogen, and R₄₃ is trifluoromethyl.

In one embodiment, R₄₂ is fluorine, and R₄₃ is trifluoromethyl.

In one embodiment, R₄₂ is hydrogen, and R₄₃ is fluorine.

In one embodiment, R₄₂ is hydrogen, and R₄₃ is methyl.

In one embodiment, R₄₂ and R₄₃, together with the carbon atom to which they are attached, form cyclopropyl or cyclobutyl.

In one embodiment, R₄₂ and R₄₃ are not both methyl.

In one embodiment, R₄₂ is hydrogen; R₄₃ is hydrogen, halogen, or substituted or unsubstituted C₁₋₆ alkyl.

In one embodiment, R₄₂ and R₄₃, together with the carbon atom to which they are attached, form substituted or unsubstituted cyclopropyl, substituted or unsubstituted cyclobutyl, or substituted or unsubstituted cyclopentyl.

In one embodiment, R₄₂ and R₄₃, together with the carbon atom to which they are attached, form unsubstituted cyclopropyl.

In one embodiment, R₆ is halogen-substituted or unsubstituted C₁₋₆ alkyl.

In one embodiment, R₄₂ and R₄₃ are both methyl.

In one embodiment, R₅ is hydrogen or methylpiperazinyl.

In one embodiment, R₁₄ is hydrogen.

In one embodiment, R₁₃ is hydrogen.

In one embodiment, R₁₂ is hydrogen.

In one embodiment, R₄₂ and R₄₃ are both methyl.

In one embodiment, R₆ is substituted or unsubstituted C₁₋₆ alkyl, or substituted or unsubstituted C₃₋₆ cycloalkyl.

In one embodiment, R₆ is C₁₋₆ alkyl substituted with halogen, preferably C₁₋₆ alkyl substituted with F.

In one embodiment, R₆ is substituted or unsubstituted cyclopropyl.

In one embodiment, R₆ is cyclopropyl substituted with C₁₋₆ alkyl, preferably cyclopropyl substituted with one or two methyl groups.

In one embodiment, R₆ is

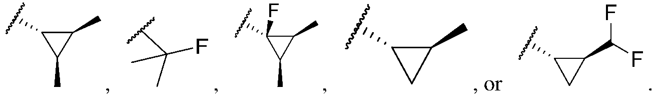

In one embodiment, R₆ is

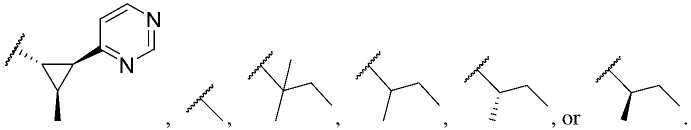

In one embodiment, R₆ is

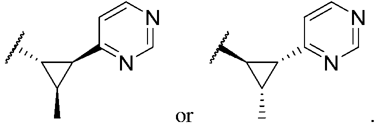

In one embodiment, R₆ is

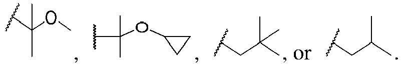

The first aspect of the present disclosure also provides a compound represented by formula (C), or a stable deuterated derivative thereof, or a stereoisomer thereof, or a pharmaceutically acceptable salt thereof, a solvate thereof, or a prodrug thereof: 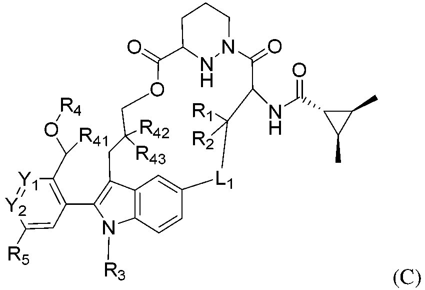 wherein in the formula, Y₁, Y₂, R₁, R₂, R₃, R₄, R₅, R₄₁, R₄₂, R₄₃, and L₁ are each as defined herein.

In one embodiment, R₅ is -NR₀₁R₀₂, -CR₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C(R₀₅)R₀₃R₀₄, -C₁₋₄ alkyl-NR₀₁R₀₂, -C₁₋₄ alkyl-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -O-C₁₋₄ alkyl-3- to 8-membered monocyclic heterocyclyl, -O-C₁₋₄ alkyl-5- to 15-membered tricyclic heterocyclyl, -O-C₁₋₄ alkyl-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl;
R₀₁ and R₀₂ are each independently selected from C₁₋₆ alkyl; or,
R₀₁ and R₀₂, together with the nitrogen atom to which they are attached, form 5- to 8-membered monocyclic heterocyclyl;
the C₁₋₆ alkyl or 5- to 8-membered monocyclic heterocyclyl is optionally substituted with a substituent selected from the aforementioned group S1;
further, the C₁₋₆ alkyl is optionally substituted with -C₁₋₄ alkyl-3- to 20-membered heterocyclyl or -C(=O)-3- to 20-membered heterocyclyl, and the 3- to 20-membered heterocyclyl is optionally substituted with a substituent selected from the aforementioned group S2.
R₀₃ and R₀₄ are each independently selected from substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6.

The C₁₋₆ alkyl, C₁₋₆ alkoxy, C₂₋₆ alkenyl, and C₂₋₆ alkynyl are optionally substituted with a substituent selected from the aforementioned group S1.

In one embodiment, R₅ is

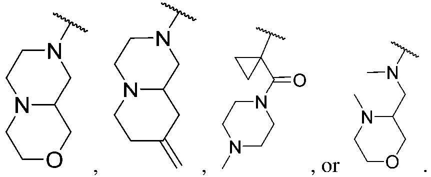

In one embodiment, R₅ is

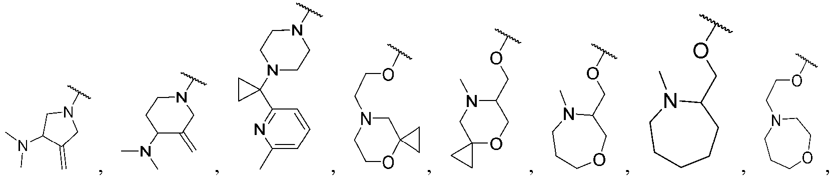

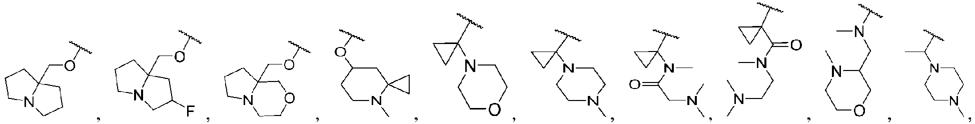

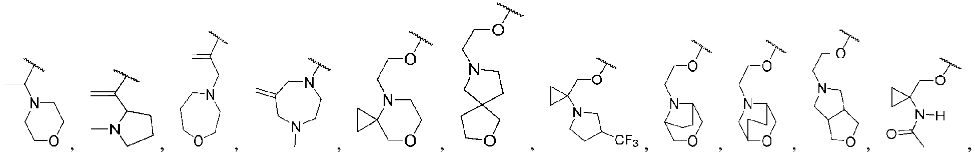

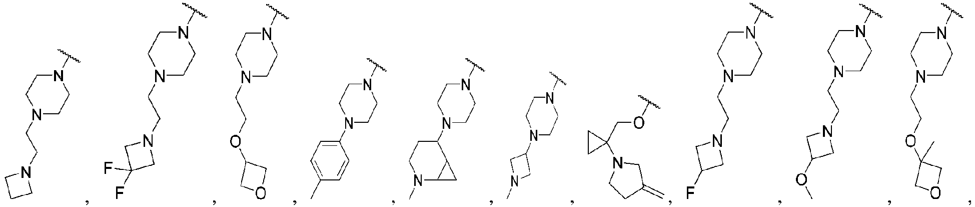

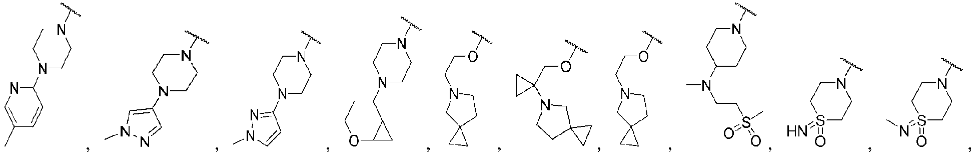

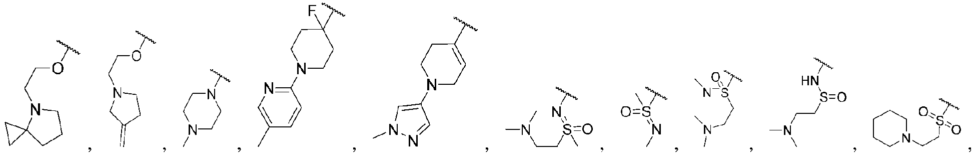

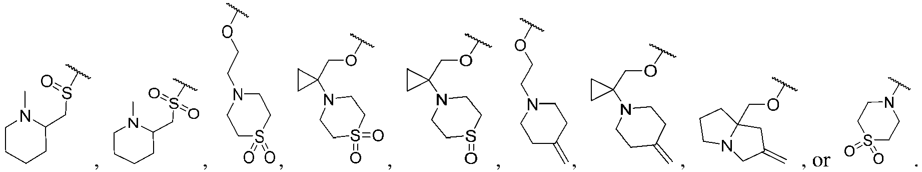

In one embodiment, R₅ is selected from the group consisting of:

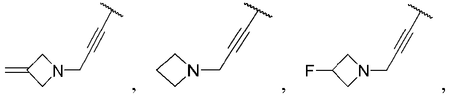

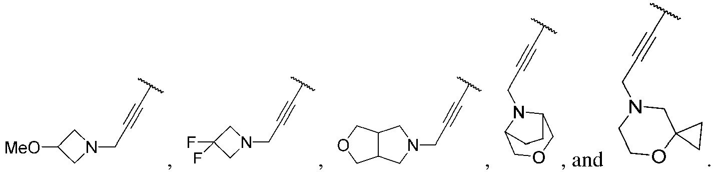

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, L₁ is 5- or 6-membered monocyclic heteroaryl.

In one embodiment, L₁ is thiazolyl.

The first aspect of the present disclosure also provides a compound represented by formula (B), or a stable deuterated derivative thereof, or a stereoisomer thereof, or a pharmaceutically acceptable salt thereof, a solvate thereof, or a prodrug thereof: 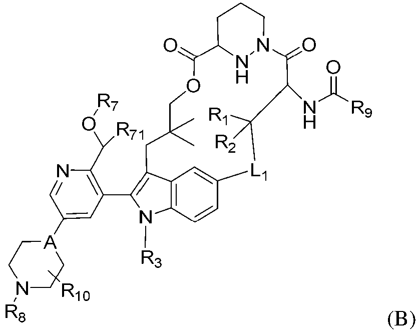 wherein in the formula,
A is N, C(H), or C(D);
L₁ is -N(R₀₀)C(=O)-, substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₇ is C₁₋₆ alkyl or deuterated C₁₋₆ alkyl;
R₇₁ is C₁₋₆ alkyl or deuterated C₁₋₆ alkyl;
R₈ is C₁₋₆ alkyl or deuterated C₁₋₆ alkyl; and when R₈ does not form a ring with R₁₀, at least one of R₇, R₇₁, and R₈ is deuterated C₁₋₆ alkyl;
R₉ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₀ is hydrogen, substituted or unsubstituted C₁₋₆ alkyl, or substituted or unsubstituted C₁₋₆ heteroalkyl;
the substitutions described above each independently refer to that 1, 2, 3, or 4 hydrogen atoms on the group described above are substituted by a group selected from group S1; the groups of group S1 are each independently selected from the group consisting of: deuterium, oxo (=O), thio (=S), =CR^{e}R^{f}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, - C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(-O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkylS(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the
C₃₋₂₀ cycloalkyl, the 3- to 20-membered heterocyclyl, the C₆₋₁₄ aryl, the 5- or 6-membered monocyclic heteroaryl, and the 8-to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
or R₈ and R₁₀, together with the atoms to which they are attached, form 3- to 20-membered heterocyclyl unsubstituted or substituted with 1, 2, 3, or 4 groups selected from group S2, 5- or 6-membered monocyclic heteroaryl unsubstituted or substituted with 1, 2, 3, or 4 groups selected from group S2, or 8- to 10-membered bicyclic heteroaryl unsubstituted or substituted with 1, 2, 3, or 4 groups selected from group S2;
in the groups described above, the groups of group S2 are each independently selected from the group consisting of: oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6;
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur.

In one embodiment, the compound is represented by formula (B-1) or formula (B-2): 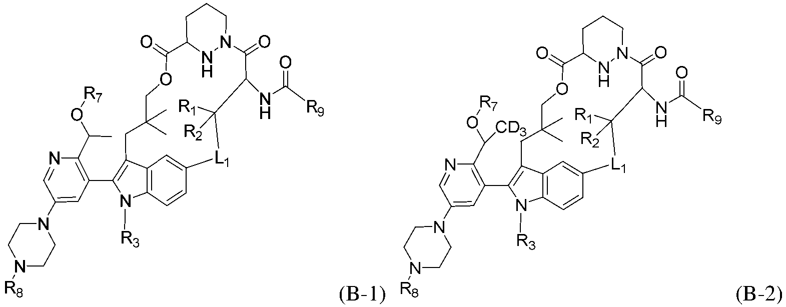 wherein in the formula, R₁, R₂, R₃, R₇, R₈, R₉, and L₁ are each as defined herein.

In one embodiment, the compound is represented by formula (B-3): 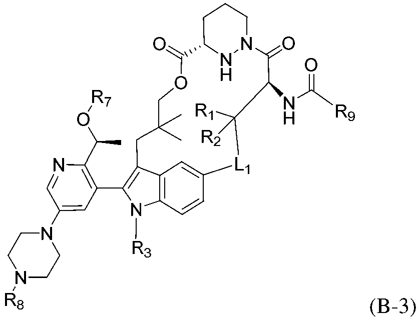 wherein in the formula, R₁, R₂, R₃, R₇, R₈, R₉, and L₁ are each as defined herein.

In one embodiment, the compound is represented by formula (B-4): 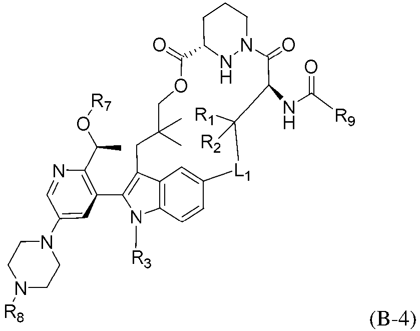 wherein in the formula, R₁, R₂, R₃, R₇, R₈, R₉, and L₁ are each as defined herein.

In one embodiment, R₇₁ is methyl or deuterated methyl.

In one embodiment, R₇ is methyl or deuterated methyl.

In one embodiment, R₈ is methyl or deuterated methyl.

In one embodiment, R₈ and R₁₀, together with the atoms to which they are attached, form 5-membered heterocyclyl unsubstituted or substituted with 1, 2, 3, or 4 groups selected from group S2, 6-membered heterocyclyl unsubstituted or substituted with 1, 2, 3, or 4 groups selected from group S2, or 5- or 6-membered monocyclic heteroaryl unsubstituted or substituted with 1, 2, 3, or 4 groups selected from group S2.

In one embodiment, R₉ is C₁₋₆ haloalkyl. Preferably, R₉ is fluoromethyl, fluoroethyl, fluoropropyl, or fluorobutyl.

In one embodiment, R₉ is substituted or unsubstituted C₃₋₆ cycloalkyl.

In one embodiment, R₉ is substituted or unsubstituted cyclopropyl. In one embodiment, R₉ is cyclopropyl substituted with C₁₋₆ alkyl, preferably cyclopropyl substituted with one or two methyl groups.

In one embodiment, R₉ is

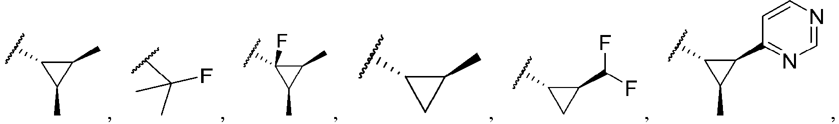

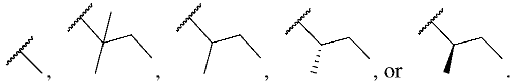

In one embodiment, R₃ is hydrogen or C₁₋₆ alkyl.

In one embodiment, R₃ is methyl, ethyl, or propyl (including isopropyl or n-propyl).

In one embodiment, R₁ is hydrogen or C₁₋₆ alkoxy; R₂ is hydrogen.

In one embodiment, R₁ is hydrogen, methoxy, ethoxy, propoxy, or butoxy; R₂ is hydrogen.

In one embodiment, L₁ is 5- or 6-membered monocyclic heteroaryl.

In one embodiment, L₁ is thiazolyl.

In one embodiment, the substituents of group S2 are selected from the group consisting of: halogen, oxo, methyl, ethyl, halomethyl, haloethyl, deuterated methyl, deuterated ethyl, and =CR^{e}R^{f}.

In one embodiment, R^{e} is each independently H, fluorine, methyl, ethyl, halomethyl, haloethyl, deuterated methyl, or deuterated ethyl; R^{f} is each independently H, fluorine, methyl, ethyl, halomethyl, haloethyl, deuterated methyl, or deuterated ethyl.

In one embodiment, the compound is selected from the group consisting of Z63 and Z66.

In one embodiment, the compound is selected from the group consisting of Z64, Z65, Z67, and Z68.

The first aspect of the present disclosure also provides a compound represented by formula (D), or a stable deuterated derivative thereof, or a stereoisomer thereof, or a pharmaceutically acceptable salt thereof, a solvate thereof, or a prodrug thereof: 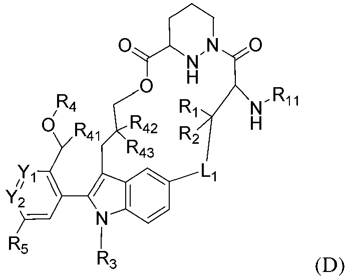 wherein in the formula, Y₁, Y₂, R₁, R₂, R₃, R₄, R₅, R₁₁, R₄₁, R₄₂, R₄₃, and L₁ are each as defined herein.

In one embodiment, the compound is represented by formula (D-1), formula (D-2), formula (D-3), formula (D-4), formula (D'-1), formula (D'-2), formula (D'-3), or formula (D'-4): 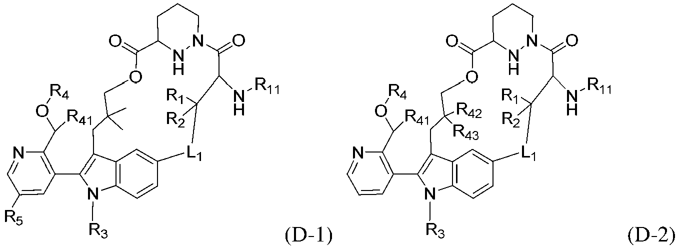 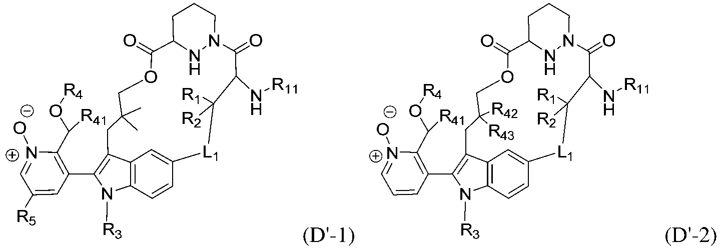 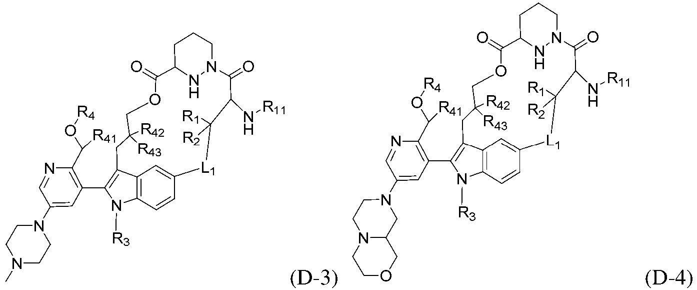 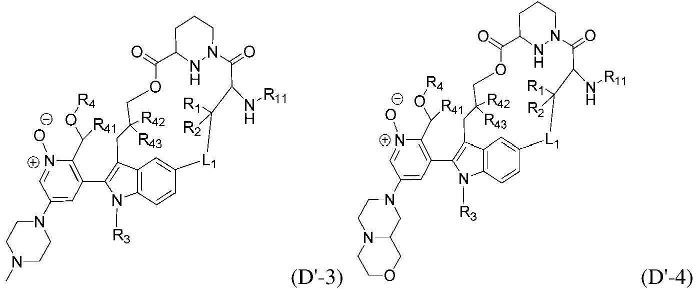 wherein in the formula, R₁, R₂, R₃, R₄, R₅, R₄₁, R₄₂, R₄₃, R₁₁, and L₁ are each as defined herein.

In one embodiment, the compound is represented by formula (D-11), formula (D-21), formula (D-31), formula (D-41), formula (D'-11), formula (D'-21), formula (D'-31), or formula (D'-41): 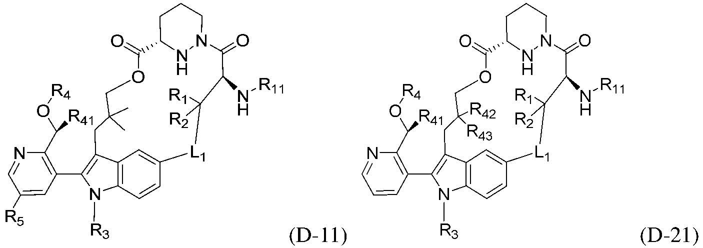 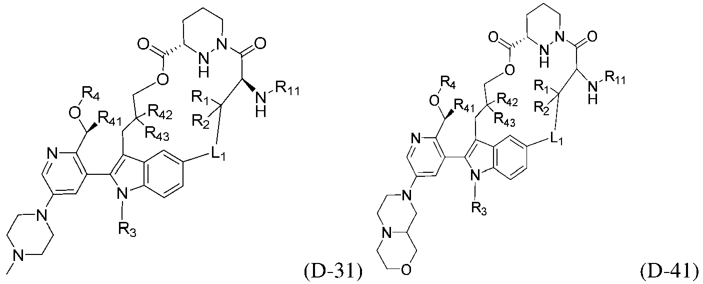 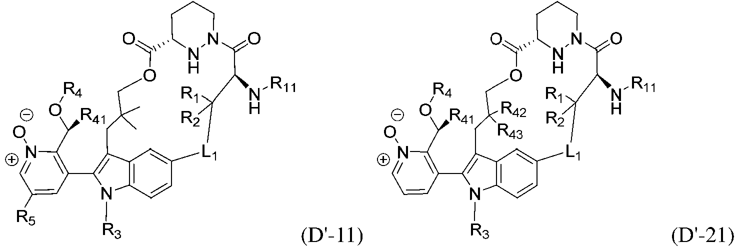 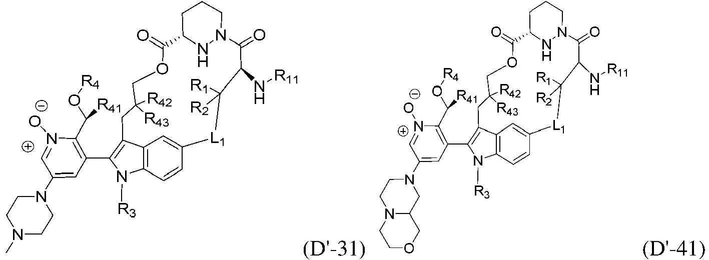 wherein in the formula, R₁, R₂, R₃, R₄, R₅, R₄₁, R₄₂, R₄₃, R₁₁, and L₁ are each as defined herein.

In one embodiment, R₁₁ is 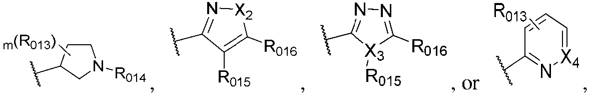
wherein X₂ is NH, O, or CR₀₁₃; X₃ and X₄ are each independently N or CR₀₁₃;
R₀₁₃ and R₀₁₄ are each independently hydrogen, halogen, oxo, C₁₋₆ alkyl, or C₁₋₆ alkoxy; m is 1, 2, or 3;
R₀₁₅ and R₀₁₆ are each independently hydrogen, C₁₋₆ alkyl, C₃₋₆ cycloalkyl, or -C₁₋₆ alkyl-5- or 6-membered monocyclic heteroaryl; or R₀₁₅ and R₀₁₆, together with the carbon atom(s) to which they are attached, form substituted or unsubstituted 3-to 8-membered monocyclic heterocyclyl; the substitution is as defined herein.

In one embodiment, R₁₁ is pyrrolidinyl, isoxazolyl, triazolyl, pyridazinyl, or triazolopyridinyl, wherein the pyrrolidinyl, isoxazolyl, triazolyl, pyridazinyl, or triazolopyridinyl is optionally substituted with a substituent selected from oxo, halogen, C₁₋₆ alkyl, C₁₋₆ alkoxy, C₃₋₆ cycloalkyl, and -C₁₋₄ alkyl-5- or 6-membered heteroaryl.

In one embodiment, R₁₁ is

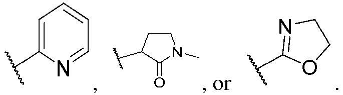

In one embodiment, R₁₁ is

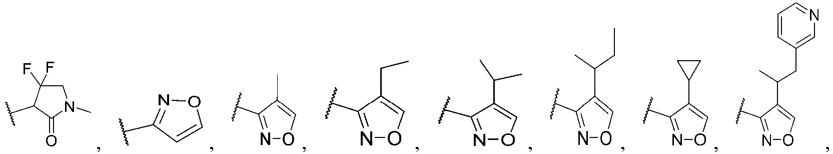

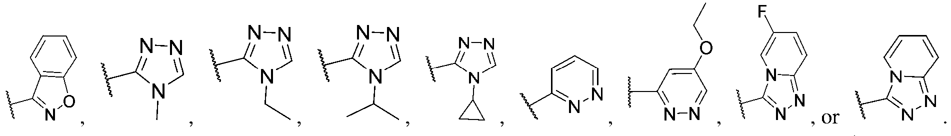

In one embodiment, R₁₁ is

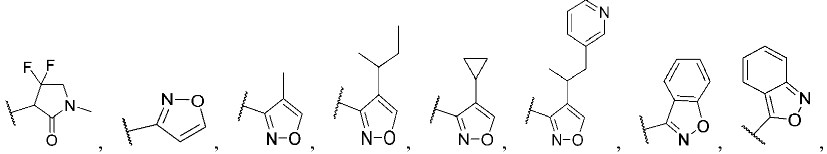

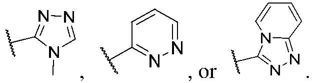

In one embodiment, R₁₁ is

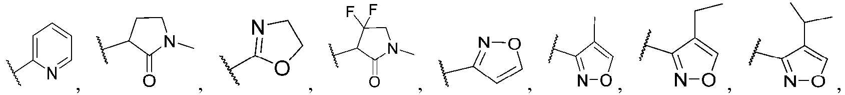

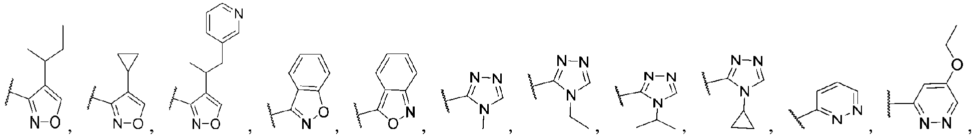

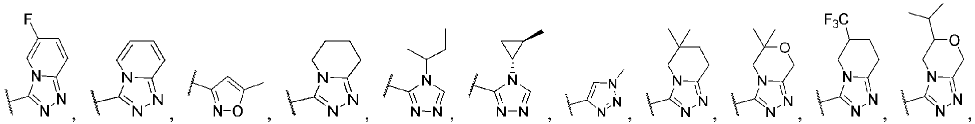

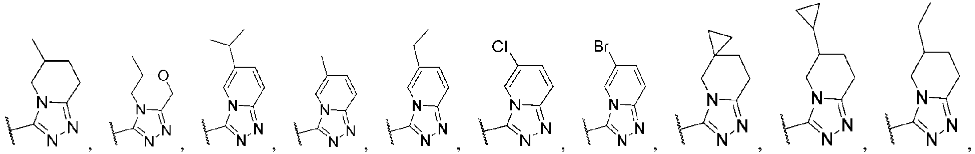

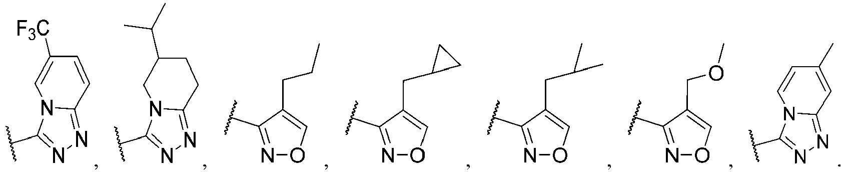

In one embodiment, R₁₁ is

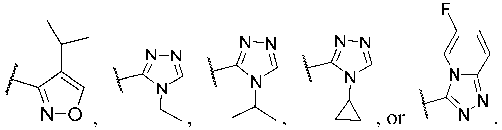

In one embodiment, R₁₁ is

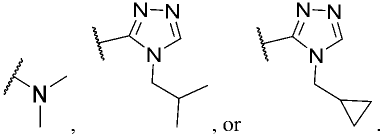

In one embodiment, R₁₁ is

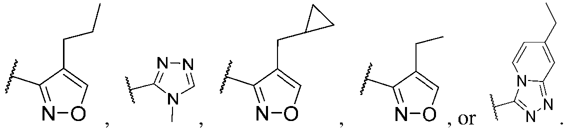

In one embodiment, R₁₁ is

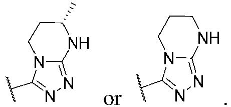

In one embodiment, R₃ is hydrogen or C₁₋₆ alkyl.

In one embodiment, R₃ is hydrogen, C₁₋₆ haloalkyl, or C₁₋₆ alkyl.

In one embodiment, R₃ is methyl, ethyl, or propyl (including isopropyl or n-propyl).

In one embodiment, R₃ is ethyl.

In one embodiment, R₃ is haloethyl. In one embodiment, R₃ is trifluoroethyl.

In one embodiment, R₁ is hydrogen or C₁₋₆ alkoxy; R₂ is hydrogen.

In one embodiment, R₁ is hydrogen, methoxy, ethoxy, propoxy, or butoxy; R₂ is hydrogen.

In one embodiment, R₁ is hydrogen; R₂ is hydrogen.

In one embodiment, L₁ is 5- or 6-membered monocyclic heteroaryl.

In one embodiment, L₁ is thiazolyl.

The first aspect of the present disclosure also provides a compound represented by formula (E), or a stable deuterated derivative thereof, or a stereoisomer thereof, or a pharmaceutically acceptable salt thereof, a solvate thereof, or a prodrug thereof: 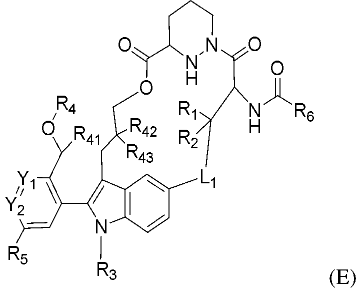 wherein in the formula, R₁, R₂, R₃, R₄, R₅, R₆, R₄₁, R₄₂, R₄₃, Y₁, Y₂, and L₁ are each as defined herein.

In one embodiment, the compound of formula (E) is represented by formula (E1) or (E2): 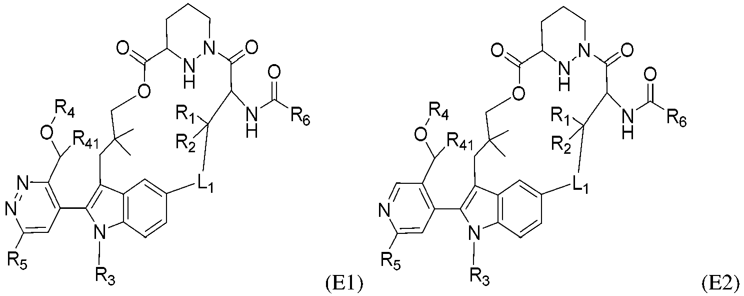 wherein in the formula, R₁, R₂, R₃, R₄, R₅, R₆, R₄₁, and L₁ are each as defined herein.

In one embodiment, R₆ is substituted or unsubstituted C₁₋₆ alkyl, or substituted or unsubstituted C₃₋₆ cycloalkyl.

In one embodiment, R₆ is C₁₋₆ alkyl substituted with halogen, preferably C₁₋₆ alkyl substituted with F.

In one embodiment, R₆ is cyclopropyl substituted with C₁₋₆ alkyl, preferably cyclopropyl substituted with one or two methyl groups.

In one embodiment, R₆ is

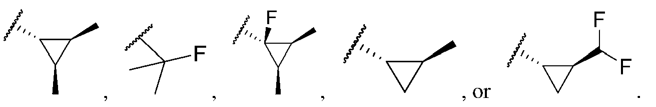

In one embodiment, R₅ is -NR₀₁R₀₂, -CR₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C(R₀₅)R₀₃R₀₄, -C₁₋₄ alkyl-NR₀₁R₀₂, -C₁₋₄ alkyl-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -O-C₁₋₄ alkyl-3- to 8-membered monocyclic heterocyclyl, -O-C₁₋₄ alkyl-5- to 15-membered tricyclic heterocyclyl, -O-C₁₋₄ alkyl-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl;
R₀₁ and R₀₂ are each independently selected from C₁₋₆ alkyl; or,
R₀₁ and R₀₂, together with the nitrogen atom to which they are attached, form 5- to 8-membered monocyclic heterocyclyl; the C₁₋₆ alkyl or 5- to 8-membered monocyclic heterocyclyl is optionally substituted with a substituent selected from the aforementioned group S1;
further, the C₁₋₆ alkyl is optionally substituted with -C₁₋₄ alkyl-3- to 20-membered heterocyclyl or -C(=O)-3- to 20-membered heterocyclyl, and the 3- to 20-membered heterocyclyl is optionally substituted with a substituent selected from the aforementioned group S2;
R₀₃ and R₀₄ are each independently selected from substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6;
the C₁₋₆ alkyl, C₁₋₆ alkoxy, C₂₋₆ alkenyl, and C₂₋₆ alkynyl are optionally substituted with a substituent selected from the aforementioned group S1.

In one embodiment, R₅ is -NR₀₁R₀₂, -C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C(R₀₅)R₀₃R₀₄, -C₁₋₄ alkyl-NR₀₁R₀₂, -C₁₋₄ alkyl-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(R₀₅)R₀₃R₀₄, -O-C₁₋₄ alkyl-3- to 8-membered monocyclic heterocyclyl, -O-C₁₋₄ alkyl-5- to 15-membered tricyclic heterocyclyl, -O-C₁₋₄ alkyl-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl, wherein each group is as defined herein.

In one embodiment, R₅ is

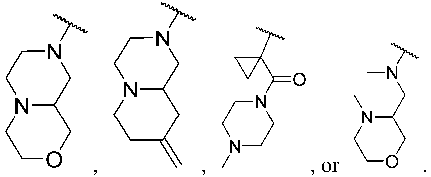

In one embodiment, R₅ is

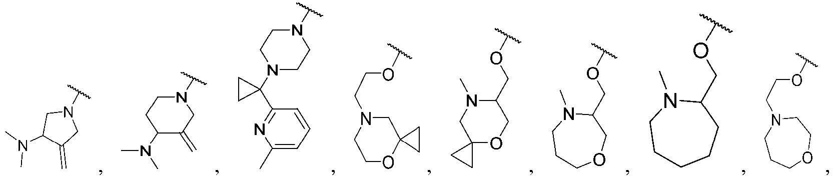

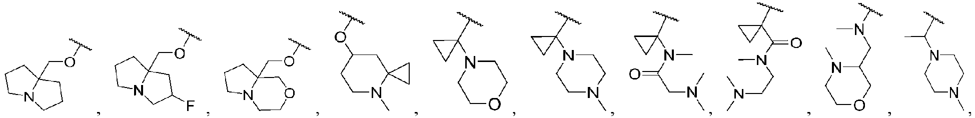

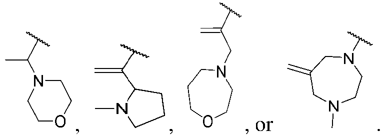

In one embodiment, R₅ is selected from the group consisting of:

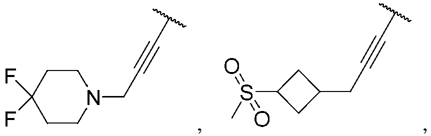

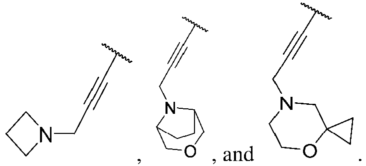

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, L₁ is 5- or 6-membered monocyclic heteroaryl.

In one embodiment, L₁ is thiazolyl.

In one embodiment, Y₁ is N; Y₂ is CH.

In one embodiment, Y₁ is CH; Y₂ is N.

In one embodiment, Y₁ is N; Y₂ is N.

In one embodiment, Y₁ is

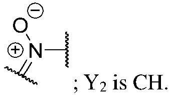

In one embodiment, Y₁ is CH; Y₂ is

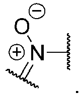

The first aspect of the present disclosure also provides a compound represented by formula (F) or formula (G), or a stable deuterated derivative thereof, or a stereoisomer thereof, or a pharmaceutically acceptable salt thereof, a solvate thereof, or a prodrug thereof: 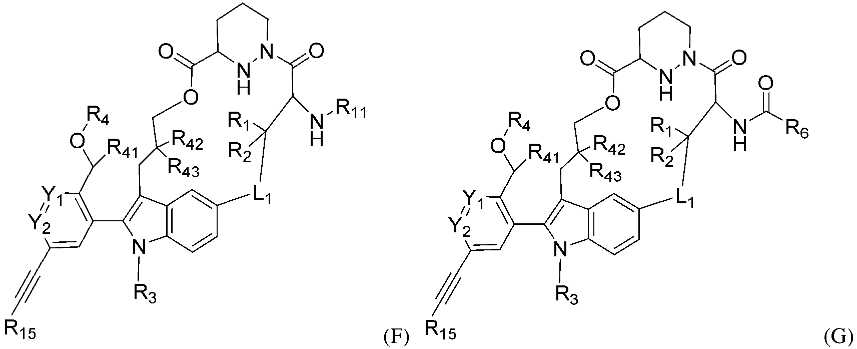
wherein in the formula, R₁, R₂, R₃, R₄, R₆, R₄₁, R₄₂, R₄₃, R₁₁, Y₁, Y₂, and L₁ are each as defined above;
R₁₅ is selected from -NR₀₁R₀₂, -C(=O)-NR₀₁R₀₂, -C(R₀₅)R₀₃R₀₄, -C₁₋₄ alkyl-NR₀₁R₀₂, and -C₁₋₄ alkyl-C(R₀₅)R₀₃R₀₄;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 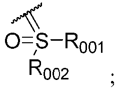 R₀₀₁, and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
the substitutions each independently refer to that 1, 2, 3, or 4 hydrogen atoms on the group described above are substituted by a group selected from group S1; the groups of group S1 are as defined above.

The first aspect of the present disclosure also provides a compound represented by formula (F1) or formula (G1), or a stable deuterated derivative thereof, or a stereoisomer thereof, or a pharmaceutically acceptable salt thereof, a solvate thereof, or a prodrug thereof: 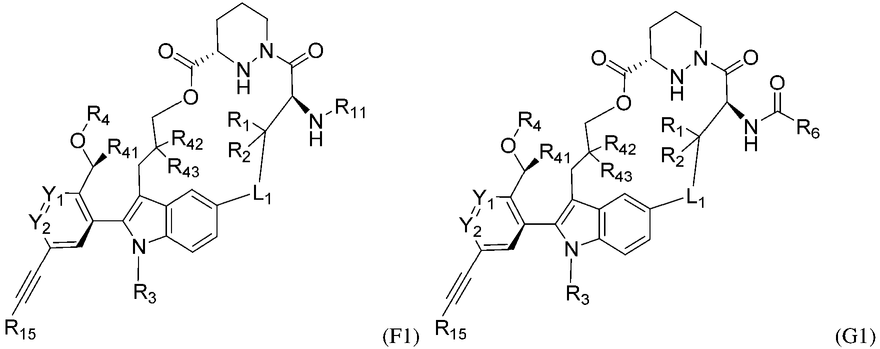
wherein in the formula, R₁, R₂, R₃, R₄, R₆, R₄₁, R₄₂, R₄₃, R₁₁, Y₁, Y₂, and L₁ are each as defined above;
R₁₅ is selected from -NR₀₁R₀₂, -C(=O)-NR₀₁R₀₂, -C(R₀₅)R₀₃R₀₄, -C₁₋₄ alkyl-NR₀₁R₀₂, and -C₁₋₄ alkyl-C(R₀₅)R₀₃R₀₄;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 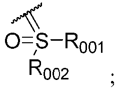 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
the substitutions each independently refer to that 1, 2, 3, or 4 hydrogen atoms on the group described above are substituted by a group selected from group S1; the groups of group S1 are as defined above.

In one embodiment, the compound is a compound prepared in any of the examples.

The aforementioned compounds of the present disclosure do not include the specific compound structures disclosed in the prior art for inhibiting RAS activity.

A second aspect of the present disclosure provides a pharmaceutical composition comprising the compound, or the stable deuterated derivative thereof, or the stereoisomer thereof, or the pharmaceutically acceptable salt thereof, the solvate thereof, or the prodrug thereof according to the first aspect described above, and a pharmaceutically acceptable carrier.

A third aspect of the present disclosure provides the aforementioned compound as a medicament.

As used herein, the term "pharmaceutically acceptable carrier" refers to any formulation or carrier medium capable of delivering an effective amount of the active substance of the present disclosure without interfering with the biological activity of the active substance and having no toxic and side effects on the host or subject. Representative carriers include water, oils, vegetables and minerals, cream bases, lotion bases, ointment bases, and the like. Such bases include suspending agents, thickeners, transdermal enhancers, and the like. Their formulations are well known to those skilled in the cosmetic or topical pharmaceutical field.

In the embodiments of the present disclosure, the pharmaceutical composition may be administered in any of the following ways: oral administration, spray inhalation, rectal administration, nasal administration, buccal administration, topical administration, parenteral administration (e.g., subcutaneous, intravenous, intramuscular, intraperitoneal, intrathecal, intraventricular, intrasternal, and intracranial injection or infusion), or administration by means of an implantable reservoir. Oral, intraperitoneal, or intravenous administration is preferred. When being administered orally, the compounds of the present disclosure may be prepared into any orally acceptable formulation forms, including but not limited to tablets, capsules, aqueous solutions, or aqueous suspensions. Carriers used for tablets generally include lactose and corn starch, and additionally, a lubricant such as magnesium stearate may be added. Diluents used for capsule formulations generally include lactose and dried corn starch. Aqueous suspension formulations are generally used by mixing the active ingredient with suitable emulsifying and suspending agents. If needed, sweetening, flavoring, or coloring agents may be added to the above oral formulation forms. When being administered topically, especially for treating affected areas or organs that are easily accessible for topical external application, such as eyes, skin, or lower intestinal neurological diseases, the compounds of the present disclosure may be formulated into different formulation forms for topical administration according to different affected areas or organs. When being administered topically to the eye, the compounds of the present disclosure may be formulated into a formulation form of a micronized suspension or solution, and the carrier used is isotonic sterile saline with a certain pH, with or without the addition of a preservative such as chlorinated benzyl alkanolate. For ophthalmic use, the compound may also be formulated into ointments, such as vaseline. When being administered topically to the skin, the compounds of the present disclosure may be formulated into a formulation form of a suitable ointment, lotion, or cream, in which the active ingredient is suspended or dissolved in one or more carriers. Carriers that can be used in the ointment formulations include, but are not limited to: mineral oil, liquid vaseline, white vaseline, propylene glycol, polyethylene oxide, polypropylene oxide, emulsifying wax, and water; carriers that can be used in the lotions or creams include, but are not limited to: mineral oil, sorbitan monostearate, Tween 60, cetyl ester wax, hexadecene aryl alcohol, 2-octyldodecanol, benzyl alcohol, and water. The compounds of the present disclosure may also be administered in the form of a sterile injectable formulation, including a sterile injectable aqueous or oil suspension or a sterile injectable solution. The carriers and solvents that may be used include water, Ringer's solution, and isotonic sodium chloride solution. In addition, sterilized non-volatile oils may also be used as solvents or suspending media, such as monoglycerides or diglycerides.

Another aspect of the present disclosure provides use of the compound, or the stable deuterated derivative thereof, or the stereoisomer thereof, or the pharmaceutically acceptable salt thereof, the solvate thereof, or the prodrug thereof according to the first aspect described above, or the pharmaceutical composition according to the second aspect described above in the preparation of a medicament for preventing and/or treating a disease or disorder, wherein the disease or disorder is a disease or disorder associated with RAS protein activity.

Another aspect of the present disclosure provides a method for preventing and/or treating a disease or disorder associated with RAS protein activity, and the method comprises: administering to a subject in need a therapeutically effective amount of the compound, or the stable deuterated derivative thereof, or the stereoisomer thereof, or the pharmaceutically acceptable salt thereof, the solvate thereof, or the prodrug thereof according to the first aspect of the present disclosure; or administering to a subject in need a therapeutically effective amount of the pharmaceutical composition according to the second aspect of the present disclosure.

Herein, in one embodiment, the disease or disorder associated with RAS protein activity is cancer, including but not limited to pancreatic cancer, colorectal cancer, non-small cell lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.

In one embodiment, the cancer comprises wild-type RAS and an RAS mutation. RAS includes HRAS, KRAS, and NRAS.

In one embodiment, the RAS mutation is KRAS G12C, KRAS G12D, KRAS G12V, KRAS G12S, KRAS G13C, KRAS G13D, KRAS Q61L, KRAS Q61H, NRAS G12C, NRAS Q61K, NRAS Q61R, or other RAS mutations.

Herein, the use or method further comprises administering an additional anti-cancer therapy. In some embodiments, the additional anti-cancer therapy is an HER2 inhibitor, an EGFR inhibitor, a second RAS inhibitor, an SHP2 inhibitor, an SOS1 inhibitor, an RAF inhibitor, an MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, or a combination thereof. Another aspect of the present disclosure provides a method for treating cancer in a subject in need thereof, and the method comprises:
(a) determining that the cancer is associated with RAS protein activity; and
(b) administering to the subject a therapeutically effective amount of the compound, or the stable deuterated derivative thereof, or the stereoisomer thereof, or the pharmaceutically acceptable salt thereof, the solvate thereof, or the prodrug thereof according to the first aspect of the present disclosure; or administering to the subject a therapeutically effective amount of the pharmaceutical composition according to the second aspect of the present disclosure.

In one embodiment, the administration is accomplished by a route selected from parenteral, intraperitoneal, intradermal, intracardiac, intraventricular, intracranial, intracerebrospinal, intracavitary, intrasynovial, intrathecal, intramuscular injection, intravitreal injection, intravenous injection, intraarterial injection, oral, buccal, sublingual, transdermal, topical, intratracheal, rectal, subcutaneous, and topical administrations.

Another aspect of the present disclosure provides a method for inhibiting RAS protein activity in a cell, and the method comprises a step of: contacting the cell with the compound, or the stable deuterated derivative thereof, or the stereoisomer thereof, or the pharmaceutically acceptable salt thereof, the solvate thereof, or the prodrug thereof according to the first aspect of the present disclosure; or contacting the cell with the pharmaceutical composition according to the second aspect of the present disclosure. The cell may be *in vivo* or *in vitro.*

Another aspect of the present disclosure provides use of the compound, or the stable deuterated derivative thereof, or the stereoisomer thereof, or the pharmaceutically acceptable salt thereof, the solvate thereof, or the prodrug thereof according to the first aspect described above, or the pharmaceutical composition according to the second aspect described above in the preparation of an RAS protein activity inhibitor.

Another aspect of the present disclosure provides a preparation method for the compound according to the first aspect described above, which comprises: 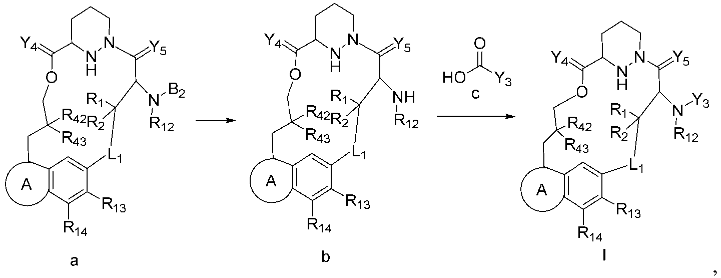 wherein in the formulas, B₂ is an amino protecting group well known in the art, such as alkoxycarbonyl (e.g., tert-butoxycarbonyl (Boc)), formyl, acyl (e.g., alkanoyl such as acetyl, trichloroacetyl, or trifluoroacetyl), arylmethoxycarbonyl (e.g., benzyloxycarbonyl (Cbz) and 9-fluorenylmethoxycarbonyl (Fmoc)), arylmethyl (e.g., benzyl (Bn), trityl (Tr), 1,1-di-(4'-methoxyphenyl)methyl), and silyl (e.g., trimethylsilyl (TMS) and tert-butyldimethylsilyl (TBS)). R₁, R₂, R₄₂, R₄₃, R₁₂, R₁₃, R₁₄, L₁, Y₃, Y₄, Y₅, and ring A are each as defined above.

The compound of formula (a) of the present disclosure may be synthesized using the following method, wherein the solvent, temperature, and other reaction conditions in each step are adapted to or similar to those described in the following examples, or using reaction conditions known in the art (e.g., the methods or conditions described in WO2022060836A1); 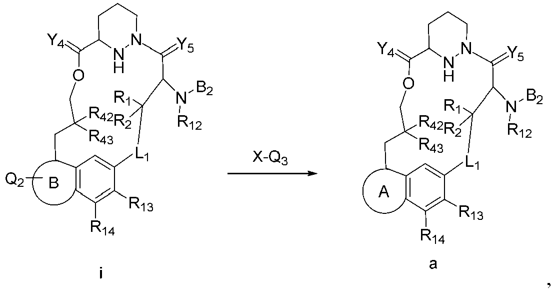 wherein in the formulas, B₂ is an amino protecting group well known in the art, such as alkoxycarbonyl (e.g., tert-butoxycarbonyl (Boc)), formyl, acyl (e.g., alkanoyl such as acetyl, trichloroacetyl, or trifluoroacetyl), arylmethoxycarbonyl (e.g., benzyloxycarbonyl (Cbz) and 9-fluorenylmethoxycarbonyl (Fmoc)), arylmethyl (e.g., benzyl (Bn), trityl (Tr), 1,1-di-(4'-methoxyphenyl)methyl), and silyl (e.g., trimethylsilyl (TMS) and tert-butyldimethylsilyl (TBS)). Q₂ and Q₃ are each independently selected from a halogen atom, a boric acid group, and a borate group; provided that when Q₂ is selected from a halogen atom, Q₃ is selected from a boric acid group and a borate group, and when Q₃ is selected from a halogen atom, Q₂ is selected from a boric acid group and a borate group; the boric acid group or borate group is selected from 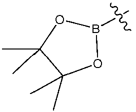 and -B(OH)₂. R₁, R₂, R₄₂, R₄₃, R₁₂, R₁₃, R₁₄, L₁, Y₃, Y₄, and Y₅ are each as defined above. 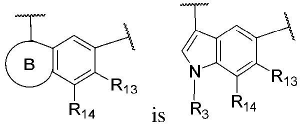 or

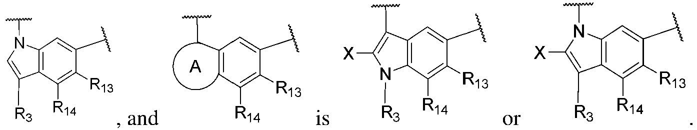

The compound of formula (i) of the present disclosure may be synthesized using the following method, wherein the solvent, temperature, and other reaction conditions in each step are adapted to or similar to those described in the following examples, or using reaction conditions known in the art; 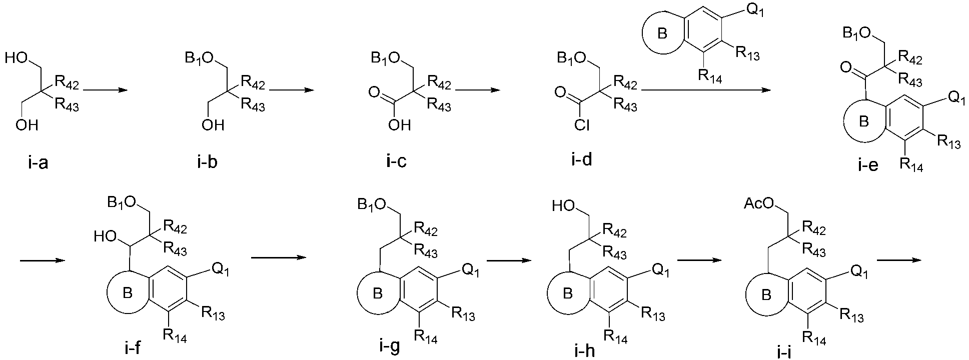 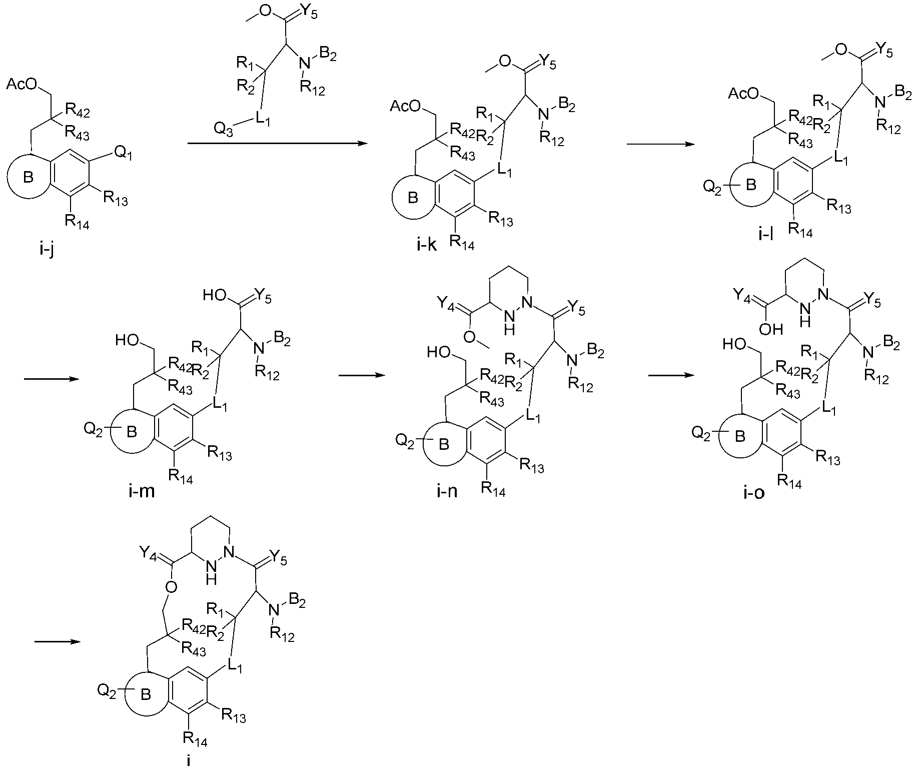 wherein in the formulas, B₁ is a hydroxy protecting group well known in the art, such as ethers, for example, methyl, tert-butyl, benzyl, methoxybenzyl, trityl, and silyl (trimethylsilyl (TMS), tert-butyldimethylsilyl (TBDMS), tert-butyldiphenylsilyl (TBDPS), triethylsilyl (TES), and triisopropylsilyl (TIPS)) ethers, sulfonyl chloride (e.g., trifluoromethanesulfonyl (Tf)), and acetals (e.g., MOM and THP). B₂ is an amino protecting group well known in the art, such as alkoxycarbonyl (e.g., tert-butoxycarbonyl (Boc)), formyl, acyl (e.g., alkanoyl such as acetyl, trichloroacetyl, or trifluoroacetyl), arylmethoxycarbonyl (e.g., benzyloxycarbonyl (Cbz) and 9-fluorenylmethoxycarbonyl (Fmoc)), arylmethyl (e.g., benzyl (Bn), trityl (Tr), 1,1-di-(4'-methoxyphenyl)methyl), and silyl (e.g., trimethylsilyl (TMS) and tert-butyldimethylsilyl (TBS)). Q₁ and Q₂ are each independently selected from a halogen atom, a boric acid group, and a borate group; provided that when Q₂ is selected from a halogen atom, Q₁ is selected from a boric acid group and a borate group, and when Q₁ is selected from a halogen atom, Q₂ is selected from a boric acid group and a borate group; the boric acid group or borate group is selected from 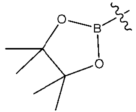 and -B(OH)₂. R₁, R₂, R₄₂, R₄₃, R₁₂, R₁₃, R₁₄, L₁, Y₃, Y₄, Y₅, and ring A are each as defined above.

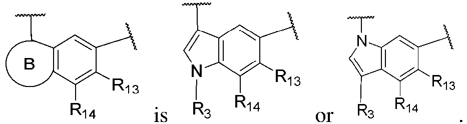

Another aspect of the present disclosure provides the following intermediate compounds and preparation methods therefor, and the intermediate compounds include:
a compound of formula (ii), which may be synthesized using the following method, wherein the solvent, temperature, and other reaction conditions in each step are adapted to or similar to those described in Preparation Example 3, or using reaction conditions known in the art; 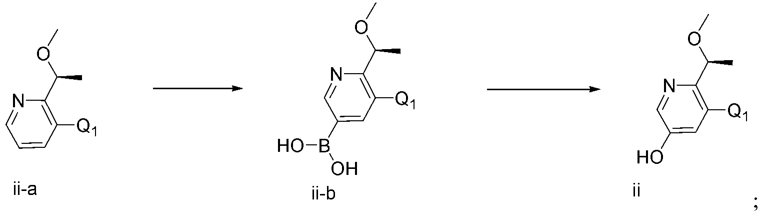
a compound of formula (iii), which may be synthesized using the following method, wherein the solvent, temperature, and other reaction conditions in each step are adapted to or similar to those described in Preparation Example 4, or using reaction conditions known in the art; 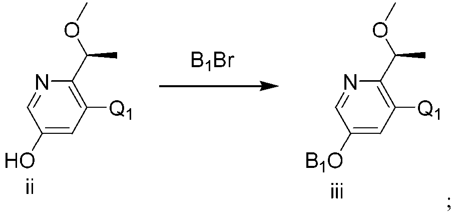
a compound of formula (iv), which may be synthesized using the following method, wherein the solvent, temperature, and other reaction conditions in each step are adapted to or similar to those described in Preparation Example 5, or using reaction conditions known in the art; 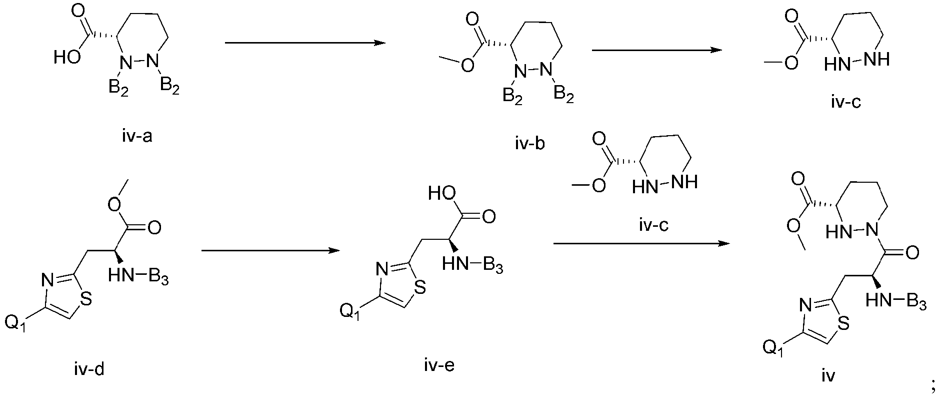
a compound of formula (v) or formula (v-A), which may be synthesized using the following method, wherein the solvent, temperature, and other reaction conditions in each step are adapted to or similar to those described in Preparation Example 6 or Preparation Example 7, or using reaction conditions known in the art;
synthetic route 1: 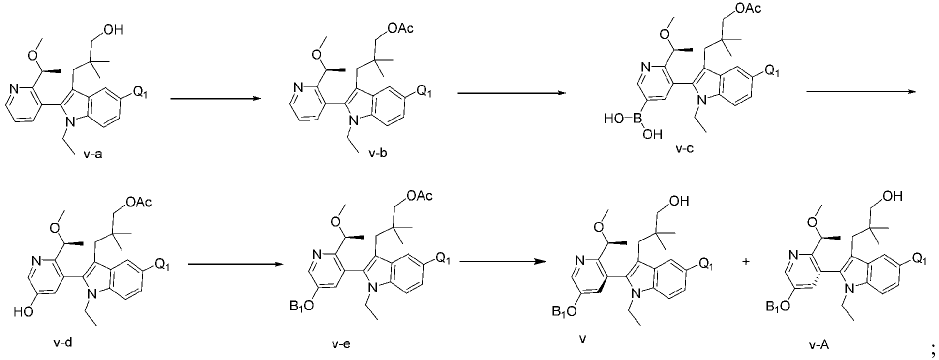
synthetic route 2: 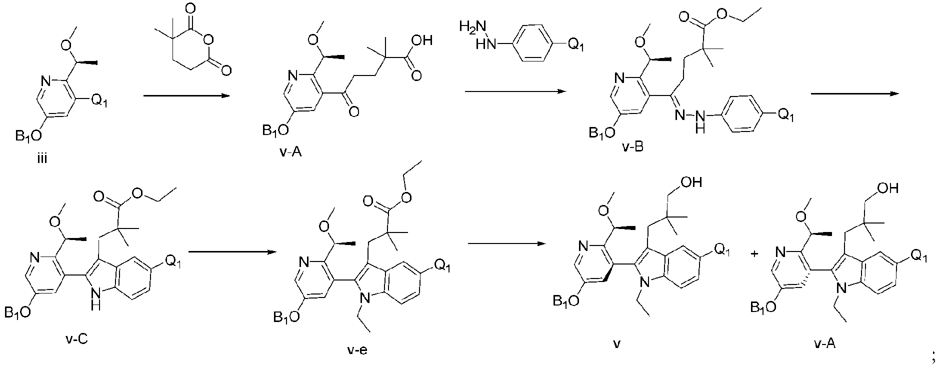
a compound of formula (vi), which may be synthesized using the following method, wherein the solvent, temperature, and other reaction conditions in each step are adapted to or similar to those described in Preparation Example 8 or Preparation Example 9, or using reaction conditions known in the art;
synthetic route 3: 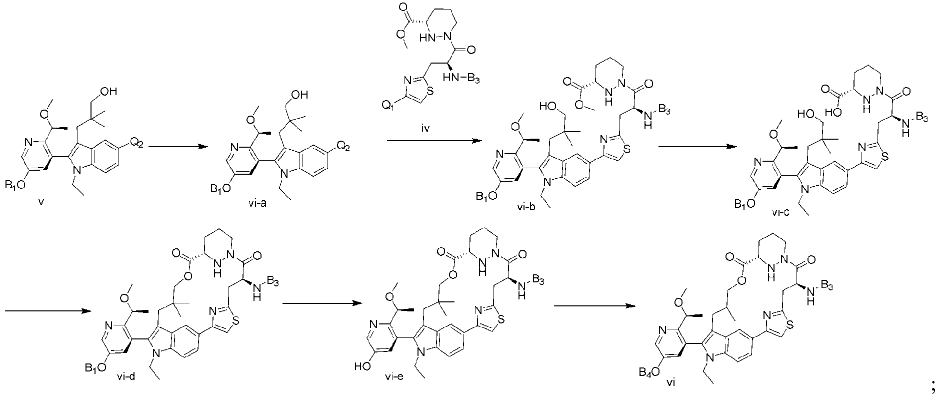
synthetic route 4: 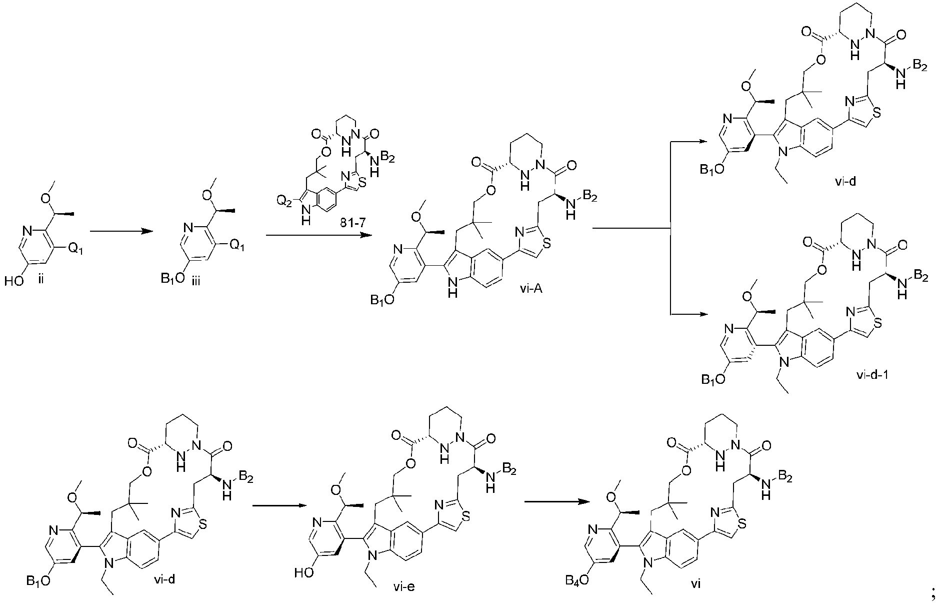
a compound of formula (vii), which may be synthesized using the following method, wherein the solvent, temperature, and other reaction conditions in each step are adapted to or similar to those described in Preparation Example 10, or using reaction conditions known in the art; 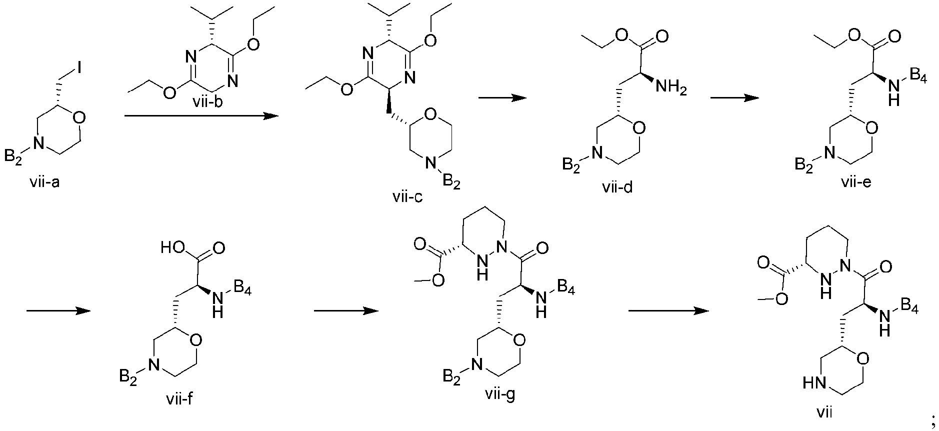 wherein in the above formulas, Q₁ and Q₂ are each independently selected from the aforementioned halogen atoms, boric acid groups, or borate groups; B₂ and B₃ are selected from the aforementioned amino protecting groups; B₁ and B₄ are selected from the aforementioned hydroxy protecting groups.

As used herein, the term "subject" refers to an animal, particularly a mammal. A human is preferred.

As used herein, the term "effective amount" or "therapeutically effective amount" refers to an amount of a medicament or agent that is sufficient to provide the desired effect and is non-toxic. In the embodiments of the present disclosure, when treating a patient according to the present disclosure, the amount of a given medicament depends on a number of factors, such as the specific dosing regimen, the type of disease or disorder and its severity, and the uniqueness of the subject or host in need of treatment (e.g., body weight). However, the dose administered can be routinely determined by methods known in the art according to the specific circumstances, including, for example, the specific medicament that has been used, the route of administration, the disorder being treated, and the subject or host being treated. In general, for dosages used for the treatment of adults, the dosage will typically be in the range of 0.02-5000 mg/day, for example, about 1-1500 mg/day. The required dose may conveniently be represented as a single dose, or as divided doses administered simultaneously (or within a short period of time) or at appropriate intervals, for example, two, three, four, or more doses per day. It will be appreciated by those skilled in the art that, although the dosage ranges described above are given, the specific effective amount may be adjusted as appropriate to the patient's condition in combination with the physician's diagnosis.

As used herein, the term "pharmaceutically acceptable salt" refers to a salt of the compound of the present disclosure that is pharmaceutically acceptable and has the pharmacological activity of the parent compound. Such salts include acid addition salts formed with inorganic acids (such as nitric acid, phosphoric acid, and carbonic acid) or with organic acids (such as propionic acid, hexanoic acid, cyclopentanepropionic acid, glycolic acid, pyruvic acid, gluconic acid, stearic acid, and muconic acid); or salts formed when an acidic proton present on the parent compound is substituted with a metal ion (e.g., an alkali metal ion or an alkaline earth metal ion); or coordination compounds formed with organic bases (such as ethanolamine). The pharmaceutically acceptable salt of the present disclosure can be synthesized from the parent compound containing an acid radical or a basic group by conventional chemical methods. In general, such salts are prepared by subjecting the compounds in a free acid or base form to a reaction with a stoichiometric amount of appropriate base or acid in water or an organic solvent or a mixture thereof. Generally, nonaqueous media such as ethers, ethyl acetate, ethanol, isopropanol, or acetonitrile, etc., are preferred. In addition to the salt forms, the compounds provided by the present disclosure also have prodrug forms. Prodrugs of the compounds described herein may readily undergo chemical changes under physiological conditions and thus be converted to the compounds of the present disclosure. In addition, prodrugs can be converted to the compounds of the present disclosure *in vivo* by chemical or biochemical methods.

As used herein, the term "solvate" refers to a substance formed by combining the compound of the present disclosure with a pharmaceutically acceptable solvent. The pharmaceutically acceptable solvent includes acetic acid and the like. The solvate includes both stoichiometric and non-stoichiometric amounts of solvates. Certain compounds of the present disclosure may exist in non-solvated forms or solvated forms. In general, the solvated forms are comparable to the non-solvated forms, and both are encompassed within the scope of the present disclosure.

As used herein, the term "stereoisomer" includes conformational isomers and configurational isomers, wherein the configurational isomers mainly include cis-trans isomers and optical isomers. The compounds described in the present disclosure may exist in the form of stereoisomers and thus encompass all possible stereoisomeric forms, including but not limited to cis-trans isomers, tautomers, enantiomers, diastereomers, atropisomers, and the like. The compounds described in the present disclosure may also exist in the form of any combination or any mixture of the aforementioned stereoisomers, such as equimolar mixtures of mesomers, racemates, and atropisomers. For example, a single enantiomer, a single diastereomer, or a mixture thereof, or a single atropisomer or a mixture thereof. When the compounds described in the present disclosure contain an olefinic double bond, they include cis isomers and trans isomers, as well as any combination thereof, unless otherwise specified. The atropisomers of the present disclosure are stereoisomers with axial or planar chirality that are produced on the basis of restricted rotation within the molecule. As medicaments, stereoisomers with excellent activity are preferred. The compounds of the present disclosure have optical isomers derived from asymmetric carbons and the like. If necessary, single isomers can be obtained by resolution through methods known in the art, such as crystallization or chiral chromatography.

As used herein, the term "alkyl" refers to a linear or branched saturated aliphatic hydrocarbyl group. The term "C₁₋₂₀ alkyl" refers to a linear or branched alkyl group having 1 to 20 carbon atoms. C₁₋₁₀ alkyl is preferred. C₁₋₆ alkyl (i.e., a linear or branched alkyl group having 1, 2, 3, 4, 5, or 6 carbon atoms) is more preferred. C₁₋₄ alkyl is more preferred. C₁₋₃ alkyl is more preferred. Specific examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. In the present disclosure, the alkyl may be optionally substituted, and when it is substituted, the substituent is preferably one or more substituent groups described in the present application.

As used herein, the term "heteroalkyl" refers to an alkyl group having at least one carbon atom substituted with a heteroatom (e.g., an O, N, or S atom), wherein the alkyl is as defined above. Heteroatoms may be present in the middle or at the end of a group. In the present disclosure, the heteroalkyl may be optionally substituted, and when it is substituted, the substituent is preferably one or more substituent groups described in the present application.

As used herein, the term "alkoxy" refers to a group having a structure of -O-alkyl, wherein the alkyl is as defined above. The term "C₁₋₁₀ alkoxy" refers to an alkoxy group having 1 to 10 carbon atoms. C₁₋₆ alkoxy is preferred. C₁₋₄ alkoxy is more preferred. C₁₋₃ alkoxy is more preferred. Specific examples include, but are not limited to, methoxy, ethoxy, n-propoxy, isopropoxy, n-butoxy, tert-butoxy, isobutoxy, n-pentoxy, and the like. In the present disclosure, the alkoxy may be optionally substituted, and when it is substituted, the substituent is preferably one or more substituent groups described in the present application.

As used herein, the term "alkenyl" refers to an alkyl group as defined above having one or more carbon-carbon double bonds at any site on the chain, and the term "C₂₋₈ alkenyl" refers to an alkenyl group having 2 to 8 carbon atoms and at least one (e.g., 1 to 2) carbon-carbon double bond. C₂₋₆ alkenyl (i.e., an alkenyl group having 2 to 6 carbon atoms and 1 to 2 carbon-carbon double bonds) is preferred. C₂₋₄ alkenyl (i.e., an alkenyl group having 2 to 4 carbon atoms and 1 to 2 carbon-carbon double bonds) is more preferred. Specific examples include, but are not limited to, ethenyl, 1-propenyl, 2-propenyl, 1-, 2-, or 3-butenyl, pentenyl, hexenyl, butadienyl, and the like. In the present disclosure, the alkenyl may be optionally substituted, and when it is substituted, the substituent is preferably one or more substituent groups described in the present application.

As used herein, the term "alkynyl" refers to an alkyl group as defined above having one or more carbon-carbon triple bonds at any site on the chain, and the term "C₂₋₈ alkynyl" refers to an alkynyl group having 2 to 8 carbon atoms and at least one (e.g., 1 to 2) carbon-carbon triple bond. C₂₋₆ alkynyl (i.e., an alkynyl group having 2 to 6 carbon atoms and 1 to 2 carbon-carbon triple bonds) is preferred. C₂₋₄ alkynyl (i.e., an alkynyl group having 2 to 4 carbon atoms and 1 to 2 carbon-carbon triple bonds) is more preferred. Specific examples include, but are not limited to, ethynyl, 1-propynyl, 2-propynyl, 1-, 2-, or 3-butynyl, and the like. In the present disclosure, the alkynyl may be optionally substituted, and when it is substituted, the substituent is preferably one or more substituent groups described in the present application.

As used herein, the term "halogen" refers to fluorine, chlorine, bromine, or iodine.

As used herein, the term "halo" refers to fluoro, chloro, bromo, or iodo.

As used herein, the term "haloalkyl" refers to an alkyl group, in which one or more (e.g., 1, 2, 3, 4, or 5) hydrogen atoms are substituted with halogen, wherein the alkyl is as defined above. The term "C₁₋₁₀ haloalkyl" refers to a haloalkyl group having 1 to 10 carbon atoms. C₁₋₆ haloalkyl is preferred. C₁₋₄ haloalkyl is more preferred. C₁₋₃ haloalkyl is more preferred. Specific examples include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, and the like.

As used herein, the term "haloalkoxy" refers to an alkoxy group, in which one or more (e.g., 1, 2, 3, 4, or 5) hydrogen atoms are substituted with halogen, wherein the alkoxy is as defined above. The term "C₁₋₁₀ haloalkoxy" refers to a haloalkoxy group having 1 to 10 carbon atoms. "C₁₋₆ haloalkoxy" is preferred. C₁₋₄ haloalkoxy is more preferred. C₁₋₃ haloalkoxy is more preferred. Specific examples include, but are not limited to, trifluoromethoxy, trifluoroethoxy, monofluoromethoxy, monofluoroethoxy, difluoromethoxy, difluoroethoxy, and the like.

As used herein, the term "deuterated" refers to a substitution of one or more hydrogen atoms in a group with deuterium atoms. As used herein, the term "deuterated alkyl" refers to an alkyl group, in which one or more (e.g., 1, 2, 3, 4, or 5) hydrogen atoms are substituted with deuterium atoms, wherein the alkyl is as defined above. The term "deuterated C₁₋₁₀ alkyl" refers to a deuterated alkyl group having 1 to 10 carbon atoms. Deuterated C₁₋₆ alkyl is preferred. Deuterated C₁₋₄ alkyl is more preferred. Deuterated C₁₋₃ alkyl is more preferred. Specific examples include, but are not limited to, monodeuteriomethyl, dideuteriomethyl, trideuteriomethyl, monodeuterioethyl, 1,2-dideuterioethyl, trideuterioethyl, and the like.

As used herein, the term "deuterated alkoxy" refers to an alkoxy group, in which one or more (e.g., 1, 2, 3, 4, or 5) hydrogen atoms are substituted with deuterium atoms, wherein the alkoxy is as defined above. The term "deuterated C₁₋₁₀ alkoxy" refers to a deuterated alkoxy group having 1 to 10 carbon atoms. Deuterated C₁₋₆ alkoxy is preferred. Deuterated C₁₋₄ alkoxy is more preferred. Deuterated C₁₋₃ alkoxy is more preferred. Specific examples include, but are not limited to, trideuteriomethoxy, trideuterioethoxy, monodeuteriomethoxy, monodeuterioethoxy, dideuteriomethoxy, dideuterioethoxy, and the like.

As used herein, the terms "cycloalkyl" and "cycloalkyl ring" are used interchangeably and refer to a saturated monocyclic or polycyclic cyclic hydrocarbyl group, for example, including monocyclic cycloalkyl, spirocycloalkyl, fused cycloalkyl, and bridged cycloalkyl. The ring carbon atoms of the cycloalkyl described in the present disclosure may be optionally substituted with 1, 2, or 3 oxo groups to form a cyclic ketone structure. The term "3- to 20-membered cycloalkyl" or "C₃₋₂₀ cycloalkyl" refers to a cycloalkyl group having 3 to 20 ring carbon atoms, including monocyclic cycloalkyl, spirocycloalkyl, fused cycloalkyl, and bridged cycloalkyl. C₃₋₁₂ cycloalkyl, C₅₋₂₀ spirocycloalkyl, C₅₋₂₀ fused cycloalkyl, or C₅₋₂₀ bridged cycloalkyl is preferred. C₃₋₈ monocyclic cycloalkyl is more preferred.

The terms "C₃₋₈ monocyclic cycloalkyl" and "3- to 8-membered monocyclic cycloalkyl" refer to a saturated monocyclic cyclic hydrocarbyl group having 3 to 8 ring carbon atoms. C₃₋₆ monocyclic cycloalkyl (i.e., 3- to 6-membered monocyclic cycloalkyl) or C₄₋₆ monocyclic cycloalkyl (i.e., 4- to 6-membered monocyclic cycloalkyl) is preferred. C₃, C₄, C₅, or C₆ monocyclic cycloalkyl is more preferred. Specific examples of monocyclic cycloalkyl include, but are not limited to, cyclopropyl, cyclobutyl, cyclopentyl, cyclohexyl, cycloheptyl, cyclooctyl, and the like.

As used herein, the terms "spirocycloalkyl" and "spirocycloalkyl ring" refer to a polycyclic cyclic hydrocarbyl group formed by sharing one carbon atom (referred to as a spiro atom) between two or more monocyclic rings. According to the number of spiro atoms shared between rings, the spirocycloalkyl may be classified as monospirocycloalkyl, bispirocycloalkyl, and polyspirocycloalkyl. The term "5- to 20-membered spirocycloalkyl" or "C₅₋₂₀ spirocycloalkyl" refers to a polycyclic cyclic hydrocarbyl group having 5 to 20 ring carbon atoms, wherein the monocyclic ring sharing a spiro atom is a 3- to 8-membered monocyclic cycloalkyl ring. 6- to 14-membered (i.e., C₆₋₁₄) spirocycloalkyl is preferred. 6- to 14-membered monospirocycloalkyl is more preferred. 7- to 11-membered (i.e., C₇₋₁₁) spirocycloalkyl is more preferred. 7- to 11-membered monospirocycloalkyl is more preferred. 7-membered (4-membered monocyclic cycloalkyl ring/4-membered monocyclic cycloalkyl ring), 8-membered (4-membered monocyclic cycloalkyl ring/5-membered monocyclic cycloalkyl ring), 9-membered (4-membered monocyclic cycloalkyl ring/6-membered monocyclic cycloalkyl ring, 5-membered monocyclic cycloalkyl ring/5-membered monocyclic cycloalkyl ring), 10-membered (5-membered monocyclic cycloalkyl ring/6-membered monocyclic cycloalkyl ring), or 11-membered (6-membered monocyclic cycloalkyl ring/6-membered monocyclic cycloalkyl ring) monospirocycloalkyl is the most preferred. Specific examples of spirocycloalkyl include, but are not limited to:

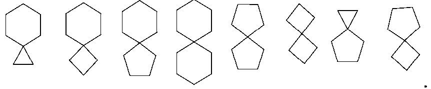

These spirocycloalkyl groups can be attached to the rest of the molecule through any one of the ring atoms.

As used herein, the terms "fused cycloalkyl" and "fused cycloalkyl ring" refer to a polycyclic cyclic hydrocarbyl group formed by sharing a pair of adjacent carbon atoms between two or more monocyclic rings. According to the number of the formed rings, the fused cycloalkyl may be classified as bicyclic, tricyclic, tetracyclic, or polycyclic fused cycloalkyl. The term "5- to 20-membered fused cycloalkyl" or "C₅₋₂₀ fused cycloalkyl" refers to a polycyclic cyclic hydrocarbyl group having 5 to 20 ring carbon atoms, wherein the monocyclic rings sharing a pair of adjacent carbon atoms are 3- to 8-membered monocyclic cycloalkyl rings. 6- to 14-membered (i.e., C₆₋₁₄) fused cycloalkyl is preferred. 6- to 14-membered bicyclic fused cycloalkyl is more preferred. 7- to 10-membered (i.e., C₇₋₁₀) fused cycloalkyl is more preferred. 7- to 10-membered bicyclic fused cycloalkyl is more preferred. 8-membered (5-membered monocyclic cycloalkyl ring fused to 5-membered monocyclic cycloalkyl ring), 9-membered (5-membered monocyclic cycloalkyl ring fused to 6-membered monocyclic cycloalkyl ring), or 10-membered (6-membered monocyclic cycloalkyl ring fused to 6-membered monocyclic cycloalkyl ring) bicyclic fused cycloalkyl is the most preferred. Specific examples of fused cycloalkyl include, but are not limited to:

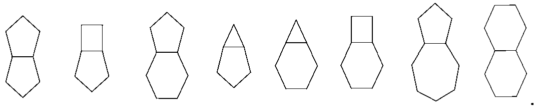

These fused cycloalkyl groups can be attached to the rest of the molecule through any one of the ring atoms.

As used herein, the terms "bridged cycloalkyl" and "bridged cycloalkyl ring" refer to a polycyclic cyclic hydrocarbyl group formed by sharing two carbon atoms that are not directly connected between two or more monocyclic rings. According to the number of the formed rings, the bridged cycloalkyl may be classified as bicyclic, tricyclic, tetracyclic, or polycyclic bridged cycloalkyl. The terms "5- to 20-membered bridged cycloalkyl" and "C₅₋₂₀ bridged cycloalkyl" refer to a polycyclic cyclic hydrocarbyl group having 5 to 20 ring carbon atoms, wherein any two rings share two carbon atoms that are not directly connected. 6- to 14-membered (i.e., C₆₋₁₄) bridged cycloalkyl is preferred. 7- to 10-membered (i.e., C₇₋₁₀) bridged cycloalkyl is more preferred. Specific examples of bridged cycloalkyl include, but are not limited to:

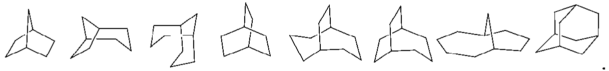

These bridged cycloalkyl groups can be attached to the rest of the molecule through any one of the ring atoms.

In the present disclosure, the cycloalkyl may be optionally substituted, and when it is substituted, the substituent is preferably one or more substituent groups described in the present application.

As used herein, the term "halocycloalkyl" refers to a cycloalkyl group, in which one or more (e.g., 1, 2, 3, 4, or 5) hydrogen atoms are substituted with halogen, wherein the cycloalkyl is as defined above.

As used herein, the term "C₃₋₈ monocyclic halocycloalkyl" refers to a monocyclic halocycloalkyl having 3 to 8 ring carbon atoms. C₃₋₆ monocyclic halocycloalkyl is preferred. C₃, C₄, C₅, or C₆ monocyclic halocycloalkyl is more preferred. Specific examples include, but are not limited to, trifluorocyclopropyl, monofluorocyclopropyl, monofluorocyclohexyl, difluorocyclopropyl, difluorocyclohexyl, and the like.

As used herein, the terms "heterocyclyl" and "heterocyclyl ring" are used interchangeably and refer to a saturated or partially unsaturated monocyclic or polycyclic cyclic hydrocarbyl group, for example, including monocyclic heterocyclyl, spiroheterocyclyl, fused heterocyclyl, and bridged heterocyclyl. The ring carbon atoms of the heterocyclyl described in the present disclosure may be optionally substituted with 1, 2, or 3 oxo groups to form a cyclic ketone, cyclic lactone, or cyclic lactam structure. The term "3- to 20-membered heterocyclyl" refers to a saturated or partially unsaturated monocyclic or polycyclic cyclic hydrocarbyl group having 3 to 20 ring atoms, of which one or more (preferably 1, 2, 3, or 4) ring atoms are heteroatoms selected from nitrogen, oxygen, and S(=O)ₘ' (where m' is an integer of 0 to 2), excluding a cyclic moiety of -O-O-, -O-S-, or -S-S-, and the other ring atoms are carbon. When the ring atom is a nitrogen atom, the nitrogen atom may be substituted or unsubstituted (i.e., N or NR, R being hydrogen or other substituents already defined herein). The 3- to 20-membered heterocyclyl described in the present disclosure includes monocyclic heterocyclyl (e.g., 3- to 8-membered monocyclic heterocyclyl), 5- to 20-membered spiroheterocyclyl, 5- to 20-membered fused heterocyclyl, and 5- to 20-membered bridged heterocyclyl.

As used herein, the terms "3- to 8-membered monocyclic heterocyclyl" and "3- to 8-membered monocyclic heterocyclyl ring" refer to a saturated or partially unsaturated monocyclic cyclic hydrocarbyl group having 3 to 8 ring atoms, of which 1, 2, or 3 ring atoms are heteroatoms selected from nitrogen, oxygen, and S(=O)ₘ' (where m' is an integer of 0 to 2). 3- to 6-membered monocyclic heterocyclyl having 3 to 6 ring atoms, of which 1 or 2 ring atoms are heteroatoms, is preferred. 4- to 6-membered monocyclic heterocyclyl having 4 to 6 ring atoms, of which 1 or 2 ring atoms are heteroatoms, is more preferred. 5- or 6-membered monocyclic heterocyclyl having 5 or 6 ring atoms, of which 1 or 2 ring atoms are heteroatoms, is more preferred. When the heteroatom is a nitrogen atom, the nitrogen atom may be substituted or unsubstituted (i.e., N or NR, R being hydrogen or other substituents already defined herein). When the heteroatom is a sulfur atom, the sulfur atom may be optionally oxidized (i.e., S(=O)ₘ', where m' is an integer of 0 to 2). The ring carbon atoms of the monocyclic heterocyclyl may be optionally substituted with 1, 2, or 3 oxo groups to form a cyclic ketone, cyclic lactone, or cyclic lactam structure. Specific examples of monocyclic heterocyclyl include, but are not limited to, aziridine, oxirane, azetidine, azetidin-2-one, oxetane, oxetan-2-one, oxazolidine, pyrrolidin-2-one, pyrrolidine-2,5-dione, 1,3-dioxolane, dihydrofuran-2(3H)-one, dihydrofuran-2,5-dione, piperidin-2-one, piperidine-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholine-3-one, morpholine-2-one, thiomorpholin-3-one 1,1-dioxide, thiomorpholine, thiomorpholin-1,1-dioxide, tetrahydropyran, 1,2-dihydroazete, 1,2-dihydrooxete, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridine-2,6(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine, and the like.

As used herein, the term "3- to 6-membered nitrogen-containing heterocyclyl" refers to a saturated or partially unsaturated monocyclic cyclic hydrocarbyl group having 3 to 6 ring atoms, of which 1 ring atom is a nitrogen atom and the other 1 or 2 ring atoms are heteroatoms selected from nitrogen, oxygen, and S(=O)ₘ' (where m' is an integer of 0 to 2). Specific examples include, but are not limited to, aziridinyl, azetidinyl, azacyclopentyl (i.e., tetrahydropyrrole), azacyclohexyl (i.e., piperidine), morpholinyl, piperazinyl, and oxazolidine.

As used herein, the term "3- to 8-membered monocyclic heterocycloalkyl" refers to a saturated monocyclic cyclic hydrocarbyl group having 3 to 8 ring atoms, of which 1 or 2 ring atoms are heteroatoms. 3- to 6-membered monocyclic heterocycloalkyl, i.e., a saturated monocyclic cyclic hydrocarbyl group having 3 to 6 ring atoms, of which 1 or 2 ring atoms are heteroatoms, is preferred. Specific examples of heterocycloalkyl include, but are not limited to, aziridinyl, oxiranyl, azetidinyl, oxetanyl, oxazolidinyl, 1,3-dioxolanyl, dioxanyl, imidazolidinyl, tetrahydrofuranyl, tetrahydrothienyl, tetrahydropyrrolyl, piperidinyl, piperazinyl, morpholinyl, thiomorpholinyl, thiomorpholine-1,1-dioxide group, tetrahydropyranyl, 1,4-oxazepanyl, 1,3-oxazepanyl, 1,3-oxazinanyl, hexahydropyrimidinyl, and 1,4-dioxanyl.

The 2 connected ring atoms on the monocyclic heterocyclyl ring described above, including C-C and N-C, may both optionally be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heterocyclyl that form a fused ring with another ring are preferably C-C.

As used herein, the terms "spiroheterocyclyl" and "spiroheterocyclyl ring" refer to a polycyclic heterocyclyl group formed by sharing one carbon atom (referred to as a spiro atom) between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the other ring atoms are carbon. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. According to the number of spiro atoms shared among the rings, the spiroheterocyclyl may be monospiroheterocyclyl, dispiroheterocyclyl, or polyspiroheterocyclyl. The term "5- to 20-membered spiroheterocyclyl" refers to a spiroheterocyclyl group having 5 to 20 ring atoms, wherein one of the monocyclic rings that share a spiro atom is a 3- to 8-membered monocyclic heterocyclyl ring, and the other monocyclic ring is a 3- to 8-membered monocyclic heterocyclyl ring or a 3- to 8-membered monocyclic cycloalkyl ring. 6- to 14-membered spiroheterocyclyl having 6 to 14 ring atoms, of which 1 or 2 ring atoms are heteroatoms, is preferred. 7- to 11-membered spiroheterocyclyl having 7 to 11 ring atoms, of which 1 or 2 ring atoms are heteroatoms, is more preferred. 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring), or 11-membered (6-membered monocyclic heterocyclyl ring/6-membered monocyclic heterocyclyl ring) monospiroheterocyclyl is the most preferred. Specific examples of spiroheterocyclyl include, but are not limited to:

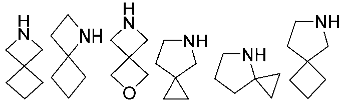

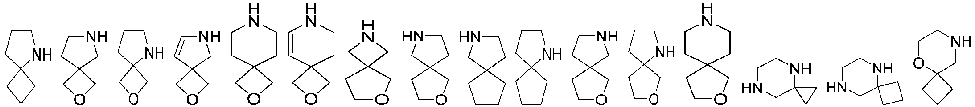

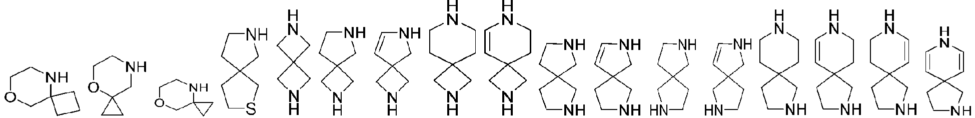

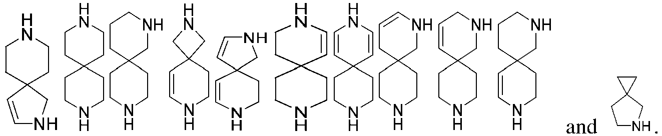

These spiroheterocyclyl groups can be attached to the rest of the molecule through any one of the suitable ring atoms.

As used herein, the terms "fused heterocyclyl" and "fused heterocyclyl ring" refer to a polycyclic heterocyclyl group formed by sharing a pair of adjacent ring atoms between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the other ring atoms are carbon. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. The shared pair of adjacent ring atoms may be C-C or N-C. According to the number of constituent rings, the fused heterocyclyl may be classified as bicyclic, tricyclic, tetracyclic, or polycyclic fused heterocyclyl. The term "5- to 20-membered fused heterocyclyl" refers to a fused heterocyclyl group having 5 to 20 ring atoms, wherein the monocyclic ring sharing a pair of adjacent ring atoms is a 3-to 8-membered monocyclic heterocyclyl ring. 6- to 14-membered fused heterocyclyl having 6 to 14 ring atoms, of which 1 or 2 ring atoms are heteroatoms, is preferred. 6- to 10-membered fused heterocyclyl having 6 to 10 ring atoms, of which 1 or 2 ring atoms are heteroatoms, is more preferred. 8- to 10-membered fused heterocyclyl having 8 to 10 ring atoms, of which 1 or 2 ring atoms are heteroatoms, is more preferred. 8-membered (5-membered monocyclic heterocyclyl ring fused to 5-membered monocyclic heterocyclyl ring), 9-membered (5-membered monocyclic heterocyclyl ring fused to 6-membered monocyclic heterocyclyl ring), or 10-membered (6-membered monocyclic heterocyclyl ring fused to 6-membered monocyclic heterocyclyl ring) bicyclic fused heterocyclyl is the most preferred. Specific examples of fused heterocyclyl include, but are not limited to:

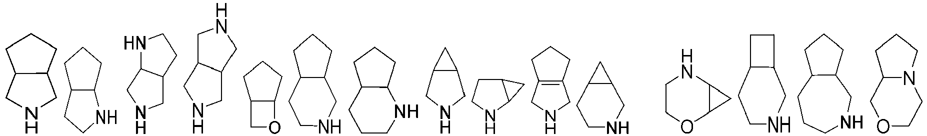

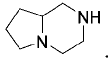

These fused heterocyclyl groups can be attached to the rest of the molecule through any one of the suitable ring atoms.

As used herein, the terms "bridged heterocyclyl" and "bridged heterocyclyl ring" refer to a polycyclic heterocyclyl group formed by sharing two ring atoms that are not directly connected between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the other ring atoms are carbon. According to the number of the formed rings, the bridged cycloalkyl may be classified as bicyclic, tricyclic, tetracyclic, or polycyclic bridged cycloalkyl. The term "5- to 20-membered bridged heterocyclyl" refers to a saturated or partially unsaturated polycyclic heterocyclyl group having 5 to 20 ring atoms, wherein any two rings share two ring atoms that are not directly connected, and each monocyclic ring may contain one or more double bonds, but none of the rings has a fully conjugated π-electron system. 6- to 14-membered bridged heterocyclyl is preferred. 7- to 10-membered bridged heterocyclyl is more preferred. Specific examples of bridged heterocyclyl include, but are not limited to:

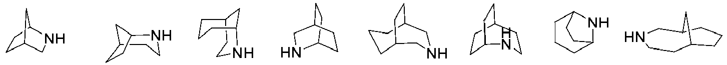

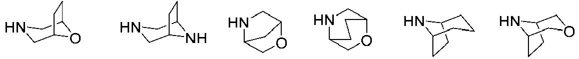

These bridged heterocyclyl groups can be attached to the rest of the molecule through any one of the suitable ring atoms.

In the present disclosure, the various heterocyclyl groups described above may be optionally substituted, and when they are substituted, the substituent is preferably one or more substituent groups described in the present application.

The term "5- to 15-membered tricyclic heterocyclyl" refers to a tricyclic heterocyclyl group having 5 to 15 ring atoms, wherein three rings may be connected in one or more ways selected from spiro connection, fused connection, and bridged connection. Specific examples may include, but are not limited to, a first monocyclic ring being a 6-membered monocyclic heterocyclic ring, a second monocyclic ring being a 6-membered monocyclic heterocyclic ring, and a third monocyclic ring being a 3- to 6-membered monocyclic heterocyclic ring; and the first monocyclic ring is fused to the second monocyclic ring, and the third monocyclic ring is spiro-connected to the second monocyclic ring.

The term "7- to 12-membered bicyclic heterocyclyl" refers to a bicyclic heterocyclyl group having 7 to 12 ring atoms, wherein two rings may be connected in one way selected from spiro connection, fused connection, and bridged connection. Specific examples may be selected from the specific examples of the spiroheterocyclyl, the specific examples of the fused heterocyclyl, and the specific examples of the bridged heterocyclyl described above.

The term "9- to 11-membered bicyclic heterocyclyl" refers to a bicyclic heterocyclyl group having 9 to 11 ring atoms, wherein two rings may be connected in one way selected from spiro connection, fused connection, and bridged connection. Specific examples may be selected from the specific examples of the spiroheterocyclyl, the specific examples of the fused heterocyclyl, and the specific examples of the bridged heterocyclyl described above.

As used herein, the terms "aryl", "aryl ring", and "aromatic ring" are used interchangeably and refer to an all-carbon monocyclic, an all-carbon non-fused polycyclic (rings are connected by covalent bonds and not fused), or an all-carbon fused polycyclic (i.e., rings that share a pair of adjacent carbon atoms) group. At least one ring in the group is aromatic, i.e., has a conjugated π-electron system. The term "C₆₋₁₄ aryl" refers to an aryl group having 6 to 14 ring atoms. C₆₋₁₀ aryl is preferred. In the present disclosure, C₆₋₁₄ aryl includes monocyclic aryl, non-fused polycyclic aryl, and aromatic fused polycyclic ring, wherein examples of monocyclic aryl include phenyl, and examples of non-fused polycyclic aryl include biphenyl and the like.

In the present disclosure, when C₆₋₁₄ aryl is an aromatic fused polycyclic ring, the aromatic fused polycyclic ring may be a polycyclic group formed by fusing a monoaryl ring to one or more monoaryl rings; non-limiting examples include naphthyl, anthryl, and the like.

In some embodiments of the present disclosure, when C₆₋₁₄ aryl is an aromatic fused polycyclic ring, the aromatic fused polycyclic ring may also be a polycyclic group formed by fusing a monoaryl ring (e.g., phenyl) to one or more non-aromatic rings, wherein the ring connected to the parent structure is an aromatic ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure) and a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure). The polycyclic group formed by fusing the monoaryl ring to one or more non-aromatic rings described above may be connected to another group or the parent structure through a nitrogen atom or carbon atom, and the ring connected to the parent structure is a monoaryl ring or non-aromatic ring.

In the present disclosure, the various aryl groups described above may be substituted or unsubstituted, and when they are substituted, the substituent is preferably one or more substituent groups described in the present application.

As used herein, the terms "heteroaryl", "heteroaryl ring", and "heteroaromatic ring" are used interchangeably and refer to a monocyclic or fused polycyclic (i.e., sharing a pair of adjacent ring atoms, which may be C-C or N-C) group, in which the ring atoms are substituted with at least one heteroatom independently selected from nitrogen, oxygen, and sulfur, wherein the nitrogen and sulfur atoms may optionally be oxidized and the nitrogen atom may optionally be quaternized. The heteroaryl has 6, 10, or 14 π electrons shared, and at least one ring in the group is aromatic. The term "5- to 14-membered heteroaryl" refers to a heteroaryl group having 5 to 14 ring atoms, of which 1, 2, 3, 4, or 5 ring atoms are heteroatoms selected from nitrogen, oxygen, and S(=O)ₘ' (where m' is an integer of 0 to 2). 5- to 10-membered heteroaryl having 5 to 10 ring atoms, of which 1, 2, 3, or 4 ring atoms are heteroatoms, is preferred. In the present disclosure, the 5- to 14-membered heteroaryl may be monocyclic heteroaryl, fused bicyclic heteroaryl, or fused tricyclic heteroaryl.

As used herein, the term "5- or 6-membered monocyclic heteroaryl" refers to a monocyclic heteroaryl group having 5 or 6 ring atoms, of which 1, 2, or 3 ring atoms are heteroatoms selected from nitrogen, oxygen, and S(=O)ₘ' (where m' is an integer of 0 to 2). Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

As used herein, the term "8- to 10-membered bicyclic heteroaryl" refers to a fused bicyclic heteroaryl group having 8 to 10 ring atoms, of which 1, 2, 3, 4, or 5 ring atoms are heteroatoms selected from nitrogen, oxygen, and S(=O)ₘ' (where m' is an integer of 0 to 2). The fused bicyclic heteroaryl may be a bicyclic group (preferably a 9- or 10-membered bicyclic heteroaryl ring) formed by fusing a monoaryl ring (e.g., phenyl) to a monocyclic heteroaryl ring (preferably a 5- or 6-membered monocyclic heteroaryl ring), or a bicyclic group formed by fusing a monocyclic heteroaryl ring (preferably a 5- or 6-membered monocyclic heteroaryl ring) to a monocyclic heteroaryl ring (preferably a 5- or 6-membered monocyclic heteroaryl ring).

Any 2 connected ring atoms on the monocyclic heteroaryl ring described above, including C-C, N-C, and N-N, may all be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heteroaryl ring that form a fused ring with another ring are preferably C-C, including but not limited to the following forms: 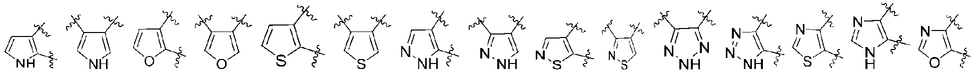 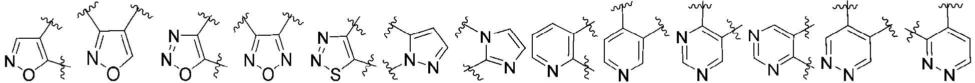 and

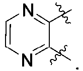

The ring atoms marked by " 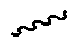 " in the groups described above are connected to other moieties of the molecule.

Non-limiting examples of 8- to 10-membered bicyclic heteroaryl include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, and the like.

Specific examples of bicyclic heteroaryl include, but are not limited to: 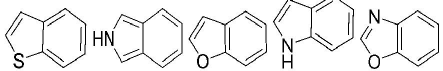 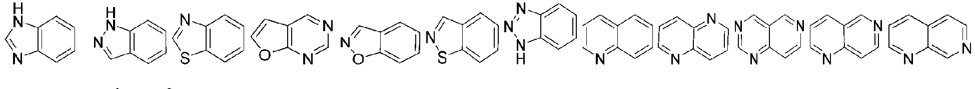 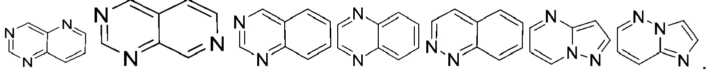 These groups can be attached to the rest of the molecule through any one of the suitable ring atoms. The ring connected to the parent structure may be a monocyclic heteroaryl ring or a benzene ring.

In some embodiments of the present disclosure, the fused bicyclic heteroaryl or fused tricyclic heteroaryl may be a polycyclic group formed by fusing a monocyclic heteroaryl ring (preferably a 5- or 6-membered monocyclic heteroaryl ring) to one or more non-aromatic rings, wherein the ring connected to the parent structure is a monocyclic heteroaryl ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure), a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure), and the like. The polycyclic group formed by fusing the monocyclic heteroaryl ring to one or more non-aromatic rings described above may be connected to another group or the parent structure through a nitrogen atom or carbon atom, and the ring connected to the parent structure is a monocyclic heteroaryl ring or non-aromatic ring.

The 6- to 12-membered heteroaryl-fused cycloalkyl is formed by fusing monocyclic heteroaryl to cycloalkyl, and the ring connected to the parent structure may be monocyclic heteroaryl or cycloalkyl. For example, 5-membered monocyclic heteroaryl is fused to 3-, 4-, 5-, 6-, or 7-membered cycloalkyl, or 6-membered monocyclic heteroaryl is fused to 3-, 4-, 5-, or 6-membered cycloalkyl.

The 6- to 12-membered heteroaryl-fused heterocyclyl is formed by fusing monocyclic heteroaryl to heterocyclyl, and the ring connected to the parent structure may be monocyclic heteroaryl or heterocyclyl. For example, 5-membered monocyclic heteroaryl is fused to 3-, 4-, 5-, 6-, or 7-membered heterocyclyl, or 6-membered monocyclic heteroaryl is fused to 3-, 4-, 5-, or 6-membered heterocyclyl.

In the present disclosure, the various heteroaryl groups described above may be substituted or unsubstituted, and when they are substituted, the substituent is preferably one or more substituent groups described in the present application.

As used herein, the term "hydroxy" refers to -OH.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "carboxyl" refers to -C(=O)OH.

As used herein, the term "acetyl" refers to -COCH₃.

As used herein, the term "substituted" means that any one or more hydrogen atoms on a specific atom are substituted with substituents that may include deuterium and hydrogen variants, as long as the valence of the specific atom is normal and the compound after substitution is stable. When the substituent is oxo (i.e., =O), it means that two hydrogen atoms are substituted. The term "optionally substituted" means that an atom may or may not be substituted with a substituent. Unless otherwise specified, the type and number of the substituent may be arbitrary as long as being chemically achievable.

When any variable (e.g., R) occurs once or more in the constitution or structure of a compound, the definition of the variable in each case is independent. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

In any embodiment, any or all hydrogens present in the compound, or hydrogens in a particular group or moiety within the compound, may be replaced by deuterium or tritium. One to the maximum number of hydrogens present in the compound may be replaced by deuterium. One to the maximum number of hydrogens present in any group in the general formula compound or specific compound may be deuterated. For example, when a certain group is described as ethyl, the ethyl may be C₂H₅ or C₂H₅ with x (1 to 5) hydrogens replaced by deuterium, e.g., C₂DₓH₅₋ₓ. When a certain group is described as deuterated ethyl, the deuterated ethyl may be C₂H₅ with x (1 to 5) hydrogens replaced by deuterium, e.g., C₂DₓH₅₋ₓ. The stable deuterated derivatives described in the present disclosure are preferably stable deuterated isotopic derivatives obtained by substituting any hydrogen atom that can be deuterated in each formula with 1 to the maximum number (e.g., 1 to 2, 1 to 3, 1 to 4, 1 to 5, 1 to 6, 1 to 7, etc.) of deuterium atoms.

### BRIEF DESCRIPTION OF THE DRAWINGS

FIG. 1 shows a diagram of a three-dimensional molecular structure of intermediate 6 determined by X-ray single-crystal diffraction.
FIG. 2 shows a diagram of a three-dimensional molecular structure of intermediate 605-1A determined by X-ray single-crystal diffraction.

### DETAILED DESCRIPTION

The compounds of the present disclosure may be prepared by a variety of synthetic methods well known to those skilled in the art, including the specific embodiments listed below, embodiments formed by combinations thereof with other chemical synthetic methods, and equivalents thereof well known to those skilled in the art. Preferred embodiments include, but are not limited to, the examples of the present disclosure.

The present disclosure is described in detail below by way of examples. However, this is by no means disadvantageously limiting the scope of the present disclosure. Although the present disclosure has been described in detail herein and specific examples have also been disclosed, it will be apparent to those skilled in the art that various changes and modifications can be made to the specific examples without departing from the spirit and scope of the present disclosure. Experimental procedures without specified conditions in the examples are conducted according to conventional conditions or conditions recommended by the manufacturers. Reagents or instruments without specified manufacturers used herein are commercially available conventional products.

The structures of the compounds were determined by methods such as nuclear magnetic resonance (NMR) and mass spectrometry (MS).

The NMR determination was performed on a Bruker Avance NEO 400 or Bruker Avance NEO 500 nuclear magnetic resonance instrument. The chemical shift (δ) was in parts per million (ppm). The solvents for determination were as shown in each of the examples. The internal standard was tetramethylsilane (TMS).

The MS determination was performed on an Agilent 1100 liquid chromatograph.

The high performance liquid chromatography (HPLC) analysis was performed on a Waters 2695 high performance liquid chromatograph.

The preparative high performance liquid chromatography was performed on a Waters 2767 high performance liquid chromatograph.

The chiral HPLC analysis was performed on a Waters 2695 high performance liquid chromatograph or a Waters Investigator SFC system.

The preparative chiral chromatography was performed on a gilson gx-281 chromatograph or a waters SFC-80 chromatograph. Qingdao GF254 or Yantai Huanghai HSGF254 silica gel plates were used as thin-layer chromatography silica gel plates. 0.15 mm-0.2 mm silica gel plates were used for thin-layer chromatography (TLC). 0.4 mm-0.5 mm silica gel plates were used for thin-layer chromatography product separation and purification.

The ISCO CombiFlash NextGen 300 column chromatography system was generally used for column chromatography, and the Agela Flash Column Silica-Cs series silica gel column was used.

The prep-HPLC used in the following examples, unless otherwise specified, may be performed under the following conditions:
Prep-HPLC (ammonium bicarbonate method): chromatographic column: Welch Xtimate C18, 21.2 × 150 mm, 5 µm; mobile phase A: 5 mmol/L aqueous ammonium bicarbonate solution, mobile phase B: acetonitrile; flow rate: 15 mL/min; column temperature: room temperature.
Prep-HPLC (formic acid method 1): chromatographic column: Welch Xtimate C18, 21.2 × 150 mm, 5 µm; mobile phase A: 0.1% aqueous formic acid solution, mobile phase B: acetonitrile; flow rate: 15 mL/min; column temperature: room temperature.
Prep-HPLC (formic acid method 2): chromatographic column: Waters XBridge C18, 19 × 250 mm, 5 µm; mobile phase system: A: 0.1% aqueous formic acid solution; B: preparative grade acetonitrile; flow rate: 15 mL/min; B% = 20%-100%; column temperature: room temperature.
Prep-HPLC (trifluoroacetic acid method): chromatographic column: Waters XBridge C18, 190 × 250 mm, 5 µm; mobile phase system: A: 0.1% aqueous trifluoroacetic acid solution; B: preparative grade acetonitrile; flow rate: 15 mL/min; B% = 20%-100%; column temperature: room temperature.
Prep-HPLC (hydrochloric acid method): chromatographic column: Waters XBridge C18, 190 × 250 mm, 5 µm; mobile phase system: A: 0.1% aqueous hydrochloric acid solution; B: preparative grade acetonitrile; flow rate: 15 mL/min; B% = 20%-100%; column temperature: room temperature.
Prep-HPLC (ammonia water method): chromatographic column: Waters Xbridge C18, 19 × 150 mm, 5 µm; mobile phase A: 0.1% ammonia water solution; mobile phase B: acetonitrile; flow rate: 15 mL/min; column temperature: room temperature.

### Preparation Example 1. Synthesis of Intermediate 1

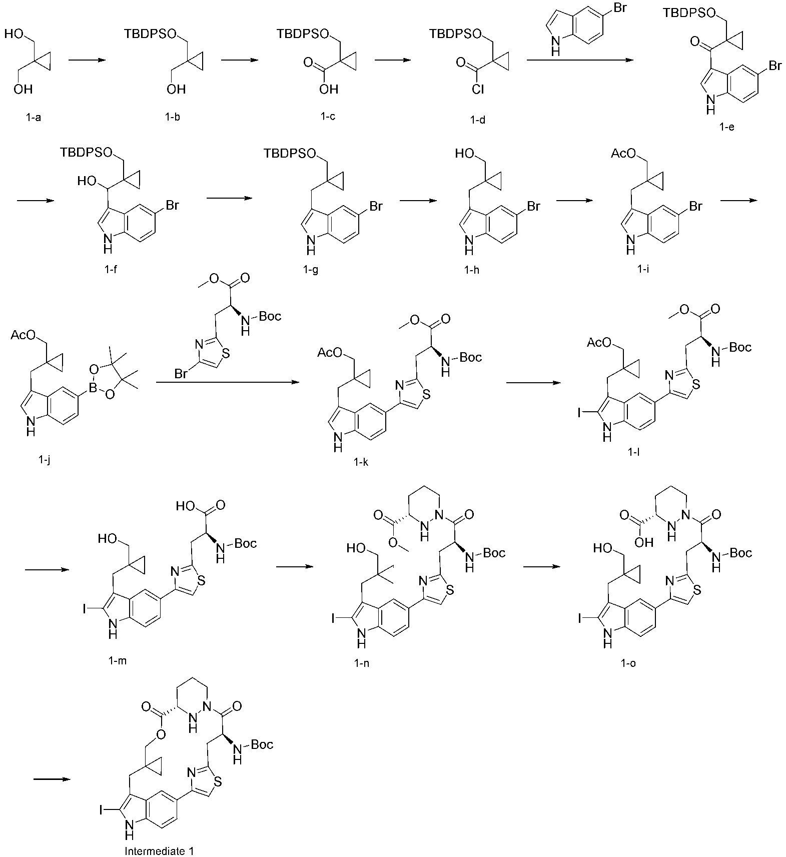

Step 1: A solution of tert-butyldiphenylchlorosilane (51.14 g, 186 mmol) in dichloromethane (100 mL) was slowly added dropwise to a solution of 1,1-bis(hydroxymethyl)cyclopropane (20 g, 196 mmol) and imidazole (14.67 g, 215 mmol) in dichloromethane (400 mL) under an ice bath. After the dropwise addition was completed, the mixture was stirred at room temperature for 1 h. After the reaction was completed, the mixture was washed with 1 M diluted hydrochloric acid (200 mL × 2) and saturated brine (200 mL × 1). The organic phase was dried over anhydrous sodium sulfate, filtered, and concentrated, and the residue was purified by a flash silica gel column (0-10% ethyl acetate/petroleum ether) to give compound 1-b (60 g, yield: 90%). ES-API [M+H-Ph]⁺ = 263.0.

Step 2: Concentrated sulfuric acid (20.66 mL, 388 mmol) was slowly added to a solution of chromium trioxide (29.07 g, 291 mmol) in water (66 mL) under an ice bath. The mixture was stirred for 0.5 h. The obtained reaction mixture was slowly added to a solution of compound 1-b (66 g, 194 mmol) in acetone (600 mL), and the mixture was stirred at room temperature overnight. After the reaction was completed, the mixture was filtered. The filtrate was concentrated, and the residue was purified by a flash silica gel column (0-16% tetrahydrofuran/petroleum ether) to give 1-c (23 g) as a white solid, ES-API [M+H-Ph]⁺ = 277.0.

Step 3: Oxalyl chloride (4.7 mL, 55.55 mmol) and N,N-dimethylformamide (0.29 mL, 3.7 mmol) were sequentially added to a solution of 1-c (13.13 g, 37 mmol) in dichloromethane (100 mL) under an ice bath. The mixture was stirred for 1 h. After the reaction was completed, the mixture was concentrated to give crude 1-d (13.8 g).

Step 4: A 1 M solution (37 mL, 37 mmol) of tin tetrachloride in tetrahydrofuran was added dropwise to a solution of 1-d (13.8 g, 37 mmol) in tetrahydrofuran (100 mL) under a nitrogen atmosphere and an ice bath. The mixture was stirred for 0.5 h. A solution of 5-bromo-1H-indole (7.25 g, 37 mmol) in tetrahydrofuran (100 mL) was then slowly added dropwise, and the mixture was stirred for another 1 h. After the reaction was completed, the reaction mixture was poured into a 1 M sodium hydroxide solution (100 mL) and filtered through celite. The filtrate was washed with water (100 mL) and extracted with ethyl acetate (50 mL). The organic phases were combined, dried over anhydrous sodium sulfate, filtered, and concentrated, and the residue was then purified by a flash silica gel column (0-35% ethyl acetate/petroleum ether) to give 1-e (11.4 g, yield: 58%) as a white solid. ES-API [M+H]⁺ = 532.1.

Step 5: 1-e (2.6 g, 4.88 mmol) was dissolved in tetrahydrofuran (26 mL) and absolute ethanol (26 mL), and sodium borohydride (0.92 g, 24.41 mmol) was slowly added. The mixture was stirred at room temperature for 5 h. After the reaction was completed, the reaction was quenched with a saturated ammonium chloride solution (30 mL), and the mixture was extracted with ethyl acetate (25 mL × 3). The organic phase was dried over anhydrous sodium sulfate, filtered, and concentrated to give crude 1-f (2.7 g), ES-API [M+H-H₂PO]⁺ = 516.1.

Step 6: 1-f (2.7 g, 5.05 mmol) obtained above was dissolved in tetrahydrofuran (40 mL) under an ice bath. p-Toluenesulfonic acid monohydrate (0.1 g, 0.5 mmol) and diethyl 1,4-dihydro-2,6-dimethyl-3,5-pyridinedicarboxylate (1.41 g, 5.56 mmol) were added. The mixture was stirred at room temperature for 2 h. Ethyl acetate (40 mL) was added to the reaction mixture, and the mixture was washed sequentially with a saturated ammonium chloride solution (40 mL) and saturated brine (40 mL). The organic phase was dried over anhydrous sodium sulfate, filtered, and concentrated, and the residue was purified by a flash silica gel column (0-50% dichloromethane/petroleum ether) to give compound 1-g (1.95 g, yield: 75%). ES-API [M+Na]⁺= 540.1, 542.1.

Step 7: Compound 1-g (1.7 g, 3.28 mmol) was dissolved in a 1 M solution (30 mL) of tetrabutylammonium fluoride in tetrahydrofuran. The mixture was stirred at 60 °C for 3 h. After the reaction was completed, water (50 mL) was added, and the mixture was extracted with ethyl acetate (50 mL). The organic phase was dried over anhydrous sodium sulfate, filtered, and concentrated, and the residue was purified by a flash silica gel column (0-50% ethyl acetate/petroleum ether) to give compound 1-h (600 mg). ES-API [M+H-H₂O]⁺ = 262.2.

Step 8: The obtained compound 1-h (600 mg, 2.14 mmol) was dissolved in dichloromethane (20 mL), and triethylamine (160 mg, 2.03 mmol) and acetyl chloride (830 mg, 6.43 mmol) were sequentially added under an ice bath. The mixture was stirred for 0.5 h. After the reaction was completed, the mixture was washed with saturated sodium bicarbonate (20 mL). The organic phase was dried over anhydrous sodium sulfate, filtered, and concentrated, and the residue was purified by a flash silica gel column (0-50% ethyl acetate/petroleum ether) to give compound 1-i (600 mg). ES-API [M+H]⁺ = 322.2.

Step 9: A mixture of compound 1-i (600 mg, 1.86 mmol), bis(pinacolato)diboron (709 mg, 2.79 mmol), [1,1'-bis(diphenylphosphino)ferrocene]palladium(II) dichloride (136 mg, 0.19 mmol), and potassium acetate (772 mg, 5.59 mmol) was stirred at 95 °C for 3 h in toluene (20 mL) under a nitrogen atmosphere. Ethyl acetate (50 mL) was added to the reaction mixture, and the mixture was washed with water (30 mL × 3). The organic phase was dried over anhydrous sodium sulfate, filtered, and concentrated, and the residue was purified by a flash silica gel column (0-50% ethyl acetate/petroleum ether) to give compound 1-j (600 mg). ES-API [M+H]⁺ = 370.2.

Step 10: A mixture of compound 1-j (130 mg, 0.35 mmol), methyl (R)-3-(4-bromothiazol-2-yl)-2-((tert-butoxycarbonyl)amino)propanoate (190 mg, 0.52 mmol), [1,1'-bis(di-tert-butylphosphino)ferrocene]dichloropalladium(II) (23 mg, 0.04 mmol), and potassium phosphate (224 mg, 1.06 mmol) was stirred at 100 °C for 2 h in dioxane (4 mL), toluene (1 mL), and water (1 mL) under a nitrogen atmosphere. After the reaction was completed, ethyl acetate (20 mL) was added, and the mixture was washed with saturated brine (20 mL). The organic phase was dried over anhydrous sodium sulfate, filtered, and concentrated, and the residue was purified by a flash silica gel column (0-70% ethyl acetate/petroleum ether) to give compound 1-k (130 mg). ES-API [M+H]⁺ = 528.1.

Step 11: A solution of elemental iodine (62 mg, 0.25 mmol) in tetrahydrofuran (1 mL) was added to a mixed solution of compound 1-k (130 mg, 0.25 mmol), sodium bicarbonate (23 mg, 0.27 mmol), and silver trifluoroacetate (70 mg, 0.27 mmol) in tetrahydrofuran (5 mL) under an ice bath. The mixture was stirred for 0.5 h. After the reaction was completed, the reaction was quenched with a sodium thiosulfate solution (10 mL), and the mixture was extracted with ethyl acetate (10 mL). The organic phase was dried over anhydrous sodium sulfate, filtered, and concentrated, and the residue was purified by a flash silica gel column (0-70% ethyl acetate/petroleum ether) to give compound 1-l (120 mg). ES-API [M+H]⁺ = 654.0.

Step 12: The obtained compound 1-l (120 mg, 0.18 mmol) was dissolved in tetrahydrofuran (5 mL) and water (1 mL), and anhydrous lithium hydroxide (22 mg, 0.92 mmol) was added. The mixture was stirred at room temperature for 5 h. After the reaction was completed, the mixture was neutralized to pH 6 with 1 M diluted hydrochloric acid and extracted with ethyl acetate (5 mL). The organic phase was dried over anhydrous sodium sulfate, filtered, and concentrated to give crude 1-m (110 mg). ES-API [M+H]⁺ = 598.1.

Step 13: A solution of 1-m (110 mg, 0.18 mmol), methyl (R)-hexahydropyridazine-3-carboxylate ditrifluoroacetate (103 mg, 0.28 mmol), N,N,N',N'-tetramethylchloroformamidinium hexafluorophosphate (56 mg, 0.20 mmol), and N-methylmorpholine (74 mg, 0.9 mmol) in acetonitrile (10 mL) was stirred at room temperature for 2 h under a nitrogen atmosphere. After the reaction was completed, the mixture was diluted with ethyl acetate (10 mL) and washed with water (10 mL). The organic phase was dried over anhydrous sodium sulfate, filtered, and concentrated, and the residue was purified by a flash silica gel column (0-70% ethyl acetate/petroleum ether) to give compound 1-n (120 mg). ES-API [M+H]⁺ = 724.2.

Step 14: The obtained compound 1-n (120 mg, 0.17 mmol) was dissolved in tetrahydrofuran (5 mL) and water (1 mL), and anhydrous lithium hydroxide (12 mg, 0.50 mmol) was added. The mixture was stirred at room temperature for 1 h. After the reaction was completed, the mixture was neutralized to pH 6 with 1 M diluted hydrochloric acid and extracted with ethyl acetate (5 mL). The organic phase was dried over anhydrous sodium sulfate, filtered, and concentrated to give crude 1-o (120 mg). ES-API [M+H]⁺ = 710.1.

Step 15: The obtained 1-o (120 mg, 0.17 mmol) was dissolved in dichloromethane (20 mL), and N,N-diisopropylethylamine (765 mg, 5.92 mmol), 1-hydroxybenzotriazole (228 mg, 1.69 mmol), and 1-(3-dimethylaminopropyl)-3-ethylcarbodiimide hydrochloride (972 mg, 5.07 mmol) were sequentially added. The mixture was stirred at room temperature overnight. After the reaction was completed, the reaction mixture was washed with 0.5 M diluted hydrochloric acid (10 mL × 3). The organic phase was dried over anhydrous sodium sulfate, filtered, and concentrated, and the residue was purified by a flash silica gel column (0-70% ethyl acetate/petroleum ether) to give intermediate 1 (50 mg). ES-API [M+H]⁺ = 692.2. ¹H NMR (400 MHz, DMSO) δ 11.65 (s, 1H), 8.48 (s, 1H), 7.81 (s, 1H), 7.64 (dd, J = 9.6, 1.6 Hz, 1H), 7.37 - 7.30 (m, 2H), 5.23 (t, J = 8.8 Hz, 1H), 5.06 (d, J = 12.0 Hz, 1H), 4.28 (d, J = 12.0 Hz, 1H), 4.22 - 4.10 (m, 2H), 3.50 (d, J = 14.4 Hz, 1H), 3.32 (d, J = 12.0 Hz, 1H), 3.29 - 3.15 (m, 2H), 2.88 - 2.74 (m, 1H), 2.22 - 2.10 (m, 1H), 2.06 - 2.00 (m, 1H), 1.89 - 1.76 (m, 2H), 1.65 - 1.51 (m, 1H), 1.44 (s, 9H), 1.07 - 1.02 (m, 1H), 0.78 - 0.67 (m, 1H), 0.64 - 0.55 (m, 1H), 0.55 - 0.46 (m, 1H).

### Preparation Example 2. Synthesis of Intermediate 2

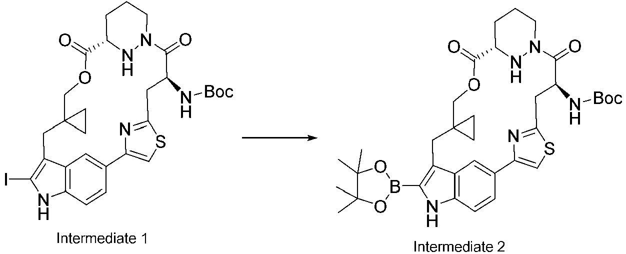

Step 1: Intermediate 1 (510 mg, 0.737 mmol) and pinacolborane (660.64 mg, 5.162 mmol) were dissolved in toluene (10 mL), and tris(dibenzylideneacetone)dipalladium(0) (67.47 mg, 0.074 mmol), 2-dicyclohexylphosphino-2',6'-dimethoxybiphenyl (45.35 mg, 0.111 mmol), and potassium acetate (216.80 mg, 2.212 mmol) were added. The mixture was reacted at 60 °C for 5 h. After the reaction was completed, the reaction mixture was concentrated under reduced pressure, and the residue was purified by silica gel column chromatography (ethyl acetate:petroleum ether = 1:2) to give intermediate 2 (300 mg, 0.434 mmol, yield: 58.82%). ES-API: [M+H]⁺ = 692.3.

### Preparation Example 3. Synthesis of Intermediate 3

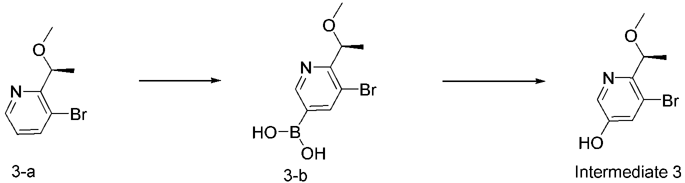

Step 1: Compound 3-a (10 g, 46.279 mmol) was weighed out and dissolved in tetrahydrofuran (100 mL), and bis(pinacolato)diboron (17.63 g, 69.419 mmol), chloro(1,5-cyclooctadiene)iridium(I) dimer (0.93 g, 1.388 mmol), and 4,4'-di-tert-butyl-2,2'-dipyridyl (1.86 g, 6.942 mmol) were added. The mixture was reacted at 80 °C for 15 h under a nitrogen atmosphere, filtered through celite, and concentrated under reduced pressure to give crude compound 3-b, which was directly used in the next step. ES-API [M+H]⁺ = 260.0.

Step 2: The crude product obtained in the previous step was dissolved in acetonitrile (100 mL), and hydrogen peroxide (1308.25 mg, 11.543 mmol) was added dropwise under an ice-water bath. The mixture was reacted at room temperature for 3 h, and the reaction was quenched with a saturated sodium sulfite solution (20 mL). The system was concentrated under reduced pressure and extracted with dichloromethane (25 mL × 3). The organic phase was washed with water (20 mL) and saturated brine (20 mL), dried over anhydrous sodium sulfate, and concentrated under reduced pressure to give a crude product. The crude product was purified by a flash silica gel column (petroleum ether/ethyl acetate = 1/1) to give compound intermediate 3 (7000 mg, 30.162 mmol, yield: 64.8%) as a yellow solid, ES-API [M+H]⁺ = 232.1, 234.1.

### Preparation Example 4. Synthesis of Intermediate 4

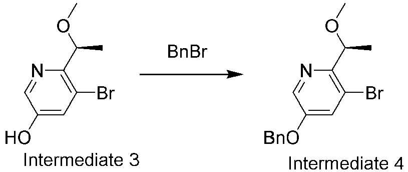

Step 1: Intermediate 3 (500 mg, 2.154 mmol) was dissolved in N,N-dimethylformamide (15 mL), and benzyl bromide (737 mg, 4.308 mmol) and potassium carbonate (893 mg, 6.462 mmol) were added to the solution described above. After the addition was completed, the reaction system was stirred at 80 °C for 17 h. After the reaction was completed, water (30 mL) was added to the reaction mixture, and the mixture was extracted with ethyl acetate (20 mL × 2). The organic phase was separated, washed with saturated brine (40 mL × 3), dried over anhydrous sodium sulfate, filtered, and concentrated to give a crude product. The crude product was purified by a flash silica gel column (methanol/dichloromethane: 0%-2%) to give compound intermediate 4 (600 mg, 1.862 mmol, yield: 86.44%) as a white solid. ES-API: [M+H]⁺ = 322.1, 324.1.

### Preparation Example 5. Synthesis of Intermediate 5

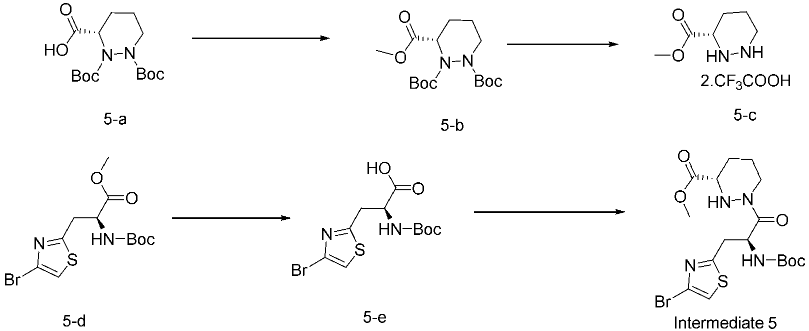

Step 1: Compound 5-a (60 g, 181.609 mmol) was dissolved in DMF (200 mL). Sodium bicarbonate (45.77 g, 544.827 mmol) was added, and iodomethane (33.918 mL, 544.827 mmol) was added dropwise under an ice-water bath and a nitrogen atmosphere. The mixture was reacted overnight at room temperature. Water (200 mL) was added, and the mixture was extracted with ethyl acetate (200 mL × 3), washed with water (200 mL) and saturated brine (200 mL × 3), dried over anhydrous sodium sulfate, and concentrated under reduced pressure to give compound 5-b (65 g, 188.729 mmol, yield: 103.92%) as an oily liquid. ES-API [M-156+H]⁺ = 189.1.

Step 2: Compound 5-b (65 g, 188.729 mmol) was dissolved in dichloromethane (200 mL), and trifluoroacetic acid (200 mL) was added dropwise under an ice-water bath and a nitrogen atmosphere. The mixture was reacted overnight at room temperature and concentrated under reduced pressure to give crude compound 5-c (ditrifluoroacetate) as a colorless transparent oily liquid. The product was directly used in the next step. ES-API [M+H]⁺ = 145.1.

Step 3: Compound 5-d (50 g, 136.896 mmol) was dissolved in tetrahydrofuran (300 mL) and water (75 mL), and lithium hydroxide monohydrate (9.38 g, 223.415 mmol) was added. The mixture was reacted at room temperature for 4 h. The reaction mixture was adjusted to pH 3 with 1 N HCl, extracted with ethyl acetate (200 mL × 3), washed with water (200 mL × 3) and saturated brine (200 mL), dried over anhydrous sodium sulfate, and concentrated under reduced pressure to give compound 5-e (48 g, 136.670 mmol, yield: 99.83%) as a colorless transparent oily liquid. ES-API [M-56+H]⁺ = 295.0.

Step 4: Compound 5-c of step 2 was dissolved in anhydrous dichloromethane (200 mL). NMM (100 g, 996 mmol) was added dropwise under an ice-water bath and a nitrogen atmosphere, and compound 5-e (35 g, 99.655 mmol), EDCI (38.07 g, 199.311 mmol), and HOBt (4.04 g, 29.897 mmol) were sequentially added. The mixture was reacted at room temperature for 4 h, washed sequentially with water (200 mL), 1 N HCl (80 mL), and saturated brine (200 mL), dried over anhydrous sodium sulfate, concentrated under reduced pressure, and slurried (petroleum ether/ethyl acetate = 8/1) to give intermediate 5 (33 g, 69.129 mmol). The mother liquor was then purified by a flash silica gel column to give intermediate 5 (4 g, 8.34 mmol). Compound intermediate 5 (in a total amount of 37 g, 77.5 mmol, yield: 78.7%) was obtained as a white solid. ES-API [M+H]⁺ = 477.1.

### Preparation Example 6. Synthesis of Intermediate 6 and Intermediate 6A

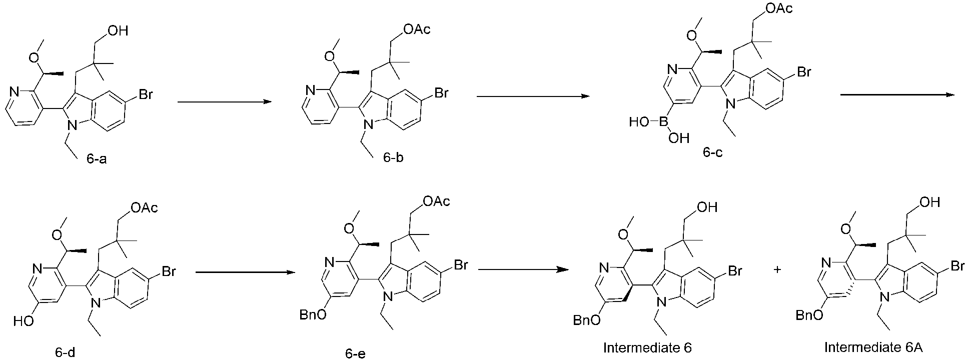

Step 1: Compound 6-a (21 g, 47.15 mmol; see intermediate 1 in WO2022060836A1 for the preparation method) was dissolved in dichloromethane (250 mL), and triethylamine (10 mL, 71 mmol), acetic anhydride (4.5 mL, 47.15 mmol), and DMAP (576 mg, 4.715 mmol) were sequentially added under an ice-water bath and a nitrogen atmosphere. The mixture was reacted at room temperature for 3 h. The reaction was monitored by LC-MS. After the reaction was completed, the mixture was washed with water (100 mL), 0.5 N HCl (50 mL), water (100 mL × 2), and saturated brine (50 mL), dried over anhydrous sodium sulfate, filtered under vacuum, and concentrated under reduced pressure to give a crude product, which was slurried (petroleum ether/ethyl acetate = 8:1) to give compound 6-b (20 g, 41.031 mmol, yield: 87.02%). The mother liquor was purified by a flash separation column to give compound 6-b (2 g, 4.1 mmol) as a white solid. ES-API [M+H]⁺ = 487.1, 489.1. Step 2: Compound 6-b (17.4 g, 35.697 mmol) was dissolved in cyclohexane (150 mL), and bis(pinacolato)diboron (13.60 g, 53.5 mmol), chloro(1,5-cyclooctadiene)iridium(I) dimer (1.20 g, 1.785 mmol), and 4,4'-di-tert-butyl-2,2'-dipyridyl (1.44 g, 5.355 mmol) were added. The mixture was reacted at 80 °C for 15 h under a nitrogen atmosphere. The reaction was monitored by LC-MS. After the reaction was completed, the mixture was filtered through celite under vacuum and concentrated under reduced pressure to give crude compound 6-c (30 g) as a black oily liquid, which was directly used in the next step. ES-API [M+H]⁺ = 531.2,533.2.

Step 3: Crude compound 6-c obtained in the previous step was dissolved in acetonitrile (100 mL), and hydrogen peroxide (11.50 mL, 101.647 mmol) was added dropwise under an ice-water bath. The mixture was reacted at room temperature for 3 h, and the reaction was quenched with a saturated sodium sulfite solution (20 mL). The system was concentrated under reduced pressure and extracted with ethyl acetate (50 mL × 3). The organic phase was washed with water (100 mL) and saturated brine (100 mL), dried over anhydrous sodium sulfate, and concentrated under reduced pressure to give crude compound 6-d as a black oily liquid, ES-API [M+H]⁺ = 503.1, 505.1.

Step 4: Crude compound 6-d obtained in the previous step was dissolved in DMF (100 mL). Potassium carbonate (11.53 g, 83.46 mmol) was added, and benzyl bromide (7.42 g, 43.38 mmol) was added dropwise under an ice-water bath. The mixture was reacted at room temperature for 8 h under a nitrogen atmosphere. The reaction was monitored by LC-MS. After the reaction was completed, the mixture was poured into water (200 mL), extracted with ethyl acetate (150 mL × 3), washed with water (200 mL × 3) and saturated brine (200 mL), dried over anhydrous sodium sulfate, and concentrated under reduced pressure to give crude compound 6-e as a black oily liquid, ES-API [M+H]⁺ = 593.2, 595.2.

Step 5: Crude compound 6-e was dissolved in tetrahydrofuran (100 mL) and water (25 mL), and lithium hydroxide monohydrate (4.2 g, 100 mmol) was added. The mixture was reacted at room temperature overnight. The reaction was monitored by LC-MS. After the reaction was completed, 1 M HCl was added to adjust the pH to 3, and the system was extracted with ethyl acetate (50 mL × 3), washed with water (50 mL × 3) and saturated brine (50 mL), dried over anhydrous sodium sulfate, and concentrated under reduced pressure to give a crude product, which was separated and purified by a flash silica gel column (petroleum ether/ethyl acetate = 65/35) to give 2 intermediate compounds. The structure of one of the intermediate compounds was arbitrarily designated as intermediate 6 (7.6 g, 13.780 mmol, yield: 38.7%, Rf = 0.55), ES-API [M+H]⁺ = 551.2, 553.2. The structure of the other intermediate compound was arbitrarily designated as intermediate 6A (7.8 g, 14.143 mmol, yield: 39.6%, Rf = 0.5), ES-API [M+H]⁺ = 551.2, 553.2.

### Preparation Example 7. Synthesis of Intermediate 6 and Intermediate 6A

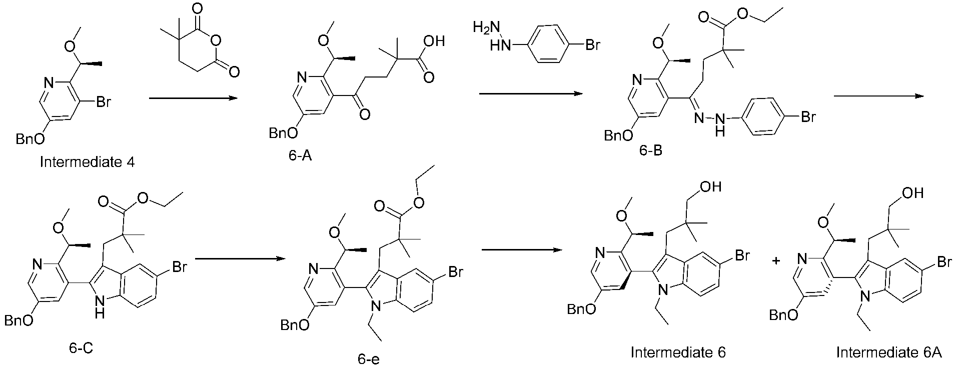

Step 1: Isopropylmagnesium chloride-lithium chloride complex (10.027 mL, 13.035 mmol, a 1.3 M solution in tetrahydrofuran) was slowly added dropwise to a solution of intermediate 4 (3 g, 9.311 mmol) in tetrahydrofuran (20 mL) at 0 °C under a nitrogen atmosphere, and the mixture was stirred at 0 °C for 0.5 h. The solution described above was then added dropwise to a solution of 3,3-dimethyltetrahydropyran-2,6-dione (1.59 g, 11.173 mmol) in tetrahydrofuran (20 mL) at -10 °C, and the mixture was stirred at 0 °C for 2 h. The pH of the solution was adjusted to 4-5 by dropwise addition of 4 M HCl/dioxane (4 mL) to quench the reaction. The reaction mixture was extracted with ethyl acetate (20 mL × 3), dried over anhydrous sodium sulfate, filtered, and concentrated, and the residue was then purified by a silica gel column (petroleum ether/ethyl acetate = 1/1) to give compound 6-A (2.25 g, 5.837 mmol, yield: 62.69%). ES-API: [M+H]⁺ = 386.2.

Step 2: (4-Bromophenyl)hydrazine (970.36 mg, 5.188 mmol) and a 4 M solution of hydrochloric acid in 1,4-dioxane (3.891 mL) were added to a solution of compound 6-A (1000 mg, 2.594 mmol) in ethanol (20 mL), and the reactants were stirred at 90 °C for 36 h. The mixture was concentrated, and the pH was adjusted to neutrality by adding an aqueous sodium bicarbonate solution. The mixture was diluted with water, extracted with ethyl acetate (20 mL × 3), dried over anhydrous sodium sulfate, filtered, and concentrated, and the residue was purified by a silica gel column (petroleum ether/ethyl acetate = 1/2) to give compound 6-B (1.15 g, 1.974 mmol, yield: 76.09%). ES-API: [M+H]⁺ = 582.2, 584.2.

Step 3: Compound 6-B (1.15 g, 1.974 mmol) was dissolved in trifluoroacetic acid (10 mL). The mixture was heated to 65 °C, stirred for 2 h, concentrated under reduced pressure, and diluted with ethyl acetate. The residue was adjusted to pH 7-8 with saturated ammonium bicarbonate and then extracted with ethyl acetate (20 mL × 3). The organic layers were combined, dried over anhydrous Na2SO4, and filtered, and the filtrate was concentrated under reduced pressure and then purified by silica gel column chromatography (petroleum ether/ethyl acetate = 1/2) to give compound 6-C (780 mg, 1.379 mmol, yield: 69.87%). ES-API: [M+H]⁺ = 565.2, 567.2.

Step 4: Compound 6-C (780 mg, 1.379 mmol) was dissolved in dry DMF (5 mL), and cesium carbonate (1347.83 mg, 4.137 mmol) and iodoethane (1075.41 mg, 6.895 mmol) were sequentially added. The mixture was reacted with stirring at room temperature for 2 h under a nitrogen atmosphere. The mixture was extracted with ethyl acetate (20 mL × 3). The organic layers were combined, washed sequentially with water (60 mL) and saturated brine (60 mL), dried over anhydrous Na2SO4, and filtered. The filtrate was concentrated under reduced pressure and then purified by silica gel column chromatography (petroleum ether/ethyl acetate = 1/2) to give compound 6-e (750 mg, 1.264 mmol, yield: 91.61%). ES-API: [M+H]⁺ = 593.2, 595.2.

Step 5: Compound 6-e (750 mg, 1.264 mmol) was dissolved in dry tetrahydrofuran (10 mL). The mixture was cooled to 0 °C under an ice-water bath, and LiAlH₄ (3.159 mL, 3.159 mmol, a 1 M solution in tetrahydrofuran) was slowly added dropwise under a nitrogen atmosphere. After the dropwise addition was completed, the mixture was stirred at 0 °C for another 1 h. After the reaction was completed as detected by LCMS, water (0.12 mL), an aqueous NaOH solution (0.12 mL, 15%), and water (0.36 mL) were slowly added sequentially to quench the reaction mixture. The suspension was stirred at room temperature for 30 min, filtered through celite, and washed with ethyl acetate (100 mL). The mother liquor was dried over anhydrous sodium sulfate, filtered, and concentrated, and the residue was then purified by a flash silica gel column (ethyl acetate/petroleum ether = 20%-60%) to give 2 intermediate compounds. The structure of one of the intermediate compounds was arbitrarily designated as: (Ra)-3-(2-(5-(benzyloxy)-2-((1S)-1-methoxyethyl)pyridin-3-yl)-5-bromo-1-ethylindol-3-yl)-2,2-dimethylpropan-1-ol (intermediate 6, 300 mg, 0.544 mmol, yield: 43.05%).

The structure of the other intermediate compound was arbitrarily designated as: (Sa)-3-(2-(5-(benzyloxy)-2-((1S)-1-methoxyethyl)pyridin-3-yl)-5-bromo-1-ethylindol-3-yl)-2,2-dimethylpropan-1-ol (intermediate 6A, 210 mg, 0.381 mmol, yield: 30.13%). ES-API: [M+H]⁺ = 551.2, 553.2.

### Single-crystal growth of intermediate 6

Single-crystal growth of 3-((Rₐ)-2-(5-(benzyloxy)-2-((1S)-1-methoxyethyl)pyridin-3-yl)-5-bromo-1-ethylindol-3-yl)-2,2-dimethylpropan-1-ol (intermediate 6).

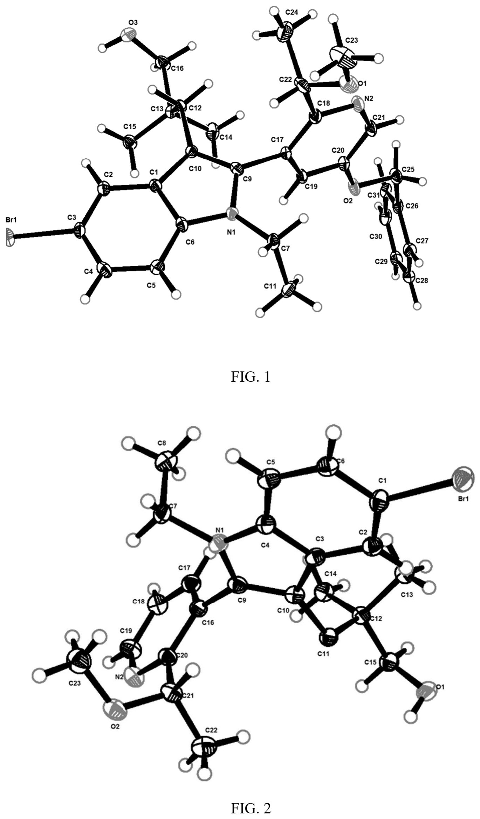

About 10 mg of a powder sample of intermediate 6 was weighed out, and 1 mL of acetonitrile was added. The mixture was completely dissolved by ultrasonication and slowly volatilized in a fume hood. After a period of time, a yellowish-brown needle-like crystal, i.e., a single crystal of intermediate 6, was precipitated. The X-ray single-crystal diffraction test was performed using a Bruker D8 Venture instrument. The results are shown in Table 1 below and FIG. 1. FIG. 1 shows an ellipsoid plot of the molecular three-dimensional structure.

### Instrument parameters:

| | | | |
|---|---|---|---|
| Light source: | Cu target | X-ray: | Cu-Ka (= 1.54178 Å) |
| Detector: | CMOS plane detector | Resolution: | 0.80 Å |
| Current and voltage: | 50 kV, 1.2 mA | Exposure time: | 3 s |
| Area detector-to-sample distance: | 40 mm | Test temperature: | 170(2) K |

### Structure analysis and refinement procedures:

The diffraction data were integrated and reduced using the SAINT program, followed by empirical absorption correction using the SADABS program. The single crystal structure was solved by the direct method using SHELXT2014 and refined by the least square method. The hydrogen atom refinement process adopted isotropic calculation. The hydrogen atoms on N and O were located from residual electron density. The hydrogen atoms on C-H were geometrically added and refined using a riding model. The Flack constant was 0.035 (14). In FIG. 1, C22 is in S configuration, and the axial chirality at positions C9-C17 is in Rₐ configuration.

### Crystal data:

**Table 1**

| | |
|---|---|
| Molecular formula | C₃₀H₃₅BrN₂O₃ |
| Molecular weight | *Mᵣ* = 551.51 |
| Shape | Block, colorless |
| Crystal system | Orthorhombic |
| *Space group* | *P*2₁2₁2₁ |
| Cell parameters | *a* = 7.3026 (2) Å; *b* = 17.4557 (4) Å; c = 20.6871 (5) Å |
| Unit cell volume | *V* = 2637.03 (11) Å³ |
| Molecule number in unit cell | *Z* = 4 |
| Unit cell dimensions | 0.19 × 0.08 × 0.05 mm |
| Electron number in unit cell | *F*(000) = 1152 |
| Calculating density | *D*ₓ = 1.389 Mg m⁻³ |
| Radiation source and wavelength | Cu Ka radiation, l = 1.54178 Å |
| Determining the number of diffraction points of unit cell | Cell parameters from 9899 reflections |
| Range for data collection | q = 2.5-63.8° |
| Absorption coefficient | m = 2.39 mm⁻¹ |
| Temperature | *T* = 170 K |

### Preparation Example 8. Synthesis of Intermediate 7

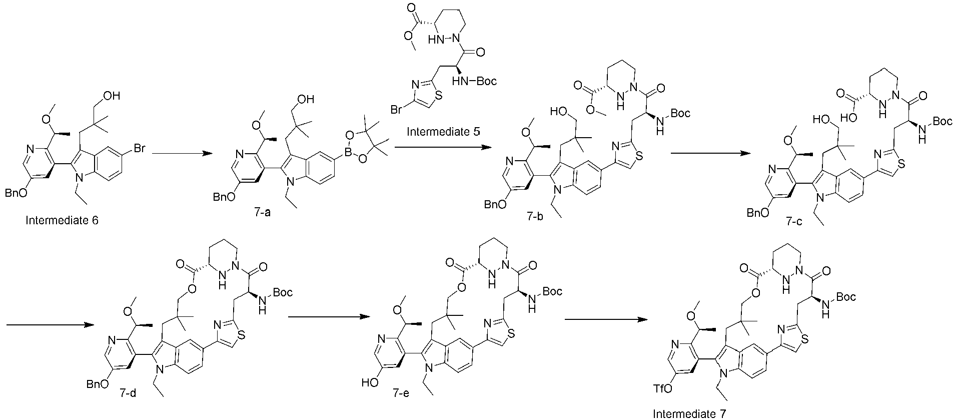

Step 1: Intermediate 6 (18 g, 32.6 mmol), bis(pinacolato)diboron (16.6 g, 65.2 mmol), potassium acetate (6.41 g, 65.27 mmol), and Pd(dppf)Cl₂ (2.39 g, 3.26 mmol) were mixed in anhydrous toluene (200 mL) under a nitrogen atmosphere, and the mixture was stirred at 90 °C for 4 h. After the reaction was completed, the mixture was cooled and concentrated to remove toluene, and 300 mL of ethyl acetate and 500 mL of water were poured into the mixture. The resulting mixture was extracted 3 times with ethyl acetate and washed sequentially with 500 mL of water and 500 mL of saturated brine. The organic phase was dried over anhydrous sodium sulfate, filtered, and concentrated, and the residue was purified by a flash silica gel column (0-50% petroleum ether/ethyl acetate) to give compound 7-a (20.8 g, yield: 85%). ES-API [M+H]⁺ = 599.3.

Step 2: Compound 7-a (24 g, 40.09 mmol), intermediate 5 (22.97 g, 48.11 mmol), K₃PO₄ (17.05 g, 80.19 mmol), and Pd(dtbpf)Cl₂ (2.07 g, 3.21 mmol) were mixed in a mixed solution of toluene (50 mL), dioxane (150 mL), and water (50 mL) under a nitrogen atmosphere, and the mixture was stirred at 70 °C for 18 h. After the reaction was completed, 300 mL of ethyl acetate and 300 mL of water were poured into the mixture. The resulting mixture was extracted 3 times with ethyl acetate and washed sequentially with 300 mL of water and 300 mL of saturated brine. The organic phase was dried over anhydrous sodium sulfate, filtered, and concentrated, and the residue was purified by a flash silica gel column (0-50% petroleum ether/ethyl acetate) to give compound 7-b (28 g, yield: 80%). ES-API [M+H]⁺ = 869.3.

Step 3: Water (50 mL) and LiOH·H₂O (2.71 g, 64.435 mmol) were added to a solution of compound 7-b (28 g, 32.218 mmol) in tetrahydrofuran (200 mL) at 0 °C. The mixture was stirred at 0 °C for 1 h. After the reaction was completed, the reaction mixture was adjusted to pH 3-4 with 1 M hydrochloric acid, and 200 mL of ethyl acetate and 200 mL of water were poured into the reaction mixture. The mixture was extracted 3 times with ethyl acetate and washed sequentially with 200 mL of water and 200 mL of saturated brine. The organic phase was dried over anhydrous sodium sulfate, filtered, and concentrated to give compound 7-c (28 g, crude product). ES-API [M+H]⁺ = 855.3.

Step 4: HOBt (39.51 g, 292.377 mmol) and DIPEA (178.254 mL, 1023.320 mmol) were added to a solution of compound 7-c (25 g, 29.3 mmol) in dichloromethane (1000 mL), and EDCI (168.15 g, 877.131 mmol) was slowly added at 0 °C. The mixture was stirred at room temperature for 16 h. After the reaction was completed, the reaction mixture was adjusted to pH 5-6 with 1 M hydrochloric acid, and 200 mL of ethyl acetate and 200 mL of water were poured into the mixture. The resulting mixture was extracted 3 times with ethyl acetate and washed sequentially with 200 mL of water and 200 mL of saturated brine. The organic phase was dried over anhydrous sodium sulfate, filtered, and concentrated, and the residue was purified by a flash silica gel column (0-80% petroleum ether/ethyl acetate) to give compound 7-d (17.1 g, purity: 86%, yield: 60%). ES-API [M+H]⁺ = 837.3.

Step 5: Pd/C 10% (7.12 g, 6.690 mmol) was added to a solution of compound 7-d (14 g, 16.725 mmol) in methanol (350 mL), and the mixture was reacted at room temperature for 40 h under a hydrogen atmosphere. The reaction mixture was filtered and concentrated to give compound 7-e (12 g, purity: 90%, yield: 87%). ES-API [M+H]⁺ = 747.3.

Step 6: Triethylamine (2.791 mL, 20.082 mmol) and N-phenyl-bis(trifluoromethanesulfonimide) (3.59 g, 10.041 mmol) were added to a solution of compound 7-e (5 g, 6.694 mmol) in dichloromethane (60 mL), and the mixture was stirred at room temperature for 1 h. After the reaction was completed, 50 mL of ethyl acetate and 50 mL of water were poured into the mixture. The resulting mixture was extracted 3 times with ethyl acetate and washed sequentially with 50 mL of water and 50 mL of saturated brine. The organic phase was dried over anhydrous sodium sulfate, filtered, and concentrated, and the residue was purified by a flash silica gel column (0-60% petroleum ether/ethyl acetate) to give compound intermediate 7 (5 g, purity: 91%, yield: 77%). ES-API [M+H]⁺ = 879.3.

### Preparation Example 9. Synthesis of Intermediate 7

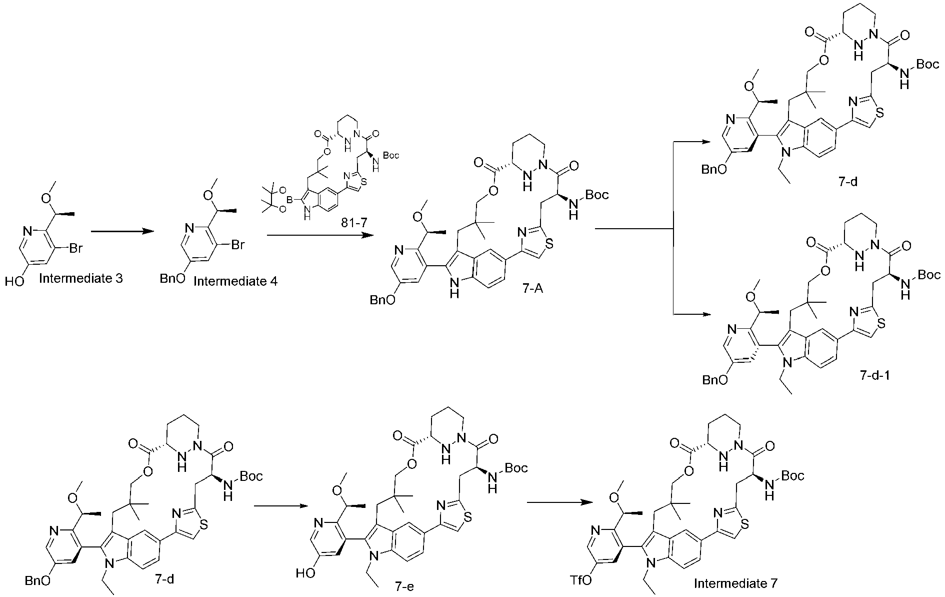

Step 1: Intermediate 3 (500 mg, 2.154 mmol) was dissolved in *N*,*N*-dimethylformamide (10 mL), and benzyl bromide (0.512 mL, 4.308 mmol) and potassium carbonate (893 mg, 6.462 mmol) were added to the solution described above. After the addition was completed, the reaction system was stirred at 80 °C for 17 h. Water (50 mL) was added to the reaction mixture, and the mixture was extracted with ethyl acetate (50 mL × 2). The organic phase was washed with saturated brine (50 mL × 3), dried over anhydrous sodium sulfate, filtered, and concentrated to give a crude product. The crude product was purified by a flash silica gel column (methanol/dichloromethane: 0%-2%) to give intermediate 4 (600 mg, 1.862 mmol, yield: 86.44%) as a white solid. ES-API: [M+H]⁺ = 322.1, 324.1.

Step 2: Intermediate 4 (279 mg, 0.865 mmol) was dissolved in dioxane (1 mL), toluene (3 mL), and water (1 mL). Compound 81-7 (300 mg, 0.432 mmol), [1,1'-bis(di-tert-butylphosphino)ferrocene]dichloropalladium(II) (28 mg, 0.043 mmol), and potassium phosphate (275.40 mg, 1.297 mmol) were added to the solution described above at room temperature. After the addition was completed, the reaction system was stirred at 70 °C for 2 h under an argon atmosphere. After the reaction was completed, water (30 mL) was added to the reaction mixture, and the mixture was extracted with ethyl acetate (30 mL × 2). The organic phase was washed with saturated brine (30 mL × 3), dried over anhydrous sodium sulfate, filtered, and concentrated to give a crude product. The crude product was purified by a flash silica gel column (methanol/dichloromethane: 0%-3%) to give compound 7-A (300 mg, 0.371 mmol, yield: 85.74%) as a light yellow solid. ES-API: [M+H]⁺ = 809.1.

Step 3: Compound 7-A (600 mg, 0.742 mmol) was dissolved in N,N-dimethylformamide (10 mL). Iodoethane (580 mg, 3.710 mmol) and cesium carbonate (725 mg, 2.226 mmol) were added to the solution described above. The reaction system was stirred at room temperature for 2 h. After the reaction was completed, water (50 mL) was added to the solution described above, and the mixture was extracted with ethyl acetate (30 mL × 2). The organic phase was washed with saturated brine (50 mL × 3), dried over anhydrous sodium sulfate, filtered, and concentrated to give a crude product. The crude product was purified by a flash silica gel column (ethyl acetate/petroleum ether: 0%-50%) to give 2 isomeric compounds. The structure of one of the isomeric compounds was arbitrarily designated as compound 7-d (200 mg, 0.239 mmol, yield: 32.22%, ethyl acetate:petroleum ether = 1:1, Rf = 0.35) as a white solid. ES-API: [M+H]⁺ = 837.1.

The structure of the other isomeric compound was arbitrarily designated as compound 7-d-1 (350 mg, 0.418 mmol, yield: 56.35%, ethyl acetate:petroleum ether = 1:1, Rf = 0.40) as a white solid. ES-API: [M+H]⁺ = 837.1.

Step 4: Compound 7-d (200 mg, 0.239 mmol) was dissolved in methanol (5 mL), and wet palladium on carbon (10 wt%, 20 mg) and wet palladium hydroxide/carbon (10 wt%, 20 mg) were added to the solution described above. After being purged three times with hydrogen, the reaction system was stirred at room temperature for 17 h under a hydrogen atmosphere (15 psi). The reaction mixture was filtered through celite, and the filter cake was washed with methanol (20 mL). The filtrate was concentrated to give compound 7-e (150 mg, 0.201 mmol, yield: 84%) as a white solid. ES-API: [M+H]⁺ = 747.1.

Step 5: Compound 7-e (40 mg, 0.054 mmol) was dissolved in dichloromethane (2 mL), and triethylamine (16 mg, 0.16 mmol) and 1,1,1-trifluoro-N-phenyl-N-[(trifluoromethyl)sulfonyl]methanesulfonamide (28.70 mg, 0.080 mmol) were added to the solution described above. After the addition was completed, the reaction system was stirred at room temperature for 2 h. Water (10 mL) was added to the reaction mixture, and the mixture was extracted with dichloromethane (15 mL × 2). The organic phase was washed with saturated brine (20 mL × 2), dried over anhydrous sodium sulfate, filtered, and concentrated to give a crude product. The crude product was purified by a flash silica gel column (methanol/dichloromethane: 0%-2%) to give intermediate 7 (30 mg, 0.034 mmol, yield: 63.73%) as a white solid. ES-API: [M+H]⁺ = 879.2.

### Preparation Example 10. Synthesis of Intermediate 8

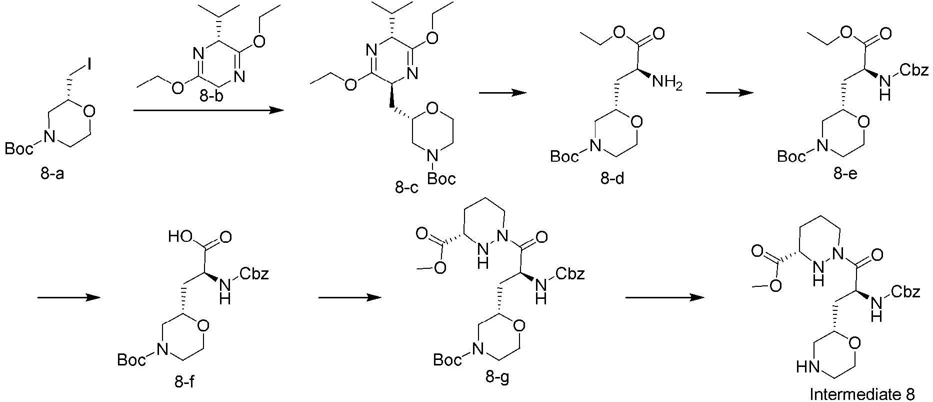

Step 1: Compound 8-b (2500 mg, 13.569 mmol) was dissolved in tetrahydrofuran (60 mL), and the mixture was cooled to - 78 °C. A solution of n-butyllithium in n-hexane (2.5 M, 5.653 mL, 14.132 mmol) was slowly added dropwise to the solution described above under a nitrogen atmosphere. After the addition was completed, the reaction system was stirred at -78 °C for another 30 min. Compound 8-a (3467.47 mg, 10.599 mmol) was then dissolved in tetrahydrofuran (60 mL), and the mixture was slowly added to the reaction system at -78 °C. After the addition was completed, the reaction system was warmed to room temperature and stirred for another 2 h. After the reaction was completed, the reaction was quenched with a saturated ammonium chloride solution (50 mL), and the system was extracted with ethyl acetate (60 mL × 2). The organic phase was washed with saturated brine (50 mL), dried over anhydrous sodium sulfate, filtered, and concentrated to give a crude product. The crude product was purified by a flash silica gel column (tetrahydrofuran/petroleum ether: 0%-25%) to give compound 8-c (4500 mg, crude product) as a colorless transparent liquid. ES-API: [M+H]⁺ = 412.1.

Step 2: Compound 8-c (4500 mg, crude product) was dissolved in acetonitrile (5 mL), and an aqueous hydrochloric acid solution (0.5 M, 10 mL) was added to the solution described above. The reaction system was stirred at room temperature for 17 h. After the reaction was completed, the reaction mixture was basified with ammonia water (25 wt%, 10 mL) and extracted with ethyl acetate (20 mL × 3). The organic phase was washed with saturated brine (50 mL), dried over anhydrous sodium sulfate, filtered, and concentrated to give crude compound 8-d (3000 mg, crude product) as a colorless transparent liquid. ES-API: [M+H]⁺ = 303.1.

Step 3: Crude compound 8d (3000 mg, crude product) was dissolved in dichloromethane (50 mL), and the mixture was cooled to 0 °C. Triethylamine (4.137 mL, 29.765 mmol) and benzyl chloroformate (2.095 mL, 14.882 mmol) were added to the solution described above. After the addition was completed, the reaction system was warmed to room temperature and stirred for another 2 h. After the reaction was completed, the reaction was quenched with water (50 mL), and the system was extracted with dichloromethane (40 mL × 2). The organic phase was washed with saturated brine (50 mL), dried over anhydrous sodium sulfate, filtered, and concentrated to give a crude product. The crude product was purified by a flash silica gel column (tetrahydrofuran/petroleum ether: 0%-20%) to give compound 8-e (1200 mg, 2.749 mmol, three-step yield: 20.3%) as a colorless transparent liquid. ES-API: [M-Boc+H]⁺ = 337.1.

Step 4: Compound 8-e (150 mg, 0.344 mmol) was dissolved in tetrahydrofuran (2 mL) and water (2 mL). Lithium hydroxide (43.26 mg, 1.031 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (20 mL × 2). The organic phase was washed with saturated brine (20 mL), dried over anhydrous sodium sulfate, filtered, and concentrated to give compound 8-f (140 mg, 0.343 mmol, yield: 99.74%) as a light yellow solid. ES-API: [M-Boc+H]⁺ = 309.1.

Step 5: Compound 8-f (370 mg, 0.906 mmol) was dissolved in dichloromethane (10 mL), and the mixture was cooled to 0 °C. Methyl (S)-hexahydropyridazine-3-carboxylate ditrifluoroacetate (505.77 mg, 1.359 mmol), N-methylmorpholine (0.996 mL, 9.059 mmol), 1-(3-dimethylaminopropyl)-3-ethylcarbodiimide hydrochloride (347.31 mg, 1.812 mmol), and 1-hydroxybenzotriazole (36.72 mg, 0.272 mmol) were added to the solution described above. After the addition was completed, the reaction system was stirred at room temperature for 17 h. After the reaction was completed, the reaction was quenched with water (20 mL), and the system was extracted with dichloromethane (20 mL × 2). The organic phase was washed with saturated brine (20 mL), dried over anhydrous sodium sulfate, filtered, and concentrated to give a crude product. The crude product was purified by a flash silica gel column (tetrahydrofuran/petroleum ether: 0%-35%) to give compound 8-g (130 mg, 0.243 mmol, yield: 26.84%) as a white solid. ES-API: [M+H]⁺ = 535.1.

Step 6: Compound 8-g (120 mg, 0.224 mmol) was dissolved in a solution of hydrogen chloride in dioxane (4 M, 2 mL). The reaction system was stirred at room temperature for 2 h. After the reaction was completed, the reaction mixture was concentrated to give crude hydrochloride. The crude product was basified with a saturated aqueous sodium bicarbonate solution (10 mL) and extracted with dichloromethane (10 mL × 3). The organic phase was washed with saturated brine (20 mL), dried over anhydrous sodium sulfate, filtered, and concentrated to give compound intermediate 8 (95 mg, 0.219 mmol, yield: 97.41 %) as a white solid. ES-API: [M+H]⁺ = 435.1.

### Example 1. Synthesis of Compounds Z1 and Z2

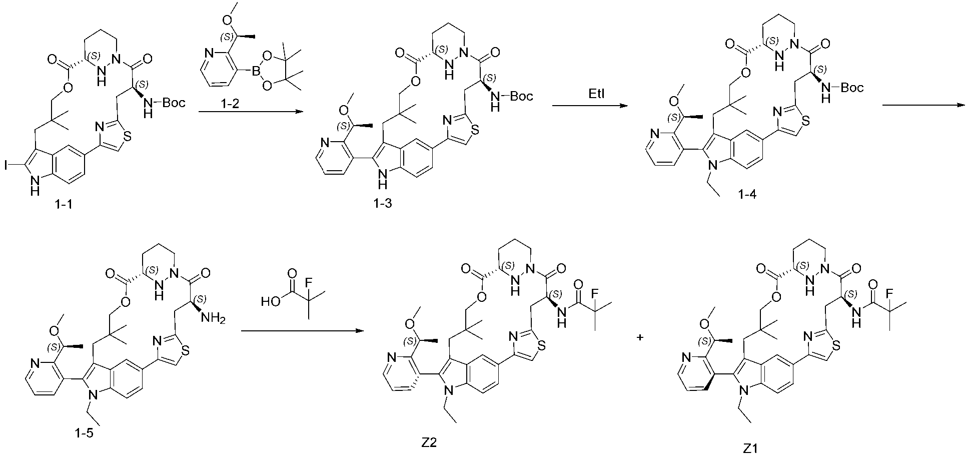

Step 1: A mixture of compound 1-1 (100 mg, 0.14 mmol, see intermediate 3 in WO2022060836A1 for the preparation method), compound 1-2 (50 mg, 0.19 mmol), [1,1'-bis(diphenylphosphino)ferrocene]palladium(II) dichloride (10 mg, 0.01 mmol), and potassium phosphate (92 mg, 0.43 mmol) was stirred under microwave at 70 °C for 0.5 h in dioxane (1.2 mL) and water (0.4 mL) under a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 1-3 (85 mg, yield: 84%) as a transparent oil. ES-API [M+H]⁺ = 703.2.

Step 2: Compound 1-3 (80 mg, 0.11 mmol) was dissolved in N,N-dimethylformamide (1 mL), and cesium carbonate (185 mg, 0.57 mmol) and iodoethane (0.02 mL, 0.23 mmol) were sequentially added. The mixture was stirred at room temperature for 2 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 1-4 (70 mg, yield: 84%) as a yellow oil. ES-API [M+H]⁺ = 731.1.

Step 3: Compound 1-4 (70 mg, 0.1 mmol) was dissolved in a solution of hydrochloric acid in dioxane (1 mL, 4 mol/L). The mixture was stirred at room temperature for 2 h. After the reaction was completed, the reaction mixture was concentrated, and the residue was basified with a saturated sodium bicarbonate solution until the pH was 8, extracted with ethyl acetate (20 mL × 3), and concentrated to give compound 1-5 (60 mg). ES-API [M+H]⁺ = 631.1.

Step 4: Compound 1-5 (70 mg, 0.11 mmol) was dissolved in N,N-dimethylformamide (1 mL), and 2-fluoro-2-methylpropionic acid (18 mg, 0.17 mmol), N,N-diisopropylethylamine (0.12 mL, 0.67 mmol), and O-(7-azabenzotriazol-1-yl)-N,N,N',N'-tetramethyluronium hexafluorophosphate (127 mg, 0.33 mmol) were sequentially added. The mixture was stirred at room temperature for 2 h. After the reaction was completed, ethyl acetate (20 mL) was added, and the mixture was washed with saturated brine (10 mL) and water (10 mL). The organic phase was dried over anhydrous sodium sulfate, filtered, and concentrated, and the residue was purified by prep-HPLC (ammonium bicarbonate method) to give two isomeric compounds. One of the structures was arbitrarily designated as N-((1²S6'S,4S,Z)-1'-ethyl-1²-(2-((S)-1-methoxyethyl)pyridin-3-yl)-10,10-dimethyl-5,7-dioxo-6¹,6²,6³,6⁴,6⁵6⁶-hexahydro-1¹H-8-oxa-2(4,2)-thiazola-1(5,3)-indola-6(1,3)-pyridazinacycloundecaphane-4-yl)-2-fluoro-2-methylpropanamide (Z2, 6.05 mg, yield: 7.6%, purity: 98%, retention time: 9.15 min). ¹H NMR (400 MHz, DMSO-d₆) δ 8.77 (dd, *J =* 4.8 Hz, 1.6 Hz, 1H), 8.53 (d, *J =* 1.2 Hz, 1H), 8.36 (dd, *J* = 9.2 Hz, 2.8 Hz, 1H), 7.93 (dd, *J =* 7.6 Hz, 1.6 Hz, 1H), 7.82 (s, 1H), 7.74 (dd, *J =* 8.8 Hz, 1.6 Hz, 1H), 7.56 - 7.48 (m, 2H), 5.54 (t, *J* = 8.8 Hz, 1H), 5.09 (d, *J =* 12.0 Hz, 1H), 4.31 - 4.18 (m, 2H), 4.04 - 3.88 (m, 2H), 3.85 - 3.72 (m, 1H), 3.70 - 3.63 (m, 1H), 3.62 - 3.52 (m, 2H), 3.29 (s, 4H), 3.08 - 3.01 (m, 1H), 2.85 - 2.71 (m, 1H), 2.40 - 2.34 (m, 1H), 2.18 - 2.08 (m, 1H), 1.81 (s, 2H), 1.62 - 1.43 (m, 7H), 1.23 (d, *J* = 6.4 Hz, 3H), 1.10 (t, *J* = 7.2 Hz, 3H), 0.93 (s, 3H), 0.48 (s, 3H). ES-API [M+H]⁺ = 719.3.

The other structure was arbitrarily designated as: N-((1²R,6³S,4S,Z)-1¹-ethyl-1²-(2-((S)-1-methoxyethyl)pyridin-3-yl)-10,10-dimethyl-5,7-dioxo-6¹,6 ² 6³,6⁴ 6⁵ 6⁶-hexahydro-1H-8-oxa-2(4,2)-thiazola-1(5,3)-indola-6(1,3)-pyridazinacycloundecaphane-4-yl)-2-fluoro-2-methylpropanamide (Z1, 3.57 mg, yield: 4.5%, purity: 98%, retention time: 10.30 min). ¹H NMR (400 MHz, DMSO-d₆) δ 8.75 (dd, *J =* 4.8 Hz, 1.6 Hz, 1H), 8.50 (d, *J =* 1.2 Hz, 1H), 8.34 (dd, *J =* 8.8 Hz, 2.4 Hz, 1H), 7.81 (s, 1H), 7.79 (dd, *J =* 7.6 Hz, 1.6 Hz, 1H), 7.75 (dd, *J =* 8.8 Hz, 1.6 Hz, 1H), 7.58 (d, *J =* 8.4 Hz, 1H), 7.52 (dd, *J=* 7.6 Hz, 4.8 Hz, 1H), 5.54 (t, *J =* 8.8 Hz, 1H), 5.12 (d, *J =* 12.0 Hz, 1H), 4.38 - 4.07 (m, 5H), 3.67 - 3.49 (m, 3H), 3.30 - 3.28 (m, 1H), 3.27 (s, 3H), 2.97 (d, *J =* 14.0 Hz, 1H), 2.82 - 2.72 (m, 1H), 2.40 (d, *J =* 14.5 Hz, 1H), 2.16 - 2.04 (m, 1H), 1.80 (s, 2H), 1.62 - 1.43 (m, 7H), 1.37 (d, *J =* 6.0 Hz, 3H), 0.91 (s, 3H), 0.85 (t, *J =* 6.8 Hz, 3H), 0.32 (s, 3H). ES-API [M+H]⁺ = 719.3.

### Example 2. Synthesis of Compounds Z3, Z4, and Z5

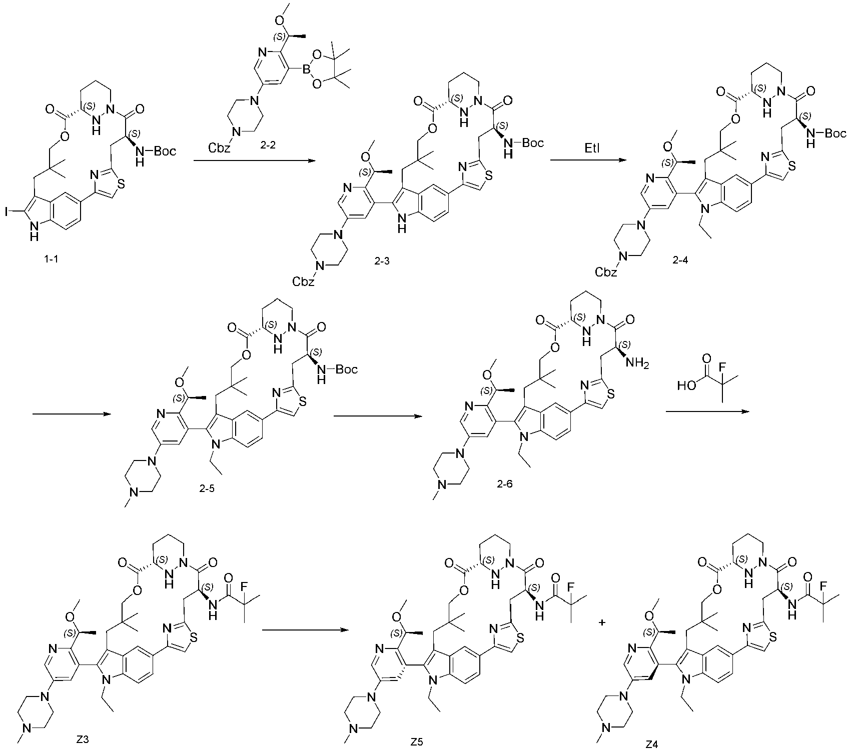

Step 1: A mixture of compound 1-1 (300 mg, 0.43 mmol), compound 2-2 (481 mg, 0.78 mmol, see intermediate 5 in WO2022060836A1 for the preparation method), [1,1'-bis(di-tert-butylphosphino)ferrocene]dichloropalladium(II) (56 mg, 0.09 mmol), and potassium phosphate (275 mg, 1.3 mmol) was stirred at 60 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 2-3 (260 mg, yield: 65%) as a transparent oil. ES-API [M+H]⁺ = 922.3.

Step 2: Compound 2-3 (250 mg, 0.27 mmol) was dissolved in N,N-dimethylformamide (1 mL), and cesium carbonate (442 mg, 0.57 mmol) and iodoethane (635 mg, 4.0 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 2-4 (255 mg, yield: 99%) as a yellow oil. ES-API [M+H]⁺ = 949.3.

Step 3: Compound 2-4 (255 mg, 0.27 mmol) was dissolved in methanol (10 mL), and paraformaldehyde (242 mg, 8.1 mmol) and Pd(OH)₂/C (377 mg, 0.27 mmol) were sequentially added. The mixture was stirred at room temperature for 16 h. After the reaction was completed, the mixture was filtered and concentrated, and the residue was purified by a flash silica gel column (0-60% tetrahydrofuran/petroleum ether) to give compound 2-5 (207 mg, yield: 93%). ES-API [M+H]⁺ = 829.3.

Step 4: Compound 2-5 (200 mg, 0.24 mmol) was dissolved in a solution of hydrochloric acid in dioxane (5 mL, 4 mol/L). The mixture was stirred at room temperature for 2 h. After the reaction was completed, the mixture was concentrated, and the residue was basified with a saturated sodium bicarbonate solution to pH 8, extracted with ethyl acetate (20 mL × 3), and concentrated to give compound 2-6 (175 mg, yield: 99%). ES-API [M+H]⁺ = 729.2.

Step 5: Compound 2-6 (87 mg, 0.12 mmol) was dissolved in N,N-dimethylformamide (6 mL), and 2-fluoro-2-methylpropionic acid (19 mg, 0.18 mmol), N,N-diisopropylethylamine (93 mg, 0.72 mmol), and O-(7-azabenzotriazol-1-yl)-N,N,N',N'-tetramethyluronium hexafluorophosphate (137 mg, 0.36 mmol) were sequentially added. The mixture was stirred at room temperature for 1 h.After the reaction was completed, ethyl acetate (20 mL) was added, and the mixture was washed with saturated brine (10 mL) and water (10 mL). The organic phase was dried over anhydrous sodium sulfate, filtered, and concentrated, and the residue was purified by prep-HPLC (ammonium bicarbonate method) to give Z3 (40 mg, yield: 41%, purity: 98%). ES-API [M+H]⁺ = 817.4.

Step 6: Z3 (40 mg) was resolved by a chiral column (IB column, 250 mm × 4.6 mm × 5 µm, mobile phase: n-hexane:EtOH:DEA = 60:40:2; flow rate: 1 mL/min, T = 30 °C) to give two isomeric compounds. One of the structures was arbitrarily designated as N-((1²S,6³S,4S,Z)-1¹-ethyl-1²-(2-((S)-1-methoxyethyl)-5-(4-methylpiperazin-1-yl)pyridin-3-yl)-10,10-dimethyl-5,7-dioxo-6¹,6²,6³,6⁴ 6⁵,6⁶-hexahydro-1¹H-8-oxa-2(4,2)-thiazola-1(5,3)-indola-6(1,3)-pyridazinacycloundecaphane-4-yl)-2-fluoro-2-methylpropanamide (Z5, 18 mg, yield: 45%, purity: 98%, retention time: 4.596 min). ES-API [M+H]⁺ = 817.4.

The other structure was arbitrarily designated as: N-((1²R,6³S,4S,Z)-1¹-ethyl-1²-(2-((S)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-4-yl)-2-fluoro-2-methylpropanamide (Z4, 10 mg, yield: 25%, purity: 98%, retention time: 6.627 min). ES-API [M+H]⁺ = 817.4.

### Example 3. Synthesis of Compound Z69

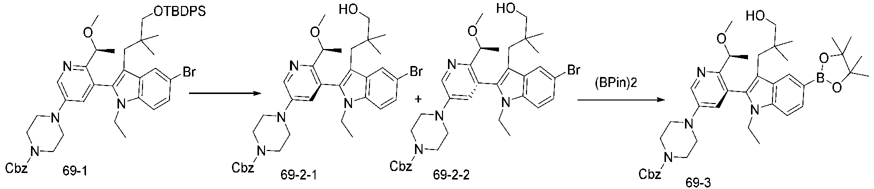

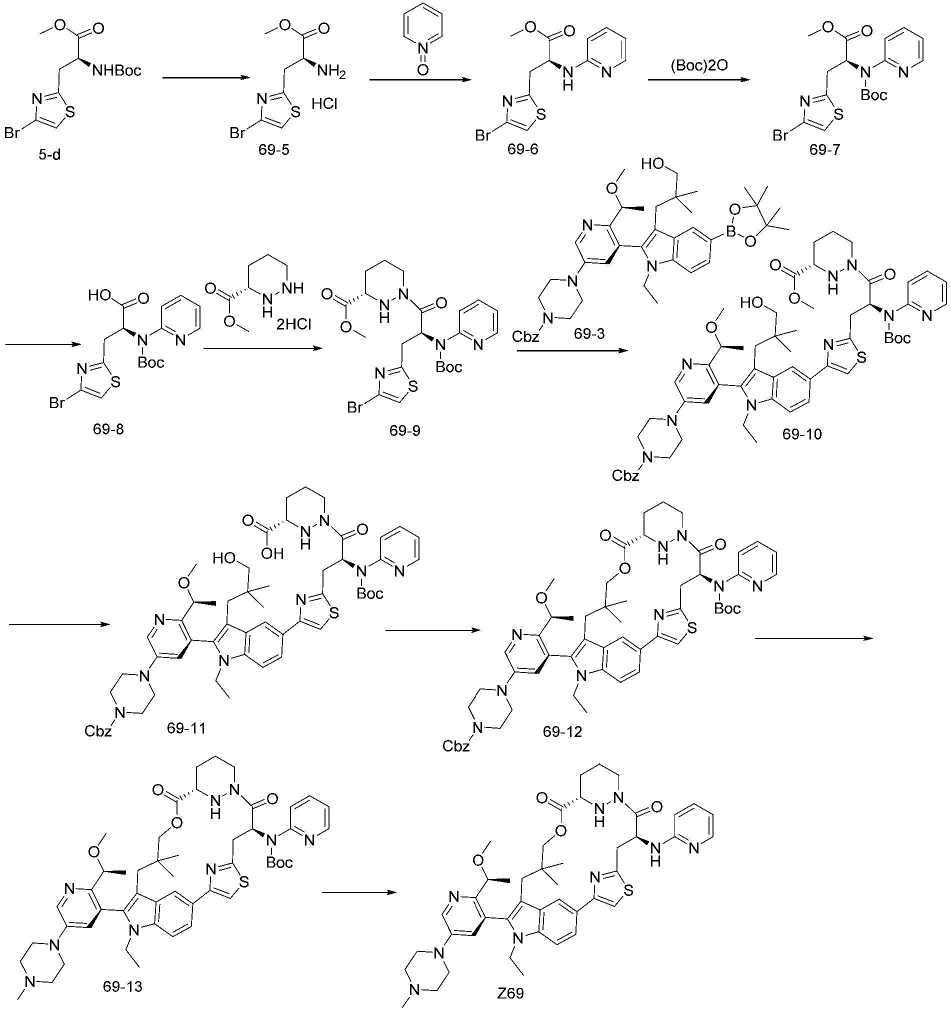

Step 1: Compound 69-1 (2.06 g, 2.28 mmol, see intermediate 5 in WO2022060836A1 for the preparation method) was dissolved in N,N-dimethylformamide (20 mL), and cesium fluoride (2.78 g, 18.27 mmol) was added at room temperature. The reaction system was stirred at 60 °C for 48 h. Ethyl acetate (120 mL) was added to the reaction mixture, and the mixture was washed sequentially with water (50 mL) and saturated brine (50 mL × 3). The organic phase was dried over anhydrous sodium sulfate and filtered, and the filtrate was concentrated under reduced pressure. The crude product was purified by a flash silica gel column (tetrahydrofuran/petroleum ether: 0-50%) to give two isomers. The structure of one of the isomers was arbitrarily designated as compound 69-2-1 (isomer 1, 700 mg, yield: 46.2%, Rf = 0.60) as a yellow solid. ES-API: [M+H]⁺ = 663.0, 665.0. The structure of the other isomer was arbitrarily designated as compound 69-2-2 (isomer 2, 500 mg, yield: 33.0%, Rf = 0.50) as a pale yellow solid. ES-API: [M+H]⁺ = 663.0, 665.0.

Step 2: Compound 69-2-1 (isomer 1, 700 mg, 1.06 mmol) was dissolved in toluene (25 mL), and bis(pinacolato)diboron (402 mg, 1.58 mmol), [1,1'-bis(diphenylphosphino)ferrocene]palladium(II) dichloride (77 mg, 0.11 mmol), and potassium acetate (310 mg, 3.16 mmol) were added. The reaction system was purged three times with nitrogen and reacted at 90 °C for 5 h. The reaction mixture was filtered through celite, and the filtrate was concentrated. The crude product was purified by a flash silica gel column (ethyl acetate/petroleum ether: 0-100%) to give the desired product of compound 69-3 (530 mg, yield: 70.7%) as a yellow solid. ES-API: [M+H]⁺ = 711.2.

Step 3: Compound 5-d (1 g, 2.74 mmol) was added to a 4.0 M hydrogen chloride/dioxane solution (20 mL) at 0 °C. The reaction system was stirred at room temperature for 3 h. The reaction mixture was concentrated to give compound 69-5 (825 mg, yield: 100%) as a white solid. ES-API: [M+H]⁺ = 264.9, 266.9.

Step 4: Compound 69-5 (700 mg, 2.32 mmol) and pyridine-N-oxide (662 mg, 6.96 mmol) were dissolved in dichloromethane (30 mL), and diisopropylethylamine (1.50 g, 11.61 mmol), bromotripyrrolidinophosphonium hexafluorophosphate (2.38 g, 5.11 mmol), and 10% palladium on carbon (150 mg) were added. The reaction system was stirred in a sealed tube at 45 °C for 18 h. Dichloromethane (30 mL) was added to the reaction mixture. The mixture was washed with water (15 mL) and a saturated sodium bicarbonate solution (15 mL), dried over anhydrous sodium sulfate, filtered, and concentrated. The crude product was purified by a flash silica gel column (tetrahydrofuran/petroleum ether: 0-30%) to give compound 69-6 (350 mg, yield: 44.1%) as an orange liquid. ¹H NMR (400 MHz, DMSO-d₆) δ 7.94 (dd, *J =* 5.2, 1.2 Hz, 1H), 7.68 (s, 1H), 7.44 - 7.36 (m, 1H), 7.13 (d, *J = 8.4* Hz, 1H), 6.62 - 6.51 (m, 2H), 4.93 - 4.83 (m, 1H), 3.61 (s, 3H), 3.54 - 3.38 (m, 2H). ES-API: [M+H]⁺ = 342.1, 344.0.

Step 5: Compound 69-6 (1.1 g, 3.21 mmol) was dissolved in tetrahydrofuran (30 mL), and di-tert-butyl dicarbonate (2.81 g, 12.86 mmol) and 4-dimethylaminopyridine (390 mg, 3.21 mmol) were added. The reaction system was stirred at 60 °C for 18 h. The reaction mixture was concentrated, and the crude product was purified by a flash silica gel column (tetrahydrofuran/petroleum ether: 0-15%) to give compound 69-7 (800 mg, yield: 56.3%) as a brown liquid. ES-API: [M+Na]⁺ = 464.0, 466.0.

Step 6: Compound 69-7 (780 mg, 1.76 mmol) was dissolved in tetrahydrofuran (20 mL) and water (5 mL), and lithium hydroxide (211 mg, 8.82 mmol) was added at 0 °C. The reaction system was stirred at room temperature for 18 h. The reaction mixture was adjusted to pH 5 with 1 M diluted hydrochloric acid and extracted with ethyl acetate (60 mL). The organic phase was washed with saturated brine (25 mL), dried over anhydrous sodium sulfate, filtered, and concentrated to give compound 69-8 (700 mg, yield: 92.7%) as a yellow solid. ES-API: [M+H]⁺ = 427.9, 429.9.

Step 7: Compound 69-8 (680 mg, 1.59 mmol) and (3S)-1,2-diazinane-3-carboxylic acid dihydrochloride (689 mg, 3.18 mmol) were dissolved in acetonitrile (40 mL), and N-methylimidazole (782 mg, 9.53 mmol) and N,N,N',N'-tetramethylchloroformamidinium hexafluorophosphate (552 mg, 1.97 mmol) were added at room temperature. The reaction system was stirred at room temperature for 2 h. Ethyl acetate (120 mL) was added to the reaction mixture, and the mixture was washed sequentially with water (50 mL × 2) and saturated brine (50 mL), dried over anhydrous sodium sulfate, filtered, and concentrated. The crude product was purified by a flash silica gel column (tetrahydrofuran/petroleum ether: 0-40%) to give compound 69-9 (850 mg, yield: 96.6%) as a yellow viscous liquid. ES-API: [M+H-100]⁺ = 454.0, 456.0.

Step 8: Compound 69-3 (250 mg, 0.35 mmol) and compound 69-9 (234 mg, 0.42 mmol) were dissolved in toluene (6 mL), dioxane (2 mL), and water (2 mL), and potassium phosphate (187 mg, 0.88 mmol) and [1,1'-bis(di-tert-butylphosphino)ferrocene]dichloropalladium(II) (23 mg, 0.035 mmol) were added. The reaction system was purged 3 times with nitrogen and stirred at 70 °C for 5 h. Ethyl acetate (40 mL) and water (5 mL) were added to the reaction mixture. The separated organic phase was washed with saturated brine (10 mL), dried over anhydrous sodium sulfate, and filtered, and the filtrate was concentrated under reduced pressure. The crude product was purified by a flash silica gel column (tetrahydrofuran/petroleum ether: 0-50%) to give compound 69-10 (300 mg, yield: 80.6%) as a pale yellow solid. ES-API: [M+H]⁺ = 1058.3.

Step 9: Compound 69-10 (270 mg, 0.26 mmol) was dissolved in tetrahydrofuran (10 mL) and water (2.2 mL), and lithium hydroxide (18 mg, 0.76 mmol) was added at 0 °C. The reaction system was stirred at room temperature for 2 h. The reaction mixture was adjusted to pH 6 with 1 M diluted hydrochloric acid and extracted with ethyl acetate (30 mL × 2). The organic phase was dried over anhydrous sodium sulfate, filtered, and concentrated to give compound 69-11 (260 mg, yield: 97.6%) as a yellow solid. ES-API: [M+H]⁺ = 1044.3.

Step 10: Compound 69-11 (260 mg, 0.23 mmol), 1-hydroxybenzotriazole (168 mg, 1.24 mmol), and diisopropylethylamine (1.03 g, 7.97 mmol) were dissolved in dichloromethane (80 mL), and 1-(3-dimethylaminopropyl)-3-ethylcarbodiimide hydrochloride (1.43 g, 7.47 mmol) was added at room temperature. The reaction system was stirred at this temperature for 18 h. The reaction mixture was washed with water (30 mL × 2) and saturated brine (30 mL), dried over anhydrous sodium sulfate, and filtered, and the filtrate was concentrated under reduced pressure. The crude product was purified by a flash silica gel column (tetrahydrofuran/petroleum ether: 0-60%) to give another crude product (300 mg), which was then purified by preparative thin-layer chromatography (dichloromethane/methanol = 25:1) to give compound 69-12 (65 mg, yield: 25.4%) as a white solid. ES-API: [M+H]⁺ = 1026.3.

Step 11: Compound 69-12 (55 mg, 0.054 mmol) and paraformaldehyde (48 mg, 1.61 mmol) were dissolved in absolute methanol (10 mL), and 10% palladium hydroxide on carbon (70 mg) was added at room temperature. The reaction system was stirred at room temperature for 24 h. The reaction mixture was filtered through celite, and the filtrate was concentrated. The crude product was purified by preparative thin-layer chromatography (dichloromethane/7 M ammonia-methanol = 12:1) to give compound 69-13 (38 mg, yield: 78.3%) as a white solid. ES-API: [M+H]⁺ = 906.2.

Step 12: Compound 69-13 (25 mg, 0.028 mmol) was dissolved in ethyl acetate (1.5 mL), and a 4 M solution of hydrogen chloride in 1,4-dioxane (3 mL) was added at 0 °C. The reaction system was stirred at room temperature for 1 h. The reaction mixture was concentrated under reduced pressure, and a saturated sodium bicarbonate solution (2 mL) was added. The mixture was extracted with dichloromethane (10 mL × 2). The organic phase was dried over anhydrous sodium sulfate and filtered, and the filtrate was concentrated under reduced pressure. The crude product was purified by prep-HPLC (ammonium bicarbonate method) to give (6³S,Z)-1¹-ethyl-(Rₐ)-1²-(2-((S)-1-methoxyethyl)-5-(4-methylpiperazin-1-yl)pyridin-3-yl)-10,10-dimethyl-4-(pyridin-2-ylamino)-6¹,6 ²6³,6⁴ 6⁵6⁶-hexahydro-1¹H-8-oxa-2(4,2)-thiazola-1(5,3)-indola-6(1,3)-pyridazinacycloundecaphane-5,7-dione (Z69, 3 mg, yield: 13.5%) as a white solid. ES-API: [M+H]⁺ = 806.2.

### Example 4. Synthesis of Compound Z66

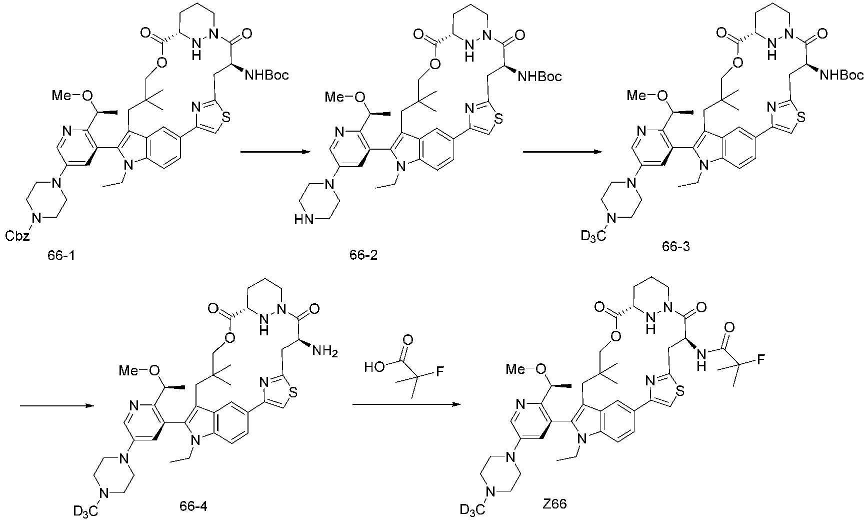

Step 1: Compound 66-1 (60 mg, 0.060 mmol, see intermediate 5 in WO2022060836A1 for the preparation method) was dissolved in methanol (5 mL), and palladium hydroxide (70 mg, 0.498 mmol) was added. The mixture was reacted overnight at room temperature under a hydrogen atmosphere, filtered under vacuum, and concentrated, and the residue was purified by silica gel column chromatography (dichloromethane/methanol = 10:1) to give compound 66-2 (39 mg, 0.045 mmol, yield: 75.14%). ES-API: [M+H]⁺ = 815.2.

Step 2: Compound 66-2 (65 mg, 0.076 mmol) was dissolved in N,N-dimethylformamide (1 mL), and potassium carbonate (20.91 mg, 0.151 mmol) and deuterated iodomethane (10.74 mg, 0.076 mmol) were added under an ice-water bath. The mixture was reacted at room temperature for 2 h. After the reaction was completed, water was added, and the mixture was extracted with ethyl acetate. The organic phase was washed with saturated brine, dried over anhydrous sodium sulfate, and purified by silica gel column chromatography (dichloromethane:methanol = 10:1) to give compound 66-3 (30 mg, 0.034 mmol, yield: 45.26%). ES-API: [M+H]⁺ = 832.3.

Step 3: Compound 66-3 (20 mg, 0.023 mmol) was dissolved in methanol (0.5 mL), and a solution of hydrochloric acid in dioxane (2 mL, 0.023 mmol) was added. The mixture was reacted at room temperature for 1 h. The reaction mixture was concentrated under reduced pressure to give compound 66-4 (16 mg, 0.021 mmol, yield: 90.34%). ES-API: [M+H]⁺ = 732.3. Step 4: Compound 66-4 (16 mg, 0.021 mmol) was dissolved in N,N-dimethylformamide (1 mL), and 2-fluoroisobutyric acid (3.26 mg, 0.031 mmol), N,N-diisopropylethylamine (15.89 mg, 0.123 mmol), and O-(7-azabenzotriazol-1-yl)-N,N,N',N'-tetramethyluronium hexafluorophosphate (23.38 mg, 0.061 mmol) were added. The mixture was reacted at room temperature for 1 h. After the reaction was completed, water (5 mL) was added, and the mixture was extracted with ethyl acetate (5 mL × 3). The organic phase was washed with saturated brine (5 mL), dried over anhydrous sodium sulfate, and purified by silica gel column chromatography (dichloromethane:methanol = 10:1) to give N-((6³S,4S,Z)-1¹-ethyl-(Rₐ)-1²-(2-((S)-1-methoxyethyl)-5-(4-(methyl-d₃)piperazin-1-yl)pyridin-3-yl)-1⁰,1⁰-dimethyl-5,7-dioxa-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-fluoro-2-methylpropanamide (Z66, 5 mg, 0.006 mmol, yield: 29.75%). ES-API: [M+H]⁺ = 820.0.

### Example 5. Synthesis of Compound Z70

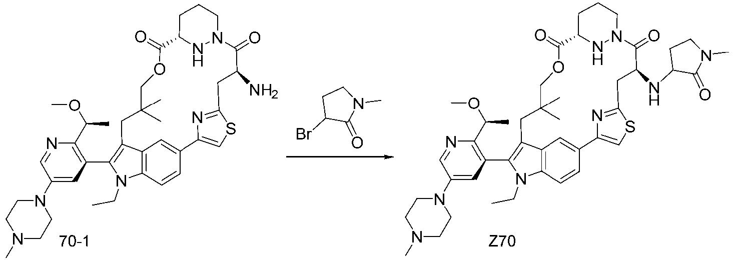

Step 1: Compound 70-1 (80 mg, 0.11 mmol, see A277 in WO2021091956A1 for the preparation method) was dissolved in acetonitrile (8 mL), and potassium carbonate (45 mg, 0.33 mmol) and 3-bromo-1-methylpyrrolidin-2-one (59 mg, 0.33 mmol) were added. The mixture was reacted with stirring in a microwave reactor at 85 °C for 64 h. Ethyl acetate (40 mL) and water (10 mL) were added to the reaction mixture. The separated organic phase was washed with saturated brine (10 mL), dried over anhydrous sodium sulfate, and filtered, and the filtrate was concentrated under reduced pressure. The crude product was subjected to prep-HPLC (formic acid method 1) to give the desired product ((6³S,4S,Z)-1¹-ethyl-(Rₐ)-1²-(2-((S)-1-methoxyethyl)-5-(4-methylpiperazin-1-yl)pyridin-3-yl)-10,10-dimethyl-4-((1-methyl-2-oxopyrrolidin-3-yl)amino)-6¹,6²,63,6⁴,6⁵,6⁶₋hexahydro-1¹H-8-oxa-2(4,2)-thiazola-1(5,3)-indola-6(1,3)-pyridazinacycloundecaphane-5,7-dione formate (Z70, 16 mg, yield: 15.8%) as a white solid. ES-API: [M+H]⁺ = 826.3.

### Example 6. Synthesis of Compound Z71

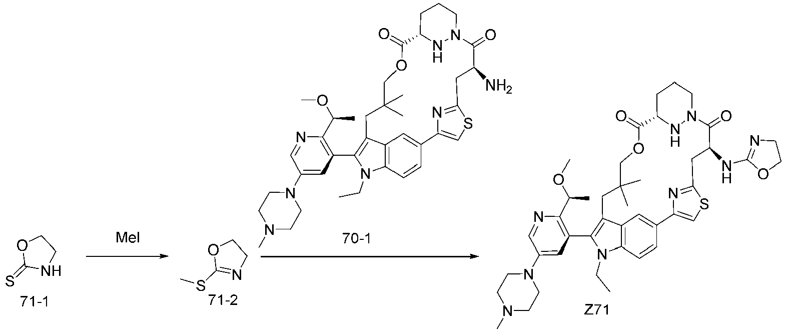

Step 1: Compound 71-1 (500 mg, 4.85 mmol) was dissolved in N,N-dimethylformamide (8 mL), and anhydrous potassium carbonate (1.0 g, 7.27 mmol) and iodomethane (1.72 g, 12.12 mmol) were sequentially added at 0 °C. The reaction system was stirred at room temperature for 24 h. The reaction mixture was poured into water (20 mL) and extracted with ethyl acetate (30 mL × 2). The organic phase was washed with saturated brine (20 mL × 2), dried over anhydrous sodium sulfate, filtered, and concentrated to give crude compound 71-2 (500 mg, yield: 90%) as a colorless liquid. ES-API: [M+H]⁺ = 118.0.

Step 2: Compound 70-1 (100 mg, 0.14 mmol) was dissolved in ethanol (3.5 mL), and N,N-diisopropylethylamine (89 mg, 0.69 mmol) and compound 71-2 (64 mg, 0.55 mmol) were added. The reaction system was stirred in a microwave reactor at 90 °C for 2 h. The reaction mixture was concentrated under reduced pressure. The residue was purified by preparative thin-layer chromatography (dichloromethane/7 M ammonia-methanol solution = 20:1) to give a crude product, which was then purified by prep-HPLC (formic acid method 1) to give (6³S,4S,Z)-1¹-ethyl-(Rₐ)-1²-(2-((S)-1-methoxyethyl)-5-(4-methylpiperazin-1-yl)pyridin-3-yl)-10,10-dimethyl-4-((4,5-dihydrooxazol-2-yl)amino)-6¹,6²,6³,6⁴ 6⁵ 6⁶-hexahydro-1¹H-8-oxa-2(4,2)-thiazola-1(5,3)-indola-6(1,3)-pyridazinacycloundecaphane-5,7-dione formate (Z71, 9 mg, yield: 7.8%) as a white solid. ES-API: [M+H]⁺ = 798.3.

### Example 7. Synthesis of Compounds Z72, Z73, and Z74

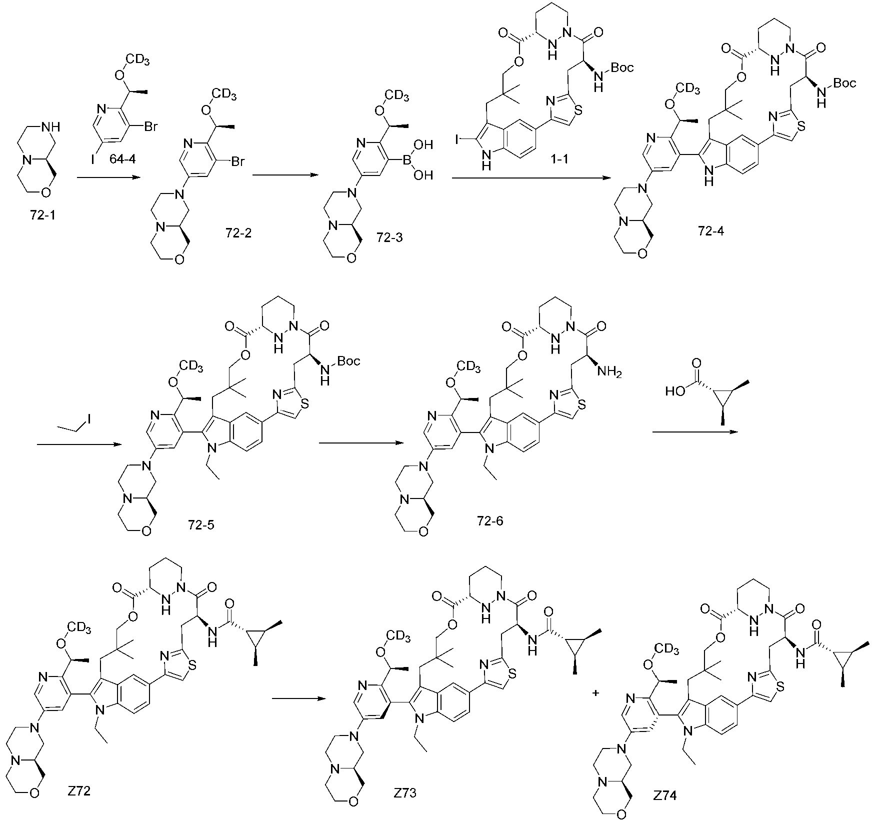

Step 1: Compound 72-1 (250 mg, 1.758 mmol), compound 64-4 (607 mg, 1.814 mmol), cesium carbonate (1432 mg, 4.395 mmol), palladium acetate (28 mg, 0.123 mmol), and 2,2'-bis(diphenylphosphino)-1,1'-binaphthyl (33 mg, 0.053 mmol) were dissolved in anhydrous toluene (20 mL) under a nitrogen atmosphere, and the reaction mixture was stirred under an oil bath at 100 °C overnight. When the reaction was completed as detected by LCMS, the reaction mixture was cooled to room temperature and concentrated under reduced pressure to remove the solvent. The crude product was purified by automated flash chromatography on silica gel (methanol:dichloromethane = 0-20%) to give compound 72-2 (581 mg, yield: 92%). ES-API: [M+H]⁺ = 359.0.

Step 2: Compound 72-2 (270 mg, 0.752 mmol), bis(pinacolato)diboron (286 mg, 1.128 mmol), [1,1'-bis(diphenylphosphino)ferrocene]palladium(II) dichloride dichloromethane complex (61 mg, 0.075 mmol), and potassium acetate (185 mg, 1.880 mmol) were dissolved in a solution of anhydrous dioxane (15 mL) under a nitrogen atmosphere. The reaction mixture was heated to 100 °C under an oil bath and stirred overnight. When the reaction was completed as detected by LCMS, the mixture was cooled to room temperature. Water (30 mL) was added to the reaction mixture, and the mixture was extracted with ethyl acetate (15 mL × 3). The aqueous phase was then extracted with a mixed solvent (dichloromethane/methanol, v/v = 10:1, 15 mL × 8). The organic phases were combined, dried over anhydrous sodium sulfate, filtered, and concentrated to dryness under reduced pressure to give crude compound 72-3 (242 mg, yield: 99%). ES-API: [M+H]⁺ = 324.4.

Step 3: Compound 72-3 (93 mg, 0.288 mmol), compound 1-1 (100 mg, 0.144 mmol), potassium phosphate (92 mg, 0.433 mmol), and [1,1'-bis(di-tert-butylphosphino)ferrocene]dichloropalladium(II) (19 mg, 0.029 mmol) were dissolved in dioxane (9 mL), toluene (3 mL), and water (3 mL) under a nitrogen atmosphere. The reaction mixture was heated to 60 °C under an oil bath and stirred overnight. When the reaction was completed as detected by LCMS, the mixture was cooled to room temperature. Ethyl acetate (30 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (methanol:dichloromethane = 0-2%) to give compound 72-4 (105 mg, yield: 74%). ES-API: [M+H]⁺ = 846.3.

Step 4: Cesium carbonate (308 mg, 0.944 mmol) and iodoethane (0.3 mL, 3.540 mmol) were sequentially added to a solution of compound 72-4 (100 mg, 0.118 mmol) in N,N-dimethylformamide (10 mL), and the reaction mixture was stirred at room temperature overnight. When the reaction was completed as detected by LCMS, the reaction mixture was diluted with ethyl acetate (20 mL) and then washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran:petroleum ether = 0-80%) to give compound 72-5 (102 mg, yield: 98%). ES-API: [M+H]⁺ = 874.3.

Step 5: Compound 72-5 (102 mg, 0.117 mmol) was dissolved in a solution of hydrochloric acid in dioxane (3 mL, 4 M) and a solution of hydrochloric acid in methanol (1 mL, 4 M). The reaction mixture was stirred at room temperature for 1 h. When the reaction was completed as detected by LCMS, the reaction mixture was concentrated under reduced pressure to give crude compound 72-6 (90 mg, yield: 99%). ES-API: [M+H]⁺ = 774.3.

Step 6: (1r,2R,3S)-2,3-Dimethylcyclopropane-1-carboxylic acid (20 mg, 0.174 mmol), N,N-diisopropylethylamine (90 mg, 0.698 mmol), and O-(7-azabenzotriazol-1-yl)-N,N,N',N'-tetramethyluronium hexafluorophosphate (133 mg, 0.349 mmol) were sequentially added to a solution of compound 72-6 (90 mg, 0.116 mmol) in N,N-dimethylformamide (3 mL) under an ice-water bath. The reaction mixture was warmed to room temperature and stirred for 10 min. When the reaction was completed as detected by LCMS, the reaction mixture was diluted with ethyl acetate (20 mL) and then washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give a mixture (1r,2R,3S)-N-((6³S,4S,Z)-1¹-ethyl-1²-(5-((S)-hexahydropyrazino[2,1-c][1,4]oxazin-8(1H)-yl)-2-((S-1-(methoxy-d₃)ethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z72, 46 mg, yield: 46%). ES-API: [M+H]⁺ = 870.4.

Step 7: Mixture Z72 (46 mg) was resolved by a chiral column (chromatographic column: CHIRALPAK^{®} IB 250 × 4.6 mm, 5 µm; mobile phase A: HEX + 0.2% DEA, mobile phase B: ETOH + 0.2% DEA; detection wavelength: 254 nm; flow rate: 1 mL/min; injection volume: 5 µL; column temperature: 30 °C; run time: 15 min; isocratic elution program: mobile phase A:mobile phase B = 50:50 (V/V)) to give two isomeric compounds. One of the structures was arbitrarily designated as (1r,2R,3S)-N-((6³S,4S,Z)-1¹-ethyl-(Rₐ)-1²-(5-((S)-hexahydropyrazino[2,1-c][1,4]oxazin-8(1H)-yl)-2-((S)-1-(methoxy-d₃)ethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z73, 11.24 mg, retention time: 10.203 min), ES-API [M+H]⁺ = 870.4.

The other structure was arbitrarily designated as (1r,2R,3S)-N-((6³S,4S,Z)-1¹-ethyl-(Sₐ)-1²-(5-((S)-hexahydropyrazino[2,1-c][1,4]oxazin-8(1H)-yl)-2-((S)-1-(methoxy-d₃)ethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z74, 25.11 mg, retention time: 5.541 min), ES-API [M+H]⁺ = 870.4.

### Example 8. Synthesis of Compounds Z63, Z64, and Z65

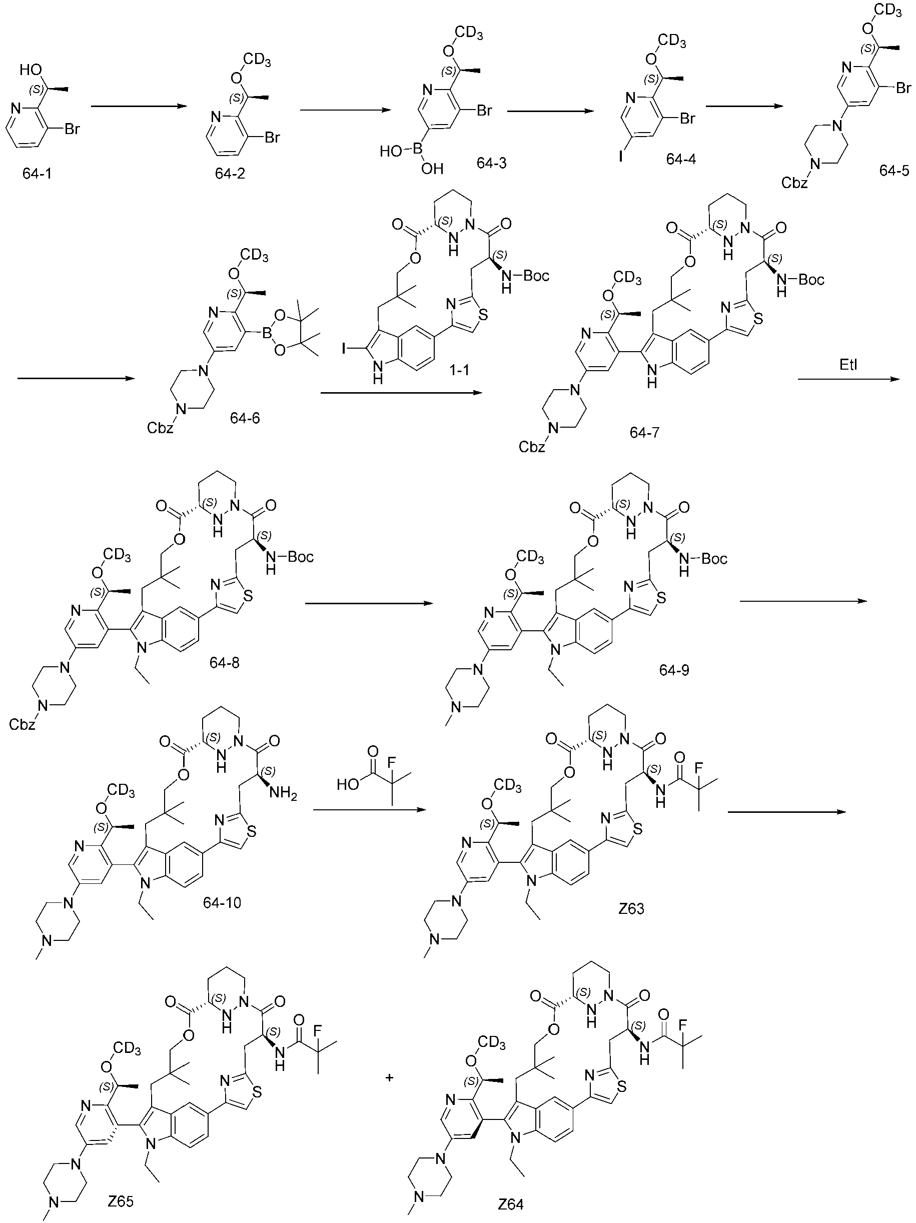

Step 1: Compound 64-1 (7.1 g, 35.14 mmol) was dissolved in N,N-dimethylformamide (100 mL), and sodium hydride (2.11 g, 52.7 mmol) was added in portions at 0 °C. The mixture was stirred for 30 min. Deuterated iodomethane (10.2 g, 70.2 mmol) was added, and the resulting mixture was stirred at room temperature for 1 h. After the reaction was completed, the mixture was added to ice water (100 mL) and extracted with ethyl acetate (100 mL × 2). The organic phase was washed with saturated brine (100 mL) and water (100 mL) and concentrated, and the residue was purified by a flash silica gel column (0-6% methanol/dichloromethane) to give compound 64-2 (6.3 g, yield: 81.8%). ES-API [M+H]⁺ = 219.1, 221.1.

Step 2: Compound 64-2 (2.4 g, 10.9 mmol) was dissolved in tetrahydrofuran (50 mL), and bis(pinacolato)diboron (4.17 g, 16.4 mmol), 4,4-di-tert-butylbipyridine (294 mg, 1.1 mmol), and chloro(1,5-cyclooctadiene)iridium(I) dimer (294 mg, 0.44 mmol) were sequentially added. The mixture was stirred at 80 °C overnight under a nitrogen atmosphere. After the reaction was completed, the mixture was added to water (30 mL). The aqueous phase was adjusted to pH 10 with a sodium hydroxide solution, and the mixture was extracted with ethyl acetate (30 mL × 2). The aqueous phase was adjusted to pH 6 with a 4 M hydrochloric acid solution, and a solid was precipitated and filtered to give compound 64-3 (2 g, yield: 69.5%). ES-API [M+H]⁺ = 262.9, 264.9.

Step 3: Compound 64-3 (5 g, 19 mmol) was dissolved in acetonitrile (100 mL), and N-iodosuccinimide (6.58 g, 38.0 mmol) was added at room temperature. The mixture was stirred at 80 °C overnight. After the reaction was completed, the mixture was added to water (100 mL) and extracted with ethyl acetate (100 mL × 2). The organic phase was washed with saturated brine (100 mL) and water (100 mL) and concentrated, and the residue was purified by a flash silica gel column (0-60% ethyl acetate/petroleum ether) to give compound 64-4 (4.1 g, yield: 62.5%). ES-API [M+H]⁺ = 344.8, 346.8.

Step 4: Compound 64-4 (3 g, 8.7 mmol) was dissolved in toluene (50 mL), and benzyl 1-piperazinecarboxylate (1.84 g, 8.3 mmol), palladium acetate (140 mg, 0.6 mmol), 2,2'-bis(diphenylphosphino)-1,1'-binaphthyl (160 mg, 0.26 mmol), and cesium carbonate (7.15 g, 21.9 mmol) were sequentially added. The mixture was stirred at 100 °C overnight under a nitrogen atmosphere. After the reaction was completed, the mixture was added to water (50 mL) and extracted with ethyl acetate (50 mL × 2). The organic phase was washed with saturated brine (50 mL) and water (50 mL) and concentrated, and the residue was purified by a flash silica gel column (0-60% ethyl acetate/petroleum ether) to give compound 64-5 (2 g, yield: 52.5%). ES-API [M+H]⁺ = 437.0, 439.0.

Step 5: Compound 64-5 (0.5 g, 1.14 mmol) was dissolved in toluene (10 mL), and bis(pinacolato)diboron (319 mg, 1.26 mmol), [1,1'-bis(diphenylphosphino)ferrocene]palladium(II) dichloride (83 mg, 0.11 mmol), and potassium acetate (224 mg, 2.29 mmol) were sequentially added. The mixture was stirred at 100 °C overnight under a nitrogen atmosphere. After the reaction was completed, the mixture was added to water (50 mL) and extracted with ethyl acetate (30 mL × 2). The organic phase was washed with saturated brine (30 mL) and water (30 mL) and concentrated, and the residue was purified by a flash silica gel column (0-60% ethyl acetate/petroleum ether) to give compound 64-6 (225 mg, yield: 40.6%). ES-API [M+H]⁺ = 485.2.

Step 6: A mixture of compound 1-1 (100 mg, 0.14 mmol), compound 64-6 (90 mg, 0.18 mmol), [1,1'-bis(di-tert-butylphosphino)ferrocene]dichloropalladium(II) (18.6 mg, 0.03 mmol), and potassium phosphate (91.8 mg, 0.43 mmol) was stirred at 60 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 64-7 (75 mg, yield: 58%) as a transparent oil. ES-API [M+H]⁺ = 924.4.

Step 7: Compound 64-7 (70 mg, 0.076 mmol) was dissolved in N,N-dimethylformamide (1 mL), and cesium carbonate (123 mg, 0.38 mmol) and iodoethane (118 mg, 0.76 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 64-8 (70 mg, yield: 97%). ES-API [M+H]⁺ = 952.3.

Step 8: Compound 64-8 (70 mg, 0.074 mmol) was dissolved in methanol (10 mL), and paraformaldehyde (60 mg, 2 mmol) and palladium hydroxide on carbon (102 mg, 0.07 mmol) were sequentially added. The mixture was stirred at room temperature for 16 h. After the reaction was completed, the mixture was filtered and concentrated, and the residue was purified by a flash silica gel column (0-60% tetrahydrofuran/petroleum ether) to give compound 64-9 (40 mg, yield: 65%). ES-API [M+H]⁺ = 832.3.

Step 9: Compound 64-9 (40 mg) was dissolved in a solution of hydrochloric acid in dioxane (5 mL, 4 mol/L). The mixture was stirred at room temperature for 2 h. After the reaction was completed, the mixture was concentrated, and the residue was basified with a saturated sodium bicarbonate solution until the pH was 8, extracted with ethyl acetate (20 mL × 3), and concentrated to give compound 64-10 (35 mg, yield: 99%). ES-API [M+H]⁺ = 732.2.

Step 10: Compound 64-10 (35 mg) was dissolved in N,N-dimethylformamide (3 mL), and 2-fluoro-2-methylpropionic acid (7.6 mg, 0.07 mmol), N,N-diisopropylethylamine (37 mg, 0.29 mmol), and O-(7-azabenzotriazol-1-yl)-N,N,N',N'-tetramethyluronium hexafluorophosphate (54 mg, 0.14 mmol) were sequentially added. The mixture was stirred at room temperature for 1 h. After the reaction was completed, ethyl acetate (20 mL) was added, and the mixture was washed with saturated brine (10 mL) and water (10 mL). The organic phase was dried over anhydrous sodium sulfate, filtered, and concentrated, and the residue was then purified by prep-HPLC (ammonium bicarbonate method) to give compound Z63 (28 mg, yield: 71%, purity: 98%). ES-API [M+H]⁺ = 820.4.

Step 11: Compound Z63 (28 mg) was resolved by a chiral column (chromatographic column: IB column, 250 mm × 4.6 mm × 5 µm, mobile phase: n-hexane:EtOH:DEA = 60:40:2, flow rate: 1 mL/min, column temperature: 30 °C) to give two isomeric compounds. One of the structures was arbitrarily designated as N-((1²S,6³S,4S,Z)-1¹-ethyl-(Sₐ)-1²-(2-((S)-1-(methoxy-d3)ethyl)-5-(4-methylpiperazin-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-fluoro-2-methylpropanamide (Z65, 14 mg, yield: 50%, purity: 98%, retention time: 4.556 min). ES-API [M+H]⁺ = 820.4.

The other structure was arbitrarily designated as N-((1²R,6³S,4S,Z)-1'-ethyl-(Rₐ)-1²-(2-((S)-1-(methoxy-d3)ethyl)-5-(4-methylpiperazin-1-yl)pyridin-3-yl)-10,10-dimethyl-5,7-dioxo-6¹,6²6³6⁴6⁵6⁶-hexahydro-1¹H-8-oxa-2(4,2)-thiazola-1(5,3)-indola-6(1,3)-pyridazinacycloundecaphane-4-yl)-2-fluoro-2-methylpropanamide (Z64, 7 mg, yield: 20%, purity: 98%, retention time: 6.670 min). ES-API [M+H]⁺ = 820.4.

### Example 9. Synthesis of Compound Z75

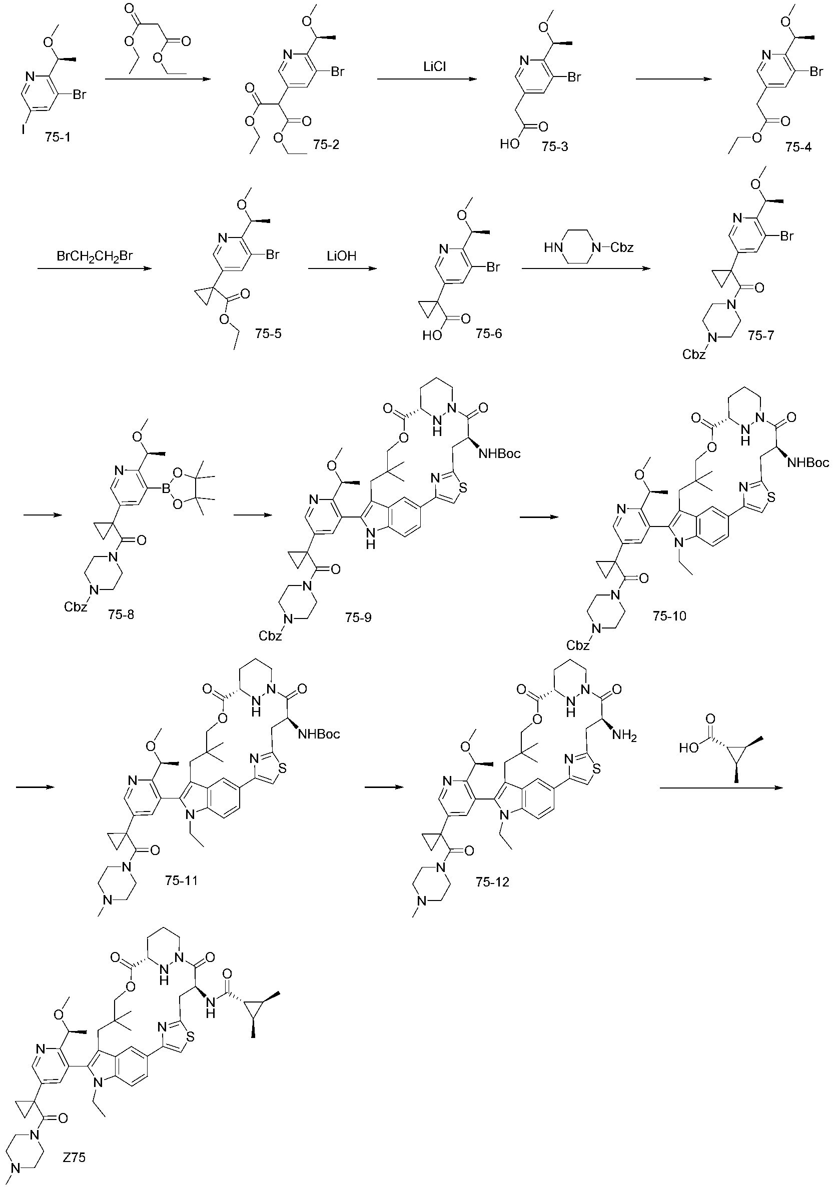

Step 1: Compound 75-1 (10 g, 29.242 mmol), copper(I) iodide (1.39 g, 4.386 mmol), and cesium carbonate (28.58 g, 87.727 mmol) were dissolved in dioxane (250 mL), and pyridinedicarboxylic acid (1.08 g, 8.773 mmol) and diethyl malonate (7.73 g, 58.485 mmol) were added to the solution described above. After the addition was completed, the reaction mixture was purged three times with nitrogen. The reaction system was stirred at 80 °C for 17 h. After the reaction was completed, the reaction mixture was poured into a saturated aqueous ammonium chloride solution (200 mL) and then extracted with ethyl acetate (200 mL × 2). The organic phase was washed with saturated brine (200 mL), dried over anhydrous sodium sulfate, filtered, and concentrated to give a crude product. The crude product was purified by a flash silica gel column (ethyl acetate/petroleum ether: 0%-20%) to give compound 75-2 (10 g, 26.722 mmol, yield: 91.38%) as a colorless transparent liquid. ES-API: [M+H]⁺ = 374.1, 376.0.

Step 2: Compound 75-2 (10 g, 26.722 mmol) was dissolved in dimethyl sulfoxide (100 mL). Lithium chloride (11.33 g, 267.215 mmol) and water (20 mL) were added. The reaction system was stirred at 100 °C for 16 h and then reacted with stirring at 120 °C for 48 h. After being cooled to room temperature, the reaction mixture was acidified until the pH was about 3 with diluted hydrochloric acid (2 M). The solution described above was extracted with ethyl acetate (100 mL × 3). The organic phases were combined, washed with saturated brine (300 mL × 3), dried over anhydrous sodium sulfate, filtered, and concentrated to give compound 75-3 (6500 mg, 23.713 mmol, yield: 88.74%) as a colorless transparent liquid. ES-API: [M+H]⁺ = 274.1, 276.0.

Step 3: Compound 75-3 (6500 mg, 23.713 mmol) was dissolved in ethanol (60 mL), and the mixture was cooled to about 0-5 °C. Thionyl chloride (3385 mg, 2.064 mL, 28.456 mmol) was slowly added dropwise to the solution described above for 2 min. After the dropwise addition was completed, the reaction mixture was warmed to 80 °C and stirred for 1 h. The reaction mixture was concentrated to remove ethanol and thionyl chloride. An aqueous sodium carbonate solution (2 M, 30 mL) was added to the residue, and the mixture was extracted with ethyl acetate (40 mL × 3). The organic phases were combined, washed with saturated brine (100 mL), dried over anhydrous sodium sulfate, filtered, and concentrated to give a crude product. The crude product was purified by a flash silica gel column (ethyl acetate/petroleum ether: 0%-20%) to give compound 75-4 (6500 mg, 21.511 mmol, yield: 90.71%) as a colorless transparent liquid. ES-API: [M+H]⁺ = 302, 304.0.

Step 4: Compound 75-4 (5.5 g, 18.202 mmol) and N,N-dimethylformamide (80 mL) were added to a three-necked flask, and the system was cooled to about 0-5 °C. Sodium hydride (2.18 g, 60 wt%, 54.605 mmol) was added to the stirred solution described above, and the mixture was stirred for 15 min. Then, 1,2-dibromoethane (5.81 g, 30.943 mmol) was added to the solution described above at about 0-5 °C. After the addition was completed, the reaction system was warmed to room temperature and stirred for 1 h. After the reaction was completed, the reaction was quenched by adding saturated ammonium chloride (100 mL) to the reaction mixture, and the reaction mixture was extracted with ethyl acetate (100 mL × 3). The organic phase was washed with saturated brine (300 mL), dried over anhydrous sodium sulfate, filtered, and concentrated to give a crude product. The crude product was purified by a flash silica gel column (ethyl acetate/petroleum ether: 0%-30%) to give compound 75-5 (5 g, yield: 83.70%) as a colorless transparent liquid. ES-API: [M+H]⁺ = 328.0, 330.1. ¹H NMR (400 MHz, CDCl₃) δ 8.51 (d, J = 2.0 Hz, 1H), 7.76 (d, J = 2.0 Hz, 1H), 4.85 (q, J = 6.4 Hz, 1H), 4.05 (q, J = 7.2 Hz, 2H), 3.26 (s, 3H), 1.61 (q, J = 4.2 Hz, 2H), 1.43 (d, J = 6.4 Hz, 3H), 1.16-1.10 (m, 5H).

Step 5: Compound 75-5 (5 g, 15.234 mmol) was dissolved in tetrahydrofuran (15 mL), methanol (15 mL), and water (15 mL), and lithium hydroxide (3196 mg, 76.171 mmol) was added to the solution described above. The reaction system was stirred at room temperature for 17 h. After the reaction was completed, the reaction mixture was adjusted to about pH 3 with diluted hydrochloric acid (6 M). The resulting solution was extracted with ethyl acetate (50 mL × 3), and the organic phases were combined, dried over anhydrous sodium sulfate, filtered, and concentrated to give compound 75-6 (4500 mg, 14.993 mmol, yield: 98.41 %). ES-API: [M+H]⁺ = 300.0, 302.0.

Step 6: Compound 75-6 (500 mg, 1.666 mmol) was dissolved in dichloromethane (10 mL). Benzyl piperazine-1-carboxylate (385.28 mg, 1.749 mmol), O-(7-azabenzotriazol-1-yl)-N,N,N',N'-tetramethyluronium hexafluorophosphate (950.12 mg, 2.499 mmol), and N,N'-diisopropylethylamine (644 mg, 4.998 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0-30%) to give compound 75-7 (700 mg, 1.393 mmol, yield: 83.64%) as a yellow oil. ES-API: [M+H]⁺ = 502.1, 504.1.

Step 7: Compound 75-7 (300 mg, 0.597 mmol) was dissolved in anhydrous toluene (5 mL), and bis(pinacolato)diboron (227.45 mg, 0.896 mmol), [1,1'-bis(diphenylphosphino)ferrocene]palladium(II) dichloride (43.69 mg, 0.060 mmol), and potassium acetate (117.20 mg, 1.194 mmol) were added to the solution described above at room temperature. After the addition was completed, the reaction system was stirred at 90 °C for 17 h under a nitrogen atmosphere. After the reaction was completed, the reaction mixture was filtered using a sand core funnel and concentrated. The residue was purified by a flash column (aluminum oxide, ethyl acetate/petroleum ether: 0-100%) to give compound 75-8 (250 mg, 0.455 mmol, yield: 76.19%) as a black oily liquid. ES-API: [M+H]⁺ = 550.1.

Step 8: Compound 75-8 (154.5 mg, 0.281 mmol) was dissolved in dioxane (9 mL), toluene (3 mL), and water (3 mL). Compound 1-1 (150 mg, 0.216 mmol), [1,1'-bis(di-tert-butylphosphino)ferrocene]dichloropalladium(II) (27.93 mg, 0.043 mmol), and potassium phosphate (137.71 mg, 0.649 mmol) were added to the solution described above at room temperature. After the addition was completed, the reaction system was stirred at 60 °C for 16 h under an argon atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 75-9 (150 mg, 0.152 mmol, yield: 70.12%) as a yellow solid. ES-API: [M+H]⁺ = 989.1.

Step 9: Compound 75-9 (150 mg, 0.152 mmol) was dissolved in N,N-dimethylformamide (5 mL). Iodoethane (236.51 mg, 1.516 mmol) and cesium carbonate (247.03 mg, 0.758 mmol) were added to the solution described above. The reaction system was stirred at room temperature for 8 h. After the reaction was completed, water (30 mL) was added to the solution described above, and the mixture was extracted with ethyl acetate (20 mL × 2). The organic phase was washed with saturated brine (30 mL × 3), dried over anhydrous sodium sulfate, filtered, and concentrated to give a crude product. The crude product was purified by a flash silica gel column (methanol/dichloromethane: 0%-3%) to give compound 75-10 (130 mg, 0.128 mmol, yield: 84.28%) as a yellow solid. ES-API: [M+H]⁺ = 1017.5.

Step 10: Compound 75-10 (100 mg, 0.098 mmol) was dissolved in methanol (5 mL), and palladium hydroxide (10 wt%, 100 mg, 0.071 mmol) and paraformaldehyde (29.49 mg, 0.983 mmol on a formaldehyde equivalent basis) were added to the solution described above. After being purged three times with hydrogen, the reaction system was reacted for 17 h under a hydrogen atmosphere (15 psi). The reaction mixture was filtered through celite and concentrated to give a crude product. The crude product was purified by a flash silica gel column (methanol/dichloromethane: 0%-4%) to give compound 75-11 (70 mg, 0.078 mmol, yield: 79.37%) as a yellow solid. ES-API: [M+H]⁺ = 897.1.

Step 11: Compound 75-11 (70 mg, 0.078 mmol) was dissolved in methanol (1 mL), and a solution (4 M) of hydrogen chloride in dioxane was added to the solution described above. The mixture was stirred at room temperature for 2 h. After the reaction was completed, the reaction mixture was concentrated to give crude compound 75-12 (70 mg) as a yellow solid. ES-API: [M+H]⁺ = 797.1.

Step 12: Crude compound 75-12 (70 mg) was dissolved in dichloromethane (5 mL), and (1r,2R,3S)-2,3-dimethylcyclopropane-1-carboxylic acid (15.04 mg, 0.132 mmol), O-(7-azabenzotriazol-1-yl)-N,N,N',N'-tetramethyluronium hexafluorophosphate (50.09 mg, 0.132 mmol), and N,N'-diisopropylethylamine (34 mg, 0.263 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1*r*,2*R*,3*S*)-N-((6³*S*,4*S*,*Z*)-1¹-ethyl-1²-(2-((*S*)-1-methoxyethyl)-5-(1-(4-methylpiperazine-1-carbonyl)cyclopropyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z75, 35 mg, two-step yield: 50%) as a white solid. ES-API: [M+H]⁺ = 893.4.

### Example 10. Synthesis of Compounds Z12, Z13, and Z14

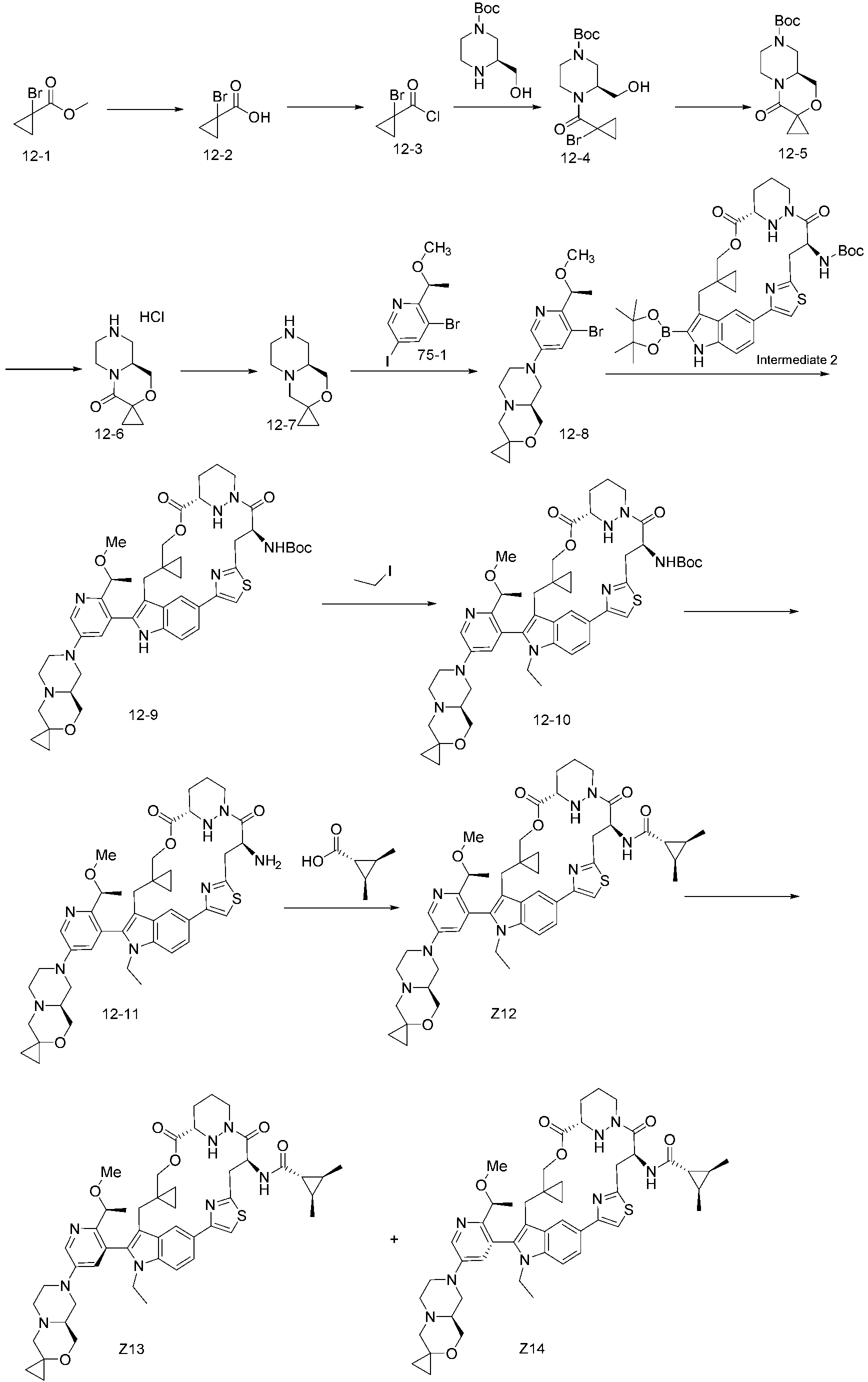

Step 1: Potassium tert-butoxide (17.16 g, 152.924 mmol) was dissolved in methyl tert-butyl ether (100 mL). The mixture was cooled to 0 °C under an ice-water bath, and distilled water (15 mL) was slowly added dropwise to the mixture. After the dropwise addition was completed, a solution of compound 12-1 (12.5 g, 69.829 mmol) in methyl tert-butyl ether (15 mL) was slowly added at 0 °C. The reaction mixture was stirred at 0-5 °C for 3 h. When the reaction was completed as detected by TLC (petroleum ether/ethyl acetate = 5:1), the reaction mixture was poured into ice water (100 mL). The aqueous phase was separated, adjusted to about pH 2.0 with diluted hydrochloric acid (6 M), and then extracted with dichloromethane/methanol (25 mL × 4, v/v = 10:1). The organic phases were combined, washed with saturated brine (20 mL), dried over anhydrous sodium sulfate, filtered, and concentrated to give crude compound 12-2 (9.035 g, yield: 78%).

Step 2: Compound 12-2 (9 g, 54.545 mmol) was dissolved in thionyl chloride (60 mL), and the reaction mixture was heated to reflux and stirred overnight. When the reaction was completed as detected by TLC (petroleum ether/ethyl acetate = 5:1), the reaction mixture was concentrated under reduced pressure to give crude compound 12-3 (7.53 g, yield: 75%).

Step 3: Triethylamine (15.5 mL, 111.51 mmol) was added to a solution of tert-butyl (S)-3-(hydroxymethyl)piperazine-1-carboxylate (8.04 g, 37.17 mmol) in dichloromethane (30 mL). The mixture was cooled to 0 °C under an ice-water bath, and compound 12-3 (7.5 g, 40.89 mmol) was slowly added dropwise to the mixture. After the dropwise addition was completed, the reaction mixture was slowly warmed to room temperature and stirred for 3 h. When the reaction was completed as detected by LCMS, the reaction mixture was poured into a saturated sodium bicarbonate solution (30 mL) and dichloromethane (30 mL). The organic phase was separated, dried over anhydrous sodium sulfate, filtered, and concentrated. The residue was purified by automated flash chromatography on silica gel (tetrahydrofuran:petroleum ether = 0-20%) to give compound 12-4 (13.385 g, yield: 99%). ES-API: [M-55]⁺ = 307.0.

Step 4: Potassium tert-butoxide (4.58 mg, 40.798 mmol) was added to a solution of compound 12-4 (9.88 g, 27.199 mmol) in tetrahydrofuran (120 mL). The reaction mixture was stirred at room temperature for 3 h. When the reaction was completed as detected by LCMS, the mixture was concentrated under reduced pressure to remove the solvent. The residue was purified by automated flash chromatography on silica gel (tetrahydrofuran:petroleum ether = 0-20%) to give compound 12-5 (712 mg, yield: 9%). ES-API: [M-55]⁺ = 227.1.

Step 5: A solution of hydrochloric acid in dioxane (25 mL, 4 M) was added to a solution of compound 12-5 (572 mg, 2.026 mmol) in dichloromethane (10 mL). The reaction mixture was stirred at room temperature for 2 h. When the reaction was completed as detected by LCMS, the mixture was concentrated under reduced pressure to remove the solvent to give crude compound 12-6 (369 mg, yield: 99%). ES-API: [M+H]⁺ = 183.3.

Step 6: A solution of borane in tetrahydrofuran (10 mL, 10 mmol, a 1 M solution in tetrahydrofuran) was added to a solution of compound 12-6 (369 mg, 2.025 mmol) in tetrahydrofuran (5 mL). The reaction mixture was heated to 90 °C and stirred overnight. When the starting materials were consumed as detected by LCMS, the reaction mixture was cooled to 0 °C, and hydrochloric acid (10 mL, 10 M) was added. The reaction mixture was then heated to 60 °C and stirred for 4 h. Then the reaction mixture was cooled to 0 °C again, and the pH of the reaction mixture was adjusted to alkalinity by adding an ammonia-methanol solution. A large number of white insoluble substances were precipitated. The mixture was filtered, and the filtrate was concentrated. The residue was purified by automated flash chromatography on silica gel (methanol:dichloromethane = 20-100%) to give crude compound 12-7 (348 mg). ES-API: [M+ H]⁺ = 169.1.

Step 7: Compound 12-7 (139 mg, 0.826 mmol), compound 75-1 (339 mg, 0.991 mmol), cesium carbonate (673 mg, 2.066 mmol), palladium acetate (19 mg, 0.083 mmol), and 2,2'-bis(diphenylphosphino)-1,1'-binaphthyl (26 mg, 0.041 mmol) were dissolved in anhydrous toluene (8 mL) under a nitrogen atmosphere, and the reaction mixture was heated to 120 °C under microwave and stirred for 5 h. When the reaction was completed as detected by LCMS, the reaction mixture was cooled to room temperature and concentrated under reduced pressure to remove the solvent. The crude product was purified by automated flash chromatography on silica gel (methanol:dichloromethane = 0-20%) to give compound 12-8 (207 mg, yield: 65%). ES-API: [M+H]⁺ = 382.1.

Step 8: Compound 12-8 (74 mg, 0.194 mmol), intermediate 2 (67 mg, 0.097 mmol), potassium phosphate (62 mg, 0.291 mmol), and [1,1'-bis(di-tert-butylphosphino)ferrocene]dichloropalladium(II) (12.6 mg, 0.019 mmol) were dissolved in dioxane (1 mL), toluene (3 mL), and water (1 mL) under a nitrogen atmosphere. The reaction mixture was heated to 65 °C under an oil bath and stirred for 2 h. When the reaction was completed as detected by LCMS, the mixture was cooled to room temperature. Ethyl acetate (30 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (methanol:dichloromethane = 0-10%) to give compound 12-9 (83 mg, yield: 98%). ES-API: [M+H]⁺ = 867.3.

Step 9: Cesium carbonate (312 mg, 0.957 mmol) and iodoethane (75 mg, 0.479 mmol) were sequentially added to a solution of compound 12-9 (83 mg, 0.096 mmol) in N,N-dimethylformamide (8 mL). The reaction mixture was stirred at room temperature for 6 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran:petroleum ether = 0-80%) to give compound 12-10 (85 mg, yield: 99%). ES-API: [M+H]⁺ = 895.3.

Step 10: Compound 12-10 (85 mg, 0.095 mmol) was dissolved in a solution of hydrochloric acid in dioxane (3 mL, 4 M) and a solution of hydrochloric acid in methanol (1 mL, 4 M). The reaction mixture was stirred at room temperature for 1 h. When the reaction was completed as detected by LCMS, the reaction mixture was concentrated under reduced pressure to give crude compound 12-11 (76 mg, yield: 99%). ES-API: [M+H]⁺ = 795.3.

Step 11: (1r,2R,3S)-2,3-Dimethylcyclopropane-1-carboxylic acid (16 mg, 0.143 mmol), N,N-diisopropylethylamine (0.1 mg, 0.574 mmol), and O-(7-azabenzotriazol-1-yl)-N,N,N',N'-tetramethyluronium hexafluorophosphate (109 mg, 0.287 mmol) were sequentially added to a solution of compound 12-11 (76 mg, 0.096 mmol) in N,N-dimethylformamide (2 mL) under an ice-water bath. The reaction mixture was warmed to room temperature and stirred for 10 min. When the reaction was completed as detected by LCMS, the reaction mixture was diluted with ethyl acetate (20 mL) and then washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give a mixture (1r,2R,3S)-N-((3'*S*,4'*S*,Z)-1'-ethyl-2'-(2-((S)-1-methoxyethyl)-5-(S)-tetrahydro-4'H-spiro[cyclopropane-1,3'-pyrazino[2,1-c][1,4]oxazin]-8'(1'H)-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z12, 52 mg, yield: 60%). ES-API: [M+H]⁺ = 891.4.

Step 12: Z12 (52 mg) was resolved by a chiral column (chromatographic column: CHIRALPAK^{®} IB 250 × 4.6 mm, 5 µm; mobile phase A: HEX + 0.2% DEA, mobile phase B: ETOH + 0.2% DEA; detection wavelength: 254 nm; flow rate: 1 mL/min; injection volume: 5 µL; column temperature: 30 °C; run time: 15 min; isocratic elution program: mobile phase A:mobile phase B = 40:60 (V/V)) to give two isomeric compounds. One of the structures was arbitrarily designated as (1*r,*2*R,*3*S*)-*N-*((3'S,4'S,Z)-1¹-ethyl-(Rₐ)-2'-(2-((S)-1-methoxyethyl)-5-(S)-tetrahydro-4'H-spiro[cyclopropane-1,3'-pyrazino[2,1-c][1,4]oxazin]-8'(1'H)-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z13, 17.22 mg, retention time: 7.799 min), ES-API [M+H]⁺ = 891.4.

The other structure was arbitrarily designated as (1*r*,2*R*,3*S*)-*N*-((3'*S*,4'*S*,*Z*)-1'-ethyl-(*S*ₐ)-2¹-(2-((S)-1-methoxyethyl)-5-(S)-tetrahydro-4'H-spiro[cyclopropane-1,3'-pyrazino[2,1-c][1,4]oxazin]-8'(1'H)-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z14, 29.14 mg, retention time: 5.174 min). ES-API [M+H]⁺ = 891.4.

### Example 11. Synthesis of Compound Z76

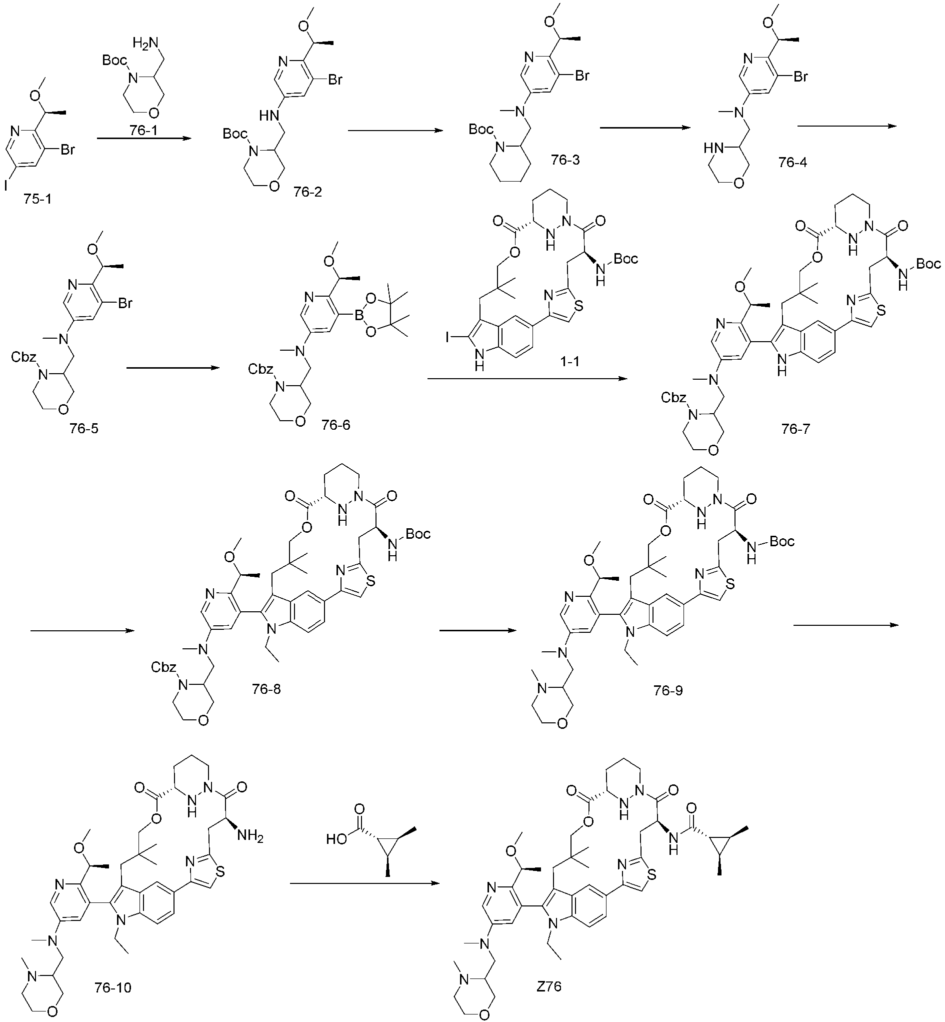

Step 1: Compound 75-1 (1 g, 2.924 mmol) and compound 76-1 (632.45 mg, 2.924 mmol) were dissolved in 1,4-dioxane (15 mL), and palladium acetate (65.65 mg, 0.292 mmol), 2,2'-bis(diphenylphosphino)-1,1'-binaphthyl (91.04 mg, 0.146 mmol), and cesium carbonate (1905.40 mg, 5.848 mmol) were added. The reaction system was stirred at 100 °C overnight. The system was cooled to room temperature, and ethyl acetate (50 mL) was added. The mixture was washed sequentially with water (30 mL × 1) and saturated brine (30 mL × 1), dried over anhydrous sodium sulfate, filtered, and concentrated, and the residue was purified by silica gel column chromatography (eluent: ethyl acetate/petroleum ether = 1/1) to give compound 76-2 (1.15 g, yield: 91.39%). ES-API: [M+H]⁺ = 430.1; 432.1.

Step 2: Compound 76-2 (1 g, 2.32 mmol) was dissolved in dichloromethane (10 mL), and a 30% aqueous formaldehyde solution (2.1 mL, 23.2 mmol), sodium cyanoborohydride (438.07 mg, 6.971 mmol), and acetic acid (28 mg, 0.464 mmol) were added. The mixture was stirred at room temperature for 3 h. Then, a 30% aqueous formaldehyde solution (2.1 mL, 23.2 mmol) and sodium cyanoborohydride (438.07 mg, 6.971 mmol) were added again, and the mixture was stirred for 15 h. After the reaction was completed, saturated sodium bicarbonate (30 mL) was slowly added under an ice-water bath. The organic phase was separated, washed sequentially with water (30 mL × 1) and saturated brine (30 mL × 1), dried over anhydrous sodium sulfate, filtered, and concentrated, and the residue was purified by silica gel column chromatography (eluent: ethyl acetate/petroleum ether = 1/1) to give compound 76-3 (500 mg, yield: 48.4%). ES-API: [M+H]⁺ = 442.1; 444.1.

Step 3: Compound 76-3 (500 mg, 1.125 mmol) was dissolved in dichloromethane (10 mL), and trifluoroacetic acid (5 mL) was slowly added under an ice-water bath. The mixture was stirred at room temperature for 1 h. The reaction mixture was concentrated to give compound 76-4 (600 mg, crude product, trifluoroacetate), which was directly used in the next step. ES-API: [M+H]⁺ = 344.1; 346.1.

Step 4: Compound 76-4 (600 mg, crude product, trifluoroacetate) was dissolved in tetrahydrofuran (5 mL) and water (3 mL), and potassium carbonate (445.61 mg, 3.224 mmol) and benzyl chloroformate (201.69 mg, 1.182 mmol) were slowly added under an ice-water bath. The mixture was stirred at room temperature for 1 h. After the reaction was completed, ethyl acetate (20 mL) was added, and the mixture was washed sequentially with water (20 mL × 1) and saturated brine (20 mL × 1), dried over anhydrous sodium sulfate, filtered, and concentrated. The residue was purified by silica gel column chromatography (eluent: ethyl acetate/petroleum ether = 1/1) to give compound 76-5 (420 mg, two-step yield: 78%). ES-API: [M+H]⁺ = 478.0; 480.0.

Step 5: Compound 76-5 (300 mg, 0.627 mmol) and 4,4,5,5-tetramethyl-2-(4,4,5,5-tetramethyl-1,3,2-dioxaborolan-2-yl)-1,3,2-dioxaborolane (238.83 mg, 0.941 mmol) were added to 1,4-dioxane (5 mL), and then [1,1'-bis(diphenylphosphino)ferrocene]palladium(II) dichloride (45.84 mg, 0.063 mmol) and potassium acetate (124.17 mg, 1.254 mmol) were sequentially added. The mixture was heated to 100 °C and reacted for 15 h. The reaction mixture was filtered and concentrated, and the residue was purified by neutral alumina column chromatography (tetrahydrofuran/petroleum ether = 1/1) to give compound 75-6 (110 mg, yield: 33.38%). ES-API: [M+H]⁺ = 526.2.

Step 6: Compound 75-6 (167.4 mg, 0.319 mmol) and compound 1-1 (170 mg, 0.245 mmol) were added to a mixed solvent of 1,4-dioxane (1.5 mL), water (0.5 mL), and toluene (0.5 mL), and then [1,1'-bis(diphenylphosphino)ferrocene]palladium(II) dichloride (15.82 mg, 0.024 mmol) and potassium phosphate (104.01 mg, 0.490 mmol) were sequentially added. The mixture was heated to 70 °C, reacted for 15 h, and cooled to room temperature. Ethyl acetate (10 mL) was added, and the mixture was washed sequentially with water (10 mL × 1) and saturated brine (10 mL × 1), dried over anhydrous sodium sulfate, filtered, and concentrated. The residue was purified by silica gel column chromatography (eluent: ethyl acetate/petroleum ether = 4/1) to give compound 76-7 (115 mg, yield: 48.6%). ES-API: [M+H]⁺ = 965.4.

Step 7: Compound 76-7 (115 mg, 0.119 mmol) was dissolved in N,N-dimethylformamide (3 mL), and cesium carbonate (77.64 mg, 0.238 mmol) and iodoethane (92.92 mg, 0.596 mmol) were added. The mixture was stirred in a sealed tube at room temperature for 15 h. After the reaction was completed, the system was purified by silica gel column chromatography (eluent: ethyl acetate/petroleum ether = 4/1) to give compound 76-8 (80 mg, yield: 67.60%), ES-API: [M+H]⁺ = 993.4.

Step 8: Compound 76-8 (80 mg, 0.081 mmol) was dissolved in methanol (10 mL), and paraformaldehyde (145 mg, 1.611 mmol) and 10% palladium hydroxide on carbon (113 mg) were added. The mixture was stirred at room temperature for 24 h under a hydrogen atmosphere. After the reaction was completed, the mixture was purified by silica gel column chromatography (ethyl acetate/petroleum ether = 10/1) to give compound 76-9 (40 mg, yield: 56.6%). ES-API: [M+H]⁺ = 873.4.

Step 9: Compound 76-9 (40 mg, 0.046 mmol) was dissolved in dichloromethane (5 mL). The mixture was cooled to 0 °C under an ice-water bath, and trifluoroacetic acid (2 mL) was slowly added. The mixture was slowly warmed to room temperature and stirred for 1 h. After the reaction was completed, the reaction mixture was concentrated to give compound 76-10 (50 mg, crude product). ES-API: [M+H]⁺ = 773.4.

Step 10: Compound 76-10 (50 mg, crude product) was dissolved in dichloromethane (5 mL), and triethylamine (30.11 mg, 0.298 mmol), (1r,2R,3S)-2,3-dimethylcyclopropane-1-carboxylic acid, and a 50% solution of propylphosphonic anhydride in ethyl acetate (0.5 mL) were sequentially added under an ice-water bath. When the reaction was completed after 10 min, dichloromethane (10 mL) was added, and the mixture was washed sequentially with an aqueous sodium bicarbonate solution (10 mL × 1) and saturated brine (10 mL × 1), dried over anhydrous sodium sulfate, filtered, and concentrated. The residue was purified by prep-HPLC (ammonium bicarbonate method) to give (1r,2R,3S)-N-((6³S,4S,Z)-1¹-ethyl-1²-(2-((S)-1-methoxyethyl)-5-(methyl((4-methylmorpholin-3-yl)methyl)amino)pyridin-3-yl)-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-4-yl)-2,3-dimethylcyclopropane-1-carboxamide (Z76, 5 mg, yield: 13%). ES-API: [M+H]⁺ = 869.3.

### Example 12. Synthesis of Compound Z77

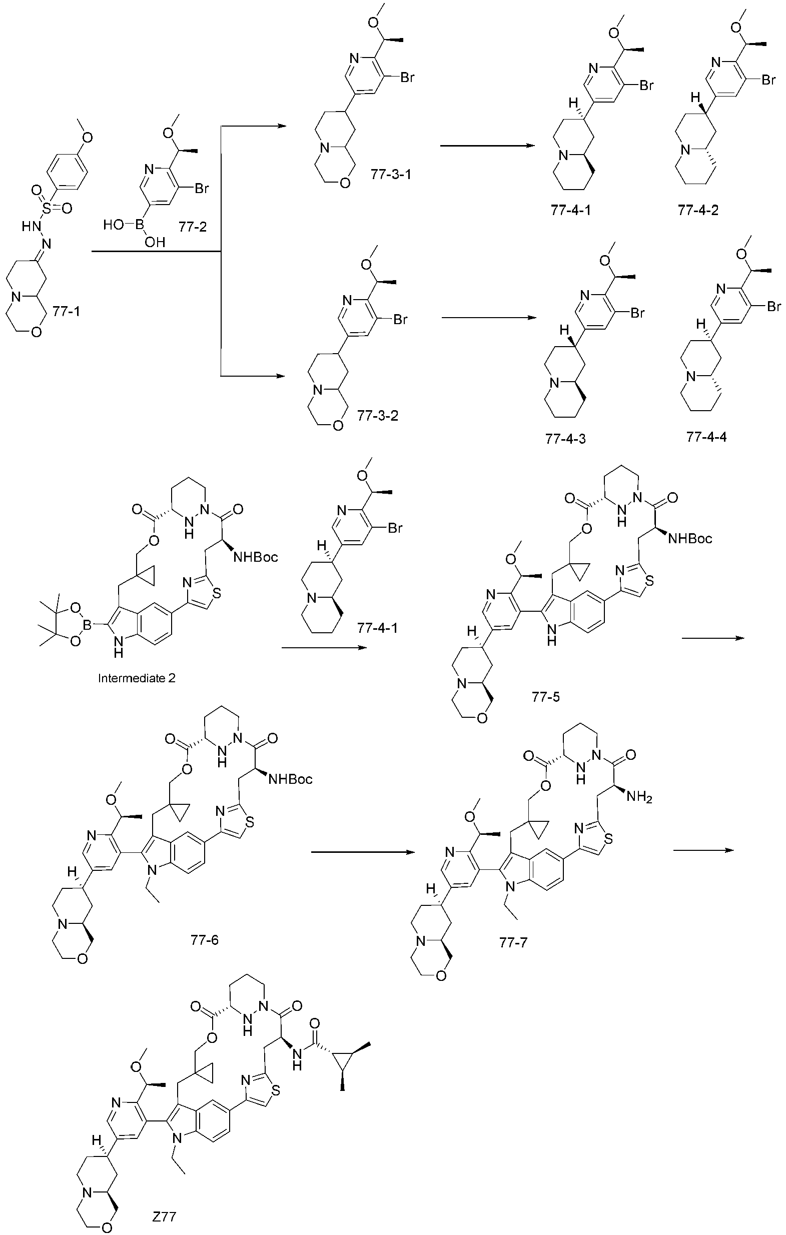

Step 1: Compound 77-1 (650 mg, 1.915 mmol), compound 77-2 (995.42 mg, 3.830 mmol), and cesium carbonate (529.33 mg, 3.830 mmol) were dissolved in 1,4-dioxane (20 mL), and the mixture was reacted at 110 °C for 16 h. After the reaction was completed, water (30 mL) was added, and the mixture was extracted with ethyl acetate (30 mL × 3). The organic phase was washed with saturated brine (30 mL), dried over anhydrous sodium sulfate, and purified by silica gel column chromatography (dichloromethane:methanol = 10:1) to give a mixture 77-3-1 (Rf = 0.5) and a mixture 77-3-2 (Rf = 0.45).

Mixture 77-3-1 was resolved by a chiral column (column type: IB 250 mm, 4.6 mm, 5 µm; mobile phase system: (A: n-hexane; B: ethanol); flow rate: 1 mL/min; B% = 0-50%; column temperature: room temperature) to give two isomers. The structure of one of the isomers was arbitrarily designated as compound 77-4-1 (155 mg, 0.436 mmol, yield: 9.9%, peak 1, retention time: 5.85 min), and the structure of the other isomer was arbitrarily designated as compound 77-4-2 (162 mg, 0.456 mmol, yield: 10.0%, peak 2, retention time: 6.68 min).

Mixture 77-3-2 was resolved by a chiral column (column type: IB 250 mm, 4.6 mm, 5 µm; mobile phase system: (A: n-hexane; B: ethanol); flow rate: 1 mL/min; B% = 0-50%; column temperature: room temperature) to give two isomers. The structure of one of the isomers was arbitrarily designated as compound 77-4-3 (96 mg, 0.27 mmol, yield: 6.2%, peak 3, retention time: 7.64 min), and the structure of the other isomer was arbitrarily designated as compound 77-4-4 (101 mg, 0.28 mmol, 6.5%, peak 4, retention time: 9.87 min).

Step 2: Intermediate 2 (60 mg, 0.086 mmol) and compound 77-4-1 (64.07 mg, 0.173 mmol) were dissolved in toluene (1.5 mL), 1,4-dioxane (0.5 mL), and water (0.5 mL), and potassium phosphate (55.24 mg, 0.260 mmol) and [1,1'-bis(di-tert-butylphosphino)ferrocene]dichloropalladium(II) (8.47 mg, 0.013 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 77-5 (55 mg, 0.064 mmol, yield: 74.2%). ES-API: [M+H]⁺ = 840.3.

Step 3: Compound 77-5 (50 mg, 0.060 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 5 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 77-6 (40 mg, 0.046 mmol, yield: 77.4%). ES-API: [M+H]⁺ = 868.3.

Step 4: Compound 77-6 (35 mg, 0.040 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 77-7 (25 mg, 0.033 mmol, yield: 81%). ES-API: [M+H]⁺ = 768.3.

Step 5: Compound 77-7 (25 mg, 0.033 mmol) was dissolved in dichloromethane (3 mL), and (1r,2R,3S)-2,3-dimethylcyclopropane-1-carboxylic acid (4.83 mg, 0.042 mmol), O-(7-azabenzotriazol-1-yl)-N,N,N',N'-tetramethyluronium hexafluorophosphate (18.57 mg, 0.049 mmol), and *N*,*N*'-diisopropylethylamine (0.016 mL, 0.098 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1r,2R,3S)-N-((3'S,4'S,Z)-1'-ethyl-2'-(2-((S)-1-methoxyethyl)-5-((2R,9aR)-octahydropyrido[2,1-c][1,4]oxazin-8-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z77, structure presumed from that of compound 77-4-1, 15 mg, yield: 53.32%) as a white solid. ES-API: [M+H]⁺ = 864.4.

### Example 13. Synthesis of Compound Z78

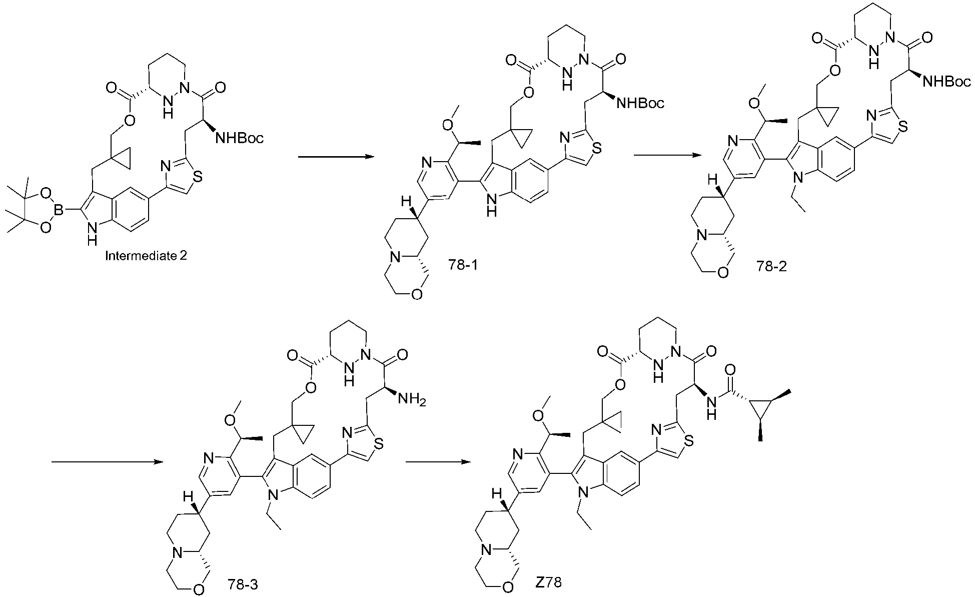

Step 1: Intermediate 2 (60 mg, 0.086 mmol) and compound 77-4-2 (64.07 mg, 0.173 mmol) were dissolved in toluene (1.5 mL), 1,4-dioxane (0.5 mL), and water (0.5 mL), and potassium phosphate (55.24 mg, 0.260 mmol) and [1,1'-bis(di-tert-butylphosphino)ferrocene]dichloropalladium(II) (8.47 mg, 0.013 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 78-1 (50 mg, 0.060 mmol, yield: 69.21%). ES-API: [M+H]⁺ = 840.3.

Step 2: Compound 78-1 (50 mg, 0.060 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 5 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 78-2 (38 mg, 0.044 mmol, yield: 73.54%). ES-API: [M+H]⁺ = 868.4.

Step 3: Compound 78-2 (38 mg, 0.044 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 78-3 (25 mg, 0.033 mmol, yield: 74%). ES-API: [M+H]⁺ = 768.3.

Step 4: Compound 78-3 (25 mg, 0.033 mmol) was dissolved in dichloromethane (3 mL), and (1r,2R,3S)-2,3-dimethylcyclopropane-1-carboxylic acid (4.83 mg, 0.042 mmol), O-(7-azabenzotriazol-1-yl)-N,N,N',N'-tetramethyluronium hexafluorophosphate (18.57 mg, 0.049 mmol), and *N*,*N*'-diisopropylethylamine (0.016 mL, 0.098 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1r,2R,3S)-N-((3'S,4'S,Z)-1'-ethyl-2'-(2-((S)-1-methoxyethyl)-5-((2S,9aS)-octahydropyrido[2,1-c][1,4]oxazin-8-yl)pyridin-3-yl)-5',7-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z78, 15 mg, yield: 53.32%) as a white solid. ES-API: [M+H]⁺ = 864.4.

### Example 14. Synthesis of Compound Z79

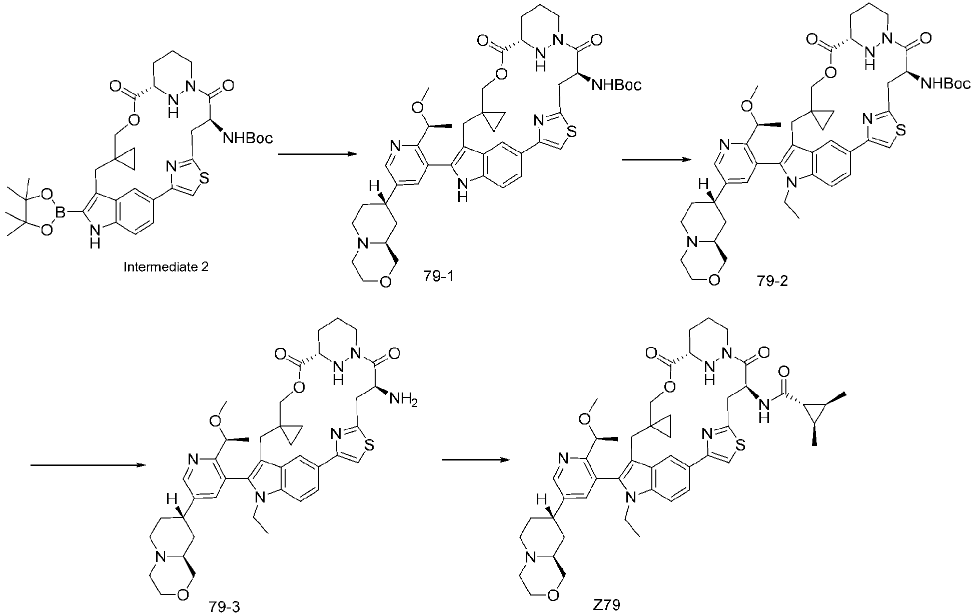

Step 1: Intermediate 2 (60 mg, 0.086 mmol) and compound 77-4-3 (64.07 mg, 0.173 mmol) were dissolved in toluene (1.5 mL), 1,4-dioxane (0.5 mL), and water (0.5 mL), and potassium phosphate (55.24 mg, 0.260 mmol) and [1,1'-bis(di-tert-butylphosphino)ferrocene]dichloropalladium(II) (8.47 mg, 0.013 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 79-1 (50 mg, 0.060 mmol, yield: 69.21%). ES-API: [M+H]⁺ = 840.3.

Step 2: Compound 79-1 (50 mg, 0.060 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 5 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 79-2 (38 mg, 0.044 mmol, yield: 73.54%). ES-API: [M+H]⁺ = 868.4.

Step 3: Compound 79-2 (38 mg, 0.044 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 79-3 (25 mg, 0.033 mmol, yield: 74%). ES-API: [M+H]⁺ = 768.3.

Step 4: Compound 79-3 (25 mg, 0.033 mmol) was dissolved in dichloromethane (3 mL), and (1r,2R,3S)-2,3-dimethylcyclopropane-1-carboxylic acid (4.83 mg, 0.042 mmol), O-(7-azabenzotriazol-1-yl)-N,N,N',N'-tetramethyluronium hexafluorophosphate (18.57 mg, 0.049 mmol), and N,N'-diisopropylethylamine (0.016 mL, 0.098 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1r,2R,3S)-N-((3'S,4'S,Z)-1'-ethyl-2'-(2-((S)-1-methoxyethyl)-5-((2S,9aR)-octahydropyrido[2,1-c][1,4]oxazin-8-yl)pyridin-3-yl)-5',7-dioxospiro[cyclopropane-1,10'-8-oxa-2(4,2)-thiazola-1(5,3)-indola-6(1,3)-pyridazinacycloundecaphan]-4'-yl)2,3-dimethylcyclopropane-1-carboxamide (Z79, 15 mg, yield: 53.32%) as a white solid. ES-API: [M+H]⁺ = 864.4.

### Example 15. Synthesis of Compound Z80

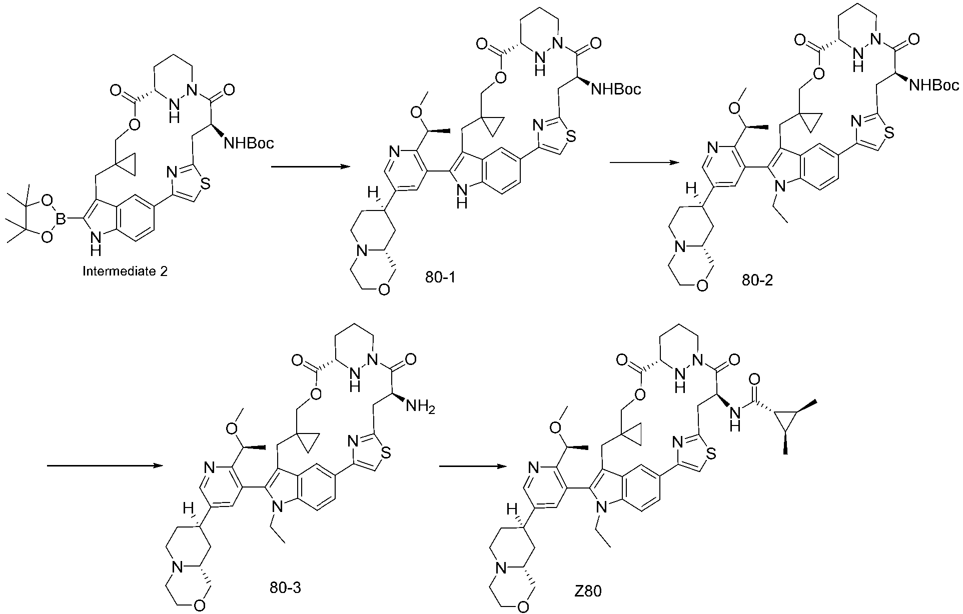

Step 1: Intermediate 2 (60 mg, 0.086 mmol) and compound 77-4-4 (64.07 mg, 0.173 mmol) were dissolved in toluene (1.5 mL), 1,4-dioxane (0.5 mL), and water (0.5 mL), and potassium phosphate (55.24 mg, 0.260 mmol) and [1,1'-bis(di-tert-butylphosphino)ferrocene]dichloropalladium(II) (8.47 mg, 0.013 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 80-1 (50 mg, 0.060 mmol, yield: 69.21%). ES-API: [M+H]⁺ = 840.3.

Step 2: Compound 80-1 (50 mg, 0.060 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 5 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 80-2 (38 mg, 0.044 mmol, yield: 73.54%). ES-API: [M+H]⁺ = 868.4.

Step 3: Compound 80-2 (38 mg, 0.044 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 80-3 (25 mg, 0.033 mmol, yield: 74%). ES-API: [M+H]⁺ = 768.3.

Step 4: Compound 80-3 (25 mg, 0.033 mmol) was dissolved in dichloromethane (3 mL), and (1r,2R,3S)-2,3-dimethylcyclopropane-1-carboxylic acid (4.83 mg, 0.042 mmol), O-(7-azabenzotriazol-1-yl)-N,N,N',N'-tetramethyluronium hexafluorophosphate (18.57 mg, 0.049 mmol), and N,N'-diisopropylethylamine (0.016 mL, 0.098 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1r,2R,3S)-N-((3'S,4'S,Z)-1'-ethyl-2'-(2-((S)-1-methoxyethyl)-5-((2R,9aS)-octahydropyrido[2,1-c][1,4]oxazin-8-yl)pyridin-3-yl)-5',7-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z80, 15 mg, yield: 53.32%) as a white solid. ES-API: [M+H]⁺ = 864.4.

### Example 16. Synthesis of Compound Z372

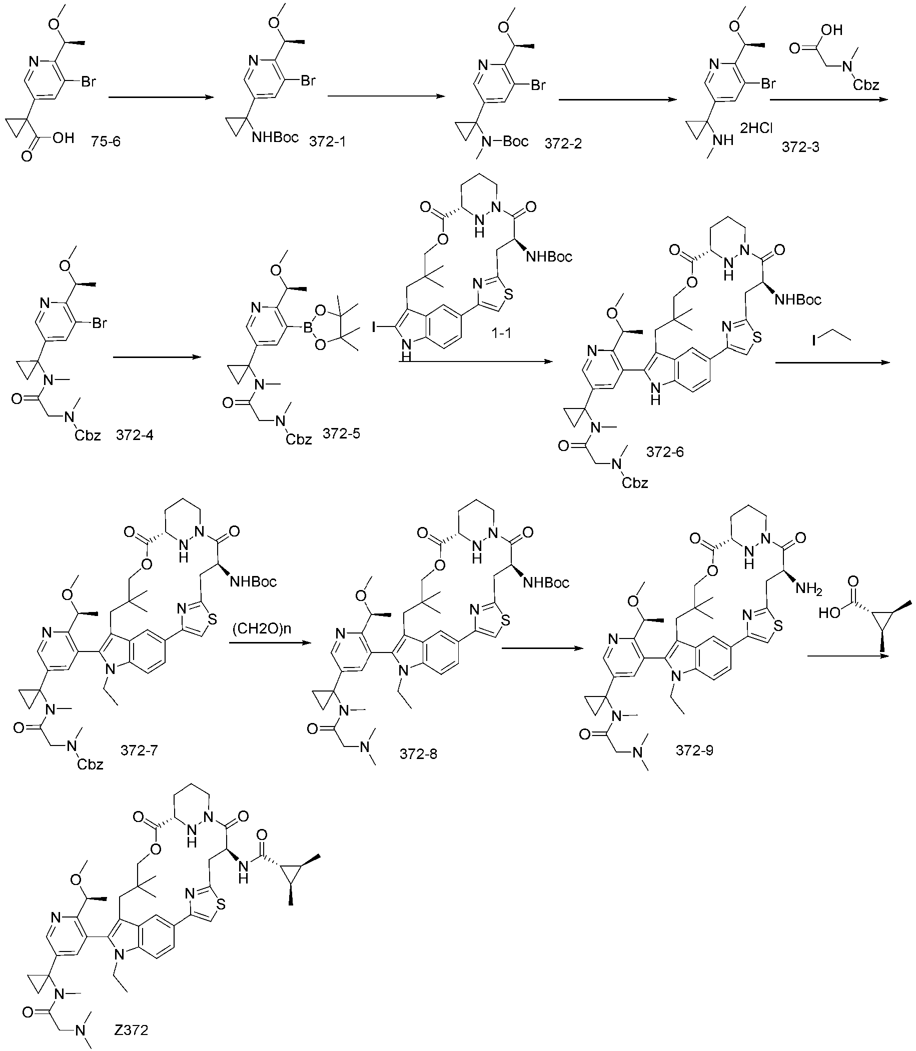

Step 1: Compound 75-6 (3.9 g, 12.99 mmol) and triethylamine (3.61 mL, 25.99 mmol) were dissolved in tert-butanol (50 mL) and toluene (25 mL), and diphenylphosphoryl azide (4.20 mL, 19.49 mmol) was added. The reaction system was heated to reflux and stirred for 6 h. The reaction mixture was concentrated under reduced pressure, and ethyl acetate (100 mL) was added. The mixture was washed sequentially with water (50 mL) and saturated brine (30 mL). The organic phase was dried over anhydrous sodium sulfate and filtered, and the filtrate was concentrated under reduced pressure. The crude product was purified by a flash silica gel column (ethyl acetate/petroleum ether: 0-50%) to give compound 372-1 (3.8 g, yield: 78.8%) as a white solid. ES-API: [M+H]⁺ = 371.1, 373.1.

Step 2: Compound 372-1 (1.0 g, 2.69 mmol) was dissolved in N,N-dimethylformamide (10 mL), and 60% sodium hydride (194 mg, 4.85 mmol) was added at 0 °C. After the reaction system was stirred at 0 °C for 30 min, a solution of iodomethane (573 mg, 4.04 mmol) in N,N-dimethylformamide (2 mL) was added, and the reaction system was stirred at room temperature for 3 h. Water (50 mL) was added to the reaction mixture, and the mixture was extracted with ethyl acetate (100 mL). The organic phases were mixed and washed with saturated brine (30 mL × 3). The organic phase was dried over anhydrous sodium sulfate and concentrated. The crude product was purified by a flash silica gel column (ethyl acetate/petroleum ether: 0-30%) to give compound 372-2 (980 mg, yield: 94.4%) as a white solid. ES-API: [M+H]⁺ = 385.1, 387.1.

Step 3: Compound 372-2 (930 mg, 2.41 mmol) was dissolved in absolute methanol (3 mL), and a 4.0 M solution of hydrogen chloride in dioxane (5 mL) was added at 0 °C. The reaction system was stirred at room temperature for 3 h. The reaction mixture was concentrated to give compound 372-3 (850 mg, yield: 98.3%) as a white solid. ES-API: [M+H]⁺ = 285.0, 287.0. Step 4: Compound 372-3 (850 mg, 2.37 mmol), N-carbobenzoxy-N-methylglycine (795 mg, 3.56 mmol), and triethylamine (2.64 mL, 18.99 mmol) were dissolved in dichloromethane (25 mL), and a 50% solution (4.53 g, 7.12 mmol) of 1-propylphosphonic anhydride in ethyl acetate was added at 0 °C. The reaction system was stirred at room temperature for 1 h. Dichloromethane (25 mL) was added to the reaction mixture. The mixture was washed sequentially with water (15 mL) and saturated brine (15 mL), dried over anhydrous sodium sulfate, and concentrated. The crude product was purified by a flash silica gel column (ethyl acetate/petroleum ether: 0-100%) to give the desired product of compound 372-4 (1.10 g, yield: 94.5%) as a colorless viscous liquid. ES-API: [M+H]⁺ = 490.1,492.1.

Step 5: Compound 372-4 (500 mg, 1.02 mmol) was dissolved in toluene (10 mL), and bis(pinacolato)diboron (336 mg, 1.32 mmol), [1,1'-bis(diphenylphosphino)ferrocene]palladium(II) dichloride (75 mg, 0.10 mmol), and potassium acetate (250 mg, 2.55 mmol) were added. The reaction system was purged three times with nitrogen and reacted at 100 °C for 18 h. The reaction mixture was filtered through celite, and the filtrate was concentrated. The crude product was purified by a neutral alumina column (tetrahydrofuran/petroleum ether: 0-100%) to give compound 372-5 (360 mg, yield: 65.7%) as a black liquid. ES-API: [M+H]⁺ = 538.3.

Step 6: Compound 372-5 (302 mg, 0.28 mmol) and compound 1-1 (150 mg, 0.22 mmol) were dissolved in toluene (6 mL), dioxane (2 mL), and water (2 mL), and potassium phosphate (138 mg, 0.65 mmol) and [1,1'-bis(di-tert-butylphosphino)ferrocene]dichloropalladium(II) (28 mg, 0.043 mmol) were added. The reaction system was purged 3 times with nitrogen and stirred at 70 °C for 18 h. Ethyl acetate (50 mL) and water (15 mL) were added to the reaction mixture. The separated organic phase was washed with saturated brine (15 mL), dried over anhydrous sodium sulfate, and filtered, and the filtrate was concentrated under reduced pressure. The crude product was purified by a flash silica gel column (tetrahydrofuran/petroleum ether: 0-80%) to give compound 372-6 (135 mg, yield: 63.9%) as a brown solid. ES-API: [M+H]⁺ = 977.3.

Step 7: Compound 372-6 (130 mg, 0.13 mmol) was dissolved in N,N-dimethylformamide (5 mL), and cesium carbonate (130 mg, 0.40 mmol) and iodoethane (42 mg, 0.27 mmol) were added at 0 °C. The reaction system was stirred at room temperature for 18 h. Ethyl acetate (40 mL) was added to the reaction mixture, and the mixture was washed sequentially with water (15 mL) and saturated brine (15 mL × 3), dried over anhydrous sodium sulfate, and concentrated. The crude product was purified by a flash silica gel column (tetrahydrofuran/petroleum ether: 0-80%) to give compound 372-7 (105 mg, yield: 78.5%) as a pale brown solid. ES-API: [M+H]⁺ = 1005.5.

Step 8: Compound 372-7 (95 mg, 0.095 mmol) and paraformaldehyde (85 mg, 2.85 mmol) were dissolved in absolute methanol (3 mL), and 10% palladium hydroxide on carbon (120 mg) was added. The reaction system was stirred at room temperature for 48 h under a hydrogen atmosphere. The reaction mixture was filtered through celite and washed with methanol, and the filtrate was concentrated. The crude product was subjected to preparative silica gel thin-layer chromatography (dichloromethane/methanol = 12:1) to give compound 372-8 (45 mg, yield: 53.8%) as a white solid. ES-API: [M+H]⁺ = 885.3.

Step 9: Compound 372-8 (45 mg, 0.051 mmol) was dissolved in absolute methanol (0.3 mL), and a 4.0 M solution of hydrogen chloride in dioxane (1.5 mL) was added at 0 °C. The reaction system was stirred at room temperature for 2 h. The reaction mixture was concentrated to give compound 372-9 (40 mg, yield: 100%) as a white solid. ES-API: [M+H]⁺ = 785.3.

Step 10: Compound 372-9 (35 mg, 0.045 mmol) was dissolved in dichloromethane (1.5 mL), and (1r,2R,3S)-2,3-dimethylcyclopropane-1-carboxylic acid (8 mg, 0.067 mmol), O-(7-azabenzotriazol-1-yl)-N,N,N',N'-tetramethyluronium hexafluorophosphate (34 mg, 0.089 mmol), and N,N'-diisopropylethylamine (35 mg, 0.27 mmol) were added. The reaction system was stirred at room temperature for 2 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was subjected to preparative silica gel thin-layer chromatography (dichloromethane/methanol = 10: 1) to give the desired product (1r,2R,3S)-N-((6³S,4S,Z)-12-(5-(1-(2-(dimethylamino)-N-methylacetamido)cyclopropyl)-2-((S)-1-methoxyethyl)pyridin-3-yl)-1¹-ethyl-10,10-dimethyl-5,7-dioxo-6¹,6²,6³,6⁴,6⁵6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z372, 35 mg, yield: 89.1%) as an off-white solid. ES-API: [M+H]⁺ = 881.3.

### Example 17. Synthesis of Compound Z81

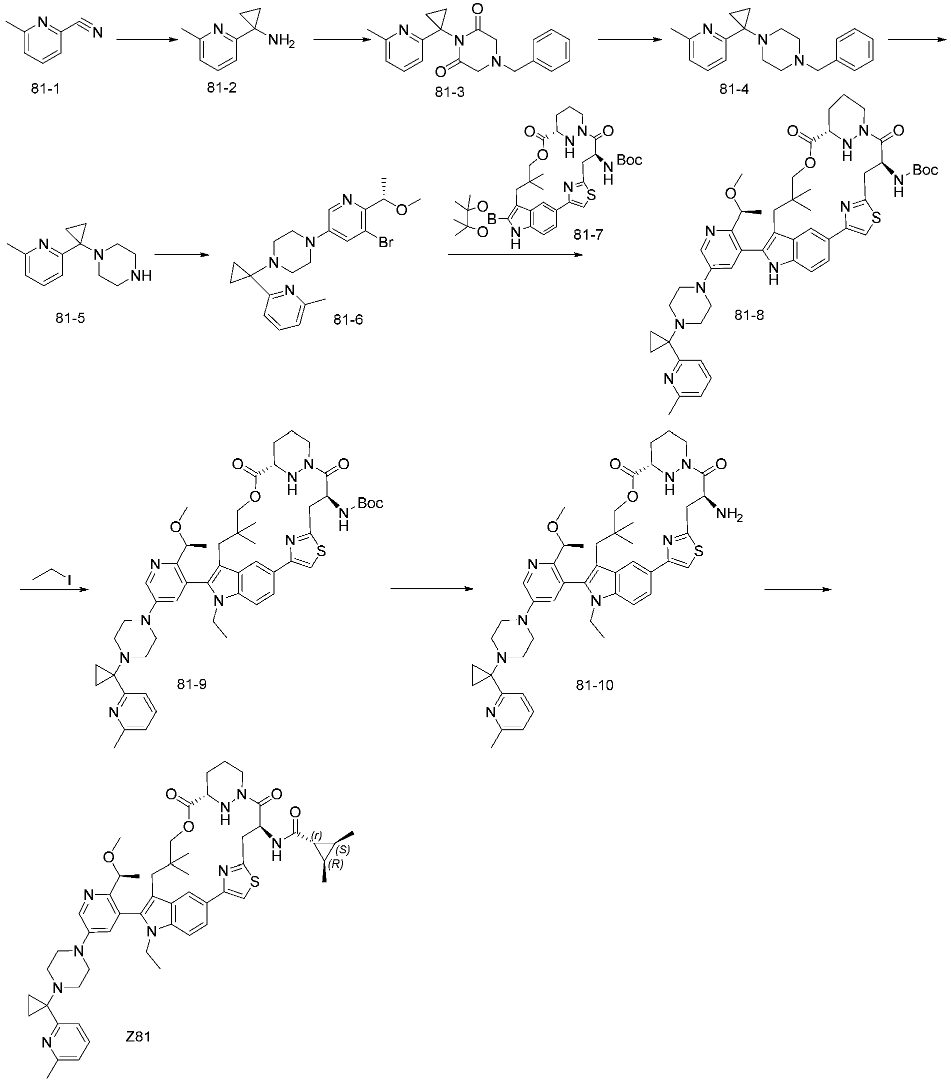

Step 1: Compound 81-1 (6 g, 50.7 mmol) was dissolved in dry tetrahydrofuran (120 mL), and titanium tetraisopropanolate (14.4 g, 50.7 mmol) was slowly added at room temperature. The mixture was stirred for 10 min, and then ethylmagnesium bromide (50.7 mL, 2 M) was slowly added. The mixture was stirred at room temperature for 1 h. After the reaction was completed, ice water (100 mL) was added, and the mixture was extracted with ethyl acetate (100 mL × 3). The organic phase was dried over anhydrous sodium sulfate, filtered, and concentrated, and the residue was purified by silica gel column chromatography (petroleum ether:ethyl acetate = 0-50%) to give compound 81-2 (1.2 g, 8.1 mmol, yield: 15.9%). ES-API [M+H]⁺ = 149.2.

Step 2: Compound N-benzyliminodiacetic acid (2.71 g, 12.1 mmol) was dissolved in dry tetrahydrofuran (60 mL), and N,N'-carbonyldiimidazole (4.33 g, 26.7 mmol) was added. The mixture was stirred at 65 °C for 1 h under a nitrogen atmosphere and cooled to room temperature. A solution of compound 81-2 (1.8 g, 12.1 mmol) in tetrahydrofuran was slowly added to the mixture solution, and the resulting mixture was stirred overnight at 65 °C. After the reaction was completed, water (100 mL) was added, and the mixture was extracted with ethyl acetate (100 mL × 3). The organic phase was dried over anhydrous sodium sulfate, filtered, and concentrated, and the residue was purified by silica gel column chromatography (petroleum ether:ethyl acetate = 0-50%) to give compound 81-3 (1 g, 2.97 mmol, yield: 25%). ES-API [M+H]⁺ = 336.2.

Step 3: Compound 81-3 (0.9 g, 2.68 mmol) was dissolved in dry tetrahydrofuran (20 mL), and lithium aluminum hydride (26.8 mL, 1 M) was added at 0 °C under a nitrogen atmosphere. The mixture was stirred at room temperature overnight. After the reaction was completed, excess sodium sulfate decahydrate was added, and the mixture was stirred for 30 min and filtered. The organic phase was dried over anhydrous sodium sulfate, filtered, and concentrated, and the residue was purified by silica gel column chromatography (petroleum ether:ethyl acetate = 0-30%) to give compound 81-4 (330 mg, 1.07 mmol, yield: 40%). ES-API [M+H]⁺ = 308.2.

Step 4: Compound 81-4 (330 mg, 1.07 mmol) was dissolved in dry 1,2-dichloroethane (10 mL), and 1-chloroethyl chloroformate (460 mg, 3.2 mmol) was added. The mixture was stirred at 60 °C for 2 h under a nitrogen atmosphere. After the mixture was cooled to room temperature, methanol (1 mL) was added. The mixture was stirred at 60 °C for 1 h under a nitrogen atmosphere. After the reaction was completed, the reaction mixture was concentrated, and the residue was purified by silica gel column chromatography (dichloromethane:methanol = 0-20%) to give compound 81-5 (50 mg, 0.23 mmol, yield: 21%). ES-API [M+H]⁺ = 218.2.

Step 5: A mixture of compound 81-5 (40 mg, 0.18 mmol), (S)-3-bromo-5-iodo-2-(1-methoxyethyl)pyridine (100 mg, 0.29 mmol), tris(dibenzylideneacetone)dipalladium(0) (20 mg, 0.02 mmol), and sodium tert-butoxide (50 mg, 0.52 mmol) was stirred at 90 °C for 18 h in dioxane (6 mL), toluene (2 mL), and water (2 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 81-6 (40 mg, 0.09 mmol, yield: 50%). ES-API [M+H]⁺ = 431.1/433.1.

Step 6: A mixture of compound 81-6 (41 mg, 0.1 mmol), compound 81-7 (100 mg, 0.14 mmol, see intermediate 3 in WO2022060836A1 for the preparation method), [1,1'-bis(di-tert-butylphosphino)ferrocene]dichloropalladium(II) (12.5 mg, 0.02 mmol), and potassium phosphate (61 mg, 0.29 mmol) was stirred at 60 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 81-8 (80 mg, 0.09 mmol, yield: 90%). ES-API [M+H]⁺ = 918.4.

Step 7: Compound 81-8 (80 mg, 0.09 mmol) was dissolved in N,N-dimethylformamide (3 mL), and cesium carbonate (142 mg, 0.43 mmol) and iodoethane (136 mg, 0.87 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 81-9 (40 mg, 0.042 mmol, yield: 48%). ES-API [M+H]⁺ = 918.4.

Step 8: Compound 81-9 (35 mg, 0.037 mmol) was dissolved in a solution of hydrochloric acid in dioxane (5 mL, 4 mol/L). The mixture was stirred at room temperature for 2 h. After the reaction was completed, the reaction mixture was concentrated to give compound 81-10 (31 mg, yield: 99%). ES-API [M+H]⁺ = 846.2.

Step 9: Compound 81-10 (37 mg, 0.04 mmol) was dissolved in N,N-dimethylformamide (3 mL), and (1r,2R,3S)-2,3-dimethylcyclopropane-1-carboxylic acid (7.5 mg, 0.06 mmol), N,N-diisopropylethylamine (34 mg, 0.26 mmol), and O-(7-azabenzotriazol-1-yl)-N,N,N',N'-tetramethyluronium hexafluorophosphate (50 mg, 0.13 mmol) were sequentially added. The mixture was stirred at room temperature for 1 h. After the reaction was completed, ethyl acetate (10 mL) was added, and the mixture was washed with saturated brine (10 mL) and water (10 mL). The organic phase was dried over anhydrous sodium sulfate, filtered, and concentrated, and the residue was purified by prep-HPLC (formic acid method 1) to give (1r,2R,3S)-N-((6³S,4S,Z)-1¹-ethyl-1²-(2-((S)-1-methoxyethyl)-5-(4-(1-(6-methylpyridin-2-yl)cyclopropyl)piperazin-1-yl)pyridin-3-yl)-10-10-dimethyl-5,7-dioxo-6¹,6²,6³,6⁴,6⁵ 6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide formate (Z81, 20 mg, yield: 48%, purity: 98%). ES-API [M+H]⁺ = 942.4.

### Example 18. Synthesis of Compound Z83

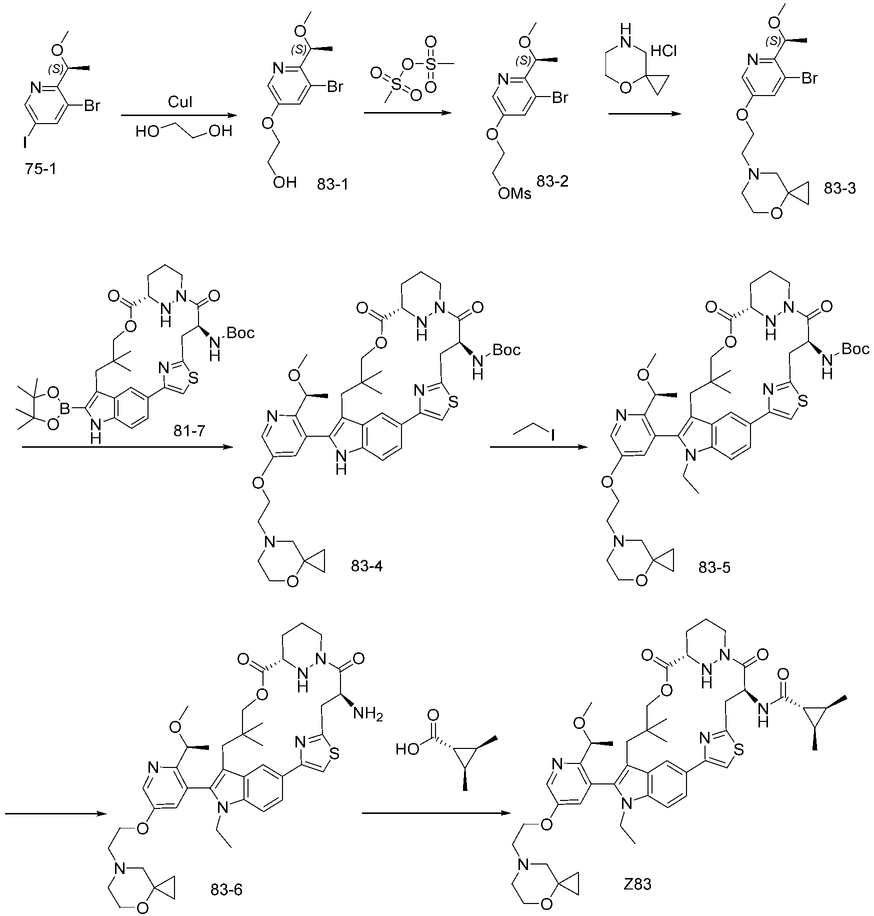

Step 1: Compound 75-1 (4.60 g, 13.45 mmol) was dissolved in anhydrous ethylene glycol (20.0 mL), and potassium carbonate (3.72 g, 26.90 mmol) and copper(I) iodide (3.72 g, 26.90 mmol) were added to the solution described above. The mixture was purged 4 times with nitrogen and reacted at 100 °C for 8 h. After the reaction was completed, the reaction mixture was cooled to room temperature, and ethyl acetate (60 mL) was added to the reaction mixture. The mixture was washed sequentially with diluted brine (30 mL × 3) and saturated brine (30 mL). The organic phase was dried over anhydrous sodium sulfate and filtered, and the filtrate was concentrated under reduced pressure. The crude product was purified by a flash silica gel column (methanol/dichloromethane: 0-10%) to give compound 83-1 (2.7 g, yield: 72.699%) as a pale yellow oily liquid. ES-API: [M+H]⁺ = 276.0/278.0.

Step 2: Under an ice-water bath, triethylamine (468.0 mg, 4.628 mmol) was added to a solution of compound 83-1 (213.0 mg, 0.771 mmol) in dichloromethane (20.0 mL), and finally a solution of methanesulfonic anhydride (132.54 mg, 1.157 mmol) in dichloromethane (2.0 mL) was added dropwise. The mixture was reacted at room temperature for 2 h. After the reaction was completed, ethyl acetate (80 mL) was added to the reaction mixture. The mixture was washed sequentially with diluted brine (40 mL × 3) and saturated brine (80 mL). The organic phase was dried over anhydrous sodium sulfate and filtered, and the filtrate was concentrated under reduced pressure. The crude product was purified by a flash silica gel column (ethyl acetate/petroleum ether: 0-50%) to give compound 83-2 (0.28 g, crude product), ES-API: [M+H]⁺ = 354.0/356.0.

Step 3: N,N-Diisopropylethylamine (596.0 mg, 4.626 mmol) and 4-oxa-7-azaspiro[2.5]octane hydrochloride (125.0 mg, 0.836 mmol) were added to a solution of compound 83-2 (0.28 g, crude product) in N,N-dimethylformamide (10.0 mL) at the room temperature, and the mixture was reacted at 70 °C overnight under a nitrogen atmosphere. After the reaction was completed, the reaction mixture was cooled to room temperature, and ethyl acetate (50 mL) was added to the reaction mixture. The mixture was washed sequentially with diluted brine (20 mL × 3) and saturated brine (50 mL). The organic phase was dried over anhydrous sodium sulfate and filtered, and the filtrate was concentrated under reduced pressure. The crude product was purified by a flash silica gel column (methanol/dichloromethane: 0-10%) to give compound 83-3 (100 mg, yield: 34.93%), ES-API: [M+H]⁺ = 371.0/373.0.

Step 4: Compound 81-7 (187.0 mg, 0.2698 mmol) and compound 83-3 (100.0 mg, 0.2698 mmol) were dissolved in toluene (6.0 mL), 1,4-dioxane (2.0 mL), and water (2.0 mL), and potassium phosphate (171.7 mg, 0.810 mmol) and [1,1'-bis(di-tert-butylphosphino)ferrocene]dichloropalladium(II) (26.0 mg, 0.040 mmol) were added. The mixture was reacted at 60 °C for 5 h under a nitrogen atmosphere. After the reaction was completed, the reaction mixture was concentrated under reduced pressure, and the residue was purified by silica gel column chromatography (dichloromethane:methanol = 10:1) to give compound 83-4 (100.0 mg, 0.1166 mmol, yield: 43.3%). ES-API: [M+H]⁺ = 858.4.

Step 5: Compound 83-4 (100.0 mg, 0.1166 mmol) was dissolved in N,N-dimethylformamide (5.0 mL), and cesium carbonate (190.0 mg, 0.583 mmol) and iodoethane (182.0 mg, 1.165 mmol) were added. The mixture was reacted at room temperature for 5 h. After the reaction was completed, water (20 mL) was added, and the mixture was extracted with ethyl acetate (50 mL × 3). The organic phase was washed with saturated brine (20 mL), dried over anhydrous sodium sulfate, and concentrated under reduced pressure to give compound 83-5 (110.0 mg, crude product). ES-API: [M+H]⁺ = 886.3.

Step 6: Compound 83-5 (110.0 mg, crude product) was dissolved in methanol (5.0 mL), and a solution of hydrochloric acid in dioxane (10 mL, 10.0 mmol) was added. The mixture was reacted at room temperature for 2 h. The reaction mixture was concentrated under reduced pressure to give compound 83-6 (130.0 mg, crude product). ES-API: [M+H]⁺ = 786.4.

Step 7: Compound 83-6 (91.96 mg, 0.117 mmol) was dissolved in N,N-dimethylformamide (5.0 mL), and (1r,2R,3S)-2,3-dimethylcyclopropane-1-carboxylic acid (16.03 mg, 0.140 mmol), O-(7-azabenzotriazol-1-yl)-N,N,N',N'-tetramethyluronium hexafluorophosphate (133.46 mg, 0.351 mmol), and *N*,*N*'-diisopropylethylamine (0.122 mL, 0.702 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was subjected to prep-HPLC (ammonium bicarbonate method) to give (1r,2R,3S)-N-((6³R,4R,Z)-1²-(5-(2-(4-oxa-7-azaspiro[2.5]octan-7-yl)ethoxy)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z83, 20.0 mg, 0.023 mmol, yield: 19.38%) as a white solid. ES-API: [M+H]⁺ = 882.3.

### Example 19. Synthesis of Compounds Z373 and Z376

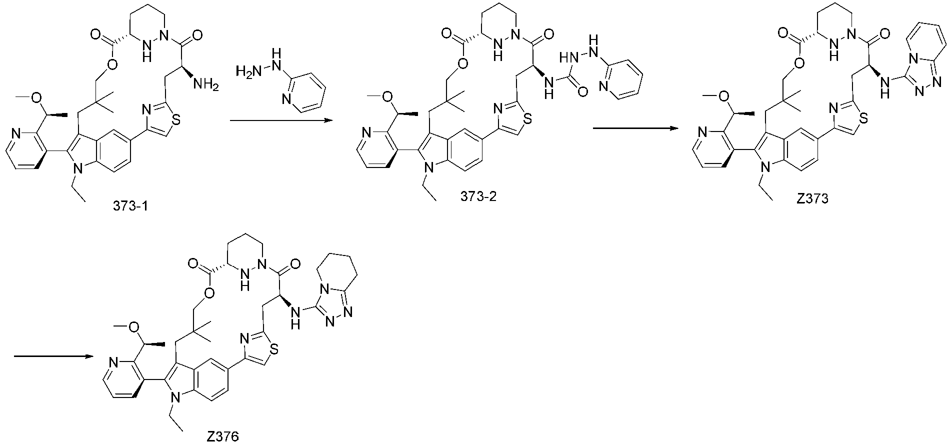

Step 1: Compound 373-1 (100 mg, 0.16 mmol, see intermediate 2 in WO2022060836A1 for the preparation method) and N,N-diisopropylethylamine (62 mg, 0.48 mmol) were dissolved in dichloromethane (8 mL), and a solution of p-nitrophenyl chloroformate (48 mg, 0.24 mmol) in dichloromethane (1 mL) was added dropwise at 0 °C. The reaction system was stirred for 1 h under an ice bath, and then 2-hydrazineylpyridine (52 mg, 0.48 mmol) was added. The reaction system was stirred at room temperature for 18 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (methanol/dichloromethane: 0-7%) to give compound 372-2 (90 mg, yield: 74.2%) as a yellow solid. ES-API: [M+H]⁺ = 766.3.

Step 2: Compound 372-2 (55 mg, 0.072 mmol) and pyridine (79 mg, 1.08 mmol) were dissolved in toluene (3 mL) and phosphorus oxychloride (33 mg, 0.215 mmol). The reaction system was stirred at 75 °C for 3 h. The reaction mixture was cooled to room temperature, and saturated sodium bicarbonate (10 mL) was added. The mixture was extracted with methanol/dichloromethane = 10:1 (30 mL × 3). The organic phases were mixed, dried over anhydrous sodium sulfate, and concentrated. The crude product was subjected to preparative silica gel thin-layer chromatography (dichloromethane/7 M ammonia-methanol solution = 20:1) to give the desired product (6³S,4S,Z)-4-([1,2,4]triazolo[4,3-a]pyridin-3-ylamino)-1¹-ethyl-(Rₐ)-1²-(2-((S)-1-methoxyethyl)pyridin-3-yl)-10,10-dimethyl-6¹,6²,6³,6⁴,6⁵,6⁶-hexahydro-11H-8-oxa-2(4,2)-thiazola-1(5,3)-indola-6(1,3)-pyridazinacycloundecaphane-5,7-dione (Z373, 10 mg, yield: 18.6%) as a pale yellow solid. ES-API: [M+H]⁺ = 748.3.

Step 3: Compound Z373 (9 mg, 0.012 mmol) was dissolved in absolute methanol (3 mL), and 10% palladium on carbon (15 mg) was added. The reaction system was stirred at room temperature for 48 h under a hydrogen atmosphere. The reaction mixture was filtered through celite and washed with methanol, and the filtrate was concentrated. The crude solid was washed with a small amount of petroleum ether to give the desired product (6³S,4S,Z)-1¹-ethyl-(Rₐ)-1²-(2-((S)-1-methoxyethyl)pyridin-3-yl)-10,10-dimethyl-4-((5,6,7,8-tetrahydro-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z376, 4 mg, yield: 44.2%) as a white solid. ES-API: [M+H]⁺ = 752.3.

### Example 20. Synthesis of Compound Z347

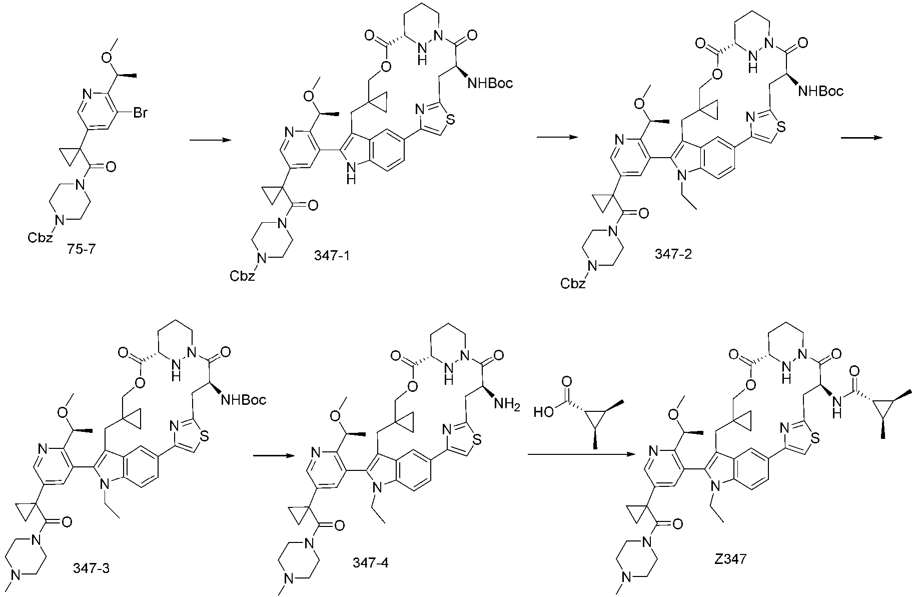

Step 1: Compound 75-1 (87.17 mg, 0.173 mmol) was dissolved in dioxane (1 mL), toluene (0.3 mL), and water (0.3 mL). Intermediate 2 (60 mg, 0.087 mmol), [1,1'-bis(di-tert-butylphosphino)ferrocene]dichloropalladium(II) (11.20 mg, 0.017 mmol), and potassium phosphate (55.24 mg, 0.260 mmol) were added to the solution described above at room temperature. After the addition was completed, the reaction system was stirred at 70 °C for 2 h under an argon atmosphere. After the reaction was completed, water (10 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 347-1 (60 mg, 0.061 mmol, yield: 70.06%) as a yellow solid. ES-API: [M+H]⁺ = 987.3.

Step 2: Compound 347-1 (60 mg, 0.061 mmol) was dissolved in N,N-dimethylformamide (3 mL), and iodoethane (94.80 mg, 0.608 mmol) and cesium carbonate (99.01 mg, 0.304 mmol) were added to the solution described above. The reaction system was stirred at room temperature for 8 h. After the reaction was completed, water (30 mL) was added to the solution described above, and the mixture was extracted with ethyl acetate (20 mL × 2). The organic phase was washed with saturated brine (30 mL × 3), dried, and concentrated to give a crude product. The crude product was purified by a flash silica gel column (methanol/dichloromethane: 0%-3%) to give compound 347-2 (55 mg, 0.054 mmol, yield: 89.13%) as a yellow solid. ES-API: [M+H]⁺ = 1015.4.

Step 3: Compound 347-2 (55 mg, 0.054 mmol) was dissolved in methanol (3 mL), and palladium hydroxide (10 wt%, 55 mg, 0.039 mmol) and paraformaldehyde (16.25 mg, 0.524 mmol on a formaldehyde equivalent basis) were added to the solution described above. After being purged three times with hydrogen, the reaction system was reacted for 17 h under a hydrogen atmosphere (15 psi). The reaction mixture was filtered through celite and concentrated to give a crude product. The crude product was purified by a flash silica gel column (methanol/dichloromethane: 0%-4%) to give compound 347-3 (45 mg, 0.050 mmol, yield: 92.80%) as a yellow solid. ES-API: [M+H]⁺ = 895.3.

Step 4: Compound 347-3 (45 mg, 0.050 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction mixture was concentrated to give crude compound 347-4 (40 mg) as a yellow solid. ES-API: [M+H]⁺ = 795.3.

Step 5: Compound 347-4 (40 mg, crude product) was dissolved in dichloromethane (5 mL). (1r,2R,3S)-2,3-Dimethylcyclopropane-1-carboxylic acid (8.61 mg, 0.075 mmol), O-(7-azabenzotriazol-1-yl)-N,N,N',N'-tetramethyluronium hexafluorophosphate (28.70 mg, 0.075 mmol), and N,N'-diisopropylethylamine (19.51 mg, 0.151 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1*r*,2*R*,3*S*)-*N*-((3'*S*,4'*S*,*Z*)-1'-ethyl-2'-(2-(1-methoxyethyl)-5-(1-(4-methylpiperazine-1-carbonyl)cyclopropyl)pyridin-3-yl)-5',7'-dioxospiro[cyclopropane-1,10'-8-oxa-2(4,2)-thiazola-1(5,3)-indola-6(1,3)-pyridazinacycloundecaphan]-4-yl)-2,3-dimethylcyclopropane-1-carboxamide (Z347, 20 mg, 0.022 mmol, two-step yield: 44%) as a white solid. ES-API: [M+H]⁺ = 891.3. ¹H NMR (400 MHz, DMSO-d₆) δ 8.64 - 8.51 (m, 2H), 8.42 - 8.34 (m, 1H), 7.84 - 7.78 (m, 1H), 7.77-7.70 (m, 1H), 7.66 - 7.50 (m, 2H), 5.54 - 5.47 (m, 1H), 5.04 - 4.95(m, 1H), 4.35 - 4.03 (m, 4H), 4.01 - 3.78 (m, 2H), 3.75 -.3.75 (m, 4H), 3.25 - 3.08 (m, 3H), 3.06 -2.98 (m, 3H), 2.93 - 2.67 (m, 2H), 2.32 - 2.18 (m, 4H), 2.13 - 1.98 (m, 2H), 1.86 - 1.71 (m, 3H), 1.54 - 1.35 (m, 5H), 1.30 - 1.23 (m, 6H), 1.19 - 1.14 (m, 3H), 1.11 -1.06 (m, 6H), 1.03 - 0.94 (m, 2H), 0.48 - 0.37 (m, 1H), 0.34 - 0.20 m, 2H), -0.35 - -0.80 (m, 1H).

### Example 21. Synthesis of Compounds Z374 and Z375

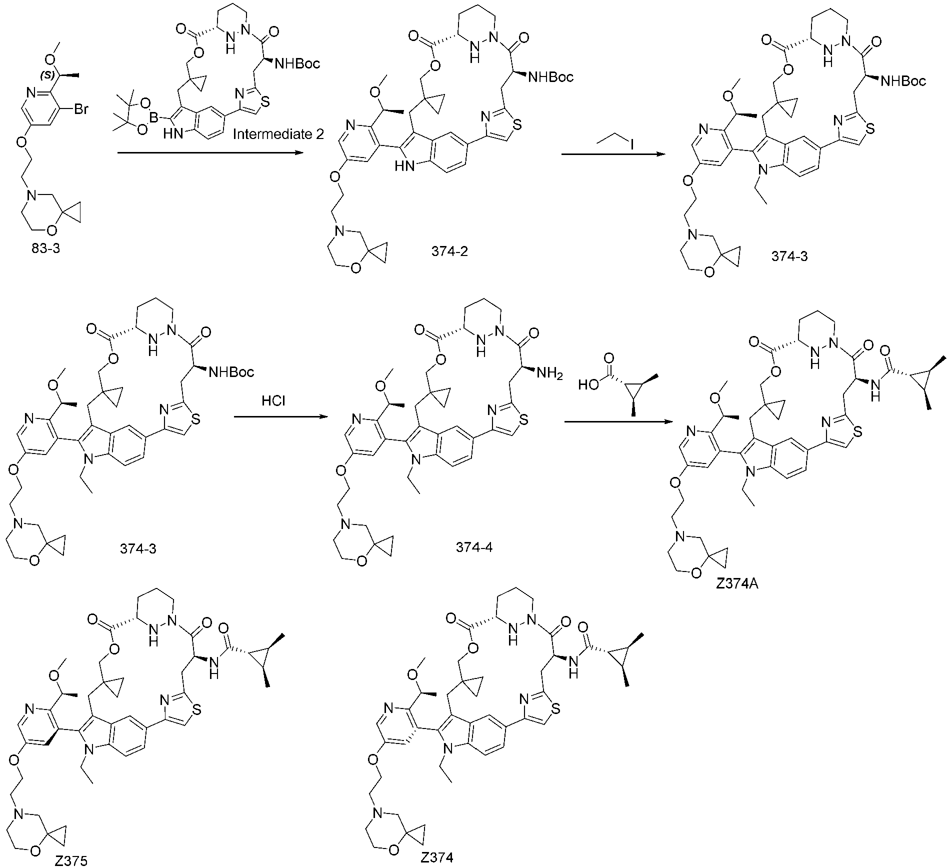

Step 1: Intermediate 2 (200.0 mg, 0.289 mmol) and compound 83-3 (214.0 mg, 0.578 mmol) were dissolved in toluene (6.0 mL), 1,4-dioxane (2.0 mL), and water (2.0 mL), and potassium phosphate (171.7 mg, 0.810 mmol) and [1,1'-bis(di-tert-butylphosphino)ferrocene]dichloropalladium(II) (26.0 mg, 0.040 mmol) were added. The mixture was reacted at 60 °C for 5 h under a nitrogen atmosphere. After the reaction was completed, the reaction mixture was concentrated under reduced pressure, and the residue was purified by silica gel column chromatography (dichloromethane:methanol = 10:1) to give compound 374-2 (160.0 mg, 0.187 mmol, yield: 64.67%). ES-API: [M+H]⁺ = 856.4.

Step 2: Compound 374-2 (120.0 mg, 0.140 mmol) was dissolved in N,N-dimethylformamide (5.0 mL), and cesium carbonate (228.4 mg, 0.701 mmol) and iodoethane (218.64 mg, 1.402 mmol) were added. The mixture was reacted at room temperature for 5 h. After the reaction was completed, water (40 mL) was added, and the mixture was extracted with ethyl acetate (80 mL × 3). The organic phase was washed with saturated brine (60 mL), dried over anhydrous sodium sulfate, filtered, and concentrated under reduced pressure to give compound 374-3 (110.0 mg, 0.124 mmol, yield: 88.76%). ES-API: [M+H]⁺ = 884.4.

Step 3: Compound 374-3 (110.0 mg, 0.124 mmol) was dissolved in methanol (5.0 mL), and a solution of hydrochloric acid in dioxane (10 mL, 10.0 mmol) was added. The mixture was reacted at room temperature for 2 h. The reaction mixture was concentrated under reduced pressure to give compound 374-4 (110.0 mg, crude product). ES-API: [M+H]⁺ = 784.3.

Step 4: Compound 374-4 (97.21 mg, 0.124 mmol) was dissolved in N,N-dimethylformamide (5.0 mL), and (1*r*,2*R*,3*S*)-2,3-dimethylcyclopropane-1-carboxylic acid (17.0 mg, 0.149 mmol), O-(7-azabenzotriazol-1-yl)-N,N,N',N'-tetramethyluronium hexafluorophosphate (133.46 mg, 0.351 mmol), and N,N'-diisopropylethylamine (0.122 mL, 0.702 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give crude Z374A. The crude product was purified by column chromatography [THF/PE = 0-50% (V/V)] and resolved by an SFC chiral column (chromatographic column: CHIRALPAK^{®} IB 250 × 4.6 mm, 5 µm; mobile phase A: HEX + 0.2% DEA, mobile phase B: ETOH + 0.2% DEA; detection wavelength: 254 nm; flow rate: 1 mL/min; injection volume: 10 µL; column temperature: 30 °C; run time: 15 min; isocratic elution program: mobile phase A:mobile phase B = 50:50 (V/V)) to give 2 isomeric compounds. One of the isomeric compounds was arbitrarily designated as: (1r,2R,3S)-N-((3'S,4'S,Z)-(Rₐ)-2'-(5-(2-(4-oxa-7-azaspiro[2.5]octan-7-yl)ethoxy)-2-((S)-1-methoxyethyl)pyridin-3-yl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z375, 13.09 mg, 0.015 mmol, yield: 11.99%, retention time: 8.455 min) as a white solid. ES-API: [M+H]⁺ = 880.4. ¹H NMR (400 MHz, DMSO-d₆) δ 8.53 (s, 1H), 8.44-8.45 (m, 1H), 8.36-8.38 (m, 1H), 7.80 (s, 1H), 7.72-7.74 (m, 1H), 7.55-7.57 (m, 1H), 7.43-7.45(m, 1H), 5.48-5.53 (m, 1H), 4.99-5.02 (m, 1H), 4.21-4.28 (m, 4H), 4.08-4.13 (m, 3H), 3.86-3.92 (m, 1H), 3.66 - 3.55 (m, 2H), 3.23 - 3.09 (m, 2H), 3.02 (s, 3H), 2.69-2.72(m, 3H), 2.53 (s, 2H), 2.44 (s, 2H), 2.01-2.05 (m, 1H), 1.88-1.91 (m, 1H), 1.71-1.76 (m, 2H), 1.46-1.49 (m, 1H), 1.34-1.37(m, 4H), 1.26 - 0.99 (m, 15H), 0.60-0.62 (m, 2H), 0.40-0.44 (m, 3H), 0.20-0.30 (m, 2H), -0.65 (s, 1H).

The other isomeric compound was arbitrarily designated as: (1r,2R,3S)-N-((3'S,4'S,Z)-(Sₐ)-2'-(5-(2-(4-oxa-7-azaspiro[2.5]octan-7-yl)ethoxy)-2-((S)-1-methoxyethyl)pyridin-3-yl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z374, 12.39 mg, 0.025 mmol, yield: 20.52%, retention time: 4.804 min) as a white solid. ES-API: [M+H]⁺ = 880.4. ¹H NMR (400 MHz, DMSO-d₆) δ 8.56 (s, 1H), 8.46-8.48(m, 1H), 8.39-8.40 (m, 1H), 7.81 (s, 1H), 7.73-7.75(m, 1H), 7.51-7.56 (m, 2H), 5.49-5.54 (m, 1H), 4.97-5.00 (m, 1H), 4.31 - 3.75 (m, 8H), 3.67 - 3.57 (m, 2H), 3.43-3.47 (m, 1H), 3.24-3.25 (m, 1H), 3.11-3.15 (m, 1H), 2.98 (s, 3H), 2.72-2.75 (m, 3H), 2.53-2.56 (m, 2H), 2.45 (s, 2H), 2.07-2.09 (m, 1H), 1.87-1.91 (m, 1H), 1.76-1.79 (m, 2H), 1.47-1.52 (m, 1H), 1.26-1.29 (m, 4H), 1.21 - 1.01 (m, 12H), 0.60-0.63 (m, 2H), 0.48 - 0.39 (m, 3H), 0.30-0.33 (m, 2H), -0.23--0.25 (m, 1H).

### Example 22. Synthesis of Compounds Z350, Z379, and Z380

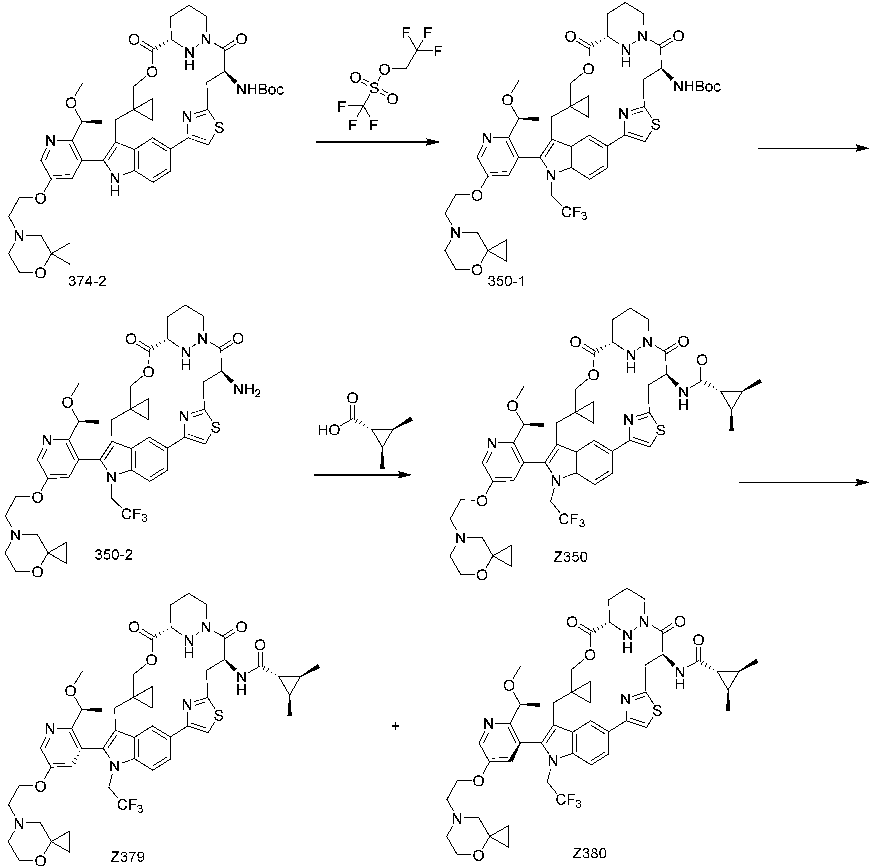

Step 1: Compound 374-2 (30 mg, 0.035 mmol) was dissolved in N,N-dimethylformamide (1 mL). 2,2,2-Trifluoroethyl trifluoromethanesulfonate (81.34 mg, 0.350 mmol) and cesium carbonate (57.09 mg, 0.175 mmol) were added to the solution described above. The reaction system was stirred at room temperature for 8 h. After the reaction was completed, water (5 mL) was added to the solution described above, and the mixture was extracted with ethyl acetate (5 mL × 2). The organic phase was washed with saturated brine (5 mL × 3), dried over anhydrous sodium sulfate, filtered, and concentrated to give a crude product. The crude product was purified by a flash silica gel column (methanol/dichloromethane: 0%-3%) to give compound 350-1 (30 mg, 0.032 mmol, yield: 91.27%) as a yellow solid. ES-API: [M+H]⁺ = 938.1.

Step 2: Compound 350-1 (30 mg, 0.032 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction mixture was concentrated to give compound 350-2 (30 mg, crude product) as a yellow solid. ES-API: [M+H]⁺ = 838.1.

Step 3: Crude compound 350-2 (30 mg) was dissolved in dichloromethane (2 mL). (1r,2R,3S)-2,3-Dimethylcyclopropane-1-carboxylic acid (5.52 mg, 0.048 mmol), O-(7-azabenzotriazol-1-yl)-*N*,*N*,*N*',*N*'-tetramethyluronium hexafluorophosphate (18.38 mg, 0.048 mmol), and *N*,*N*'-diisopropylethylamine (12.5 mg, 0.097 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) and then purified by preparative thin-layer chromatography (silica, dichloromethane:methanol = 10:1, v/v) to give (1*r*,2*R*,3*S*)-*N*-((3'*S*,4'*S*,*Z*)-2'-(5-(2-(4-oxa-7-azaspiro[2.5]octan-7-yl)ethoxy)-2-((S)-1-methoxyethyl)pyridin-3-yl)-5',7'-dioxo-1'-(2,2,2-trifluoroethyl)spiro[cyclopropane-1,10'-8-oxa-2(4,2)-thiazola-1(5,3)-indola-6(1,3)-pyridazinacycloundecaphan]-4-yl)-2,3-dimethylcyclopropane-1-carboxamide (Z350, 16 mg, 0.017 mmol, two-step yield: 53%) as a white solid. ES-API: [M+H]⁺ = 934.1.

Step 4: Compound Z350 (16 mg, 0.017 mmol) was subjected to chiral resolution (chromatographic column: CHIRALPAK^{®}IB 250 mm, 4.6 mm, 5 µm; mobile phase system: (A: n-hexane; B: ethanol); flow rate: 1 mL/min; B% = 0-30%; column temperature: 30 °C) to give 2 isomeric compounds. The structure of one of the isomeric compounds was arbitrarily designated as (1*r*,2*R*,3*S*)-N-((3'*S*,4'*S*,*Z*)-(*S*ₐ)-2'-(5-(2-(4-oxa-7-azaspiro[2.5]octan-7-yl)ethoxy)-2-((S)-1-methoxyethyl)pyridin-3-yl)-5',7'-dioxo-1'-(2,2,2-trifluoroethyl)spiro[cyclopropane-1,10'-8-oxa-2(4,2)-thiazola-1(5,3)-indola-6(1,3)-pyridazinacycloundecaphan]-4-yl)-2,3-dimethylcyclopropane-1-carboxamide (Z379, 6 mg, 0.006 mmol, yield: 37.5%, retention time: 4.007 min) as a white solid. ES-API: [M+H]⁺ = 934.1.

The other isomeric compound was arbitrarily designated as (1*r*,2*R*,3*S*)-*N*-((3'*S*,4'*S*,*Z*)-(*R*ₐ)-2'-(5-(2-(4-oxa-7-azaspiro[2.5]octan-7-yl)ethoxy)-2-((S)-1-methoxyethyl)pyridin-3-yl)-5',7'-dioxo-1'-(2,2,2-trifluoroethyl)spiro[cyclopropane-1,10'-8-oxa-2(4,2)-thiazola-1(5,3)-indola-6(1,3)-pyridazinacycloundecaphan]-4-yl)-2,3-dimethylcyclopropane-1-carboxamide (Z380, 3 mg, 0.006 mmol, yield: 18.8%, retention time: 8.516 min) as a white solid. ES-API: [M+H]⁺ = 934.1.

### Example 23. Synthesis of Compound Z329

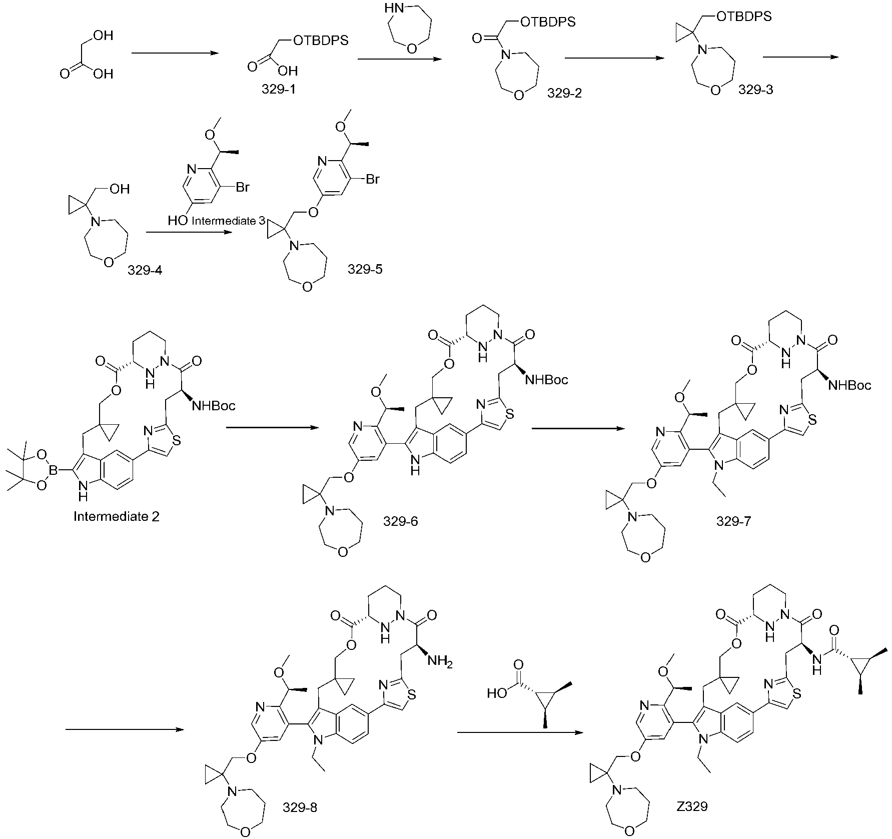

Step 1: Glycolic acid (2.00 g, 26.3 mmol), 4-dimethylaminopyridine (318 mg, 2.62 mmol), and triethylamine (10.9 mL, 116.8 mmol) were dissolved in tetrahydrofuran (50 mL), and tert-butyldiphenylchlorosilane (7.6 mL, 25.0 mmol) was added at 0 °C. The mixture was reacted at room temperature for 16 h. Diluted hydrochloric acid (1 M) was added to adjust the pH to 1, and the mixture was extracted with ethyl acetate (50 mL × 3), washed with water (50 mL × 2) and saturated brine (50 mL), dried over anhydrous sodium sulfate, and purified by silica gel column chromatography (petroleum ether:ethyl acetate = 2:1) to give compound 329-1 (7 g, 22.260 mmol, yield: 84.64%). ES-API: [M+NH₄]⁺ = 332.0.

Step 2: Compound 329-1 (1.5 g, 4.770 mmol) was dissolved in dichloromethane (15 mL), and 1-oxa-4-azepane (0.48 g, 4.770 mmol), O-(7-azabenzotriazol-1-yl)-N,N,N',N'-tetramethyluronium hexafluorophosphate (2.72 g, 7.155 mmol), and N,N-diisopropylethylamine (3.08 g, 23.850 mmol) were added at 0 °C. The mixture was reacted at room temperature for 16 h. The reaction mixture was washed with water (10 mL × 2) and saturated brine (10 mL), dried over anhydrous sodium sulfate, and purified by silica gel column chromatography (petroleum ether:ethyl acetate = 3:1) to give compound 329-2 (1.5 g, 3.773 mmol, yield: 78.95%).

Step 3: Compound 329-2 (1.23 g, 3.094 mmol) was dissolved in tetrahydrofuran (15 mL), and tetraisopropyl titanate (1.832 mL, 6.187 mmol) and ethylmagnesium bromide (6 mL, 12 mmol) were added at 0 °C under a nitrogen atmosphere. The mixture was reacted at room temperature for 3 h. Saturated ammonium chloride (10 mL) was added to the reaction mixture, and the mixture was extracted with ethyl acetate (50 mL × 3), dried over anhydrous sodium sulfate, filtered, and concentrated. The residue was purified by silica gel column chromatography (petroleum ether:ethyl acetate = 5:1) to give compound 329-3 (0.5 g, 1.221 mmol, yield: 39.45%). ES-API: [M+H]⁺ = 410.2.

Step 4: Compound 329-3 (400 mg, 0.976 mmol) was dissolved in tetrahydrofuran (5 mL), and a 1 M solution of tetrabutylammonium fluoride in tetrahydrofuran (1.562 mL, 1.562 mmol) was added. The mixture was reacted at room temperature for 3 h. The reaction mixture was concentrated under reduced pressure, and the residue was purified by silica gel column chromatography (petroleum ether:ethyl acetate = 1:1) to give compound 329-4 (120 mg, 0.701 mmol, yield: 71.77%). ES-API: [M+H]⁺ = 172.1.

Step 5: Intermediate 3 (100 mg, 0.431 mmol) was dissolved in dichloromethane (5 mL), and compound 329-4 (110.68 mg, 0.646 mmol), triphenylphosphine (203.43 mg, 0.776 mmol), and diisopropyl azodicarboxylate (174.26 mg, 0.862 mmol) were added at 0 °C under a nitrogen atmosphere. The mixture was reacted at room temperature for 3 h. Saturated ammonium chloride (5 mL) was added to the reaction mixture, and the mixture was extracted with ethyl acetate (10 mL × 3), dried over anhydrous sodium sulfate, filtered, and concentrated. The residue was purified by silica gel column chromatography (petroleum ether:ethyl acetate = 3:1) to give compound 329-5 (60 mg, 0.156 mmol, yield: 35.29%). ES-API: [M+H]⁺ = 385.1. Step 6: Intermediate 2 (60 mg, 0.086 mmol) and compound 329-5 (60 mg, 0.156 mmol) were dissolved in toluene (1.5 mL), 1,4-dioxane (0.5 mL), and water (0.5 mL), and potassium phosphate (55.24 mg, 0.260 mmol) and [1,1'-bis(di-tert-butylphosphino)ferrocene]dichloropalladium(II) (8.47 mg, 0.013 mmol) were added. The mixture was reacted at 60 °C for 5 h under a nitrogen atmosphere. After the reaction was completed, the reaction mixture was concentrated under reduced pressure, and the residue was purified by silica gel column chromatography (dichloromethane:methanol = 10:1) to give compound 329-6 (40 mg, 0.046 mmol, yield: 63.59%). ES-API: [M+H]⁺ = 870.1.

Step 7: Compound 329-6 (40 mg, 0.046 mmol, 63.59%) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 5 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 329-7 (40 mg, 0.045 mmol, yield: 80.00%). ES-API: [M+H]⁺ = 898.3. Step 8: Compound 329-7 (20 mg, 0.022 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 329-8 (15 mg, 0.019 mmol, yield: 84.41%). ES-API: [M+H]⁺ = 798.3. Step 9: Compound 329-8 (15 mg, 0.019 mmol) was dissolved in dichloromethane (3 mL), and (1r,2R,3S)-2,3-dimethylcyclopropane-1-carboxylic acid (4.83 mg, 0.042 mmol), O-(7-azabenzotriazol-1-yl)-N,N,N',N'-tetramethyluronium hexafluorophosphate (18.57 mg, 0.049 mmol), and N,N'-diisopropylethylamine (0.016 mL, 0.098 mmol) were added. The mixture was reacted with stirring at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by preparative thin-layer chromatography (silica, dichloromethane:methanol = 10:1, v/v) to give (1r,2R,3S)-N-((3'S,4'S,Z)-2'-(5-((1-(1,4-oxazepan-4-yl)cyclopropyl)methoxy)-2-((S)-1-methoxyethyl)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z329, 12 mg, 0.013 mmol, yield: 69.32%) as a white solid. ES-API: [M+H]⁺ = 894.3.

### Example 24. Synthesis of Compounds Z367 and Z377

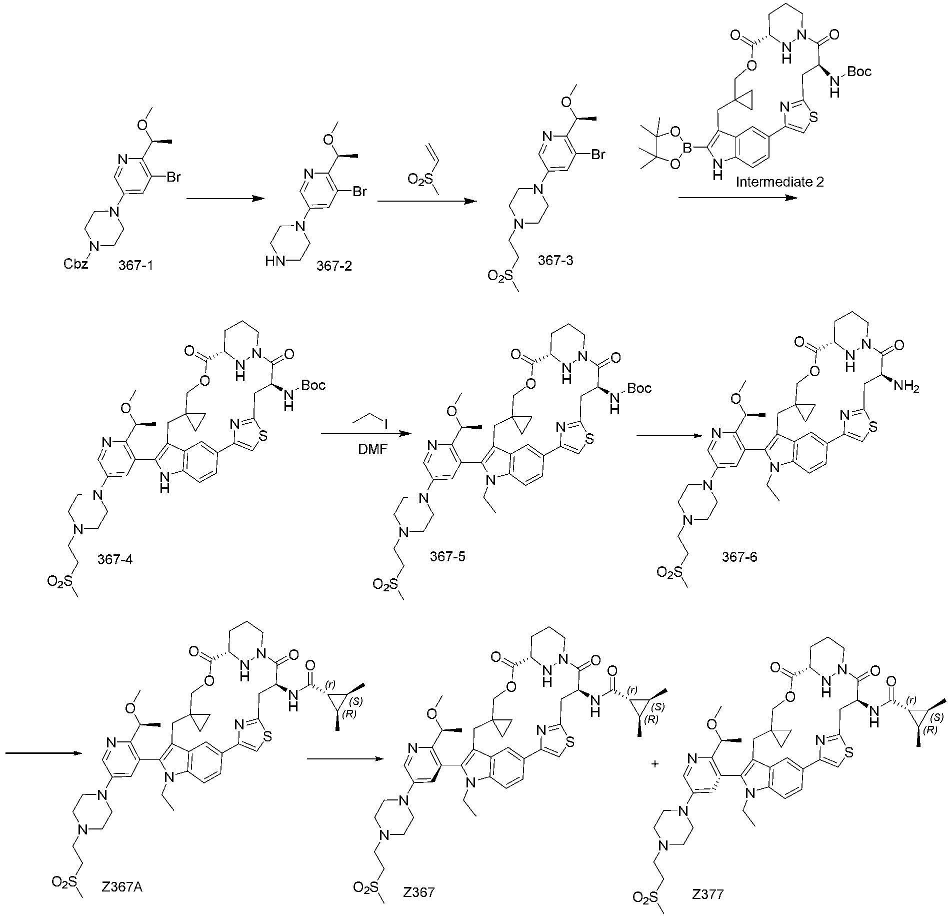

Step 1: Compound 367-1 (150 mg, 0.345 mmol) was dissolved in dry dichloromethane (6 mL), and iodotrimethylsilane (345 mg, 1.72 mmol) was slowly added at room temperature. The mixture was stirred at room temperature for 3 h. After the reaction was completed, ice water (30 mL) was added, and the mixture was extracted with dichloromethane (30 mL × 3). The organic phase was dried over anhydrous sodium sulfate, filtered, and concentrated, and the residue was purified by silica gel column chromatography (dichloromethane:methanol = 0-10%) to give compound 367-2 (40 mg, 0.13 mmol, yield: 40%). ES-API [M+H]⁺ = 300.1/302.1.

Step 2: Compound 367-2 (40 mg, 0.13 mmol) was dissolved in dry tetrahydrofuran (2 mL), and methyl vinyl sulfone (141 mg, 1.33 mmol) was added. The mixture was stirred overnight at 70 °C under a nitrogen atmosphere. After the reaction was completed, the reaction mixture was concentrated, and the residue was purified by silica gel column chromatography (petroleum ether:ethyl acetate = 0-50%) to give compound 367-3 (40 mg, 0.1 mmol, yield: 74%). ES-API [M+H]⁺ = 406.1/408.1.

Step 3: A mixture of compound 367-3 (40 mg, 0.1 mmol), intermediate 2 (100 mg, 0.14 mmol), [1,1'-bis(di-tert-butylphosphino)ferrocene]dichloropalladium(II) (12.5 mg, 0.02 mmol), and potassium phosphate (61 mg, 0.29 mmol) was stirred at 60 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-6% methanol/dichloromethane) to give compound 367-4 (60 mg, 0.07 mmol, yield: 68%). ES-API [M+H]⁺ = 891.3.

Step 4: Compound 367-4 (60 mg, 0.07 mmol) was dissolved in N,N-dimethylformamide (3 mL), and cesium carbonate (142 mg, 0.43 mmol) and iodoethane (136 mg, 0.87 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 367-5 (60 mg, 0.06 mmol, yield: 97%). ES-API [M+H]⁺ = 919.3.

Step 5: Compound 367-5 (60 mg, 0.07 mmol) was dissolved in a solution of hydrochloric acid in dioxane (5 mL, 4 mol/L). The mixture was stirred at room temperature for 2 h. After the reaction was completed, the reaction mixture was concentrated to give compound 367-6 (53 mg, yield: 100%). ES-API [M+H]⁺ = 819.3.

Step 6: Compound 367-6 (53 mg, 0.05 mmol) was dissolved in N,N-dimethylformamide (3 mL), and (1r,2R,3S)-2,3-dimethylcyclopropane-1-carboxylic acid (5.1 mg, 0.05 mmol), N,N-diisopropylethylamine (35 mg, 0.27 mmol), and O-(7-azabenzotriazol-1-yl)-N,N,N',N'-tetramethyluronium hexafluorophosphate (51 mg, 0.14 mmol) were sequentially added. The mixture was stirred at room temperature for 1 h. After the reaction was completed, ethyl acetate (10 mL) was added, and the mixture was washed with saturated brine (10 mL) and water (10 mL). The organic phase was dried over anhydrous sodium sulfate, filtered, and concentrated to give crude Z367A, which was purified by prep-HPLC (ammonium bicarbonate method) to give 2 isomeric compounds. One of the isomeric compounds was arbitrarily designated as (1r,2R,3S)-N-((3'S,4'S,Z)-1'-ethyl-(Rₐ)-2'-(2-((S)-1-methoxyethyl)-5-(4-(2-(methylsulfonyl)ethyl)piperazin-1-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z367, 11 mg, yield: 27%, purity: 98%, retention time: 7.159 min). ES-API [M+H]⁺ = 915.3.

The other isomeric compound was arbitrarily designated as: (1r,2R,3S)-N-((3'S,4'S,Z)-1'-ethyl-(Sₐ)-2'-(2-((S)-1-methoxyethyl)-5-(4-(2-(methylsulfonyl)ethyl)piperazin-1-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z377, 9 mg, yield: 22%, purity: 98%, retention time: 7.379 min). ES-API [M+H]⁺ = 915.3.

### Example 25. Synthesis of Compounds Z378-1 and Z378-2

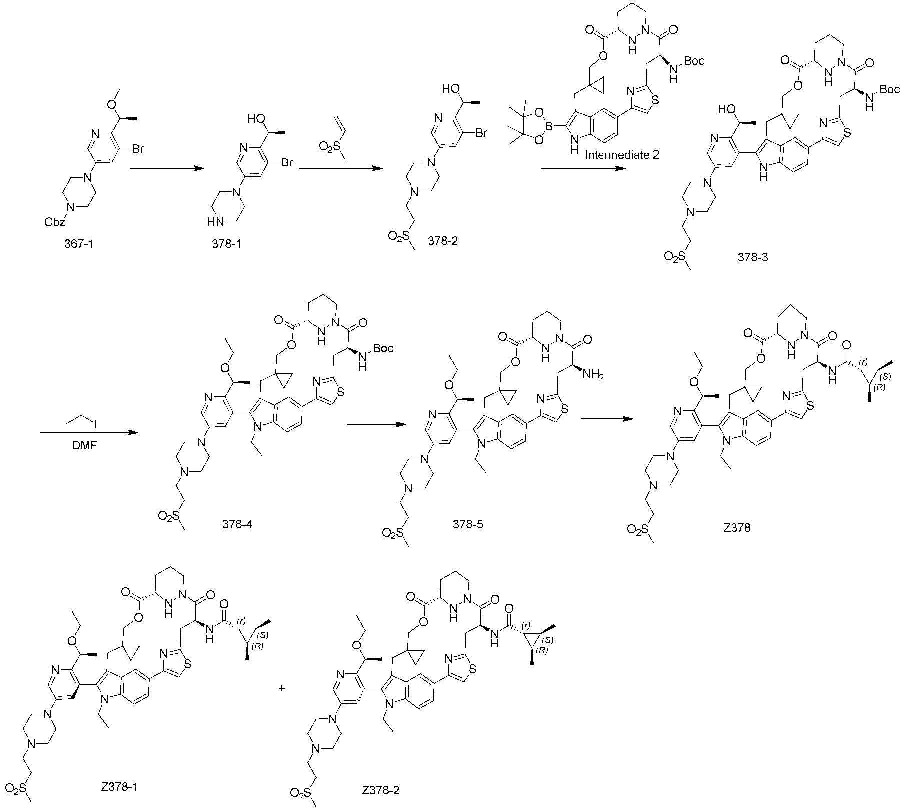

Step 1: Compound 367-1 (150 mg, 0.345 mmol) was dissolved in dry dichloromethane (6 mL), and iodotrimethylsilane (345 mg, 1.72 mmol) was slowly added at room temperature. The mixture was stirred at room temperature for 3 h. After the reaction was completed, 30 mL of ice water was added, and the mixture was extracted three times with 30 mL of dichloromethane. The organic phase was dried over anhydrous sodium sulfate, filtered, and concentrated, and the residue was then purified by silica gel column chromatography (dichloromethane:methanol = 0-10%) to give compound 378-1 (40 mg, 0.13 mmol, yield: 40%). ES-API [M+H]⁺ = 300.1/302.1.

Step 2: Compound 378-1 (40 mg, 0.13 mmol) was dissolved in dry tetrahydrofuran (2 mL), and methyl vinyl sulfone (141 mg, 1.33 mmol) was added. The mixture was stirred overnight at 70 °C under a nitrogen atmosphere. After the reaction was completed, the mixture was concentrated, and the residue was purified by silica gel column chromatography (petroleum ether:ethyl acetate = 0-50%) to give compound 378-2 (40 mg, 0.1 mmol, yield: 74%). ES-API [M+H]⁺ = 406.1/408.1.

Step 3: A mixture of compound 378-2 (40 mg, 0.1 mmol), intermediate 2 (100 mg, 0.14 mmol), [1,1'-bis(di-tert-butylphosphino)ferrocene]dichloropalladium(II) (12.5 mg, 0.02 mmol), and potassium phosphate (61 mg, 0.29 mmol) was stirred at 60 °C for 18 h in dioxane (9 mL), toluene (3 mL), and water (3 mL) under an oil bath and a nitrogen atmosphere. After the reaction was completed, the mixture was poured into 30 mL of ethyl acetate and washed sequentially with 15 mL of water and 15 mL of saturated brine. The organic phase was dried over anhydrous sodium sulfate, filtered, and concentrated, and the residue was purified by a flash silica gel column (0-6% methanol/dichloromethane) to give compound 378-3 (60 mg, 0.07 mmol, yield: 68%). ES-API [M+H]⁺ = 891.3.

Step 4: Compound 378-3 (60 mg, 0.07 mmol) was dissolved in N,N-dimethylformamide (3 mL), and cesium carbonate (142 mg, 0.43 mmol) and iodoethane (136 mg, 0.87 mmol) were sequentially added. The mixture was stirred at room temperature for 16 h. After the reaction was completed, 20 mL of ethyl acetate was added, and the mixture was washed sequentially with 10 mL of saturated brine and 10 mL of diluted brine. The organic phase was dried over anhydrous sodium sulfate, filtered, and concentrated, and the residue was purified by a flash silica gel column (0-60% tetrahydrofuran/petroleum ether) to give compound 378-4 (60 mg, 0.06 mmol, yield: 97%). ES-API [M+H]⁺ = 919.3.

Step 5: Compound 378-4 (60 mg, 0.07 mmol) was dissolved in a solution of hydrochloric acid in dioxane (5 mL, 4 mol/L). The mixture was stirred at room temperature for 2 h. After the reaction was completed, the reaction mixture was concentrated to give compound 378-5 (53 mg, yield: 100%). ES-API [M+H]⁺ = 819.3.

Step 6: Compound 378-5 (53 mg, 0.05 mmol) was dissolved in N,N-dimethylformamide (3 mL), and (1r,2R,3S)-2,3-dimethylcyclopropane-1-carboxylic acid (5.1 mg, 0.05 mmol), N,N-diisopropylethylamine (35 mg, 0.27 mmol), and O-(7-azabenzotriazol-1-yl)-N,N,N',N'-tetramethyluronium hexafluorophosphate (51 mg, 0.14 mmol) were sequentially added. The mixture was stirred at room temperature for 1 h. After the reaction was completed, 10 mL of ethyl acetate was added, and the mixture was washed with 10 mL of saturated brine and 10 mL of water. The organic phase was dried over anhydrous sodium sulfate, filtered, and concentrated to give crude Z378, which was purified by prep-HPLC (ammonium bicarbonate) to give 2 isomeric compounds. One of the structures was arbitrarily designated as: (1r,2R,3S)-N-((3'S,4'S,Z)-1'-ethyl-(Rₐ)-2'-(2-((S)-1-ethoxyethyl)-5-(4-(2-(methylsulfonyl)ethyl)piperazin-1-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z378-1, 2 mg, yield: 5%, purity: 98%, retention time: 7.344 min). ES-API [M+H]⁺ = 929.3. The other structure was arbitrarily designated as: (1r,2R,3S)-N-((3'S,4'S,Z)-1'-ethyl-(Sₐ)-2'-(2-((S)-1-ethoxyethyl)-5-(4-(2-(methylsulfonyl)ethyl)piperazin-1-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z378-2, 3 mg, yield: 7%, purity: 98%, retention time: 7.604 min). ES-API [M+H]⁺ = 929.3.

### Example 26. Synthesis of Compound Z456

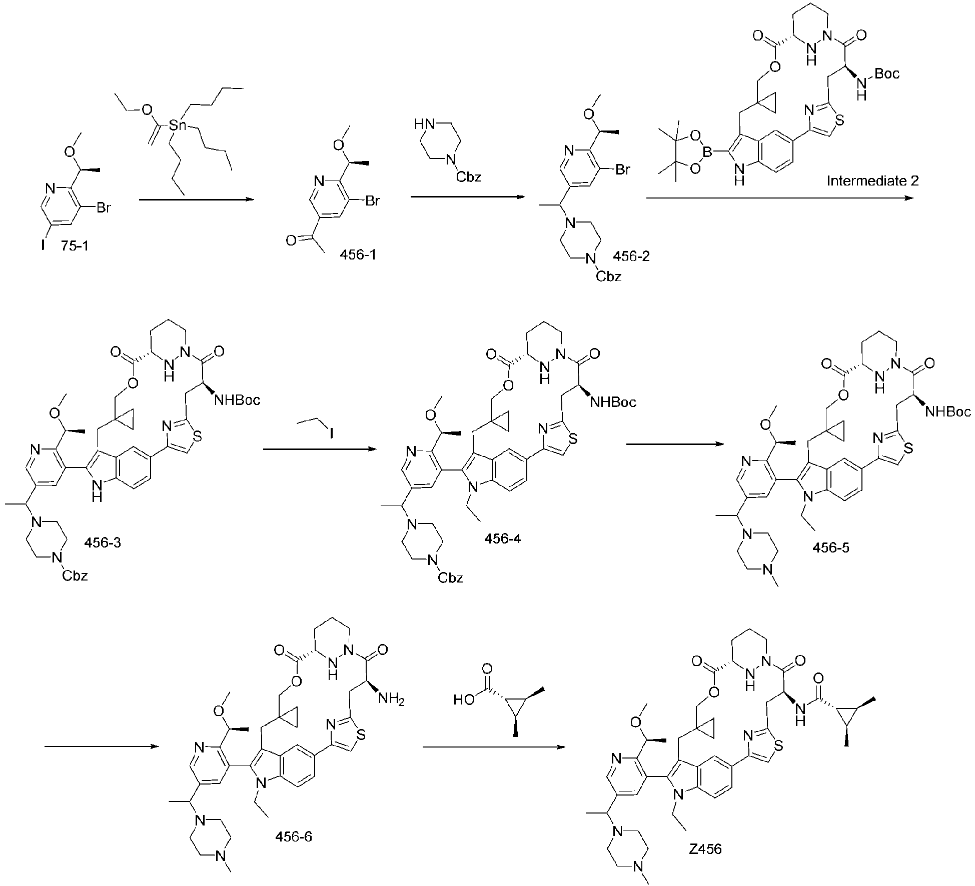

Step 1: Bis(triphenylphosphine)palladium(II) dichloride (51 mg, 0.073 mmol) was added to a solution of compound 75-1 (2.5 g, 7.311 mmol) and tributyl(1-ethoxyvinyl)stannane (2.64 g, 7.311 mmol) in anhydrous N,N-dimethylformamide (20 mL) under a nitrogen atmosphere. The reaction mixture was stirred at 85 °C for 5 h. The reaction was completed as detected by LCMS. The reaction mixture was cooled to room temperature, and petroleum ether (40 mL) and a cesium fluoride solution (1.4 g of cesium fluoride dissolved in 40 mL of water) were added to the reaction mixture. The reaction mixture was rapidly stirred for 0.5 h and filtered to remove insoluble substances. Ethyl acetate (50 mL) was added to the filtrate, and the mixture was washed with a saturated sodium bicarbonate solution (30 mL) and brine (30 mL) and concentrated under reduced pressure to give a residue. The residue was dissolved in tetrahydrofuran (20 mL) and 2 M diluted hydrochloric acid (20 mL), and the mixture was rapidly stirred at room temperature for 15 min and concentrated under reduced pressure to remove the solvent tetrahydrofuran. The residue was extracted with dichloromethane (50 mL × 3). The organic phases were combined and concentrated to dryness under reduced pressure. The residue was purified by automated flash chromatography on silica gel (tetrahydrofuran:petroleum ether = 0-12%) to give compound 456-1 (1.386 g, yield: 73%). ES-API: [M+ H]⁺ = 258.0.

Step 2: Sodium cyanoborohydride (146 mg, 2.325 mmol) was added to a solution of compound 456-1 (500 mg, 1.937 mmol), benzyl piperazine-1-carboxylate (512 mg, 2.325 mmol), and titanium tetraisopropanolate (1101 mg, 3.874 mmol) in tetrahydrofuran (15 mL) at room temperature. The reaction mixture was heated to 70 °C and stirred for 5 h. When the reaction was completed as detected by LCMS, the mixture was cooled to room temperature. Water (30 mL) and ethyl acetate (30 mL) were added to the reaction mixture, and the organic phase was separated, dried over anhydrous sodium sulfate, filtered, and concentrated. The residue was purified by automated flash chromatography on silica gel (methanol/dichloromethane = 0-1.7%) to give crude compound 456-2 (320 mg, yield: 18%, purity: 50%). ES-API: [M+H]⁺ = 462.1.

Step 3: Intermediate 2 (64 mg, 0.093 mmol), compound 456-2 (129 mg, 0.279 mmol), potassium phosphate (59 mg, 0.278 mmol), and [1,1'-bis(di-tert-butylphosphino)ferrocene]dichloropalladium(II) (12 mg, 0.019 mmol) were dissolved in dioxane (2 mL), toluene (6 mL), and water (2 mL) under a nitrogen atmosphere. The reaction mixture was heated to 70 °C under an oil bath and stirred for 2 h. When the reaction was completed as detected by LCMS, the mixture was cooled to room temperature. 30 mL of ethyl acetate was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-80%) to give compound 456-3 (36 mg, yield: 41%). ES-API: [M+H]⁺ = 947.3.

Step 4: Cesium carbonate (124 mg, 0.380 mmol) and iodoethane (178 mg, 1.140 mmol) were sequentially added to a solution of compound 456-3 (36 mg, 0.038 mmol) in N,N-dimethylformamide (3 mL). The reaction mixture was stirred at room temperature for 2 h. When the reaction was completed as detected by LCMS, the reaction mixture was diluted with 20 mL of ethyl acetate and washed with water (10 mL) and saturated brine (15 mL × 3). The organic phase was dried over anhydrous sodium sulfate, filtered, and concentrated to give crude compound 456-4 (37 mg, yield: 99%). ES-API: [M+H]⁺ = 975.3.

Step 5: Palladium hydroxide (50 mg, 0.356 mmol) and paraformaldehyde (30 mg, 1.000 mmol) were added to a solution of compound 456-4 (37 mg, 0.038 mmol) in methanol (5 mL) under a hydrogen atmosphere. The reaction mixture was stirred at room temperature for 17 h under a hydrogen atmosphere. When the reaction was completed as detected by LCMS, the mixture was filtered, and the filtrate was concentrated under reduced pressure. The residue was purified by automated flash chromatography on silica gel (methanol/dichloromethane = 0-3%) to give compound 456-5 (9.4 mg, yield: 28%). ES-API: [M+H]⁺ = 855.4.

Step 6: Compound 456-5 (9.4 mg, 0.011 mmol) was dissolved in a solution of hydrochloric acid in dioxane (3 mL, 4 M) and a solution of hydrochloric acid in methanol (1 mL, 4 M). The reaction mixture was stirred at room temperature for 1 h. When the reaction was completed as detected by LCMS, the mixture was concentrated under reduced pressure to give crude compound 456-6 (8.3 mg, yield: 99%). ES-API: [M+H]⁺ = 755.3.

Step 7: (1r,2R,3S)-2,3-Dimethylcyclopropane-1-carboxylic acid (1.88 mg, 0.016 mmol), N,N-diisopropylethylamine (8.53 mg, 0.066 mmol), and O-(7-azabenzotriazol-1-yl)-N,N,N',N'-tetramethyluronium hexafluorophosphate (12.5 mg, 0.033 mmol) were sequentially added to a solution of compound 456-6 (8.3 mg, 0.011 mmol) in N,N-dimethylformamide (2 mL) under an ice-water bath.The reaction mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give a compound (3'R,4'R,Z)-1'-ethyl-2'-(2-((R)-1-methoxyethyl)-5-(1-(4-methylpiperazin-1-yl)ethyl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z456, 5.95 mg, yield: 64%). ES-API: [M+H]⁺ = 851.5.

### Example 27. Synthesis of Compound Z361

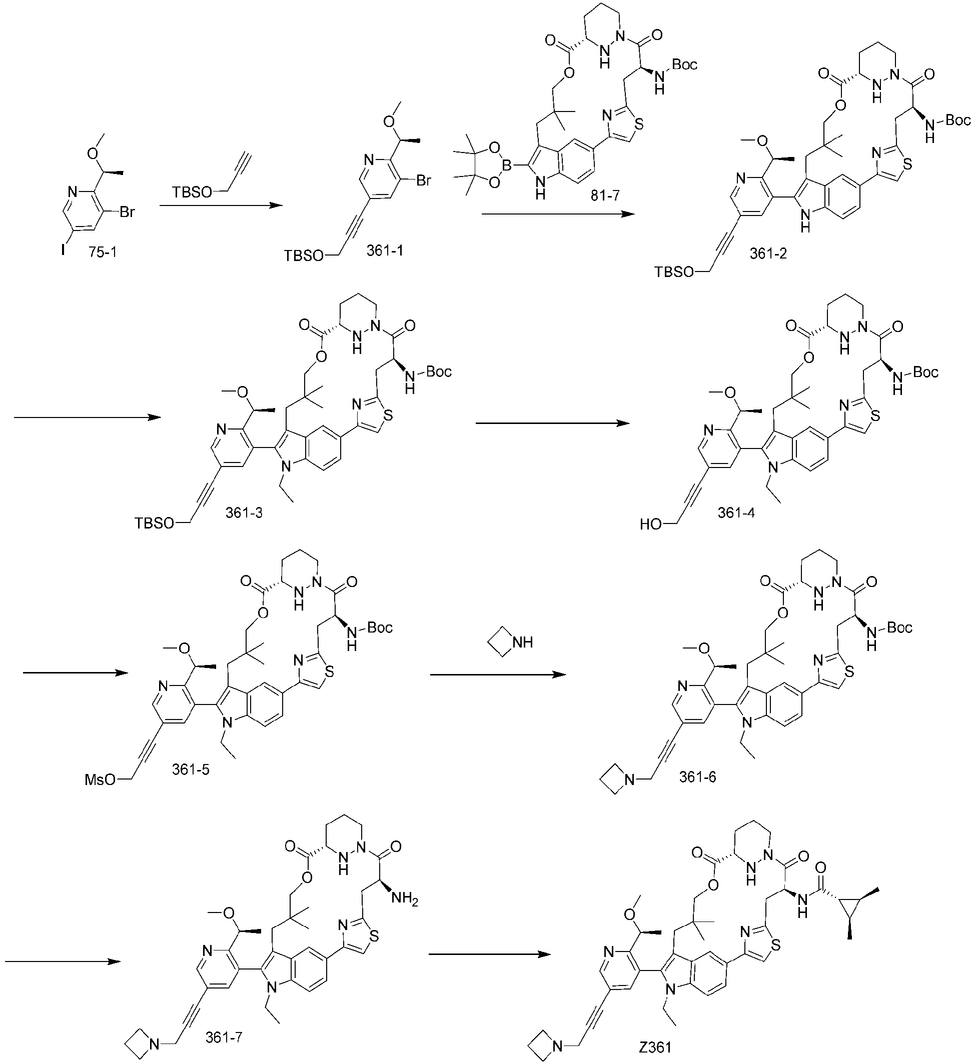

Step 1: tert-Butyldimethyl(2-propynyloxy)silane (2.09 g, 12.282 mmol) and compound 75-1 (4 g, 11.697 mmol) were dissolved in acetonitrile (20 mL), and DIPEA (3.02 g, 23.394 mmol), PdCl₂(PPh₃)₂ (0.41 g, 0.585 mmol), and copper(I) iodide (0.22 g, 1.170 mmol) were sequentially added. The mixture was reacted under an argon atmosphere. The reaction mixture was stirred at room temperature overnight, diluted with ethyl acetate, and filtered. The filtrate was concentrated by evaporation to remove the solvent, and the residue was purified by a flash silica gel column (gradient elution, 0-10% solution of EtOAc in heptane) to give compound 361-1 (4.3 g, 11.187 mmol, yield: 95.64%) as a pale yellow liquid. ES-API: [M+H]⁺ = 384.1/386.1.

Step 2: Compound 81-7 (100 mg, 0.144 mmol) and compound 361-1 (110.83 mg, 0.288 mmol) were dissolved in 1,4-dioxane (1.5 mL), toluene (0.5 mL), and water (0.5 mL), and then potassium phosphate (91.70 mg, 0.432 mmol) and Pd(dtbpf)Cl₂ (18.88 mg, 0.029 mmol) were added. The mixture was stirred at 65 °C overnight under an argon atmosphere. After the reaction was completed, the mixture was diluted with ethyl acetate, washed with brine, and concentrated to give a residue. The residue was purified by a flash silica gel column (0-70% ethyl acetate/petroleum ether) to give compound 361-2 (110 mg, 0.126 mmol, yield: 87.59%) as a yellow oil. ES-API [M+H]⁺ = 871.3.

Step 3: Compound 361-2 (110 mg, 0.126 mmol) was dissolved in DMF (10 mL), and then cesium carbonate (329.12 mg, 1.010 mmol) and iodoethane (0.202 mL, 2.525 mmol) were added. The mixture was stirred at room temperature overnight. After the reaction was completed, the mixture was diluted with ethyl acetate, washed with brine, and concentrated to give a residue. The residue was purified by a flash silica gel column (0-90% ethyl acetate/petroleum ether) to give compound 361-3 (100 mg, 0.111 mmol, yield: 88.07%) as a yellow oil. ES-API [M+H]⁺ = 899.4.

Step 4: Compound 361-3 (90 mg, 0.100 mmol) was dissolved in 3 mL of tetrahydrofuran. A TBAF solution (0.300 mL, 0.300 mmol) was added at 0 °C. The mixture was stirred at room temperature for 1 h. After the reaction was completed, the mixture was diluted with ethyl acetate, washed with water and brine, and concentrated to give a residue. The residue was purified by silica gel column chromatography (0-100% ethyl acetate/petroleum ether) to give compound 361-4 (51 mg, 0.065 mmol, yield: 64.92%) as a pale yellow oil. ES-API [M+H]⁺ = 785.3.

Step 5: Compound 361-4 (51 mg, 0.065 mmol) was dissolved in dichloromethane (3 mL), and DIPEA (0.057 mL, 0.325 mmol) and methanesulfonic anhydride (16.98 mg, 0.097 mmol) were added at 0 °C. The mixture was stirred at room temperature for 1 h. After the reaction was completed, the mixture was diluted with ethyl acetate, washed with brine, and concentrated to give compound 361-5 (52 mg, crude product) as a pale yellow oil. ES-API [M+H]⁺ = 863.3.

Step 6: Compound 361-5 (45 mg, 0.052 mmol) was dissolved in dichloromethane (5 mL), and then DIPEA (0.045 mL, 0.261 mmol) and azetidine (5.95 mg, 0.104 mmol) were added. The mixture was stirred at room temperature for 3 h. The reaction mixture was concentrated, and the residue was purified by flash column chromatography (0-10% methanol/dichloromethane) to give compound 361-6 (40 mg, 0.049 mmol, yield: 93.10%) as a yellow oil. ES-API [M+H]⁺ = 824.3.

Step 6: Compound 361-6 (40 mg, 0.045 mmol) was dissolved in anhydrous dichloromethane (0.3 mL), and trifluoroacetic acid (1.5 mL) was added at 0 °C. The reaction system was stirred at room temperature for 2 h. The reaction mixture was concentrated, and dichloromethane (50 mL) was added. The mixture was washed sequentially with saturated sodium bicarbonate (10 mL) and saturated brine (10 mL), dried over anhydrous sodium sulfate, filtered, and concentrated to give compound 361-7 (20 mg, crude product) as a yellow liquid. ES-API: [M+H]⁺ = 724.4.

Step 7: Compound 361-7 (20 mg, crude product) and (1r,2R,3S)-2,3-dimethylcyclopropane-1-carboxylic acid were dissolved in dichloromethane (5 mL), and HATU (15.76 mg, 0.041 mmol) and ethyldiisopropylamine (0.014 mL, 0.083 mmol) were sequentially added. The reaction system was stirred at room temperature for 2 h. The reaction system was diluted with water (20 mL) and extracted with ethyl acetate (10 mL × 3). The organic layer was separated, washed with an additional saturated sodium chloride solution, and concentrated in vacuum. The residue was purified by a flash silica gel column (elution by a 0% to 5% solution of methanol in dichloromethane) and then subjected to prep-HPLC (formic acid method 1) to give (1r,2R,3S)-N-((6³S,4S,Z)-1²-(5-(3-(azetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide formate (Z361, 1.67 mg, yield: 7.22%) as a white solid. ES-API: [M+H]⁺ = 820.4.

### Example 28. Synthesis of Compound Z457

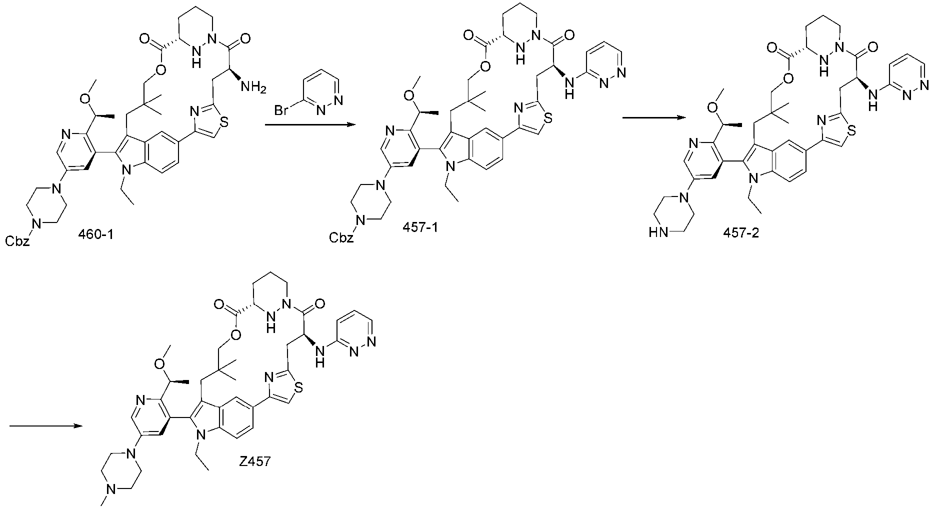

Step 1: Compound 460-1 (65 mg, 0.077 mmol) and 3-bromo-1,2-diazine (60.86 mg, 0.383 mmol) were dissolved in toluene (2 mL), and methanesulfonato(2-(di-t-butylphosphino)-3,6-dimethoxy-2',4',6'-tri-i-propyl-1,1'-biphenyl)(2'-amino-1,1'-biphenyl-2-yl)palladium(II) (6.55 mg, 0.008 mmol) and sodium tert-butoxide (14.72 mg, 0.153 mmol) were added. The mixture was reacted at 110 °C for 2 h under a nitrogen atmosphere. After the reaction was completed, the mixture was concentrated to dryness under reduced pressure, and the residue was purified by thin-layer chromatography (dichloromethane:methanol = 10:1) to give compound 457-1 (50 mg, 0.035 mmol, yield: 45.79%). ES-API: [M+H]⁺ = 927.1. Step 2: Compound 457-1 (50 mg, 0.054 mmol) was dissolved in methanol (4 mL), and palladium hydroxide (50 mg, 0.356 mmol) and paraformaldehyde (40 mg, 1.111 mmol) were added. The mixture was reacted at room temperature for 18 h under a hydrogen atmosphere. After the reaction was completed, the mixture was filtered under vacuum and concentrated under reduced pressure. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give compound 457-2 (40 mg, 0.05 mmol, yield: 92%). ES-API: [M+H]⁺ = 793.3.

Step 3: Compound 457-2 (15 mg, 0.019 mmol) was dissolved in methanol (2 mL), and an aqueous formaldehyde solution (12.6 mg, 0.38 mmol), formic acid (8.70 mg, 0.189 mmol), and sodium cyanoborohydride (2.15 mg, 0.057 mmol) were added. The mixture was reacted at room temperature for 1 h. After the reaction was completed, the mixture was concentrated under reduced pressure, and water (5 mL) was added. The mixture was extracted with ethyl acetate (10 mL × 3). The organic phase was washed with water (5 mL) and saturated brine (5 mL), dried over anhydrous sodium sulfate, filtered, and concentrated under reduced pressure. The residue was subjected to prep-HPLC (ammonium bicarbonate method) to give (6³*S*,4*S*,*Z*)-1¹-ethyl-(*Rₐ*)-1²-(2-((*S*)-1-methoxyethyl)-5-(4-methylpiperazin-1-yl)pyridin-3-yl)-10,10-dimethyl-4-(pyridazin-3-ylamino)-6¹,6²6³,6⁴,6⁵,6⁶-hexahydro-1¹H-8-oxa-2(4,2)-thiazola-1(5,3)-indola-6(1,3)-pyridazinacycloundecaphane-5,7-dione (Z457, 4.4 mg, 0.005 mmol, yield: 26.3%). ES-API: [M+H]⁺ = 807.3.

### Example 29. Synthesis of Compound Z458

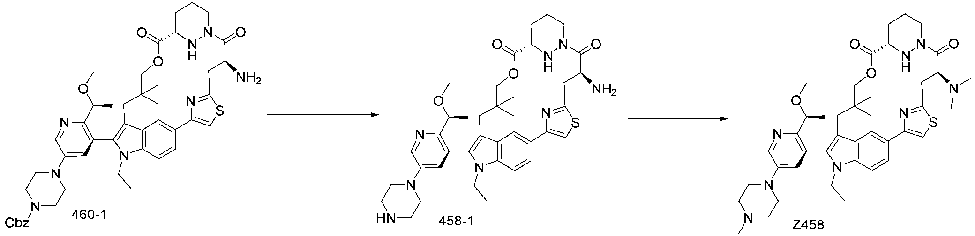

Step 1: Compound 460-1 (40 mg, 0.047 mmol) was dissolved in methanol (4 mL), and palladium hydroxide (50 mg, 0.356 mmol) and paraformaldehyde (40 mg, 1.111 mmol) were added. The mixture was reacted at room temperature for 18 h under a hydrogen atmosphere. After the reaction was completed, the mixture was filtered under vacuum and concentrated under reduced pressure. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give compound 458-1 (40 mg, 0.041 mmol, yield: 88%). ES-API: [M+H]⁺ = 715.3.

Step 3: Compound 458-1 (13.52 mg, 0.019 mmol) was dissolved in methanol (2 mL), and an aqueous formaldehyde solution (12.6 mg, 0.38 mmol), formic acid (8.70 mg, 0.189 mmol), and sodium cyanoborohydride (2.15 mg, 0.057 mmol) were added. The mixture was reacted at room temperature for 1 h.After the reaction was completed, the mixture was concentrated under reduced pressure, and water (5 mL) was added. The mixture was extracted with ethyl acetate (10 mL × 3). The organic phase was washed with water (5 mL) and saturated brine (5 mL), dried over anhydrous sodium sulfate, filtered, and concentrated under reduced pressure. The residue was purified by prep-HPLC (ammonium bicarbonate method) to give (6³*S*,4*S*,*Z*)-4-(dimethylamino)-1¹-ethyl-(*Rₐ*)-1²-(2-((*S*)-1-methoxyethyl)-5-(4-methylpiperazin-1-yl)pyridin-3-yl)-10,10-dimethyl-4-(pyridazin-3-ylamino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z458, 3.4 mg, 0.004 mmol, yield: 23.74%). ES-API: [M+H]⁺ = 757.3.

### Example 30. Synthesis of Compound Z460

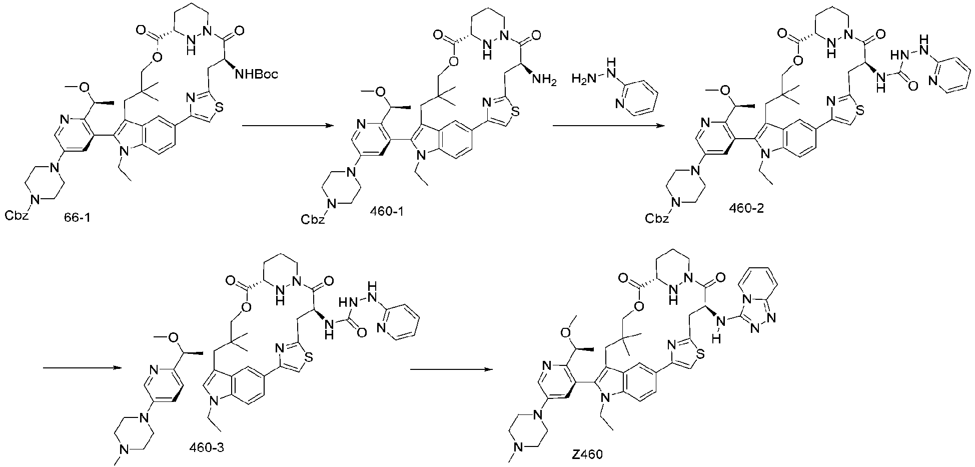

Step 1: Compound 66-1 (500 mg, 0.53 mmol) was dissolved in absolute methanol (2 mL), and a 4.0 M hydrogen chloride/dioxane solution (8 mL) was added at 0 °C. The reaction system was stirred at room temperature for 2 h. The reaction mixture was concentrated, and saturated sodium bicarbonate (15 mL) was added. The mixture was extracted with dichloromethane (40 mL × 2). The organic phases were mixed, dried over anhydrous sodium sulfate, filtered, and concentrated to give the desired product of compound 460-1 (447 mg, yield: 100%) as a pale yellow solid. ES-API: [M+H]⁺ = 849.3.

Step 2: Compound 460-1 (250 mg, 0.29 mmol) and N,N-diisopropylethylamine (114 mg, 0.88 mmol) were dissolved in dichloromethane (10 mL), and a solution of p-nitrophenyl chloroformate (77 mg, 0.38 mmol) in dichloromethane (1 mL) was added dropwise at 0 °C. The reaction system was stirred for 1 h under an ice bath, and then 2-hydrazineylpyridine (96 mg, 0.88 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (tetrahydrofuran/petroleum ether: 0-100%) to give compound 460-2 (240 mg, yield: 83.0%) as a pale yellow solid. ES-API: [M+H]⁺ = 984.4.

Step 3: Compound 460-2 (85 mg, 0.086 mmol) and paraformaldehyde (117 mg, 1.30 mmol) were dissolved in absolute methanol (6 mL), and 10% palladium hydroxide on carbon (100 mg) was added. The reaction system was stirred at room temperature for 40 h under a hydrogen atmosphere. The reaction mixture was filtered through celite and washed with methanol, and the filtrate was concentrated. The crude product was purified by a flash silica gel column (7 M ammonia-methanol solution/dichloromethane: 0-10%) to give compound 460-3 (55 mg, yield: 73.7%) as a pale yellow solid. ES-API: [M+H]⁺ = 864.4.

Step 4: Compound 460-3 (50 mg, 0.058 mmol) and pyridine (69 mg, 0.87 mmol) were dissolved in toluene (3 mL), and phosphorus oxychloride (45 mg, 0.29 mmol) was slowly added. The reaction system was stirred at 75 °C for 1 h. The reaction mixture was cooled to room temperature, and saturated sodium bicarbonate (10 mL) was added. The mixture was extracted with methanol/dichloromethane = 10:1 (30 mL × 3). The organic phases were mixed, dried over anhydrous sodium sulfate, filtered, and concentrated. The crude product was subjected to preparative silica gel thin-layer chromatography (dichloromethane/7 M ammonia-methanol solution = 20:1) to give the desired product (6³S,4S,Z)-4-([1,2,4]triazolo[4,3-a]pyridin-3-ylamino)-1¹-ethyl-(Rₐ)-1²-(2-((S)-1-methoxyethyl)-5-(4-methylpiperazin-1-yl)pyridin-3-yl)-10-10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z460, 1.5 mg, yield: 3.1%) as a pale yellow solid. ES-API: [M+H]⁺ = 846.3.

### Example 31. Synthesis of Compounds Z339 and Z339-1

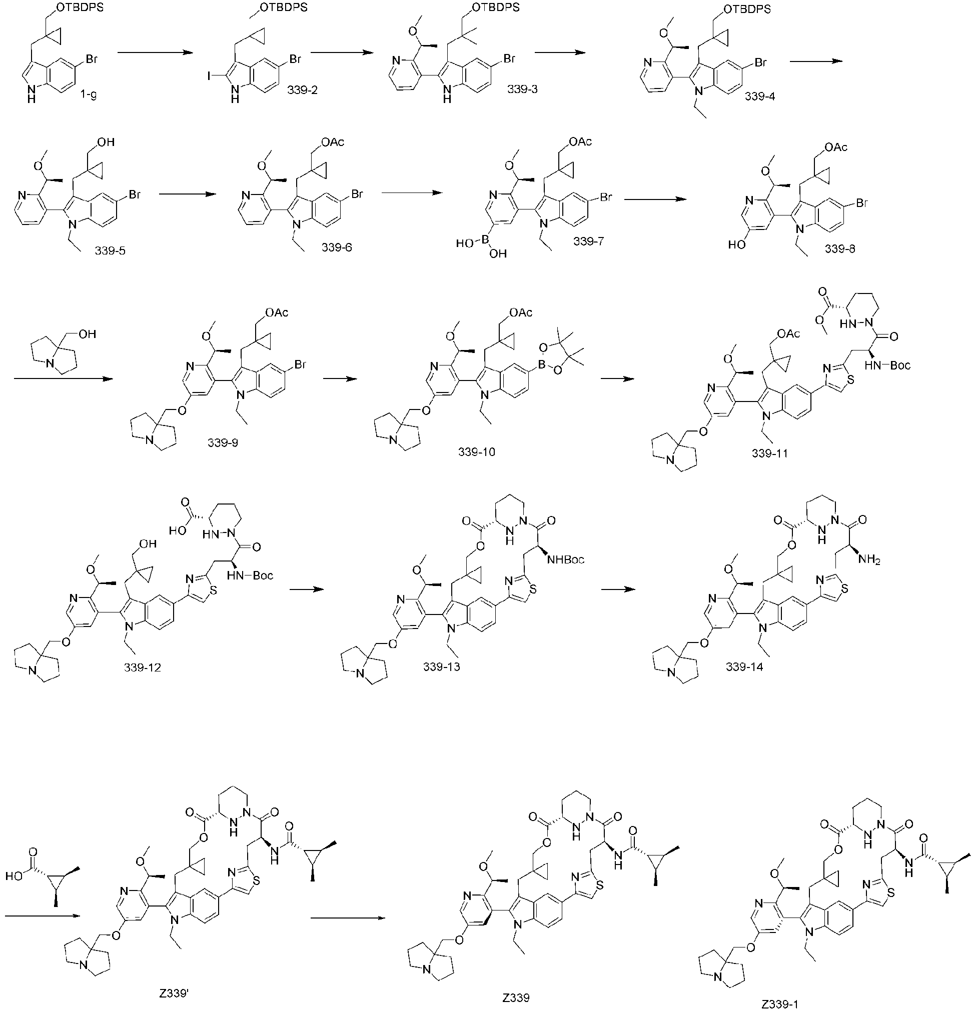

Step 1: Compound 1-g (3 g, 5.785 mmol) was dissolved in tetrahydrofuran (50 mL), and silver trifluoromethanesulfonate (1.78 g, 6.942 mmol) was added. The mixture was cooled to 0 °C. Elemental iodine (1.47 g, 5.785 mmol) was slowly added dropwise to the suspension described above. After the dropwise addition was completed, the reaction system was warmed to room temperature and stirred for 2 h. After the reaction was completed, water (100 mL) was added to the solution described above, and the mixture was extracted with ethyl acetate (100 mL × 2). The organic phase was washed with saturated brine (100 mL), dried over anhydrous sodium sulfate, filtered, and concentrated to give a crude product. The crude product was purified by a flash silica gel column (ethyl acetate/petroleum ether: 0%-5%) to give compound 339-2 (3.5 g, 5.431 mmol, yield: 93.88%) as a light yellow solid. ES-API: [M+H]⁺ = 644.1, 646.3.

Step 2: Compound 339-2 (3000 mg, 4.655 mmol) was dissolved in dioxane (30 mL), toluene (10 mL), and water (10 mL). (S)-2-(1-Methoxyethyl)-3-(4,4,5,5-tetramethyl-1,3,2-dioxaborolan-2-yl)pyridine (1224.91 mg, 4.655 mmol), [1,1'-bis(diphenylphosphino)ferrocene]palladium(II) dichloride (340.61 mg, 0.465 mmol), and potassium phosphate (2470.17 mg, 11.637 mmol) were added to the solution described above at room temperature. After the addition was completed, the reaction system was stirred at 60 °C for 16 h under an argon atmosphere. After the reaction was completed, water (50 mL) was added to the solution described above, and the mixture was extracted with ethyl acetate (60 mL × 3). The organic phase was washed with saturated brine (100 mL), dried over anhydrous sodium sulfate, filtered, and concentrated to give a crude product. The crude product was purified by a flash silica gel column (ethyl acetate/petroleum ether: 0%-60%) to give compound 339-3 (1100 mg, 1.683 mmol, yield: 36.15%) as a yellow solid. ES-API: [M+H]⁺ = 653.1, 655.3.

Step 3: Compound 339-3 (1100 mg, 1.683 mmol) was dissolved in N,N-dimethylformamide (15 mL). Iodoethane (787.32 mg, 5.048 mmol) and cesium carbonate (1096.47 mg, 3.365 mmol) were added to the solution described above. The reaction system was stirred at room temperature for 8 h. After the reaction was completed, water (50 mL) was added to the solution described above, and the mixture was extracted with ethyl acetate (50 mL × 2). The organic phase was washed with saturated brine (100 mL × 3), dried over anhydrous sodium sulfate, filtered, and concentrated to give a crude product. The crude product was purified by a flash silica gel column (ethyl acetate/petroleum ether: 0%-45%) to give compound 339-4 (1100 mg, 1.613 mmol, yield: 95.89%) as a yellow solid. ES-API: [M+H]⁺ = 681.3, 683.3.

Step 4: Compound 339-4 (1100 mg, 1.613 mmol) was dissolved in a solution of tetrabutylammonium fluoride in tetrahydrofuran (1 M, 10 mL). The reaction system was stirred at 50 °C for 17 h under a nitrogen atmosphere. After the reaction was completed, water (50 mL) was added to the solution described above, and the mixture was extracted with ethyl acetate (50 mL × 2). The organic phase was washed with saturated brine (100 mL × 5), dried over anhydrous sodium sulfate, filtered, and concentrated to give a crude product. The crude product was purified by a flash silica gel column (ethyl acetate/petroleum ether: 0%-70%) to give compound 339-5 (650 mg, 1.466 mmol, yield: 90.86%) as a yellow oily liquid. ES-API: [M+H]⁺ = 442.1, 444.3.

Step 5: Compound 339-5 (650 mg, 1.466 mmol), triethylamine (0.408 mL, 2.932 mmol), and 4-dimethylaminopyridine (17.91 mg, 0.147 mmol) were dissolved in dichloromethane (20 mL). Acetic anhydride (1.652 mL, 17.592 mmol) was slowly added to the solution described above under an ice bath. After the addition was completed, the reaction system was warmed to room temperature and stirred for another 2 h. After the reaction was completed, water (50 mL) was added to the solution described above, and the mixture was extracted with dichloromethane (50 mL × 2). The organic phase was washed sequentially with a saturated aqueous sodium bicarbonate solution (100 mL × 5) and a saturated sodium chloride solution (100 mL), dried over anhydrous sodium sulfate, filtered, and concentrated to give a crude product. The crude product was purified by a flash silica gel column (ethyl acetate/petroleum ether: 0%-40%) to give compound 339-6 (650 mg, 1.339 mmol, yield: 91.34%) as a yellow solid, ES-API: [M+H]⁺ = 485.1, 487.3.

Step 6: Compound 339-6 (650 mg, 1.339 mmol), bis(pinacolato)diboron (578.06 mg, 2.276 mmol), chloro(1,5-cyclooctadiene)iridium(I) dimer (62.96 mg, 0.094 mmol), and 4,4'-di-tert-butyl-2,2'-bipyridine (50.32 mg, 0.187 mmol) were dissolved in n-hexane (15 mL). The reaction system was stirred at 80 °C for 17 h under a nitrogen atmosphere. After the reaction was completed, the reaction system was concentrated to remove n-hexane to give crude compound 339-7 (1340 mg) as a black oily liquid. ES-API: [M+H]⁺ = 529.1, 531.1.

Step 7: Compound 339-7 (1340 mg) was dissolved in acetonitrile (15 mL), and hydrogen peroxide (417.58 mg, 30 wt%, 3.685 mmol) was added to the solution described above under an ice bath. After the addition was completed, the reaction system was warmed to room temperature and stirred for 3 h. After the reaction was completed, the reaction was quenched with saturated sodium sulfite (20 mL). The mixture was extracted with ethyl acetate (20 mL × 2). The organic phase was washed with saturated brine (20 mL × 2), dried over anhydrous sodium sulfate, filtered, and concentrated to give a crude product. The crude product was purified by a flash silica gel column (methanol/dichloromethane: 0%-4%) to give compound 339-8 (500 mg, 0.997 mmol, two-step yield: 74.5%) as a yellow solid. ES-API: [M+H]⁺ = 501.1,503.1. ¹H NMR (400 MHz, DMSO-d₆) δ 10.22- 10.24 (m, 1H), 8.29 - 8.33 (m, 1H), 7.76 - 7.81 (m, 1H), 7.48 - 7.5 (m, 1H), 7.27 - 7.33 (m, 1H), 7.08 - 7.12 (m, 1H), 4.11 - 3.51 (m, 5H), 2.96 - 2.82 (m, 3H), 2.75 - 2.53 (m, 2H), 1.85-1.90 (m, 3H), 1.24 -1.34 (m, 3H), 1.13 - 1.03 (m, 3H), 0.52 - 0.07 (m, 4H).

Step 8: Compound 339-8 (220 mg, 0.439 mmol), triphenylphosphine (230.16 mg, 0.878 mmol), and (hexahydro-1H-pyrrolin-7a-yl)methanol (185.87 mg, 1.316 mmol) were dissolved in dichloromethane (5 mL), and the mixture was cooled to 0 °C. A solution of diisopropyl azodicarboxylate (266.16 mg, 1.316 mmol) in dichloromethane (1 mL) was slowly added dropwise to the solution described above under a nitrogen atmosphere. After the addition was completed, the reaction system was stirred at room temperature for another 2 h. After the reaction was completed, the reaction system was concentrated to give a crude product. The crude product was purified by a flash silica gel column (methanol/dichloromethane: 0%-5%) to give compound 339-9 (150 mg, 0.240 mmol, yield: 54.73%) as a light yellow solid. ES-API: [M+H]⁺ = 624.0, 626.0.

Step 9: Compound 339-9 (180 mg, 0.288 mmol) was dissolved in anhydrous toluene (5 mL), and bis(pinacolato)diboron (109.77 mg, 0.432 mmol), [1,1'-bis(diphenylphosphino)ferrocene]palladium(II) dichloride (21.09 mg, 0.029 mmol), and potassium acetate (56.56 mg, 0.576 mmol) were added to the solution described above at room temperature. After the addition was completed, the reaction system was stirred at 90 °C for 3 h under a nitrogen atmosphere. After the reaction was completed, the reaction mixture was filtered using a sand core funnel and concentrated. The residue was purified by a silica gel column (ethyl acetate/petroleum ether: 0-100%) to give compound 339-10 (140 mg, 0.208 mmol, yield: 72.33%) as a yellow oily liquid. ES-API: [M+H]⁺ = 672.4.

Step 10: Compound 339-10 (140 mg, 0.208 mmol) was dissolved in dioxane (3 mL), toluene (1 mL), and water (1 mL). Methyl (S)-1-((S)-3-(4-bromothiazol-2-yl)-2-((tert-butoxycarbonyl)amino)propanoyl)hexahydropyridazine-3-carboxylate (150 mg, 0.216 mmol), [1,1'-bis(di-tert-butylphosphino)ferrocene]dichloropalladium(II) (13.46 mg, 0.021 mmol), and potassium phosphate (110.60 mg, 0.521 mmol) were added to the solution described above at room temperature. After the addition was completed, the reaction system was stirred at 70 °C for 16 h under an argon atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 339-11 (150 mg, 0.159 mmol, yield: 76.38%) as a yellow solid. ES-API: [M+H]⁺ = 942.4.

Step 11: Compound 339-11 (150 mg, 0.159 mmol) was dissolved in methanol (2 mL) and water (2 mL). Lithium hydroxide (22.93 mg, 0.955 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (20 mL × 2). The organic phase was washed with saturated brine (20 mL), dried over anhydrous sodium sulfate, filtered, and concentrated to give compound 339-12 (140 mg, 0.158 mmol, yield: 99.24%) as a yellow solid. ES-API: [M+H]⁺ = 886.4.

Step 12: Compound 339-12 (140 mg, 0.158 mmol), 1-hydroxybenzotriazole (106.75 mg, 0.790 mmol), and N,N-diisopropylethylamine (0.826 mL, 4.740 mmol) were dissolved in dichloromethane (30 mL). 1-(3-Dimethylaminopropyl)-3-ethylcarbodiimide hydrochloride (848.04 mg, 4.424 mmol) was added to the solution described above under an ice bath. After the addition was completed, the reaction system was stirred at room temperature for 17 h. After the reaction was completed, the reaction mixture was washed sequentially with diluted hydrochloric acid (0.5 M, 10 mL × 2) and saturated brine (10 mL × 2). The organic phase was washed with saturated brine (20 mL), dried over anhydrous sodium sulfate, filtered, and concentrated to give a crude product. The crude product was purified by a flash silica gel column (methanol/dichloromethane: 0%-5%) to give compound 339-13 (40 mg, 0.046 mmol, yield: 29.16%) as a light yellow solid. ES-API: [M+H]⁺ = 868.3.

Step 13: Compound 339-13 (40 mg, 0.046 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give crude compound 339-14 (38 mg) as a yellow solid. ES-API: [M+H]⁺ = 768.4.

Step 14: Crude compound 339-14 (38 mg) was dissolved in dichloromethane (5 mL). (1*r*,2*R*,3*S*)-2,3-Dimethylcyclopropane-1-carboxylic acid (8.61 mg, 0.075 mmol), O-(7-azabenzotriazol-1-yl)-*N,N,N',N'*-tetramethyluronium hexafluorophosphate (28.70 mg, 0.075 mmol), and *N,N'*-diisopropylethylamine (19.51 mg, 0.151 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was first purified by a flash silica gel column (methanol/dichloromethane: 0-5%) to give (1r,2R,3S)-N-((3'S,4'S,Z)-1'-ethyl-2'-(2-((S)-1-methoxyethyl)-5-((tetrahydro-1H-pyrrolizin-7a(5H)-yl)methoxy)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z339', 25 mg, 0.029 mmol, two-step yield: 63%). The compound was subjected to prep-HPLC (trifluoroacetic acid method) to give two isomers. The structure of one isomer was arbitrarily designated as (1r,2R,3S)-N-((3'S,4'S,Z)-R₍ₐ₎-2'-(2-((S)-1-methoxyethyl)-5-((tetrahydro-1H-pyrrolizin-7a(5H)-yl)methoxy)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide

(trifluoroacetate, Z339, 9 mg, 0.009 mmol, yield: 31%, retention time: 7.97 min) as a white solid; the structure of the other isomer was arbitrarily designated as (1r,2R,3S)-N-((3'S,4'S,Z)-S₍ₐ₎-2'-(2-((S)-1-methoxyethyl)-5-((tetrahydro-1H-pyrrolizin-7a(5H)-yl)methoxy)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (trifluoroacetate, crude product, retention time: 8.20 min). The crude product was purified by prep-HPLC (hydrochloric acid method) to give (1r,2R,3S)-N-((3'S,4'S,Z)-S₍ₐ₎-2'-(2-((S)-1-methoxyethyl)-5-((tetrahydro-1H-pyrrolizin-7a(5H)-yl)methoxy)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (hydrochloride, Z339-1, 12 mg, 0.013 mmol, yield: 46%) as a white solid. ES-API: [M+H]⁺ = 864.4.

### Example 32. Synthesis of Compound Z461

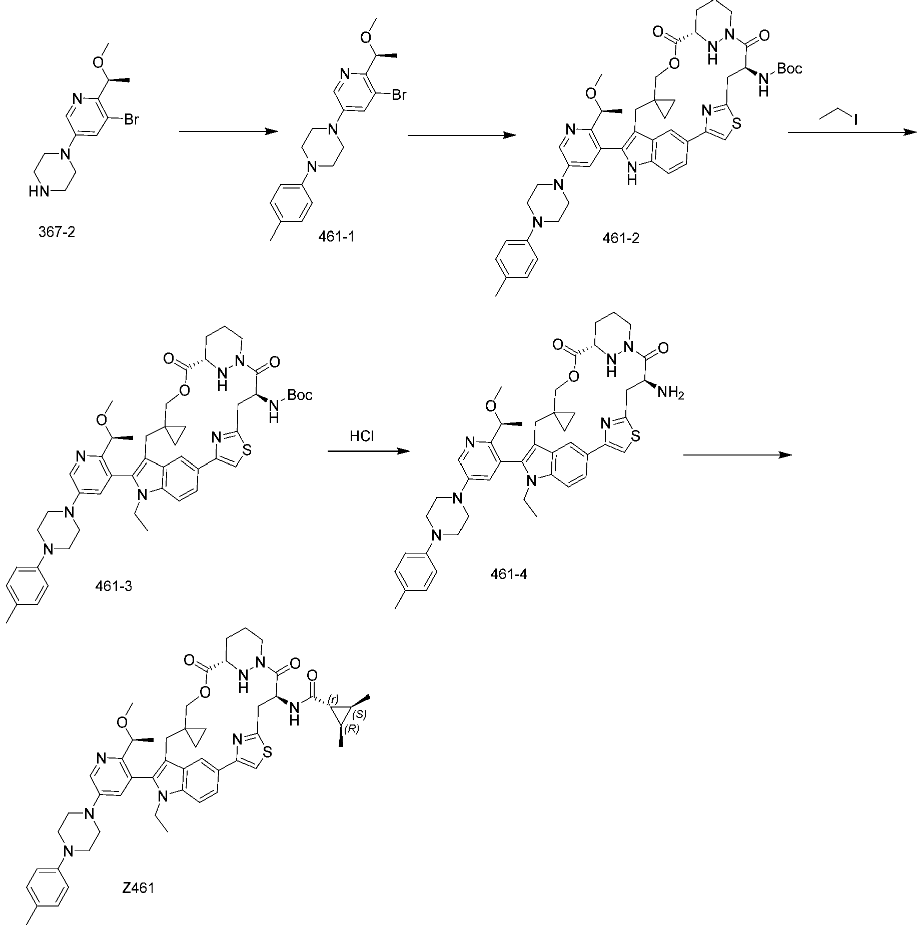

Step 1: A solution of compound 367-1 (75 mg, 0.25 mmol), 4-methylbenzeneboronic acid (75 mg, 0.55 mmol), triethylamine (151 mg, 1.5 mmol), and copper(II) acetate (100 mg, 0.55 mmol) in DMF (10 mL) was stirred at 60 °C for 16 h under an oil bath and a nitrogen atmosphere. After the reaction was completed, the mixture was poured into 50 mL of ethyl acetate and washed sequentially with 50 mL of water and 50 mL of saturated brine. The organic phase was dried over anhydrous sodium sulfate, filtered, and concentrated, and the residue was purified by a flash silica gel column (0-6% methanol/dichloromethane) to give compound 461-1 (60 mg, 0.16 mmol, yield: 64%). ES-API [M+H]⁺ = 390.1/392.1.

Step 2: A mixture of intermediate 2 (127 mg, 0.18 mmol), compound 461-1 (60 mg, 0.15 mmol), [1,1'-bis(di-tert-butylphosphino)ferrocene]dichloropalladium(II) (20 mg, 0.03 mmol), and potassium phosphate (98 mg, 0.46 mmol) was stirred at 60 °C for 18 h in dioxane (9 mL), toluene (3 mL), and water (3 mL) under an oil bath and a nitrogen atmosphere. After the reaction was completed, the mixture was poured into 30 mL of ethyl acetate and washed sequentially with 15 mL of water and 15 mL of saturated brine. The organic phase was dried over anhydrous sodium sulfate, filtered, and concentrated, and the residue was purified by a flash silica gel column (0-6% methanol/dichloromethane) to give compound 461-2 (40 mg, 0.046 mmol, yield: 30%). ES-API [M+H]⁺ = 566.2.

Step 3: Compound 461-2 (40 mg, 0.05 mmol) was dissolved in N,N-dimethylformamide (3 mL), and cesium carbonate (142 mg, 0.43 mmol) and iodoethane (287 mg, 1.84 mmol) were sequentially added. The mixture was stirred at room temperature for 16 h. After the reaction was completed, 20 mL of ethyl acetate was added, and the mixture was washed sequentially with 10 mL of saturated brine and 10 mL of diluted brine. The organic phase was dried over anhydrous sodium sulfate, filtered, and concentrated, and the residue was purified by a flash silica gel column (0-60% tetrahydrofuran/petroleum ether) to give compound 461-3 (28 mg, 0.03 mmol, yield: 67%). ES-API [M+H]⁺ = 903.4.

Step 4: Compound 461-3 (28 mg, 0.03 mmol) was dissolved in a solution of hydrochloric acid in dioxane (5 mL, 4 mol/L). The mixture was stirred at room temperature for 2 h. After the reaction was completed, the reaction mixture was concentrated to give compound 461-4 (53 mg, yield: 100%). ES-API [M+H]⁺ = 819.3.

Step 5: Compound 461-4 (24 mg, 0.03 mmol) was dissolved in N,N-dimethylformamide (3 mL), and (1r,2R,3S)-2,3-dimethylcyclopropane-1-carboxylic acid (5.1 mg, 0.05 mmol), N,N-diisopropylethylamine (23 mg, 0.18 mmol), and O-(7-azabenzotriazol-1-yl)-N,N,N',N'-tetramethyluronium hexafluorophosphate (34 mg, 0.09 mmol) were sequentially added. The mixture was stirred at room temperature for 1 h. After the reaction was completed, 10 mL of ethyl acetate was added, and the mixture was washed with 10 mL of saturated brine and 10 mL of water. The organic phase was dried over anhydrous sodium sulfate, filtered, and concentrated, and the residue was purified by prep-HPLC (ammonium bicarbonate method) to give (1r,2R,3S)-N-((3'S,4'S,Z)-1'-ethyl-2'-(2-((S)-1-methoxyethyl)-5-(4-(p-tolyl)piperazin-1-yl)pyridin-3-yl)-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z461, 3 mg, yield: 11%, purity: 98%). ES-API [M+H]⁺ = 899.4.

### Example 33. Synthesis of Compounds Z462 and Z463

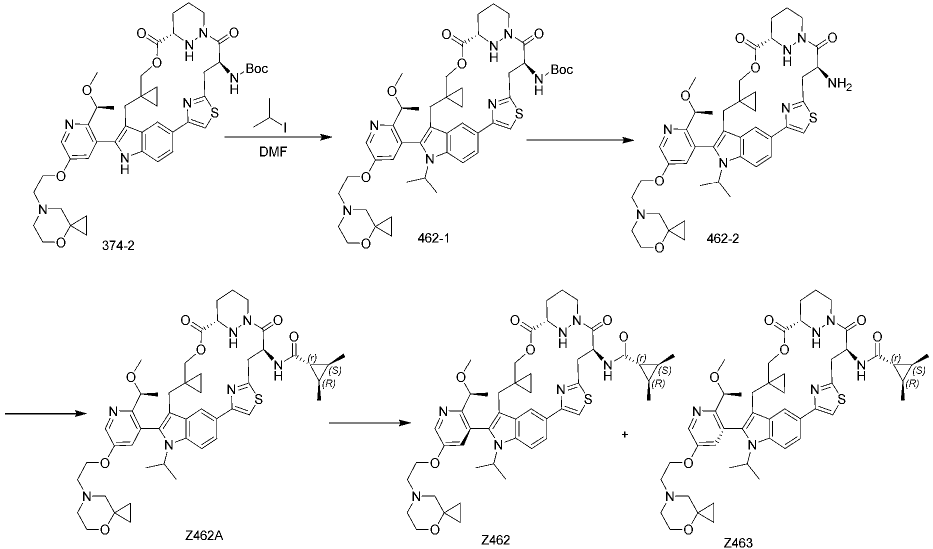

Step 1: Compound 374-2 (100 mg, 0.12 mmol) was dissolved in N,N-dimethylformamide (3 mL), and cesium carbonate (380 mg, 1.16 mmol) and 2-iodopropane (784 mg, 4.63 mmol) were sequentially added. The mixture was stirred at room temperature for 48 h. After the reaction was completed, 20 mL of ethyl acetate was added, and the mixture was washed sequentially with 10 mL of saturated brine and 10 mL of diluted brine. The organic phase was dried over anhydrous sodium sulfate, filtered, and concentrated, and the residue was purified by a flash silica gel column (0-60% tetrahydrofuran/petroleum ether) to give compound 462-1 (100 mg, 0.06 mmol, yield: 95%). ES-API [M+H]⁺ = 898.3.

Step 2: Compound 462-1 (120 mg, 0.13 mmol) was dissolved in a solution of hydrochloric acid in dioxane (5 mL, 4 mol/L). The mixture was stirred at room temperature for 2 h. After the reaction was completed, the reaction mixture was concentrated to give compound 462-2 (105 mg, yield: 100%). ES-API [M+H]⁺ = 798.3.

Step 3: Compound 462-2 (105 mg, 0.13 mmol) was dissolved in N,N-dimethylformamide (3 mL), and (1r,2R,3S)-2,3-dimethylcyclopropane-1-carboxylic acid (23 mg, 0.2 mmol), N,N-diisopropylethylamine (103 mg, 0.8 mmol), and O-(7-azabenzotriazol-1-yl)-N,N,N',N'-tetramethyluronium hexafluorophosphate (152 mg, 0.4 mmol) were sequentially added. The mixture was stirred at room temperature for 1 h. After the reaction was completed, 10 mL of ethyl acetate was added, and the mixture was washed with 10 mL of saturated brine and 10 mL of water. The organic phase was dried over anhydrous sodium sulfate, filtered, and concentrated to give crude Z462A, which was purified by prep-HPLC (ammonium bicarbonate method) to give 2 isomeric compounds. One of the structures was arbitrarily designated as: (1r,2R,35)-N-((3'S,4'S,Z)-2'-(5-(2-(4-oxa-7-azaspiro[2.5]octan-7-yl)ethoxy)-(Rₐ)-2-((S)-1-methoxyethyl)pyridin-3-yl)-1'-isopropyl 5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z462, retention time: 7.862 min, 20 mg, yield: 17%, purity: 98%). ES-API [M+H]⁺ = 894.4.

The other structure was arbitrarily designated as: (1r,2R,3S)-N-((3'S,4'S,Z)-2'-(5-(2-(4-oxa-7-azaspiro[2.5]octan-7-yl)ethoxy)-(Sₐ)-2-((S)-1-methoxyethyl)pyridin-3-yl)-1'-isopropyl 5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z463, retention time: 8.159 min, 20 mg, yield: 17%, purity: 98%). ES-API [M+H]⁺ = 894.4. ¹H NMR (400 MHz, DMSO-d₆) δ 8.54 (s, 1H), 8.48 (d, J = 2.7 Hz, 1H), 8.39 (d, J = 9.1 Hz, 1H), 7.80 (s, 1H), 7.68 (s, 2H), 7.44 (d, J = 2.7 Hz, 1H), 5.51 (t, J = 9.4 Hz, 1H), 4.98 (d, J = 12.1 Hz, 1H), 4.29 - 3.95 (m, 7H), 3.66 - 3.58 (m, 2H), 3.43 (d, J = 14.6 Hz, 1H), 3.33 (s, 1H), 3.27 - 3.08 (m, 2H), 2.98 (s, 3H), 2.74 (dd, J = 12.8, 7.1 Hz, 3H), 2.56-2.51 (m, 2H), 2.44 (s, 2H), 2.07 (d, J = 10.3 Hz, 1H), 1.89-1.70 (m, 3H), 1.58-1.42 (m, 7H), 1.28 (d, J = 6.2 Hz, 3H), 1.23 - 1.13 (m, 3H), 1.08 (dd, J = 12.2, 5.5 Hz, 6H), 0.61 (t, J = 5.7 Hz, 2H), 0.49 - 0.25 (m, 5H), -0.29 (s, 1H).

### Example 34. Synthesis of Compounds Z464 and Z465

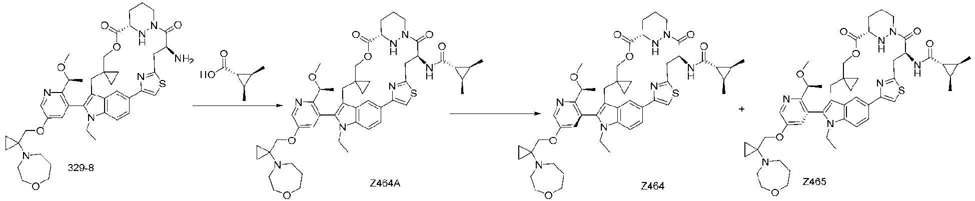

Step 1: Compound 329-8 (75 mg, 0.09 mmol) was dissolved in dichloromethane (3 mL), and (1r,2R,3S)-2,3-dimethylcyclopropane-1-carboxylic acid (10 mg, 0.1 mmol), O-(7-azabenzotriazol-1-yl)-N,N,N',N'-tetramethyluronium hexafluorophosphate (38 mg, 0.1 mmol), and N,N'-diisopropylethylamine (0.5 mL, 0.5 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give crude Z464A. The crude product was purified by prep-HPLC (hydrochloric acid method) to give 2 isomeric compounds. One of the structures was arbitrarily designated as: (1*r*,2*R*,3*S*)-*N*-((3'*S*,4'*S*,*Z*)-(*Rₐ*)-2'-(5-((1-(1,4-oxazepan-4-yl)cyclopropyl)methoxy)-2-((*S*)-1-methoxyethyl)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (hydrochloride, Z464, 20 mg, 0.022 mmol, yield: 24.8%, retention time: 1.45 min).

The other structure was arbitrarily designated as: (1*r*,2*R*,3*S*)-*N*-((3'*S*,4'*S*,*Z*)-(*Sₐ*)-2'-(5-((1-(1,4-oxazepan-4-yl)cyclopropyl)methoxy)-2-((*S*)-1-methoxyethyl)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (hydrochloride, Z465, 30 mg, 0.033 mmol, yield: 37.2%, retention time: 1.48 min) as a white solid. ES-API: [M+H]⁺ = 894.3.

### Example 35. Synthesis of Compound Z355

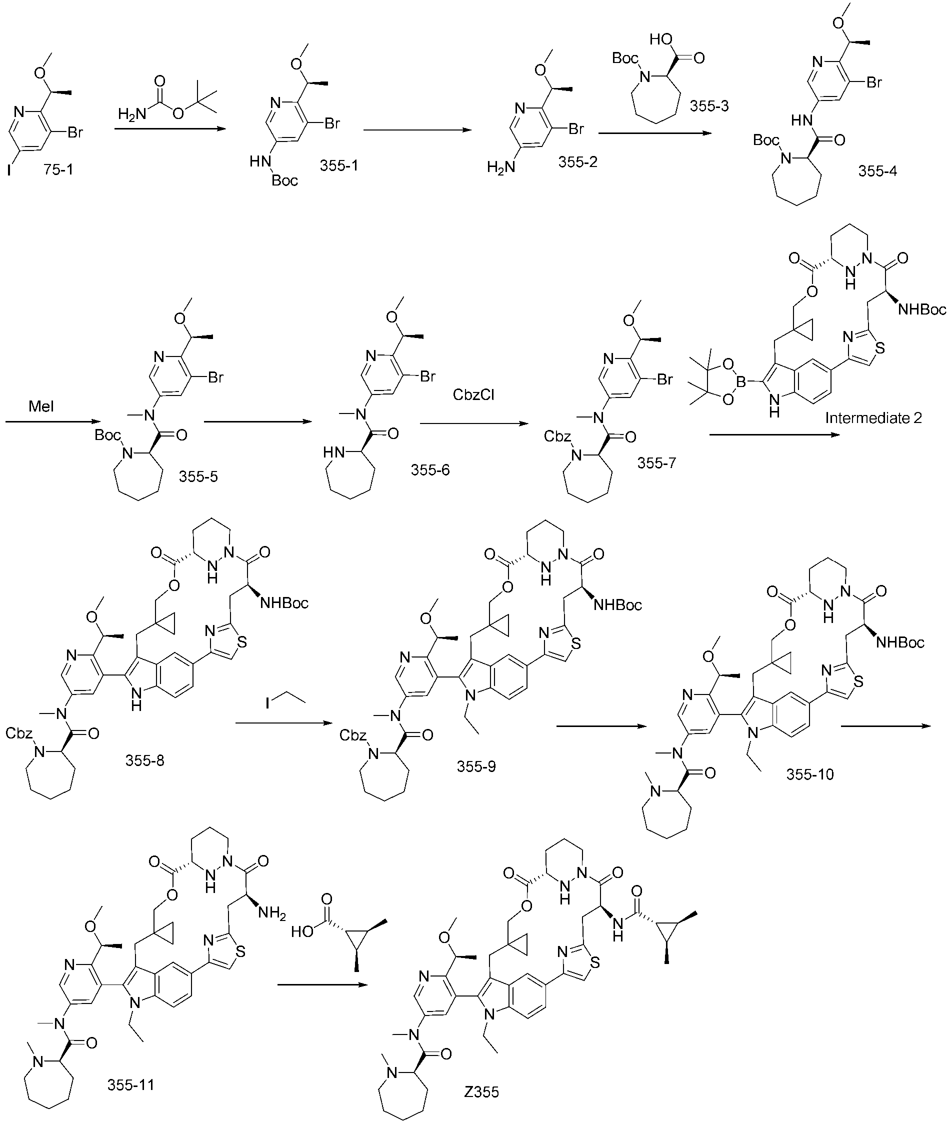

Step 1: Compound 75-1 (2.0 g, 5.85 mmol), tert-butyl carbamate (890 mg, 7.60 mmol), cesium carbonate (3.81 g, 11.701 mmol), 4,5-bis(diphenylphosphino)-9,9-dimethylxanthene (340 mg, 0.59 mmol), and tris(dibenzylideneacetone)dipalladium(0) (270 mg, 0.29 mmol) were dissolved in dioxane (40 mL), and the reaction system was purged three times with nitrogen and reacted at 85 °C for 18 h. The reaction mixture was filtered, and the filtrate was concentrated under reduced pressure. The crude product was purified by a flash silica gel column (ethyl acetate/petroleum ether: 0-35%) to give compound 355-1 (630 mg, yield: 32.5%) as a yellow solid. ES-API: [M+H]⁺ = 331.3,333.3.

Step 2: Compound 355-1 (630 mg, 1.90 mmol) was dissolved in absolute methanol (3 mL), and a 4.0 M hydrogen chloride/dioxane solution (10 mL) was added at 0 °C. The reaction system was stirred at room temperature for 3 h. The reaction mixture was concentrated, and saturated sodium bicarbonate (15 mL) was added. The mixture was extracted with dichloromethane (40 mL × 2). The organic phases were mixed, dried over anhydrous sodium sulfate, filtered, and concentrated to give compound 355-2 (350 mg, yield: 79.6%) as a pale yellow solid. ES-API: [M+H]⁺ = 231.3, 233.3.

Step 3: Compound 355-2 (335 mg, 1.45 mmol), compound 355-3 (529 mg, 2.17 mmol), and triethylamine (1.21 mL, 8.70 mmol) were dissolved in dichloromethane (15 mL), and a 50% solution (2.77 g, 4.35 mmol) of 1-propylphosphonic anhydride in ethyl acetate was added at 0 °C. The reaction system was stirred at room temperature for 18 h. Dichloromethane (25 mL) was added to the reaction mixture. The mixture was washed sequentially with water (15 mL) and saturated brine (15 mL), dried over anhydrous sodium sulfate, filtered, and concentrated. The crude product was purified by a flash silica gel column (ethyl acetate/petroleum ether: 0-50%) to give compound 355-4 (420 mg, yield: 63.5%) as a white solid. ES-API: [M+H]⁺ = 456.2, 458.1.

Step 4: Compound 355-4 (400 mg, 0.88 mmol) was dissolved in N,N-dimethylformamide (10 mL), and cesium carbonate (714 mg, 2.19 mmol) and iodomethane (249 mg, 1.75 mmol) were added at 0 °C. The reaction system was stirred at room temperature for 3 h. Water (20 mL) was added to the reaction mixture, and the mixture was extracted with ethyl acetate (30 mL × 2). The organic phases were mixed and washed with saturated brine (20 mL × 3). The organic phase was dried over anhydrous sodium sulfate, filtered, and concentrated. The crude product was purified by a flash silica gel column (ethyl acetate/petroleum ether: 0-50%) to give compound 355-5 (400 mg, yield: 97.0%) as a colorless viscous liquid. ES-API: [M+H]⁺ = 470.1,472.1.

Step 5: Compound 355-5 (350 mg, 0.74 mmol) was dissolved in absolute methanol (2 mL), and a 4.0 M hydrogen chloride/dioxane solution (5 mL) was added at 0 °C. The reaction system was stirred at room temperature for 3 h. The reaction mixture was concentrated to give the desired product of compound 355-6 (302 mg, yield: 100%) as a white solid. ES-API: [M+H]⁺ = 370.1, 372.1.

Step 6: Compound 355-6 (302 mg, 0.74 mmol) was dissolved in tetrahydrofuran (10 mL) and water (10 mL), and sodium bicarbonate (374 mg, 4.45 mmol) and benzyl chloroformate (190 mg, 1.11 mmol) were added at 0 °C. The reaction system was stirred at room temperature for 2 h. The reaction mixture was extracted with ethyl acetate (50 mL). The organic phase was washed with saturated brine (20 mL), dried over anhydrous sodium sulfate, filtered, and concentrated. The crude product was purified by a flash silica gel column (ethyl acetate/petroleum ether: 0-50%) to give compound 355-7 (370 mg, yield: 98.8%) as a colorless viscous liquid. ES-API: [M+H]⁺ = 504.1, 506.2.

Step 7: Intermediate 2 (80 mg, 0.12 mmol) and compound 355-7 (117 mg, 0.23 mmol) were dissolved in toluene (0.5 mL), dioxane (1.5 mL), and water (0.5 mL), and potassium phosphate (74 mg, 0.35 mmol) and [1,1'-bis(di-tert-butylphosphino)ferrocene]dichloropalladium(II) (15 mg, 0.023 mmol) were added. The reaction system was purged 3 times with nitrogen and stirred at 70 °C for 2 h. Ethyl acetate (30 mL) and water (15 mL) were added to the reaction mixture. The separated organic phase was washed with saturated brine (15 mL), dried over anhydrous sodium sulfate, and filtered, and the filtrate was concentrated under reduced pressure. The crude product was purified by a flash silica gel column (tetrahydrofuran/petroleum ether: 0-60%) to give compound 355-8 (110 mg, yield: 95.9%) as a white solid. ES-API: [M+H]⁺ = 989.3.

Step 8: Compound 355-8 (110 mg, 0.11 mmol) was dissolved in N,N-dimethylformamide (5 mL), and cesium carbonate (109 mg, 0.33 mmol) and iodoethane (70 mg, 0.45 mmol) were added at 0 °C. The reaction system was stirred at room temperature for 18 h. Ethyl acetate (50 mL) was added to the reaction mixture, and the mixture was washed sequentially with water (15 mL) and saturated brine (15 mL × 3), dried over anhydrous sodium sulfate, filtered, and concentrated. The crude product was purified by a flash silica gel column (tetrahydrofuran/petroleum ether: 0-60%) to give compound 355-9 (90 mg, yield: 79.6%) as a pale brown solid. ES-API: [M+H]⁺ = 1017.5.

Step 9: Compound 355-9 (90 mg, 0.088 mmol) and paraformaldehyde (120 mg, 1.33 mmol) were dissolved in absolute methanol (8 mL), and 10% palladium hydroxide on carbon (100 mg) was added. The reaction system was stirred at 30 °C for 40 h under a hydrogen atmosphere. The reaction mixture was filtered through celite and washed with methanol, and the filtrate was concentrated. The crude product was subjected to preparative silica gel thin-layer chromatography (dichloromethane/methanol = 12:1) to give compound 355-10 (40 mg, yield: 50.4%) as a white solid. ES-API: [M+H]⁺ = 897.5.

Step 10: Compound 355-10 (40 mg, 0.045 mmol) was dissolved in absolute methanol (0.3 mL), and a 4.0 M hydrogen chloride/dioxane solution (1.5 mL) was added at 0 °C. The reaction system was stirred at room temperature for 2 h. The reaction mixture was concentrated to give compound 355-11 (35 mg, yield: 100%) as a white solid. ES-API: [M+H]⁺ = 797.3. Step 11: Compound 355-11 (30 mg, 0.038 mmol) was dissolved in dichloromethane (5 mL), and (1r,2R,3S)-2,3-dimethylcyclopropane-1-carboxylic acid (8 mg, 0.056 mmol), O-(7-azabenzotriazol-1-yl)-N,N,N',N'-tetramethyluronium hexafluorophosphate (29 mg, 0.075 mmol), and N,N'-diisopropylethylamine (29 mg, 0.23 mmol) were added. The reaction system was stirred at room temperature for 2 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was subjected to preparative silica gel thin-layer chromatography (dichloromethane/methanol = 10:1) to give the desired product (2R)-N-(5-((6³S,4S,Z)-4-((1r, 2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-6-((S)-1-methoxyethyl)pyridin-3-yl)-N,1-dimethylazepane-2-carboxamide (Z355, 30 mg, yield: 89.2%) as an off-white solid. ES-API: [M+H]⁺= 893.3.

### Example 36. Synthesis of Compound Z467

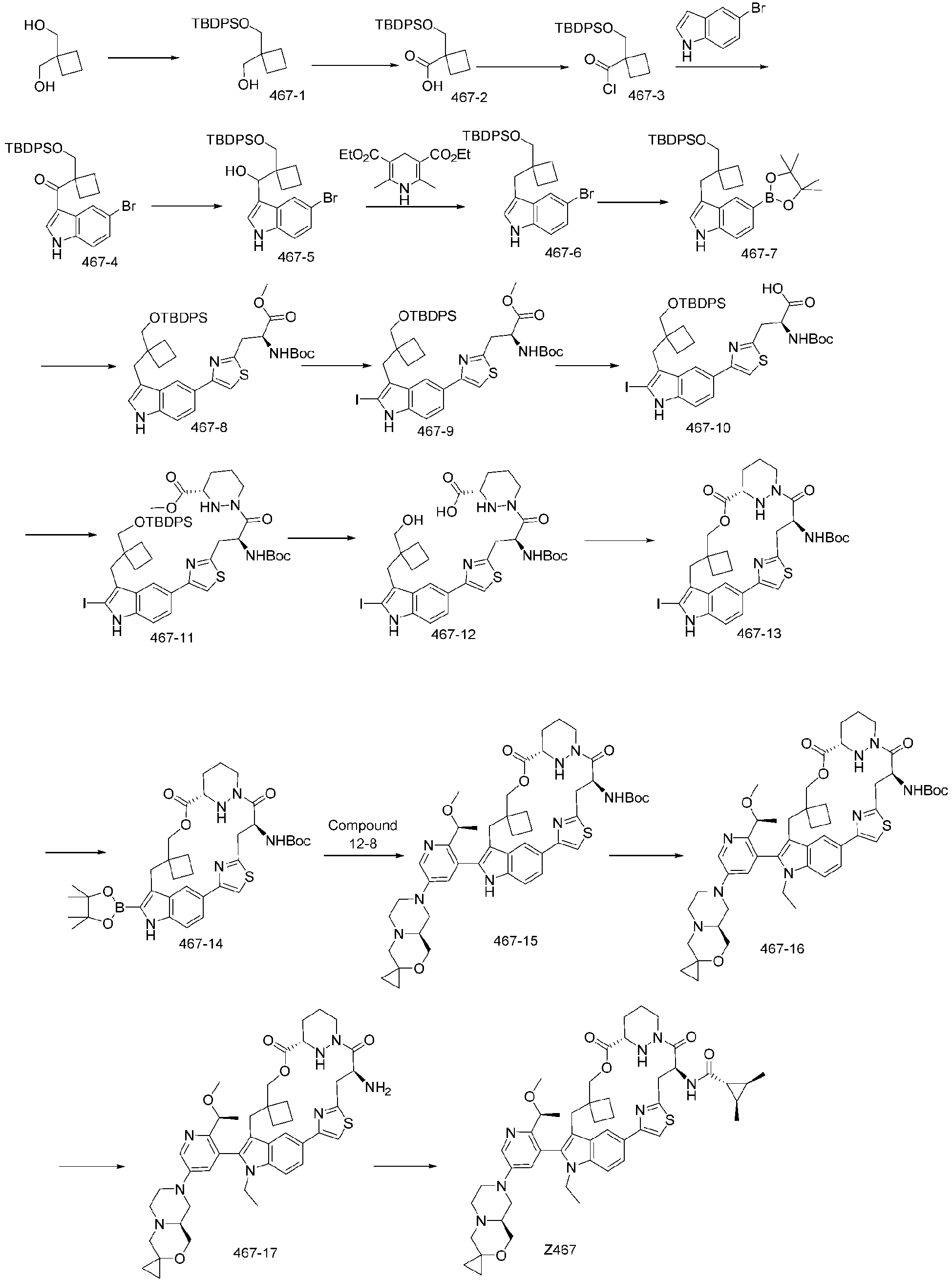

Step 1: A solution of tert-butyldiphenylchlorosilane (56.20 g, 204.459 mmol) in dichloromethane (100 mL) was slowly added dropwise to a solution of cyclobutane-1,1-diyldimethanol (25.0 g, 215.22 mmol) and imidazole (16.12 g, 236.74 mmol) in dichloromethane (400 mL) under an ice bath. After the dropwise addition was completed, the mixture was stirred at room temperature for 1 h. After the reaction was completed, the mixture was washed twice with 1 M diluted hydrochloric acid (200 mL) and once with saturated brine (200 mL). The organic phase was dried over anhydrous sodium sulfate, filtered, and concentrated, and the residue was purified by a flash silica gel column (0-10% ethyl acetate/petroleum ether) to give compound 467-1 (57.0 g, yield: 74.70%). ES-API [M+H-Ph⁺] = 277.0.

Step 2: Concentrated sulfuric acid (17.13 mL, 321.52 mmol) was slowly added to a solution of chromium trioxide (24.11 g, 241.144 mmol) in water (55.0 mL) under an ice bath. The mixture was stirred for 0.5 h. The obtained reaction mixture was slowly added to a solution of compound 467-1 (57.0 g, 160.763 mmol) in acetone (600 mL), and the mixture was stirred at room temperature overnight. After the reaction was completed, the mixture was filtered, and the filtrate was concentrated. The residue was purified by a flash silica gel column (0-16% tetrahydrofuran/petroleum ether) to give compound 467-2 (50.0 g, yield: 84.39%) as a white solid, ES-API [M+H-Ph⁺] = 291.1.

Step 3: Oxalyl chloride (17.21 mL, 203.50 mmol) and N,N-dimethylformamide (1.04 mL, 13.567 mmol) were sequentially added to a solution of compound 467-2 (50.0 g, 135.67 mmol) in dichloromethane (100 mL) under an ice bath. The mixture was stirred for 1 h. After the reaction was completed, the mixture was concentrated to give compound 467-3 (53.0 g, crude product).

Step 4: A 1 M solution (136.954 mL, 136.954 mmol) of tin tetrachloride in tetrahydrofuran was added dropwise to a solution of compound 467-3 (53.0 g, crude product) in tetrahydrofuran (100 mL) under a nitrogen atmosphere and an ice bath. The mixture was stirred for 0.5 h. A solution of 5-bromo-1H-indole (29.53 g, 150.650 mmol) in tetrahydrofuran (200 mL) was then slowly added dropwise, and the mixture was stirred for another 1 h. After the reaction was completed, the reaction mixture was poured into a 1 M sodium hydroxide solution (500 mL) and filtered through celite. The filtrate was washed with 300 mL of water and extracted with 500 mL of ethyl acetate. The organic phases were combined, dried over anhydrous sodium sulfate, filtered, and concentrated, and the residue was purified by a flash silica gel column (0-35% ethyl acetate/petroleum ether) to give compound 467-4 (43.0 g, yield: 57.44%) as a white solid. ES-API [M+H⁺] = 546.1.

Step 5: Compound 467-4 (8.0 g, 15.022 mmol) was dissolved in tetrahydrofuran (50.0 mL) and absolute ethanol (50.0 mL), and sodium borohydride (2.84 g, 75.110 mmol) was slowly added. The mixture was stirred at room temperature for 5 h. After the reaction was completed, the reaction was quenched with a saturated ammonium chloride solution (60.0 mL), and the mixture was extracted three times with 80.0 mL of ethyl acetate. The organic phase was dried over anhydrous sodium sulfate and concentrated to give compound 467-5 (8.1 g, crude product), ES-API [M+H⁺-H₂O] = 530.2.

Step 6: Crude compound 467-5 (8.10 g, 14.76 mmol) obtained above was dissolved in tetrahydrofuran (40 mL) under an ice bath. p-Toluenesulfonic acid monohydrate (0.28 g, 1.476 mmol) and diethyl 2,6-dimethyl-1,4-dihydropyridine-3,5-dicarboxylate (4.11 g, 16.241 mmol) were added. The mixture was stirred at room temperature for 2 h. 100 mL of ethyl acetate was added to the reaction mixture, and the mixture was washed sequentially with 100 mL of a saturated ammonium chloride solution and 100 mL of saturated brine. The organic phase was dried over anhydrous sodium sulfate and concentrated, and the residue was purified by a flash silica gel column (0-50% dichloromethane/petroleum ether) to give compound 467-6 (5.40 g, yield: 68.67%). ES-API [M+Na⁺] = 554.1, 556.1.

Step 7: A mixture of compound 467-6 (5.6 g, 10.757 mmol), bis(pinacolato)diboron (5.46 g, 21.514 mmol), [1,1'-bis(diphenylphosphino)ferrocene]palladium(II) dichloride (1.18 g, 1.614 mmol), and potassium acetate (3.17 g, 32.271 mmol) was stirred at 95 °C for 3 h in toluene (50.0 mL) under a nitrogen atmosphere. 200 mL of ethyl acetate was added to the reaction mixture, and the mixture was washed three times with 100 mL of water. The organic phase was dried over anhydrous sodium sulfate and concentrated, and the residue was purified by a flash silica gel column (0-50% ethyl acetate/petroleum ether) to give compound 467-7 (4.60 g, yield: 75.33%). ES-API [M+H⁺] = 580.0.

Step 8: A mixture of compound 467-7 (6.40 g, 11.041 mmol), methyl (R)-3-(4-bromothiazol-2-yl)-2-((tert-butoxycarbonyl)amino)propanoate (4.84 g, 13.249 mmol), [1,1'-bis(di-tert-butylphosphino)ferrocene]dichloropalladium(II) (1.07 g, 1.656 mmol), and potassium phosphate (7.03 g, 33.123 mmol) was stirred at 100 °C for 2 h in dioxane (120 mL), toluene (40 mL), and water (40 mL) under a nitrogen atmosphere. After the reaction was completed, 100 mL of ethyl acetate was added, and the mixture was washed with 100 mL of saturated brine. The organic phase was dried over anhydrous sodium sulfate and concentrated, and the residue was purified by a flash silica gel column (0-70% ethyl acetate/petroleum ether) to give compound 467-8 (6.40 g, yield: 78.54%). ES-API [M+H⁺] = 738.3.

Step 9: A solution of elemental iodine (1.96 g, 7.708 mmol) in tetrahydrofuran (20.0 mL) was added to a mixed solution of compound 467-8 (6.20 g, 8.564 mmol), sodium bicarbonate (0.79 g, 9.420 mmol), and silver trifluoroacetate (2.42 g, 9.420 mmol) in tetrahydrofuran (80.0 mL) under an ice bath. The mixture was stirred for 0.5 h. After the reaction was completed, the reaction was quenched with 100 mL of a sodium thiosulfate solution, and the mixture was extracted with 200 mL of ethyl acetate. The organic phase was dried over anhydrous sodium sulfate and concentrated, and the residue was purified by a flash silica gel column (0-70% ethyl acetate/petroleum ether) to give compound 467-9 (5.6 g, yield: 76.94%). ES-API [M+H⁺] = 864.22.

Step 10: The obtained compound 467-9 (6.482 g, 6.482 mmol) was dissolved in tetrahydrofuran (20.0 mL) and water (5.0 mL), and anhydrous lithium hydroxide (0.82 g, 19.446 mmol) was added. The mixture was stirred at room temperature for 5 h. After the reaction was completed, the mixture was neutralized to pH = 6 with 1 M diluted hydrochloric acid and extracted with 100.0 mL of ethyl acetate. The organic phase was dried over anhydrous sodium sulfate and concentrated to give crude compound 467-10 (5.2 g, yield: 94.39%). ES-API [M+H⁺] = 850.2.

Step 11: A solution of compound 467-10 (5.20 g, 6.118 mmol), methyl (R)-hexahydropyridazine-3-carboxylate ditrifluoroacetate (5.69 g, 15.296 mmol), N,N,N',N'-tetramethylchloroformamidinium hexafluorophosphate (0.25 g, 1.836 mmol), and N-methylmorpholine (3.71 g, 36.710 mmol) in dichloromethane (60.0 mL) was stirred at room temperature for 2 h under a nitrogen atmosphere. After the reaction was completed, the mixture was diluted with 100.0 mL of ethyl acetate and washed with 200 mL of water. The organic phase was dried over anhydrous sodium sulfate and concentrated, and the residue was purified by a flash silica gel column (0-70% ethyl acetate/petroleum ether) to give compound 467-11 (5.0 g, yield: 83.73%). ES-API [M+H⁺] = 976.2.

Step 12: The obtained compound 467-11 (5.0 g, 5.123 mmol) was dissolved in tetrahydrofuran (30.0 mL), and the mixture was stirred at 60 °C for 5 h. After the reaction was completed, the mixture was neutralized to pH = 6 with 1 M diluted hydrochloric acid and extracted with 100.0 mL of ethyl acetate. The organic phase was dried over anhydrous sodium sulfate, filtered, and concentrated to give compound 467-12 (2.10 g, yield: 56.65%). ES-API [M+H⁺] = 724.2.

Step 13: The obtained compound 467-12 (4.70 g, 6.495 mmol) was dissolved in dichloromethane (60.0 mL), and N,N-diisopropylethylamine (33.58 g, 259.801 mmol), 1-hydroxybenzotriazole (8.78 g, 64.950 mmol), and 1-(3-dimethylaminopropyl)-3-ethylcarbodiimide hydrochloride (37.35 g, 194.851 mmol) were sequentially added. The mixture was stirred at room temperature overnight. After the reaction was completed, the reaction mixture was washed three times with 100 mL of 0.5 M diluted hydrochloric acid. The organic phase was dried over anhydrous sodium sulfate and concentrated, and the residue was purified by a flash silica gel column (0-70% ethyl acetate/petroleum ether) to give compound 467-13 (2.10 g, yield: 45.82%). ES-API [M+H⁺] = 706.2.

Step 14: 4,4,5,5-Tetramethyl-1,3,2-dioxaborolane (0.63 g, 4.960 mmol), tris(dibenzylideneacetone)dipalladium(0) (60 mg, 0.071 mmol), 2-dicyclohexylphosphino-2',6'-dimethoxybiphenyl (290.0 mg, 0.709 mmol), and potassium acetate (98.0 g, 2.126 mmol) were added to a solution of compound 467-13 (500 mg, 0.709 mmol) in toluene (10.0 mL) under a nitrogen atmosphere. The mixture was purged 4 times with nitrogen and then reacted at 60 °C for 5 h. After the reaction was completed, the mixture was cooled to room temperature, filtered under reduced pressure, and then purified by a flash silica gel column (0-50% ethyl acetate/petroleum ether) to give compound 467-14 (350.0 mg, yield: 69.99%). ES-API [M+H⁺] = 706.34.

Step 15: Compound 467-14 (200.0 mg, 0.283 mmol) and compound 12-8 (100.0 mg, 0.281 mmol) were dissolved in toluene (6.0 mL), 1,4-dioxane (2.0 mL), and water (2.0 mL), and potassium phosphate (184.0 mg, 0.867 mmol) and [1,1'-bis(di-tert-butylphosphino)ferrocene]dichloropalladium(II) (26.0 mg, 0.040 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 467-15 (150.0 mg, yield: 60.7%). ES-API: [M+H]⁺ = 881.3.

Step 16: Compound 467-15 (150.0 mg, 0.175 mmol) was dissolved in N,N-dimethylformamide (10.0 mL), and cesium carbonate (258.78 mg, 0.8779 mmol) and iodoethane (408.5 mg, 2.619 mmol) were added. The mixture was reacted at room temperature for 1 h. After the reaction was completed, water (80 mL) was added, and the mixture was extracted with ethyl acetate (60 mL × 3). The organic phase was washed with saturated brine (20 mL), dried over anhydrous sodium sulfate, and concentrated under reduced pressure to give compound 467-16 (160.0 mg, crude product). ES-API: [M+H]⁺ = 909.4.

Step 17: Compound 467-16 (20.0 mg, crude product) was dissolved in methanol (2.0 mL), and a solution of hydrochloric acid in dioxane (2 mL, 2.0 mmol) was added. The mixture was reacted at room temperature for 2 h. The reaction mixture was concentrated under reduced pressure to give compound 467-17 (20.0 mg, crude product). ES-API [M+H]⁺ = 809.3.

Step 18: Compound 467-17 (20.0 mg, crude product) was dissolved in N,N-dimethylformamide (1.0 mL), and (1r,2R,3S)-2,3-dimethylcyclopropane-1-carboxylic acid (3.15 mg, 0.028 mmol), O-(7-azabenzotriazol-1-yl)-N,N,N',N'-tetramethyluronium hexafluorophosphate (26.24 mg, 0.069 mmol), and N,N'-diisopropylethylamine (17.84 mg, 0.138 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was subjected to prep-HPLC (ammonia water method) to give (1r,2R,3S)-N-((3'S,4'S,Z)-1'-ethyl-2'-(2-((S)-1-methoxyethyl)-5-((S)-tetrahydro-4'H-spiro[cyclopropane-1,3'-pyrazino[2,1-c][1,4]oxazin]-8'(1'H)-yl)pyridin-3-yl)-5',7'-dioxospiro[cyclobutane-1,10'-8-oxa-2(4,2)-thiazola-1(5,3)-indola-6(1,3)-pyridazinacycloundecaphan]-4'-yl)-2,3-dimethylcyclopropane-1-carboxamide (Z467, 6.0 mg, 0.007 mmol, yield: 28.82%) as a white solid. ES-API: [M+H]⁺ = 905.5.

### Example 37. Synthesis of Compound Z468

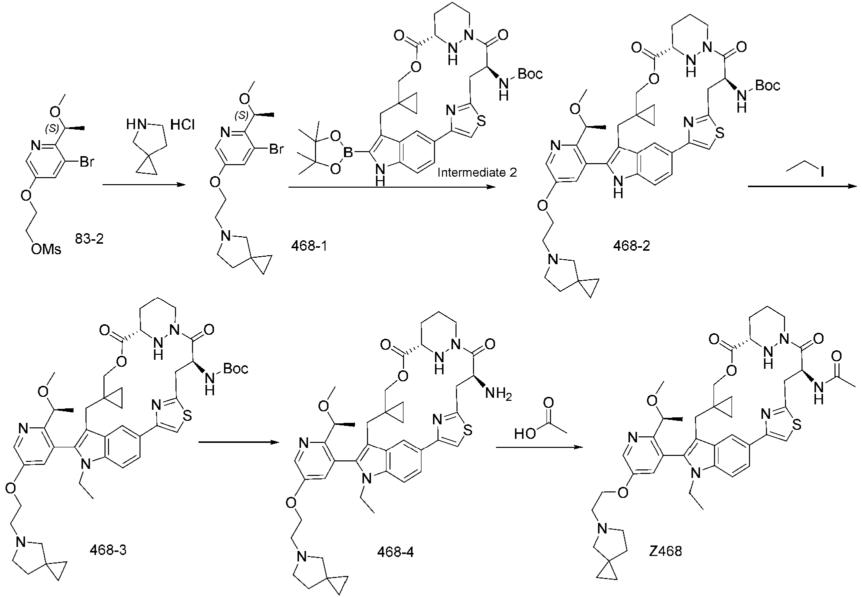

Step 1: N,N-Diisopropylethylamine (1.240 mg, 9.65 mmol) and 5-azaspiro[2.4]heptane hydrochloride (250 mg, 1.879 mmol) were added to a solution of compound 83-2 (0.57 g, 1.609 mmol) in N,N-dimethylformamide (10.0 mL) at room temperature, and the mixture was reacted at 70 °C overnight under a nitrogen atmosphere. After the reaction was completed, the reaction mixture was cooled to room temperature, and ethyl acetate (50 mL) was added to the reaction mixture. The mixture was washed sequentially with diluted brine (20 mL × 3) and saturated brine (50 mL). The organic phase was dried over anhydrous sodium sulfate and filtered, and the filtrate was concentrated under reduced pressure. The crude product was purified by a flash silica gel column (methanol/dichloromethane: 0-10%) to give compound 468-1 (280 mg, yield: 49.0%), ES-API: [M+H]⁺ = 355.0/357.0.

Step 2: Intermediate 2 (200.0 mg, 0.289 mmol) and compound 468-1 (154.0 mg, 0.433 mmol) were dissolved in toluene (6.0 mL), 1,4-dioxane (2.0 mL), and water (2.0 mL), and potassium phosphate (184.0 mg, 0.867 mmol) and [1,1'-bis(di-tert-butylphosphino)ferrocene]dichloropalladium(II) (26.0 mg, 0.040 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 468-2 (220.0 mg, 0.2620 mmol, yield: 90.0%). ES-API: [M+H]⁺ = 840.4.

Step 3: Compound 468-2 (220.0 mg, 0.2620 mmol) was dissolved in N,N-dimethylformamide (10.0 mL), and cesium carbonate (426.6 mg, 1.309 mmol) and iodoethane (408.5 mg, 2.619 mmol) were added. The mixture was reacted at room temperature for 1 h. After the reaction was completed, water (80 mL) was added, and the mixture was extracted with ethyl acetate (60 mL × 3). The organic phase was washed with saturated brine (20 mL), dried over anhydrous sodium sulfate, filtered, and concentrated under reduced pressure to give compound 468-3 (80.0 mg, 0.092 mmol, yield: 35.19%). ES-API: [M+H]⁺ = 868.4.

Step 4: Compound 468-3 (80.0 mg, 0.092 mmol) was dissolved in methanol (5.0 mL), and a solution of hydrochloric acid in dioxane (10 mL, 10.0 mmol) was added. The mixture was reacted at room temperature for 2 h. The reaction mixture was concentrated under reduced pressure to give compound 468-4 (90.0 mg, crude product). ES-API [M+H]⁺ = 768.3.

Step 5: Compound 468-4 (90.0 mg, crude product) was dissolved in N,N-dimethylformamide (5.0 mL), and acetic acid (5.52 mg, 0.092 mmol), O-(7-azabenzotriazol-1-yl)-N,N,N',N'-tetramethyluronium hexafluorophosphate (105.0 mg, 0.276 mmol), and N,N'-diisopropylethylamine (0.122 mL, 0.702 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was subjected to prep-HPLC (ammonia water method) to give N-((3'S,4'S,Z)-2'-(5-(2-(5-azaspiro[2.4]heptan-5-yl)ethoxy)-2-((S)-1-methoxyethyl)pyridin-3-yl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)acetamide (Z468, 18.0 mg, 0.022 mmol, yield: 24.15%) as a white solid. ES-API: [M+H]⁺ = 810.3.

### Example 38. Synthesis of Compound Z415

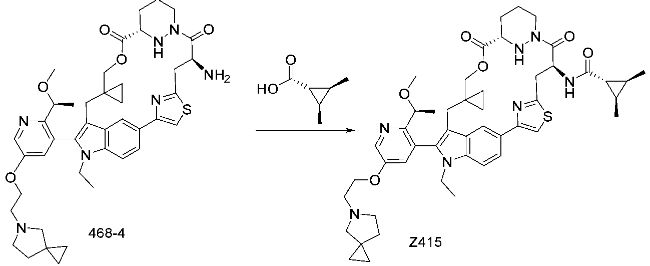

Step 1: Compound 468-4 (90.0 mg, crude product) was dissolved in N,N-dimethylformamide (5.0 mL), and (1r,2R,3S)-2,3-dimethylcyclopropane-1-carboxylic acid (12.6 mg, 0.110 mmol), O-(7-azabenzotriazol-1-yl)-N,N,N',N'-tetramethyluronium hexafluorophosphate (105.0 mg, 0.276 mmol), and N,N'-diisopropylethylamine (0.122 mL, 0.702 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was purified by prep-HPLC (ammonia water method) to give (1r,2R,3S)-N-((3'S,4'S,Z)-2'-(5-(2-(5-azaspiro[2.4]heptan-5-yl)ethoxy)-2-((S)-1-methoxyethyl)pyridin-3-yl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z415, 8.0 mg, 0.009 mmol, yield: 10.06%) as a white solid. ES-API: [M+H]⁺ = 864.3.

### Example 39. Synthesis of Compounds Z469A, Z469, and Z470

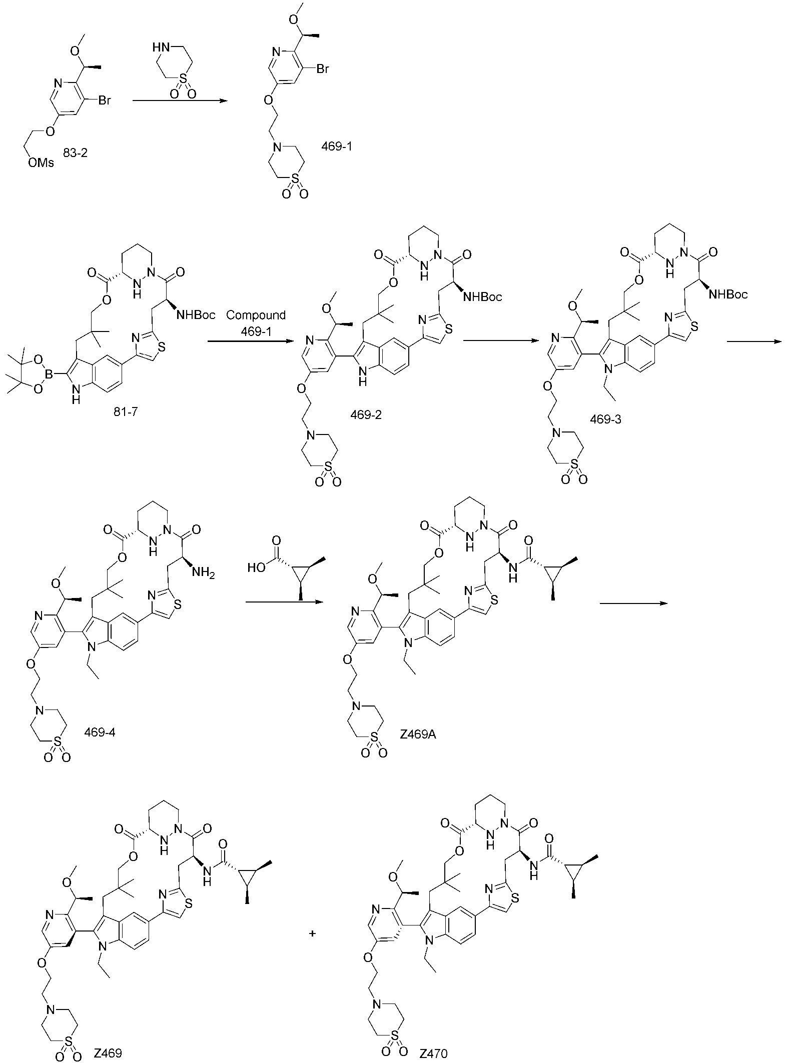

Step 1: Thiomorpholine-1,1-dioxide (38.12 mg, 0.282 mmol) was dissolved in N,N-dimethylformamide (2 mL), and compound 83-2 (100 mg, 0.282 mmol) and N,N-diisopropylethylamine (109.35 mg, 0.846 mmol) were added. The mixture was reacted at 70 °C for 18 h. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated, and the residue was purified by silica gel column chromatography (ethyl acetate:petroleum ether = 1:2) to give compound 469-1 (70 mg, 0.178 mmol, yield: 63.11%). ES-API: [M+H]⁺ = 393.0,395.0.

Step 2: Compound 81-7 (70 mg, 0.101 mmol) and compound 469-1 (59.53 mg, 0.151 mmol) were dissolved in toluene (1.5 mL), 1,4-dioxane (0.5 mL), and water (0.5 mL), and potassium phosphate (64.26 mg, 0.303 mmol) and [1,1'-bis(di-tert-butylphosphino)ferrocene]dichloropalladium(II) (6.58 mg, 0.010 mmol) were added. The mixture was reacted at 70 °C for 3 h under a nitrogen atmosphere. The reaction mixture was concentrated under reduced pressure, and the residue was purified by silica gel column chromatography (ethyl acetate:petroleum ether = 1:1) to give compound 469-2 (60 mg, 0.068 mmol, yield: 67.56%). ES-API: [M+H]⁺ = 880.3.

Step 3: Compound 469-2 (60 mg, 0.068 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 469-3 (0.06 g, 0.066 mmol, yield: 97.2%). ES-API: [M+H]⁺ = 908.3.

Step 4: Compound 469-3 (60 mg, 0.066 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 469-4 (0.05 g, 0.062 mmol, yield: 93.66%). ES-API: [M+H]⁺ = 808.3. Step 5: Compound 469-4 (50 mg, 0.062 mmol) was dissolved in dichloromethane (1 mL), and (1R,2R,3S)-2,3-dimethylcyclopropane-1-carboxylic acid (3.23 mg, 0.028 mmol), O-(7-azabenzotriazol-1-yl)-N,N,N',N'-tetramethyluronium hexafluorophosphate (14.68 mg, 0.039 mmol), and N,N-diisopropylethylamine (13.31 mg, 0.103 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, and concentrated under reduced pressure to give crude Z469A. The crude product was purified by preparative thin-layer chromatography (dichloromethane:methanol = 15:1) to give 2 isomeric compounds. The structure of one of the isomeric compounds was arbitrarily designated as (1r,2R,3S)-*N*-((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹ *H*-8-oxa-2(4,2)-thiazola-1(5,3)-indola-6(1,3)-pyridazinacycloundecaphane-4-yl)-2,3-dimethylcyclopropane-1-carboxamide (Z469, 11 mg, 0.012 mmol, yield: 19.66%, Rf = 0.45), ES-API: [M+H]⁺ = 904.3. ¹H NMR (400 MHz, DMSO-d₆) δ 8.49 (s, 1H), 8.46 (d, J = 2.8 Hz, 1H), 8.39 (d, J = 8.8 Hz, 1H), 7.81 (s, 1H), 7.74 (dd, J = 8.8, 1.2 Hz, 1H), 7.57 (d, J = 8.8 Hz, 1H), 7.38 (d, J = 2.8 Hz, 1H),5.55 (t, J = 9.2 Hz, 1H), 5.07 (d, J = 12.0 Hz, 1H), 4.46 - 4.01 (m, 7H), 3.57 (s, 2H), 3.29 (s, 1H), 3.22 (s, 3H), 3.20 - 2.91 (m, 12H), 2.75-2.71 (m, 1H), 2.45 (d, J = 14.4 Hz, 1H), 2.07-2.05 (m, 1H), 1.78-1.76 (m, 2H), 1.51-1.50 (m, 1H), 1.34 (d, J = 6.0 Hz, 3H), 1.22 - 1.03 (m, 9H), 0.97 - 0.80 (m, 6H), 0.34 (s, 3H).

The structure of the other isomeric compound was arbitrarily designated as (1*r*,2*R*,3*S*)-*N*-((6³*S*,4*S*,*Z*)-(*Sₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z470, 15 mg, 0.017 mmol, yield: 26.81%, Rf = 0.40), ES-API: [M+H]⁺ = 904.3.

### Example 40. Synthesis of Compound Z423

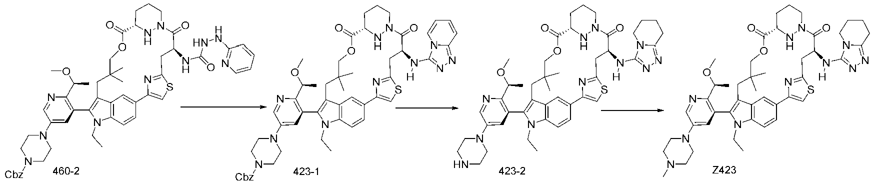

Step 1: Compound 460-2 (215 mg, 0.22 mmol) and pyridine (260 mg, 3.28 mmol) were dissolved in toluene (10 mL), and phosphorus oxychloride (168 mg, 1.09 mmol) was added. The reaction system was stirred at 75 °C for 1 h. The reaction mixture was cooled to room temperature, and saturated sodium bicarbonate (10 mL) was added. The mixture was extracted with dichloromethane/methanol (30 mL × 3, 10:1). The organic phases were mixed, dried over anhydrous sodium sulfate, filtered, and concentrated. The crude product was purified by preparative silica gel thin-layer chromatography (dichloromethane/7 M ammonia-methanol solution = 20:1) to give compound 423-1 (70 mg, yield: 33.2%) as a pale yellow solid. ES-API: [M+H]⁺ = 966.3.

Step 2: Compound 423-1 (60 mg, 0.062 mmol) was dissolved in absolute methanol (12 mL), and 10% palladium on carbon (300 mg) was added. The reaction system was stirred at 40 °C for 24 h under a hydrogen atmosphere. The reaction mixture was filtered through celite and washed with dichloromethane/7 M ammonia-methanol solution (150 mL, 5:1). The filtrate was concentrated to give compound 423-2 (50 mg, yield: 96.3%) as a white solid. ES-API: [M+H]⁺ = 836.3.

Step 3: Compound 423-2 (45 mg, 0.054 mmol) and a 37% formaldehyde solution (13 mg, 0.16 mmol) were dissolved in methanol (5 mL), and acetic acid (10 mg, 0.16 mmol) and sodium cyanoborohydride (6 mg, 0.16 mmol) were added at room temperature. The reaction system was stirred at room temperature for 1 h. The reaction mixture was quenched with water (10 mL), and saturated sodium bicarbonate (1 mL) was added. The mixture was extracted with dichloromethane (50 mL). The organic phase was washed with saturated brine (10 mL), dried over anhydrous sodium sulfate, and concentrated. The crude product was purified by prep-HPLC (ammonium bicarbonate method) to give the desired product (6³S,4S,Z)-4-([1,2,4]triazolo[4,3-a]pyridin-3-ylamino)-1¹-ethyl-(Rₐ)-1²-(2-((S)-1-methoxyethyl)-5-(4-methylpiperazin-1-yl)pyridin-3-yl)-10-10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z423, 10 mg, yield: 21.8%) as a white solid. ES-API: [M+H]⁺ = 850.4. ¹H NMR (400 MHz, DMSO-*d₆*) δ 8.49 (d, *J =* 1.2 Hz, 1H), 8.45 (d, *J* = 2.8 Hz, 1H), 7.78 (s, 1H), 7.72 (dd, *J =* 8.8, 1.2 Hz, 1H), 7.54 (d, *J =* 8.8 Hz, 1H), 7.22 (d, *J =* 2.8 Hz, 1H), 6.33 (d, *J =* 10.8 Hz, 1H), 5.31 (t, *J =* 10.0 Hz, 1H), 5.07 (d, *J =* 12.0 Hz, 1H), 4.38 - 4.06 (m, 5H), 3.91 - 3.80 (m, 1H), 3.72 - 3.63 (m, 1H), 3.62 - 3.50 (m, 2H), 3.39 - 3.34 (m, 1H), 3.29 - 3.19 (m, 8H), 2.95 (d, *J =* 14.3 Hz, 1H), 2.86 - 2.74 (m, 1H), 2.71 - 2.59 (m, 2H), 2.48 - 2.40 (m, 5H), 2.22 (s, 3H), 2.10 (d, *J =* 10.0 Hz, 1H), 1.95 - 1.86 (m, 2H), 1.83 - 1.71 (m, 4H), 1.59 - 1.48 (m, 1H), 1.33 (d, *J =* 6.0 Hz, 3H), 0.95 - 0.85 (m, 6H), 0.35 (s, 3H).

### Example 41. Synthesis of Compound Z333

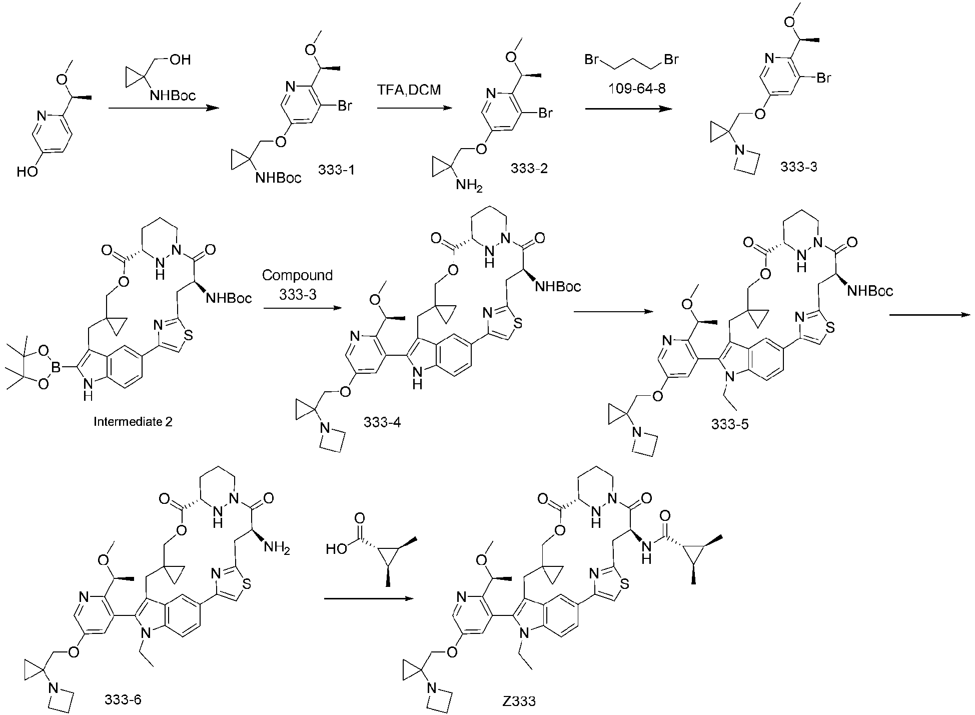

Step 1: rac-(R)-6-(1-Methoxyethyl)pyridin-3-ol (1300 mg, 5.602 mmol), methyl 2-methylpropan-2-yl {[(hydroxymethyl)cyclopropyl]amino}carbamate (1048.83 mg, 5.602 mmol), and triphenylphosphine (2644.83 mg, 10.084 mmol) were dissolved in dichloromethane (10 mL), and diisopropyl azodicarboxylate (2265.56 mg, 11.204 mmol) was added. The mixture was reacted at room temperature for 18 h under a nitrogen atmosphere. Water (15 mL) was added, and the mixture was extracted with ethyl acetate (20 mL × 3). The organic phase was washed with water (15 mL) and saturated brine (15 mL), dried over anhydrous sodium sulfate, and purified by silica gel column chromatography (ethyl acetate:petroleum ether = 1:3) to give compound 333-1 (1200 mg, 3.722 mmol, yield: 66.45%). ES-API: [M+H]⁺ = 401.2.

Step 2: Compound 333-1 (1200 mg, 2.990 mmol) was dissolved in dichloromethane (20 mL), and trifluoroacetic acid (10 mL, 2.990 mmol) was added. The mixture was reacted at room temperature for 1 h. The reaction mixture was concentrated under reduced pressure, adjusted to pH 8 with a sodium bicarbonate solution, and extracted with ethyl acetate (20 mL × 3). The organic phase was washed with water (15 mL) and saturated brine (15 mL), dried over anhydrous sodium sulfate, and concentrated under reduced pressure to give compound 333-2 (800 mg, 2.656 mmol, yield: 88.83%), ES-API: [M+H]⁺ = 301.2.

Step 3: Compound 333-2 (300 mg, 0.996 mmol) was dissolved in acetonitrile (3 mL), and 1,3-dibromopropane (260.28 mg, 1.295 mmol) and N,N-diisopropylethylamine (642.47 mg, 4.980 mmol) were added. The mixture was reacted at 70 °C for 18 h. Water (15 mL) was added, and the mixture was extracted with ethyl acetate (20 mL × 3). The organic phase was washed with water (15 mL) and saturated brine (15 mL), dried over anhydrous sodium sulfate, and purified by silica gel column chromatography (dichloromethane:methanol = 10:1) to give compound 333-3 (100 mg, 0.293 mmol, yield: 29.42%). ES-API: [M+H]⁺ = 341.2.

Step 4: Intermediate 2 (50 mg, 0.072 mmol) and compound 333-3 (11.14 mg, 0.029 mmol) were dissolved in toluene (1.5 mL), 1,4-dioxane (0.5 mL), and water (0.5 mL), and potassium phosphate (46.03 mg, 0.217 mmol) and [1,1'-bis(di-tert-butylphosphino)ferrocene]dichloropalladium(II) (6.58 mg, 0.010 mmol) were added. The mixture was reacted at 70 °C for 3 h under a nitrogen atmosphere. The reaction mixture was concentrated under reduced pressure, and the residue was purified by silica gel column chromatography (ethyl acetate:petroleum ether = 1:1) to give compound 333-4 (30 mg, 0.036 mmol, yield: 50.00%). ES-API: [M+H]⁺ = 826.3.

Step 5: Compound 333-4 (30 mg, 0.036 mmol) was dissolved in N,N-dimethylformamide (1 mL), and cesium carbonate (35.50 mg, 0.109 mmol) and iodoethane (10 mg, 0.064 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 333-5 (30 mg, 0.035 mmol, yield: 96.72%). ES-API: [M+H]⁺ = 854.3.

Step 6: Compound 333-5 (30 mg, 0.035 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 333-6 (25 mg, 0.033 mmol, yield: 94.40%). ES-API: [M+H]⁺ = 754.3. Step 7: Compound 333-6 (25 mg, 0.033 mmol) was dissolved in dichloromethane (1 mL), and (1R,2R,3S)-2,3-dimethylcyclopropane-1-carboxylic acid (3.23 mg, 0.028 mmol), O-(7-azabenzotriazol-1-yl)-N,N,N',N'-tetramethyluronium hexafluorophosphate (18.91 mg, 0.050 mmol), and N,N-diisopropylethylamine (21.43 mg, 0.166 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, and concentrated under reduced pressure, and the residue was purified by silica gel column chromatography (dichloromethane:methanol = 98:2) to give (1*r*,2*R*,3*S*)-*N*-((6³*S,*4*S*,*Z*)-1²-(5-((1-(azetidin-1-yl)cyclopropyl)methoxy)-2-((*S*)-1-methoxyethyl)pyridin-3-yl)-1'-ethyl-10,10-dimethyl-5,7-dioxa-6¹,6²,6³,6⁴,6⁵,6⁶-hexahydro-1¹*H*-8-oxa-2(4,2)-thiazola-1(5,3)-indola-6(1,3)-pyridazinacycloundecaphan]-4'-yl)-2,3-dimethylcyclopropane-1-carboxamide (Z333, 11 mg, 0.013 mmol, yield: 39.02%). ES-API: [M+H]⁺ = 850.3.

### Example 42. Synthesis of Compounds Z504 and Z547

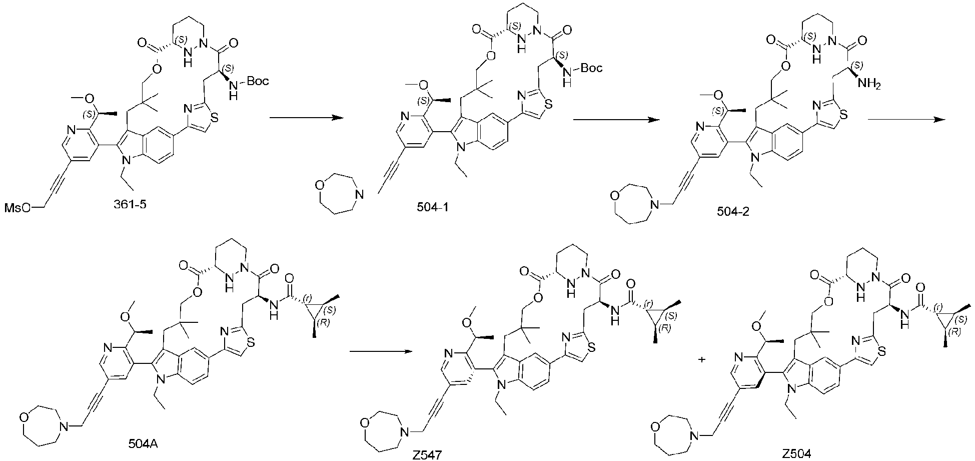

Step 1: A solution of compound 361-5 (30 mg, 0.035 mmol), 1,4-oxazepane (17.6 mg, 0.174 mmol), and N,N-diisopropylethylamine (22.4 mg, 0.174 mmol) in dichloromethane (10 mL) was stirred at room temperature for 16 h under a nitrogen atmosphere. After the reaction was completed, the mixture was poured into 20 mL of dichloromethane and washed sequentially with 30 mL of water and 30 mL of saturated brine. The organic phase was dried over anhydrous sodium sulfate, filtered, and concentrated, and the residue was purified by a flash silica gel column (0-6% methanol/dichloromethane) to give compound 504-1 (30 mg, 0.035 mmol, yield: 99%). ES-API [M+H]⁺ = 868.5.

Step 2: Compound 504-1 (30 mg, 0.035 mmol) was dissolved in a solution of hydrochloric acid in dioxane (3 mL, 4 mol/L). The mixture was stirred at room temperature for 2 h. After the reaction was completed, the reaction mixture was concentrated to give compound 504-2 (26 mg, yield: 98%). ES-API [M+H]⁺ = 768.4.

Step 3: Compound 504-2 (26 mg, 0.035 mmol) was dissolved in N,N-dimethylformamide (3 mL), and (1r,2R,3S)-2,3-dimethylcyclopropane-1-carboxylic acid (4.8 mg, 0.04 mmol), N,N-diisopropylethylamine (23 mg, 0.18 mmol), and O-(7-azabenzotriazol-1-yl)-N,N,N',N'-tetramethyluronium hexafluorophosphate (40 mg, 0.105 mmol) were sequentially added. The mixture was stirred at room temperature for 1 h. After the reaction was completed, 10 mL of ethyl acetate was added, and the mixture was washed with 10 mL of saturated brine and 10 mL of water. The organic phase was dried over anhydrous sodium sulfate and concentrated to give crude compound 504A. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1 ,3)-pyridazinacycloundecaphane-4-yl)-2,3-dimethylcyclopropane-1-carboxamide (Z504, 3 mg, yield: 10%, purity: 98%, retention time: 7.921 min). ES-API [M+H]⁺ = 864.9. ¹H NMR (400 MHz, DMSO-d₆) δ 8.80 (d, *J =* 2.1 Hz, 1H), 8.50 (d, *J =* 1.7 Hz, 1H), 8.39 (d, *J =* 8.9 Hz, 1H), 7.84 (d, *J =* 2.1 Hz, 1H), 7.81 (s, 1H), 7.75 (dd, *J =* 8.7, 1.7 Hz, 1H), 7.58 (d, *J =* 8.7 Hz, 1H), 5.56 (t, *J =* 9.1 Hz, 1H), 5.07 (d, *J =* 12.1 Hz, 1H), 4.37 - 4.04 (m, 5H), 3.71 - 3.66 (m, 4H), 3.65 - 3.62 (m, 2H), 3.59 - 3.56 (m, 2H), 3.33 - 3.29 (m, 1H), 3.25 (s, 3H), 3.15 (dd, *J =* 14.7, 9.2 Hz, 1H), 2.96 (d, *J =* 14.5 Hz, 1H), 2.79 - 2.71 (m, 5H), 2.39 (d, *J =* 14.4 Hz, 1H), 2.08 (d, *J =* 12.0 Hz, 1H), 1.88-1.73 (m, 4H), 1.57-1.46 (m, 1H), 1.35 (d, *J =* 6.1 Hz, 3H), 1.21-1.14 (m, 3H), 1.1-1.05 (m, 6H), 0.94 - 0.85 (m, 6H), 0.35 (s, 3H).

The structure of the other isomeric compound was arbitrarily designated as (1r,2R,3S)-N-((6³S,4S,Z)-(Sₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z547, 7 mg, yield: 23%, purity: 98%, retention time: 8.019 min). ES-API [M+H]⁺ = 864.9. ¹H NMR (400 MHz, DMSO-d6) δ 8.82 (d, J = 2.0 Hz, 1H), 8.54 (s, 1H), 8.41 (d, J = 9.0 Hz, 1H), 7.99 (d, J = 2.1 Hz, 1H), 7.83 (s, 1H), 7.75 (dd, J = 8.6, 1.3 Hz, 1H), 7.55 (d, J = 8.7 Hz, 1H), 5.54 (t, J = 9.3 Hz, 1H), 5.05 (d, J = 12.1 Hz, 1H), 4.22 (t, J = 12.1 Hz, 2H), 4.01 - 3.91 (m, 2H), 3.83 (dd, J = 14.7, 7.3 Hz, 1H), 3.72 - 3.62 (m, 7H), 3.56 (d, J = 11.0 Hz, 1H), 3.31 (s, 1H), 3.21 - 3.00 (m, 5H), 2.82 - 2.72 (m, 5H), 2.33 (d, J = 14.3 Hz, 1H), 2.12 (d, J = 10.2 Hz, 1H), 1.88-1.75 (m, 4H), 1.59 - 1.46 (m, 1H), 1.27 - 1.05 (m, 15H), 0.94 (s, 3H), 0.51 (s, 3H).

### Example 43. Synthesis of Compounds Z498 and Z548

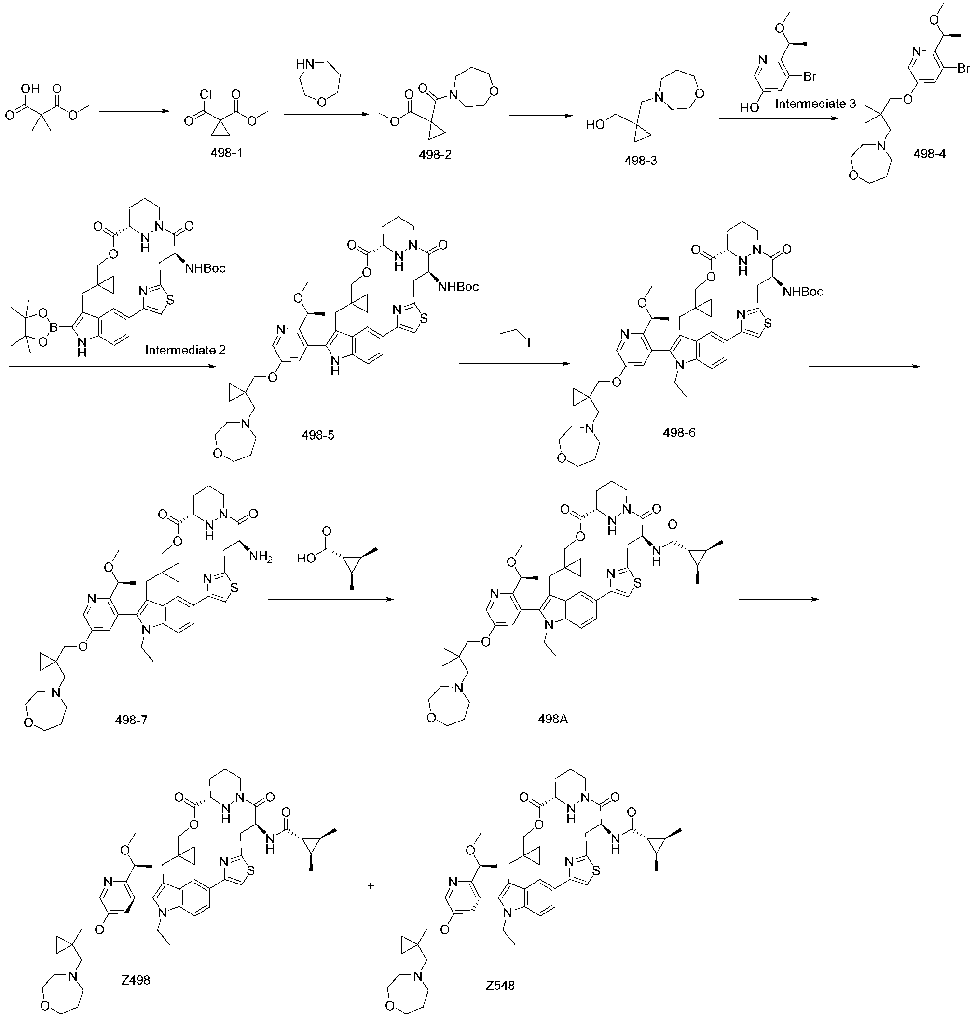

Step 1: Oxalyl chloride (2.6 mL, 31.222 mmol) was added to a solution of 1-(methoxycarbonyl)cyclopropane-1-carboxylic acid (3.0 g, 20.815 mmol) and N,N-dimethylformamide (152 mg, 2.081 mmol) in dichloromethane (20 mL) under an ice-water bath. The reaction mixture was stirred at 0-20 °C for 1 h. The reaction mixture was concentrated under reduced pressure to give compound 498-1 (3.383 g, yield: 99%) as a yellow oil.

Step 2: A solution of compound 498-1 (1.11 g, 6.766 mmol) in dichloromethane (5 mL) and triethylamine (2.05 g, 20.299 mmol) were added to a solution of 1,4-oxazepane (680 mg, 6.766 mmol) in dichloromethane (20 mL) under an ice-water bath. The reaction mixture was stirred at room temperature for 1 h. When the reaction was completed as detected by LCMS, 20 mL of water was added to the reaction mixture. The organic phase was separated, washed with saturated brine (15 mL × 3), dried over anhydrous sodium sulfate, filtered, and concentrated. The residue was purified by automated flash chromatography on silica gel (ethyl acetate:petroleum ether = 0-60%) to give compound 498-2 (820 mg, yield: 53%). ES-API: [M+H]⁺ = 228.3.

Step 3: Lithium aluminum hydride (2.5 mL, 2.464 mmol) was slowly added to a solution of compound 498-2 (560 mg, 2.464 mmol) in tetrahydrofuran (15 mL) at -20 °C under a nitrogen atmosphere. After the dropwise addition was completed, the reaction mixture was slowly warmed to 70 °C and stirred for 5 h. The reaction was completed as detected by LCMS. The reaction mixture was cooled to 0 °C, and 93 mg of water, 280 mg of a 15% sodium hydroxide solution, and 93 mg of water were sequentially and slowly added dropwise to the reaction mixture to quench the reaction. Insoluble substances were removed by filtration and washed with ethyl acetate. The organic phase in the filtrate was then separated, washed with saturated brine (15 mL × 2), dried over anhydrous sodium sulfate, filtered, and concentrated under reduced pressure to give compound 498-3 (598 mg, crude product). ES-API: [M+H]⁺ = 186.3.

Step 4: Compound 498-3 (598 mg, 3.232 mmol), triphenylphosphine (509 mg, 1.939 mmol), and diisopropyl azodicarboxylate (436 mg, 2.154 mmol) were added to a solution of intermediate 3 (250 mg, 1.077 mmol) in dichloromethane (10 mL). The reaction mixture was stirred at room temperature for 18 h under a nitrogen atmosphere. When the reaction was completed as detected by LCMS, the mixture was concentrated under reduced pressure to remove the solvent. The residue was purified by automated flash chromatography on silica gel (tetrahydrofuran:petroleum ether = 0-70%) to give compound 498-4 (360 mg, yield: 84%). ES-API: [M+H]⁺ = 399.2.

Step 5: Intermediate 2 (147 mg, 0.213 mmol), compound 498-4 (85 mg, 0.213 mmol), potassium phosphate (136 mg, 0.639 mmol), and [1,1'-bis(di-tert-butylphosphino)ferrocene]dichloropalladium(II) (14 mg, 0.021 mmol) were dissolved in dioxane (2 mL), toluene (6 mL), and water (2 mL) under a nitrogen atmosphere. The reaction mixture was heated to 70 °C under an oil bath and stirred for 2 h. When the reaction was completed as detected by LCMS, the mixture was cooled to room temperature. 20 mL of ethyl acetate was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran:petroleum ether = 0-70%) to give compound 498-5 (92 mg, yield: 49%). ES-API: [M+H]⁺ = 884.3.

Step 6: Cesium carbonate (102 mg, 0.312 mmol) and iodoethane (24 mg, 0.156 mmol) were sequentially added to a solution of compound 498-5 (92 mg, 0.104 mmol) in N,N-dimethylformamide (2 mL). The reaction mixture was stirred at room temperature overnight. When the reaction was completed as detected by LCMS, the reaction mixture was diluted with 20 mL of ethyl acetate and washed with water (10 mL) and saturated brine (15 mL × 3). The organic phase was dried over anhydrous sodium sulfate, filtered, and concentrated to dryness to give crude compound 498-6 (94 mg, yield: 99%). ES-API: [M+H]⁺ = 912.4.

Step 7: Compound 498-6 (94 mg, 0.103 mmol) was dissolved in a solution of hydrochloric acid in dioxane (3 mL, 4 M) and a solution of hydrochloric acid in methanol (1 mL, 4 M). The reaction mixture was stirred at room temperature for 1 h. When the reaction was completed as detected by LCMS, the reaction mixture was concentrated under reduced pressure to give crude compound 498-7 (83 mg, yield: 99%). ES-API: [M+H]⁺ = 812.3.

Step 8: (1r,2R,3S)-2,3-Dimethylcyclopropane-1-carboxylic acid (15 mg, 0.133 mmol), N,N-diisopropylethylamine (79 mg, 0.613 mmol), and O-(7-azabenzotriazol-1-yl)-N,N,N',N'-tetramethyluronium hexafluorophosphate (117 mg, 0.307 mmol) were sequentially added to a solution of compound 498-7 (83 mg, 0.102 mmol) in N,N-dimethylformamide (2 mL) under an ice-water bath. The reaction mixture was warmed to room temperature and stirred for 10 min. When the reaction was completed as detected by LCMS, the reaction mixture was diluted with 20 mL of ethyl acetate and washed with water (10 mL) and saturated brine (10 mL × 3). The organic phase was dried over anhydrous sodium sulfate, filtered, and concentrated to dryness to give crude compound 498A. The crude product was purified by prep-HPLC (ammonium bicarbonate method) to give 2 isomeric compounds. The structure of one of the isomeric compounds was arbitrarily designated as (1r,2R,3S)-N-((3'S,4'S,Z)-(Rₐ)-2'-(5-((1-((1,4-oxolan-4-yl)methyl)cyclopropyl)methoxy)-2-((S)-1-methoxyethyl)phenyl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z498, 18.85 mg, yield: 20%, retention time: 2.16 min), ES-API [M+H]⁺ = 908.4. ¹H NMR (400 MHz, DMSO-d₆) δ 8.52 (s, 1 H), 8.44 (d, J= 2.8 Hz, 1 H), 8.37 (d, J= 8.8 Hz, 1 H), 7.80 (s, 1 H), 7.73 (dd, J1= 1.2 Hz, J2= 8.8 Hz, 1 H), 7.56 (d, J= 8.8 Hz, 1 H), 7.43 (d, J= 2.8 Hz, 1 H), 5.51 (t, J= 9.2 Hz, 1 H), 5.01 (d, J= 12.0 Hz, 1 H), 4.29- 4.22 (m, 2 H), 4.13- 3.98 (m, 5 H), 3.94- 3.85 (m, 1 H), 3.55 (t, J= 6.0 Hz, 2 H), 3.50- 3.47 (m, 2 H), 3.37- 3.29 (m, 1 H), 3.19- 3.10 (m, 2 H), 3.02 (s, 3 H), 2.76- 2.72 (m, 1 H), 2.70- 2.62 (m, 4 H), 2.53- 2.45 (m, 1 H), 2.05- 2.02 (m, 1 H), 1.90- 1.86 (m, 1 H), 1.76- 1.65 (m, 4 H), 1.52- 1.43 (m, 1 H), 1.38 (d, J= 6.4 Hz, 3 H), 1.27- 1.01 (m, 14 H), 0.60- 0.58 (m, 2 H), 0.42- 0.39 (m, 3 H), 0.27- 0.21 (m, 2 H), -0.62-0.66 (m, 1 H).

The structure of the other isomeric compound was arbitrarily designated as: (1r,2R,3S)-N-((3'S,4'S,Z)-(Sₐ)-2'-(5-((1-((1,4-oxolan-4-yl)methyl)cyclopropyl)methoxy)-2-((S)-1-methoxyethyl)phenyl)-1'-ethyl-5',7'-dioxaspiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z548, 32.38 mg, yield: 35%, retention time: 2.23 min), ES-API [M+H]⁺ = 908.4. ¹H NMR (400 MHz, DMSO-d₆) δ 8.55 (d, J= 0.8 Hz,1 H), 8.47 (d, J= 2.8 Hz, 1 H), 8.39 (d, J= 9.2 Hz, 1 H), 7.81 (s, 1 H), 7.73 (dd, J1= 1.2 Hz, J2= 8.4 Hz, 1 H), 7.54 (d, J= 8.8 Hz, 1 H), 7.49 (d, J= 2.8 Hz, 1 H), 5.50 (t, J= 9.6 Hz, 1 H), 4.99 (d, J= 12.4 Hz, 1 H), 4.25- 4.22 (m, 1 H), 4.14-3.89 (m, 6 H), 3.84- 3.79 (m, 1 H), 3.57 (t, J= 5.6 Hz, 2 H), 3.51 (t, J= 4.8 Hz, 2 H), 3.47- 3.43 (m, 1 H), 3.24- 3.21 (m, 1 H), 3.17- 3.11 (m, 1 H), 2.97 (s, 3 H), 2.67- 2.61 (m, 4 H), 2.56- 2.53 (m, 1 H), 2.46- 2.43 (m, 1 H), 2.09- 2.06 (m, 1 H), 1.89-1.86 (m, 1 H), 1.78- 1.66 (m, 4 H), 1.54- 1.44 (m, 1 H), 1.27 (d, J= 6.4 Hz, 3 H), 1.23- 1.06 (m, 14 H), 0.63- 0.59 (m, 2 H), 0.44- 0.41 (m, 3 H), 0.35- 0.29 (m, 2 H), -0.19-0.22 (m, 1 H).

### Example 44. Synthesis of Compounds Z549 and Z309

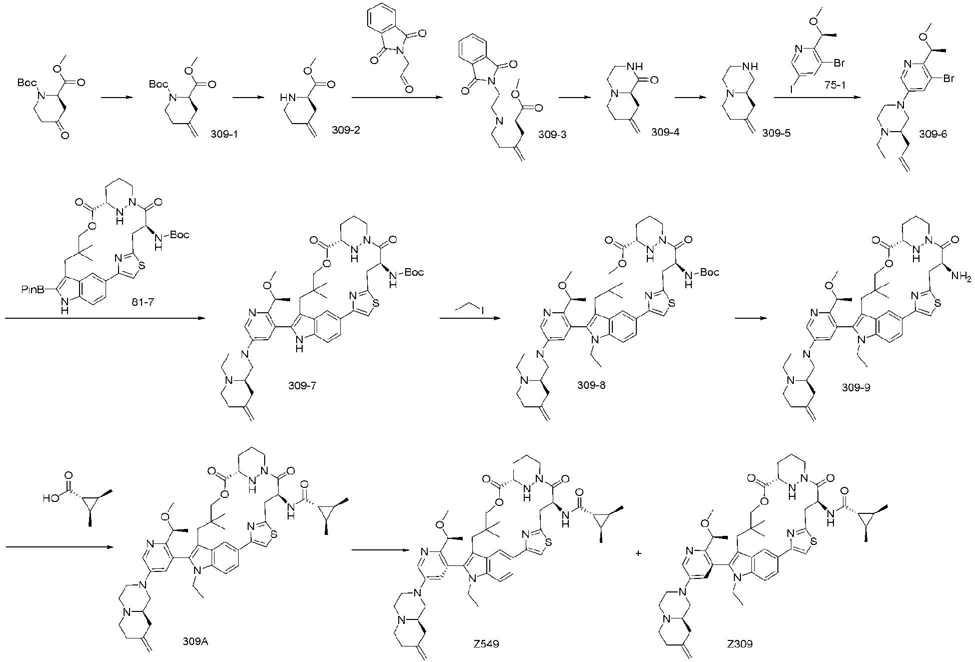

Step 1: A 1 M solution (7.77 mL, 7.77 mmol) of potassium tert-butoxide in tetrahydrofuran was added to a solution of methyltriphenylphosphonium bromide (2.78 g, 7.77 mmol) in tetrahydrofuran (20 mL) under an ice bath. The reaction mixture was stirred at room temperature for 2 h. A solution of methyl (2R)-1-{[(2-methylpropan-2-yl)oxy]carbonyl}-4-oxohexahydropyridine-2-carboxylate (1 g, 3.89 mmol) in tetrahydrofuran (10 mL) was then added to the reaction mixture described above, and the mixture was stirred for another 1 h. The reaction mixture was concentrated, and 20 mL of ethyl acetate was added. The mixture was washed three times with 10 mL of water. The organic phase was dried over anhydrous sodium sulfate, filtered, and concentrated, and the residue was purified by a flash silica gel column (0-9% tetrahydrofuran/petroleum ether) to give compound 309-1 (550 mg) as a transparent oil. ES-API: [M+H-Boc]⁺ = 156.3.

Step 2: Trifluoroacetic acid (5 mL, 65 mmol) was added dropwise to a solution of compound 309-1 (550 mg, 2.15 mmol) in dichloromethane (5 mL) under an ice bath. The reaction mixture was stirred at room temperature for 2 h and then concentrated under reduced pressure to give compound 309-2 (550 mg, trifluoroacetate) as a yellow oil. ES-API: [M+H]⁺ = 156.2.

Step 3: 2-(1,3-Dioxo-2,3-dihydro-1H-isoindolin-2-yl)acetaldehyde (3.51 g, 18.57 mmol) was added to a solution of trifluoroacetate of compound 309-2 (2.5 g, 9.29 mmol) in dichloromethane (50 mL), and the mixture was stirred overnight at room temperature. Sodium triacetoxyborohydride (3.94 g, 18.57 mmol) was then added to the reaction mixture, and the mixture was stirred for another 1 h. After the reaction was completed, the reaction was quenched with 50 mL of a saturated ammonium chloride solution, and the mixture was extracted three times with 30 mL of dichloromethane. The organic phases were combined, dried over anhydrous sodium sulfate, filtered, and concentrated, and the residue was purified by a flash silica gel column (0-80% ethyl acetate/petroleum ether) to give compound 309-3 (2.8 g) as a transparent oil. ES-API: [M+H]⁺ = 329.2.

Step 4: An 80% hydrazine hydrate solution (15 mL, 0.39 mmol) was added to a solution of compound 309-3 (2.8 g, 8.53 mmol) in tetrahydrofuran (8 mL). The mixture was stirred at room temperature for 2 h. After the reaction was completed, the mixture was concentrated, and the residue was purified by prep-HPLC (ammonium bicarbonate method) to give compound 309-4 (900 mg) as a white solid. ES-API: [M+H]⁺ = 167.3.

Step 5: A 1 M solution (1.81 mL, 1.81 mmol) of lithium aluminum hydride in tetrahydrofuran was added dropwise to a solution of compound 309-4 (300 mg, 1.81 mmol) in tetrahydrofuran (5 mL) under a nitrogen atmosphere. The mixture was then warmed to 70 °C and stirred for 1 h. The mixture was cooled under an ice bath, and the reaction was quenched with sodium sulfate decahydrate. The mixture was filtered and concentrated to give compound 309-5 (250 mg) as a yellow oil. ES-API: [M+H]⁺ = 153.3.

Step 6: A mixture of compound 75-1 (150 mg, 0.44 mmol), compound 309-5 (67 mg, 0.44 mmol), tris(dibenzylideneacetone)dipalladium(0) (40 mg, 0.04 mmol), 2,2'-bis(diphenylphosphino)-1,1'-binaphthyl (27 mg, 0.04 mmol), and cesium carbonate (429 mg, 1.32 mmol) was stirred at 80 °C overnight in dioxane (2 mL) under a nitrogen atmosphere. After the reaction was completed, the mixture was filtered and concentrated, and the residue was purified by a flash silica gel column (0-20% tetrahydrofuran/petroleum ether) to give compound 309-6 (80 mg). ES-API: [M+H]⁺ = 366.1. Step 7: A mixture of compound 81-7 (65 mg, 0.09 mmol), compound 309-6 (69 mg, 0.19 mmol), [1,1'-bis(di-tert-butylphosphino)ferrocene]dichloropalladium(II) (6 mg, 0.01 mmol), and potassium phosphate (60 mg, 0.28 mmol) was stirred at 70 °C for 2 h in dioxane (1.5 mL), toluene (0.5 mL), and water (0.5 mL) under a nitrogen atmosphere. After the reaction was completed, the reaction mixture was poured into 10 mL of ethyl acetate, and the mixture was washed sequentially with 5 mL of saturated brine and 5 mL of water. The organic phase was dried over anhydrous sodium sulfate, filtered, and concentrated, and the residue was purified by a flash silica gel column (0-10% methanol/dichloromethane) to give compound 309-7 (50 mg) as a transparent oil. ES-API: [M+H]⁺ = 853.3.

Step 8: Compound 309-7 (76 mg, 0.09 mmol) was dissolved in N,N-dimethylformamide (2 mL), and cesium carbonate (87 mg, 0.27 mmol) and iodoethane (69 mg, 0.45 mmol) were added. The mixture was stirred at room temperature overnight. The reaction mixture was dissolved in 20 mL of ethyl acetate, and the mixture was washed sequentially with 20 mL of saturated brine and 20 mL of water, dried over anhydrous sodium sulfate, filtered, and concentrated. The residue was purified by a flash silica gel column (0-10% methanol/petroleum ether) to give compound 309-8 (60 mg) as a yellow solid. ES-API: [M+H]⁺ = 881.3.

Step 9: Compound 309-8 (60 mg, 0.07 mmol) obtained above was dissolved in methanol (0.4 mL), and a 4 M solution of hydrogen chloride in dioxane (2 mL, 8 mmol) was added. The mixture was stirred at room temperature for 2 h. After the reaction was completed, the mixture was concentrated to give crude compound 309-9 (53 mg). ES-API: [M+H]⁺ = 781.3.

Step 10: N,N-Diisopropylethylamine (0.06 mL, 0.34 mmol) and HATU (39 mg, 0.10 mmol) were sequentially added to a solution of (1r,2R,3S)-2,3-dimethylcyclopropane-1-carboxylic acid (12 mg, 0.10 mmol) and compound 309-9 (53 mg, 0.07 mmol) in dichloromethane (2 mL), and the mixture was stirred at room temperature for 2 h. After the reaction was completed, the mixture was washed with 2 mL of water and concentrated to give crude compound 309A. The crude product was purified by prep-HPLC (hydrochloric acid method) to give 2 isomeric compounds. The structure of one of the isomeric compounds was arbitrarily designated as (1r,2R,3S)-*N*-((6³*S*,4*S*,*Z*)-1¹-ethyl-(*Sₐ*)-1²-(2-((*S*)*-*1-methoxyethyl)-5-((R)-8-methyleneoctahydro-2H-pyrido[1,2-a]pyrazin-2-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (hydrochloride, Z549, 10.57 mg, yield: 17%, purity: 95%, retention time: 7.52 min). ES-API: [M+H]⁺ = 877.4. ¹H NMR (400 MHz, DMSO-d₆) δ 12.18 (s, 1H), 8.58 - 8.52 (m, 2H), 8.42 (d, J = 8.8 Hz, 1H), 8.16 (s, 1H), 7.85 (s, 1H), 7.78 (d, J = 8.4 Hz, 1H), 7.59 (d, J = 8.8 Hz, 1H), 5.55 (t, J = 9.2 Hz, 1H), 5.05 (d, J = 11.2 Hz, 1H), 4.93 (s, 1H), 4.90 (s, 1H), 4.32 - 4.16 (m, 5H), 4.10 - 4.00 (m, 1H), 3.95 - 3.85 (m, 1H), 3.72 (d, J = 10.8 Hz, 1H), 3.59 - 3.46 (m, 4H), 3.41 - 3.25 (m, 7H), 3.22 - 3.08 (m, 2H), 3.02 - 2.90 (m, 1H), 2.82 - 2.66 (m, 2H), 2.50 - 2.42 (m, 1H), 2.28 (d, J = 14.0 Hz, 1H), 2.12 (d, J = 11.8 Hz, 1H), 1.87 - 1.75 (m, 2H), 1.62 - 1.45 (m, 1H), 1.32 - 1.16 (m, 10H), 1.15 - 1.04 (m, 7H), 0.97 (s, 3H), 0.53 (s, 3H).

The structure of the other compound was arbitrarily designated as (1r,2R,3S)-*N*-((6³*S*,4*S*,*Z*)-1¹-ethyl-(*Rₐ*)-1²-(2-((*S*)-1-methoxyethyl)-5-((R)-8-methyleneoctahydro-2H-pyrido[1,2-a]pyrazin-2-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (hydrochloride, Z309, 10.57 mg, yield: 17%, purity: 95%, retention time: 7.70 min). ES-API: [M+H]⁺ = 877.4.

### Example 45. Synthesis of Compounds Z550 and Z551

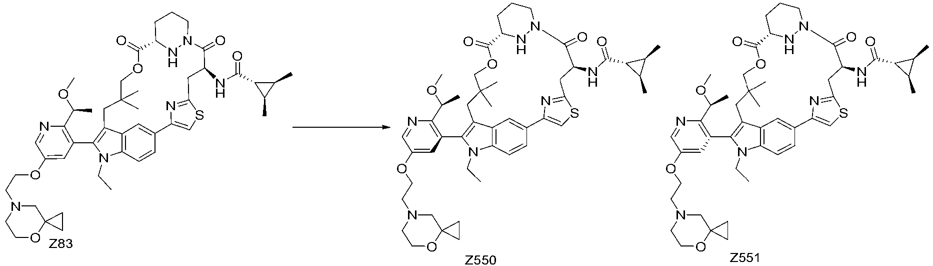

Step 1: Compound Z83 (16.0 mg, 0.018 mmol) was resolved by an SFC chiral column (CHIRALPAK^{®} IB 250 × 30 mm, 10 µm; mobile phase A: HEX + 0.2% DEA, mobile phase B: ETOH + 0.2% DEA; detection wavelength: 214 nm/254 nm; flow rate: 25 mL/min; injection volume: 10 µL; column temperature: 30 °C; run time: 15 min; isocratic elution program: mobile phase A:mobile phase B = 50:50 (V/V)) to give 2 isomeric compounds. The structure of one of the isomeric compounds was arbitrarily designated as (1r,2R,3S)-N-((6³R,4R,Z)-(*Rₐ*)-1²-(5-(2-(4-oxa-7-azaspiro[2.5]octan-7-yl)ethoxy)-2-((S)-1-methoxyethyl)-3-ylpyridin)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z550, 8.34 mg, 0.00945 mmol, yield: 52.12%, retention time: 4.216 min) as a white solid. ES-API: [M+H]⁺ = 882.3. ¹H NMR (400 MHz, DMSO-d₆) δ 8.54~8.53 (m, 1H), 8.49~8.47 (m, 1H), 8.41~8.39(m, 1H), 7.82 (s, 1H), 7.75~7.72 (m, 1H), 7.59 - 7.47 (m, 2H), 5.57~5.52(m, 1H), 5.05-5.02 (m, 1H), 4.26-4.19 (m, 4H), 4.03 - 3.80 (m, 3H), 3.73 - 3.53 (m, 4H), 3.19~3.15 (m, 1H), 3.08 (s, 3H), 3.05~3.02(m, 1H), 2.77-2.72 (m, 3H), 2.44~2.41 (m, 3H), 2.14~2.11 (m, 1H), 1.82~1.80(m, 2H), 1.61 - 1.45 (m, 1H), 1.28 - 1.03 (m, 17H), 0.96~0.93 (m, 3H), 0.65~0.63 (m, 2H), 0.54~0.51 (m, 3H), 0.48~0.46(m, 2H).

The structure of the other isomeric compound was arbitrarily designated as (1r,2R,3S)-N-((6³R,4R,Z)-(*Sₐ*)-1²-(5-(2-(4-oxa-7-azaspiro[2.5]octan-7-yl)ethoxy)-2-((S)-1-methoxyethyl)-3-ylpyridin)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z551, 4.07 mg, 0.0046 mmol, yield: 25.43%, retention time: 6.879 min) as a white solid. ES-API: [M+H]⁺ = 882.3.

### Example 46. Synthesis of Compounds Z434 and Z431

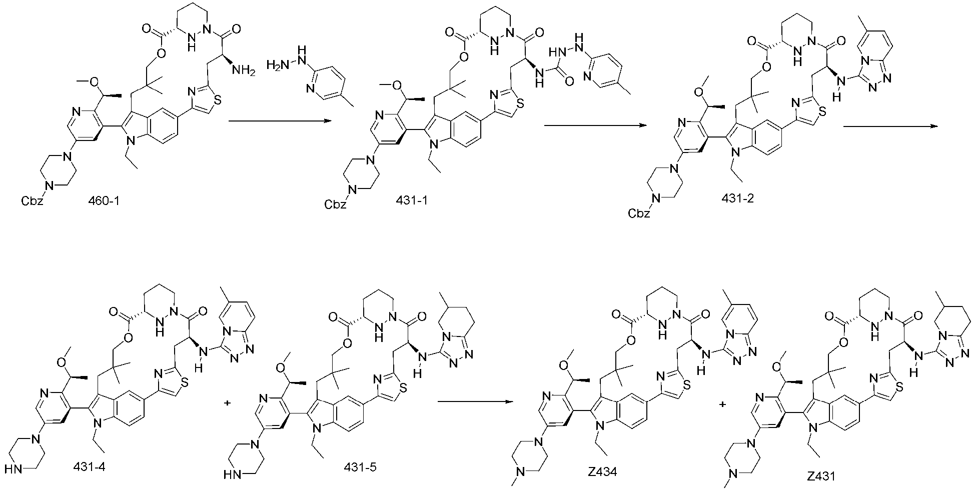

Step 1: Compound 460-1 (250 mg, 0.29 mmol) and N,N-diisopropylethylamine (114 mg, 0.88 mmol) were dissolved in dichloromethane (10 mL), and a solution of p-nitrophenyl chloroformate (77 mg, 0.38 mmol) in dichloromethane (1 mL) was added dropwise at 0 °C. The reaction system was stirred for 1 h under an ice bath, and then 2-hydrazineyl-5-methylpyridine (109 mg, 0.88 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (tetrahydrofuran/petroleum ether: 0-100%) to give compound 431-1 (230 mg, yield: 78.4%) as a pale yellow solid. ES-API: [M+H]⁺ = 998.3.

Step 2: Compound 431-1 (200 mg, 0.20 mmol) and pyridine (238 mg, 3.0 mmol) were dissolved in toluene (10 mL) and phosphorus oxychloride (154 mg, 1.00 mmol). The reaction system was stirred at 75 °C for 2 h. The reaction mixture was cooled to room temperature, and the organic layer was discarded. Dichloromethane/7 M ammonia-methanol solution (15 mL, 5:1) was added to the remaining solid, and the mixture was stirred at room temperature for 30 min. The solution was concentrated, and the crude product was subjected to preparative silica gel thin-layer chromatography (dichloromethane/7 M ammonia-methanol solution = 20:1) to give compound 431-2 (60 mg, yield: 30.5%) as a pale yellow solid. ES-API: [M+H]⁺ = 980.3.

Step 3: Compound 431-2 (50 mg, 0.051 mmol) was dissolved in absolute methanol (6 mL), and 10% palladium on carbon (150 mg) was added. The reaction system was stirred at 45 °C for 24 h under a hydrogen atmosphere. The reaction mixture was filtered through celite and washed with dichloromethane/7 M ammonia-methanol solution (250 mL, 5:1). The filtrate was concentrated to give a mixture (43 mg, yield: 99.2%) of compound 431-4 and compound 431-5 as a white solid. ES-API: [M+H]⁺ = 846.3, 850.4.

Step 4: A mixture of compound 431-4 and compound 431-5 (43 mg, 0.051 mmol) and a 37% formaldehyde solution (13 mg, 0.16 mmol) were dissolved in methanol (5 mL), and acetic acid (9 mg, 0.15 mmol) and sodium cyanoborohydride (6 mg, 0.16 mmol) were added at room temperature. The reaction system was stirred at room temperature for 1 h. The reaction mixture was quenched with water (10 mL), and saturated sodium bicarbonate (1 mL) was added. The mixture was extracted with dichloromethane (50 mL). The organic phase was washed with saturated brine (10 mL), dried over anhydrous sodium sulfate, filtered, and concentrated. The crude product was first purified by preparative silica gel thin-layer chromatography (ethyl acetate/7 M ammonia-methanol solution = 10:1) to give crude product 1 (Rf = 0.65) and crude product 2 (Rf = 0.55). Then crude product 1 was purified by prep-HPLC (ammonium bicarbonate method) to give a compound with a structure arbitrarily designated as (6³S,4S,Z)-1¹-ethyl-(Rₐ)-1²-(2-((S)-1-methoxyethyl)-5-(4-methylpiperazin-1-yl)pyridin-3-yl)-10,10-dimethyl-4-((6-methyl-[1,2,4]triazolo[4,3-a]pyridin-3-yl)amino)-6¹,6²,6¹,6⁴,6⁵,6⁶-hexahydro-1¹H-8-oxa-2(4,2)-thiazola-1(5,3)-indola-6(1,3)-pyridazinacycloundecaphane-5,7-dione (Z434, 1.9 mg, yield: 4.4%) as a white solid. ES-API: [M+H]⁺ = 860.3. Crude product 2 was purified by prep-HPLC (ammonium bicarbonate method) to give a compound with a structure arbitrarily designated as (6³S,4S,Z)-1¹-ethyl-(Rₐ)-1²-(2-((S)-1-methoxyethyl)-5-(4-methylpiperazin-1-yl)pyridin-3-yl)-10,10-dimethyl-4-((6-methyl-5,6,7,8-tetrahydro-[1,2,4]triazolo[4,3-a]pyridin-3-ylamino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z431, 11 mg, yield: 25.2%) as a white solid. ¹H NMR (400 MHz, DMSO-d₆) δ 8.49 (d, *J =* 1.2 Hz, 1H), 8.45 (d, *J* = 2.8 Hz, 1H), 7.80 - 7.76 (m, 1H), 7.72 (dd, *J =* 8.4, 1.2 Hz, 1H), 7.54 (d, *J* = 8.8 Hz, 1H), 7.22 (d, *J =* 2.8 Hz, 1H), 6.36 - 6.23 (m, 1H), 5.32 (t, *J =* 9.6 Hz, 1H), 5.07 (d, *J =* 12.0 Hz, 1H), 4.33 - 4.05 (m, 5H), 3.88 (dd, *J =* 11.6, 5.2 Hz, 1H), 3.64 - 3.51 (m, 2H), 3.40 - 3.36 (m, 1H), 3.28 - 3.15 (m, 9H), 2.94 (d, *J* = 14.0 Hz, 1H), 2.84 - 2.73 (m, 2H), 2.68 - 2.57 (m, 1H), 2.49 - 2.40 (m, 5H), 2.23 (s, 3H), 2.14 - 2.01 (m, 2H), 1.91 - 1.72 (m, 3H), 1.59 - 1.40 (m, 2H), 1.33 (d, *J =* 6.0 Hz, 3H), 1.11 - 1.06 (m, 3H), 0.96 - 0.84 (m, 6H), 0.35 (s, 3H). ES-API: [M+H]⁺ = 864.3.

### Example 47. Synthesis of Compounds Z554 and Z555

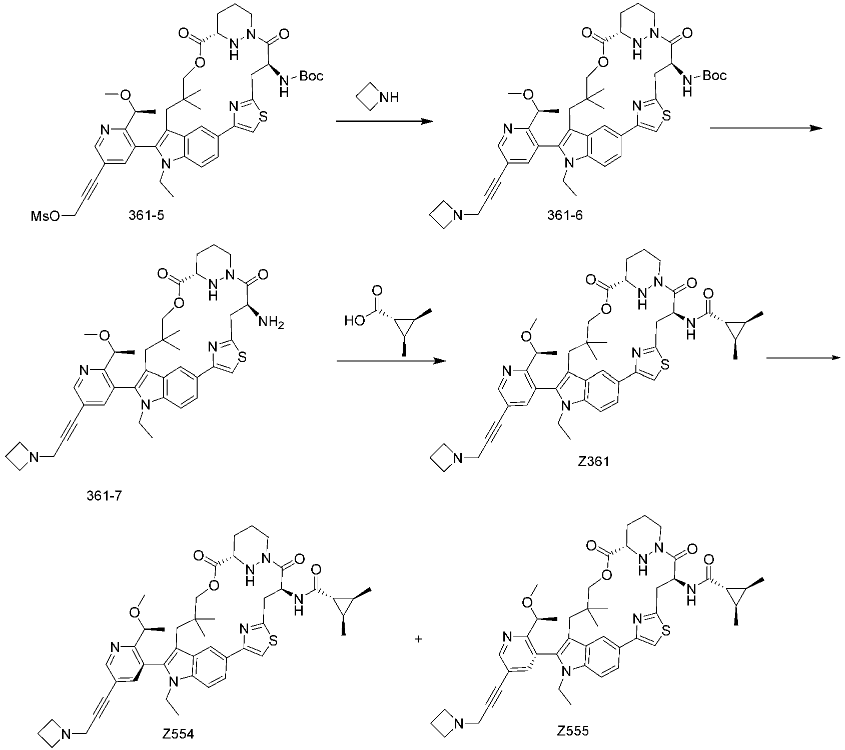

Step 1: Compound 361-5 (180 mg, 0.208 mmol) was dissolved in dichloromethane (10 mL), and then N,N-diisopropylethylamine (0.182 mL, 1.043 mmol) and azetidine (24 mg, 0.42 mmol) were added. The mixture was stirred at room temperature for 3 h. The reaction mixture was concentrated, and the residue was purified by flash column chromatography (0-10% methanol/dichloromethane) to give compound 361-6 (158 mg, 0.192 mmol, yield: 91.9%) as a yellow oil. ES-API [M+H]⁺ = 824.3.

Step 2: Compound 361-6 (158 mg, 0.192 mmol) was dissolved in anhydrous dichloromethane (3 mL), and trifluoroacetic acid (3 mL) was added at 0 °C. The reaction system was stirred at room temperature for 2 h. The reaction mixture was concentrated, and the residue was extracted with dichloromethane/isopropanol (60 mL/10 mL) and washed sequentially with saturated sodium bicarbonate (20 mL) and saturated brine (10 mL). The organic phase was dried over anhydrous sodium sulfate and concentrated to give the desired product of compound 361-7 (140 mg, crude product) as a yellow liquid. ES-API: [M+H]⁺ = 724.4.

Step 3: Compound 361-7 (140 mg, crude product) and (1r,2R,3S)-2,3-dimethylcyclopropane-1-carboxylic acid (28.5 mg, 0.25 mmol) were dissolved in dichloromethane (15 mL), and ethyldiisopropylamine (0.098 mL, 0.581 mmol) and HATU (100 mg, 0.263 mmol) were sequentially added. The reaction system was stirred at room temperature for 3 h. The reaction system was diluted with water (20 mL) and extracted with dichloromethane (20 mL × 3). The organic layer was separated, washed with a saturated sodium chloride solution, and concentrated in vacuum to give crude compound Z361. The crude product was subjected to prep-HPLC (ammonium bicarbonate method) to give 2 isomeric compounds. The structure of one of the isomeric compounds was arbitrarily designated as (1r,2R,3S)-N-((6³S,4S,Z)-(Rₐ)-1²-(5-(3-(azetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z554, 3.05 mg, retention time: 8.429 min, yield: 1.9%) as a white solid. ES-API: [M+H]⁺ = 820.4. ¹H NMR (400 MHz, DMSO-d₆) δ 8.81 (d, J= 2.0 Hz, 1 H), 8.50 (d, J= 1.2 Hz, 1 H), 8.38 (d, J= 8.8 Hz, 1 H), 7.85 (d, J= 2.0 Hz, 1 H), 7.81 (s, 1 H), 7.75 (dd, J1= 1.6 Hz, J2= 8.8 Hz, 1 H), 7.58 (d, J= 8.4 Hz, 1 H), 5.56 (t, J= 9.2 Hz, 1 H), 5.06 (t, J= 12.0 Hz, 1 H), 4.36- 4.05 (m, 5 H), 3.58 (s, 2 H), 3.45 (m, 2 H), 3.33- 3.29 (m, 1 H), 3.26- 3.22 (m, 7 H), 3.17- 3.11 (m, 1 H), 2.95 (d, J= 14.4 Hz, 1 H), 2.79- 2.72 (m, 1 H), 2.39 (d, J= 14.0 Hz, 1 H), 2.09- 2.06 (m, 1 H), 2.00- 1.93 (m, 2 H), 1.80- 1.75 (m, 2 H), 1.53- 1.49 (m, 1 H), 1.36- 1.35 (m, 3 H), 1.23- 1.14 (m, 3 H),, 1.09- 1.05 (m, 6 H), 0.91- 0.86 (m, 6 H), 0.34 (s, 3H).

The structure of the other isomeric compound was arbitrarily designated as (1r,2R,3S)-N-((6³S,4S,Z)-(Sₐ)-1²-(5-(3-(azetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵ 6⁶-hexahydro-1¹H-8-oxa-2(4,2)-thiazola-1 (5,3)-indola-6(1,3)-pyridazinacycloundecaphane-4-yl)-2,3-dimethylcyclopropane-1-carboxamide (Z555, 3.35 mg, retention time: 8.204 min, yield: 2.13%) as a white solid. ES-API: [M+H]⁺ = 820.4. ¹H NMR (400 MHz, DMSO-d₆) δ 8.83 (d, J= 2.0 Hz, 1 H), 8.53 (d, J= 1.2 Hz, 1 H), 8.40 (d, J= 9.2 Hz, 1 H), 7.99 (d, J= 2.0 Hz, 1 H), 7.82 (s, 1 H), 7.74 (dd, J1= 1.6 Hz, J2= 8.8 Hz, 1 H), 7.54 (d, J= 8.8 Hz, 1 H), 5.54 (t, J= 9.2 Hz, 1 H), 5.03 (d, J= 12.0 Hz, 1 H), 4.25- 4.18 (m, 2 H), 3.99- 3.92 (m, 2 H), 3.84- 3.79 (m, 1 H), 3.67 (d, J= 10.8 Hz, 1 H), 3.55 (d, J= 10.8 Hz, 1 H), 3.45 (s, 2 H), 3.24 (t, J= 6.8 Hz, 4 H), 3.18- 3.02 (m, 6 H), 2.80- 2.73 (m, 1 H), 2.33- 2.30 (m, 1 H), 2.13- 2.10 (m, 1 H), 2.00- 1.92 (m, 2 H), 1.81- 1.76 (m, 2 H), 1.57- 1.48 (m, 1 H), 1.22- 1.16 (m, 6 H), 1.13- 1.06 (m, 9 H), 0.93 (s, 3 H), 0.50 (s, 3 H).

### Example 48. Synthesis of Compound Z385

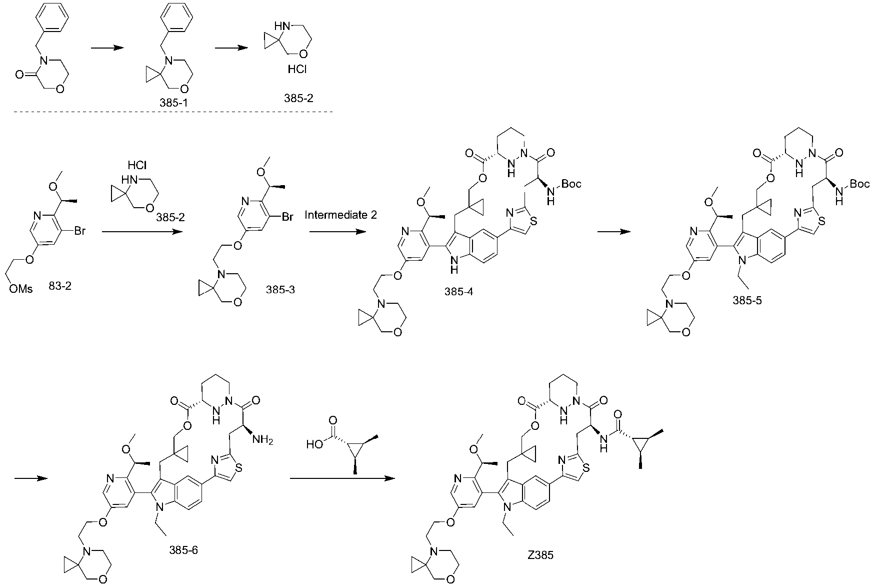

Step 1: 4-Benzylmorpholin-3-one (2000 mg, 10.459 mmol) and tetraisopropyl titanate (5945.09 mg, 20.917 mmol) were dissolved in anhydrous tetrahydrofuran (40 mL), and ethylmagnesium bromide (1 M, 20.92 mL, 20.917 mmol) was slowly added to the solution described above at 0 °C under a nitrogen atmosphere. After the addition was completed, the reaction system was warmed to room temperature and stirred for another 3 h. After the reaction was completed, the reaction was quenched with a saturated ammonium chloride solution (30 mL). The resulting black solid was filtered using a sand core funnel, and the filtrate was extracted with ethyl acetate (30 mL × 2). The organic phase was washed with saturated brine (50 mL), dried over anhydrous sodium sulfate, filtered, and concentrated to give a crude product. The crude product was purified by a flash silica gel column (ethyl acetate/petroleum ether: 0%-35%) to give compound 385-1 (1.7 g, 8.363 mmol, yield: 79.81%) as a colorless transparent liquid. ES-API: [M+H]⁺ = 204.3.

Step 2: Compound 385-1 (1.6 g, 7.871 mmol) was dissolved in ethanol (20 mL), and wet palladium hydroxide/carbon (1105 mg, 10 wt%, 0.787 mmol) was added to the solution described above. After the addition was completed, the reaction system was stirred for 17 h under a hydrogen atmosphere (15 psi). After the reaction was completed, the reaction mixture was filtered using a sand core funnel. A solution of hydrogen chloride in ethyl acetate (4 M, 10 mL) was added to the resulting filtrate. Finally, the mixture was concentrated to give compound 385-2 (1.46 g, 7.846 mmol, yield: 99.68%) as a white solid. ES-API: [M+H]⁺ = 114.1.

Step 3: Compound 83-2 (100 mg, 0.282 mmol) was dissolved in N,N-dimethylformamide (5 mL), and compound 385-2 (63.36 mg, 0.423 mmol), potassium carbonate (117.05 mg, 0.847 mmol), and sodium iodide (52.49 mg, 0.282 mmol) were added to the solution described above. After the addition was completed, the reaction system was stirred at 80 °C for 17 h. After the reaction was completed, water (20 mL) was added to the solution described above, and the mixture was extracted with ethyl acetate (20 mL × 3). The organic phase was washed with saturated brine (30 mL × 2), dried over anhydrous sodium sulfate, filtered, and concentrated to give a crude product. The crude product was purified by a flash silica gel column (methanol/dichloromethane: 0%-3%) to give compound 385-3 (100 mg, 0.269 mmol, yield: 95.40%) as a light yellow solid. ES-API: [M+H]⁺ = 371.3, 373.3.

Step 4: Compound 385-3 (48.45 mg, 0.131 mmol) was dissolved in dioxane (1 mL), toluene (0.3 mL), and water (0.3 mL). Intermediate 2 (60 mg, 0.087 mmol), [1,1'-bis(di-tert-butylphosphino)ferrocene]dichloropalladium(II) (5.60 mg, 0.009 mmol), and potassium phosphate (55.24 mg, 0.260 mmol) were added to the solution described above at room temperature. After the addition was completed, the reaction system was stirred at 70 °C for 2 h under an argon atmosphere. After the reaction was completed, water (10 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 385-4 (40 mg, 0.047 mmol, yield: 53.86%) as a yellow solid. ES-API: [M+H]⁺ = 856.3.

Step 5: Compound 385-4 (40 mg, 0.047 mmol) was dissolved in N,N-dimethylformamide (2 mL). Iodoethane (36.44 mg, 0.234 mmol) and cesium carbonate (76.12 mg, 0.234 mmol) were added to the solution described above. The reaction system was stirred at room temperature for 8 h. After the reaction was completed, water (10 mL) was added to the solution described above, and the mixture was extracted with ethyl acetate (20 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-3%) to give compound 385-5 (40 mg, 0.045 mmol, yield: 96.83%) as a light yellow solid. ES-API: [M+H]⁺ = 884.3.

Step 6: Compound 385-5 (40 mg, 0.045 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give crude compound 385-6 (40 mg) as a white solid. ES-API: [M+H]⁺ = 784.3.

Step 7: Crude compound 385-6 (40 mg) was dissolved in dichloromethane (2 mL). (1r,2R,3S)-2,3-Dimethylcyclopropane-1-carboxylic acid (7.86 mg, 0.069 mmol), O-(7-azabenzotriazol-1-yl)-N,N,N',N'-tetramethyluronium hexafluorophosphate (26.19 mg, 0.069 mmol), and N,N'-diisopropylethylamine (19.51 mg, 0.151 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was subjected to prep-HPLC (ammonium bicarbonate method) to give (1r,2R,3S)-N-((3'S,4'S,Z)-2'-(5-(2-(7-oxa-4-azaspiro[2.5]octan-4-yl)ethoxy)-2-((S)-1-methoxyethyl)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2,3-dimethylcyclopropane-1-carboxamide (Z385, 7.3 mg, 0.008 mmol, two-step yield: 18%) as a white solid. ES-API: [M+H]⁺ = 880.3.

### Example 49. Synthesis of Compound Z557

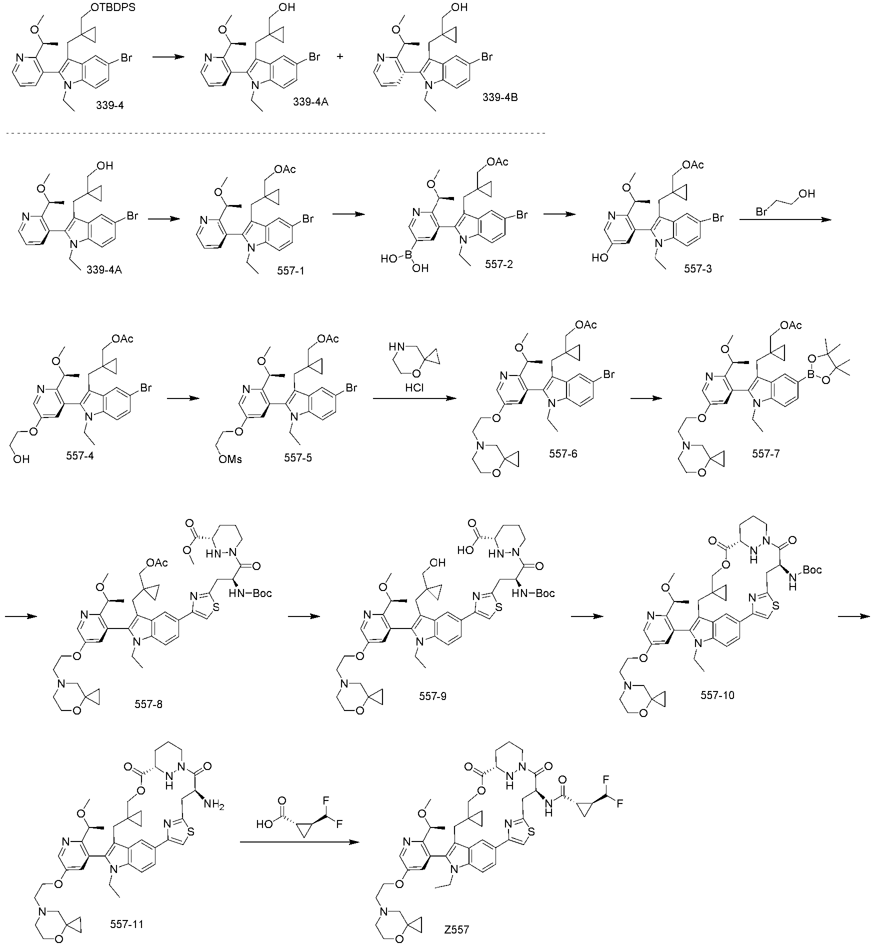

Step 1: Compound 339-4 (8800 mg, 12.907 mmol) was dissolved in a solution of tetrabutylammonium fluoride in tetrahydrofuran (1 M, 60 mL). The reaction system was stirred at 50 °C for 17 h under a nitrogen atmosphere. After the reaction was completed, water (200 mL) was added to the solution described above, and the mixture was extracted with ethyl acetate (100 mL × 2). The organic phase was washed with saturated brine (200 mL × 5), dried over anhydrous sodium sulfate, filtered, and concentrated to give a crude product. The crude product was purified by a flash silica gel column (ethyl acetate/petroleum ether: 0%-70%) to give compound 339-4A (2600 mg, 5.864 mmol, yield: 45.43%, Rf value: 0.6, a structure arbitrarily designated) and compound 339-4B (2600 mg, 5.864 mmol, yield: 45.43%, Rf value: 0.5, a structure arbitrarily designated) as yellow oily liquids. ES-API: [M+H]⁺ = 442.1, 444.3.

Step 2: Compound 339-4A (2500 mg, 5.639 mmol), triethylamine (1.567 mL, 11.277 mmol), and 4-dimethylaminopyridine (68.89 mg, 0.564 mmol) were dissolved in dichloromethane (40 mL). Acetic anhydride (6.355 mL, 67.662 mmol) was slowly added to the solution described above under an ice bath. After the addition was completed, the reaction system was warmed to room temperature and stirred for another 2 h. After the reaction was completed, the reaction mixture was concentrated. Then water (100 mL) was added to the residue, and the mixture was extracted with dichloromethane (100 mL × 2). The organic phase was washed sequentially with a saturated aqueous sodium bicarbonate solution (100 mL × 2) and a saturated sodium chloride solution (200 mL), dried over anhydrous sodium sulfate, filtered, and concentrated to give a crude product. The crude product was purified by a flash silica gel column (ethyl acetate/petroleum ether: 0%-40%) to give compound 557-1 (2600 mg, 5.356 mmol, yield: 94.99%) as a yellow solid. ES-API: [M+H]⁺ = 485.1, 487.3.

Step 3: Compound 557-1 (2500 mg, 5.150 mmol), bis(pinacolato)diboron (2223.32 mg, 8.755 mmol), chloro(1,5-cyclooctadiene)iridium(I) dimer (221.17 mg, 0.824 mmol), and 4,4'-di-tert-butyl-2,2'-bipyridine (50.32 mg, 0.187 mmol) were dissolved in n-hexane (45 mL). The reaction system was stirred at 80 °C for 17 h under a nitrogen atmosphere. After the reaction was completed, the reaction system was concentrated to remove n-hexane to give compound 557-2 (5000 mg, crude product) as a black oily liquid. ES-API: [M+H]⁺ = 529.1, 531.1.

Step 4: Crude compound 557-2 (5000 mg) was dissolved in acetonitrile (30 mL), and hydrogen peroxide (1750.62 mg, 30 wt%, 15.447 mmol) was added to the solution described above under an ice bath. After the addition was completed, the reaction system was warmed to room temperature and stirred for 3 h. After the reaction was completed, the reaction was quenched with saturated sodium sulfite (30 mL). The mixture was extracted with ethyl acetate (50 mL × 2). The organic phase was washed with saturated brine (50 mL × 2), dried over anhydrous sodium sulfate, filtered, and concentrated to give a crude product. The crude product was purified by a flash silica gel column (methanol/dichloromethane: 0%-4%) to give compound 557-3 (1700 mg, 3.390 mmol, two-step yield: 65.8%) as a yellow solid. ES-API: [M+H]⁺ = 501.1,503.1.

Step 5: Compound 557-3 (1000 mg, 1.994 mmol) was dissolved in N,N-dimethylformamide (15 mL). 2-Bromoethanol (0.707 mL, 9.972 mmol) and potassium carbonate (826.85 mg, 5.983 mmol) were added to the solution described above. After the addition was completed, the reaction system was stirred at 70 °C for 6 h. After the reaction was completed, water (50 mL) was added to the reaction mixture, and the mixture was extracted with ethyl acetate (30 mL × 2). The organic phase was washed with saturated brine (50 mL × 2), dried over anhydrous sodium sulfate, filtered, and concentrated to give a crude product. The crude product was purified by a flash silica gel column (methanol/dichloromethane: 0%-3%) to give compound 557-4 (850 mg, 1.558 mmol, yield: 78.14%) as a light yellow solid. ES-API: [M+H]⁺ = 545.1, 547.1.

Step 6: Compound 557-4 (400 mg, 0.733 mmol) was dissolved in anhydrous dichloromethane (5 mL), and the mixture was cooled to 0 °C. Triethylamine (0.306 mL, 2.200 mmol) and methanesulfonic anhydride (255.47 mg, 1.467 mmol) were added to the solution described above. After the addition was completed, the reaction system was stirred at room temperature for another 2 h. After the reaction was completed, water (20 mL) was added to the reaction mixture, and the mixture was extracted with dichloromethane (30 mL × 2). The organic phase was washed with saturated sodium bicarbonate (20 mL) and saturated brine (20 mL), dried over anhydrous sodium sulfate, filtered, and concentrated to give compound 557-5 (400 mg, 0.641 mmol, yield: 87.48%) as a colorless transparent oily liquid. ES-API: [M+H]⁺ = 623.1, 625.1.

Step 7: Compound 557-5 (200 mg, 0.321 mmol) was dissolved in N,N-dimethylformamide (5 mL), and 4-oxa-7-azaspiro[2.5]octane hydrochloride (48.03 mg, 0.321 mmol), potassium carbonate (133.09 mg, 0.963 mmol), and potassium iodide (48.11 mg, 0.321 mmol) were added to the solution described above. After the addition was completed, the reaction system was stirred at 80 °C for 17 h. After the reaction was completed, water (20 mL) was added to the reaction mixture, and the mixture was extracted with ethyl acetate (20 mL × 2). The organic phase was washed with saturated brine (20 mL × 2), dried over anhydrous sodium sulfate, filtered, and concentrated to give a crude product. The crude product was purified by a flash silica gel column (methanol/dichloromethane: 0%-3%) to give compound 557-6 (150 mg, 0.234 mmol, yield: 72.94%) as a light yellow solid. ES-API: [M+H]⁺ = 640.1, 642.1.

Step 8: Compound 557-6 (140 mg, 0.219 mmol) was dissolved in anhydrous toluene (5 mL), and bis(pinacolato)diboron (83.24 mg, 0.328 mmol), [1,1'-bis(diphenylphosphino)ferrocene]palladium(II) dichloride (16 mg, 0.022 mmol), and potassium acetate (42.89 mg, 0.437 mmol) were added to the solution described above at room temperature. After the addition was completed, the reaction system was stirred at 90 °C for 3 h under a nitrogen atmosphere. After the reaction was completed, the reaction mixture was filtered using a sand core funnel and concentrated. The residue was purified by silica gel column chromatography (ethyl acetate/petroleum ether: 0-100%) to give compound 557-7 (100 mg, 0.145 mmol, yield: 66.54%) as a yellow oily liquid. ES-API: [M+H]⁺ = 688.3.

Step 9: Compound 557-7 (100 mg, 0.145 mmol) was dissolved in dioxane (3 mL), toluene (1 mL), and water (1 mL). Methyl (S)-1-((S)-3-(4-bromothiazol-2-yl)-2-((tert-butoxycarbonyl)amino)propanoyl)hexahydropyridazine-3-carboxylate (139 mg, 0.291 mmol), [1,1'-bis(di-tert-butylphosphino)ferrocene]dichloropalladium(II) (9.4 mg, 0.015 mmol), and potassium phosphate (77 mg, 0.364 mmol) were added to the solution described above at room temperature. After the addition was completed, the reaction system was stirred at 70 °C for 16 h under an argon atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 557-8 (90 mg, 0.094 mmol, yield: 64.59%) as a yellow solid. ES-API: [M+H]⁺ = 958.1.

Step 10: Compound 557-8 (90 mg, 0.094 mmol) was dissolved in tetrahydrofuran (2 mL) and water (2 mL). Lithium hydroxide (22.93 mg, 0.955 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (20 mL × 2). The organic phase was washed with saturated brine (20 mL), dried over anhydrous sodium sulfate, filtered, and concentrated to give compound 557-9 (90 mg, crude product) as a yellow solid. ES-API: [M+H]⁺ = 902.3.

Step 11: Compound 557-9 (90 mg, crude product), 1-hydroxybenzotriazole (67.41 mg, 0.499 mmol), and N,N-diisopropylethylamine (0.521 mL, 2.993 mmol) were dissolved in dichloromethane (20 mL). 1-(3-Dimethylaminopropyl)-3-ethylcarbodiimide hydrochloride (535.50 mg, 2.793 mmol) was added to the solution described above under an ice bath. After the addition was completed, the reaction system was stirred at room temperature for 17 h. After the reaction was completed, the reaction mixture was washed sequentially with diluted hydrochloric acid (0.5 M, 10 mL × 2) and saturated brine (10 mL × 2). The organic phase was washed with saturated brine (20 mL), dried over anhydrous sodium sulfate, filtered, and concentrated to give a crude product. The crude product was purified by a flash silica gel column (methanol/dichloromethane: 0%-5%) to give compound 557-10 (40 mg, 0.045 mmol, yield: 47.9%) as a light yellow solid. ES-API: [M+H]⁺ = 884.3.

Step 12: Compound 557-10 (40 mg, 0.045 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction mixture was concentrated to give crude compound 557-11 (36 mg, crude product) as a yellow solid. ES-API: [M+H]⁺ = 784.3.

Step 13: Crude product 557-11 (36 mg, crude product) was dissolved in dichloromethane (2 mL). trans-2-(Difluoromethyl)cyclopropane-1-carboxylic acid (9.19 mg, 0.067 mmol), O-(7-azabenzotriazol-1-yl)-*N,N,N',N'-*tetramethyluronium hexafluorophosphate (25.46 mg, 0.067 mmol), and N,N'-diisopropylethylamine (0.022 mL, 0.134 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0-5%) to give (1S,2S)-N-((3'S,4'S,Z)-(Rₐ)-2'-(5-(2-(7-oxa-4-azaspiro[2.5]octan-4-yl)ethoxy)-2-((S)-1-methoxyethyl)pyridin-3-yl)-1'-ethyl-5',7'-dioxospiro[cyclopropane-1,10'-8-oxa-2(4,2)-thiazola-1(5,3)-indola-6(1,3)-pyridazinacycloundecaphan]-4'-yl)-2-trans-(difluoromethyl)cyclopropane-1-carboxamide (Z557, 20 mg, 0.022 mmol, two-step yield: 48.9%) as a white solid. ES-API: [M+H]⁺ = 902.1.

### Example 50. Synthesis of Compound Z556

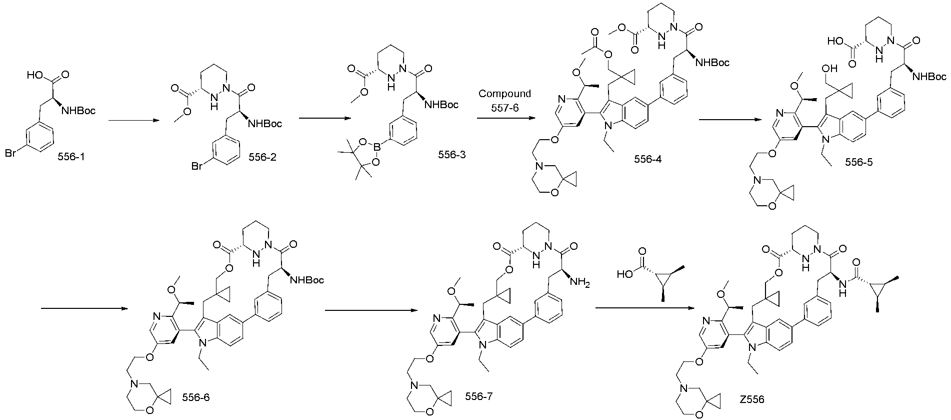

Step 1: Compound 556-1 (1.19 g, 3.46 mmol) and compound 5-c (0.75 g, 5.2 mmol) were dissolved in dichloromethane (20 mL), and 1-(3-dimethylaminopropyl)-3-ethylcarbodiimide hydrochloride (1.33 g, 6.920 mmol), N-methylmorpholine (3.50 g, 34.600 mmol), and 1-hydroxybenzotriazole (0.14 g, 1.038 mmol) were added. The mixture was reacted at room temperature for 2 h. After the reaction was completed, the mixture was concentrated under reduced pressure. Water (5 mL) was added, and the mixture was extracted with ethyl acetate (10 mL × 3). The organic phase was washed with water (5 mL) and saturated brine (5 mL), dried over anhydrous sodium sulfate, and purified by silica gel column chromatography (ethyl acetate:petroleum ether = 1:2) to give compound 556-2 (1.0 g, 2.126 mmol, yield: 61.35%). ES-API: [M+H]⁺ = 470.0.

Step 2: Compound 556-2 (500 mg, 1.063 mmol), bis(pinacolato)diboron (540.01 mg, 2.126 mmol), [1,1'-bis(diphenylphosphino)ferrocene]palladium(II) dichloride (77.78 mg, 0.106 mmol), and potassium acetate (312 mg, 3.19 mmol) were dissolved in 1,4-dioxane (5 mL), and the mixture was reacted at 90 °C for 16 h under a nitrogen atmosphere. The reaction mixture was concentrated under reduced pressure, and the residue was purified by silica gel column chromatography (ethyl acetate:petroleum ether = 1:2) to give compound 556-3 (0.4 g, 0.773 mmol, yield: 72.72%) as a colorless oil. ES-API: [M+H]⁺ = 518.3.

Step 3: Compound 557-6 (70 mg, 0.109 mmol) and compound 556-3 (70 mg, 0.135 mmol) were dissolved in 1,4-dioxane (0.50 mL), toluene (1.50 mL), and water (0.5 mL), and potassium phosphate (69.58 mg, 0.328 mmol) and [1,1'-bis(di-tert-butylphosphino)ferrocene]dichloropalladium(II) (7.11 mg, 0.011 mmol) were added. The mixture was reacted at 70 °C for 3 h under a nitrogen atmosphere. The mixture was concentrated under reduced pressure, and the residue was purified by silica gel column chromatography (ethyl acetate:petroleum ether = 1:2) to give compound 556-4 (75 mg, 0.079 mmol, yield: 72.16%) as an oily liquid. ES-API: [M+H]⁺ = 951.4.

Step 4: Compound 556-4 (50 mg, 0.053 mmol) was dissolved in tetrahydrofuran (1 mL) and water (1 mL), and lithium hydroxide (5.05 mg, 0.210 mmol) was added. The mixture was reacted at room temperature for 2 h. 1 M HCl was added to adjust the pH to 6. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give compound 556-5 (40 mg, 0.045 mmol, yield: 85.02%). ES-API: [M+H]⁺ = 895.5.

Step 5: Compound 556-5 (40 mg, 0.045 mmol) and 1-hydroxybenzotriazole (12.08 mg, 0.089 mmol) were dissolved in dichloromethane (5 mL), and 1-(3-dimethylaminopropyl)-3-ethylcarbodiimide hydrochloride (171.33 mg, 0.894 mmol) and N,N-diisopropylethylamine (115.52 mg, 0.894 mmol) were added. The mixture was reacted at room temperature for 18 h. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give compound 556-6 (30 mg, 0.034 mmol, yield: 76.54%). ES-API: [M+H]⁺ = 877.5.

Step 6: Compound 556-6 (30 mg, 0.034 mmol) was dissolved in methanol (1 mL), and a solution of hydrochloric acid in dioxane (3 mL, 0.034 mmol) was added. The mixture was reacted at room temperature for 1 h. The reaction mixture was concentrated under reduced pressure to give compound 556-7 (26 mg, 0.034 mmol, yield: 100%) as a white solid. ES-API: [M+H]⁺ = 777.3.

Step 7: Compound 556-7 (20 mg, 0.026 mmol) was dissolved in dichloromethane (1 mL), and (1R,2R,3S)-2,3-dimethylcyclopropane-1-carboxylic acid (3.23 mg, 0.028 mmol), O-(7-azabenzotriazol-1-yl)-N,N,N',N'-tetramethyluronium hexafluorophosphate (14.68 mg, 0.039 mmol), and N,N-diisopropylethylamine (13.31 mg, 0.103 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give a crude product. The crude product was subjected to prep-HPLC (formic acid method 1) to give (1*r,*2*R*,3*S*)-*N*-((3'*S*,4'*S*)-(*Rₐ*)-2'-(5-(2-(4-oxa-7-azaspiro[2.5]octan-7-yl)ethoxy)-2-((*S*)-1-methoxyethyl)pyridin-3-yl)-1'-ethyl-5',7'-dioxospiro[cyclopropane-1,10'-8-oxa-1(5,3)-indola-6(1,3)-pyridazina-2(1,3)-benzenacycloundecaphan]-4'-yl)-2,3-dimethylcyclopropane-1-carboxamide (Z556, 12 mg, 0.014 mmol, yield: 52.86%, formate). ES-API: [M+H]⁺ = 873.4. ¹H NMR (400 MHz, DMSO-d₆) δ 8.25 (d, J = 2.4 Hz, 1H), 8.02 (d, J = 8.4 Hz, 1H), 7.67 (s, 1H), 7.46 - 7.33 (m, 4H), 7.23 (d, J = 2.4 Hz, 1H), 7.13 (t, J = 8.0 Hz, 1H), 6.99 (d, J = 7.6 Hz, 1H), 5.16 (t, J = 10.0Hz, 1H), 4.81 (d, J = 12.4 Hz, 1H), 4.21 - 3.95 (m, 4H), 3.91 - 3.67 (m, 4H), 3.41 - 3.39 (m, 2H), 3.10 - 2.97 (m, 2H), 2.86 (s, 3H), 2.70 (d, J = 12.8 Hz, 1H), 2.55 - 2.49(m, 1H), 2.42 - 2.21 (m, 10H), 1.87- 1.77 (m, 2H), 1.59-1.56 (m, 1H), 1.53 - 1.10 (m, 5H), 0.84 - 0.81 (m, 9H), 0.42 - 0.39 (m, 2H), 0.25- 0.23 (m, 3H), 0.07 (s, 1H), -0.05 (s, 1H), -0.88 (s, 1H).

### Example 51. Synthesis of Compound Z435

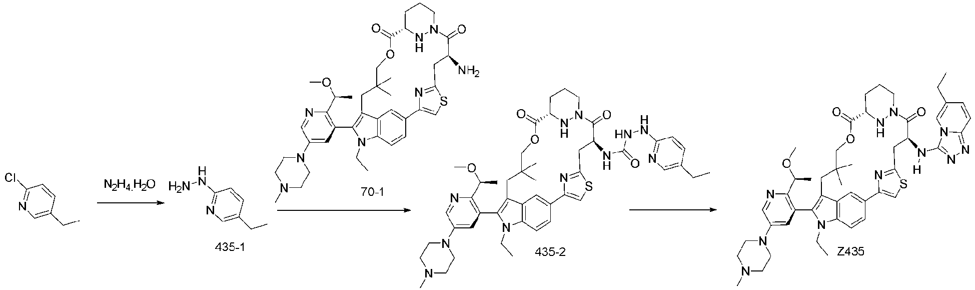

Step 1: 2-Chloro-5-ethylpyridine (900 mg, 6.36 mmol) was dissolved in absolute ethanol (3 mL), and 80% hydrazine hydrate (7.7 mL, 127.12 mmol) was added. The reaction system was stirred at 150 °C for 2 h in a microwave reactor. Dichloromethane (50 mL) was added to the reaction mixture, and the mixture was washed with saturated brine (20 mL). The organic phase was dried over anhydrous sodium sulfate and filtered, and the filtrate was concentrated under reduced pressure. The crude product was purified by a flash silica gel column (methanol/dichloromethane: 0-5%) to give compound 435-1 (710 mg, yield: 81.4%) as a pale yellow solid. ES-API: [M+H]⁺ = 138.3.

Step 2: Compound 70-1 (180 mg, 0.25 mmol) and N,N-diisopropylethylamine (160 mg, 1.24 mmol) were dissolved in dichloromethane (10 mL), and nitrophenyl chloroformate (65 mg, 0.32 mmol) was added at 0 °C. The reaction system was stirred for 1 h under an ice bath, and then compound 435-1 (101 mg, 0.74 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (7 M ammonia-methanol solution/dichloromethane: 0-5%) to give compound 435-2 (22 mg, yield: 10.0%) as a pale yellow solid. ES-API: [M+H]⁺ = 892.3.

Step 3: Compound 435-2 (22 mg, 0.025 mmol) and pyridine (30 mg, 0.37 mmol) were dissolved in toluene (2 mL) and phosphorus oxychloride (19 mg, 0.12 mmol). The reaction system was stirred at 75 °C for 1 h. The reaction mixture was cooled to room temperature, and the organic layer was discarded. Dichloromethane/7 M ammonia-methanol solution (15 mL, 5:1) was added to the remaining solid, and the mixture was stirred at room temperature for 30 min. The solution was concentrated. The residue was purified by a flash silica gel column (7 M ammonia-methanol solution/dichloromethane: 0-5%) to give a crude product (8 mg). The crude product was then purified by prep-HPLC (ammonium bicarbonate method) to give the desired product (6³S,4S,Z)-1¹-ethyl-4-((6-ethyl-[1,2,4]triazolo[4,3-a]pyridin-3-yl)amino)-(Rₐ)-1²-(2-((S)-1-methoxyethyl)-5-(4-methylpiperazin-1-yl)pyridin-3-yl)-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z435, 2.8 mg, yield: 13.0%) as a white solid. ES-API: [M+H]⁺ = 874.3.

### Example 52. Synthesis of Compound Z417

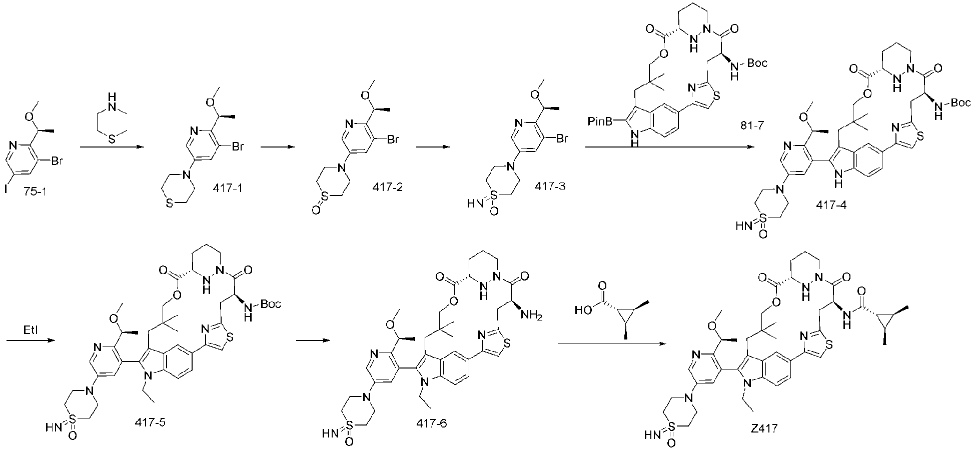

Step 1: A mixture of compound 75-1 (2 g, 5.85 mmol), thiomorpholine (0.91 g, 8.77 mmol), tris(dibenzylideneacetone)dipalladium(0) (0.54 g, 0.59 mmol), 2,2'-bis(diphenylphosphino)-1,1'-binaphthyl (0.36 g, 0.59 mmol), and a 1 M solution (17.55 mL, 17.55 mmol) of sodium tert-butoxide in tetrahydrofuran was stirred at 80 °C for 5 h in dioxane (20 mL) under a nitrogen atmosphere. After the reaction was completed, the mixture was filtered and concentrated, and the residue was purified by a flash silica gel column (0-20% tetrahydrofuran/petroleum ether) to give compound 417-2 (1.4 g). ES-API: [M+H]⁺ = 317.0, 319.0.

Step 2: Sodium periodate (135 mg, 0.63 mmol) was added to compound 417-1 (200 mg, 0.63 mmol) in methanol (5 mL) and water (2 mL), and the mixture was stirred at room temperature overnight. The reaction mixture was poured into 30 mL of dichloromethane, and the mixture was washed with 20 mL of water. The organic phase was dried over anhydrous sodium sulfate, filtered, and concentrated, and the residue was purified by a flash silica gel column (0-10% methanol/dichloromethane) to give compound 417-2 (180 mg). ES-API: [M+H]⁺ = 333.1, 335.0.

Step 3: Ammonium carbonate (865 mg, 9 mmol) and (diacetoxyiodo)benzene (1.75 g, 5.4 mmol) were sequentially added to a solution of compound 417-2 (300 mg, 0.9 mmol) in methanol (10 mL). The reaction mixture was stirred at room temperature for 2 h. Then 10 mL of water was added, and the mixture was extracted three times with 30 mL of dichloromethane. The organic phases were combined, dried over anhydrous sodium sulfate, filtered, and concentrated, and the residue was purified by a flash silica gel column (0-10% methanol/dichloromethane) to give compound 417-3 (200 mg) as a yellow solid. ES-API: [M+H]⁺ = 348.0, 350.0.

Step 4: A mixture of compound 81-7 (50 mg, 0.07 mmol), compound 417-3 (30 mg, 0.09 mmol), [1,1'-bis(di-tert-butylphosphino)ferrocene]dichloropalladium(II) (5 mg, 0.01 mmol), and potassium phosphate (46 mg, 0.22 mmol) was stirred at 70 °C for 2 h in dioxane (1.5 mL), toluene (0.5 mL), and water (0.5 mL) under a nitrogen atmosphere. After the reaction was completed, the reaction mixture was poured into 10 mL of ethyl acetate and washed sequentially with 5 mL of saturated brine and 5 mL of water. The organic phase was dried over anhydrous sodium sulfate, filtered, and concentrated, and the residue was purified by a flash silica gel column (0-10% methanol/dichloromethane) to give compound 417-4 (60 mg) as a transparent oil. ES-API: [M+H]⁺ = 835.3.

Step 4: Compound 417-4 (60 mg, 0.07 mmol) was dissolved in N,N-dimethylformamide (2 mL), and cesium carbonate (70 mg, 0.22 mmol) and iodoethane (112 mg, 0.72 mmol) were added. The mixture was stirred at room temperature for 2 h. The reaction mixture was dissolved in 20 mL of ethyl acetate, and the mixture was washed sequentially with 20 mL of saturated brine and 20 mL of water, dried, and concentrated. The residue was purified by a flash silica gel column (0-10% methanol/petroleum ether) to give compound 417-5 (50 mg) as a yellow solid. ES-API: [M+H]⁺ = 863.3.

Step 5: Compound 417-5 (60 mg, 0.07 mmol) obtained above was dissolved in methanol (0.4 mL), and a 4 M solution of hydrogen chloride in dioxane (2 mL, 8 mmol) was added. The mixture was stirred at room temperature for 2 h. After the reaction was completed, the mixture was concentrated to give compound 417-6 (45 mg, crude product). ES-API: [M+H]⁺ = 763.3.

Step 6: N,N-Diisopropylethylamine (0.05 mL, 0.3 mmol) and HATU (34 mg, 0.09 mmol) were sequentially added to a solution of (1r,2R,3S)-2,3-dimethylcyclopropane-1-carboxylic acid (10 mg, 0.09 mmol) and compound 417-6 (45 mg, 0.06 mmol) in dichloromethane (2 mL), and the mixture was stirred at room temperature for 2 h. After the reaction was completed, the mixture was washed with 2 mL of water and concentrated, and the residue was purified by prep-HPLC (ammonium bicarbonate method) to give (1r,2R,3S)-*N*-((6³*S*,4*S*,*Z*)-1¹-ethyl-1²-(5-(1-imino-1-oxido-1λ⁶-thiomorpholino)-2-((R)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z417, 5 mg, yield: 10%, purity: 95%). ES-API: [M+H]⁺ = 859.3.

### Example 53. Synthesis of Compound Z653

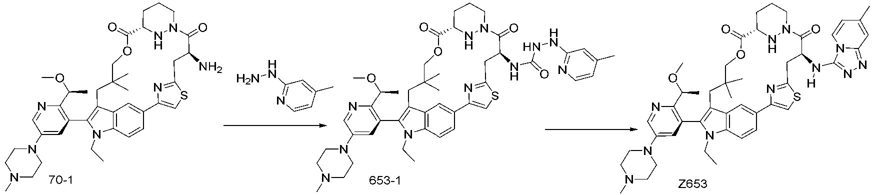

Step 1: Compound 70-1 (100 mg, 0.14 mmol) and N,N-diisopropylethylamine (177 mg, 1.37 mmol) were dissolved in dichloromethane (6 mL), and nitrophenyl chloroformate (67 mg, 0.54 mmol) was added at 0 °C. The reaction system was stirred for 1 h under an ice bath, and then 2-hydrazineyl-4-methylpyridine (101 mg, 0.74 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (7 M ammonia-methanol solution/dichloromethane: 0-7%) to give compound 653-1 (65 mg, yield: 54.3%) as a pale yellow solid. ES-API: [M+H]⁺ = 878.3.

Step 2: Compound 653-1 (50 mg, 0.057 mmol) and pyridine (68 mg, 0.85 mmol) were dissolved in toluene (2 mL) and phosphorus oxychloride (44 mg, 0.29 mmol). The reaction system was stirred at 75 °C for 2 h. The reaction mixture was cooled to room temperature, and the organic layer was discarded. Dichloromethane/7 M ammonia-methanol solution (15 mL, 5:1) was added to the remaining solid, and the mixture was stirred at room temperature for 30 min. The solution was concentrated. The residue was purified by a flash silica gel column (7 M ammonia-methanol solution/dichloromethane: 0-5%) to give a crude product (25 mg). The crude product was then purified by prep-HPLC (ammonium bicarbonate method) to give the desired product (6³S,4S,Z)-1¹-ethyl-(Rₐ)-1^{?}-(2-((S)-1-methoxyethyl)-5-(4-methylpiperazin-1-yl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z653, 8 mg, yield: 16.3%) as a white solid. ¹H NMR (400 MHz, DMSO-d₆) δ 8.51 (s, 1H), 8.45 (d, *J* = 2.8 Hz, 1H), 8.26 (d, *J =* 7.2 Hz, 1H), 7.77 (s, 1H), 7.72 (d, *J =* 8.8 Hz, 1H), 7.54 (d, *J* = 8.8 Hz, 1H), 7.25 - 7.19 (m, 2H), 7.15 (d, *J =* 10.4 Hz, 1H), 6.71 (d, *J =* 7.2 Hz, 1H), 5.53 (t, *J =* 10.0 Hz, 1H), 5.19 (d, *J =* 12.0 Hz, 1H), 4.38 - 4.07 (m, 5H), 3.65 - 3.43 (m, 3H), 3.30 - 3.23 (m, 5H), 3.21 (s, 3H), 2.95 (d, *J =* 14.2Hz, 1H), 2.88 - 2.77 (m, 1H), 2.48 - 2.40 (m, 5H), 2.30 (s, 3H), 2.22 (s, 3H), 2.12 (d, *J =* 11.4 Hz, 1H), 1.88 - 1.74 (m, 2H), 1.65 - 1.49 (m, 1H), 1.33 (d, *J =* 6.0 Hz, 3H), 0.98 - 0.83 (m, 6H), 0.36 (s, 3H). ES-API: [M+H]⁺ = 860.3.

### Example 54. Synthesis of Compounds Z604 and Z604-1

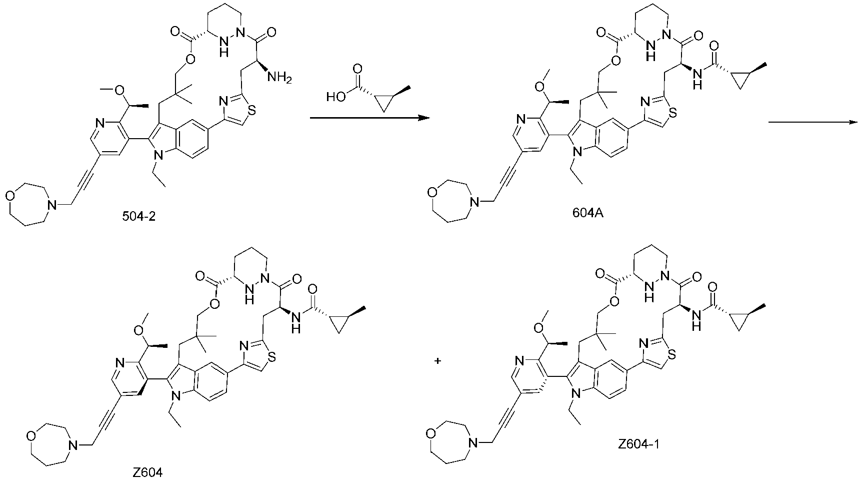

Step 1: Compound 504-2 (90 mg, 0.117 mmol) and (1S,2S)-2-methylcyclopropane-1-carboxylic acid (11.73 mg, 0.117 mmol) were dissolved in dichloromethane (3 mL), and O-(7-azabenzotriazol-1-yl)-N,N,N',N'-tetramethyluronium hexafluorophosphate (66.84 mg, 0.176 mmol) and *N,N'*-diisopropylethylamine (0.5 mL, 0.5 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give crude compound 604A. The crude product was purified by prep-HPLC (ammonium bicarbonate method) to give 2 isomeric compounds. The structure of one of the isomeric compounds was arbitrarily designated as (*1S,2S*)-*N-*((6³*S*,4*S*,*Z*)-(*Rₐ*)-1²-(5-(3-(1,4-oxazepan-4-yl)propyn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z604, 30 mg, 0.035 mmol, yield: 30.11%, retention time = 8.01 min), ES-API: [M+H]⁺ = 850.3. ¹H NMR (400 MHz, DMSO-d₆) δ 8.81 (d, *J =* 2.0 Hz, 1H), 8.55- 8.21 (m, 2H), 7.85 (d, *J =* 2.0 Hz, 1H), 7.81 (s, 1H), 7.76 (dd, *J =* 8.8, 1.6 Hz, 1H), 7.60(d, *J =* 8.8 Hz, 1H), 5.57 (t, *J =* 9.2 Hz, 1H), 5.08 (d, *J =* 12.4 Hz, 1H), 4.36 - 4.02 (m, 5H), 3.971 - 3.58 (m, 8H), 3.36 - 3.30 (m, 1H), 3.34 - 3.20 (m, 1H), 3.26 (s, 3H), 3.18 - 3.0 (m, 1H), 3.07 - 2.95 (m, 1H), 2.78 - 2.75 (m, 5H), 2.40 - 2.36 (m, 1H), 2.12 - 2.07(m, 1H),1.87 - 1.81 (m, 4H), 1.52 - 1.49 (m, 2H), 1.36 (d, *J* = 6.0 Hz, 3H),1.10 - 1.05 (m, 4H), 0.92 - 0.87 (m, 7H), 0.35 (s, 3H).

The structure of the other isomeric compound was arbitrarily designated as (*1S*,*2S*)-*N*-((6³*S*,4*S*,*Z*)-(*Sₐ*)-1²-(5-(3-(1,4-oxazepan-4-yl)propyn-1-yl)-2-((*S*)-1-methoxyethyl)pyridin-3-yl)-1¹-ethyl-10,10-dimethyl-5,7-dioxo-6¹,6²,6³,6⁴,6⁵ 6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z604-1, 30 mg, 0.035 mmol, yield: 30.11%, retention time = 7.83 min), ES-API: [M+H]⁺ = 7.87. ¹H NMR (400 MHz, DMSO-d₆) δ 8.81 (d, J = 2.0 Hz, 1H), 8.55- 8.21 (m, 2H), 7.98 (d, J = 2.0 Hz, 1H), 7.81 (s, 1H), 7.74 (dd, J = 8.8, 1.6 Hz, 1H), 7.54 (d, J = 8.8 Hz, 1H), 5.55 (t, J = 9.2 Hz, 1H), 5.06 (d, J = 12.4 Hz, 1H), 4.25 - 4.19 (m, 2H), 3.97 - 3.91 (m, 2H), 3.85 - 3.79 (m, 1H), 3.70 - 3.63 (m, 7H), 3.55 - 3.53 (m, 1H), 3.34 - 3.20 (m, 1H), 3.18 - 3.0 (m, 5H), 2.76 - 2.74 (m, 5H), 2.37 - 2.21 (m, 2H), 1.87 - 1.81 (m, 4H), 1.52 - 1.49 (m, 2H), 1.22 - 1.21 (m, 3H), 1.17 - 1.02 (m, 7H), 0.93 (s, 3H), 0.87 - 0.86 (m, 1H), 0.70 - 0.42 (m, 4H).

### Example 55. Synthesis of Compounds Z613-1 and Z613-2

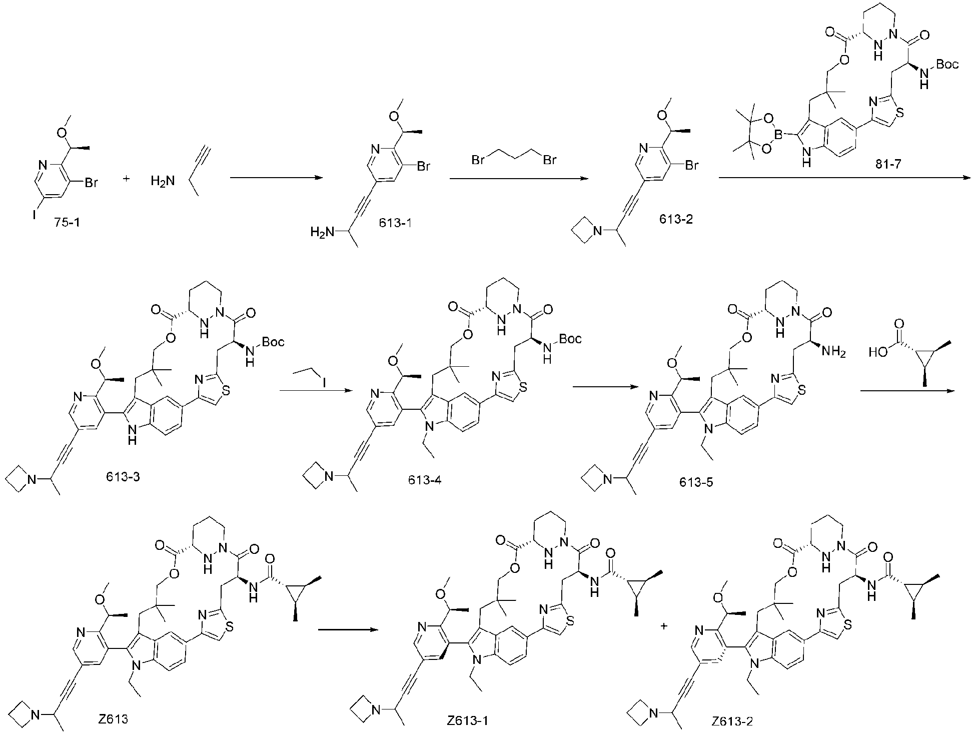

Step 1: Compound 75-1 (1000 mg, 2.924 mmol), but-3-yn-2-amine (222 mg, 3.217 mmol), bis(triphenylphosphine)palladium(II) dichloride (103 mg, 0.146 mmol), copper(I) iodide (56 mg, 0.292 mmol), and N,N-diisopropylethylamine (1 mL, 5.848 mmol) were dissolved in 10 mL of anhydrous acetonitrile under a nitrogen atmosphere. The reaction mixture was stirred at room temperature overnight. When the reaction was completed as detected by LCMS, the mixture was concentrated under reduced pressure to remove the solvent. The residue was purified by automated flash chromatography on silica gel (methanol/dichloromethane = 0-3%) to give compound 613-1 (795 mg, yield: 96%). ES-API: [M+H]⁺ = 283.1.

Step 2: Compound 613-1 (795 mg, 2.808 mmol), 1,3-dibromopropane (0.37 mL, 3.650 mmol), and N,N-diisopropylethylamine (2.5 mL, 14.038 mmol) were dissolved in 6 mL of anhydrous acetonitrile in a sealed tube. The reaction mixture was heated to 65 °C and stirred overnight. When the reaction was completed as detected by LCMS, 25 mL of ethyl acetate was added to the reaction mixture, and the mixture was washed with saturated brine (15 mL × 3), dried over anhydrous sodium sulfate, filtered, and concentrated. The residue was purified by automated flash chromatography on silica gel (methanol/dichloromethane = 0-10%) to give compound 613-2 (134 mg, yield: 15%). ES-API: [M+H]⁺ = 323.1.

Step 3: Compound 613-2 (100 mg, 0.309 mmol), compound 81-7 (107 mg, 0.155 mmol), potassium phosphate (99 mg, 0.465 mmol), and [1,1'-bis(di-tert-butylphosphino)ferrocene]dichloropalladium(II) (20 mg, 0.031 mmol) were dissolved in dioxane (6 mL), toluene (2 mL), and water (2 mL) under a nitrogen atmosphere. The reaction mixture was heated to 70 °C under an oil bath and stirred for 5 h. When the reaction was completed as detected by LCMS, the mixture was cooled to room temperature. Ethyl acetate (20 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-80%) to give compound 613-3 (100 mg, yield: 80%). ES-API: [M+H]⁺ = 810.3.

Step 4: Cesium carbonate (302 mg, 0.926 mmol) and iodoethane (144 mg, 0.926 mmol) were sequentially added to a solution of compound 613-3 (100 mg, 0.123 mmol) in N,N-dimethylformamide (2 mL). The reaction mixture was stirred at room temperature for 1 h. When the reaction was completed as detected by LCMS, the reaction mixture was diluted with 20 mL of ethyl acetate, and then the mixture was washed with water (10 mL) and saturated brine (15 mL × 3). The organic phase was dried over anhydrous sodium sulfate, filtered, and concentrated to dryness. The residue was purified by automated flash chromatography on silica gel (methanol/dichloromethane = 0-2%) to give compound 613-4 (92 mg, yield: 89%). ES-API: [M+H]⁺ = 838.3.

Step 5: Trifluoroacetic acid (1 mL) was slowly added dropwise to a solution of compound 613-4 (92 mg, 0.110 mmol) in dichloromethane (2 mL) under an ice-water bath. The reaction mixture was stirred at room temperature for 1.5 h. When the reaction was completed as detected by LCMS, the mixture was concentrated under reduced pressure at a low temperature (30 °C) to remove the solvent. 5 mL of a saturated sodium bicarbonate solution was added to the residue under an ice-water bath, and the mixture was extracted with dichloromethane (6 mL × 3). The organic phases were combined, washed with saturated brine (6 mL × 2), dried over anhydrous sodium sulfate, filtered, and concentrated to give crude compound 613-5 (70 mg, yield: 86%). ES-API: [M+H]⁺ = 738.3.

Step 6: (1r,2R,3S)-2,3-Dimethylcyclopropane-1-carboxylic acid (14 mg, 0.124 mmol), N,N-diisopropylethylamine (37 mg, 0.285 mmol), and O-(7-azabenzotriazol-1-yl)-N,N,N',N'-tetramethyluronium hexafluorophosphate (54 mg, 0.142 mmol) were sequentially added to a solution of compound 613-5 (70 mg, 0.095 mmol) in N,N-dimethylformamide (2 mL) under an ice-water bath. The reaction mixture was warmed to room temperature and stirred for 10 min. When the reaction was completed as detected by LCMS, the reaction mixture was diluted with 20 mL of ethyl acetate, and then the mixture was washed with water (10 mL) and saturated brine (10 mL × 3). The organic phase was dried over anhydrous sodium sulfate, filtered, and concentrated to dryness to give crude compound Z613. The crude product was purified by prep-HPLC (ammonium bicarbonate method) to give 2 isomeric compounds. The structure of one of the isomeric compounds was arbitrarily designated as: (1r,2R,3S)-N-((6³S,4S,Z)-12-(5-(3-(azetidin-1-yl)but-1-yn-1-yl)-(Ra)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z613-1, 0.55 mg, yield: 0.7%, retention time = 2.15 min), ES-API [M+H]⁺ = 834.4. The structure of the other isomeric compound was arbitrarily designated as: (1r,2R,3S)-N-((6³S,4S,Z)-12-(5-(3-(azetidin-1-yl)but-1-yn-1-yl)-(Sa)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z613-2, 0.80 mg, yield: 1%, retention time = 2.23 min), ES-API [M+H]⁺ = 834.4. ¹H NMR (400 MHz, DMSO-d6) δ 8.82 (d, *J =* 1.1 Hz, 1H), 8.52 (d, *J =* 1.0 Hz, 1H), 8.40 (d, *J* = 9.0 Hz, 1H), 7.99 (d, *J =* 1.9 Hz, 1H), 7.82 (s, 1H), 7.74 (dd, *J =* 8.7, 1.5 Hz, 1H), 7.54 (d, *J =* 8.7 Hz, 1H), 5.53 (t, *J =* 9.3 Hz, 1H), 5.03 (d, *J =* 12.2 Hz, 1H), 4.21 (dd, *J =* 20.0, 7.5 Hz, 2H), 4.05 - 3.45 (m, 12H), 3.28 - 2.98 (m, 6H), 2.80-2.61 (m, 2H), 2.43-2.21 (m, 5H), 2.12 (d, *J* = 9.7 Hz, 1H), 1.79 (s, 2H), 1.61-1.40 (m, 2H), 1.23 (d, *J =* 3.6 Hz, 3H), 1.15 - 1.04 (m, 9H), 0.93 (s, 3H), 0.50 (s, 3H).

### Example 56. Synthesis of Compound Z418

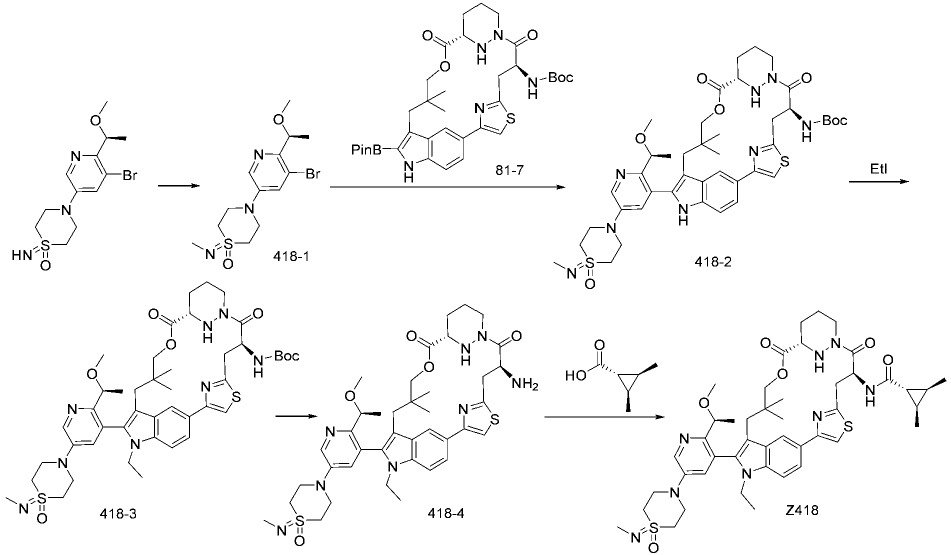

Step 1: A mixed solution of 1-azaidene-4-{5-bromo-6-[(1S)-1-methoxyethyl]pyridin-3-yl}-λ⁶-1,4-thiazin-1-one (120 mg, 0.345 mmol), methylboronic acid (41.25 mg, 0.689 mmol), anhydrous copper(II) acetate (93.88 mg, 0.517 mmol), and pyridine (0.067 mL, 0.827 mmol) was stirred at 100 °C for 1 h in dioxane (2 mL). The reaction mixture was filtered and concentrated, and the residue was purified by a flash silica gel column (0-10% methanol/dichloromethane) to give compound 418-1 (60 mg). ES-API: [M+H]⁺ = 362.0, 364.0.

Step 2: A mixture of compound 81-7 (50 mg, 0.07 mmol), compound 418-1 (30 mg, 0.09 mmol), [1,1'-bis(di-tert-butylphosphino)ferrocene]dichloropalladium(II) (5 mg, 0.01 mmol), and potassium phosphate (46 mg, 0.22 mmol) was stirred at 70 °C for 2 h in dioxane (1.5 mL), toluene (0.5 mL), and water (0.5 mL) under a nitrogen atmosphere. After the reaction was completed, the reaction mixture was poured into ethyl acetate (10 mL) and washed sequentially with saturated brine (5 mL) and water (5 mL). The organic phase was dried over anhydrous sodium sulfate, filtered, and concentrated, and the residue was purified by a flash silica gel column (0-10% methanol/dichloromethane) to give compound 418-2 (60 mg) as a transparent oil. ES-API: [M+H]⁺ = 849.3.

Step 3: Compound 418-2 (60 mg, 0.07 mmol) was dissolved in N,N-dimethylformamide (2 mL), and cesium carbonate (69 mg, 0.22 mmol) and iodoethane (110 mg, 0.71 mmol) were added. The mixture was stirred at room temperature for 2 h. The reaction mixture was dissolved in 20 mL of ethyl acetate, and the mixture was washed sequentially with saturated brine (20 mL) and water (20 mL), dried over anhydrous sodium sulfate, filtered, and concentrated, and the residue was purified by a flash silica gel column (0-10% methanol/petroleum ether) to give compound 418-3 (50 mg) as a yellow solid. ES-API: [M+H]⁺ = 877.3.

Step 4: Compound 418-3 (50 mg, 0.06 mmol) obtained above was dissolved in methanol (0.2 mL), and a 4 M solution of hydrogen chloride in dioxane (0.5 mL, 2 mmol) was added. The mixture was stirred at room temperature for 2 h. After the reaction was completed, the mixture was concentrated to give crude compound 418-4 (44 mg). ES-API: [M+H]⁺ = 777.3.

Step 5: N,N-Diisopropylethylamine (0.05 mL, 0.3 mmol) and HATU (44 mg, 0.12 mmol) were sequentially added to a solution of (1r,2R,3S)-2,3-dimethylcyclopropane-1-carboxylic acid (10 mg, 0.09 mmol) and compound 418-4 (44 mg, 0.06 mmol) in dichloromethane (2 mL), and the mixture was stirred at room temperature for 2 h. After the reaction was completed, the mixture was washed with 2 mL of water and concentrated, and the residue was purified by prep-HPLC (ammonium bicarbonate method) to give (1r,2R,3S)-*N*-((6³S,4S,Z)-1¹-ethyl-1²-(5-(1-methylimino-1-oxido-1λ⁶-thiomorpholino)-2-((R)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z418, 15.5 mg, yield: 29%, purity: 95%). ES-API: [M+H]⁺ = 873.2.

### Example 57. Synthesis of Compounds Z485A, Z485-1, and Z485

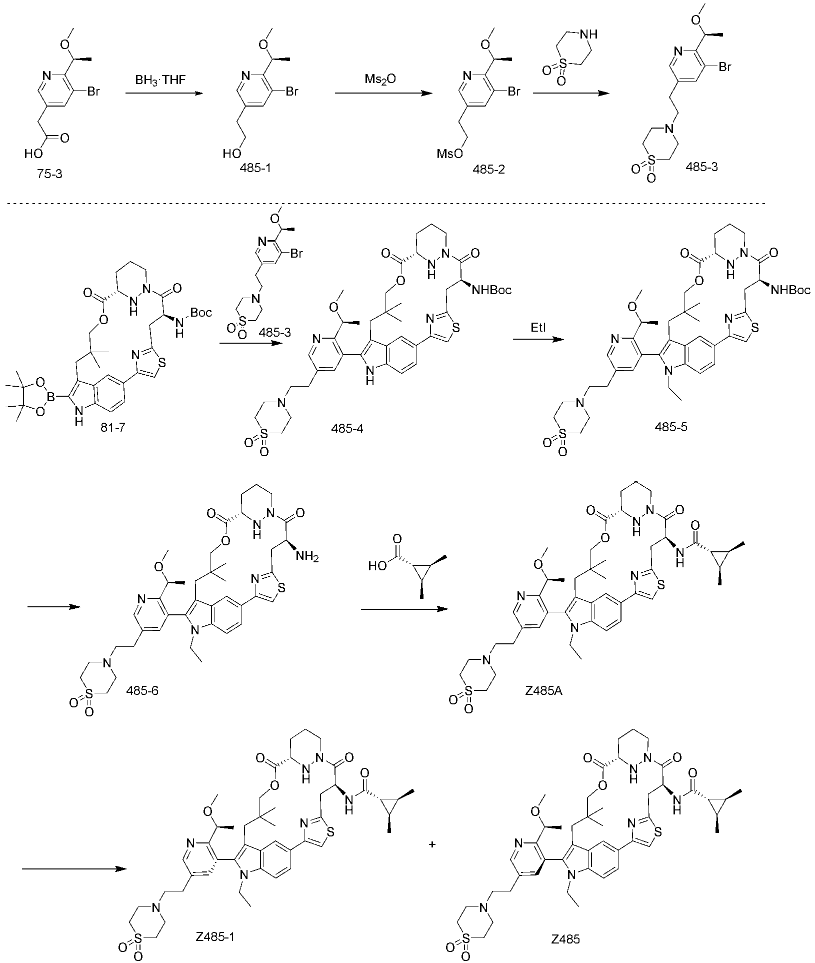

Step 1: Compound 75-3 (500 mg, 1.824 mmol) was dissolved in tetrahydrofuran (5 mL), and the mixture was cooled to 0 °C. Borane tetrahydrofuran complex (1 M, 5.5 mL, 5.5 mmol) was slowly added to the solution described above. After the addition was completed, the reaction system was warmed to room temperature and stirred for 17 h. After the reaction was completed, the reaction mixture was quenched with methanol (5 mL). After the reaction was quenched, the mixture was concentrated to give a crude product. The crude product was purified by a flash silica gel column (methanol/dichloromethane: 0%-4%) to give compound 485-1 (300 mg, 1.153 mmol, yield: 63.23%) as a colorless transparent liquid. ES-API: [M+H]⁺ = 260.0, 262.0. Step 2: Compound 485-1 (300 mg, 1.153 mmol) was dissolved in dichloromethane (5 mL). Triethylamine (0.481 mL, 3.460 mmol) and methanesulfonic anhydride (401.78 mg, 2.307 mmol) were added to the solution described above. After the addition was completed, the reaction system was stirred at room temperature for 2 h. After the reaction was completed, water (20 mL) was added to the solution described above, and the mixture was extracted with ethyl acetate (20 mL × 3). The organic phase was washed with saturated brine (30 mL × 2), dried over anhydrous sodium sulfate, filtered, and concentrated to give a crude product. The crude product was purified by a flash silica gel column (ethyl acetate/petroleum ether: 0%-40%) to give compound 485-2 (120 mg, 0.355 mmol, yield: 30.76%). ES-API: [M+H]⁺ = 338.1, 340.1.

Step 3: Compound 485-2 (120 mg, 0.355 mmol), thiomorpholine 1,1-dioxide (95.92 mg, 0.710 mmol), triethylamine (0.049 mL, 0.355 mmol), and N,N-dimethylformamide (3 mL) were added to a sealed tube reactor. After the addition was completed, the reaction system was stirred at 80 °C for 17 h. After the reaction was completed, water (20 mL) was added to the solution described above, and the mixture was extracted with ethyl acetate (20 mL × 3). The organic phase was washed with saturated brine (30 mL × 2), dried over anhydrous sodium sulfate, filtered, and concentrated to give a crude product. The crude product was purified by a flash silica gel column (methanol/dichloromethane: 0%-3%) to give compound 485-3 (120 mg, 0.355 mmol, yield: 30.76%). ES-API: [M+H]⁺ = 377.3, 379.3.

Step 4: Compound 485-3 (70.71 mg, 0.187 mmol) was dissolved in dioxane (1 mL), toluene (0.3 mL), and water (0.3 mL). Compound 81-7 (100 mg, 0.144 mmol), [1,1'-bis(di-tert-butylphosphino)ferrocene]dichloropalladium(II) (9.31 mg, 0.014 mmol), and potassium phosphate (91.80 mg, 0.432 mmol) were added to the solution described above at room temperature. After the addition was completed, the reaction system was stirred at 70 °C for 2 h under an argon atmosphere. After the reaction was completed, water (10 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 485-4 (90 mg, 0.104 mmol, yield: 72.25%) as a yellow solid. ES-API: [M+H]⁺ = 864.3.

Step 5: Compound 485-4 (90 mg, 0.104 mmol) was dissolved in N,N-dimethylformamide (3 mL). Iodoethane (48.74 mg, 0.312 mmol) and cesium carbonate (101.81 mg, 0.312 mmol) were added to the solution described above. The reaction system was stirred at room temperature for 8 h. After the reaction was completed, water (10 mL) was added to the solution described above, and the mixture was extracted with ethyl acetate (20 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-3%) to give compound 485-5 (90 mg, 0.101 mmol, yield: 96.86%) as a light yellow solid. ES-API: [M+H]⁺ = 892.3.

Step 6: Compound 485-5 (90 mg, 0.101 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give crude compound 485-6 (80 mg) as a white solid. ES-API: [M+H]⁺ = 792.2.

Step 7: Crude compound 485-6 (80 mg) was dissolved in dichloromethane (3 mL). (1*r*,2*R*,3*S*)-2,3-Dimethylcyclopropane-1-carboxylic acid (17.29 mg, 0.152 mmol), O-(7-azabenzotriazol-1-yl)-*N*,*N*,*N*',*N*'-tetramethyluronium hexafluorophosphate (57.61 mg, 0.152 mmol), and *N*,*N*'-diisopropylethylamine (0.050 mL, 0.303 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-6%) to give (1r,2R,3S)-N-((6³S,4S,Z)-1²-(5-(2-(1,1-dioxidothiomorpholino)ethyl)-2-((S)-1-methoxyethyl)pyridin-3-yl)-11-ethyl-10,10-dimethyl-5,7-dioxo-6',6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z485A, 50 mg, 0.056 mmol, two-step yield: 55.4%) as a white solid. ES-API: [M+H]⁺ = 888.3.

Step 8: Compound Z485A (50 mg, 0.056 mmol) was subjected to chiral resolution (column type: IB 250 mm, 10 mm, 5 µm; mobile phase system: (A: n-hexane; B: ethanol; C: diethylamine); flow rate: 1 mL/min; A/B/C = 30/70/2; column temperature: 30 °C) to give 2 isomeric compounds. The structure of one of the isomeric compounds was arbitrarily designated as (1r,2R,3S)-N-((6³S,4S,Z)-(Sₐ)-1²-(5-(2-(1,1-dioxidothiomorpholino)ethyl)-2-((S)-1-methoxyethyl)pyridin-3-yl)-11-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z485-1, 29.8 mg, 0.034 mmol, yield: 59.6%, retention time: 7.174) as a white solid. ES-API: [M+H]⁺ = 888.3. ¹H NMR (400 MHz, DMSO-d₆) δ 8.64 (d, *J* = 2.0 Hz, 1H), 8.53 (s, 1H), 8.40 (d, *J =* 9.2 Hz, 1H), 7.84 - 7.79 (m, 2H), 7.73 (dd, *J =* 8.4, 1.6 Hz, 1H), 7.53 (d, *J =* 8.4 Hz, 1H), 5.54 (t, *J* = 9.2 Hz, 1H), 5.03 (d, *J =* 12.0 Hz, 1H), 4.28 - 4.18 (m, 2H), 3.98 - 3.90 (m, 2H), 3.84 - 3.76 (m, 1H), 3.67 (d, *J* = 10.8 Hz, 1H), 3.55 (d, *J =* 10.8 Hz, 1H), 3.31 - 3.28 (m, 1H), 3.20 - 2.96 (m, 14H), 2.90 - 2.70 (m, 6H), 2.38 (d, *J =* 14.2 Hz, 1H), 2.16 - 2.08 (m, 1H), 1.85 - 1.75 (m, 2H), 1.59 - 1.45 (m, 1H), 1.24 - 1.18 (m, 5H), 1.11 - 1.06 (m, 8H), 0.92 (s, 3H), 0.48 (s, 3H). The structure of the other isomeric compound was arbitrarily designated as (1r,2R,3S)-N-((6³S,4S,Z)-(Rₐ)-1²-(5-(2-(1,1-dioxidothiomorpholino)ethyl)-2-((S)-1-methoxyethyl)pyridin-3-yl)-11-ethyl-10,10-dimethyl-5,7-dioxo-6',6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z485, 16.8 mg, 0.019 mmol, yield: 33.6%, retention time: 12.809) as a white solid. ES-API: [M+H]⁺ = 888.3.

### Example 58. Synthesis of Compounds Z449, Z614, and Z614-1

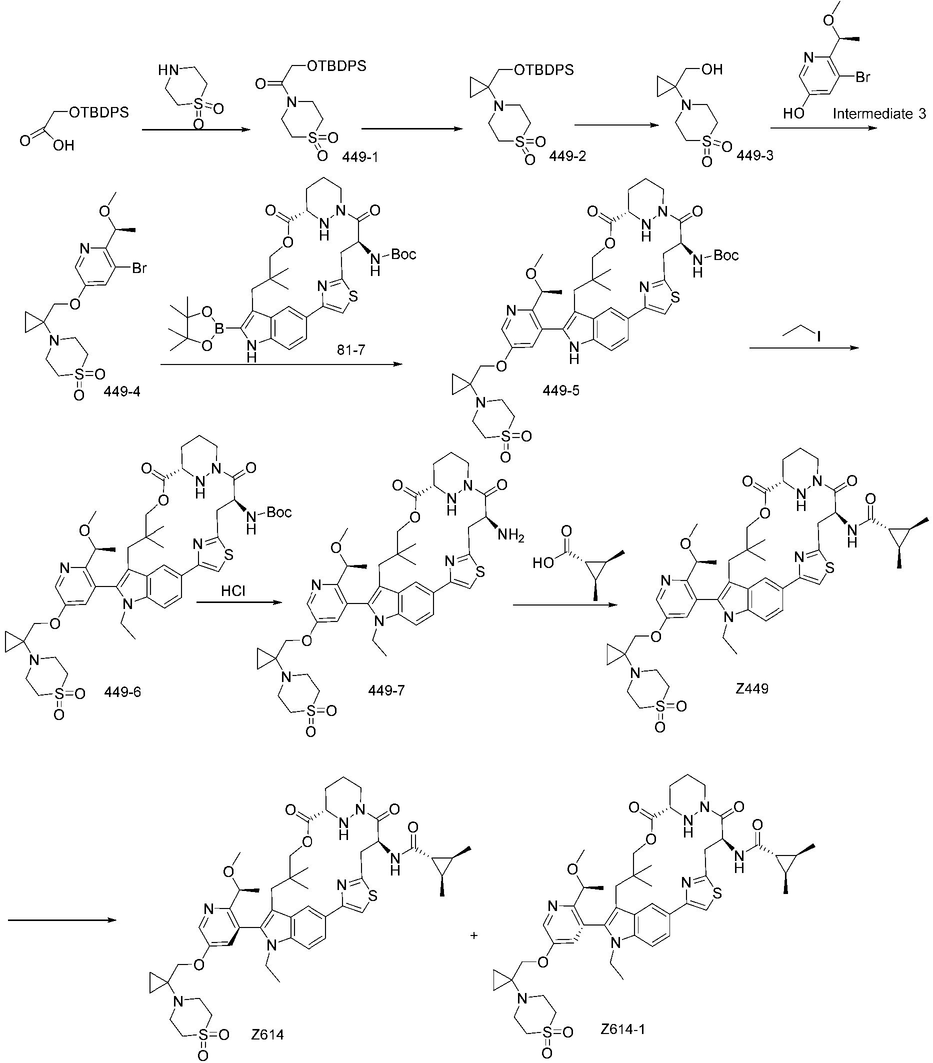

Step 1: Thiomorpholine 1,1-dioxide (1.50 g, 11.130 mmol), O-(7-azabenzotriazol-1-yl)-N,N,N',N'-tetramethyluronium hexafluorophosphate (6.34 g, 16.695 mmol), and N,N-diisopropylethylamine (7.19 g, 55.651 mmol) were added to a solution of 2-((tert-butyldiphenylsilyl)oxy)acetic acid (3.5 g, 11.130 mmol) in dichloromethane (50.0 mL) at room temperature, and the mixture was reacted at room temperature for 18 h. After the reaction was completed, the mixture was concentrated under reduced pressure, and the residue was purified by silica gel column chromatography (0-70% ethyl acetate/petroleum ether) to give compound 449-1 (3.50 g, 8.109 mmol, yield: 72.86%). ES-API: [M-Ph+H]⁺ = 354.1.

Step 2: Tetraisopropyl titanate (4.802 mL, 16.218 mmol) and ethylmagnesium bromide (16.218 mL, 2 M, 32.436 mmol) were added to a solution of compound 449-1 (3.50 g, 8.109 mmol) in tetrahydrofuran (30.0 mL) under an ice-water bath and a nitrogen atmosphere. The mixture was reacted at room temperature for 3 h. After the reaction was completed, a saturated ammonium chloride solution (30 mL) was added, and the mixture was extracted with ethyl acetate (80 mL), dried over anhydrous sodium sulfate, and filtered. The filtrate was concentrated to dryness by rotary evaporation under reduced pressure, and the residue was purified by silica gel column chromatography (0-50% ethyl acetate/petroleum ether) to give compound 449-2 (1.10 g, 2.479 mmol, yield: 30.57%). ES-API: [M+H]⁺ = 444.3.

Step 3: Tetrabutylammonium fluoride (1.80 mL, 2 M, 3.71 mmol) was added to a solution of compound 449-2 (1.10 g, 2.479 mmol) in tetrahydrofuran (5.0 mL) under an ice-water bath, and the mixture was reacted at room temperature for 2 h. After the reaction was completed, the mixture was directly purified by silica gel column chromatography [petroleum ether:tetrahydrofuran = 100:0 to 50:50 (v/v)] to give compound 449-3 (0.45 g, 2.192 mmol, yield: 88.42%). ES-API: [M+H]⁺ = 206.2.

Step 4: Compound 449-3 (450 mg, 2.192 mmol), triphenylphosphine (915.44 mg, 3.490 mmol), and diisopropyl azodicarboxylate (784.16 mg, 3.878 mmol) were added to a solution of intermediate 3 (450 mg, 1.939 mmol) in dichloromethane (5.0 mL) at room temperature. The system was reacted at room temperature for 18 h. After the reaction was completed, the mixture was directly purified by silica gel column chromatography [petroleum ether:ethyl acetate = 100:0 to 30:70 (v/v)] to give compound 449-4 (1.10 g, crude product). ES-API: [M+H]⁺ = 419.0.

Step 5: Compound 81-7 (500.0 mg, 0.721 mmol) and compound 449-4 (140.0 mg, 0.202 mmol) were dissolved in toluene (12.0 mL), 1,4-dioxane (4.0 mL), and water (4.0 mL), and potassium phosphate (128.0 mg, 0.605 mmol) and [1,1'-bis(di-tert-butylphosphino)ferrocene]dichloropalladium(II) (27.0 mg, 0.040 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 449-5 (205.0 mg, crude product). ES-API: [M+H]⁺ = 906.3.

Step 6: Compound 449-5 (205.0 mg, crude product) was dissolved in N,N-dimethylformamide (10.0 mL), and cesium carbonate (0.33 g, 1.010 mmol) and iodoethane (315.06 mg, 2.020 mmol) were added. The mixture was reacted at room temperature for 2 h. After the reaction was completed, water (20 mL) was added, and the mixture was extracted with ethyl acetate (50 mL × 3). The organic phase was washed with saturated brine (20 mL), dried over anhydrous sodium sulfate, and concentrated under reduced pressure to give compound 449-6 (140.0 mg, 74.35%). ES-API: [M+H]⁺ = 934.3.

Step 7: Compound 449-6 (140.0 mg, 0.150 mmol) was dissolved in methanol (5.0 mL), and a solution of hydrochloric acid in dioxane (10 mL, 10.0 mmol) was added. The mixture was reacted at room temperature for 2 h. The mixture was concentrated under reduced pressure to give compound 449-7 (180.0 mg, crude product). ES-API: [M+H]⁺ = 834.3.

Step 8: Compound 449-7 (180.0 mg, crude product) was dissolved in N,N-dimethylformamide (5.0 mL), and (1r,2R,3S)-2,3-dimethylcyclopropane-1-carboxylic acid (21.0 mg, 0.180 mmol), O-(7-azabenzotriazol-1-yl)-N,N,N',N'-tetramethyluronium hexafluorophosphate (171.0 mg, 0.450 mmol), and *N,N*'-diisopropylethylamine (0.16 mL, 0.900 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was purified by prep-HPLC (ammonia water method) to give the desired compound (1r,2R,3S)-N-((6³S,4S,Z)-1²-(5-((1-(1,1-dioxidothiomorpholino)cyclopropyl)methoxy)-2-((S)-1-methoxyethyl)pyridin-3-yl)-1¹-ethyl-10,10-dimethyl-5,7-dioxo-6¹,6²,6³,6⁴,6⁵ ,6⁶-hexahydro-1¹hydrogen-8-oxa-2(4,2)-thiazola-1(5,3)-indola-6(1,3)-pyridazinacycloundecaphane-4-yl)-2,3-dimethylcyclopropane-1-carboxamide (Z449, 38.0 mg, yield: 27.23%) as a white solid. ES-API: [M+H]⁺ = 930.2.

Step 9: Compound Z449 (38.0 mg, 0.041 mmol) was resolved by an SFC chiral column (chromatographic column: CHIRALPAK^{®} IB 250 × 30 mm, 10 µm; mobile phase A: HEX + 0.2% DEA, mobile phase B: ETOH + 0.2% DEA; detection wavelength: 214 nm/254 nm; flow rate: 25 mL/min; injection volume: 10 µL; column temperature: 30 °C; run time: 15 min; isocratic elution program: mobile phase A:mobile phase B = 30:70 (V/V)) to give 2 isomeric compounds. The structure of one of the isomeric compounds was arbitrarily designated as: (1r,2R,3S)-N-((6³S,4S,Z)-1²-(5-((1-(1,1-dioxidothiomorpholino)cyclopropyl)methoxy)-2-((S)-1-methoxyethyl)pyridin-3-yl)-1'-ethyl-(Rₐ)-10,10-dimethyl-5,7-dioxo-6¹,6²,6³,6⁴,6⁵,6⁶-hexahydro-1¹hydrogen-8-oxa-2(4,2)-thiazola-1(5,3)-indola-6(1,3)-pyridazinacycloundecaphane-4-yl)-2,3-dimethylcyclopropane-1-carboxamide (Z614, 7.67 mg, 0.008 mmol, yield: 20.18%, retention time: 11.149 min)as a white solid. ES-API: [M+H]⁺ = 930.2. ¹H NMR (400 MHz, CDCl₃) δ 8.51 (s, 1H), 8.36 (s, 1H), 7.54~7.52 (m, 1H), 7.28-7.26(m, 1H), 7.21 (s, 1H), 7.02~7.00 (m, 1H), 6.35~6.32 (m, 1H), 5.88~5.84 (m, 1H), 4.53~4.50 (m, 1H), 4.28 - 3.83 (m, 9H), 3.75 ~3.73(m, 1H), 3.66~3.63 (m, 1H), 3.38~3.30 (m, 90H), 2.92 (s, 5H), 2.63-2.60(m, 1H), 2.40 ~2.38(m, 1H), 2.15-2.12(m, 1H), 1.38~1.36 (m, 4H), 1.09-1.07(m, 4H), 1.02 ~1.00(m, 4H), 0.93~0.90 (m, 3H), 0.82~0.75 (m, 8H), 0.39 (s, 3H).

The structure of the other isomeric compound was arbitrarily designated as: (1r,2R,3S)-N-((6³S,4S,Z)-1²-(5-((1-(1,1-dioxidothiomorpholino)cyclopropyl)methoxy)-2-((S)-1-methoxyethyl)pyridin-3-yl)-1¹-ethyl-(Sₐ)-10,10-dimethyl-5,7-dioxo-6¹,6^{2,}6³,6⁴,6⁵,6⁶-hexahydro-1¹hydrogen-8-oxa-2(4,2)-thiazola-1(5,3)-indola-6(1,3)-pyridazinacycloundecaphane-4-yl)-2,3-dimethylcyclopropane-1-carboxamide (Z614-1, 16.51 mg, 0.018 mmol, yield: 43.45%, retention time: 6.503 min) as a white solid. ES-API: [M+H]⁺ = 930.2. ¹H NMR (400 MHz, CDCl₃) δ 8.54 (s, 1H), 8.43 (s, 1H), 7.55~7.53 (m, 1H), 7.28~7.26 (m, 1H), 7.22 (s, 1H), 7.09 (s, 1H), 6.36~6.34 (m, 1H), 5.82~5.77 (m, 1H), 4.53~4.50 (m, 1H), 4.29~4.26 (m, 1H), 4.08 - 3.58 (m, 9H), 3.42~3.38 (m, 1H), 3.31 (s, 4H), 3.21 - 3.12 (m, 4H), 3.06~3.02 (m, 1H), 2.90-2.95(m, 5H), 2.63~2.57 (m, 1H), 2.30~2.26 (m, 1H), 2.20~2.17 (m, 1H), 1.96-1.88(m, 3H), 1.76-1.73(m, 2H), 1.09-1.07(m, 4H), 1.04~1.02 (m, 4H), 0.90 (s, 3H), 0.85 - 0.70 (m, 8H), 0.49 (s, 3H).

### Example 59. Synthesis of Compounds Z383-1 and Z383-2

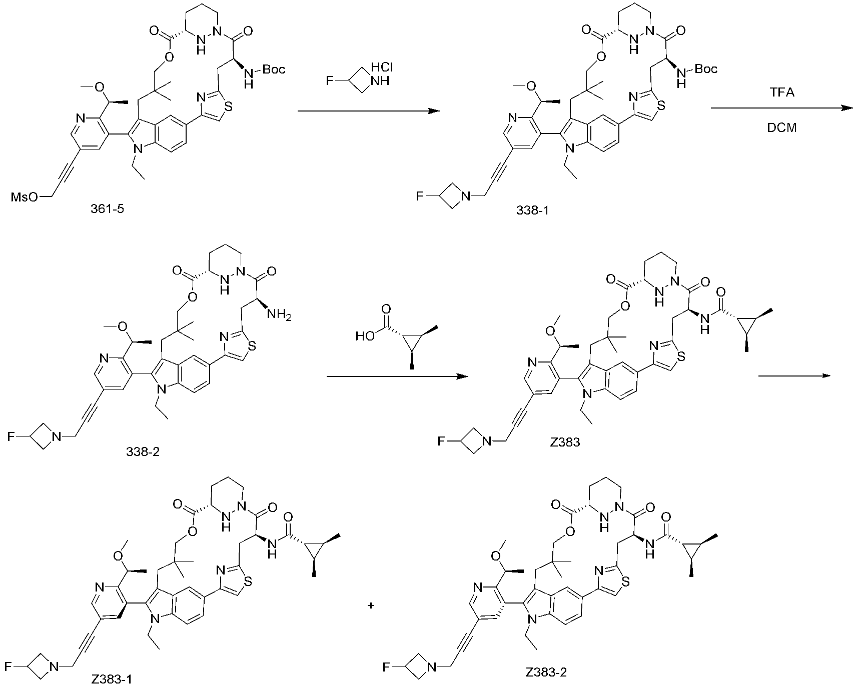

Step 1: Compound 361-5 (20 mg, 0.023 mmol) was dissolved in dichloromethane (5 mL), and then N,N-diisopropylethylamine (9 mg, 0.07 mmol) and 3-fluoroazetidine hydrochloride (5.1 mg, 0.046 mmol) were added. The mixture was stirred at room temperature for 3 h. The reaction mixture was concentrated, and the residue was purified by flash column chromatography (0-8% methanol/dichloromethane) to give compound 383-1 (16 mg, 0.019 mmol, yield: 82.0%) as a yellow oil. ES-API [M+H]⁺ = 842.3.

Step 2: Compound 383-1 (16 mg, 0.019 mmol) was dissolved in anhydrous dichloromethane (3 mL), and trifluoroacetic acid (1 mL) was added at 0 °C. The reaction system was stirred at room temperature for 2 h. The reaction mixture was concentrated, and the pH of the residue was adjusted to about 8-9. The residue was extracted 3 times with dichloromethane/isopropanol (9 mL/3 mL) and washed sequentially with saturated sodium bicarbonate (10 mL) and saturated brine (10 mL). The organic phase was dried over anhydrous sodium sulfate and concentrated to give compound 383-2 (12 mg, crude product) as a yellow liquid. ES-API: [M+H]⁺ = 742.3.

Step 3: Compound 383-2 (12 mg, crude product) and (1r,2R,3S)-2,3-dimethylcyclopropane-1-carboxylic acid (2.4 mg, 0.021 mmol) were dissolved in dichloromethane (5 mL), and ethyldiisopropylamine (0.008 mL, 0.049 mmol) and HATU (8 mg, 0.021 mmol) were sequentially added. The reaction system was stirred at room temperature for 3 h. The reaction system was diluted with water (5 mL), and the mixture was extracted with dichloromethane (5 mL × 3). The organic layer was separated, washed with a saturated NaCl solution, and concentrated in vacuum to give crude compound Z383. The crude product was subjected to prep-HPLC (formic acid method 1) to give 2 isomeric compounds. The structure of one of the isomeric compounds was arbitrarily designated as (1*r,2R,3S*)*-*N-((6*³S*,4*S*,*Z*)-(*Rₐ*)-1²-(5-(3-(3-fluoroazetidin-1-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z383-1, 0.52 mg, retention time: 8.227 min, yield: 3.84%, formate) as a white solid. ES-API: [M+H]⁺ = 838.3. The structure of the other isomeric compound was arbitrarily designated as (1*r*,2*R*,3*S*)-N-((6³*S*,4*S*,*Z*)-(*Sₐ*)-1²-(5-(3-(3-fluoroazetidin-1-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (0.33 mg, Z383-2, retention time: 8.122 min, yield: 2.43%, formate) as a white solid. ES-API: [M+H]⁺ = 838.3.

### Example 60. Synthesis of Compound Z659

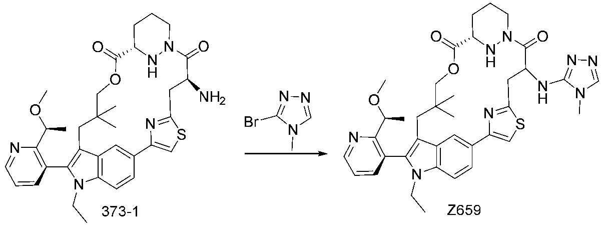

Step 1: Compound 373-1 (50 mg, 0.079 mmol), 3-bromo-4-methyl-1,2,4-thiazole (52 mg, 0.32 mmol), tetrahydrofuran (2 mL), cesium carbonate (78 mg, 0.24 mmol), and (SP-4-2)-[[3,6-dimethoxy-2',4',6'-tris(1-methylethyl)[1,1'-biphenyl]-2-yl-κC1']bis(1,1-dimethylethyl)phosphine-κP][4-[[2-(trimethylsilyl)ethoxy]carbonyl]phenyl]palladium(III) bromide (CAS: 2097600-19-4, 14 mg, 0.016 mmol) were added to a 5-mL microwave tube. The reaction mixture was purged with nitrogen for 30 s, capped, and stirred at 120 °C for 1 h in a microwave reactor. The reaction mixture was filtered and washed with ethyl acetate (30 mL), and the filtrate was concentrated under reduced pressure. The crude product was purified by a flash silica gel column (7 M ammonia-methanol solution/dichloromethane: 0-10%) and prep-HPLC (ammonium bicarbonate method) to give the desired product (6³S,Z)-1¹-ethyl-(Rₐ)-1²-(2-((S)-1-methoxyethyl)-10-10-dimethyl-4-((4-methyl-4H-1,2,4-triazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z659, 2 mg, yield: 3.6%) as a white solid. ES-API: [M+H]⁺ = 712.3.

### Example 61. Synthesis of Compounds Z600 and Z600-1

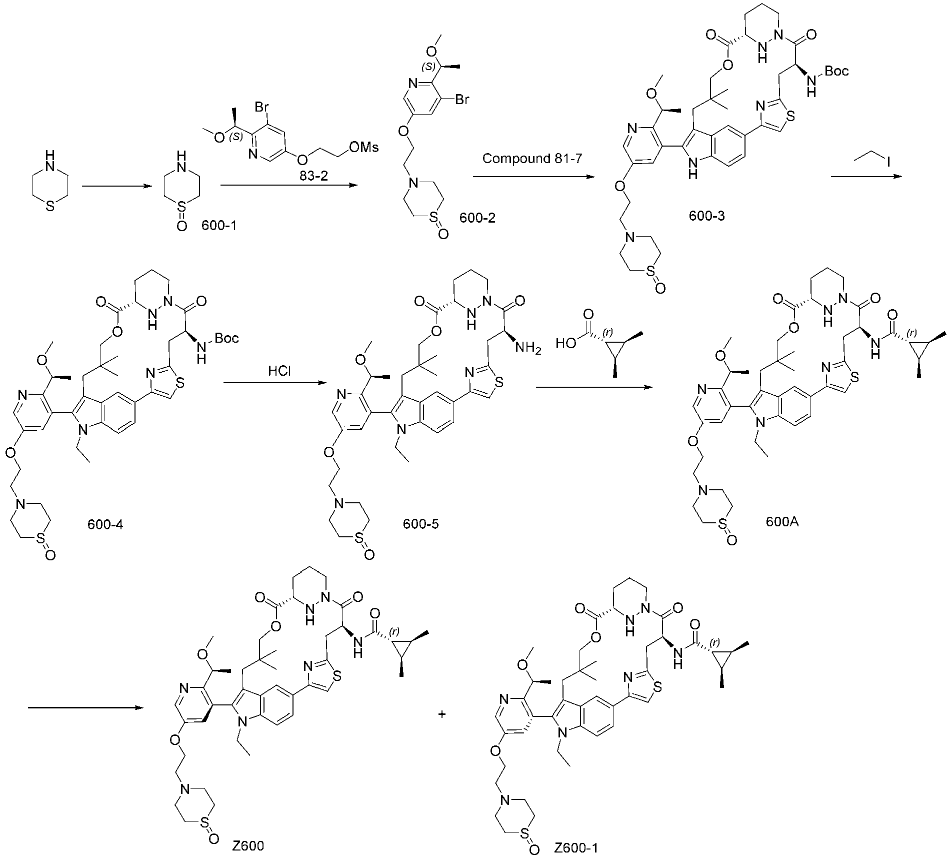

Step 1: Sodium periodate (3.45 g, 15.91 mmol) was added in portions to a solution of thiomorpholine (1.5 g, 14.538 mmol) in a mixed solvent of methanol/water (15.0 mL/6.0 mL) under an ice-water bath, and the system was reacted at room temperature for 24 h. After the reaction was completed, the mixture was filtered, and the filter cake was washed 3 times with ethyl acetate (3 × 50 mL). The filtrates were combined, dried over anhydrous sodium sulfate, and filtered, and the filtrate was concentrated by rotary evaporation under reduced pressure to remove the solvent to give compound 600-1 (1.47 g, yield: 85.0%). ES-API: [M+H]⁺ = 120.1.

Step 2: N,N-Diisopropylethylamine (4.0 g, 31.055 mmol) and compound 600-1 (1.11 g, 9.317 mmol) were added to a solution of compound 83-2 (1.10 g, 3.106 mmol) in N,N-dimethylformamide (10.0 mL) at room temperature, and the mixture was reacted at 70 °C overnight under a nitrogen atmosphere. After the reaction was completed, the reaction mixture was cooled to room temperature, and ethyl acetate (50 mL) was added to the reaction mixture. The mixture was washed sequentially with diluted brine (20 mL × 3) and saturated brine (50 mL). The organic phase was dried over anhydrous sodium sulfate and filtered, and the filtrate was concentrated under reduced pressure. The crude product was purified by a flash silica gel column (methanol/dichloromethane: 0-10%) to give compound 600-2 (900.0 mg, yield: 76.81%), ES-API: [M+H]⁺ = 377.0.

Step 3: Compound 81-7 (500.0 mg, 0.721 mmol) and compound 600-2 (325.0 mg, 0.861 mmol) were dissolved in toluene (12.0 mL), 1,4-dioxane (4.0 mL), and water (4.0 mL), and potassium phosphate (460.0 mg, 2.162 mmol) and [1,1'-bis(di-tert-butylphosphino)ferrocene]dichloropalladium(II) (94.0 mg, 0.144 mmol) were added. The mixture was reacted at 60 °C for 5 h under a nitrogen atmosphere. After the reaction was completed, the mixture was concentrated under reduced pressure, and the residue was purified by silica gel column chromatography (dichloromethane:methanol = 10:1) to give compound 600-3 (540.0 mg, 0.626 mmol, yield: 86.90%). ES-API: [M+H]⁺ = 864.3.

Step 4: Compound 600-3 (540.0 mg, 0.626 mmol) was dissolved in N,N-dimethylformamide (10.0 mL), and cesium carbonate (1.02 g, 3.125 mmol) and iodoethane (974.71 mg, 6.249 mmol) were added. The mixture was reacted at room temperature for 2 h. After the reaction was completed, water (20 mL) was added, and the mixture was extracted with ethyl acetate (50 mL × 3). The organic phase was washed with saturated brine (20 mL), dried over anhydrous sodium sulfate, and concentrated under reduced pressure to give compound 600-4 (350.0 mg, yield: 62.78%). ES-API: [M+H]⁺ = 892.3.

Step 5: Compound 600-4 (350.0 mg, 0.392 mmol) was dissolved in methanol (5.0 mL), and a solution of hydrochloric acid in dioxane (10 mL, 10.0 mmol) was added. The mixture was reacted at room temperature for 2 h. The mixture was concentrated under reduced pressure to give compound 600-5 (360.0 mg, crude product). ES-API: [M+H]⁺ = 792.3.

Step 6: Compound 600-5 (360.0 mg, crude product) was dissolved in N,N-dimethylformamide (5.0 mL), and (1r,2R,3S)-2,3-dimethylcyclopropane-1-carboxylic acid (54.0 mg, 0.470 mmol), O-(7-azabenzotriazol-1-yl)-N,N,N',N'-tetramethyluronium hexafluorophosphate (450.0 mg, 1.176 mmol), and N,N'-diisopropylethylamine (0.41 mL, 2.352 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was purified by prep-HPLC (ammonium bicarbonate method) to give compound 600A (210 mg, yield: 60.4%) as a white solid. ES-API: [M+H]⁺ = 888.2.

Step 7: Compound 600A (210 mg, 0.2367 mmol) was resolved by an SFC chiral column (chromatographic column: CHIRALPAK^{®} IB 250 × 30 mm, 10 µm; mobile phase A: HEX + 0.2% DEA, mobile phase B: ETOH + 0.2% DEA; detection wavelength: 214 nm/254 nm; flow rate: 25 mL/min; injection volume: 10 µL; column temperature: 30 °C; run time: 15 min; isocratic elution program: mobile phase A:mobile phase B = 30:70 (V/V)) to give 2 isomeric compounds. One of the isomeric compounds was arbitrarily designated as: (1r,2R,3S)-N-((6³S,4S,Z)-1¹-ethyl-(Rₐ)-1²-(2-((S)-1-methoxyethyl)-5-(2-(1-oxidothiomorpholino)ethoxy)pyridin-3-yl-10,10-dimethyl-5,7-dioxo-6¹,6²,6³,6⁴,6⁵,6⁶-hexahydro-1¹thydrogen-8-oxa-2(4,2)-thiazola-1(5,3)-indola-6(1,3)-pyridazinacycloundecaphane-4-yl)-2,3-dimethylcyclopropane-1-carboxamide (Z600, 94.0 mg, 0.106 mmol, yield: 27.0%, retention time: 6.039 min) as a white solid. ES-API: [M+H]⁺ = 888.2. The structure of the other isomeric compound was arbitrarily designated as: (1r,2R,3S)-N-((6³S,4S,Z)-1¹-ethyl-(Sₐ)-1²-(2-((S)-1-methoxyethyl)-5-(2-(1-oxidothiomorpholino)ethoxy)pyridin-3-yl-10,10-dimethyl-5,7-dioxo-6¹,6²,6³,6⁴,6⁵,6⁶-hexahydro-1¹hydrogen-8-oxa-2(4,2)-thiazola-1(5,3)-indola-6(1,3)-pyridazinacycloundecaphane-4-yl)-2,3-dimethylcyclopropane-1-carboxamide (Z600-1, 48.0 mg, 0.054 mmol, yield: 13.79%, retention time: 12.369 min) as a white solid. ES-API: [M+H]⁺ = 888.2.

### Example 62. Synthesis of Compounds Z661-1 and Z661-2

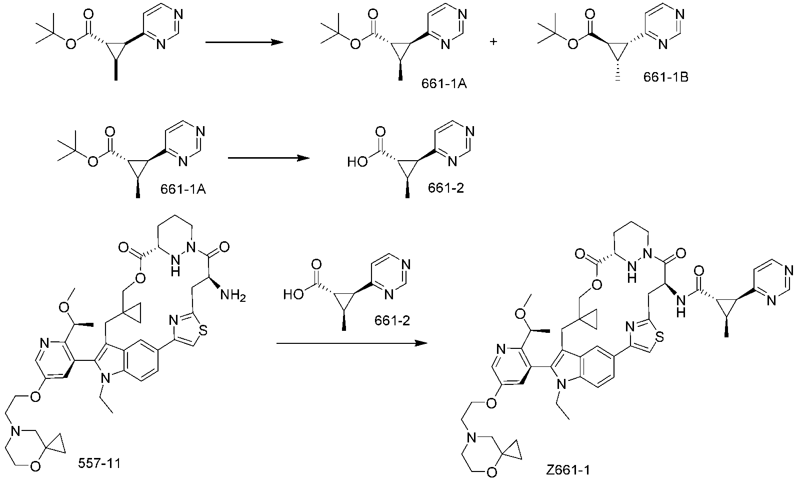

Step 1: tert-Butyl 2-methyl-3-(pyrimidin-4-yl)cyclopropane-1-carboxylate (100 mg, 0.427 mmol, see A504 in WO2022060836A1 for the preparation method) was subjected to chiral resolution (column type: IC 250 mm, 10 mm, 5 µm; mobile phase system: (A: n-hexane; B: isopropanol); flow rate: 1 mL/min; A/B = 95/5; column temperature: 30 °C) to give 2 isomeric compounds. The structure of one of the isomeric compounds was arbitrarily designated as tert-butyl (1S,2R,3S)-2-methyl-3-(pyrimidin-4-yl)cyclopropane-1-carboxylate (compound 661-1A, 40 mg, 0.171 mmol, yield: 40.00%, peak 1, retention time: 8.655 min). The structure of the other isomeric compound was arbitrarily designated as tert-butyl (1R,2S,3R)-2-methyl-3-(pyrimidin-4-yl)cyclopropane-1-carboxylate (compound 661-1B, 40 mg, 0.171 mmol, yield: 40.00%, peak 2, retention time: 9.767 min). Both were white solids. ES-API: [M+H]⁺ = 235.1.

Step 2: Compound 661-1A (10 mg, 0.043 mmol, peak 1) was dissolved in dichloromethane (1 mL), and trifluoroacetic acid (0.5 mL) was added to the solution described above. After the addition was completed, the reaction system was stirred at room temperature for 5 h. The reaction mixture was concentrated to give compound 661-2 (7.61 mg, 0.043 mmol, yield: 100.00%) as a colorless oily liquid. ES-API: [M+H]⁺ = 179.1.

Step 3: Compound 557-11 (13 mg, 0.017 mmol) was dissolved in dichloromethane (2 mL). Compound 661-2 (9.19 mg, 0.067 mmol), O-(7-azabenzotriazol-1-yl)-*N,N,N',N'*-tetramethyluronium hexafluorophosphate (9.5 mg, 0.025 mmol), and *N,N'-*diisopropylethylamine (0.008 mL, 0.050 mmol) were added to the solution described above. After the addition was completed, the reaction system was stirred at room temperature for 2 h. After the reaction was completed, the reaction mixture was concentrated to remove dichloromethane. Water (5 mL) was added to the residue, and the mixture was extracted with ethyl acetate (5 mL × 2). The organic phase was washed with saturated brine (10 mL), dried over anhydrous sodium sulfate, filtered, and concentrated to give a crude product. The crude product was purified by prep-HPLC (ammonium bicarbonate method) to give (1S,2R,3S)-N-((6³S,4S,Z)-(Rₐ)-1²-(5-(2-(4-oxa-7-azaspiro[2.5]octan-7-yl)ethoxy)-2-((S)-1-methoxyethyl)pyridin-3-yl)-11-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methyl-3-(pyrimidin-4-yl)cyclopropane-1-carboxamide (Z661-1, 2.8 mg, 0.003 mmol, yield: 17.95%) as a white solid. ES-API: [M+H]⁺ = 944.3.

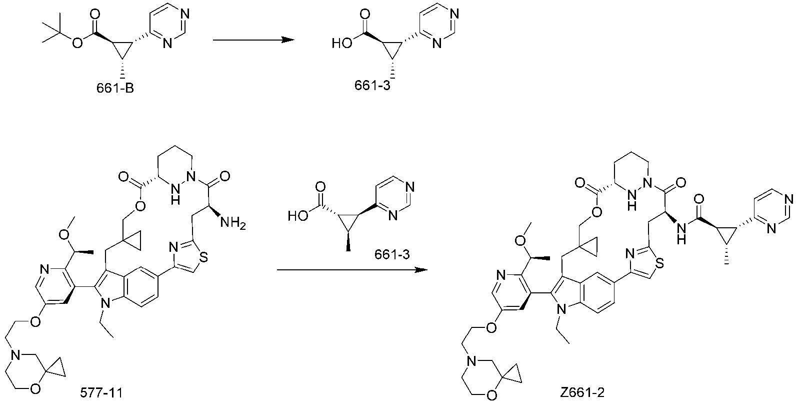

Step 1: Compound 661-1B (10 mg, 0.043 mmol, peak 2) was dissolved in dichloromethane (1 mL), and trifluoroacetic acid (0.5 mL) was added to the solution described above. After the addition was completed, the reaction system was stirred at room temperature for 5 h. The reaction mixture was concentrated to give compound 661-3 (7.61 mg, 0.043 mmol, yield: 100.00%) as a colorless oily liquid. ES-API: [M+H]⁺ = 179.1.

Step 2: Compound 577-11 (13 mg, 0.017 mmol) was dissolved in dichloromethane (2 mL). Compound 661-3 (9.19 mg, 0.067 mmol), O-(7-azabenzotriazol-1-yl)-*N,N,N',N'*-tetramethyluronium hexafluorophosphate (9.5 mg, 0.025 mmol), and *N,N'-*diisopropylethylamine (0.008 mL, 0.050 mmol) were added to the solution described above. After the addition was completed, the reaction system was stirred at room temperature for 2 h. After the reaction was completed, the reaction mixture was concentrated to remove dichloromethane. Water (5 mL) was added to the residue, and the mixture was extracted with ethyl acetate (5 mL × 2). The organic phase was washed with saturated brine (10 mL), dried over anhydrous sodium sulfate, filtered, and concentrated to give a crude product. The crude product was purified by prep-HPLC (ammonium bicarbonate method) to give (1R,2S,3R)-N-((6³S,4S,Z)-(Rₐ)-1²-(5-(2-(4-oxa-7-azaspiro[2.5]octan-7-yl)ethoxy)-2-((S)-1-methoxyethyl)pyridin-3-yl)-11-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methyl-3-(pyrimidin-4-yl)cyclopropane-1-carboxamide (Z661-2, 3.8 mg, 0.004 mmol, yield: 24.27%) as a white solid. ES-API: [M+H]⁺ = 944.3.

### Example 63. Synthesis of Compounds Z598A, Z598, and Z598-1

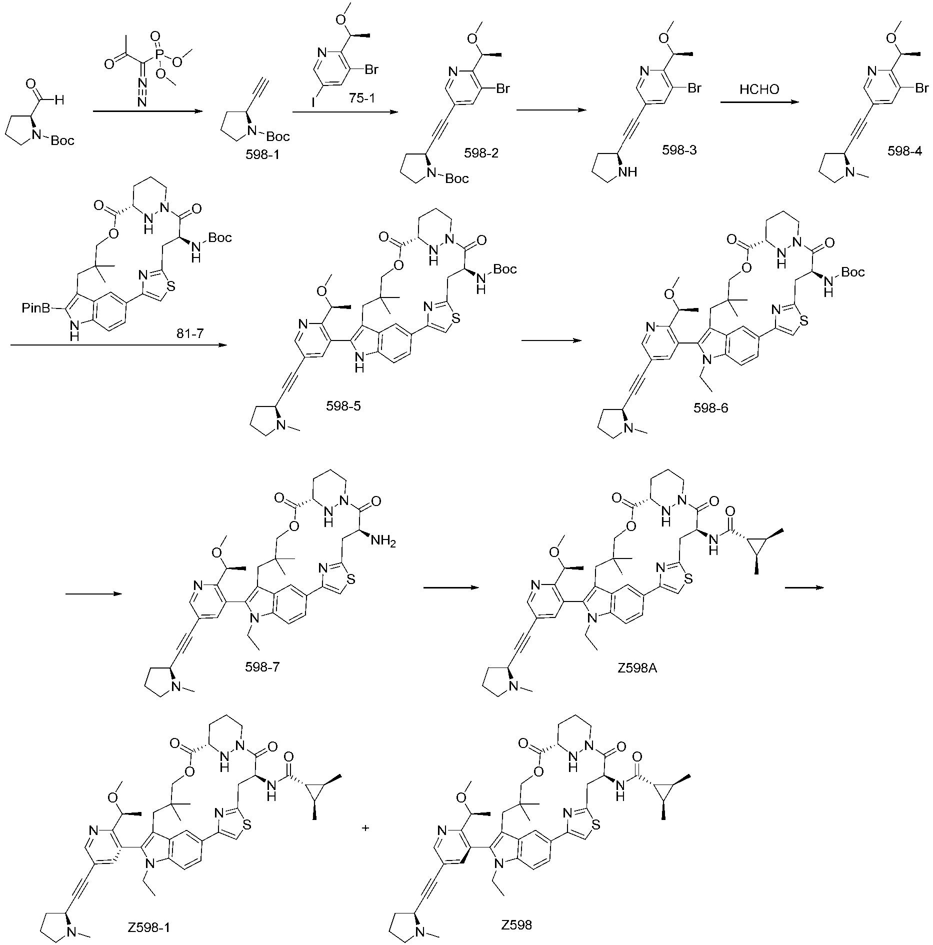

Step 1: tert-Butyl (S)-2-formylpyrrolidine-1-carboxylate (1 g, 5.02 mmol) was added dropwise to a mixed solution of dimethyl (1-diazo-2-oxopropyl)phosphonate (1.16 g, 6.02 mmol) and potassium carbonate (1.39 g, 10.04 mmol) in methanol (20 mL). The mixture was stirred at room temperature for 3 h. 50 mL of ethyl acetate was added to the reaction mixture, and the mixture was washed with 20 mL of water. The organic phase was dried over anhydrous sodium sulfate, filtered, and concentrated, and the residue was purified by a flash silica gel column (0-30% ethyl acetate/petroleum ether) to give compound 598-1 (750 mg) as a transparent oil. ES-API: [M+H-56]⁺ = 140.2.

Step 2: A solution of compound 75-1 (500 mg, 1.46 mmol), compound 598-2 (300 mg, 1.54 mmol), bis(triphenylphosphine)palladium(II) dichloride (51 mg, 0.07 mmol), copper(I) iodide (28 mg, 0.15 mmol), and N,N-diisopropylethylamine (378 mg, 2.92 mmol) in acetonitrile (10 mL) was stirred at room temperature for 3 h under a nitrogen atmosphere. 50 mL of ethyl acetate was added to the reaction mixture, and the mixture was washed with 20 mL of water. The organic phase was dried over anhydrous sodium sulfate, filtered, and concentrated, and the residue was purified by a flash silica gel column (0-20% ethyl acetate/petroleum ether) to give compound 598-2 (560 mg) as a yellow oil. ES-API: [M+H]⁺ = 409.1, 411.1.

Step 3: Trifluoroacetic acid (1 mL, 13.06 mmol) was added dropwise to a solution of compound 598-2 (250 mg, 0.61 mmol) in dichloromethane (2 mL). The reaction mixture was stirred at room temperature for 2 h. After the reaction was completed, the mixture was concentrated under reduced pressure to give compound 598-3 (260 mg, trifluoroacetate) as a yellow liquid. ES-API: [M+H]⁺ = 309.1, 311.1.

Step 4: A 37% aqueous formaldehyde solution (0.14 mL, 1.84 mmol) was added dropwise to a solution of compound 598-3 (260 mg, 0.61 mmol, trifluoroacetate) in dichloromethane (5 mL). The mixture was stirred at room temperature for 2 h. Sodium triacetoxyborohydride (260 mg, 1.23 mmol) was then added to the reaction mixture, and the mixture was stirred for another 1 h. After the reaction was completed, the reaction was quenched with 20 mL of saturated sodium bicarbonate, and the mixture was extracted three times with 20 mL of dichloromethane. The organic phases were combined, dried over anhydrous sodium sulfate, filtered, and concentrated, and the residue was purified by a flash silica gel column (0-90% tetrahydrofuran (1% solution of ammonia in methanol)/petroleum ether) to give compound 598-4 (180 mg) as a yellow liquid. ES-API: [M+H]⁺ = 323.1, 325.1.

Step 5: A mixture of compound 81-7 (120 mg, 0.17 mmol), compound 598-4 (62 mg, 0.19 mmol), [1,1'-bis(di-tert-butylphosphino)ferrocene]dichloropalladium(II) (11 mg, 0.02 mmol), and potassium phosphate (110 mg, 0.52 mmol) was stirred at 70 °C for 2 h in dioxane (2 mL), toluene (0.5 mL), and water (0.5 mL) under a nitrogen atmosphere. After the reaction was completed, the reaction mixture was poured into 10 mL of ethyl acetate and washed sequentially with 5 mL of saturated brine and 5 mL of water. The organic phase was dried over anhydrous sodium sulfate, filtered, and concentrated, and the residue was purified by a flash silica gel column (0-90% tetrahydrofuran (1% solution of ammonia in methanol)/petroleum ether) to give compound 598-5 (120 mg) as a yellow solid. ES-API: [M+H]⁺ = 810.3.

Step 6: Compound 598-5 (120 mg, 0.15 mmol) was dissolved in N,N-dimethylformamide (2 mL), and cesium carbonate (241 mg, 0.74 mmol) and iodoethane (86 mg, 0.74 mmol) were added. The mixture was stirred at room temperature for 2 h. The reaction mixture was quenched with 2 mL of a solution of ammonia in methanol, and the mixture was dissolved in 20 mL of ethyl acetate, washed sequentially with 10 mL of saturated brine and 10 mL of water, dried over anhydrous sodium sulfate, filtered, and concentrated. The residue was purified by a flash silica gel column (0-90% tetrahydrofuran (1% solution of ammonia in methanol)/petroleum ether) to give compound 598-6 (50 mg) as a yellow oil. ES-API: [M+H]⁺ = 838.3.

Step 7: Compound 598-6 (50 mg, 0.06 mmol) was dissolved in methanol (0.2 mL), and a 4 M solution of hydrogen chloride in dioxane (1 mL, 4 mmol) was added. The mixture was stirred at room temperature for 2 h. After the reaction was completed, the mixture was concentrated to give crude compound 598-7 (44 mg). ES-API: [M+H]⁺ = 738.3.

Step 8: N,N-Diisopropylethylamine (77 mg, 0.60 mmol) and O-(7-azabenzotriazol-1-yl)-N,N,N',N'-tetramethyluronium hexafluorophosphate (34 mg, 0.09 mmol) were sequentially added to a solution of (1r,2R,3S)-2,3-dimethylcyclopropane-1-carboxylic acid (8 mg, 0.07 mmol) and compound 598-7 (44 mg, 0.06 mmol) in dichloromethane (2 mL). The mixture was stirred at room temperature for 2 h. After the reaction was completed, the mixture was washed with 2 mL of water and concentrated, and the residue was purified by a flash silica gel column (0-90% tetrahydrofuran (1% solution of ammonia in methanol)/petroleum ether) to give (1*r*,2*R*,3*S*)-N-((6³*S*,4*S*,*Z*)-1¹-ethyl-1²-(2-((*S*)-1-methoxyethyl)-5-(((*S*)-1-methylpyrrolidin-2-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z598A, 34 mg, yield: 68%, purity: 90%). ES-API: [M+H]⁺ = 834.3.

Step 9: Compound Z598A (30 mg, 0.04 mmol) was purified by prep-HPLC (ammonium bicarbonate method) to give 2 isomeric compounds. The structure of one of the isomeric compounds was arbitrarily designated as: (1*r*,2*R*,3*S*)-N-((6³*S*,4*S*,*Z*)-(*Sₐ*)-1'-ethyl-1²-(2-((*S*)-1-methoxyethyl)-5-(((*S*)-1-methylpyrrolidin-2-yl)ethynyl)pyridin-3-y1)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z598-1, 1.4 mg, yield: 5%, purity: 95%, retention time: 2.41 min). ES-API: [M+H]⁺ = 834.3. The structure of the other isomeric compound was arbitrarily designated as: (1*r*,2*R*,3*S*)-N-((6³*S*,4*S*,Z)-(*Rₐ*)-1¹-ethyl-1²-(2-((*S*)-1-methoxyethyl)-5-(((*S*)-1-methylpyrrolidin-2-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z598, 0.6 mg, yield: 2%, purity: 95%, retention time: 2.55 min). ES-API: [M+H]⁺ = 834.3.

### Example 64. Synthesis of Compound Z662

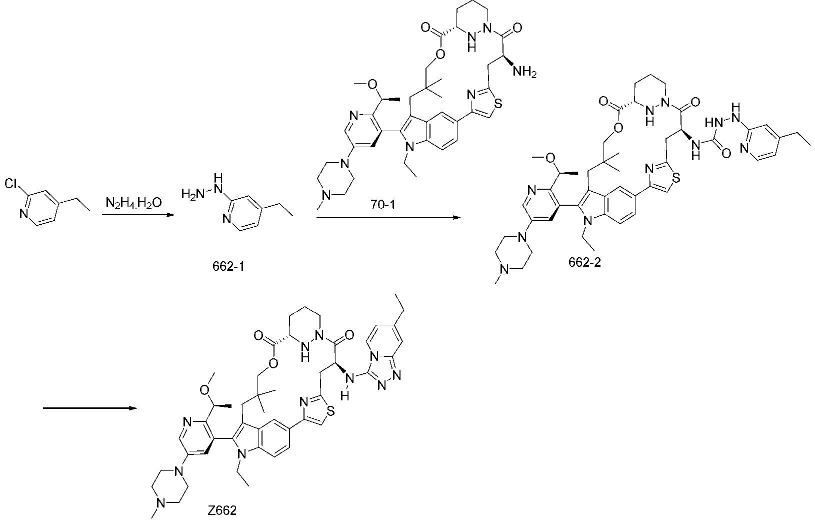

Step 1: 2-Chloro-4-ethylpyridine (900 mg, 6.36 mmol) was dissolved in absolute ethanol (3 mL), and 80% hydrazine hydrate (7.7 mL, 127.12 mmol) was added. The reaction system was stirred at 150 °C for 1.5 h in a microwave reactor. Dichloromethane (50 mL) was added to the reaction mixture, and the mixture was washed with saturated brine (20 mL). The organic phase was dried over anhydrous sodium sulfate and filtered, and the filtrate was concentrated under reduced pressure. The crude product was purified by a flash silica gel column (methanol/dichloromethane: 0-5%) to give compound 662-1 (250 mg, yield: 28.7%) as a pale yellow solid. ES-API: [M+H]⁺ = 138.3.

Step 2: Compound 70-1 (120 mg, 0.17 mmol) and N,N-diisopropylethylamine (213 mg, 1.65 mmol) were dissolved in dichloromethane (8 mL), and nitrophenyl chloroformate (100 mg, 0.49 mmol) was added at 0 °C. The reaction system was stirred for 1 h under an ice bath, and then compound 662-1 (90 mg, 0.66 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (7 M ammonia-methanol solution/dichloromethane: 0-5%) to give compound 662-2 (80 mg, yield: 54.6%) as a pale yellow solid. ES-API: [M+H]⁺ = 892.3.

Step 3: Compound 662-2 (65 mg, 0.073 mmol) and pyridine (86 mg, 1.09 mmol) were dissolved in toluene (5 mL) and phosphorus oxychloride (56 mg, 0.36 mmol). The reaction system was stirred at 75 °C for 3 h. The reaction mixture was cooled to room temperature, and the organic layer was discarded. Dichloromethane/7 M ammonia-methanol solution (5:1, 15 mL) was added to the remaining solid, and the mixture was stirred at room temperature for 30 min. The solution was concentrated. The residue was purified by a flash silica gel column (7 M ammonia-methanol solution/dichloromethane: 0-5%) to give a crude product (25 mg). The crude product was then purified by prep-HPLC (ammonium bicarbonate method) to give the desired product (6³S,4S,Z)-1¹-ethyl-4-((7-ethyl-[1,2,4]triazolo[4,3-a]pyridin-3-yl)amino)-(Rₐ)-1²-(2-((S)-1-methoxyethyl)-5-(4-methylpiperazin-1-yl)pyridin-3-yl)-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z662, 16 mg, yield: 25.1%) as a white solid. ¹H NMR (400 MHz, DMSO-d₆) δ 8.51 (s, 1H), 8.45 (d, *J =* 2.8 Hz, 1H), 8.28 (d, *J =* 7.2 Hz, 1H), 7.77 (s, 1H), 7.72 (d, *J =* 8.8 Hz, 1H), 7.54 (d, *J =* 8.8 Hz, 1H), 7.27 - 4.19 (m, 2H), 7.16 (d, *J =* 10.8 Hz, 1H), 6.76 (d, *J =* 7.2 Hz, 1H), 5.54 (t, *J =* 10.0 Hz, 1H), 5.19 (d, *J =* 12.0 Hz, 1H), 4.35 - 4.21 (m, 3H), 4.20 - 4.07 (m, 2H), 3.67 - 3.44 (m, 3H), 3.30 - 3.15 (m, 8H), 2.96 (d, *J =* 14.2 Hz, 1H), 2.88 - 2.76 (m, 1H), 2.67 - 2.51 (m, 6H), 2.45 (d, *J =* 14.2 Hz, 1H), 2.30 (s, 3H), 2.12 (d, *J =* 10.0 Hz, 1H), 1.89 - 1.74 (m, 2H), 1.66 - 1.50 (m, 1H), 1.33 (d, *J =* 6.0 Hz, 3H), 1.23 - 1.19 (m, 4H), 0.97 - 0.86 (m, 6H), 0.36 (s, 3H). ES-API: [M+H]⁺ = 874.3

### Example 65. Synthesis of Compound Z136-1

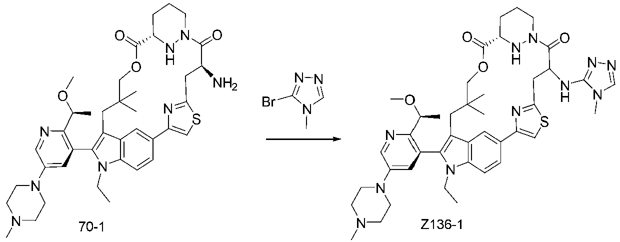

Step 1: Compound 70-1 (150 mg, 0.21 mmol), 3-bromo-4-methyl-1,2,4-thiazole (100 mg, 0.62 mmol), tetrahydrofuran (10 mL), cesium carbonate (201 mg, 0.62 mmol), and (SP-4-2)-[[3,6-dimethoxy-2',4',6'-tris(1-methylethyl)[1,1'-biphenyl]-2-yl-κC1']bis(1,1-dimethylethyl)phosphine-κP][4-[[2-(trimethylsilyl)ethoxy]carbonyl]phenyl]palladium(III) bromide (CAS: 2097600-19-4, 37 mg, 0.041 mmol) were added to a 5-mL microwave tube. The reaction mixture was purged with nitrogen for 30 s, capped, and stirred at 120 °C for 1 h in a microwave reactor. The reaction mixture was filtered and washed with ethyl acetate (30 mL), and the filtrate was concentrated under reduced pressure. The crude product was first purified by a flash silica gel column (7 M ammonia-methanol solution/dichloromethane: 0-8%) and then subjected to prep-HPLC (ammonium bicarbonate method) to give the desired product (6³S,Z)-1¹-ethyl-(Rₐ)-1²-(2-((S)-1-methoxyethyl)-5-(4-methylpiperazin-1-yl)pyridin-3-yl)-10-10-dimethyl-4-((4-methyl-4H-1,2,4-triazol-3-yl)amino)-6¹,6²,6³,6⁴,6⁵ 6⁶-hexahydro-1¹H-8-oxa-2(4,2)-thiazola-1(5,3)-indola-6(1,3)-pyridazinacycloundecaphane-5,7-dione (Z136-1, 2.2 mg, yield: 1.3%) as a white solid. ES-API: [M+H]⁺ = 810.3.

### Example 66. Synthesis of Compounds Z601 and Z601-1

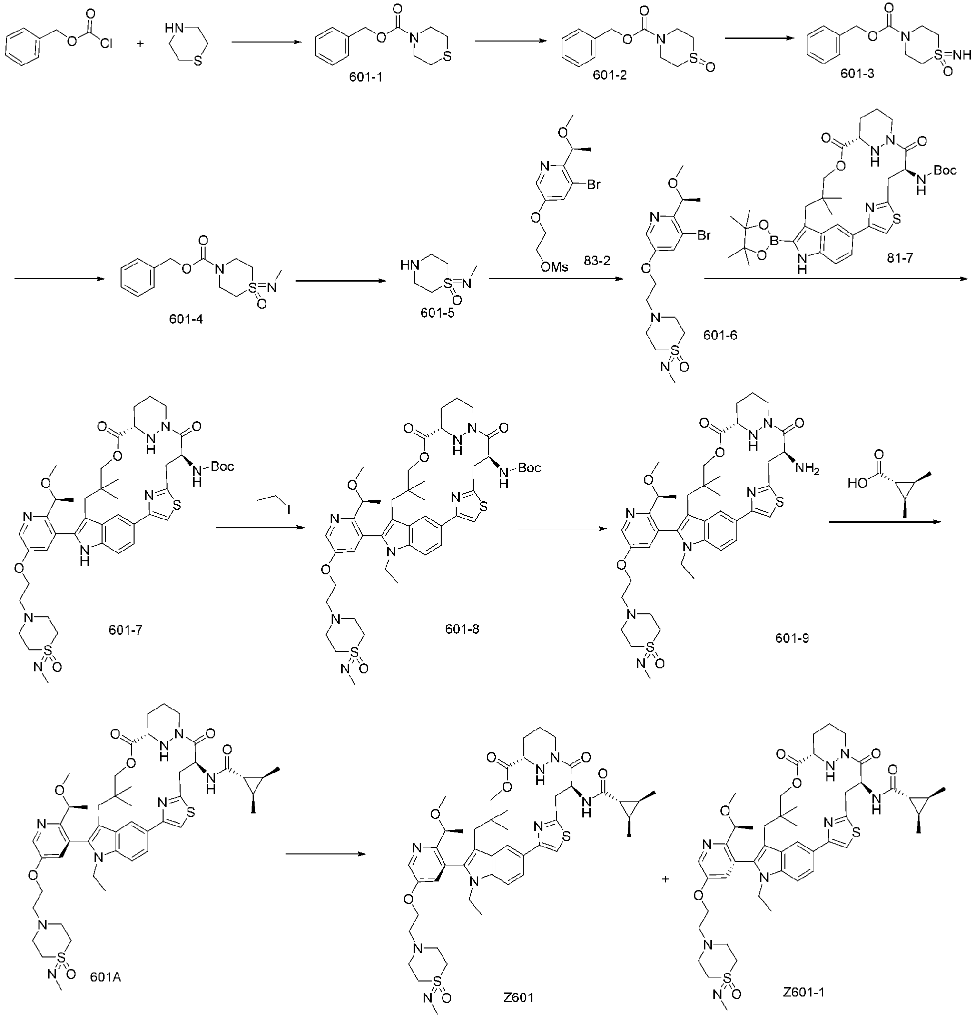

Step 1: Benzyl chloroformate (5.0 mL, 35.382 mmol) was added dropwise to a solution of thiomorpholine (6 g, 58.151 mmol) in sodium hydroxide (35 mL, 1 mol/L) under an ice-water bath. The reaction mixture was stirred at room temperature for 4 h. The reaction was completed as detected by LCMS. The reaction mixture was cooled and adjusted to weak acidity with 1 mol/L diluted hydrochloric acid, and then the mixture was extracted with ethyl acetate (15 mL × 3). The organic phases were combined, washed with saturated brine (15 mL × 3), dried over anhydrous sodium sulfate, filtered, and concentrated to give crude compound 601-1 (8.5 g). ES-API: [M+H]⁺ = 238.1.

Step 2: Sodium periodate (7.59 g, 35.472 mmol) was added in portions to a solution of compound 601-1 (7.653 g, 32.248 mmol) in methanol (100 mL) and water (40 mL) under an ice-water bath. The reaction mixture was stirred at room temperature overnight. The reaction was completed as detected by LCMS. The mixture was filtered to remove insoluble substances, the filter cake was washed with ethyl acetate, and the filtrate was dried, filtered, and concentrated to dryness. The residue was purified by automated flash chromatography on silica gel (ethyl acetate/petroleum ether = 0-60%) to give compound 601-2 (4.6 g, yield: 56%). ES-API: [M+H]⁺ = 254.2.

Step 3: Iodobenzene diacetate (10.172 g, 31.580 mmol) and ammonium carbamate (3.698 g, 47.370 mmol) were added to a solution of compound 601-2 (2.0 g, 7.895 mmol) in methanol (40 mL). The reaction mixture was stirred at room temperature for 2 h. The reaction was completed as detected by LCMS. The mixture was concentrated under reduced pressure to remove the solvent. The residue was purified by automated flash chromatography on silica gel (ethyl acetate/petroleum ether = 0-65%) to give compound 601-3 (2.116 g, yield: 99%). ES-API: [M+H]⁺ = 269.2.

Step 4: Methylboronic acid (446 mg, 7.454 mmol), copper(II) acetate (1015 mg, 5.590 mmol), and pyridine (0.72 mL, 8.944 mmol) were added to a solution of compound 601-3 (1.0 g, 3.727 mmol) in dioxane (20 mL). The reaction mixture was heated to 100 °C and stirred overnight. The reaction was completed as detected by LCMS. The mixture was concentrated under reduced pressure to remove the solvent. The residue was purified by automated flash chromatography on silica gel (ethyl acetate/petroleum ether = 0-50%) to give compound 601-4 (1.085 g). ES-API: [M+H]⁺ = 283.1.

Step 5: Compound 601-4 (1.085 g, 3.843 mmol) was dissolved in a solution of hydrobromic acid in acetic acid (8 mL), and the reaction mixture was stirred at room temperature overnight. The reaction was completed as detected by LCMS. The reaction mixture was adjusted to weak basicity with a 2 M sodium hydroxide solution and diluted with 10 mL of water. The mixture was extracted with a mixed solution (15 mL × 4, V/V = 10:1) of dichloromethane and methanol. The organic phases were combined, washed with saturated brine (10 mL × 2), dried over anhydrous sodium sulfate, filtered, and concentrated to give crude compound 601-5 (750 mg). ES-API: [M+H]⁺ = 149.2.

Step 6: Compound 601-5 (750 mg, 5.082 mmol) and N,N-diisopropylethylamine (3.0 mL, 16.939 mmol) were added to a solution of compound 83-2 (1.2 g, 3.388 mmol) in N,N-dimethylformamide (30 mL). The reaction mixture was heated to 70 °C and stirred for 6 h. The reaction was completed as detected by LCMS. The reaction mixture was diluted with ethyl acetate (30 mL), and the mixture was washed with saturated brine (15 mL × 3). The organic phase was dried over anhydrous sodium sulfate, filtered, and concentrated. The residue was purified by automated flash chromatography on silica gel (methanol/dichloromethane = 0-10%) to give compound 601-6 (378 mg, yield: 27%). ES-API: [M+H]⁺ = 406.1.

Step 7: Compound 601-6 (58.5 mg, 0.144 mmol), compound 81-7 (100 mg, 0.144 mmol), potassium phosphate (92 mg, 0.432 mmol), and [1,1'-bis(di-tert-butylphosphino)ferrocene]dichloropalladium(II) (19 mg, 0.029 mmol) were dissolved in dioxane (6 mL), toluene (2 mL), and water (2 mL) under a nitrogen atmosphere. The reaction mixture was heated to 70 °C under an oil bath and stirred for 5 h. When the reaction was completed as detected by LCMS, the mixture was cooled to room temperature. 20 mL of ethyl acetate was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-90%) to give compound 601-7 (92 mg, yield: 72%). ES-API: [M+H]⁺ = 893.2.

Step 8: Cesium carbonate (336 mg, 1.030 mmol) and iodoethane (161 mg, 1.030 mmol) were sequentially added to a solution of compound 601-7 (92 mg, 0.103 mmol) in N,N-dimethylformamide (2 mL). The reaction mixture was stirred at room temperature for 1 h. When the reaction was completed as detected by LCMS, the reaction mixture was diluted with 20 mL of ethyl acetate, and then the mixture was washed with water (10 mL) and saturated brine (15 mL × 3). The organic phase was dried over anhydrous sodium sulfate, filtered, and concentrated to dryness to give crude compound 601-8 (98 mg). ES-API: [M+H]⁺ = 921.3.

Step 9: Compound 601-8 (98 mg, 0.106 mmol) was dissolved in a solution of hydrochloric acid in dioxane (3 mL) and a solution of hydrochloric acid in methanol (1 mL). The reaction mixture was stirred at room temperature for 1 h. When the reaction was completed as detected by LCMS, the mixture was concentrated under reduced pressure at a low temperature (30 °C) to remove the solvent to give crude compound 601-9 (72 mg, yield: 82%). ES-API: [M+H]⁺ = 821.3.

Step 10: (1r,2R,3S)-2,3-Dimethylcyclopropane-1-carboxylic acid (15 mg, 0.132 mmol), N,N-diisopropylethylamine (57 mg, 0.438 mmol), and O-(7-azabenzotriazol-1-yl)-N,N,N',N'-tetramethyluronium hexafluorophosphate (50 mg, 0.132 mmol) were sequentially added to a solution of compound 601-9 (72 mg, 0.088 mmol) in N,N-dimethylformamide (2 mL) under an ice-water bath. The reaction mixture was warmed to room temperature and stirred for 10 min. When the reaction was completed as detected by LCMS, the reaction mixture was diluted with 20 mL of ethyl acetate, and then the mixture was washed with water (10 mL) and saturated brine (10 mL × 3). The organic phase was dried over anhydrous sodium sulfate, filtered, and concentrated to dryness to give crude product 601A. The crude product was purified by prep-HPLC (ammonium bicarbonate method) to give 2 isomeric compounds. The structure of one of the isomeric compounds was arbitrarily designated as: (1r,2R,3S)-N-((6³S,4S,Z)-1¹-ethyl-1²-(Rₐ)-(2-((S)-1-methoxyethyl)-5-(2-(1-(methylimino)-1-oxido-1λ⁶-thiomorpholino)ethoxy)pyridin-3-yl)-10-10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z601, 15.56 mg, yield: 19%, retention time = 1.73 min), ES-API [M+H]⁺ = 917.3. ¹H NMR (400 MHz, DMSO-d₆) δ 8.50- 8.49 (m, 2 H), 8.40 (d, J= 9.2 Hz, 1 H), 7.82 (s, 1 H), 7.75 (dd, J1= 1.6 Hz, J2= 8.8 Hz, 1 H), 7.58 (d, J= 8.4 Hz, 1 H), 7.40 (d, J= 2.8 Hz, 1 H), 5.55 (t, J= 9.2 Hz, 1 H), 5.07 (d, J= 12.4 Hz, 1 H), 4.34- 4.11 (m, 7 H), 3.64- 3.29 (m, 9 H), 3.23 (s, 3 H), 3.18- 3.02 (m, 4 H), 2.97-2.94 (m, 1 H), 2.77- 2.72 (m, 4 H), 2.47- 2.43 (m, 1 H), 2.09- 2.06 (m, 1 H), 1.79- 1.74 (m, 2 H), 1.53- 1.49 (m, 1 H), 1.34 (d, J= 6.0 Hz, 3 H), 1.23- 1.05 (m, 10 H), 0.92- 0.88 (m, 6 H), 0.34 (s, 3 H).

The structure of the other isomeric compound was arbitrarily designated as: (1r,2R,3S)-N-((6³S,4S,Z)-1¹-ethyl-1²-(Sₐ)-(2-((S)-1-methoxyethyl)-5-(2-(1-(methylimino)-1-oxido-1λ⁶-thiomorpholino)ethoxy)pyridin-3-yl)-10-10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z601-1, 34.09 mg, yield: 42%, retention time = 1.76 min), ES-API [M+H]⁺ = 917.3. ¹H NMR (400 MHz, DMSO-d₆) δ 8.53 (s,1 H), 8.48 (d, J= 2.8 Hz, 1 H), 8.40 (d, J= 8.8 Hz, 1 H), 7.81 (s, 1 H), 7.73 (dd, J1= 1.2 Hz, J2= 8.8 Hz, 1 H), 7.55- 7.52 (m, 2 H), 5.54 (t, J= 9.2 Hz, 1 H), 5.04 (d, J= 12.4 Hz, 1 H), 4.26- 4.15 (m, 4 H), 3.98-3.83 (m, 3 H), 3.68 (d, J= 10.8 Hz, 1 H), 3.55 (d, J= 11.2 Hz, 1 H), 3.16- 3.08 (m, 6 H), 3.04- 3.00 (m, 5 H), 2.92- 2.85 (m, 4 H), 2.80- 2.73 (m, 1 H), 2.61 (s, 3 H), 2.44- 2.40 (m, 1 H), 2.14- 2.10 (m, 1 H), 1.82- 1.76 (m, 2 H), 1.54- 1.50 (m, 1 H), 1.23-1.06 (m, 16 H), 0.93 (s, 3 H), 0.52 (s, 3 H).

### Example 67. Synthesis of Compound Z605

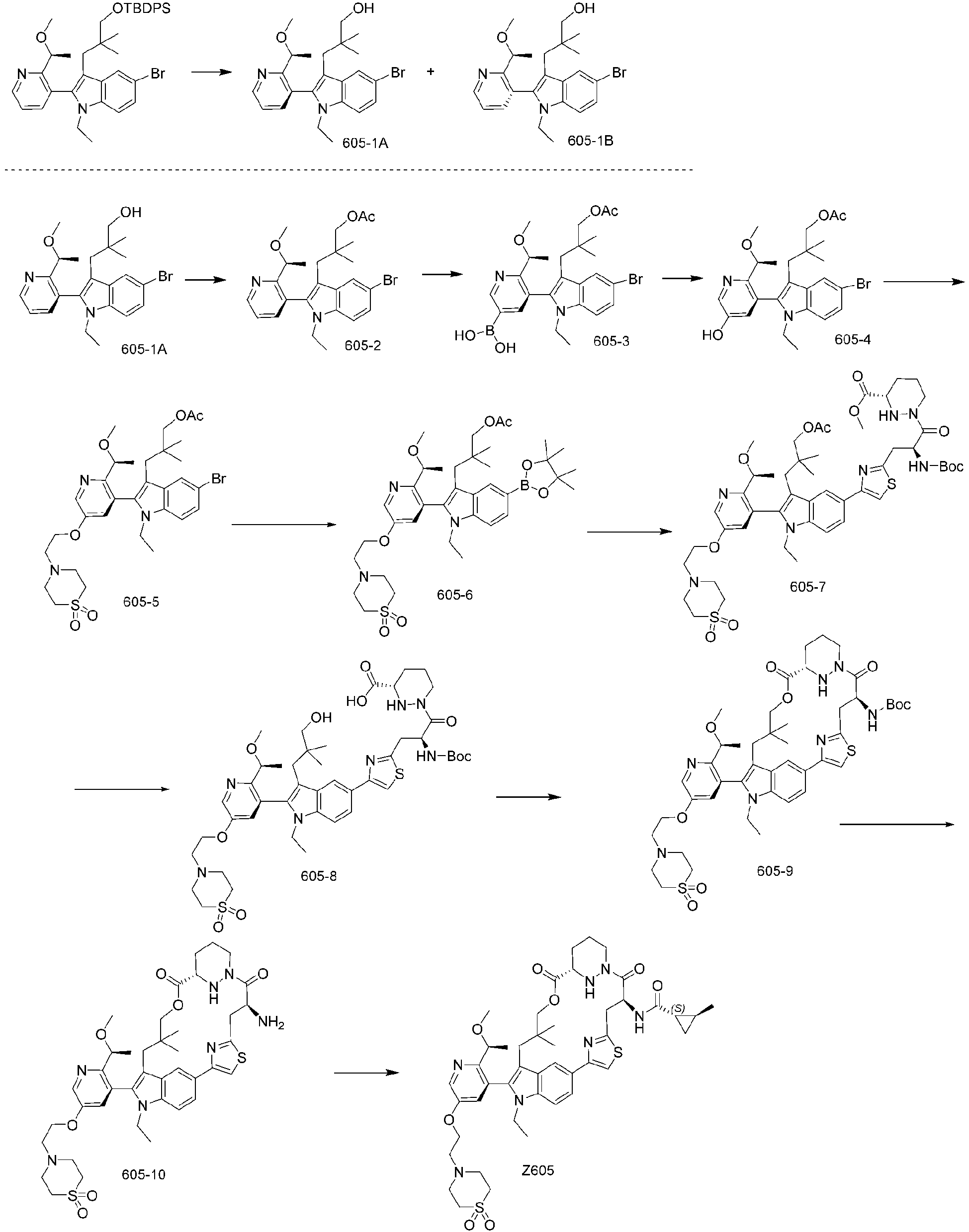

Step 1: (S)-5-Bromo-3-(3-((tert-butyldiphenylsilyl)oxy)-2,2-dimethylpropyl)-1-ethyl-2-(2-(1-methoxyethyl)pyridin-3-yl)-1H-indole (8800 mg, 12.907 mmol, see intermediate 1 in WO2022060836A1 for the preparation method) was dissolved in a solution of tetrabutylammonium fluoride in tetrahydrofuran (1 M, 60 mL). The reaction system was stirred at 50 °C for 17 h under a nitrogen atmosphere. After the reaction was completed, water (200 mL) was added to the solution described above, and the mixture was extracted with ethyl acetate (100 mL × 2). The organic phase was washed with saturated brine (200 mL × 5), dried over anhydrous sodium sulfate, filtered, and concentrated to give a crude product. The crude product was purified by a flash silica gel column (ethyl acetate/petroleum ether: 0%-70%) to give (S)-3-(5-bromo-1-ethyl-(Rₐ)-2-(2-(1-methoxyethyl)pyridin-3-yl)-1H-indol-3-yl)-2,2-dimethylpropan-1-ol (compound 605-1A, 2600 mg, 5.864 mmol, yield: 45.43%, configuration arbitrarily designated, Rf = 0.43) as a yellow oily liquid. ES-API: [M+H]⁺ = 445.2. (S)-3-(5-Bromo-1-ethyl-(Sₐ)-2-(2-(1-methoxyethyl)pyridin-3-yl)-1H-indol-3-yl)-2,2-dimethylpropan-1-ol (compound 605-1B, 2600 mg, 5.864 mmol, yield: 45.43%, configuration arbitrarily designated, Rf = 0.40) as a yellow oily liquid. ES-API: [M+H]⁺ = 445.2. Step 2: Compound 605-1A (2500 mg, 5.639 mmol), triethylamine (1.567 mL, 11.277 mmol), and 4-dimethylaminopyridine (68.89 mg, 0.564 mmol) were dissolved in dichloromethane (40 mL). Acetic anhydride (6.355 mL, 67.662 mmol) was slowly added to the solution described above under an ice bath. After the addition was completed, the reaction system was warmed to room temperature and stirred for another 2 h. After the reaction was completed, the reaction mixture was concentrated. Then water (100 mL) was added to the residue, and the mixture was extracted with dichloromethane (100 mL × 2). The organic phase was washed sequentially with a saturated aqueous sodium bicarbonate solution (100 mL × 2) and a saturated sodium chloride solution (200 mL), dried over anhydrous sodium sulfate, filtered, and concentrated to give a crude product. The crude product was purified by a flash silica gel column (ethyl acetate/petroleum ether: 0%-40%) to give compound 605-2 (2600 mg, 5.356 mmol, yield: 94.99%) as a yellow solid. ES-API: [M+H]⁺ = 487.1.

Step 3: Compound 605-2 (2500 mg, 5.150 mmol), bis(pinacolato)diboron (2223.32 mg, 8.755 mmol), chloro(1,5-cyclooctadiene)iridium(I) dimer (221.17 mg, 0.824 mmol), and 4,4'-di-tert-butyl-2,2'-bipyridine (50.32 mg, 0.187 mmol) were dissolved in n-hexane (45 mL). The reaction system was stirred at 80 °C for 17 h under a nitrogen atmosphere. After the reaction was completed, the reaction system was concentrated to remove n-hexane to give crude compound 605-3 (5000 mg) as a black oily liquid. ES-API: [M+H]⁺ = 531.5.

Step 4: Compound 605-3 (5000 mg) was dissolved in acetonitrile (30 mL), and hydrogen peroxide (1750.62 mg, 30 wt%, 15.447 mmol) was added to the solution described above under an ice bath. After the addition was completed, the reaction system was warmed to room temperature and stirred for 3 h. After the reaction was completed, the reaction was quenched with saturated sodium sulfite (30 mL). The mixture was extracted with ethyl acetate (50 mL × 2). The organic phase was washed with saturated brine (50 mL × 2), dried over anhydrous sodium sulfate, filtered, and concentrated to give a crude product. The crude product was purified by a flash silica gel column (methanol/dichloromethane: 0%-4%) to give compound 605-4 (1700 mg, 3.390 mmol, two-step yield: 65.8%) as a yellow solid. ES-API: [M+H]⁺ = 503.2.

Step 5: Compound 605-4 (1800 mg, 3.575 mmol), triphenylphosphine (1686.16 mg, 6.436 mmol), and 4-(2-hydroxyethyl)-1λ⁶-1,4-thiazine-1,1-dione (1153.47 mg, 6.436 mmol) were dissolved in tetrahydrofuran. Diisopropyl azodicarboxylate (1445.96 mg, 7.151 mmol) was added to the solution described above. After the addition was completed, the reaction system was stirred at 25 °C for 6 h. After the reaction was completed, water (50 mL) was added to the reaction mixture, and the mixture was extracted with ethyl acetate (30 mL × 2). The organic phase was washed with saturated brine (50 mL × 2), dried over anhydrous sodium sulfate, filtered, and concentrated to give a crude product. The crude product was purified by a flash silica gel column (ethyl acetate/petroleum ether: 0%-60%) to give compound 605-5 (2000 mg, 3.009 mmol, yield: 84.16%) as a light yellow solid. ES-API: [M+H]⁺ = 664.1.

Step 6: Compound 605-5 (1000 mg, 1.505 mmol) was dissolved in anhydrous toluene (15 mL), and bis(pinacolato)diboron (573.09 mg, 2.257 mmol), [1,1'-bis(diphenylphosphino)ferrocene]palladium(II) dichloride (110.09 mg, 0.150 mmol), and potassium acetate (369.14 mg, 3.761 mmol) were added to the solution described above at room temperature. After the addition was completed, the reaction system was stirred at 90 °C for 3 h under a nitrogen atmosphere. After the reaction was completed, the reaction mixture was filtered using a sand core funnel and concentrated. The residue was purified by a silica gel column (ethyl acetate/petroleum ether: 0-100%) to give compound 605-6 (900 mg, 1.265 mmol, yield: 84.05%) as a yellow oily liquid. ES-API: [M+H]⁺ = 712.3.

Step 7: Compound 605-6 (1100 mg, 1.546 mmol) was dissolved in dioxane (2 mL), toluene (6 mL), and water (2 mL). Methyl (S)-1-((S)-3-(4-bromothiazol-2-yl)-2-((tert-butoxycarbonyl)amino)propanoyl)hexahydropyridazine-3-carboxylate (737.80 mg, 1.546 mmol), [1,1'-bis(di-tert-butylphosphino)ferrocene]dichloropalladium(II) (150.92 mg, 0.232 mmol), and potassium phosphate (984.18 mg, 4.637 mmol) were added to the solution described above at room temperature. After the addition was completed, the reaction system was stirred at 70 °C for 16 h under an argon atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 605-7 (1100 mg, 1.120 mmol, yield: 72.46%) as a yellow solid. ES-API: [M+H]⁺ = 982.3.

Step 8: Compound 605-7 (1100 mg, 1.120 mmol) was dissolved in tetrahydrofuran (2 mL) and water (2 mL). Lithium hydroxide (140.97 mg, 3.360 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (20 mL × 2). The organic phase was washed with saturated brine (20 mL), dried over anhydrous sodium sulfate, filtered, and concentrated to give compound 605-8 (760 mg, 0.821 mmol, yield: 73.27%) as a yellow solid. ES-API: [M+H]⁺ = 926.2.

Step 9: Compound 605-8 (720 mg, 0.777 mmol), 1-hydroxybenzotriazole (525.25 mg, 3.887 mmol), and N,N-diisopropylethylamine (3014.38 mg, 23.322 mmol) were dissolved in dichloromethane (20 mL). 1-(3-Dimethylaminopropyl)-3-ethylcarbodiimide hydrochloride (4470.85 mg, 23.322 mmol) was added to the solution described above under an ice bath. After the addition was completed, the reaction system was stirred at room temperature for 17 h. After the reaction was completed, the reaction mixture was washed sequentially with diluted hydrochloric acid (0.5 M, 10 mL × 2) and saturated brine (10 mL × 2). The organic phase was washed with saturated brine (20 mL), dried over anhydrous sodium sulfate, filtered, and concentrated to give a crude product. The crude product was purified by a flash silica gel column (methanol/dichloromethane: 0%-5%) to give compound 605-9 (470 mg, 0.518 mmol, yield: 66.57%) as a light yellow solid. ES-API: [M+H]⁺ = 908.3.

Step 10: Compound 605-9 (150 mg, 0.165 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give compound 605-10 (130 mg, 0.161 mmol, yield: 97.41%) as a yellow solid. ES-API: [M+H]⁺ = 808.3.

Step 11: Compound 605-10 (50 mg, 0.062 mmol) was dissolved in dichloromethane (2 mL). (1S,2S)-2-Methylcyclopropane-1-carboxylic acid (6.21 mg, 0.062 mmol), O-(7-azabenzotriazol-1-yl)-N,N,N',N'-tetramethyluronium hexafluorophosphate (35.29 mg, 0.093 mmol), and N,N'-diisopropylethylamine (0.022 mL, 0.134 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by prep-HPLC (ammonium bicarbonate method) to give (1*S*,2*S*)-N-((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6',6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z605, 20 mg, 0.022 mmol, yield: 36.24%), ES-API: [M+H]⁺ = 890.3.

### Single-crystal growth of intermediate 605-1A

Single-crystal growth of 3-(5-bromo-1-ethyl-(Rₐ)-2-(2-((S)-1-methoxyethyl)pyridin-3-yl)-1H-indol-3-yl)-2,2-dimethylpropan-1-ol (intermediate 605-1A).

30 mg of intermediate 605-1A was dissolved in 0.5 mL of chloroform, and 1.5 mL of n-hexane was added. The mixture was shaken until it was clear. The solution was filtered and sealed. Several small holes were made in the sealed part by using a needle. After slow evaporation for 24 h, a single crystal was grown and obtained. The X-ray single-crystal diffraction test was performed using a Bruker D8 Venture instrument. The results are shown in Table 2 below and FIG. 2. FIG. 2 shows an ellipsoid plot of the molecular three-dimensional structure.

### Instrument parameters:

| | | | |
|---|---|---|---|
| Light source: | Cu target | X-ray: | Cu-Kα (λ = 1.54178 Å) |
| Detector: | CMOS area detector | Resolution: | 0.80 Å |
| Current and voltage: | 50 kV, 1.2 mA | Exposure time: | 10 s |
| Area detector-to-sample distance: | 40 mm | Test temperature: | 100(2) K |

### Structure analysis and refinement procedures:

After the diffraction data were integrated and converted by adopting the SAINT program, empirical absorption correction was performed on the data by adopting the SADABS program; the structure of the single crystal was analyzed by a direct method using SHELXT2014, and the structure was refined by adopting the least square method; hydrogen atom refinement procedures were obtained by isotropic calculation, and the hydrogen atoms on C-H were obtained by calculation of hydrogenation and refined by adopting a riding model. The Flack constant was 0.043 (10). In FIG. 2, C21 is in S configuration, and the axial chirality at positions C9-C16 is in Rₐ configuration.

### Crystal data:

**Table 2**

| | |
|---|---|
| Molecular formula | 0.09(C₂₃H₂₇BrN₂O₂) |
| Molecular weight | *Mᵣ =* 41.24 |
| Shape | Block, colorless |
| Crystal system | Orthorhombic |
| Space group | *P*2₁2₁2₁ |
| Cell parameters | *a* = 7.7650 (2) Å; *b* = 10.7737 (3) Å; *c* = 25.6701 (6) Å |
| Unit cell volume | *V* = 2147.50 (10) Å³ |
| Molecule number in unit cell | *Z* = 43 |
| Unit cell dimensions | *0.15* × *0.08* × *0.05 mm* |
| Electron number in unit cell | *F*(000) = 920 |
| Calculating density | *Dₓ* = 1.371 Mg m⁻³ |
| Radiation source and wavelength | Cu Ka radiation, 1 = 1.54178 Å |
| Determining the number of diffraction points of unit cell | Cell parameters from 9839 reflections |
| Range for data collection | q = 4.1-72.3° |
| Absorption coefficient | m = 2.76 mm⁻¹ |
| Temperature | *T* = 100 K |

### Example 68. Synthesis of Compound Z559

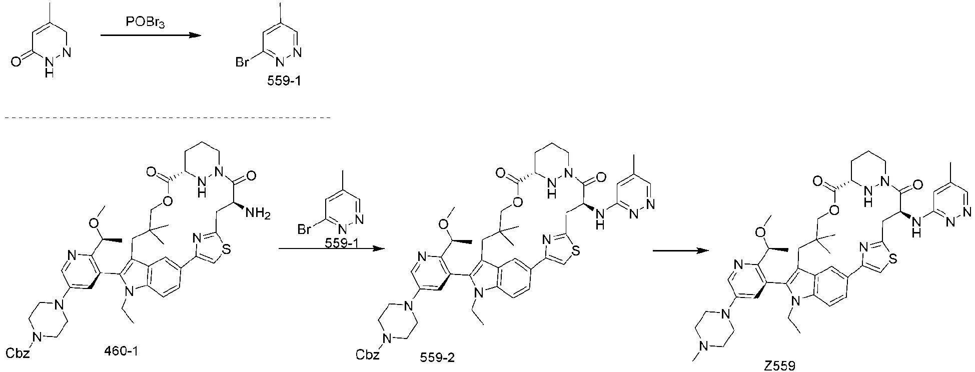

Step 1: 5-Methylpyridazin-3(2H)-one (500 mg, 4.541 mmol) and phosphorus oxybromide (2603.34 mg, 9.081 mmol) were dissolved in acetonitrile (5 mL). The reaction system was stirred at 80 °C for 4 h. After the reaction was completed, the reaction mixture was poured into ice water and basified with ammonia water. The resulting solution was extracted with ethyl acetate (20 mL × 3). The organic phase was washed with saturated brine (30 mL), dried over anhydrous sodium sulfate, filtered, and concentrated to give a crude product. The crude product was purified by a flash silica gel column (petroleum ether/ethyl acetate: 5%-40%) to give compound 559-1 (500 mg, 2.890 mmol, yield: 63.65%) as a white solid. ES-API: [M+H]⁺ = 173.1, 175.1.

Step 2: Compound 460-1 (100 mg, 0.118 mmol), compound 559-1 (101.88 mg, 0.589 mmol), (SP-4-2)-bromo[[3,6-dimethoxy-2',4',6'-tris(1-methylethyl)[1,1'-biphenyl]-2-yl-κC1']bis(1,1-dimethylethyl)phosphine-κP][4-[[2-(trimethylsilyl)ethoxy]carbonyl]phenyl]palladium (CAS: 2097600-19-4, 21.02 mg, 0.024 mmol), cesium carbonate (115.12 mg, 0.353 mmol), and anhydrous tetrahydrofuran (5 mL) were added to a microwave reaction tube. The reaction system was microwaved at 120 °C for 1 h under a nitrogen atmosphere. The reaction mixture was filtered and concentrated to give a crude product. The crude product was purified by preparative thin-layer chromatography (silicon dioxide, ethyl acetate/(dichloromethane/methanol = 10:1) = 10:1, R_{f} = 0.3) to give compound 559-2 (7 mg, 0.007 mmol, yield: 6.31%) as a white solid. ES-API: [M+H]⁺ = 941.3.

Step 3: Compound 559-2 (7 mg, 0.007 mmol) was dissolved in methanol (2 mL), and wet palladium hydroxide/carbon (10 wt%, 10 mg, 0.007 mmol) and paraformaldehyde (2.23 mg, 0.074 mmol on a formaldehyde equivalent basis) were added to the solution described above. The reaction system was stirred for 17 h under a hydrogen atmosphere (15 psi). The reaction mixture was filtered through celite, and the filter cake was washed with a solution of ammonia in methanol (7 M, 20 mL). The filtrate was concentrated to give a crude product. The crude product was purified by prep-HPLC (ammonium bicarbonate method) to give (6³S,4S,Z)-1'-ethyl-(Rₐ)-1²-(2-((S)-1-methoxyethyl)-5-(4-methylpiperazin-1-yl)pyridin-3-yl)-10,10-dimethyl-4-((5-methylpyridazin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z559, 0.45 mg, 0.001 mmol, yield: 7.36%) as a white solid. ES-API: [M+H]⁺ = 821.3.

### Example 69. Synthesis of Compounds Z628-1 and Z628-2

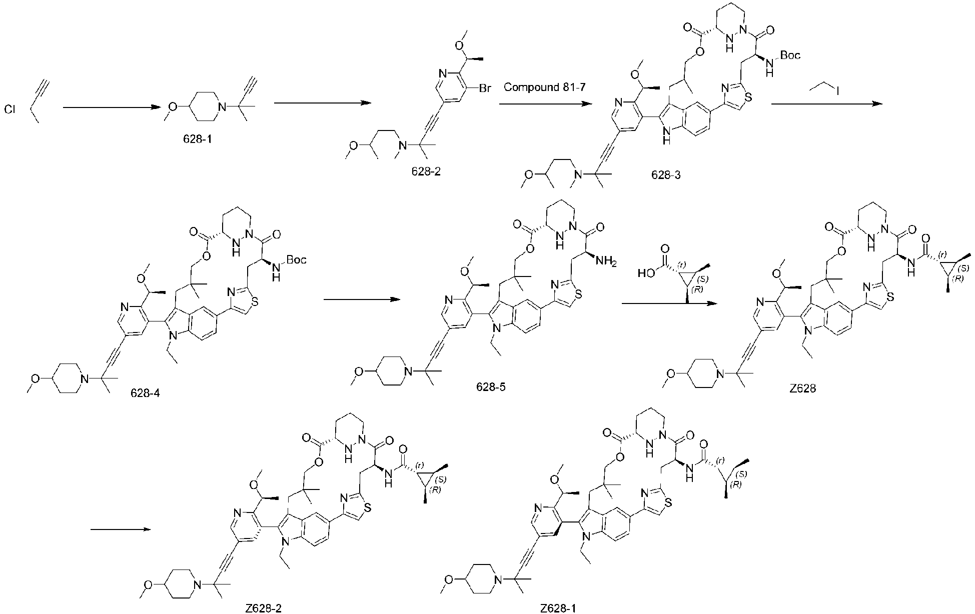

Step 1: Copper(II) chloride (0.10 g, 0.975 mmol) was added to a solution of 3-chloro-3-methylbut-1-yne (1 g, 9.75 mmol), 4-methoxypiperidine (1.12 g, 9.75 mmol), and triethylamine (1.97 g, 19.5 mmol) in tetrahydrofuran (10 mL) under a nitrogen atmosphere. Subsequently, an exothermic reaction was performed, forming a precipitate. The mixture was stirred at room temperature for 0.5 h. After the reaction was completed, water (20 mL) and 1 M hydrochloric acid (8 mL) were added. The mixture was washed with ethyl acetate (20 mL × 2) and basified with potassium carbonate. The mixture was extracted with ethyl acetate (20 mL × 3), washed with brine (30 mL), dried over anhydrous sodium sulfate, filtered, and concentrated, and the residue was purified by a flash silica gel column (0-40% petroleum ether/ethyl acetate) to give compound 628-1 (1.2 g, 6.620 mmol, yield: 67.80%). ES-API [M+H]⁺ = 182.3.

Step 2: A mixture of compound 628-1 (1.13 g, 3.310 mmol), N,N-diisopropylethylenediamine (0.57 g, 4.413 mmol), 4-methoxy-1-(2-methylbut-3-en-2-yl)piperidine (0.4 g, 2.207 mmol), and copper(I) iodide (0.04 g, 0.221 mmol) was stirred at room temperature overnight in acetonitrile (20 mL) under a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-40% petroleum ether/ethyl acetate) to give compound 628-2 (0.6 g, 1.52 mmol, yield: 68.70%). ES-API [M+H]⁺ = 395.1/397.1.

Step 3: A mixture of compound 81-7 (100 mg, 0.144 mmol), compound 628-2 (113.99 mg, 0.288 mmol), [1,1'-bis(di-tert-butylphosphino)ferrocene]dichloropalladium(II) (18.77 mg, 0.029 mmol), and potassium phosphate (91.80 mg, 0.432 mmol) was stirred at 65 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-10% methanol/dichloromethane) to give compound 628-3 (110 mg, yield: 86.5%) as a transparent oil. ES-API [M+H]⁺ = 882.4.

Step 4: Compound 628-3 (110 mg, 0.125 mmol) was dissolved in N,N-dimethylformamide (5 mL), and cesium carbonate (121.89 mg, 0.374 mmol) and iodoethane (388.98 mg, 2.494 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 628-4 (110 mg, yield: 96.9%) as a yellow oil. ES-API [M+H]⁺ = 910.3.

Step 5: Compound 628-4 (110 mg, 0.121 mmol) was dissolved in a solution of hydrochloric acid in dioxane (5 mL, 4 mol/L). The mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 628-5 (97 mg, yield: 99%). ES-API [M+H]⁺ = 810.3.

Step 6: Compound 628-5 (97.21 mg, 0.12 mmol) was dissolved in N,N-dimethylformamide (6 mL), and (1r,2R,3S)-2,3-dimethylcyclopropane-1-carboxylic acid (16.4 mg, 0.14 mmol), triethylamine (0.100 mL, 0.720 mmol), and O-(7-azabenzotriazol-1-yl)-N,N,N',N'-tetramethyluronium hexafluorophosphate (91.26 mg, 0.240 mmol) were sequentially added. The mixture was stirred at room temperature for 1 h. After the reaction was completed, ethyl acetate (20 mL) was added, and the mixture was washed with saturated brine (10 mL) and water (10 mL). The organic phase was dried over anhydrous sodium sulfate, filtered, and concentrated to give crude compound Z628. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-1¹-ethyl-(Sₐ)-1²-(2-((S)-1-methoxyethyl)-5-(3-(4-methoxypiperidin-1-yl)-3-methylbut-1-yn-1-yl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z628-2, 20 mg, yield: 18%, purity: 98%, retention time: 8.831 min). ES-API [M+H]⁺ = 906.5.

The structure of the other isomeric compound was arbitrarily designated as (1r,2R,3S)-N-((6³S,4S,Z)-1¹-ethyl-(Rₐ)-1²-(2-((S)-1-methoxyethyl)-5-(3-(4-methoxypiperidin-1-yl)-3-methylbut-1-yn-1-yl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z628-1, 10 mg, yield: 9%, purity: 98%, retention time: 9.013 min). ES-API [M+H]⁺ = 906.5.

### Example 70. Synthesis of Compounds Z625 and Z625-1

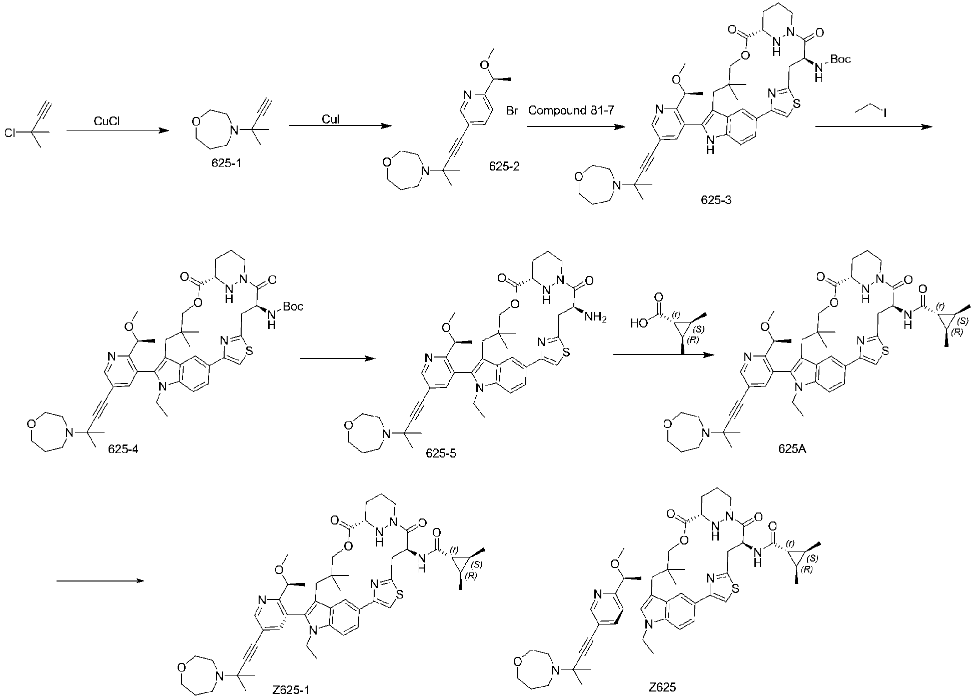

Step 1: Copper(II) chloride (0.10 g, 0.975 mmol) was added to a solution of 3-chloro-3-methylbut-l-yne (1 g, 9.75 mmol), 1,4-oxazepane (0.99 g, 9.75 mmol), and triethylamine (1.97 g, 19.5 mmol) in tetrahydrofuran (10 mL) under a nitrogen atmosphere. Subsequently, an exothermic reaction was performed, forming a precipitate. The mixture was stirred at room temperature for 0.5 h. After the reaction was completed, water (20 mL) and 1 M hydrochloric acid (8 mL) were added. The mixture was washed with ethyl acetate (20 mL × 2) and basified with potassium carbonate. The mixture was extracted with ethyl acetate (20 mL × 3). The combined extract was washed with brine (30 mL), dried over anhydrous sodium sulfate, filtered, and concentrated, and the residue was subjected to flash silica gel column chromatography (0-40% petroleum ether/ethyl acetate) to give compound 625-1 (1.3 g, yield: 79.80%). ES-API [M+H]⁺ = 168.3.

Step 2: A mixture of 3-bromo-5-iodo-2-[(1S)-1-methoxyethyl]pyridine (1.22 g, 3.58 mmol), N,N-diisopropylethylenediamine (0.62 g, 4.8 mmol), compound 625-1 (0.4 g, 2.4 mmol), and copper(I) iodide (0.04 g, 0.221 mmol) was stirred at room temperature overnight in acetonitrile (20 mL) under a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-40% petroleum ether/ethyl acetate) to give compound 625-2 (0.4 g, yield: 43.80%). ES-API [M+H]⁺ = 381.1/383.1.

Step 3: A mixture of compound 81-7 (150 mg, 0.216 mmol), compound 625-2 (113 mg, 0.296 mmol), [1,1'-bis(di-tert-butylphosphino)ferrocene]dichloropalladium(II) (18.77 mg, 0.029 mmol), and potassium phosphate (91.80 mg, 0.432 mmol) was stirred at 65 °C for 18 h in dioxane (9 mL), toluene (3 mL), and water (3 mL) under an oil bath and a nitrogen atmosphere. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-10% methanol/dichloromethane) to give compound 625-3 (130 mg, yield: 69%) as a transparent oil. ES-API [M+H]⁺ = 868.4.

Step 4: Compound 625-3 (130 mg, 0.125 mmol) was dissolved in N,N-dimethylformamide (5 mL), and cesium carbonate (146 mg, 0.45 mmol) and iodoethane (233.57 mg, 1.5 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% tetrahydrofuran/petroleum ether) to give compound 625-4 (130 mg, yield: 97%) as a yellow oil. ES-API [M+H]⁺ = 896.3.

Step 5: Compound 625-4 (110 mg, 0.121 mmol) was dissolved in a solution of hydrochloric acid in dioxane (5 mL, 4 M). The mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 625-5 (119 mg, yield: 99%). ES-API [M+H]⁺ = 796.3.

Step 6: Compound 625-5 (119 mg, 0.15 mmol) was dissolved in N,N-dimethylformamide (6 mL), and (1r,2R,3S)-2,3-dimethylcyclopropane-1-carboxylic acid (20 mg, 0.18 mmol), triethylamine (0.100 mL, 0.720 mmol), and O-(7-azabenzotriazol-1-yl)-N,N,N',N'-tetramethyluronium hexafluorophosphate (91.26 mg, 0.240 mmol) were sequentially added. The mixture was stirred at room temperature for 1 h. After the reaction was completed, ethyl acetate (20 mL) was added, and the mixture was washed with saturated brine (10 mL) and water (10 mL). The organic phase was dried over anhydrous sodium sulfate, filtered, and concentrated to give crude compound 625A. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-1¹-ethyl-(Sₐ)-1²-(2-((S)-1-methoxyethyl)-5-(3-(1,4-oxazepan-4-yl)-3-methylbut-1-yn-1-yl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z625-1, 20 mg, yield: 15%, purity: 98%, retention time: 8.864 min). ES-API [M+H]⁺ = 892.5.

The structure of the other isomeric compound was arbitrarily designated as (1r,2R,3S)-N-((6³S,4S,Z)-1¹-ethyl-(Rₐ)-1²-(2-((S)-1-methoxyethyl)-5-(3-(1,4-oxazepan-4-yl)-3-methylbut-1-yn-1-yl)pyridin-3-yl)-10,10-dimethyl-5,7-dioxo-6¹,6²,6³,6⁴,6⁵,6⁶hexahydro-1¹H-8-oxa-2(4,2)-thiazola-l(5,3)-indola-6(1,3)-pyridazinacycloundecaphane-4-yl)-2,3-dimethylcyclopropane-1-carboxamide (Z625, 20 mg, yield: 15%, purity: 98%, retention time: 9.073 min). ES-API [M+H]⁺ = 892.5.

### Example 71. Synthesis of Compound Z777

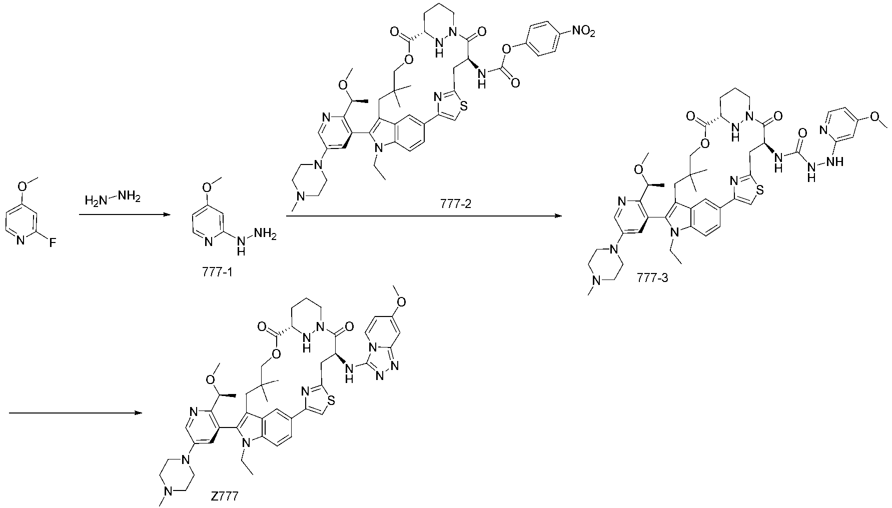

Step 1: 2-Fluoro-4-methoxypyridine (750 mg, 5.900 mmol) was dissolved in absolute ethanol (1 mL), and hydrazine hydrate (7.169 mL, 117.999 mmol) was added. The mixture was heated to 90 °C in microwaves and stirred for 20 min. The reaction mixture was concentrated, and ethyl acetate (30 mL) was added. The mixture was dried over anhydrous sodium sulfate, filtered, and concentrated, and the residue was purified by silica gel column chromatography (eluent: tetrahydrofuran/petroleum ether = 2/1) to give compound 777-1 (650 mg, yield: 79.17%). ES-API: [M+H]⁺ = 140.2.

Step 2: Compound 777-1 (115.5 mg, 0.830 mmol) was dissolved in dry dichloromethane (5 mL), and compound 777-2 (148 mg, 0.166 mmol, see A173 in WO2022060836A1 for the preparation method) was added under an ice-water bath. The mixture was reacted for 1 h under an ice-water bath and a nitrogen atmosphere. The mixture was extracted with dichloromethane (30 mL × 3). The organic phases were combined, washed with saturated brine (30 mL × 1), dried over anhydrous sodium sulfate, filtered, and concentrated, and the residue was purified by silica gel column chromatography (0-7% methanol/dichloromethane) to give compound 777-3 (50 mg, yield: 34%), ES-API: [M+H]⁺ = 894.3.

Step 3: Compound 777-3 (50 mg, 0.056 mmol) and pyridine (0.067 mL, 0.839 mmol) were dissolved in toluene (5 mL), and phosphorus oxychloride (0.026 mL, 0.280 mmol) was slowly added under an ice-water bath. The mixture was heated to 75 °C and stirred for 3 h. The mixture was cooled to 0 °C, and the reaction was quenched with a 7 M ammonia-methanol solution (0.5 mL). The reaction mixture was concentrated, and the residue was purified by prep-HPLC (ammonia water method) to give N-((6³S,4S,Z)-1¹-ethyl-(*Rₐ*)-1²-(2-((S)-1-methoxyethyl)-5-(4-methylpiperazin-1-yl)pyridin-3-yl)-10,10-dimethyl-5,7-dioxo-6¹,6²,6³,6⁴,6⁵ ,6⁶-hexahydro-1¹H-8-oxa-2(4,2)-thiazola-1(5,3)-indola-6(1,3)-pyridazinacycloundecaphane-4-yl)-4-(7-methoxy-[1,2,4]triazolo[4,3-a]pyridin-3-yl)amine (Z777, 5.7 mg, yield: 11.64%). ES-API: [M+H]⁺ = 876.4. ¹H NMR (400 MHz, DMSO-d₆) δ 8.51 (d, J = 1.6 Hz, 1H), 8.45 (d, J = 2.9 Hz, 1H), 8.24 (d, J = 7.5 Hz, 1H), 7.77 (s, 1H), 7.72 (dd, J = 8.6, 1.6 Hz, 1H), 7.54 (d, J = 8.7 Hz, 1H), 7.22 (d, J = 2.9 Hz, 1H), 7.12 (d, J = 10.7 Hz, 1H), 6.77 (d, J = 2.4 Hz, 1H), 6.60 (dd, J = 7.5, 2.4 Hz, 1H), 5.50 (t, J = 10.0 Hz, 1H), 5.17 (d, J = 12.2 Hz, 1H), 4.28 (q, J = 12.2, 9.7 Hz, 3H), 4.15 (q, J = 6.5, 5.7 Hz, 2H), 3.81 (s, 3H), 3.58 (q, J = 10.9 Hz, 2H), 3.48 (d, J = 14.5 Hz, 1H), 3.27 (t, J = 5.1 Hz, 4H), 3.21 (s, 3H), 2.95 (d, J = 14.3 Hz, 1H), 2.81 (d, J = 14.8 Hz, 1H), 2.48 - 2.39 (m, 5H), 2.22 (s, 3H), 2.12 (d, J = 12.2 Hz, 1H), 2.00 (q, J = 7.0 Hz, 1H), 1.82 (s, 2H), 1.58 (d, J = 8.0 Hz, 1H), 1.33 (d, J = 6.1 Hz, 3H), 0.91 (dd, J = 13.6, 6.6 Hz, 6H), 0.36 (s, 3H).

### Example 72. Synthesis of Compound Z607

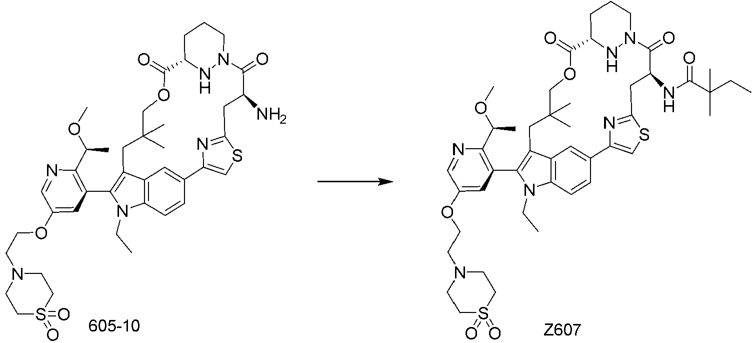

Step 1: Compound 605-10 (50 mg, 0.062 mmol) was dissolved in dichloromethane (2 mL). 2,2-Dimethylbutyric acid (6.47 mg, 0.056 mmol), O-(7-azabenzotriazol-1-yl)-N,N,N',N'-tetramethyluronium hexafluorophosphate (35.29 mg, 0.093 mmol), and N,N'-diisopropylethylamine (0.022 mL, 0.134 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by prep-HPLC (ammonium bicarbonate method) to give *N*-((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylbutanamide (Z607, 23 mg, 0.025 mmol, yield: 45.58%). ES-API: [M+H]⁺ = 906.3.

### Example 73. Synthesis of Compound Z610

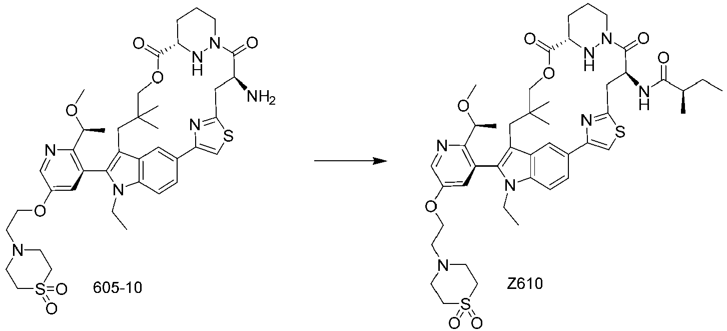

Step 1: Compound 605-10 (15 mg, 0.019 mmol) was dissolved in dichloromethane (2 mL). (2R)-2-Methylbutyric acid (2 mg, 0.019 mmol), O-(7-azabenzotriazol-1-yl)-*N,N,N',N'*-tetramethyluronium hexafluorophosphate (10.29 mg, 0.028 mmol), and *N,N'*-diisopropylethylamine (0.022 mL, 0.134 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by prep-HPLC (ammonium bicarbonate method) to give (2R)-*N*-((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-methylbutanamide (Z610, 2.8 mg, 0.003 mmol, yield: 16.91%). ES-API: [M+H]⁺ = 892.3.

### Example 74. Synthesis of Compound Z609

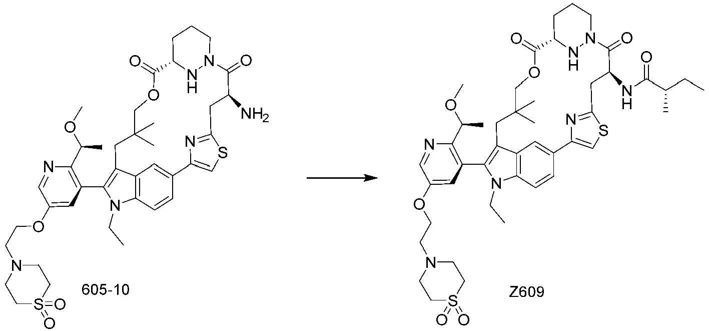

Step 1: Compound 605-10 (15 mg, 0.019 mmol) was dissolved in dichloromethane (2 mL). (2S)-2-Methylbutyric acid (2 mg, 0.019 mmol), O-(7-azabenzotriazol-1-yl)-*N,N,N',N'*-tetramethyluronium hexafluorophosphate (10.29 mg, 0.028 mmol), and *N,N'*-diisopropylethylamine (0.022 mL, 0.134 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by prep-HPLC (ammonium bicarbonate method) to give (2S)-*N*-((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-methylbutanamide (Z609, 3 mg, 0.003 mmol, yield: 17.70%). ES-API: [M+H]⁺ = 892.3.

### Example 75. Synthesis of Compounds Z642 and Z642-1

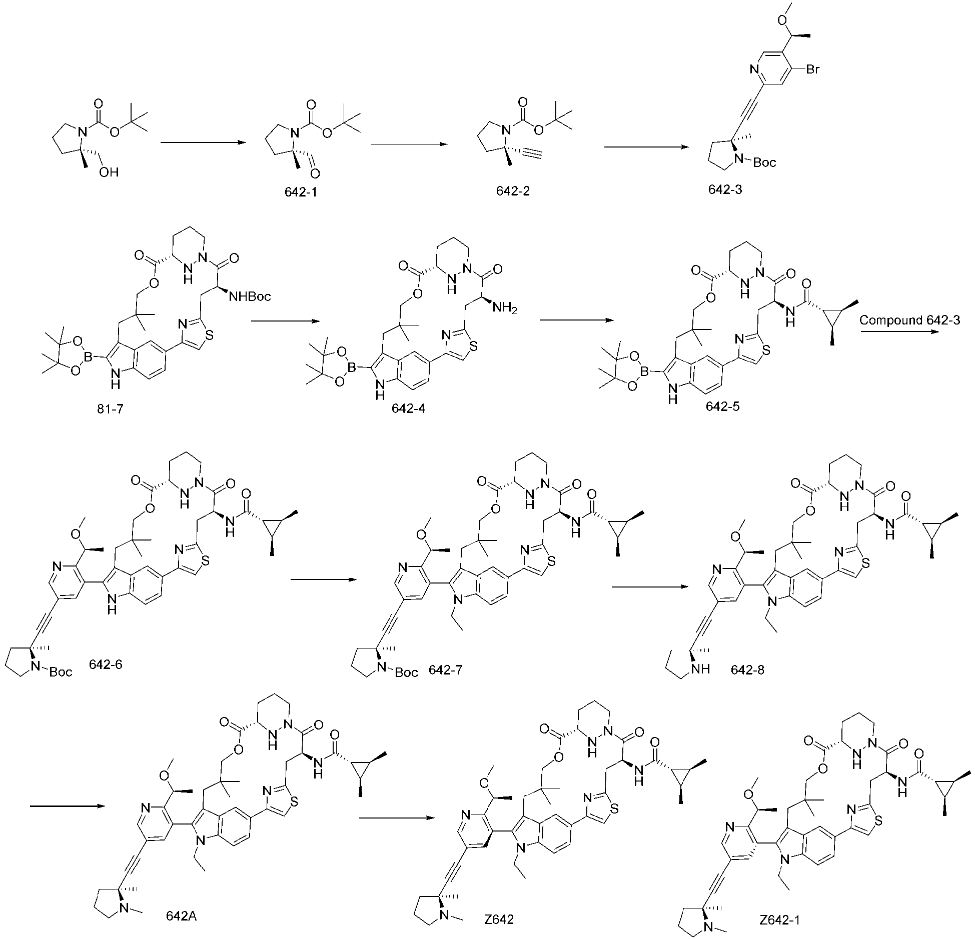

Step 1: 2-Methylpropan-2-yl (2R)-2-(hydroxymethyl)-2-methyltetrahydropyrrole-1-carboxylate (1.2 g, 5.574 mmol) was dissolved in dichloromethane (20 mL), and Dess-Martin periodinane (2.84 g, 6.689 mmol) was added. The mixture was reacted at room temperature for 1 h. A saturated sodium bicarbonate solution (10 mL) and water (10 mL) were added, and the mixture was extracted with dichloromethane (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 642-1 (0.8 g, 3.751 mmol, yield: 67.23%). ES-API: [M-56]⁺ = 158.2.

Step 2: Compound 642-1 (0.8 g, 3.751 mmol) and potassium carbonate (1.04 g, 7.502 mmol) were dissolved in methanol (10.00 mL), and dimethyl (1-diazo-2-oxopropyl)phosphonate (1.08 g, 5.626 mmol) was added. The mixture was reacted at room temperature for 1 h. A saturated sodium bicarbonate solution (10 mL) and water (10 mL) were added, and the mixture was extracted with dichloromethane (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 642-2 (0.6 g, 2.867 mmol, yield: 76.43%). ES-API: [M-56]⁺ = 154.2.

Step 3: 3-Bromo-5-iodo-2-[(1S)-1-methoxyethyl]pyridine (550 mg, 1.608 mmol), copper(I) iodide (153.15 mg, 0.804 mmol), and [1,1'-bis(diphenylphosphino)ferrocene]palladium(II) dichloride dichloromethane complex (328.36 mg, 0.402 mmol) were dissolved in N,N-dimethylformamide (2.00 mL), and compound 642-2 (673.21 mg, 3.217 mmol) and N,N-diisopropylethylamine (1039.38 mg, 8.042 mmol) were added. The mixture was reacted at 50 °C for 2 h, and water (10 mL) was added. The mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 642-3 (0.6 g, 2.867 mmol, yield: 76.43%). ES-API: [M+H]⁺ = 423.1.

Step 4: Compound 81-7 (120 mg, 0.173 mmol) was dissolved in methanol (0.5 mL), and a solution of hydrochloric acid in dioxane (3 mL, 0.173 mmol) was added. The mixture was reacted at room temperature for 1 h. The reaction mixture was concentrated under reduced pressure to give compound 642-4 (100 mg, 0.168 mmol, yield: 97.39%). ES-API: [M+H]⁺ = 594.3.

Step 5: Compound 642-4 (80 mg, 0.099 mmol) was dissolved in N,N-dimethylformamide (2 mL), and (2R,3S)-2,3-dimethylcyclopropane-1-carboxylic acid (17.31 mg, 0.152 mmol), O-(7-azabenzotriazol-1-yl)-N,N,N',N'-tetramethyluronium hexafluorophosphate (86.48 mg, 0.227 mmol), and N,N-diisopropylethylamine (58.79 mg, 0.455 mmol) were added. The mixture was reacted at room temperature for 1 h. Water (10 mL) was added to the reaction mixture, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 642-5 (90 mg, 0.130 mmol, yield: 86.06%). ES-API: [M+H]⁺ = 690.3.

Step 6: Compound 642-5 (90 mg, 0.130 mmol) and compound 642-3 (110.49 mg, 0.261 mmol) were dissolved in toluene (1.5 mL), 1,4-dioxane (0.5 mL), and water (0.5 mL), and potassium phosphate (69.25 mg, 0.326 mmol) and [1,1'-bis(di-tert-butylphosphino)ferrocene]dichloropalladium(II) (6.58 mg, 0.010 mmol) were added. The mixture was reacted at 70 °C for 3 h under a nitrogen atmosphere. Water (5 mL) was added, and the mixture was extracted with ethyl acetate (10 mL × 3). The organic phase was washed with saturated brine (5 mL), dried over anhydrous sodium sulfate, and concentrated under reduced pressure, and the residue was purified by silica gel column chromatography (ethyl acetate:petroleum ether = 1:1) to give compound 642-6 (65 mg, 0.072 mmol, yield: 54.97%). ES-API: [M+H]⁺ = 906.3.

Step 7: Compound 642-6 (65 mg, 0.072 mmol) was dissolved in N,N-dimethylformamide (1 mL), and cesium carbonate (58.18 mg, 0.179 mmol) and iodoethane (13.93 mg, 0.089 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give compound 642-7 (0.06 g, 0.07 mmol, yield: 97.2%). ES-API: [M+H]⁺ = 934.1.

Step 8: Compound 642-7 (60 mg, 0.064 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 642-8 (53 mg, 0.06 mmol, yield: 99.67%). ES-API: [M+H]⁺ = 834.4. Step 9: Compound 642-8 (45 mg, 0.054 mmol) was dissolved in methanol (1 mL), and formic acid (12.41 mg, 0.270 mmol), formaldehyde (8.09 mg, 0.270 mmol), and sodium cyanoborohydride (3.72 mg, 0.060 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, and concentrated under reduced pressure to give crude compound 642A. The crude product was purified by prep-HPLC (ammonium bicarbonate method) to give 2 isomeric compounds. The structure of one of the isomeric compounds was arbitrarily designated as (1r,2R,3S)-*N*-((6³*S*,4*S*,*Z*)-(*R*ₐ)-1²-(5-(((*S*)-1,2-dimethylpyrrolidin-2-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide) (Z642, 8 mg, 0.009 mmol, yield: 17.48%, retention time: 2.59 min). ES-API: [M+H]⁺ = 848.3.

The structure of the other isomeric compound was arbitrarily designated as (1r,2R,3S)-*N*-((6³*S*,4*S*,*Z*)-(*Sₐ*)-1²-(5-(((S)-1,2-dimethylpyrrolidin-2-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z642-1, 10 mg, 0.012 mmol, 21%, retention time: Rt = 2.46 min). ES-API: [M+H]⁺ = 848.3. ¹H NMR (400 MHz, DMSO-d₆) δ 8.78 (d, J = 1.6 Hz, 1H), 8.53 (s, 1H), 8.41 (d, J = 9.2 Hz, 1H), 7.93 (d, J = 1.6 Hz, 1H), 7.82 (s, 1H), 7.74 (d, J = 8.4 Hz, 1H), 7.55 (d, J = 8.4 Hz, 1H), 5.54 (t, J = 9.2 Hz, 1H), 5.04 (d, J = 12.4 Hz, 1H), 4.25 - 4.19 (m, 2H), 3.96 - 3.82 (m, 3H), 3.68 - 3.66 (m, 1H), 3.56 - 3.54 (m, 1H), 3.18-2.92 (m, 6H), 2.37 - 2.21 (m, 5H), 2.15-2.13 (m, 2H), 1.89 - 1.66 (m, 5H), 1.38 (s, 3H), 1.32 - 1.01 (m, 18H), 0.98 (s, 3H), 0.51 (s, 3H).

### Example 76. Synthesis of Compound Z569

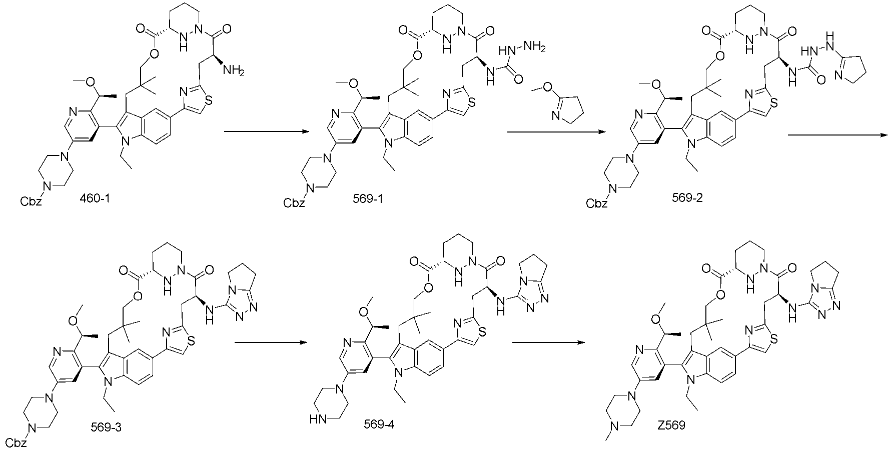

Step 1: Compound 460-1 (447 mg, 0.53 mmol) and N,N-diisopropylethylamine (272 mg, 2.11 mmol) were dissolved in dichloromethane (15 mL), and nitrophenyl chloroformate (138 mg, 0.68 mmol) was added at 0 °C. The reaction system was stirred for 30 min under an ice bath, and then 80% hydrazine hydrate (184 mg, 2.94 mmol) was added. The reaction system was stirred at room temperature for 3 h. Dichloromethane (50 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (methanol/dichloromethane: 0-7%) to give compound 569-1 (430 mg, yield: 80.6%) as a yellow solid. ES-API: [M+H]⁺ = 907.3.

Step 2: Compound 569-1 (400 mg, 0.44 mmol) was dissolved in acetonitrile (15 mL), and 2-methoxy-1-pyrrolidine (175 mg, 1.76 mmol) was added at room temperature. The reaction system was stirred at 75 °C for 18 h. The reaction mixture was concentrated, and the crude product was purified by a flash silica gel column (methanol/dichloromethane: 0-10%) to give compound 569-2 (250 mg, yield: 58.2%) as a white solid. ES-API: [M+H]⁺ = 974.3.

Step 3: Compound 569-2 (250 mg, 0.26 mmol) and pyridine (305 mg, 3.85 mmol) were dissolved in toluene (15 mL) and phosphorus oxychloride (197 mg, 1.28 mmol). The reaction system was stirred at 75 °C for 1 h. The reaction mixture was cooled to room temperature, and the organic layer was discarded. Dichloromethane/7 M ammonia-methanol solution (20 mL, 5:1) was added to the remaining solid, and the mixture was stirred at room temperature for 30 min. The solution was concentrated. The residue was purified by a flash silica gel column (7 M ammonia-methanol solution/dichloromethane: 2-8%) to give compound 569-3 (60 mg, yield: 24.4%) as a pale yellow solid. ES-API: [M+H]⁺ = 956.3.

Step 4: Compound 569-3 (60 mg, 0.063 mmol) was dissolved in absolute methanol (10 mL), and 10% palladium hydroxide on carbon (60 mg) was added. The reaction system was stirred at 30 °C for 24 h under a hydrogen atmosphere. The reaction mixture was filtered through celite and washed with dichloromethane/7 M ammonia-methanol solution (100 mL, 5:1). The filtrate was concentrated to give compound 569-4 (50 mg, yield: 96.9%) as a white solid. ES-API: [M+H]⁺ = 822.3.

Step 5: Compound 569-4 (50 mg, 0.061 mmol) was dissolved in methanol (5 mL), and a 37% formaldehyde solution (15 mg, 0.18 mmol), acetic acid (11 mg, 0.18 mmol), and sodium cyanoborohydride (7 mg, 0.18 mmol) were added at room temperature. The reaction system was stirred at room temperature for 1 h. The reaction mixture was quenched with water (10 mL), and saturated sodium bicarbonate (1 mL) was added. The mixture was extracted with dichloromethane (50 mL). The organic phase was washed with saturated brine (10 mL), dried over anhydrous sodium sulfate, and concentrated to give a crude product. The crude product was purified by prep-HPLC (ammonium bicarbonate method) to give (6³S,4S,Z)-4-((6,7-dihydro-5H-pyrrolo[2,1-c][1,2,4]triazol-3-yl)amino)-1¹-ethyl-(Rₐ)-1²-(2-((S)-1-methoxyethyl)-5-(4-methylpiperazin-1-yl)pyridin-3-yl)-10-10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z569, 16 mg, yield: 31.5%) as a white solid. ¹H NMR (400 MHz, DMSO-d₆) δ 8.49 (s, 1H), 8.44 (d, *J =* 2.4 Hz, 1H), 7.78 (s, 1H), 7.72 (d, *J =* 8.4 Hz, 1H), 7.55 (d, *J =* 8.8 Hz, 1H), 7.22 (d, *J =* 2.4 Hz, 1H), 6.65 (d, *J =* 10.8 Hz, 1H), 5.29 (t, *J =* 10.0 Hz, 1H), 5.06 (d, *J =* 12.0 Hz, 1H), 4.32 - 4.05 (m, 5H), 3.86 - 3.70 (m, 2H), 3.64 - 3.50 (m, 2H), 3.40 - 3.34 (m, 1H), 3.29 - 3.23 (m, 4H), 3.22 - 3.11 (m, 4H), 2.94 (d, *J =* 14.0 Hz, 1H), 2.83 - 2.73 (m, 1H), 2.68 - 2.54 (m, 4H), 2.48 - 2.39 (m, 5H), 2.22 (s, 3H), 2.09 (d, *J =* 11.2 Hz, 1H), 1.84 - 1.68 (m, 2H), 1.60 - 1.43 (m, 1H), 1.33 (d, *J =* 6.0 Hz, 3H), 0.95 - 0.85 (m, 6H), 0.35 (s, 3H). ES-API: [M+H]⁺ = 836.4.

### Example 77. Synthesis of Compound Z624

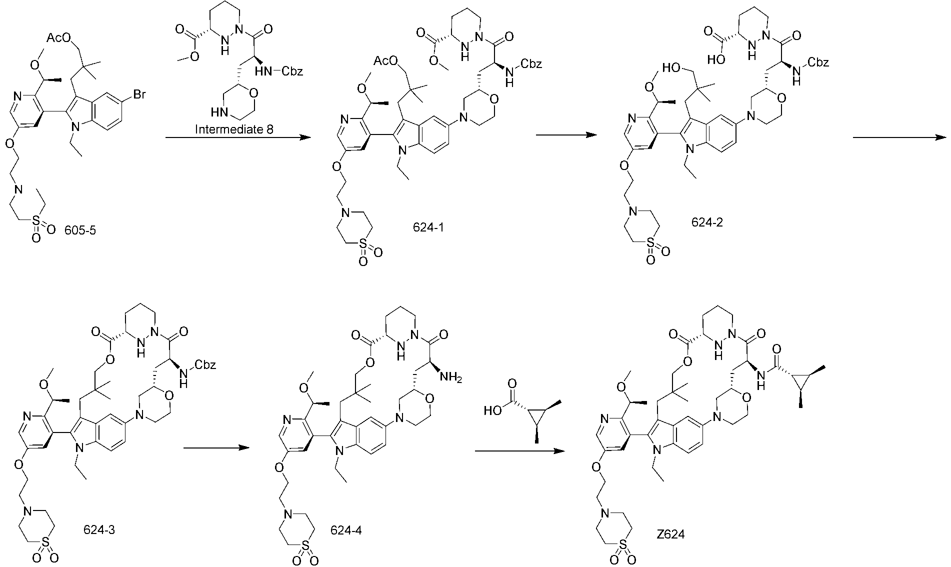

Step 1: Compound 605-5 (100 mg, 0.150 mmol) was dissolved in anhydrous dioxane (2 mL), and compound intermediate 8 (78.44 mg, 0.181 mmol), cesium carbonate (98.04 mg, 0.301 mmol), chloro(2-dicyclohexylphosphino-2',6'-diisopropoxy-1,1'-biphenyl)[2-(2'-amino-1,1'-biphenyl)]palladium(II) (11.69 mg, 0.015 mmol), and 2-dicyclohexylphosphino-2',6'-diisopropoxy-1,1'-biphenyl (14.04 mg, 0.030 mmol) were added to the solution described above. After the addition was completed, the reaction system was stirred at 100 °C for 17 h under a nitrogen atmosphere. After the reaction was completed, water (10 mL) was added to the solution described above, and the mixture was extracted with ethyl acetate (10 mL × 2). The organic phase was washed with saturated brine (10 mL × 2), dried over anhydrous sodium sulfate, filtered, and concentrated to give a crude product. The crude product was purified by a flash silica gel column (methanol/dichloromethane: 0%-3%) to give compound 624-1 (30 mg, 0.029 mmol, yield: 19.58%) as a light yellow solid. ES-API: [M+H]⁺ = 1018.3.

Step 2: Compound 624-1 (30 mg, 0.029 mmol) was dissolved in tetrahydrofuran (0.5 mL) and water (0.5 mL). Lithium hydroxide (1.50 mg, 0.059 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (10 mL × 2). The organic phase was washed with saturated brine (10 mL), dried over anhydrous sodium sulfate, filtered, and concentrated to give compound 624-2 (10 mg, crude product) as a yellow solid. ES-API: [M+H]⁺ = 962.3.

Step 3: Compound 624-2 (10 mg, crude product), 1-hydroxybenzotriazole (7.02 mg, 0.052 mmol), and N,N-diisopropylethylamine (40 mg, 0.312 mmol) were dissolved in dichloromethane (5 mL). 1-(3-Dimethylaminopropyl)-3-ethylcarbodiimide hydrochloride (56 mg, 0.291 mmol) was added to the solution described above under an ice bath. After the addition was completed, the reaction system was stirred at room temperature for 17 h. After the reaction was completed, the reaction mixture was washed sequentially with diluted hydrochloric acid (0.5 M, 3 mL × 2) and saturated brine (3 mL × 2). The organic phase was washed with saturated brine (10 mL), dried over anhydrous sodium sulfate, filtered, and concentrated to give a crude product. The crude product was purified by a flash silica gel column (methanol/dichloromethane: 0%-4%) to give compound 624-3 (3 mg, 0.003 mmol, yield: 30.57%) as a light yellow solid. ES-API: [M+H]⁺ = 944.3.

Step 4: Compound 624-3 (3 mg, 0.003 mmol) was dissolved in methanol (2 mL), and wet Pd/C (3 mg, 10 Wt%, 0.003 mmol) was added to the solution described above. After the addition was completed, the reaction system was stirred for 2 h under a hydrogen atmosphere (15 psi). After the reaction was completed, the reaction mixture was filtered through celite, and the filter cake was washed with a solution of ammonia in methanol (7 M, 20 mL). The filtrate was concentrated to give compound 624-4 (2.5 mg, crude product) as a light yellow oily liquid. ES-API: [M+H]⁺ = 810.3.

Step 5: Compound 624-4 (2.5 mg, crude product) was dissolved in dichloromethane (1 mL). (2R,3S)-2,3-Dimethylcyclopropane-1-carboxylic acid (0.6 mg, 0.005 mmol), O-(7-azabenzotriazol-1-yl)-*N,N,N',N'*-tetramethyluronium hexafluorophosphate (1.8 mg, 0.005 mmol), and *N,N'*-diisopropylethylamine (2.5 mg, 0.02 mmol) were added to the solution described above. After the addition was completed, the reaction system was stirred at room temperature for 2 h. After the reaction was completed, the reaction mixture was concentrated to remove dichloromethane. Water (2 mL) was added to the residue, and the mixture was extracted with ethyl acetate (2 mL × 2). The organic phase was washed with saturated brine (5 mL), dried over anhydrous sodium sulfate, filtered, and concentrated to give a crude product. The crude product was purified by prep-HPLC (ammonium bicarbonate method) to give (2R,3S)-N-((2²S,6³S,4S)-(Rₐ)-1²-(5-(2-(1,1-dioxidothiomorpholino)ethoxy)-2-((S)-1-methoxyethyl)pyridin-3-yl)-11-ethyl-10,10-dimethyl-5,7-dioxo-6¹,6²,6³,6⁴,6⁵,6⁶-hexahydro-1¹H-8-oxa-2(4,2)-morpholina-1(5,3)-indola-6(1,3)-pyridazinacycloundecaphane-4-yl)-2,3-dimethylcyclopropane-1-carboxamide (Z624, 0.85 mg, 0.001 mmol, two-step yield: 30%) as a white solid. ES-API: [M+H]⁺ = 906.1.

### Example 78. Synthesis of Compounds Z599 and Z599-1

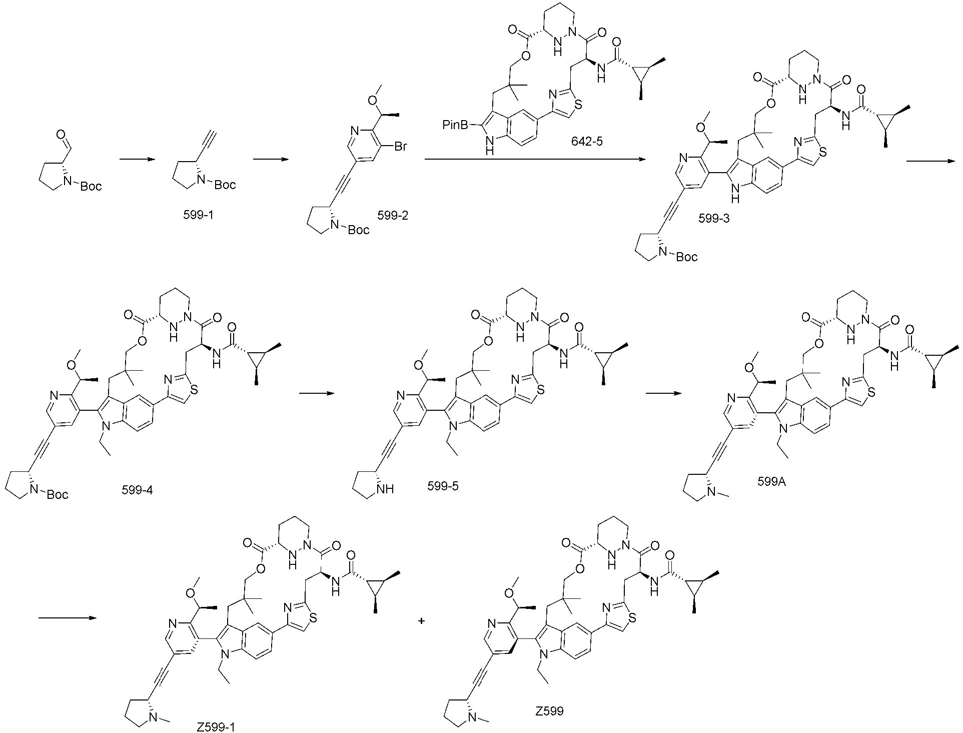

Step 1: 2-Methylpropan-2-yl (2R)-2-formyltetrahydropyrrole-1-carboxylate (1 g, 5.019 mmol) was added to a solution of a mixture of dimethyl (1-diazo-2-oxopropyl)phosphonate (1.16 g, 6.023 mmol) and potassium carbonate (1.39 g, 10.038 mmol) in methanol (20 mL). The mixture was stirred at room temperature for 3 h. The reaction mixture was dissolved in 100 mL of ethyl acetate, and the mixture was washed sequentially with 20 mL of water and 20 mL of saturated brine. The organic phase was dried over anhydrous sodium sulfate and concentrated, and the residue was purified by a flash silica gel column (0-50% ethyl acetate/petroleum ether) to give compound 599-1 (750 mg) as a colorless oil. ES-API: [M+H-56]⁺ = 140.2.

Step 2: A solution of a mixture of 3-bromo-5-iodo-2-[(1S)-1-methoxyethyl]pyridine (500 mg, 1.46 mmol), compound 599-1 (300 mg, 1.54 mmol), [1,1'-bis(diphenylphosphino)ferrocene]palladium(II) dichloride (51 mg, 0.07 mmol), copper(I) iodide (28 mg, 0.15 mmol), and N,N-diisopropylethylamine (378 mg, 2.92 mmol) in acetonitrile (10 mL) was stirred at room temperature for 3 h under a nitrogen atmosphere. The reaction mixture was dissolved in ethyl acetate (50 mL), and the mixture was washed sequentially with water (20 mL) and saturated brine (20 mL). The organic phase was dried over anhydrous sodium sulfate and concentrated, and the residue was purified by a flash silica gel column (0-50% ethyl acetate/petroleum ether) to give compound 599-2 (560 mg) as a colorless oil. ES-API: [M+H]⁺ = 409.1, 411.1.

Step 3: A mixture of compound 642-5 (250 mg, 0.36 mmol), compound 599-2 (148 mg, 0.36 mmol), [1,1'-bis(di-tert-butylphosphino)ferrocene]dichloropalladium(II) (23 mg, 0.04 mmol), and potassium phosphate (231 mg, 1.09 mmol) was stirred at 70 °C for 2 h in dioxane (6 mL), toluene (2 mL), and water (2 mL) under a nitrogen atmosphere. The reaction mixture was dissolved in ethyl acetate (20 mL), and the mixture was washed with saturated brine (5 mL). The organic phase was dried over anhydrous sodium sulfate, filtered, and concentrated, and the residue was purified by a flash silica gel column (0-80% ethyl acetate/petroleum ether) to give compound 599-3 (250 mg) as a yellow solid. ES-API: [M+H]⁺ = 892.3.

Step 4: Compound 599-3 (250 mg, 0.28 mmol) was dissolved in N,N-dimethylformamide (2 mL), and cesium carbonate (457 mg, 1.40 mmol) and iodoethane (437 mg, 2.80 mmol) were added. The mixture was stirred at room temperature for 2 h. The reaction mixture was dissolved in ethyl acetate (20 mL), and the mixture was washed sequentially with saturated brine (20 mL) and water (20 mL), dried over anhydrous sodium sulfate, filtered, and concentrated. The residue was purified by a flash silica gel column (0-10% methanol/petroleum ether) to give compound 599-4 (180 mg) as a yellow solid. ES-API: [M+H]⁺ = 920.4.

Step 5: Compound 599-4 (180 mg, 0.20 mmol) was dissolved in methanol (0.5 mL), and a 4 M solution of hydrochloric acid in dioxane (2 mL, 8 mmol) was added. The mixture was stirred at room temperature for 1 h. The reaction mixture was concentrated, and the residue was redissolved in dichloromethane (5 mL). The mixture was neutralized with saturated sodium bicarbonate, extracted, and concentrated to give compound 599-5 (160 mg). ES-API: [M+H]⁺ = 820.3.

Step 6: Compound 595-5 (160 mg, 0.20 mmol) was dissolved in methanol (1 mL), and a 37% aqueous formaldehyde solution (0.1 mL, 1.34 mmol) and acetic acid (0.01 mL, 0.20 mmol) were sequentially added. The mixture was stirred at room temperature for 1 h. Sodium cyanoborohydride (25 mg, 0.39 mmol) was then added, and the mixture was stirred for another 0.5 h. After the reaction was completed, the reaction was quenched with a saturated sodium bicarbonate solution (5 mL), and the mixture was extracted with dichloromethane (5 mL) and concentrated to give crude compound 599A. The crude product was purified by prep-HPLC (ammonium bicarbonate method) to give 2 isomeric compounds. The structure of one of the isomeric compounds was arbitrarily designated as (1r,2S,3R)-*N*-((6³S,4S,Z)-(*Sₐ*)-1²-(5-(((R)-1-methylpyrrolidin-2-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z599-1, 14.72 mg, yield: 9%, retention time: 2.22 min). ES-API: [M+H]⁺ = 834.3.

The structure of the other isomeric compound was arbitrarily designated as (1r,2S,3R)-*N*-((6³*S*,4*S*,*Z*)-(*Rₐ*)-1²-(5-(((R)-1-methylpyrrolidin-2-yl)ethynyl)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z599, 10.84 mg, yield: 7%, retention time: 2.26 min). ES-API: [M+H]⁺ = 834.3.

### Example 79. Synthesis of Compounds Z700 and Z700-1

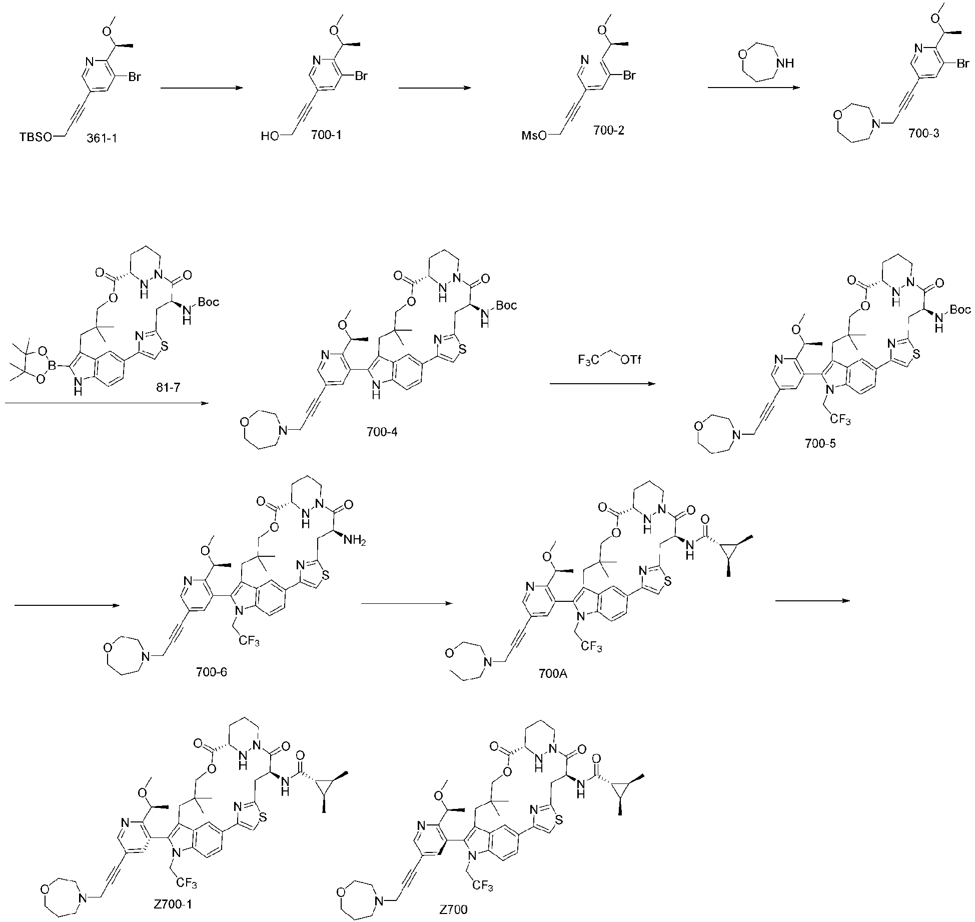

Step 1: Compound 361-1 (700 mg, 1.821 mmol) was added to tetrabutylammonium fluoride (3 mL, a 1 M solution in tetrahydrofuran). The mixture was stirred at room temperature for 2 h. Ethyl acetate (30 mL) was added, and the mixture was washed sequentially with water (20 mL × 3) and saturated brine (20 mL × 1), dried over anhydrous sodium sulfate, filtered, and concentrated. The residue was purified by silica gel column chromatography (eluent: tetrahydrofuran/petroleum ether = 1/2) to give compound 700-1 (430 mg, yield: 87.41 %) as a yellow oil. ES-API: [M+H]⁺ = 384.1.

Step 2: Compound 700-1 (430 mg, 1.592 mmol) was added to dichloromethane (10 mL), and N,N-diisopropylethylenediamine (0.606 mL, 3.665 mmol) and methanesulfonic anhydride (319.19 mg, 1.832 mmol) were added at 0 °C. The mixture was stirred at room temperature for 2 h to give a reaction mixture of compound 700-2. The reaction mixture was used in the next step without post-treatment. ES-API: [M+H]⁺ = 270.0.

Step 3: 1,4-Oxazepane (247.14 mg, 2.443 mmol) was added directly to the reaction mixture of compound 700-2 described above, and the mixture was stirred at room temperature for 18 h. Dichloromethane (10 mL) was added, and the mixture was washed sequentially with water (20 mL × 1) and saturated brine (20 mL × 1), dried over anhydrous sodium sulfate, filtered, and concentrated. The residue was purified by silica gel column chromatography (eluent: tetrahydrofuran/petroleum ether = 2/1) to give compound 700-3 (345.25 mg, yield: 80%). ES-API: [M+H]⁺ = 348.0.

Step 4: Compound 81-7 (200 mg, 0.288 mmol), compound 700-3 (122 mg, 0.346 mmol), [1,1'-bis(di-tert-butylphosphino)ferrocene]dichloropalladium(II) (27.68 mg, 0.043 mmol), and potassium carbonate (88.88 mg, 0.643 mmol) were dissolved in a mixed solution of toluene (3 mL), water (1 mL), and 1,4-dioxane (1 mL), and the mixture was stirred at 70 °C for 1 h under a nitrogen atmosphere. The mixture was diluted with ethyl acetate (15 mL) and washed with water (10 mL) and brine (10 mL). The organic layer was dried over anhydrous sodium sulfate, filtered, and concentrated to give a residue. The residue was purified by a flash column (petroleum ether/ethyl acetate = 1:3) to give compound 700-4 (200 mg, yield: 97%). ES-API [M+H⁺] = 840.4.

Step 5: Compound 700-4 (235 mg, 0.280 mmol) was dissolved in anhydrous N,N-dimethylformamide (3 mL), and 2,2,2-trifluoroethyl trifluoromethanesulfonate (649.88 mg, 2.800 mmol) and cesium carbonate (456.15 mg, 1.400 mmol) were added. The mixture was stirred at room temperature for 2 h. Ethyl acetate (30 mL) was added, and the mixture was washed once with water (20 mL) and saturated brine (20 mL), dried over anhydrous sodium sulfate, filtered, and concentrated. The residue was purified by silica gel column chromatography (eluent: tetrahydrofuran/petroleum ether = 2/1) to give compound 700-5 (210 mg, yield: 81.34%). ES-API: [M+H]⁺ = 922.3.

Step 6: Compound 700-5 (200 mg, 0.217 mmol) was dissolved in dry dichloromethane (3 mL), and trifluoroacetic acid (2 mL) was slowly added under an ice-water bath. The mixture was slowly warmed to room temperature and stirred for 1 h. After the reaction was completed, the mixture was concentrated to give compound 700-6 (178.28 mg, crude product). ES-API: [M+H]⁺ = 822.3.

Step 7: Compound 700-6 (178.28 mg, crude product) was dissolved in dichloromethane (5 mL), and triethylamine (30.11 mg, 0.298 mmol), (2R,3S)-2,3-dimethylcyclopropane-1-carboxylic acid (49.4 mg, 0.433 mmol), and a solution of 1-propylphosphonic anhydride in ethyl acetate (0.5 mL, 50%) were sequentially added under an ice-water bath. The reaction was completed after 10 min, dichloromethane (10 mL) was added, and the mixture was washed sequentially with an aqueous sodium bicarbonate solution (10 mL × 1) and saturated brine (10 mL × 1), dried over anhydrous sodium sulfate, filtered, and concentrated to give crude compound 700A. The crude product was purified by prep-HPLC (ammonium bicarbonate method) to give 2 isomeric compounds. The structure of one of the isomeric compounds was arbitrarily designated as *N*-((6³S,4S,Z)-1¹-(2,2,2-trifluoroethyl)-(*Sₐ*)-1²-(2-((S)-1-methoxyethyl)-(5-(3-(1,4-oxazepan-4-yl)prop-1-yn-1-yl))-pyridin-3-yl)-10,10-dimethyl-5,7-dioxo-6¹,6²,6³,6⁴,6⁵, 6⁶-hexahydro-11H-8-oxa-2(4,2)-thiazola- 1(5,3)-indola-6(1,3)-pyridazinacycloundecaphane-4-yl)-2,3-dimethylcyclopropane-1-carboxamide (Z700-1, 50 mg, yield: 25.11%, retention time: 2.07 min). ¹H NMR (400 MHz, DMSO-d₆) δ 8.82 (d, J = 2.1 Hz, 1H), 8.55 (d, J = 1.7 Hz, 1H), 8.41 (d, J = 9.0 Hz, 1H), 7.95 (d, J = 2.2 Hz, 1H), 7.88 (s, 1H), 7.83 (dd, J = 8.7, 1.6 Hz, 1H), 7.70 (d, J = 8.6 Hz, 1H), 5.53 (t, J = 9.2 Hz, 1H), 5.05 (d, J = 12.2 Hz, 1H), 4.88 (dp, J = 25.8, 8.5 Hz, 2H), 4.23 (td, J = 12.1, 3.1 Hz, 2H), 3.90 (q, J = 6.2 Hz, 1H), 3.72 - 3.62 (m, 7H), 3.52 (d, J = 11.0 Hz, 1H), 3.35 (s, 1H), 3.16 (dd, J = 14.7, 9.2 Hz, 1H), 3.09 (s, 4H), 2.81 - 2.72 (m, 5H), 2.44 (d, J = 14.3 Hz, 1H), 2.12 (d, J = 12.1 Hz, 1H), 1.84 (td, J = 14.1, 13.0, 7.1 Hz, 4H), 1.54 (q, J = 10.2, 8.3 Hz, 1H), 1.29 - 1.16 (m, 6H), 1.09 (dd, J = 11.8, 5.7 Hz, 6H), 0.94 (s, 3H), 0.47 (s, 3H).

The structure of the other isomeric compound was arbitrarily designated as *N*-((6³S,4S,Z)-1¹-(2,2,2-trifluoroethyl)-(*Rₐ*)-1²-(2-((S)-1-methoxyethyl)-(5-(3-(1,4-oxazepan-4-yl)prop-1-yn-1-yl))-pyridin-3-yl)-10,10-dimethyl-5,7-dioxo-6¹,6^{2,}6³,6⁴,6⁵,6⁶-hexahydro-11*H*-8-oxa-2(4,2)-thiazola-1(5,3)-indola-6(1,3)-pyridazinacycloundecaphane-4-yl)-2,3-dimethylcyclopropane-1-carboxamide (Z700, 20 mg, yield: 10.4%, retention time: 2.12 min). ES-API: [M+H]⁺ = 918.3. ¹H NMR (400 MHz, DMSO-d₆) δ 8.84 (d, *J =* 2.1 Hz, 1H), 8.52 (d, *J =* 1.5 Hz, 1H), 8.41 (d, *J* = 9.0 Hz, 1H), 7.89 (s, 1H), 7.85 (d, *J* = 8.1 Hz, 1H), 7.83 - 7.75 (m, 2H), 5.66-5.51 (m, 2H), 5.06 (d, *J =* 12.2 Hz, 1H), 4.92-4.78 (m, 1H), 4.28 - 4.18 (m, 3H), 3.72 - 3.67 (m, 4H), 3.66 - 3.63 (m, 2H), 3.57 (d, *J =* 12.6 Hz, 2H), 3.38-3.32 (m, 2H), 3.30 (s, 3H), 3.19-3.13 (m, 1H), 3.04 (d, *J =* 14.4 Hz, 1H), 2.80-2.72 (m, 5H), 2.39 (d, *J =* 14.4 Hz, 1H), 2.11 (d, *J =* 12.1 Hz, 1H), 1.89-1.77 (m, 4H), 1.53 (d, *J =* 9.1 Hz, 1H), 1.37 (d, *J =* 6.0 Hz, 3H), 1.20-1.16 (m, 2H), 1.11-1.07(m, 6H), 0.95 (s, 3H), 0.31 (s, 3H).

### Example 80. Synthesis of Compounds Z501 and Z501-1

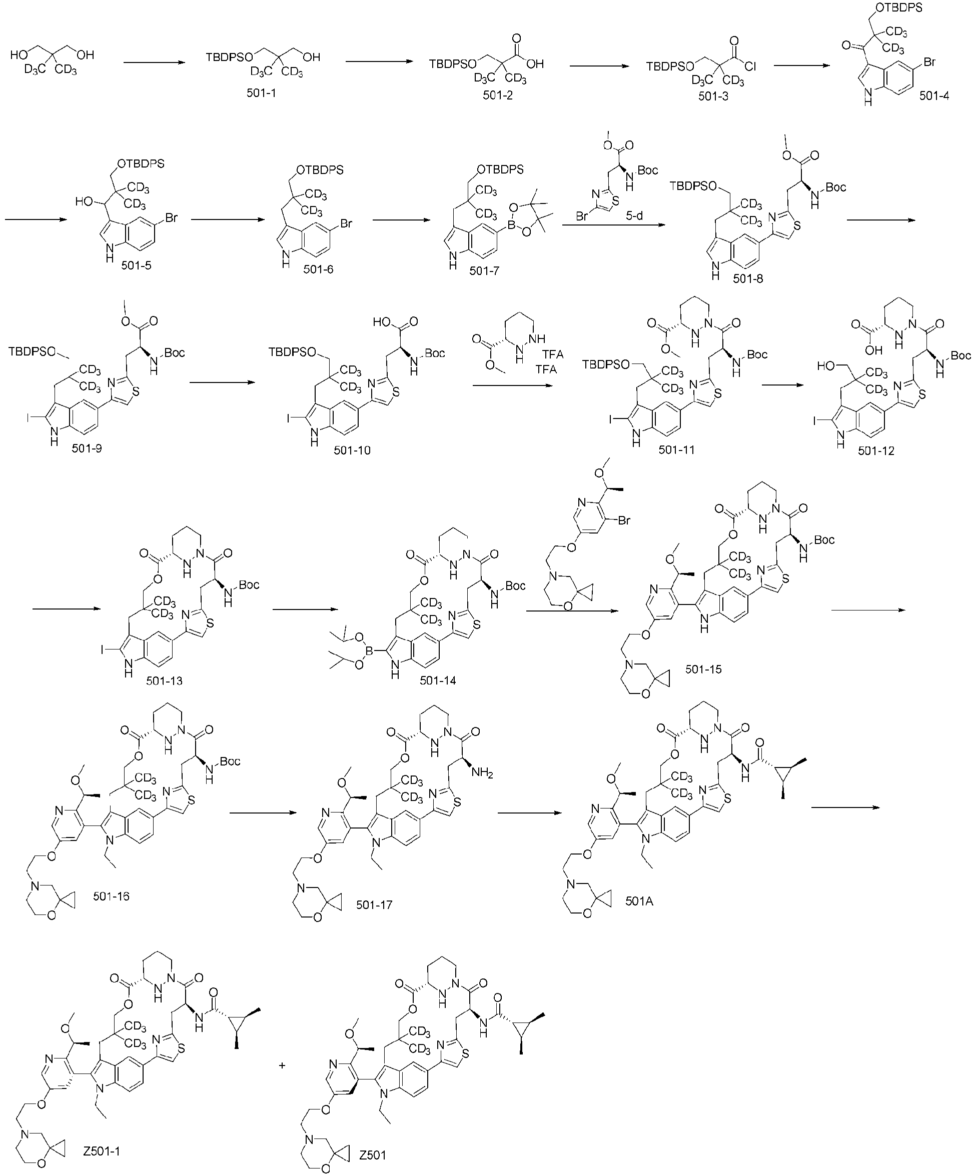

Step 1: 2,2-Bis(trideuteriomethyl)propane-1,3-diol (4 g, 36.301 mmol) was dissolved in dichloromethane (40 mL) and tetrahydrofuran (10 mL) under an ice-water bath, and imidazole (4.94 g, 72.602 mmol) and tert-butyldiphenylchlorosilane (8.98 g, 32.671 mmol) were added. The mixture was reacted at room temperature for 18 h under a nitrogen atmosphere. After the reaction was completed, the reaction mixture was concentrated, and the residue was purified by silica gel column chromatography (petroleum ether/ethyl acetate = 1/50) to give compound 501-1 (9 g, 25.818 mmol, yield: 71.12%). ES-API: [M+H-Ph]⁺ = 271.

Step 2: Sulfuric acid (2.752 mL, 51.637 mmol) was added to a solution of chromium trioxide (3.87 g, 38.727 mmol) in water (8.747 mL, 485.384 mmol) at 0 °C, and the mixture was stirred at 0 °C for 30 min. The mixture was then added to a solution of compound 501-1 (9 g, 25.818 mmol) in acetone (100 mL), and the resulting mixture was stirred at room temperature overnight. Ethyl acetate (100 mL) was added. The mixture was filtered, dried over anhydrous sodium sulfate, and concentrated to give a residue. The residue was purified by a flash silica gel column (0-10% tetrahydrofuran/petroleum ether) to give compound 501-2 (7.7 g, 21.237 mmol, yield: 82.26%) as a white solid. ES-API: [M+H-Ph]⁺ = 285.3.

Step 3: Compound 501-2 (5.62 g, 15.500 mmol) was dissolved in dichloromethane (50 mL), and oxalyl chloride (1.967 mL, 23.251 mmol) and N,N-dimethylformamide (0.11 g, 1.550 mmol) were added at 0 °C. The mixture was stirred at room temperature for 3 h. The reaction mixture was concentrated to give compound 501-3 (5.91 g, 15.500 mmol, yield: 100%). ES-API: [M+H-Ph+Me]⁺ = 299.2.

Step 4: Compound 501-3 (5.91 g, 15.511 mmol) was dissolved in dichloromethane (60 mL). Tin tetrachloride (1.815 mL, 15.511 mmol) was slowly added at 0 °C under a nitrogen atmosphere, and the mixture was stirred at 0 °C for 0.5 h. 5-Bromo-1H-indole (3.35 g, 17.062 mmol) was then added dropwise to the mixture, and the resulting mixture was stirred at 0 °C for another 45 min. The mixture was poured into a 1 M aqueous sodium hydroxide solution (50 mL) and filtered through celite. The aqueous layer was extracted with dichloromethane (100 mL × 3). The organic layers were combined and concentrated, and the residue was purified by a flash silica gel column (petroleum ether:ethyl acetate = 3:1) to give compound 501-4 (5.1 g, yield: 60.82%) as a white solid. ES-API: [M+H]⁺ = 540.2/542.2.

Step 5: Compound 501-4 (6 g, 11.099 mmol) was dissolved in tetrahydrofuran (30 mL) and ethanol (30 mL), and sodium borohydride (2.10 g, 55.493 mmol) was added in portions. The mixture was stirred at room temperature for 2 h. The mixture was cooled to 0 °C, and a saturated aqueous ammonium chloride solution (10 mL) was slowly added to quench the reaction. The mixture was extracted with ethyl acetate (100 mL × 3) and concentrated to give compound 501-5 (7 g, 12.900 mmol, yield: 116.23%). ES-API: [M+H-18]⁺ = 524,526.

Step 6: p-Toluenesulfonic acid monohydrate (0.19 g, 1.014 mmol) and dimethyl diethyl 1,4-dihydro-2,6-3,5-pyridinedicarboxylate (4.48 g, 17.692 mmol) were added to a solution of compound 501-5 (5.5 g, 10.136 mmol) in tetrahydrofuran (40 mL). The mixture was stirred at room temperature for 18 h. The mixture was poured into water (100 mL) and extracted with ethyl acetate (100 mL × 3). The ethyl acetate layers were combined, washed with a saturated sodium chloride solution (100 mL × 1), dried over anhydrous sodium sulfate, filtered, and concentrated. The residue was purified by silica gel column chromatography (petroleum ether/dichloromethane = 7/3) to give compound 501-6 (4.3 g, 8.165 mmol, yield: 80.56%). ES-API: [M+H-18]⁺ = 526,528.

Step 7: Compound 501-6 (4.2 g, 7.975 mmol), bis(pinacolato)diboron (4.05 g, 15.951 mmol), [1,1'-bis(diphenylphosphino)ferrocene]palladium(II) dichloride (0.88 g, 1.196 mmol), and potassium acetate (3.13 g, 31.902 mmol) were added to toluene (50 mL). The mixture was stirred at 100 °C for 18 h under a nitrogen atmosphere. The mixture was diluted with ethyl acetate (100 mL), washed with water (100 mL) and saturated brine (100 mL), and concentrated. The residue was purified by a flash silica gel column (0-20% tetrahydrofuran/petroleum ether) to give compound 501-7 (4 g, 6.972 mmol, yield: 87.42%) as a yellow oil. ES-API: [M+H]⁺ = 574.4.

Step 8: Compound 501-7 (4 g, 6.972 mmol), compound 5-d (3.31 g, 9.064 mmol), [1,1'-bis(diphenylphosphino)ferrocene]palladium(II) dichloride (0.45 g, 0.697 mmol), and potassium phosphate (6.19 g, 29.197 mmol) were added to a mixed solvent of toluene (3 mL), water (3 mL), and 1,4-dioxane (10 mL). The mixture was stirred at 90 °C for 2 h under an argon atmosphere. The mixture was concentrated, and the residue was extracted with ethyl acetate (100 mL). The mixture was washed with water (100 mL × 3) and saturated brine (100 mL × 1) and dried over anhydrous sodium sulfate. The mixture was filtered, and the filtrate was concentrated under reduced pressure. The residue was purified by silica gel column chromatography (petroleum ether/ethyl acetate, 1:1) to give compound 501-8 (3 g, 4.098 mmol, yield: 58.77%), ES-API: [M+H] ⁺ = 732.4.

Step 9: Sodium bicarbonate (0.38 g, 4.508 mmol) and silver trifluoromethanesulfonate (1.16 g, 4.508 mmol) were added to a solution of compound 501-8 (3 g, 4.098 mmol) in tetrahydrofuran (40 mL). Then iodine (0.94 g, 3.688 mmol) was added at 0 °C. The mixture was stirred at this temperature for 0.5 h. The mixture was quenched with an aqueous sodium sulfite solution, and the resulting mixture was extracted with ethyl acetate (100 mL × 3). The mixture was washed with water (100 mL × 1) and saturated brine (100 mL × 1) and dried over anhydrous sodium sulfate. The mixture was filtered, and the filtrate was concentrated under reduced pressure. The residue was purified by silica gel column chromatography (petroleum ether/ethyl acetate, 1:1) to give compound 501-9 (3 g, 3.497 mmol, yield: 85.33%). ES-API: [M+H]⁺ = 858.4.

Step 10: Lithium hydroxide (0.44 g, 10.490 mmol) was added to a solution of compound 501-9 (3 g, 3.497 mmol) in tetrahydrofuran (30 mL) and water (10 mL) at 0 °C, and the mixture was stirred at room temperature for 2 h. The mixture was acidified to pH 6 with 1 M hydrochloric acid, extracted with ethyl acetate (100 mL × 3), and concentrated to give compound 501-10 (2.94 g, 3.484 mmol, yield: 99.63%) as a yellow solid. ES-API: [M+H]⁺ = 844.4.

Step 11: N-Methylmorpholine (2.83 g, 27.964 mmol) and methyl (R)-hexahydropyridazine-3-carboxylate trifluoroacetate (2.60 g, 6.991 mmol) were added to a solution of compound 501-10 (2.94 g, 3.484 mmol) in dichloromethane (30 mL), and the mixture was cooled to 0 °C. 1-Propylphosphonic anhydride (4.45 g, 6.991 mmol, a 50% solution in ethyl acetate) was slowly added. The mixture was stirred at 25 °C for 2 h. The mixture was concentrated, and the residue was poured into water (100 mL). The mixture was extracted with ethyl acetate (100 mL × 3). The ethyl acetate layers were combined, washed with a saturated sodium chloride solution (100 mL × 1), dried over anhydrous sodium sulfate, filtered, and concentrated. The residue was purified by silica gel column chromatography (petroleum ether/ethyl acetate = 50/50) to give compound 501-11 (2.72 g, yield: 80.21%). ES-API: [M+H]⁺ = 970.2.

Step 12: Tetrabutylammonium fluoride (27 mL, 27.000 mmol, a 1 M solution in tetrahydrofuran) was added to compound 501-11 (2.7 g, 1.78 mmol), and the mixture was stirred at 60 °C for 18 h. The mixture was then concentrated, and the residue was redissolved with ethyl acetate (30 mL) and washed 3 times with a 1 M aqueous hydrochloric acid solution. The organic layer was concentrated, and the residue was purified by a flash column (80% tetrahydrofuran/petroleum ether) to give compound 501-12 (1.73 g, 2.4 mmol, yield: 85.11%). ES-API: [M+H]⁺ = 718.2.

Step 13: N,N-Diisopropylethylenediamine (16.797 mL, 96.426 mmol), 1-hydroxybenzotriazole (3.26 g, 24.106 mmol), and 1-(3-dimethylaminopropyl)-3-ethylcarbodiimide hydrochloride (13.86 g, 72.319 mmol) were added to a solution of compound 501-12 (1.73 g, 2.4 mmol) in dichloromethane (170 mL). The mixture was stirred at room temperature overnight. The mixture was eluted with a 1 M aqueous hydrochloric acid solution (100 mL). The organic phase was concentrated, and the residue was purified by a flash silica gel column (0-80% ethyl acetate/petroleum ether) to give compound 501-13 (750 mg, 1.072 mmol, yield: 44.47%). ES-API [M+H⁺] = 700.2.

Step 14: Compound 501-13 (400 mg, 0.572 mmol), tris(dibenzylideneacetone)dipalladium(0) (62.82 mg, 0.069 mmol), 2-dicyclohexylphosphino-2',6'-dimethoxy-1,1'-biphenyl (70.41 mg, 0.172 mmol), and potassium acetate (196.38 mg, 2.001 mmol) were added to dry toluene (10 mL). The mixture was degassed with nitrogen and cooled to 0 °C, and 4,4,5,5-tetramethyl-1,3,2-dioxaborolane (0.622 mL, 4.288 mmol) was slowly added. After the addition was completed, the mixture was stirred at 60 °C for 3 h. The reaction was quenched with saturated ammonium chloride (aqueous solution) at 0 °C. The mixture was extracted with ethyl acetate (30 mL × 3). The organic layers were combined, washed with water (30 mL × 3), and dried over anhydrous sodium sulfate. The mixture was filtered, and the filtrate was concentrated under reduced pressure. The residue was purified by silica gel column chromatography (elution with petroleum ether/ethyl acetate, 3:1) to give compound 501-14 (340 mg, 0.486 mmol, yield: 85%). ES-API [M+H⁺] = 700.4.

Step 15: Compound 501-14 (150 mg, 0.214 mmol), (R)-7-(2-((5-bromo-6-(1-methoxyethyl)pyridin-3-yl)oxy)ethyl)-4-oxa-7-azaspiro[2.5]octane (159.18 mg, 0.429 mmol), [1,1'-bis(di-tert-butylphosphino)ferrocene]dichloropalladium(II) (27.68 mg, 0.043 mmol), and potassium carbonate (88.88 mg, 0.643 mmol) were dissolved in a mixed solution of toluene (3 mL), water (1 mL), and 1,4-dioxane (1 mL). The mixture was stirred at 70 °C for 1 h under a nitrogen atmosphere. The mixture was diluted with ethyl acetate (15 mL) and washed with water and brine (10 mL). The organic layer was dried over anhydrous sodium sulfate, filtered, and concentrated to give a residue. The residue was purified by a flash silica gel column (petroleum ether/ethyl acetate = 1:3) to give compound 501-15 (130 mg, 0.150 mmol, yield: 70.18%). ES-API [M+H⁺] = 864.4.

Step 16: Cesium carbonate (301.65 mg, 0.926 mmol) and iodoethane (252.70 mg, 1.620 mmol) were added to a solution of compound 501-15 (160 mg, 0.185 mmol) in N,N-dimethylformamide (5 mL). The mixture was stirred at 25 °C for 2 h. The mixture was diluted with ethyl acetate (15 mL) and washed with water (10 mL) and brine (10 mL). The organic layer was dried over anhydrous sodium sulfate, filtered, and concentrated to give a residue. The residue was purified by a flash silica gel column (petroleum ether/ethyl acetate = 1:3) to give compound 501-16 (130 mg, 0.150 mmol, yield: 70.18%). ES-API [M+H⁺] = 892.4.

Step 17: Compound 501-16 (120 mg, 0.135 mmol) was dissolved in dichloromethane (2 mL), and trifluoroacetic acid (3 mL) was added. The mixture was reacted at room temperature for 1 h. The reaction mixture was concentrated to give compound 501-17 (106 mg, crude product), which was directly used in the next step without purification. ES-API [M+H⁺] = 792.4.

Step 18: Compound 501-17 (106 mg, crude product) was dissolved in dichloromethane (5 mL), and N,N-diisopropylethylamine (86.49 mg, 0.669 mmol), (1r,2R,3S)-2,3-dimethylcyclopropane-1-carboxylic acid (22.91 mg, 0.201 mmol), and O-(7-azabenzotriazol-1-yl)-N,N,N',N'-tetramethyluronium hexafluorophosphate (101.78 mg, 0.268 mmol) were sequentially added. The mixture was reacted at room temperature for 1 h. The mixture was extracted with dichloromethane (100 mL × 3), washed with a saturated sodium chloride solution (100 mL × 1), dried over anhydrous sodium sulfate, filtered, and concentrated to give crude compound 501A. The crude product was purified by prep-HPLC (ammonia water method) to give 2 isomeric compounds. One of the isomeric compounds was arbitrarily designated as (1r,2R,3S)-N-((6³S,4S,Z)-1¹-ethyl-(*Sₐ*)-1²-(5-(2-(4-oxa-7-azaspiro[2.5]octan-7-yl)ethoxy)-2-((S)-1-methoxyethyl)pyridin-3-yl)-10,10-bis(methyl-d3)-5,7-dioxo-6¹,6²,6³,6⁴,6⁵,6⁶-hexahydro-11*H*-8-oxo-2(4,2)-thiazola-1(5,3)-indola-6(1,3)-pyridazinacycloundecaphane-4-yl)-2,3-dimethylcyclopropane-1-carboxamide (Z501-1, 26 mg, yield: 21.87%, retention time: 4.303 min). ¹H NMR (400 MHz, DMSO-d₆) δ 8.53 (d, J = 1.6 Hz, 1H), 8.48 (d, J = 2.9 Hz, 1H), 8.40 (d, J = 9.0 Hz, 1H), 7.81 (s, 1H), 7.73 (dd, J = 8.7, 1.6 Hz, 1H), 7.56 - 7.51 (m, 2H), 5.55 (t, J = 9.2 Hz, 1H), 5.04 (d, J = 12.1 Hz, 1H), 4.31 - 4.14 (m, 4H), 3.98 - 3.83 (m, 3H), 3.71 - 3.53 (m, 4H), 3.16 (dd, J = 14.7, 9.3 Hz, 1H), 3.08 (s, 3H), 3.03 (d, J = 14.4 Hz, 1H), 2.82 - 2.69 (m, 3H), 2.54 (d, J = 5.9 Hz, 2H), 2.42 (s, 3H), 2.12 (d, J = 12.1 Hz, 1H), 1.81 (s, 2H), 1.52 (t, J = 8.9 Hz, 1H), 1.34 - 1.00 (m, 16H), 0.69 - 0.59 (m, 2H), 0.50 - 0.40 (m, 2H).

The structure of the other isomeric compound was arbitrarily designated as (1r,2R,3S)-N-((6³S,4S,Z)-1¹-ethyl-(*Rₐ*)-1²-(5-(2-(4-oxa-7-azaspiro[2.5]octan-7-yl)ethoxy)-2-((S)-1-methoxyethyl)pyridin-3-yl)-10,10-bis(methyl-d3)-5,7-dioxo-6¹,6²,6³,6⁴,6⁵,6⁶-hexahydro-11*H*-8-oxo-2(4,2)-thiazola-1(5,3)-indola-6(1,3)-pyridazinacycloundecaphane-4-yl)-2,3-dimethylcyclopropane-1-carboxamide (Z501, 16.6 mg, yield: 13.95%, retention time: 7.224 min). ES-API: [M+H]⁺ = 888.5. ¹H NMR (400 MHz, DMSO-d₆) δ 8.53 (d, *J =* 1.6 Hz, 1H), 8.48 (d, *J =* 2.9 Hz, 1H), 8.40 (d, *J =* 9.0 Hz, 1H), 7.82 (s, 1H), 7.74 (dd, *J* = 8.7, 1.7 Hz, 1H), 7.57 - 7.49 (m, 2H), 5.55 (t, *J* = 9.2 Hz, 1H), 5.03 (d, *J* = 12.1 Hz, 1H), 4.29-4.15 (m, 4H), 3.99-3.83 (m, 3H), 3.72 - 3.51 (m, 4H), 3.35-3.32 (m, 2H), 3.19-3.13 (m, 1H), 3.08 (s, 3H), 3.03 (d, *J =* 14.3 Hz, 1H), 2.83 - 2.65 (m, 3H), 2.54 (m, 6H), 2.45-2.40 (m, 4H), 2.12 (d, *J =* 12.0 Hz, 1H), 1.85-1.77 (m, 2H), 1.59 - 1.45 (m, 1H), 1.25 - 1.16 (m, 6H), 1.13 - 1.05 (m, 9H), 0.67 - 0.60 (m, 2H), 0.50 - 0.42 (m, 2H).

### Example 81. Synthesis of Compound Z775

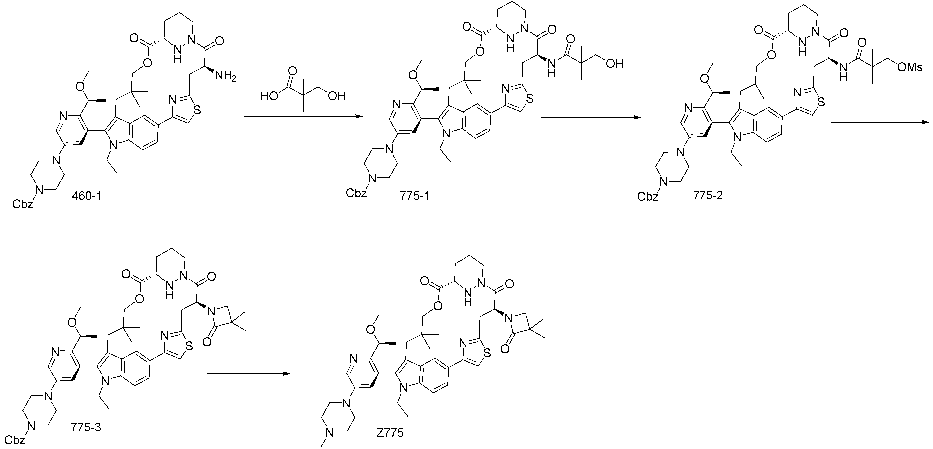

Step 1: Compound 460-1 (150 mg, 0.18 mmol) was dissolved in dichloromethane (8 mL), and 2,2-dimethyl-3-hydroxypropanoic acid (31 mg, 0.27 mmol), N,N'-diisopropylethylamine (68 mg, 0.53 mmol), and O-(7-azabenzotriazol-1-yl)-N,N,N',N'-tetramethyluronium hexafluorophosphate (101 mg, 0.27 mmol) were added. The reaction system was stirred at room temperature for 2 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was purified by a flash silica gel column (methanol/dichloromethane = 0-4%) to give compound 775-1 (150 mg, yield: 89.4%) as a white solid. ES-API: [M+H]⁺= 949.3.

Step 2: Compound 775-1 (125 mg, 0.13 mmol) was dissolved in dichloromethane (8 mL), and N,N'-diisopropylethylamine (170 mg, 1.32 mmol) and methanesulfonyl chloride (75 mg, 0.65 mmol) were added under an ice bath. The reaction system was stirred for 1 h under an ice bath. Dichloromethane (15 mL) was added to the reaction mixture. The mixture was washed sequentially with water (5 mL) and a saturated aqueous sodium bicarbonate solution (5 mL), dried over anhydrous sodium sulfate, and concentrated. The crude product was purified by a flash silica gel column (methanol/dichloromethane: 0-4%) to give compound 775-2 (135 mg, yield: 99.8%) as a pale yellow solid. ES-API: [M+H]⁺ = 1027.3.

Step 3: Compound 775-2 (115 mg, 0.11 mmol) was dissolved in acetonitrile (10 mL), and potassium carbonate (47 mg, 0.34 mmol) was added at 0 °C. The reaction system was stirred at 85 °C for 5 h. Ethyl acetate (60 mL) was added to the reaction mixture, and the mixture was washed sequentially with water (15 mL) and saturated brine (15 mL). The organic phase was dried over anhydrous sodium sulfate and concentrated. The crude product was purified by a flash silica gel column (methanol/dichloromethane: 0-5%) to give compound 775-3 (80 mg, yield: 76.7%) as a white solid. ES-API: [M+H]⁺ = 931.3. Step 4: Compound 775-3 (80 mg, 0.086 mmol) and paraformaldehyde (15 mg, 0.17 mmol) were dissolved in absolute methanol (5 mL), and 10% palladium hydroxide on carbon (100 mg) was added. The reaction system was stirred at 30 °C for 24 h under a hydrogen atmosphere. The reaction mixture was filtered through celite and washed with methanol, and the filtrate was concentrated. The crude product was subjected to prep-HPLC (ammonium bicarbonate method) to give (6³S,4S,Z)-4-(3,3-dimethyl-2-oxoazetidin-1-yl)-1¹-ethyl-(Rₐ)-1²-(2-(((S)-1-methoxyethyl)-5-(4-methylpiperazin-1-yl)pyridin-3-yl)-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z775, 23 mg, yield: 33.0%) as a white solid. ES-API: [M+H]⁺ = 811.4. ¹H NMR (400 MHz, DMSO-d6) δ 8.49 (d, J = 1.2 Hz, 1H), 8.44 (d, J = 2.8 Hz, 1H), 7.85 (s, 1H), 7.74 (dd, J = 8.4, 1.2 Hz, 1H), 7.56 (d, J = 8.4 Hz, 1H), 7.22 (d, J = 2.8 Hz, 1H), 5.44 (d, J = 9.2 Hz, 1H), 5.21 (d, J = 12.0 Hz, 1H), 4.36 - 4.07 (m, 5H), 3.66 - 3.40 (m, 5H), 3.30 - 3.24 (m, 5H), 3.21 (s, 3H), 2.93 (d, J = 14.4 Hz, 1H), 2.80 - 2.69 (m, 1H), 2.49 - 2.38 (m, 5H), 2.21 (s, 3H), 2.07 (dd, J = 12.4, 2.2 Hz, 1H), 1.86 - 1.72 (m, 2H), 1.56 - 1.43 (m, 1H), 1.38 - 1.26 (m, 6H), 1.21 (s, 3H), 0.96 - 0.79 (m, 6H), 0.36 (s, 3H).

### Example 82. Synthesis of Compound Z677

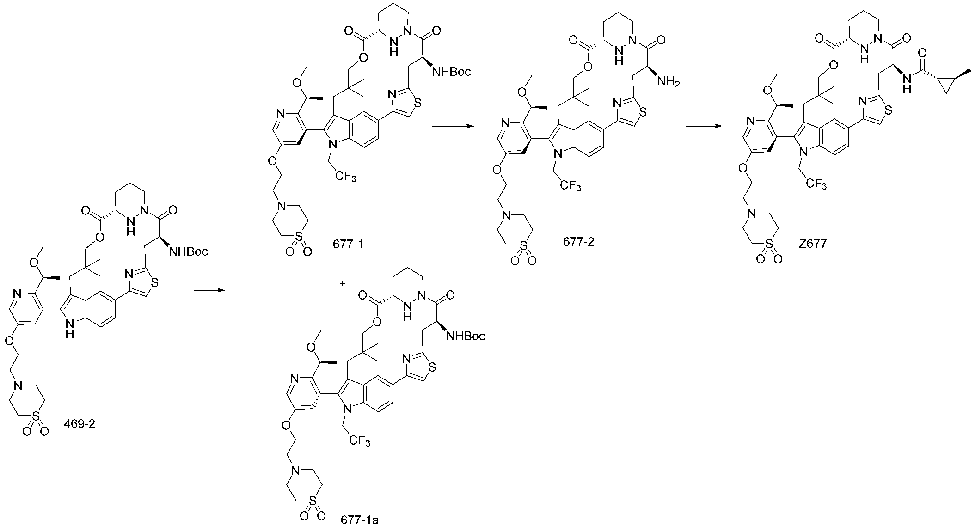

Step 1: Compound 469-2 (170 mg, 0.193 mmol) was dissolved in N,N-dimethylformamide (1 mL), and cesium carbonate (314.68 mg, 0.966 mmol) and 2,2,2-trifluoroethyl trifluoromethanesulfonate (448.33 mg, 1.932 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure, and the residue was purified by silica gel column chromatography (methanol:dichloromethane = 0-3%) to give two isomers. The structure of one of the isomeric compounds was arbitrarily designated as tert-butyl ((6³*S*,4*S*,*Z*)-(*Rₐ*)-1²-(5-(2-(1,1-dioxidothiomorpholino)ethoxy)-2-((*S*)-1-methoxyethyl)pyridin-3-yl)-10,10-dimethyl-5,7-dioxo-1¹-(2,2,2-trifluoroethyl)-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)carbamate (compound 677-1, 40 mg, 0.042 mmol, yield: 21.52%, (DCM:MeOH = 15:1, Rf = 0.43)), ES-API: [M+H]⁺ = 962.2. The structure of the other isomeric compound was arbitrarily designated as tert-butyl ((6³*S*,4*S*,*Z*)-(*Sₐ*)-1²-(5-(2-(1,1-dioxidothiomorpholino)ethoxy)-2-((*S*)-1-methoxyethyl)pyridin-3-yl)-10,10-dimethyl-5,7-dioxo-1¹-(2,2,2-trifluoroethyl)-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)carbamate (compound 677-1a, 110 mg, 0.114 mmol, 59.24%, (DCM:MeOH = 15:1, Rf = 0.40)), ES-API: [M+H]⁺ = 962.2.

Step 2: Compound 677-1 (40 mg, 0.042 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 677-2 (35 mg, 0.041 mmol, yield: 97.66%). ES-API: [M+H]⁺ = 862.2. Step 3: Compound 677-2 (40 mg, 0.046 mmol) was dissolved in dichloromethane (1 mL), and (1S,2S)-2-methylcyclopropane-1-carboxylic acid (5.53 mg, 0.055 mmol), O-(7-azabenzotriazol-1-yl)-N,N,N',N'-tetramethyluronium hexafluorophosphate (26.24 mg, 0.069 mmol), and N,N-diisopropylethylamine (29.73 mg, 0.230 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, and concentrated under reduced pressure. The residue was purified by prep-HPLC (ammonium bicarbonate method) to give (1S,2S)-*N-*((6³S,4S,Z)-(Rₐ)-1²-(5-(2-(1,1-dioxidothiomorpholino)ethoxy)-2-((*S*)-1-methoxyethyl)pyridin-3-yl)-10,10-dimethyl-5,7-dioxo-1¹-(2,2,2-trifluoroethyl)-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-methylcyclopropane-1-carboxamide (Z677, 15 mg, 0.016 mmol, yield: 34.54%). ES-API: [M+H]⁺ = 944.2. ¹H NMR (400 MHz, DMSO-d₆) δ 8.55 - 8.50 (m, 3H), 7.87- 7.81 (m, 2H), 7.77 (d, *J* = 8.8 Hz, 1H), 7.36 (d, *J =* 2.4 Hz, 1H), 5.60 - 5.52 (m, 2H), 5.07- 4.95 (m, 2H), 4.36 - 4.07 (m, 5H), 3.66 - 3.47 (m, 2H), 3.36 (s, 1H), 3.28 (s, 3H), 3.21 - 3.00 (m, 10H), 2.96 - 2.93 (m, 2H), 2.77-2.76 (m, 1H), 2.48-2.46 (m, 1H), 2.11-2.09 (m, 1H), 1.83-1.81 (s, 2H), 1.52-1.51 (m, 2H), 1.36 (d, *J =* 6.4 Hz, 3H), 1.08-1.07 (m, 4H), 0.95 (s, 3H), 0.88-0.87 (m, 1H), 0.56-0.54 (m, 1H), 0.29 (s, 3H).

### Example 83. Synthesis of Compounds Z672 and Z672-1

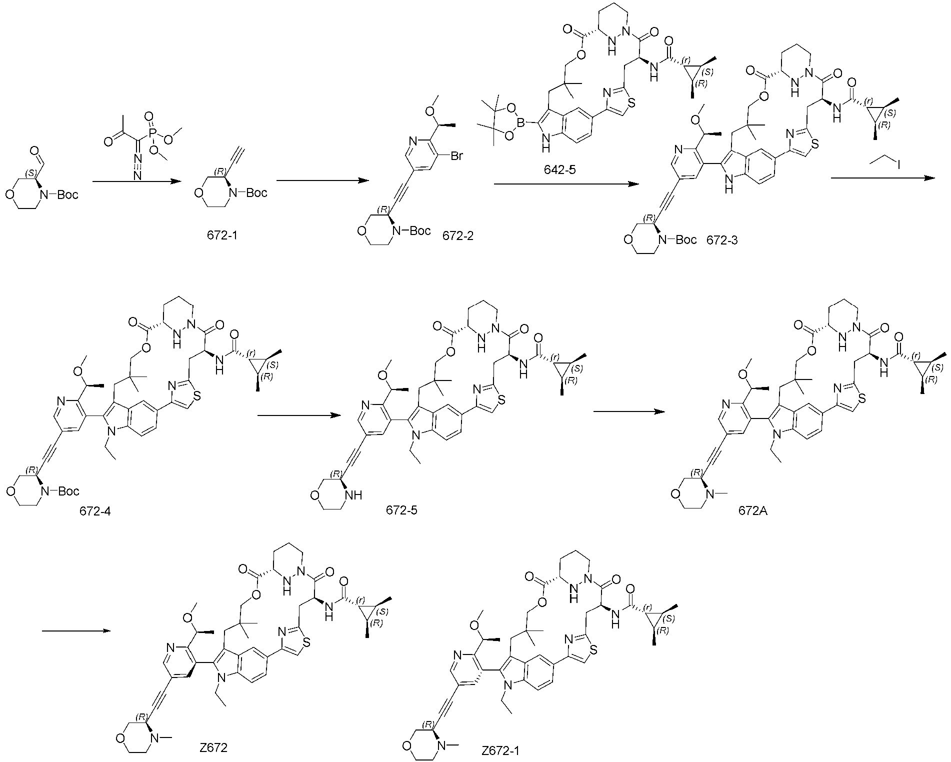

Step 1: Potassium carbonate (552.16 mg, 3.995 mmol) and dimethyl (1-diazo-2-oxopropyl)phosphonate (498.91 mg, 2.597 mmol) were added to a solution of tert-butyl (S)-3-formylmorpholine-4-carboxylate (430 mg, 1.998 mmol) in methanol (10 mL), and the mixture was stirred at room temperature for 2 h. After the reaction was completed, the mixture was extracted with ethyl acetate (20 mL × 3). The combined extract was washed with brine (30 mL), dried over anhydrous sodium sulfate, filtered, and concentrated, and the residue was purified by a flash silica gel column (0-40% petroleum ether/ethyl acetate) to give compound 672-1 (350 mg, yield: 82.9%). ES-API [M+H-56]⁺ = 156.1.

Step 2: 3-Bromo-5-iodo-2-[(1S)-1-methoxyethyl]pyridine (80.94 mg, 0.237 mmol), compound 672-1 (50 mg, 0.237 mmol), bis(triphenylphosphine)palladium(II) dichloride (8.31 mg, 0.012 mmol), copper(I) iodide (4.51 mg, 0.024 mmol), and N,N-diisopropylethylenediamine (0.082 mL, 0.473 mmol) were mixed in acetonitrile (10 mL) under a nitrogen atmosphere, and the mixture was stirred at room temperature for 3 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 672-2 (0.083 g, yield: 82%). ES-API [M+H]⁺ = 425.2.

Step 3: Compound 642-5 (130 mg, 0.188 mmol) and compound 672-2 (96.20 mg, 0.226 mmol) were in dioxane (6 mL), toluene (2 mL), and water (2 mL) under a nitrogen atmosphere, and potassium phosphate (120.03 mg, 0.565 mmol) and [1,1'-bis(di-tert-butylphosphino)ferrocene]dichloropalladium(II) (24.54 mg, 0.038 mmol) were added. The mixture was stirred at 65 °C for 16 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-80% petroleum ether/ethyl acetate) to give compound 672-3 (100 mg, yield: 58%). ES-API [M+H]⁺ = 908.3.

Step 4: Compound 672-3 (50 mg, 0.055 mmol) was dissolved in N,N-dimethylformamide (5 mL), and cesium carbonate (89.70 mg, 0.275 mmol) and iodoethane (85.87 mg, 0.551 mmol) were sequentially added. The mixture was stirred at room temperature for 6 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% petroleum ether/ethyl acetate) to give compound 672-4 (50 mg, yield: 97%) as a yellow oil. ES-API [M+H]⁺ = 936.3.

Step 5: Trifluoroacetic acid (3 mL) was added to a solution of compound 672-4 (100 mg, 0.107 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 672-5 (90 mg, yield: 99%). ES-API [M+H]⁺ = 836.3.

Step 6: Compound 672-5 (60 mg, 0.07 mmol) was dissolved in methanol (10 mL), and a 37% aqueous formaldehyde solution (0.5 mg), sodium cyanoborohydride (22.53 mg, 0.359 mmol), and 2 drops of acetic acid were sequentially added. The mixture was stirred at room temperature for 0.5 h. After the reaction was completed, ethyl acetate (20 mL) was added, and the mixture was washed with saturated brine (10 mL) and water (10 mL). The organic phase was dried over anhydrous sodium sulfate, filtered, and concentrated to give crude compound 672A. The crude product was purified by preparative thin-layer chromatography (dichloromethane/methanol = 20/1) to give 2 isomeric compounds. The structure of one of the isomeric compounds was arbitrarily designated as (1r,2R,3S)-N-((6³S,4S,Z)-1¹-ethyl-(Rₐ)-1²-(2-((S)-1-methoxyethyl)-5-(((R)-4-methylmorpholin-3-yl)ethynyl)pyridin-3-yl)-10,10-dimethyl-5,7-dioxo-6¹,6²,6³,6⁴,6⁵,6⁶hexahydro-11H-8-oxa-2(4,2)-thiazola-1(5,3)-indola-6(1,3)-pyridazinacycloundecaphane-4-yl)-2,3-dimethylcyclopropane-1-carboxamide (Z672, 8 mg, yield: 13%, purity: 98%, Rf = 0.5). ES-API [M+H]⁺ = 850.3.

The structure of the other isomeric compound was arbitrarily designated as (1r,2R,3S)-N-((6³S,4S,Z)-1¹-ethyl-(Sₐ)-1²-(2-((S)-1-methoxyethyl)-5-(((R)-4-methylmorpholin-3-yl)ethynyl)pyridin-3-yl)-10,10-dimethyl-5,7-dioxo-6¹,6²,6³,6⁴,6⁵,6⁶hexahydro-11H-8-oxa-2(4,2)-thiazola-1(5,3)-indola-6(1,3)-pyridazinacycloundecaphane-4-yl)-2,3-dimethylcyclopropane-1-carboxamide (Z672-1, 6 mg, yield: 10%, purity: 98%, Rf = 0.4). ES-API [M+H]⁺ = 850.3.

### Example 84. Synthesis of Compounds Z673 and Z673-1

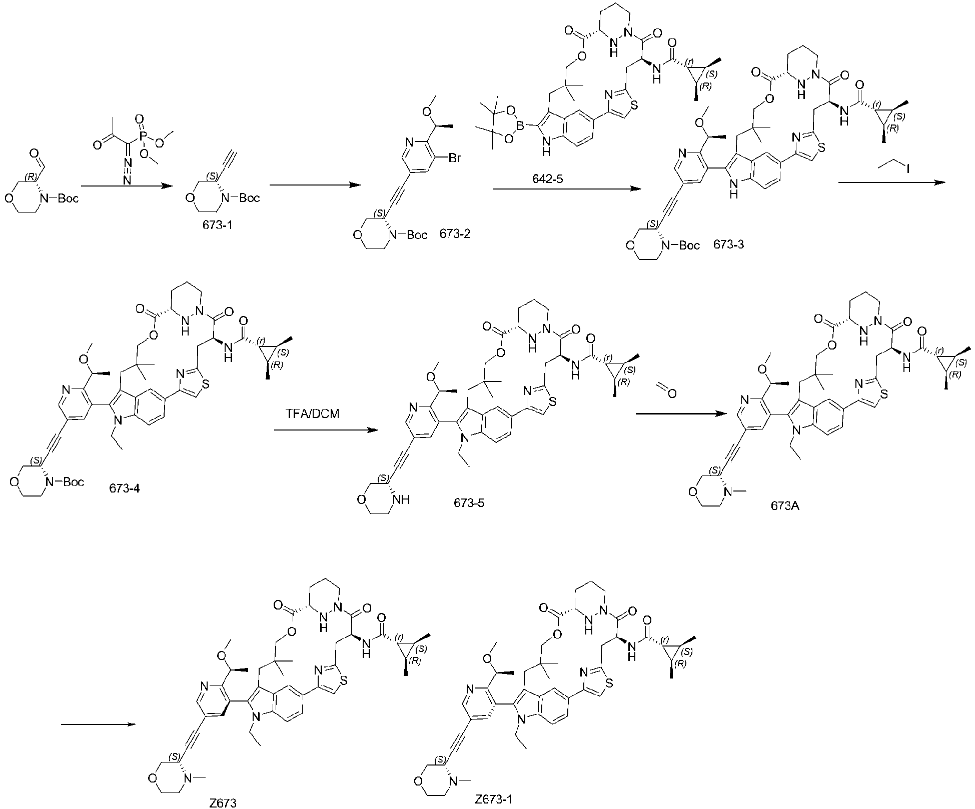

Step 1: Potassium carbonate (642.04 mg, 4.646 mmol) and dimethyl (1-diazo-2-oxopropyl)phosphonate (535.50 mg, 2.787 mmol) were added to a solution of tert-butyl (R)-3-formylmorpholine-4-carboxylate (500 mg, 2.323 mmol) in methanol (10 mL), and the mixture was stirred at room temperature for 2 h. After the reaction was completed, the mixture was extracted with ethyl acetate (20 mL × 2). The combined extract was washed with brine (30 mL), dried over anhydrous sodium sulfate, filtered, and concentrated, and the residue was subjected to flash silica gel column chromatography (0-40% petroleum ether/ethyl acetate) to give compound 673-1 (400 mg, yield: 82%). ES-API [M+H-56]⁺ = 156.1.

Step 2: 3-Bromo-5-iodo-2-[(1S)-1-methoxyethyl]pyridine (712.24 mg, 2.083 mmol), compound 673-1 (400 mg, 1.893 mmol), bis(triphenylphosphine)palladium(II) dichloride (66.45 mg, 0.095 mmol), copper(I) iodide (36.06 mg, 0.189 mmol), and N,N-diisopropylethylenediamine (0.660 mL, 3.787 mmol) were mixed in acetonitrile (15 mL) under a nitrogen atmosphere, and the mixture was stirred at room temperature for 16 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 673-2 (0.66 g, yield: 82%). ES-API [M+H]⁺ = 425.2.

Step 3: Compound 642-5 (130 mg, 0.188 mmol) and compound 673-2 (96.20 mg, 0.226 mmol) were in dioxane (6 mL), toluene (2 mL), and water (2 mL) under a nitrogen atmosphere, and potassium phosphate (120.03 mg, 0.565 mmol) and [1,1'-bis(di-tert-butylphosphino)ferrocene]dichloropalladium(II) (24.54 mg, 0.038 mmol) were added. The mixture was stirred at 65 °C for 16 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-80% petroleum ether/ethyl acetate) to give compound 673-3 (100 mg, yield: 58%). ES-API [M+H]⁺ = 908.3.

Step 4: Compound 673-3 (100 mg, 0.11 mmol) was dissolved in N,N-dimethylformamide (5 mL), and cesium carbonate (179.39 mg, 0.551 mmol) and iodoethane (171.75 mg, 1.101 mmol) were sequentially added. The mixture was stirred at room temperature for 6 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by a flash silica gel column (0-60% petroleum ether/ethyl acetate) to give compound 673-4 (100 mg, yield: 97%). ES-API [M+H]⁺ = 936.3.

Step 5: Trifluoroacetic acid (3 mL) was added to a solution of compound 673-4 (100 mg, 0.107 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 673-5 (80 mg, yield: 89%). ES-API [M+H]⁺ = 836.3.

Step 6: Compound 673-5 (80 mg, 0.096 mmol) was dissolved in methanol (10 mL), and a 37% aqueous formaldehyde solution (0.5 mg), sodium cyanoborohydride (22.53 mg, 0.359 mmol), and 2 drops of acetic acid were sequentially added. The mixture was stirred at room temperature for 0.5 h. After the reaction was completed, ethyl acetate (20 mL) was added, and the mixture was washed with saturated brine (10 mL) and water (10 mL). The organic phase was dried over anhydrous sodium sulfate, filtered, and concentrated to give crude compound 673A. The crude product was purified by preparative thin-layer chromatography (dichloromethane/methanol = 20/1) to give 2 isomeric compounds. The structure of one of the isomeric compounds was arbitrarily designated as (1r,2R,3S)-N-((6³S,4S,Z)-1¹-ethyl-(Rₐ)-1²-(2-((S)-1-methoxyethyl)-5-(((S)-4-methylmorpholin-3-yl)ethynyl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z673, 4 mg, yield: 5%, purity: 98%, Rf = 0.5). ES-API [M+H]⁺ = 850.3.

The structure of the other isomeric compound was arbitrarily designated as (1r,2R,3S)-N-((6³S,4S,Z)-1¹-ethyl-(Sₐ)-1²-(2-((S)-1-methoxyethyl)-5-(((S)-4-methylmorpholin-3-yl)ethynyl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z673-1, 6 mg, yield: 7%, purity: 98%, Rf = 0.4). ES-API [M+H]⁺ = 850.3.

### Example 85. Synthesis of Compounds Z603 and Z603-1

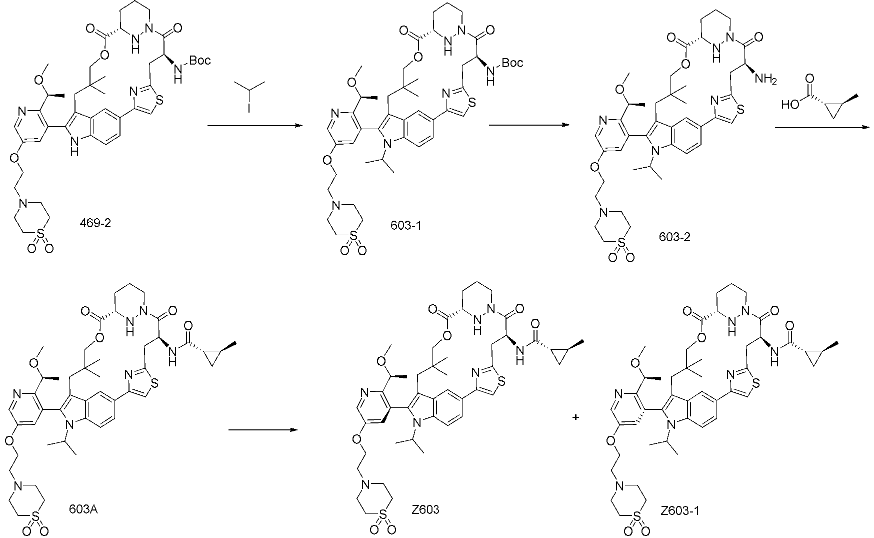

Step 1: Cesium carbonate (629 mg, 1.932 mmol) and 2-iodopropane (328 mg, 1.932 mmol) were sequentially added to a solution of compound 469-2 (170 mg, 0.193 mmol) in N,N-dimethylformamide (10 mL). The reaction mixture was stirred at room temperature for 24 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give crude compound 603-1 (200 mg). ES-API: [M+H]⁺ = 922.3.

Step 2: Compound 603-1 (200 mg, 0.217 mmol) was dissolved in a solution of hydrochloric acid in dioxane (5 mL) and a solution of hydrochloric acid in methanol (1.5 mL). The reaction mixture was stirred at room temperature for 1 h. When the reaction was completed as detected by LCMS, the mixture was concentrated under reduced pressure at a low temperature (30 °C) to remove the solvent to give crude compound 603-2 (178 mg, yield: 99%). ES-API: [M+H]⁺ = 822.3.

Step 3: (1S,2S)-2-Methylcyclopropane-1-carboxylic acid (28 mg, 0.281 mmol), N,N-diisopropylethylamine (0.19 mL, 1.083 mmol), and O-(7-azabenzotriazol-1-yl)-N,N,N',N'-tetramethyluronium hexafluorophosphate (123.5 mg, 0.325 mmol) were sequentially added to a solution of compound 603-2 (178 mg, 0.217 mmol) in N,N-dimethylformamide (5 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness to give crude compound 603A. The crude product was purified by prep-HPLC (ammonium bicarbonate method) to give 2 isomeric compounds. The structure of one of the isomeric compounds was arbitrarily designated as (1S,2S)-N-((6³S,4S,Z)-(Rₐ)-1²-(5-(2-(1,1-dioxidothiomorpholino)ethoxy)2-(((S)-1-methoxyethyl)pyridin-3-yl)-1¹-isopropyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide (Z603, 34.12 mg, yield: 17%, retention time: 1.83 min), ES-API [M+H]⁺ = 904.3. ¹H NMR (400 MHz, DMSO-d₆) δ 8.43- 8.39 (m, 3 H), 7.70 (s, 1 H), 7.65- 7.58 (m, 2 H), 7.20 (d, J= 2.8 Hz, 1 H), 5.53 (t, J= 9.2 Hz, 1 H), 5.03 (d, J= 12.0 Hz, 1 H), 4.40- 4.36 (m, 1 H), 4.26- 4.02 (m, 5 H), 3.57- 3.51 (m, 2 H), 3.13 (s, 3 H), 3.10- 2.95 (m, 10 H), 2.88- 2.85 (m, 2 H), 2.81- 2.66 (m, 2 H), 2.35- 2.32 (m, 1 H), 1.98- 1.95 (m, 1 H), 1.70- 1.66 (m, 4 H), 1.49- 1.39 (m, 2 H), 1.31 (d, J= 6.0 Hz, 3 H), 1.16- 1.12 (m, 4 H), 0.99 (s, 4 H), 0.79 (s, 4 H), 0.48-0.45 (m, 1 H), 0.38 (s, 3 H).

The structure of the other isomeric compound was arbitrarily designated as (1S,2S)-N-((6³S,4S,Z)-(Sₐ)-1²-(5-(2-(1,1-dioxidothiomorpholino)ethoxy)-2-(((S)-1-methoxyethyl)pyridin-3-yl)-1¹-isopropyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide (Z603-1, 44.87 mg, yield: 23%, retention time 1.86 min), ES-API [M+H]⁺ = 904.3. ¹H NMR (400 MHz, DMSO-d₆) δ 8.56- 8.53 (m,2 H), 8.49 (d, J= 2.8 Hz, 1 H), 7.80 (s, 1 H), 7.71- 7.66 (m, 2 H), 7.48 (d, J= 2.8 Hz, 1 H), 5.56 (t, J= 9.2 Hz, 1 H), 5.06 (d, J= 12.4 Hz, 1 H), 4.25- 4.15 (m, 4 H), 4.07- 4.00 (m, 1 H), 3.96- 3.92 (m, 1 H), 3.70 (d, J= 10.8 Hz, 1 H), 3.58 (d, J= 10.8 Hz, 1 H), 3.18- 2.75 (m, 17 H), 2.38- 2.34 (m, 1 H), 2.13- 2.10 (m, 1 H), 1.80- 1.76 (m, 2 H), 1.59 (d, J= 6.8 Hz, 3 H), 1.55- 1.45 (m, 2 H), 1.42 (d, J= 6.8 Hz, 3 H), 1.23 (d, J= 6.0 Hz, 3 H), 1.08- 1.03 (m, 4 H), 0.92- 0.87 (m, 4 H), 0.59- 0.53 (m, 4 H).

### Example 86. Synthesis of Compounds Z667A, Z667, and Z667-1

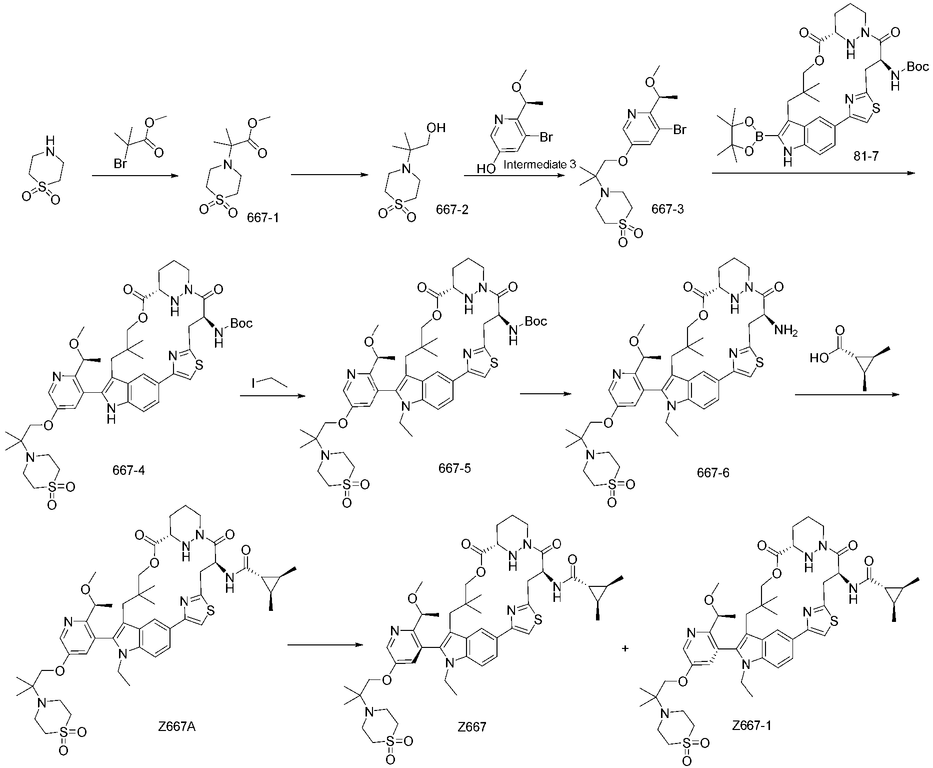

Step 1: N,N-Diisopropylethylamine (4.1 mL, 24.858 mmol) and thiomorpholine 1,1-dioxide (3.36 g, 24.858 mmol) were added to a solution of methyl 2-bromo-2-methylpropanoate (1.5 g, 8.286 mmol) in N,N-dimethylformamide (20 mL). The reaction mixture was stirred at 80 °C overnight. When the reaction was completed as detected by LCMS, ethyl acetate (50 mL) was added to the reaction mixture, and the mixture was washed with saturated brine (20 mL × 4), dried over anhydrous sodium sulfate, filtered, and concentrated. The residue was purified by automated flash chromatography on silica gel (tetrahydrofuran:petroleum ether = 0-35%) to give compound 667-1 (354 mg, yield: 18%). ES-API: [M+H]⁺ = 236.2.

Step 2: Lithium aluminum hydride (3.3 mL, 3.310 mmol) was slowly added to a solution of methyl 2-(1,1-dioxidodimorpholinyl)-2-methylpropanoate (354 mg, 1.504 mmol) in tetrahydrofuran (10 mL) under an ice-water bath and a nitrogen atmosphere. After the dropwise addition was completed, the reaction mixture was stirred at 0 °C for 1 h. The reaction was completed as detected by LCMS. Water (0.2 mL), a 10% sodium hydroxide solution (0.2 mL), and water (0.6 mL) were slowly and sequentially added dropwise to the reaction mixture to quench the reaction, then a small amount of anhydrous sodium sulfate was added, and the mixture was stirred for 10 min. The mixture was filtered to remove insoluble substances and concentrated under reduced pressure to give crude compound 667-2 (311 mg, yield: 99%). ES-API: [M+H]⁺ = 208.

Step 3: Compound 667-2 (311 mg, 1.500 mmol), triphenylphosphine (433 mg, 1.651 mmol), and diisopropyl azodicarboxylate (371 mg, 1.834 mmol) were added to a solution of intermediate 3 (200 mg, 0.917 mmol) in dichloromethane (15 mL). The reaction mixture was stirred at room temperature for 18 h under a nitrogen atmosphere. When the reaction was completed as detected by LC-MS, the mixture was concentrated under reduced pressure to remove the solvent. The residue was purified by automated flash chromatography on silica gel (ethyl acetate:petroleum ether = 0-25%) to give compound 667-3 (320 mg, yield: 29%, purity: 35%). ES-API: [M+H]⁺ = 421.1.

Step 4: Compound 667-3 (300 mg, 0.249 mmol), compound 81-7 (173 mg, 0.249 mmol), potassium phosphate (159 mg, 0.748 mmol), and [1,1'-bis(di-tert-butylphosphino)ferrocene]dichloropalladium(II) (32 mg, 0.050 mmol) were dissolved in dioxane (9 mL), toluene (3 mL), and water (3 mL) under a nitrogen atmosphere. The reaction mixture was heated to 70 °C under an oil bath and stirred for 5 h. When the reaction was completed as detected by LC-MS, the mixture was cooled to room temperature. Ethyl acetate (20 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran:petroleum ether = 0-85%) to give compound 667-4 (133 mg, yield: 59%). ES-API: [M+H]⁺ = 908.3.

Step 5: Cesium carbonate (476 mg, 1.460 mmol) and iodoethane (228 mg, 1.460 mmol) were sequentially added to a solution of compound 667-4 (133 mg, 0.146 mmol) in N,N-dimethylformamide (6 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give crude compound 667-5 (137 mg, yield: 99%). ES-API: [M+H]⁺ = 936.3.

Step 6: Compound 667-5 (137 mg, 0.146 mmol) was dissolved in a solution of hydrochloric acid in dioxane (5 mL, 4 M) and a solution of hydrochloric acid in methanol (1.5 mL, 4 M). The reaction mixture was stirred at room temperature for 1 h. When the reaction was completed as detected by LCMS, the mixture was concentrated under reduced pressure to give crude compound 667-6 (122 mg, yield: 99%). ES-API: [M+H]⁺ = 836.3.

Step 7: (1r,2R,3S)-2,3-Dimethylcyclopropane-1-carboxylic acid (19 mg, 0.190 mmol), N,N-diisopropylethylamine (0.2 mL, 1.148 mmol), and O-(7-azabenzotriazol-1-yl)-N,N,N',N'-tetramethyluronium hexafluorophosphate (83 mg, 0.219 mmol) were sequentially added to a solution of compound 667-2 (122 mg, 0.146 mmol) in N,N-dimethylformamide (5 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness to give crude compound Z667A. The crude product was purified by prep-HPLC (chromatographic column: CHIRALPAK^{®} IG, 10 µm, 30 × 250 mm; mobile phase A: ACN + 0.2% DEA, mobile phase B: IPA + 0.2% DEA; flow rate: 25 mL/min; isocratic elution program: mobile phase A:mobile phase B = 90:10 (V/V); column temperature: room temperature) to give 2 isomeric compounds. The structure of one of the isomeric compounds was arbitrarily designated as (1r,2R,3S)-N-((6³S,4S,Z)-(Rₐ)-1²-(5-(2-(1,1-dioxidothiomorpholino)-2-methylpropoxy)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z667, 10.56 mg, yield: 8%, retention time: 10.333 min), ES-API [M+H]⁺ = 932.3.

The structure of the other isomeric compound was arbitrarily designated as (1r,2R,3S)-N-((6⁷S,4S,Z)-(S)-1²-(5-(2-(1,1-dioxidothiomorpholino)-2-methylpropoxy)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-l(5,3)-indola-6(1,3)-pyridazinacycloundecaphane-4-yl)-2,3-dimethylcyclopropane-1-carboxamide (Z667-1, 24.98 mg, yield: 18%, retention time: 7.598 min), ES-API [M+H]⁺ = 932.3. ¹H NMR (400 MHz, DMSO-d₆) δ 8.54 (d, J= 0.8 Hz, 1 H), 8.47 (d, J= 2.8 Hz, 1 H), 8.42 (d, J= 8.8 Hz, 1 H), 7.83 (s, 1 H), 7.74 (dd, J1= 1.6 Hz, J2= 8.8 Hz, 1 H), 7.58 (d, J= 2.8 Hz, 1 H), 7.54 (d, J= 8.8 Hz, 1 H), 5.55 (t, J= 9.2 Hz, 1 H), 5.05 (d, J= 12.4 Hz, 1 H), 4.26- 4.19 (m, 2 H), 3.97- 3.84 (m, 3 H), 3.69 (d, J= 10.8 Hz, 1 H), 3.56 (d, J= 11.2 Hz, 1 H), 3.31- 3.29 (m, 1 H), 3.19- 3.04 (m, 13 H), 2.81- 2.73 (m, 3 H), 2.47- 2.44 (m, 1 H), 2.20- 2.11 (m, 1 H), 1.81- 1.76 (m, 2 H), 1.58- 1.47 (m, 1 H), 1.29 (d, J= 5.2 Hz, 6 H), 1.24- 1.16 (m, 6 H), 1.11- 1.07 (m, 9 H), 0.95 (s, 3 H), 0.53 (s, 3 H).

### Example 87. Synthesis of Compounds Z484 and Z484-1

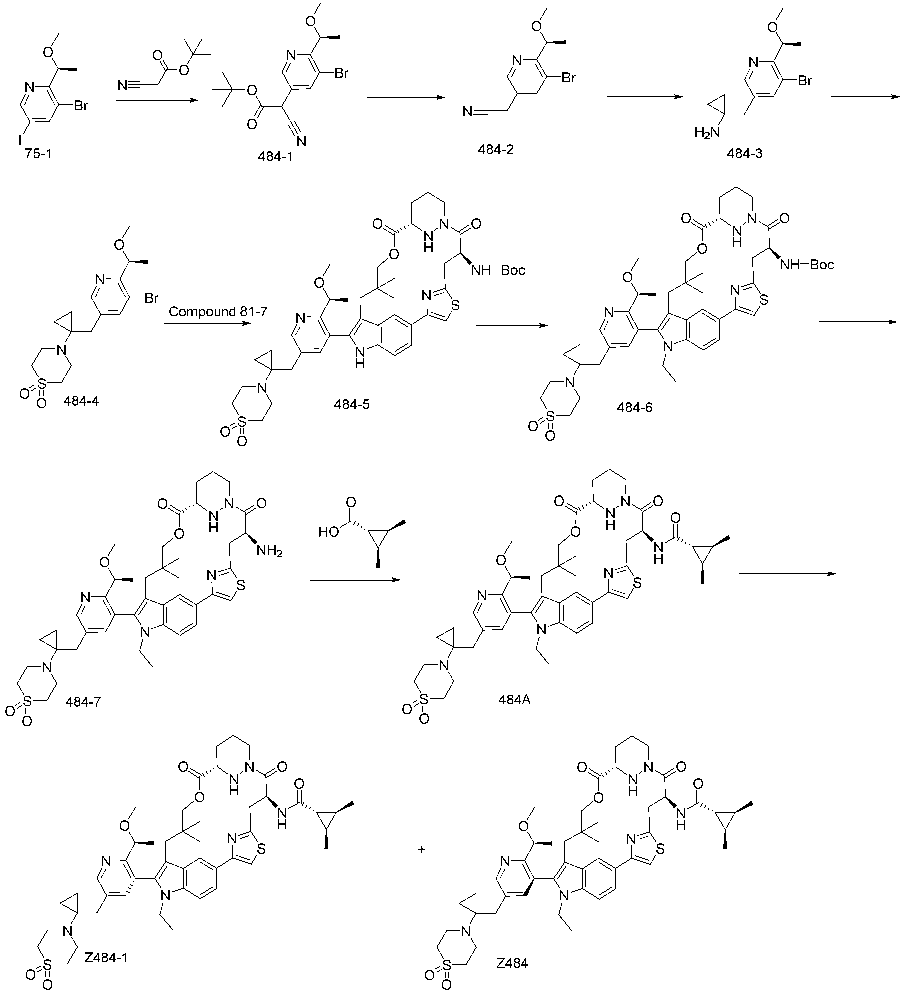

Step 1: Compound 75-1 (5 g, 14.621 mmol), copper(I) iodide (0.70 g, 2.193 mmol), and cesium carbonate (14.29 g, 43.863 mmol) were dissolved in dioxane (100 mL), and pyridinedicarboxylic acid (0.54 g, 4.386 mmol) and tert-butyl cyanoacetate (4.13 g, 29.242 mmol) were added to the solution described above. After the addition was completed, the reaction mixture was purged three times with nitrogen. The reaction system was stirred at 80 °C for 17 h. After the reaction was completed, the reaction mixture was poured into a saturated aqueous ammonium chloride solution (200 mL) and extracted with ethyl acetate (200 mL × 2). The organic phase was washed with saturated brine (200 mL), dried over anhydrous sodium sulfate, filtered, and concentrated to give a crude product. The crude product was purified by a flash silica gel column (ethyl acetate/petroleum ether: 0%-20%) to give compound 484-1 (3900 mg, 10.979 mmol, yield: 75.09%) as a colorless transparent liquid. ES-API: [M+H]⁺ = 355.1, 357.0.

Step 2: Compound 484-1 (3900 mg, 10.979 mmol) was dissolved in acetonitrile (50 mL), and p-toluenesulfonic acid monohydrate (321.26 mg, 1.689 mmol) was added to the solution described above. After the addition was completed, the reaction system was stirred at 80 °C for 1 h. After the reaction was completed, water (50 mL) was added to the solution described above, and the mixture was extracted with ethyl acetate (50 mL × 2). The organic phase was washed with saturated brine (50 mL × 2), dried over anhydrous sodium sulfate, filtered, and concentrated to give a crude product. The crude product was purified by a flash silica gel column (methanol/dichloromethane: 0%-2%) to give compound 484-2 (2500 mg, 9.799 mmol, yield: 89.26%) as a colorless oily liquid. ES-API: [M+H]⁺ = 255.1, 257.1.

Step 3: Compound 484-2 (2000 mg, 7.839 mmol) and tetraisopropyl titanate (4.642 mL, 15.679 mmol) were dissolved in anhydrous tetrahydrofuran (40 mL), and ethylmagnesium bromide (2 M, 15.68 mL, 31.36 mmol) was slowly added to the solution described above at room temperature under a nitrogen atmosphere. After the addition was completed, the reaction system was stirred at room temperature for another 1 h. Boron trifluoride etherate (48%, 4636.05 mg, 15.679 mmol) was then added to the reaction mixture, and the mixture was stirred for another 0.5 h. After the reaction was completed, the reaction mixture was sequentially quenched with water (20 mL) and an aqueous hydrochloric acid solution (1 M, 20 mL). Dichloromethane (50 mL) was then added, and the pH value was adjusted to basicity with an aqueous sodium hydroxide solution (1 M). After the liquid separation was performed, the aqueous phase was extracted with dichloromethane (30 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-8%) to give compound 484-3 (150 mg, 0.526 mmol, yield: 6.71%) as a colorless transparent liquid. ES-API: [M+H]⁺ = 285.1, 287.1.

Step 4: Compound 484-3 (150 mg, 0.526 mmol) was dissolved in N,N-dimethylformamide (3 mL), and 1-chloro-2-(2-chloroethylsulfonyl)ethane (130.65 mg, 0.684 mmol), sodium carbonate (167.24 mg, 1.578 mmol), and sodium iodide (157.67 mg, 1.052 mmol) were added to the solution described above. After the addition was completed, the reaction system was stirred at 100 °C for 17 h under a nitrogen atmosphere. After the reaction was completed, water (10 mL) was added to the solution described above, and the mixture was extracted with ethyl acetate (10 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-4%) to give compound 484-4 (35 mg, 0.087 mmol, yield: 16.50%) as a light yellow solid. ES-API: [M+H]⁺ = 403.1, 405.1. ¹H NMR (400 MHz, CDCl₃) δ 8.34 (s, 1H), 7.58 (s, 1H), 4.87 - 4.77 (m, 1H), 3.25 (s, 3H), 3.15 - 3.08 (m, 4H), 2.94 - 2.87 (m, 4H), 2.79 (s, 2H), 1.41 (d, J=6.4 Hz, 3H), 0.64 - 0.56 (m, 2H), 0.43 - 0.38 (m, 2H).

Step 5: Compound 484-4 (41.86 mg, 0.104 mmol) was dissolved in dioxane (2 mL), toluene (0.7 mL), and water (0.7 mL). Compound 81-7 (60 mg, 0.086 mmol), [1,1'-bis(di-tert-butylphosphino)ferrocene]dichloropalladium(II) (5.60 mg, 0.009 mmol), and potassium phosphate (55.24 mg, 0.260 mmol) were added to the solution described above at room temperature. After the addition was completed, the reaction system was stirred at 70 °C for 2 h under an argon atmosphere. After the reaction was completed, water (10 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 484-5 (45 mg, 0.051 mmol, yield: 58.45%) as a light yellow solid. ES-API: [M+H]⁺ = 890.1.

Step 6: Compound 484-5 (45 mg, 0.051 mmol) was dissolved in N,N-dimethylformamide (2 mL). Iodoethane (39.42 mg, 0.253 mmol) and cesium carbonate (82.36 mg, 0.253 mmol) were added to the solution described above. The reaction system was stirred at room temperature for 17 h. After the reaction was completed, water (20 mL) was added to the solution described above, and the mixture was extracted with ethyl acetate (10 mL × 3). The organic phase was washed with saturated brine (20 mL × 3), dried over anhydrous sodium sulfate, filtered, and concentrated to give a crude product. The crude product was purified by a flash silica gel column (methanol/dichloromethane: 0%-3%) to give compound 484-6 (45 mg, 0.049 mmol, yield: 96.95%) as a white solid. ES-API: [M+H]⁺ = 918.1.

Step 7: Compound 484-6 (40 mg, 0.044 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give crude compound 484-7 (36 mg, crude product) as a white solid. ES-API: [M+H]⁺ = 818.3.

Step 8: Compound 484-7 (36 mg, crude product) was dissolved in dichloromethane (2 mL). (1r,2R,3S)-2,3-Dimethylcyclopropane-1-carboxylic acid (7.53 mg, 0.066 mmol), O-(7-azabenzotriazol-1-yl)-*N,N,N',N'*-tetramethyluronium hexafluorophosphate (25.10 mg, 0.066 mmol), and N,N'-diisopropylethylamine (17 mg, 0.132 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-5%) to give compound 484A (35 mg, yield: 83%). The compound was subjected to prep-HPLC (formic acid method 1) to give 2 isomeric compounds. The structure of one of the isomeric compounds was arbitrarily designated as (1*r*,2*R*,3*S*)-*N*-((6³*S*,4*S*,Z)-(*Sₐ*)-1²-(5-((1-(1,1-dioxidothiomorpholino)cyclopropyl)methyl)-2-((S)-l-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (formate, Z484-1, 7.88 mg, yield: 18.65%, retention time = 7.05 min). ¹H NMR (400 MHz, CD₃OD) δ 8.68 (d, J = 1.6 Hz, 1H), 8.56 (s, 1H), 7.86 (s, 1H), 7.71 (dd, dd, J = 1.2 Hz, 8.4 Hz, 1H), 7.56 (s, 1H), 7.47 (d, J = 8.8 Hz, 1H), 5.66 (d, J = 8.8 Hz, 1H), 4.43 (d, J = 12.0 Hz, 1H), 4.31 - 4.25 (m, 1H), 4.23 - 4.17 (m, 1H), 4.13 - 4.03 (m, 1H), 3.81 - 3.66 (m, 3H), 3.49 - 3.40 (m, 1H), 3.28 - 3.26 (m, 3H), 3.24 - 3.14 (m, 6H), 3.04 - 2.95 (m, 5H), 2.82 - 2.73 (m, 1H), 2.38 - 2.32 (m, 1H), 2.29 - 2.22 (m, 1H), 2.00 - 1.93 (m, 1H), 1.90 - 1.77 (m, 1H), 1.67 - 1.55 (m, 1H), 1.42 - 1.31 (m, 3H), 1.30 -1.26 (m, 3H), 1.22 - 1.12 (m, 10H), 0.99 (s, 3H), 0.78 - 0.74 (m, 2H), 0.66 - 0.60 (m, 1H), 0.60 - 0.53 (m, 4H). The structure of the other isomeric compound was arbitrarily designated as (1*r*,2*R*,3*S*)*-N*-((6³*S*,4*S*,Z)-(*Rₐ*)-1²-(5-((1-(1,1-dioxidothiomorpholino)cyclopropyl)methyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (formate, Z484, 6.03 mg, yield: 14.27%, retention time = 7.56 min) as a white solid. ES-API: [M+H]⁺ = 914.3. ¹H NMR (400 MHz, DMSO-d₆) δ 8.63 (d, *J* = 2.0 Hz, 1H), 8.50 (s, 1H), 8.39 (d, *J =* 8.8 Hz, 1H), 7.82 (s, 1H), 7.75 (dd, *J* = 8.4 Hz, 1.2 Hz, 1H), 7.68 (d, *J* =2.0 Hz, 1H), 7.58 (d, *J* = 8.8 Hz, 1H), 5.56 (t, *J* = 9.2 Hz, 1H), 5.06 (d, *J* = 12.4 Hz, 1H), 4.37 - 4.08 (m, 5H), 3.58 (s, 2H), 3.36 - 3.33 (m, 1H), 3.25 (s, 3H), 3.20 -3.12 (m, 1H), 3.08- 2.90 (m, 12H), 2.80 - 2.70 (m, 1H), 2.45 - 2.37 (m, 1H), 2.12 - 2.04 (m, 1H), 1.84 - 1.74 (s, 2H), 1.58 -1.46 (m, 1H), 1.35 (d, *J* = 6.0 Hz, 3H), 1.26 -1.25 (m, 1H), 1.10 - 1.05 (m, 6H), 0.91 - 0.85 (m, 6H), 0.6 - 0.61 (m, 2H), 0.53 - 0.48 (m, 2H), 0.31 (s, 3H).

### Example 88. Synthesis of Compounds Z643 and Z643-1

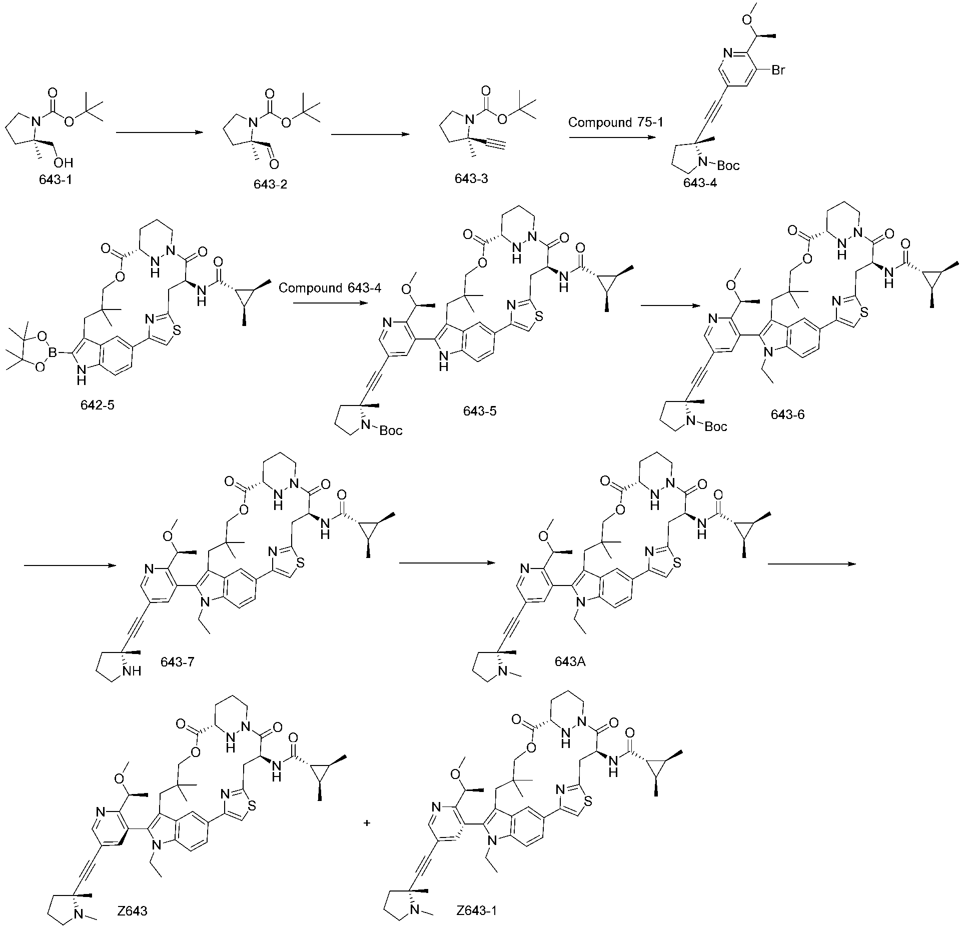

Step 1: Compound 643-1 (1.2 g, 5.574 mmol) was dissolved in dichloromethane (20 mL), and Dess-Martin periodinane (2.84 g, 6.689 mmol) was added. The mixture was reacted at room temperature for 1 h. A saturated sodium bicarbonate solution (10 mL) and water (10 mL) were added, and the mixture was extracted with dichloromethane (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 643-2 (0.8 g, 3.751 mmol, yield: 67.23%). ES-API: [M-56]⁺ = 158.2.

Step 2: Compound 643-2 (0.8 g, 3.751 mmol) and potassium carbonate (1.04 g, 7.502 mmol) were dissolved in methanol (10.00 mL), and dimethyl (1-diazo-2-oxopropyl)phosphonate (1.08 g, 5.626 mmol) was added. The mixture was reacted at room temperature for 1 h. A saturated sodium bicarbonate solution (10 mL) and water (10 mL) were added, and the mixture was extracted with dichloromethane (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 643-3 (0.6 g, 2.867 mmol, yield: 76.43%). ES-API: [M-56]⁺ = 154.2.

Step 3: Compound 75-1 (550 mg, 1.608 mmol), copper(I) iodide (153.15 mg, 0.804 mmol), and [1,1'-bis(diphenylphosphino)ferrocene]palladium(II) dichloride dichloromethane complex (328.36 mg, 0.402 mmol) were dissolved in N,N-dimethylformamide (2.00 mL), and compound 643-3 (673.21 mg, 3.217 mmol) and N,N-diisopropylethylamine (1039.38 mg, 8.042 mmol) were added. The mixture was reacted at 50 °C for 2 h. Water (10 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 643-4 (0.6 g, 2.867 mmol, yield: 76.43%). ES-API: [M+H]⁺ = 423.1.

Step 4: Compound 642-5 (200 mg, 0.290 mmol) and compound 643-4 (92.08 mg, 0.217 mmol) were dissolved in toluene (1.5 mL), 1,4-dioxane (0.5 mL), and water (0.5 mL), and potassium phosphate (115.41 mg, 0.544 mmol) and [1,1'-bis(di-tert-butylphosphino)ferrocene]dichloropalladium(II) (18.88 mg, 0.029 mmol) were added. The mixture was reacted at 70 °C for 3 h under a nitrogen atmosphere. Water (5 mL) was added, and the mixture was extracted with ethyl acetate (10 mL × 3). The organic phase was washed with saturated brine (5 mL), dried over anhydrous sodium sulfate, filtered, and concentrated under reduced pressure, and the residue was purified by silica gel column chromatography (ethyl acetate:petroleum ether = 1:1) to give compound 643-5 (210 mg, 0.232 mmol, yield: 79.91%). ES-API: [M+H]⁺ = 906.3.

Step 5: Compound 643-5 (180 mg, 0.199 mmol) was dissolved in N,N-dimethylformamide (1 mL), and cesium carbonate (194.16 mg, 0.596 mmol) and iodoethane (92.96 mg, 0.596 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, filtered, and concentrated under reduced pressure to give compound 643-6 (175 mg, 0.187 mmol, yield: 94.30%). ES-API: [M+H]⁺ = 934.3.

Step 6: Compound 643-6 (170 mg, 0.182 mmol) was dissolved in methanol (1 mL), and a solution of hydrochloric acid in dioxane (4 mL, 0.110 mmol) was added. The mixture was reacted at room temperature for 2 h. The reaction mixture was concentrated under reduced pressure to give compound 643-7 (150 mg, 0.180 mmol, yield: 98.83%). ES-API: [M+H]⁺ = 834.4.

Step 7: Compound 643-7 (160 mg, 0.192 mmol) was dissolved in methanol (1 mL), and formic acid (44.12 mg, 0.959 mmol), an aqueous formaldehyde solution (36%, 28.77 mg, 0.959 mmol), and sodium cyanoborohydride (3.72 mg, 0.060 mmol) were added. The mixture was reacted at room temperature for 1 h. Water (5 mL) was added, and the mixture was extracted with ethyl acetate (10 mL × 3). The organic phase was washed with water (5 mL) and saturated brine (5 mL), dried over anhydrous sodium sulfate, filtered, and concentrated under reduced pressure to give crude compound 643A. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-(Rₐ)-1²-(5-(((R)-1,2-dimethylpyrrolidin-2-yl)ethynyl)-2-((S)-1-methoxyethyl)pyridin-3-yl)-1¹-ethyl-10,10-dimethyl-5,7-dioxo-6¹,6²6³,6⁴6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z643, 3 mg, 0.035 mmol, yield: 18.44%, retention time = 9.19 min). ES-API: [M+H]⁺ = 848.3.

The structure of the other isomeric compound was arbitrarily designated as (1r,2R,3S)-N-((6³S,4S,7)-(Sₐ)-1²-(5-(((R)-1,2-dimethylpyrrolidin-2-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z643-1, 38 mg, 0.045 mmol, yield: 23.36%, retention time = 8.93 min). ES-API: [M+H]⁺ = 848.3. ¹H NMR (400 MHz, DMSO-d₆) δ 8.71 (d, *J* = 2.0 Hz, 1H), 8.45 (d, *J* = 1.2 Hz, 1H), 8.33 (d, *J* = 9.2 Hz, 1H), 7.85 (d, *J* = 2.4 Hz, 1H), 7.75 (s, 1H), 7.67 (dd, *J* = 8.4, 1.2 Hz, 1H), 7.47 (d, *J* = 8.4 Hz, 1H), 5.46 (t, *J* = 9.2 Hz, 1H), 4.97 (d, *J* = 4.0 Hz, 1H), 4.27 - 4.05 (m, 2H), 3.96 - 3.70 (m, 3H), 3.60 (d, *J =* 10.4 Hz, 1H), 3.48 (d, *J =* 10.4 Hz, 1H), 3.18 - 2.80 (m, 6H), 2.71-2.69 (m, 1H), 2.39-2.37 (m, 1H), 2.27 - 2.15 (m, 4H), 2.07-2.04 (m, 2H), 1.71-1.65 (m, 5H), 1.44-1.42 (m, 1H), 1.30 (s, 3H), 1.20 - 0.92 (m, 16H), 0.89 - 0.78 (m, 3H), 0.43 (s, 3H).

### Example 89. Synthesis of Compound Z665

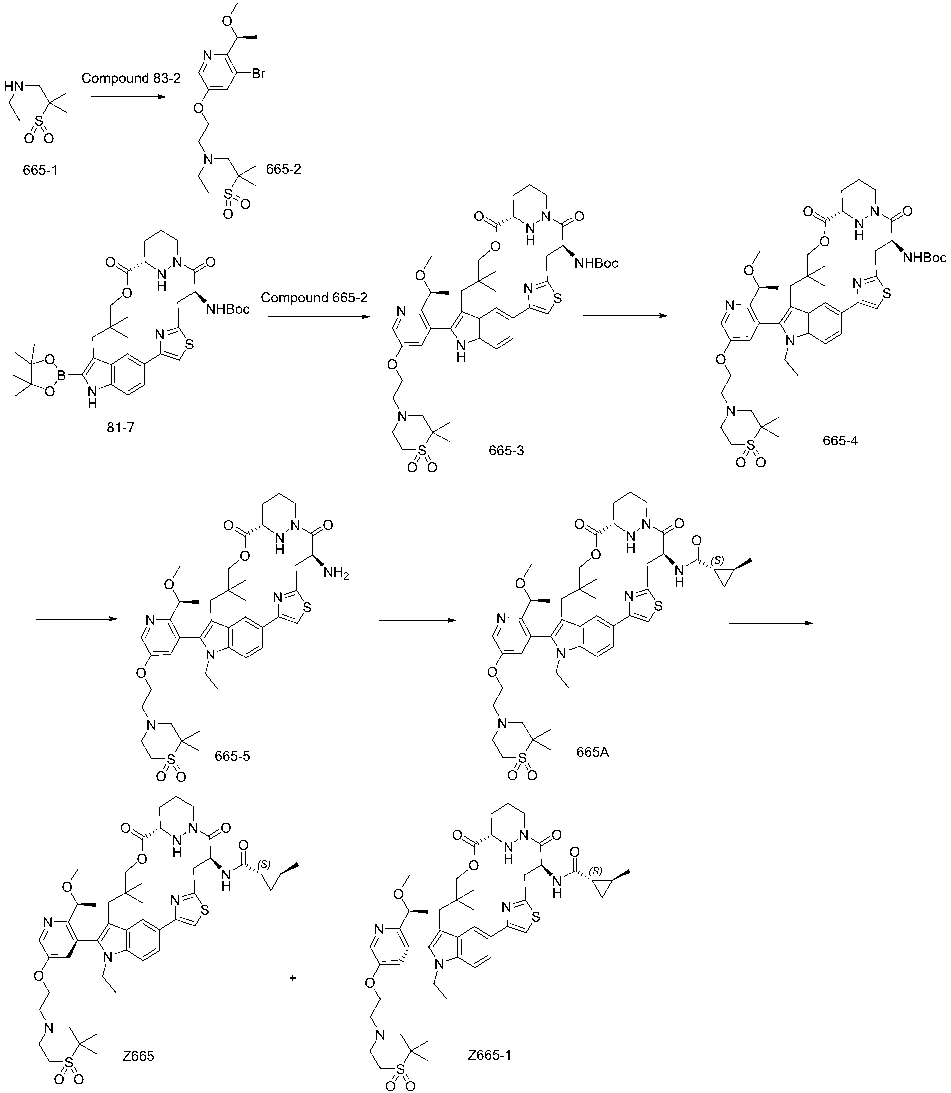

Step 1: Compound 83-2 (177.10 mg, 0.5 mmol) was dissolved in N,N-dimethylformamide (2 mL), and compound 665-1 (70 mg, 0.429 mmol) and N,N-diisopropylethylamine (0.2 mL, 1.250 mmol) were added. The mixture was reacted at 75 °C for 24 h. After the reaction was completed, water (15 mL) was added, and the mixture was extracted with ethyl acetate (10 mL × 3). The organic phase was washed with saturated brine (15 mL), dried over anhydrous sodium sulfate, filtered, and concentrated under reduced pressure, and the residue was purified by a flash silica gel column (ethyl acetate/petroleum ether: 0%-70%) to give compound 665-2 (150 mg, 0.356 mmol, yield: 83.33%) as a yellow oily liquid. ES-API: [M+H]⁺ = 421.1, 423.1.

Step 2: Compound 81-7 (150 mg, 0.216 mmol) and compound 665-2 (59.53 mg, 0.151 mmol) were dissolved in toluene (1.5 mL), 1,4-dioxane (0.5 mL), and water (0.5 mL), and potassium phosphate (137.70 mg, 0.649 mmol) and [1,1'-bis(di-tert-butylphosphino)ferrocene]dichloropalladium(II) (18.88 mg, 0.029 mmol) were added. The mixture was reacted at 70 °C for 3 h under a nitrogen atmosphere. Water (5 mL) was added, and the mixture was extracted with ethyl acetate (10 mL × 3). The organic phase was washed with saturated brine (5 mL), dried over anhydrous sodium sulfate, filtered, and concentrated under reduced pressure, and the residue was purified by a flash silica gel column (methanol/dichloromethane: 0%-10%) to give compound 665-3 (130 mg, 0.143 mmol, yield: 66.20%) as a grayish-white solid. ES-API: [M+H]⁺ = 908.3.

Step 3: Compound 665-3 (130 mg, 0.143 mmol) was dissolved in N,N-dimethylformamide (1 mL), and cesium carbonate (194.16 mg, 0.596 mmol) and iodoethane (92.96 mg, 0.596 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, filtered, and concentrated under reduced pressure to give compound 665-4 (130 mg, 0.143 mmol, yield: 97.01%) as a white solid. ES-API: [M+H]⁺ = 936.3.

Step 4: Compound 665-4 (130 mg, 0.143 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give crude compound 665-5 (130 mg, 0.143 mmol, yield: 100%) as a yellow solid. ES-API: [M+H]⁺ = 836.3.

Step 5: Compound 665-5 (120 mg, 0.144 mmol) was dissolved in N,N-dimethylformamide (2 mL). trans-2-Methylcyclopropanecarboxylic acid (17.24 mg, 0.172 mmol), O-(7-azabenzotriazol-1-yl)-N,N,N',N'-tetramethyluronium hexafluorophosphate (81.86 mg, 0.215 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried over anhydrous sodium sulfate, filtered, and concentrated to give crude compound 665A. The crude product was purified by preparative thin-layer chromatography (methanol/dichloromethane = 1/20) to give 2 isomeric compounds. The structure of one of the isomeric compounds was arbitrarily designated as (1S,3S)-N-((6³S,4S,Z)-(Rₐ)-12-(5-(2-(2,2-dimethyl-1,1-dioxidothiomorpholino)ethoxy)-(2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z665, 12 mg, 0.013 mmol, yield: 9.11 %, Rf = 0.35) as a white solid. ES-API: [M+H]⁺ = 918.3. ¹H NMR (400 MHz, DMSO-d6) δ 8.55 - 8.50 (m, 3H), 7.81 (s, 1H), 7.75 (d, *J* = 7.6 Hz, 1H), 7.58 (d, *J* = 8.8 Hz, 1H), 7.39 (d, *J* = 2.8 Hz, 1H), 5.57 (t, *J* = 9.4 Hz, 1H), 5.08 (d, *J* = 12.4 Hz, 1H), 4.58 - 3.98 (m, 6H), 3.23 (s, 3H),3.26 - 2.88 (m, 8H), 2.76 (s, 3H),2.45-2.43 (m, 2H), 2.08-2.07 (m, 1H), 1.80-1.79 (m, 1H), 1.51-1.50 (m, 2H), 1.43 - 1.17 (m, 14H), 1.08-1.07 (m, 4H), 0.89-0.87 (m, 7H), 0.34 (s, 3H).

The structure of the other isomeric compound was arbitrarily designated as (1S,3S)-*N*-((6³*S*,4*S,Z*)-(*Sₐ*)-1²-(5-(2-(2,2-dimethyl-1,1-dioxidothiomorpholino)ethoxy)-(2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z665-1, 15 mg, 0.016 mmol, 11.11%) (dichloromethane:methanol = 20:1, Rf = 0.31) as a white solid. ES-API: [M+H]⁺ = 918.3.

### Example 90. Synthesis of Compounds Z711 and Z711-1

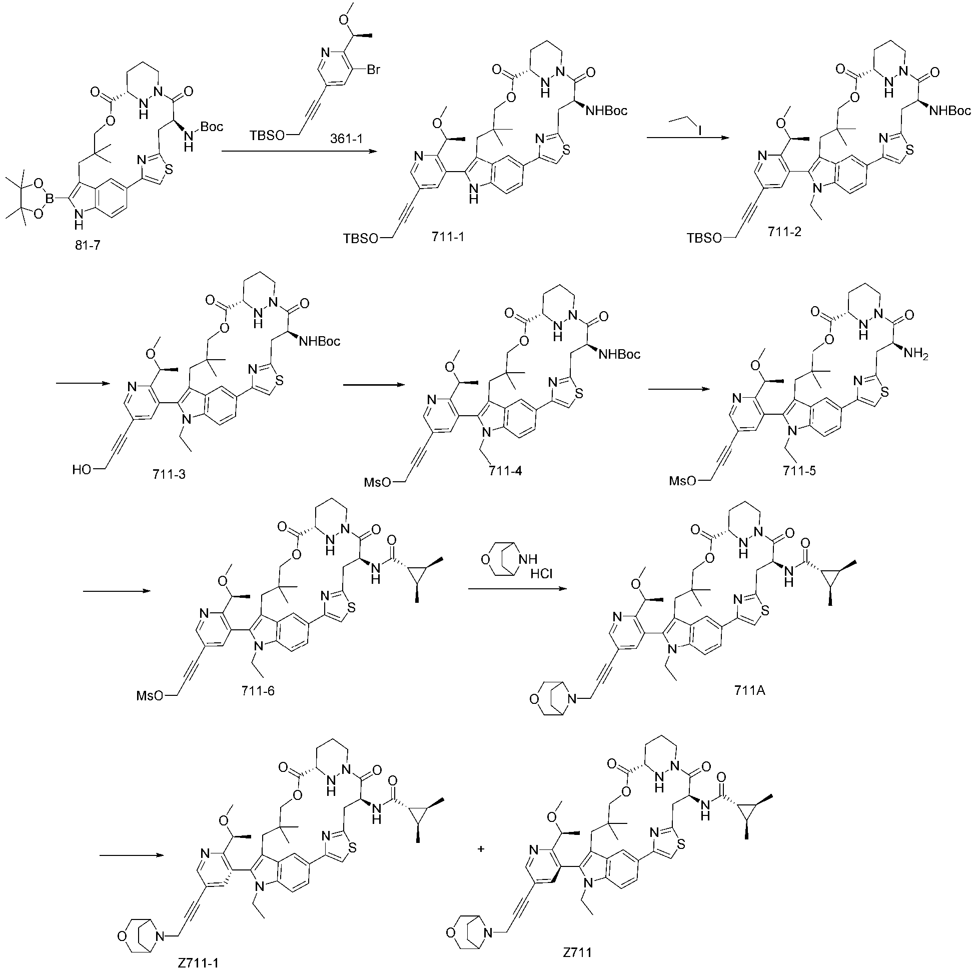

Step 1: Compound 81-7 (1166 mg, 1.68 mmol), compound 361-1 (776 mg, 2.018 mmol), potassium phosphate (1070 mg, 5.042 mmol), and [1,1'-bis(di-tert-butylphosphino)ferrocene]dichloropalladium(II) (218 mg, 0.336 mmol) were dissolved in dioxane (18 mL), toluene (6 mL), and water (6 mL) under a nitrogen atmosphere. The reaction mixture was heated to 70 °C under an oil bath and stirred for 2 h. When the reaction was completed as detected by LCMS, the mixture was cooled to room temperature. Ethyl acetate (30 mL) was added to the reaction mixture, and the mixture was washed with water (15 mL) and saturated brine (15 mL × 3). The organic phase was dried over anhydrous sodium sulfate, filtered, and concentrated to dryness. The residue was purified by an automated flash silica gel column (tetrahydrofuran/petroleum ether = 0-40%) to give compound 711-1 (956 mg, yield: 65%). ES-API: [M+H]⁺ = 871.3.

Step 2: Cesium carbonate (3572 mg, 10.962 mmol) and iodoethane (0.88 mL, 10.962 mmol) were sequentially added to a solution of compound 711-1 (955 mg, 1.096 mmol) in N,N-dimethylformamide (10 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give crude compound 711-2 (1136 mg, yield: 99%). ES-API: [M+H]⁺ = 899.3.

Step 3: A tetrabutylammonium fluoride solution (3.670 mL, 3.670 mmol) was added to a solution of compound 711-2 (1100 mg, 1.233 mmol) in tetrahydrofuran (30 mL) under an ice-water bath. The reaction mixture was stirred at 0 °C for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to dryness to give crude compound 711-3 (960 mg, yield: 99%). ES-API: [M+H]⁺ = 785.3.

Step 4: N,N-Diisopropylethylamine (1.0 mL, 5.733 mmol) and methanesulfonic anhydride (300 mg, 1.720 mmol) were sequentially added to a solution of compound 711-3 (900 mg, 1.147 mmol) in dichloromethane (10 mL) under an ice-water bath. The reaction mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 711-4 (970 mg, yield: 98%). ES-API: [M+H]⁺ = 863.2.

Step 5: Trifluoroacetic acid (5 mL) was added to a solution of compound 711-4 (970 mg, 1.124 mmol) in dichloromethane (5 mL), and the reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined and dried over anhydrous sodium sulfate. The mixture was concentrated, and the crude product was purified by automated flash chromatography on silica gel (methanol/dichloromethane = 0-6%) to give compound 711-5 (591 mg, yield: 69%). ES-API: [M+H]⁺ = 763.1.

Step 6: (1r,2R,3S)-2,3-Dimethylcyclopropane-1-carboxylic acid (101 mg, 0.885 mmol), N,N-diisopropylethylamine (0.52 mL, 2.949 mmol), and O-(7-azabenzotriazol-1-yl)-N,N,N',N'-tetramethyluronium hexafluorophosphate (113 mg, 0.297 mmol) were sequentially added to a solution of compound 711-5 (450 mg, 0.590 mmol) in N,N-dimethylformamide (10 mL) under an ice-water bath. The reaction mixture was warmed to room temperature and stirred for 10 min. When the reaction was completed as detected by LCMS, the reaction mixture was diluted with ethyl acetate (20 mL) and then washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give compound 711-6 (400 mg, yield: 79%). ES-API: [M+H]⁺ = 859.2.

Step 7: N,N-Diisopropylethylamine (0.13 mL, 0.728 mmol) and 8-aza-3-oxabicyclo[3.2.1]octane hydrochloride (44 mg, 0.291 mmol) were sequentially added to a solution of compound 711-6 (125 mg, 0.146 mmol) in dichloromethane (6 mL), and the reaction mixture was stirred at room temperature overnight. When the reaction was completed as detected by LCMS, the mixture was concentrated under reduced pressure to remove the solvent to give crude compound 711A. The crude product was purified by prep-HPLC (ammonium bicarbonate method) to give 2 isomeric compounds. The structure of one of the isomeric compounds was arbitrarily designated as: (1r,2R,3S)-N-((6³S,4S,Z)-(Sₐ)-1²-(5-(3-(3-oxa-8-azabicyclo[3.2.1]octan-8-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z711-1, 23.79 mg, yield: 19%, retention time = 1.51 min), ES-API [M+H]⁺ = 876.3. ¹H NMR (400 MHz, DMSO-d₆) δ 8.82 (d, J= 2.0 Hz, 1 H), 8.53 (s, 1 H), 8.40 (d, J= 8.8 Hz, 1 H), 8.00 (d, J= 2.0 Hz, 1 H), 7.82 (s, 1 H), 7.74 (dd, J1= 1.6 Hz, J2= 8.8 Hz, 1 H), 7.55 (d, J= 8.4 Hz, 1 H), 5.54 (t, J= 9.2 Hz, 1 H), 5.04 (d, J= 12.0 Hz, 1 H), 4.25- 4.19 (m, 2 H), 3.98- 3.93 (m, 2 H), 3.87- 3.80 (m, 1 H), 3.67 (d, J= 10.8 Hz, 1 H), 3.56 (d, J= 10.0 Hz, 3 H), 3.46- 3.45 (m, 2 H), 3.39 (s, 2 H), 3.30-3.28 (m, 1 H), 3.23- 3.19 (m, 2 H), 3.17- 3.03 (m, 5 H), 2.81- 2.74 (m, 1 H), 2.33 (d, J= 14.4 Hz, 1 H), 2.14- 2.11 (m, 1 H), 1.92- 1.89 (m, 2 H), 1.80- 1.73 (m, 4 H), 1.58- 1.47 (m, 1 H), 1.24- 1.17 (m, 6 H), 1.13- 1.07 (m, 9 H), 0.93 (s, 3 H), 0.50 (s, 3 H).

The structure of the other isomeric compound was arbitrarily designated as: (1r,2R,3S)-N-((6³S,4S,Z)-(Rₐ)-1²-(5-(3-(3-oxa-8-azabicyclo[3.2.1]octan-8-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z711, 12.36 mg, yield: 10%, retention time = 1.50 min), ES-API [M+H]⁺ = 876.3. ¹H NMR (400 MHz, CD₃OD) δ 8.77 (d, J = 2.0 Hz, 1H), 8.57 (s, 1H), 7.88 (d, J = 2.0 Hz, 1H), 7.71 (dd, J = 8.4, 1.2 Hz, 1H), 7.55 (s, 1H), 7.49 (d, J = 8.8 Hz, 1H), 5.75 (d, J = 7.6 Hz, 1H), 4.42 (d, J = 11.6 Hz, 1H), 4.38 - 4.25 (m, 2H), 4.21 (dd, J = 11.6, 2.8 Hz, 1H), 4.16 - 4.08 (m, 1H), 3.76 - 3.67 (m, 4H), 3.58 - 3.52 (m, 2H), 3.47 - 3.33 (m, 8H), 3.08 (d, J = 14.4 Hz, 1H), 2.81 - 2.72 (m, 1H), 2.54 (d, J = 14.4 Hz, 1H), 2.23 - 2.13 (m, 1H), 2.05 - 1.89 (m, 5H), 1.83 - 1.68 (m, 1H), 1.65 - 1.55 (m, 1H), 1.43 (d, J = 6.0 Hz, 3H), 1.41 - 1.32 (m, 3H), 1.18 - 1.11 (m, 7H), 0.98 - 0.91 (m, 6H), 0.46 (s, 3H).

### Example 91. Synthesis of Compounds Z812 and Z812-1

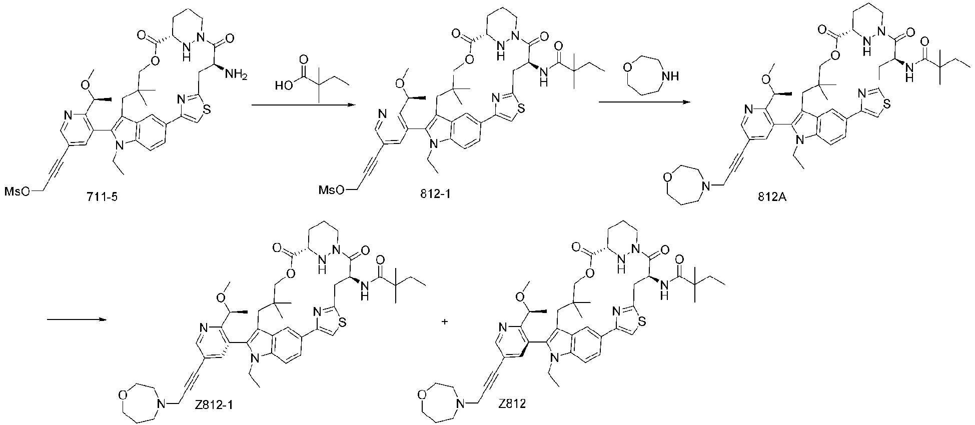

Step 1: 2,2-Dimethylbutyric acid (32 mg, 0.275 mmol), N,N-diisopropylethylamine (0.16 mL, 0.918 mmol), and O-(7-azabenzotriazol-1-yl)-N,N,N',N'-tetramethyluronium hexafluorophosphate (105 mg, 0.275 mmol) were sequentially added to a solution of compound 711-5 (140 mg, 0.184 mmol) in N,N-dimethylformamide (2 mL) under an ice-water bath. The reaction mixture was warmed to room temperature and stirred for 10 min. When the reaction was completed as detected by LCMS, the reaction mixture was diluted with ethyl acetate (20 mL) and then washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give compound 812-1 (119 mg, yield: 75%). ES-API: [M+H]⁺ = 861.2.

Step 2: N,N-Diisopropylethylamine (0.12 mL, 0.728 mmol) and 1,4-oxazepane (28 mg, 0.276 mmol) were sequentially added to a solution of compound 812-1 (119 mg, 0.138 mmol) in dichloromethane (6 mL). The reaction mixture was stirred at room temperature overnight. When the reaction was completed as detected by LCMS, the mixture was concentrated under reduced pressure to remove the solvent to give crude compound 812A. The crude product was purified by prep-HPLC (ammonium bicarbonate method) to give 2 isomeric compounds. The structure of one of the isomeric compounds was arbitrarily designated as: N-((6³S,4S,Z)-(Sₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,2-dimethylbutanamide (Z812-1, 19 mg, yield: 16%, retention time = 1.58 min), ES-API [M+H]⁺ = 866.3. ¹H NMR (400 MHz, DMSO-d₆) δ 8.86 (s, 1 H), 8.53 (d, J= 1.2 Hz, 1 H), 8.03 (s, 1 H), 7.82 (s, 1 H), 7.76-7.70 (m, 2 H), 7.54 (d, J= 8.4 Hz, 1 H), 5.51 (t, J= 9.2 Hz, 1 H), 5.01 (d, J= 12.0 Hz, 1 H), 4.28- 4.21 (m, 2 H), 3.98- 3.80 (m, 3 H), 3.71 (t, J= 6.0 Hz, 3 H), 3.66 (d, J= 11.2 Hz, 1 H), 3.56 (d, J= 11.2 Hz, 1 H), 3.51- 3.45 (m, 1 H), 3.30- 3.25 (m, 4 H), 3.10- 2.74 (m, 9 H), 2.34- 2.31 (m, 1 H), 2.14- 2.11 (m, 1 H), 1.93- 1.76 (m, 4 H), 1.65- 1.50 (m, 3 H), 1.22 (d, J= 6.4 Hz, 3 H), 1.15- 1.09 (m, 9 H), 0.94 (s, 3 H), 0.81 (t, J= 7.6 Hz, 3 H), 0.50 (s, 3 H).

The structure of the other isomeric compound was arbitrarily designated as: N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,2-dimethylbutanamide (Z812, 14.48 mg, yield: 12%, retention time = 2.22 min), ES-API [M+H]⁺ = 866.3.

### Example 92. Synthesis of Compound Z810A

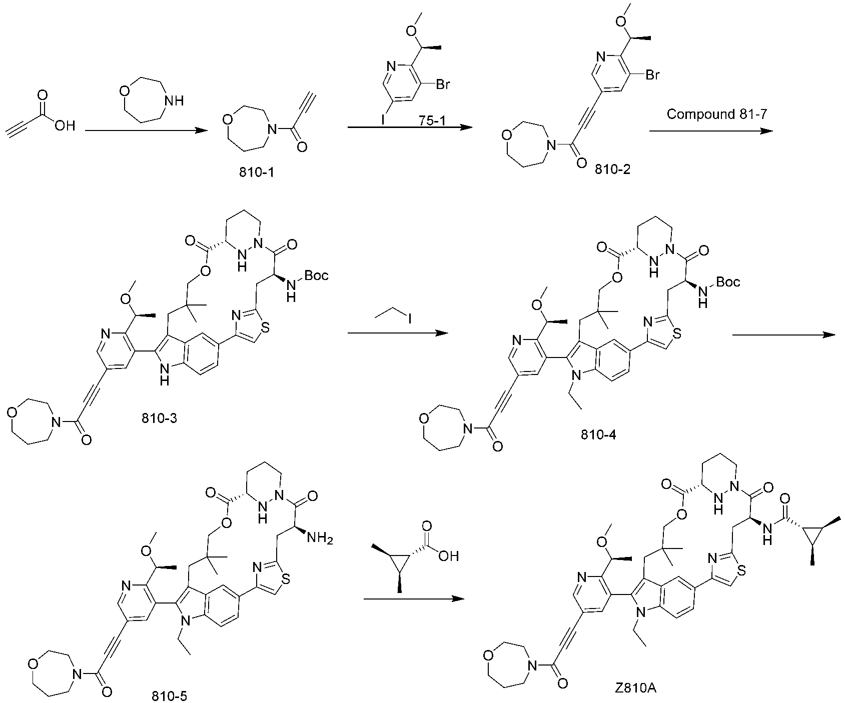

Step 1: Propynoic acid (1.25 g, 17.79 mmol) and triethylamine (4.5 g, 44.48 mmol) were added to a solution of 1,4-oxazepane (1.5 g, 14.83 mmol) in N,N-dimethylformamide (10 mL), and O-(7-azabenzotriazol-1-yl)-N,N,N',N'-tetramethyluronium hexafluorophosphate (8.46 g, 22.24 mmol) was added under an ice bath. The mixture was stirred at room temperature for 1 h. After the reaction was completed, the mixture was extracted with ethyl acetate (20 mL × 3). The combined extract was washed with brine (30 mL), dried over sodium sulfate, filtered, and concentrated, and the residue was subjected to flash silica gel column chromatography (0-40% petroleum ether/ethyl acetate) to give compound 810-1 (1.82 g, yield: 80%). ES-API [M+H]⁺ = 153.5.

Step 2: Compound 810-1 (455 mg, 2.97 mmol), compound 75-1 (1000 mg, 2.924 mmol), bis(triphenylphosphine)palladium(II) dichloride (104.11 mg, 0.149 mmol), copper(I) iodide (56.44 mg, 0.297 mmol), and N,N-diisopropylethylenediamine (766.35 mg, 5.941 mmol) were mixed in acetonitrile (10 mL) under a nitrogen atmosphere, and the mixture was stirred at room temperature for 1 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 810-2 (0.9 g, yield: 82.5%). ES-API [M+H]⁺ = 367/369.

Step 3: Compound 81-7 (340 mg, 0.49 mmol) and compound 810-2 (150 mg, 0.41 mmol) were in dioxane (6 mL)/toluene (2 mL)/water (2 mL) under a nitrogen atmosphere, and potassium phosphate (260.09 mg, 1.225 mmol) and bis(triphenylphosphine)palladium(II) dichloride (260.09 mg, 1.225 mmol) were added. The mixture was stirred at 65 °C for 16 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-80% petroleum ether/ethyl acetate) to give compound 810-3 (155 mg, yield: 44%). ES-API [M+H]⁺ = 854.3.

Step 4: Compound 810-3 (85 mg, 0.1 mmol) was dissolved in N,N-dimethylformamide (5 mL), and cesium carbonate (97 mg, 0.3 mmol) and iodoethane (155 mg, 0.1 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by preparative thin-layer chromatography (dichloromethane/methanol = 20/1) to give compound 810-4 (50 mg, yield: 57%). ES-API [M+H]⁺ = 882.4.

Step 5: Trifluoroacetic acid (3 mL) was added to a solution of compound 810-4 (50 mg, 0.06 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 810-5 (40 mg, yield: 90%). ES-API [M+H]⁺ = 782.1.

Step 6: (1r,2R,3S)-2,3-Dimethylcyclopropane-1-carboxylic acid (7 mg, 0.06 mmol) and N,N-diisopropylethylenediamine (40 mg, 0.3 mmol) were added to a solution of compound 810-5 (40 mg, 0.05 mmol) in N,N-dimethylformamide (5 mL), and O-(7-azabenzotriazol-1-yl)-N,N,N',N'-tetramethyluronium hexafluorophosphate (38.8 mg, 0.1 mmol) was added under an ice bath. The mixture was stirred at room temperature for 1 h. After the reaction was completed, the mixture was extracted with ethyl acetate (20 mL × 3), and the combined extract was washed with brine (30 mL). The organic phase was dried over anhydrous sodium sulfate and concentrated, and then the residue was purified by preparative thin-layer chromatography (dichloromethane/methanol = 20/1) to give (1r,2R,3S)-N-((6³S,4S,Z)-1¹-ethyl-1²-(5-(3-(1,4-oxazepan-4-yl)-3-oxoprop-1-yn-1-yl)-2-((S)-1-methoxyethyl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z810A, 13 mg, yield: 29%, purity: 98%). ES-API [M+H]⁺ = 878.3.

### Example 93. Synthesis of Compounds Z780 and Z780-1

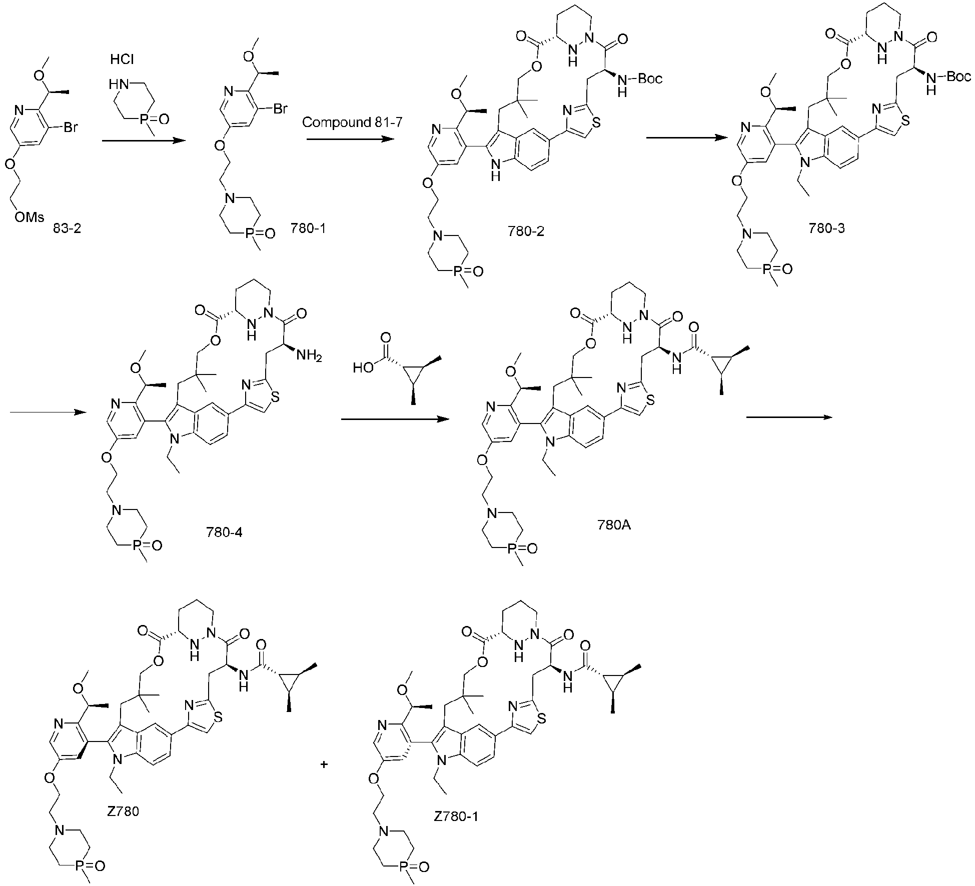

Step 1: Compound 83-2 (100 mg, 0.282 mmol) was dissolved in N,N-dimethylformamide (2 mL), and 4-methyl-1,4-azaphosphinane 4-oxide hydrochloride (71.82 mg, 0.423 mmol), N,N'-diisopropylethylamine (182.45 mg, 1.412 mmol), and sodium iodide (84.54 mg, 0.564 mmol) were added to the solution described above. After the addition was completed, the reaction system was stirred at 100 °C for 48 h. The reaction mixture was concentrated to remove N,N-dimethylformamide, and then dichloromethane (20 mL) was added. The insoluble substances were filtered using a sand core funnel, and the filtrate was concentrated to give a crude product. The crude product was purified by a flash silica gel column (methanol/dichloromethane: 0%-7%) to give compound 780-1 (90 mg, 0.230 mmol, yield: 81.48%) as a white solid. ES-API: [M+H]⁺ = 391.1, 393.1.

Step 2: Compound 780-1 (73.32 mg, 0.187 mmol) was dissolved in dioxane (2 mL), toluene (0.7 mL), and water (0.7 mL). Compound 81-7 (100 mg, 0.144 mmol), [1,1'-bis(di-tert-butylphosphino)ferrocene]dichloropalladium(II) (9.31 mg, 0.014 mmol), and potassium phosphate (92 mg, 0.432 mmol) were added to the solution described above at room temperature. After the addition was completed, the reaction system was stirred at 70 °C for 2 h under an argon atmosphere. After the reaction was completed, the reaction mixture was concentrated to remove the solvent, and then dichloromethane (20 mL) was added. The insoluble substances were filtered using a sand core funnel, and the filtrate was concentrated to give a crude product. The crude product was purified by a flash silica gel column (methanol/dichloromethane: 0%-8%) to give compound 780-2 (100 mg, 0.114 mmol, yield: 79.00%) as a white solid. ES-API: [M+H]⁺ = 878.1.

Step 3: Compound 780-2 (100 mg, 0.114 mmol) was dissolved in N,N-dimethylformamide (5 mL). Iodoethane (89 mg, 0.569 mmol) and cesium carbonate (186 mg, 0.569 mmol) were added to the solution described above. The reaction system was stirred at room temperature for 18 h. After the reaction was completed, water (20 mL) was added to the solution described above, and the mixture was extracted with ethyl acetate (10 mL × 3). The organic phase was washed with saturated brine (20 mL × 3), dried over anhydrous sodium sulfate, filtered, and concentrated to give a crude product. The crude product was purified by a flash silica gel column (methanol/dichloromethane: 0%-7%) to give compound 780-3 (100 mg, 0.110 mmol, yield: 96%) as a white solid. ES-API: [M+H]⁺ = 906.1.

Step 4: Compound 780-3 (100 mg, 0.110 mmol) was dissolved in methanol (1 mL), and a solution of hydrogen chloride in dioxane (4 M, 3 mL) was added to the solution described above. The mixture was stirred at room temperature for 2 h. After the reaction was completed, the reaction system was concentrated to give crude compound 780-4 (100 mg, crude product) as a white solid. ES-API: [M+H]⁺ = 806.1.

Step 5: Compound 780-4 (100 mg, crude product) was dissolved in dichloromethane (5 mL). (1r,2R,3S)-2,3-Dimethylcyclopropane-1-carboxylic acid (21.24 mg, 0.186 mmol), O-(7-azabenzotriazol-1-yl)-*N,N,N',N'-*tetramethyluronium hexafluorophosphate (94.35 mg, 0.248 mmol), and N,N'-diisopropylethylamine (48 mg, 0.372 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-5%) to give (1*r*,2*R*,3*S*)-N-((6³*S*,4*S*,*Z*)-1¹-ethyl-1²-(2-((*S*)-1-methoxyethyl)-5-(2-(4-methyl-4-oxido-1,4-azaphosphinan-1-yl)ethoxy)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (compound 780A, 70 mg, purity: 80%). Compound 780A was subjected to prep-HPLC (column type: Waters XBridge C18, 190 × 250 mm, 5 µm; mobile phase system: A: 0.1% aqueous ammonium bicarbonate solution; B: preparative grade acetonitrile; flow rate: 15 mL/min; B% = 20%-100%; column temperature: room temperature) to give 2 isomeric compounds. The structure of one of the isomeric compounds was arbitrarily designated as (1*r*,2*R*,3*S*)-N-((6³*S*,4*S*,*Z*)-1¹-ethyl-*R*₍ₐ₎-1²-(2-((*S*)-1-methoxyethyl)-5-(2-(4-methyl-4-oxido-1,4-azaphosphinan-1-yl)ethoxy)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z780, 18 mg, 0.020 mmol, yield: 18.18%, retention time = 7.60 min). ES-API: [M+H]⁺ = 902.3. ¹H NMR (400 MHz, DMSO-d₆) δ 8.50 (d, J = 1.2 Hz, 1H), 8.47 (d, J = 2.8 Hz, 1H), 8.39 (d, J = 9.0 Hz, 1H), 7.81 (s, 1H), 7.75 (dd, J = 8.8, 2.8 Hz, 1H), 7.58 (d, J = 8.4 Hz, 1H), 7.37 (d, J = 2.8 Hz, 1H), 5.56 (t, J = 8.8 Hz, 1H), 5.07 (d, J = 12.0 Hz, 1H), 4.36 - 4.06 (m, 7H), 3.58 (s, 2H), 3.32 - 3.30 (m, 1H), 3.23 (s, 3H), 3.20 - 3.12 (m, 1H), 3.04 - 2.92 (m, 3H), 2.86 (t, J = 5.6 Hz, 2H), 2.81 - 2.65 (m, 3H), 2.49 - 2.43 (m, 1H), 2.15 - 2.05 (m, 1H), 1.87 - 1.68 (m, 6H), 1.56 - 1.45 (m, 1H), 1.41 (d, J = 12.8 Hz, 3H), 1.35 (d, J = 6.0 Hz, 3H), 1.25 - 1.19 (m, 2H), 1.11 - 1.05 (m, 6H), 0.95 - 0.88 (m, 6H), 0.35 (s, 3H).

The structure of the other isomeric compound was arbitrarily designated as (1*r*,2*R*,3*S*)-N-((6³*S*,4*S,Z*)-1¹-ethyl-*S₍ₐ₎*-1²-(2-((*S*)-1-methoxyethyl)-5-(2-(4-methyl-4-oxido-1,4-azaphosphinan-1-yl)ethoxy)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z780-1, 24 mg, 0.027 mmol, yield: 24.55%, retention time = 7.84 min) as a white solid. ES-API: [M+H]⁺ = 902.3. ¹H NMR (400 MHz, DMSO-d₆) δ 8.54 (d, *J* = 1.2 Hz, 1H), 8.48 (d, *J* = 2.8 Hz, 1H), 8.40 (d, *J =* 9.2 Hz, 1H), 7.82 (s, 1H), 7.74 (dd, *J =* 8.8, 2.0 Hz, 1H), 7.56 - 7.51 (m, 2H), 5.55 (t, *J* = 8.8 Hz, 1H), 5.04 (d, *J =* 12.4 Hz, 1H), 4.29 - 4.12 (m, 4H), 4.00 - 3.80 (m, 3H), 3.69 (d, *J* = 10.8 Hz, 1H), 3.56 (d, *J* = 10.8 Hz, 1H), 3.31 - 3.27 (m, 1H), 3.22 - 3.12 (m, 1H), 3.09 (s, 3H), 3.03 - 2.90 (m, 2H), 2.86 (t, *J* = 5.8 Hz, 2H), 2.80 - 2.65 (m, 3H), 2.43 (d, *J* = 14.0 Hz, 1H), 2.17 - 2.10 (m, 1H), 1.88 - 1.69 (m, 6H), 1.61 - 1.49 (m, 1H), 1.42 (d, *J* = 13.2 Hz, 3H), 1.27 - 1.18 (m, 6H), 1.13 - 1.06(m, 8H), 0.94 (s, 3H), 0.53 (s, 3H).

### Example 94. Synthesis of Compounds Z814 and Z814-1

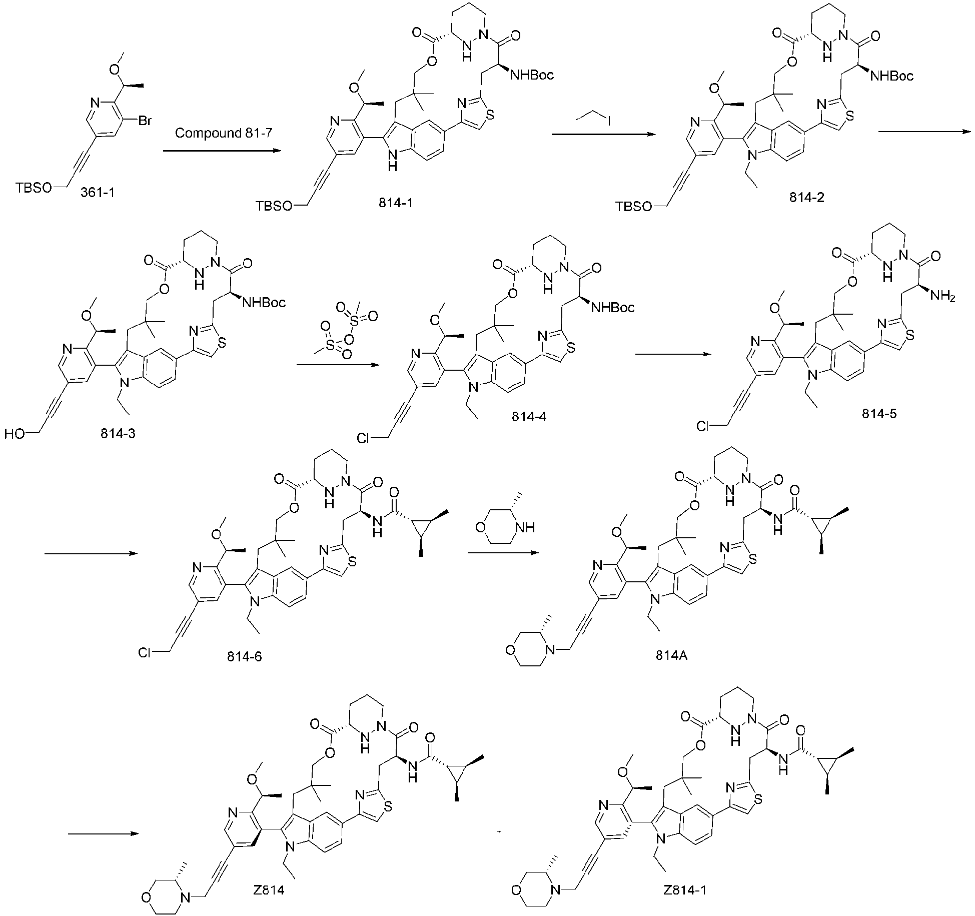

Step 1: Compound 81-7 (1166 mg, 1.68 mmol), compound 361-1 (776 mg, 2.018 mmol), potassium phosphate (1070 mg, 5.042 mmol), and [1,1'-bis(di-tert-butylphosphino)ferrocene]dichloropalladium(II) (218 mg, 0.336 mmol) were dissolved in dioxane (18 mL), toluene (6 mL), and water (6 mL) under a nitrogen atmosphere. The reaction mixture was heated to 70 °C under an oil bath and stirred for 2 h. When the reaction was completed as detected by LCMS, the mixture was cooled to room temperature. Ethyl acetate (30 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-40%) to give compound 814-1 (956 mg, yield: 65%). ES-API: [M+H]⁺ = 871.3.

Step 2: Cesium carbonate (3572 mg, 10.962 mmol) and iodoethane (0.88 mL, 10.962 mmol) were sequentially added to a solution of compound 814-1 (955 mg, 1.096 mmol) in N,N-dimethylformamide (10 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give crude compound 814-2 (1136 mg, yield: 99%). ES-API: [M+H]⁺ = 899.3.

Step 3: A tetrabutylammonium fluoride solution (3.670 mL, 3.670 mmol) was added to a solution of compound 814-2 (1100 mg, 1.233 mmol) in tetrahydrofuran (30 mL) under an ice-water bath. The reaction mixture was stirred at 0 °C for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to dryness to give crude compound 814-3 (960 mg, yield: 99%). ES-API: [M+H]⁺ = 785.3.

Step 4: N,N-Diisopropylethylamine (0.428 mL, 2.455 mmol) and methanesulfonic anhydride (128.29 mg, 0.736 mmol) were sequentially added to a solution of compound 814-3 (385.42 mg, 0.491 mmol) in dichloromethane (10 mL) under an ice-water bath. The reaction mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 814-4 (420.0 mg, crude product). ES-API: [M+H]⁺ = 803.2.

Step 5: Trifluoroacetic acid (5 mL) was added to a solution of compound 814-4 (420.0 mg, crude product) in dichloromethane (5 mL). The reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined and dried over anhydrous sodium sulfate. The mixture was filtered and concentrated, and the crude product was purified by automated flash chromatography on silica gel (methanol/dichloromethane = 0-6%) to give compound 814-5 (280.0 mg, yield: 81.08%). ES-API: [M+H]⁺ = 703.3.

Step 6: (1r,2R,3S)-2,3-Dimethylcyclopropane-1-carboxylic acid (69.0 mg, 0.597 mmol), N,N-diisopropylethylamine (0.35 mL, 1.991 mmol), and O-(7-azabenzotriazol-1-yl)-N,N,N',N'-tetramethyluronium hexafluorophosphate (227.0 mg, 0.597 mmol) were sequentially added to a solution of compound 814-5 (280.0 mg, 0.398 mmol) in N,N-dimethylformamide (10 mL) under an ice-water bath. The reaction mixture was warmed to room temperature and stirred for 10 min. When the reaction was completed as detected by LCMS, the reaction mixture was diluted with ethyl acetate (20 mL) and then washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give compound 814-6 (350.0 mg, crude product). ES-API: [M+H]⁺ = 799.3.

Step 7: Potassium carbonate (147.0 mg, 1.063 mmol) and 3-(S)-3-methylmorpholine (65.0 mg, 0.638 mmol) were sequentially added to a solution of compound 814-6 (170.0 mg, 0.213 mmol) in acetonitrile (10.0 mL). The reaction mixture was stirred at 70 °C overnight. When the reaction was completed as detected by LCMS, the mixture was concentrated under reduced pressure to remove the solvent to give crude compound 814A. The crude product was purified by prep-HPLC (ammonium bicarbonate method) to give 2 isomeric compounds. The structure of one of the isomeric compounds was arbitrarily designated as: (1r,2R,3S)-N-((4S,Z)-1¹-ethyl-(Rₐ)-1²-(2-((S)-1-methoxyethyl)-5-(3-((S)-3-methylmorpholino))prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z814, 12.9 mg, yield: 7.02%, retention time = 2.44 min), ES-API [M+H]⁺ = 864.4.

The structure of the other isomeric compound was arbitrarily designated as: (1r,2R,3S)-N-((4S,Z)-1¹-ethyl-(Sₐ)-1²-(2-((S)-1-methoxyethyl)-5-(3-((S)-3-methylmo^{r}pholino))prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z814-1, 20.3 mg, yield: 11.05%, retention time = 2.40 min), ES-API [M+H]⁺ = 864.4. ¹H NMR (400 MHz, DMSO-d6) δ 8.83 (s, 1H), 8.54 (s, 1H), 8.41 (d, *J* = 9.0 Hz, 1H), 8.01 (s, 1H), 7.82 (s, 1H), 7.76-7.74 (m, 1H), 7.56-7.54 (m, 1H), 5.57-5.52 (m, 1H), 5.06-5.03(m, 1H), 4.25-4.19 (m, 2H), 4.03 - 3.91 (m, 2H), 3.88 - 3.74 (m, 3H), 3.69-3.67(m, 3H), 3.57-3.54(m, 2H), 3.20 - 3.15 (m, 1H), 3.08 ~3.04(m, 4H), 2.85 - 2.73 (m, 2H), 2.35-2.31(m, 1H), 2.15-2.11 (m, 1H), 1.83-1.75 (m, 2H), 1.59 - 1.46 (m, 1H), 1.24-1.06(m, 19H), 0.94 (s, 6H), 0.50 (s, 3H).

### Example 95. Synthesis of Compounds Z813 and Z813-1

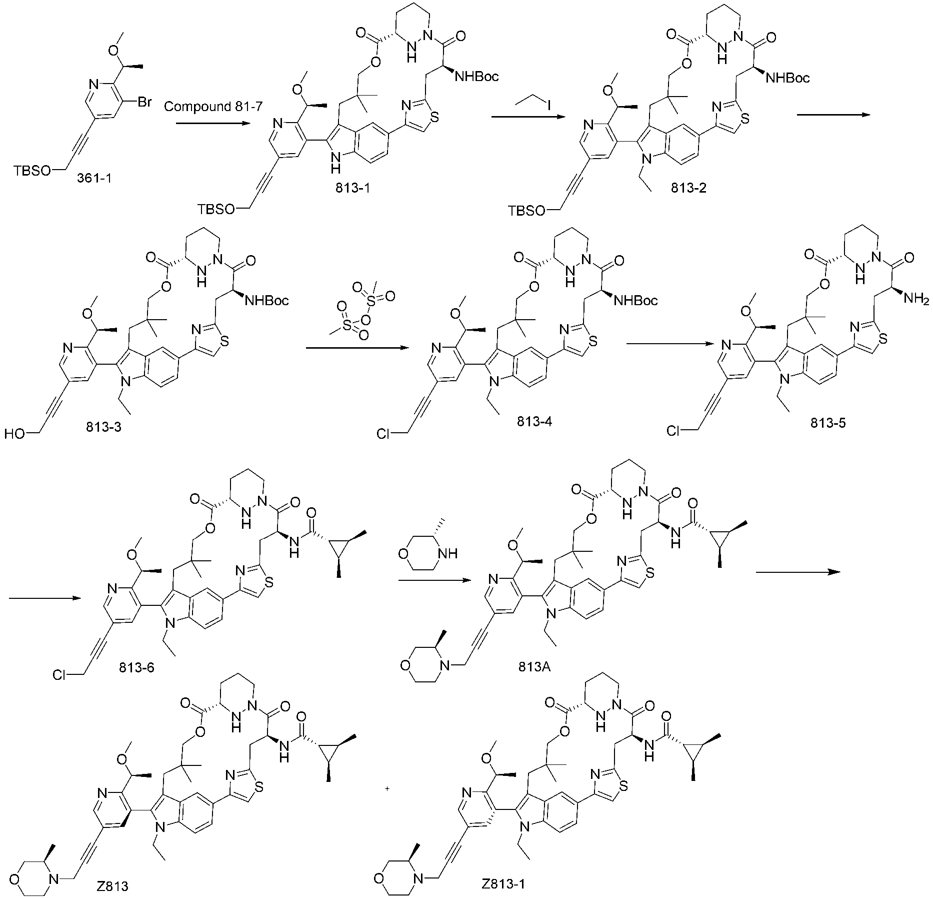

Step 1: Compound 81-7 (1166 mg, 1.68 mmol), compound 361-1 (776 mg, 2.018 mmol), potassium phosphate (1070 mg, 5.042 mmol), and [1,1'-bis(di-tert-butylphosphino)ferrocene]dichloropalladium(II) (218 mg, 0.336 mmol) were dissolved in dioxane (18 mL), toluene (6 mL), and water (6 mL) under a nitrogen atmosphere. The reaction mixture was heated to 70 °C under an oil bath and stirred for 2 h. When the reaction was completed as detected by LCMS, the mixture was cooled to room temperature. Ethyl acetate (30 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-40%) to give compound 813-1 (956 mg, yield: 65%). ES-API: [M+H]⁺ = 871.3.

Step 2: Cesium carbonate (3572 mg, 10.962 mmol) and iodoethane (0.88 mL, 10.962 mmol) were sequentially added to a solution of compound 813-1 (955 mg, 1.096 mmol) in N,N-dimethylformamide (10 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give crude compound 813-2 (1136 mg, yield: 99%). ES-API: [M+H]⁺ = 899.3.

Step 3: A tetrabutylammonium fluoride solution (3.670 mL, 3.670 mmol) was added to a solution of compound 813-2 (1100 mg, 1.233 mmol) in tetrahydrofuran (30 mL) under an ice-water bath. The reaction mixture was stirred at 0 °C for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to dryness to give crude compound 813-3 (960 mg, yield: 99%). ES-API: [M+H]⁺ = 785.3.

Step 4: N,N-Diisopropylethylamine (0.428 mL, 2.455 mmol) and methanesulfonic anhydride (128.29 mg, 0.736 mmol) were sequentially added to a solution of compound 813-3 (385.42 mg, 0.491 mmol) in dichloromethane (10 mL) under an ice-water bath. The reaction mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 813-4 (420.0 mg, crude product). ES-API: [M+H]⁺ = 803.2.

Step 5: Trifluoroacetic acid (5 mL) was added to a solution of compound 813-4 (420.0 mg, crude product) in dichloromethane (5 mL). The reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined and dried over anhydrous sodium sulfate. The mixture was filtered and concentrated, and the crude product was purified by automated flash chromatography on silica gel (methanol/dichloromethane = 0-6%) to give compound 813-5 (280.0 mg, yield: 81.08%). ES-API: [M+H]⁺ = 703.3.

Step 6: (1r,2R,3S)-2,3-Dimethylcyclopropane-1-carboxylic acid (69.0 mg, 0.597 mmol), N,N-diisopropylethylamine (0.35 mL, 1.991 mmol), and O-(7-azabenzotriazol-1-yl)-N,N,N',N'-tetramethyluronium hexafluorophosphate (227.0 mg, 0.597 mmol) were sequentially added to a solution of compound 813-5 (280.0 mg, 0.398 mmol) in N,N-dimethylformamide (10 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give compound 813-6 (350.0 mg, crude product). ES-API: [M+H]⁺ = 799.3.

Step 7: Potassium carbonate (165.0 mg, 1.188 mmol) and 3-(S)-3-methylmorpholine (72.12 mg, 0.713 mmol) were sequentially added to a solution of compound 813-6 (190.0 mg, 0.238 mmol) in acetonitrile (10.0 mL). The reaction mixture was stirred at 70 °C overnight. When the reaction was completed as detected by LCMS, the mixture was concentrated under reduced pressure to remove the solvent to give crude compound 813A. The crude product was purified by prep-HPLC (ammonium bicarbonate method) to give 2 isomeric compounds. The structure of one of the isomeric compounds was arbitrarily designated as: (1r,2R,3S)-N-((4S,Z)-1¹-ethyl-(Rₐ)-1²-(2-((S)-1-methoxyethyl)-5-(3-((R)-3-methylmorpholino))prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z813, 15.53 mg, yield: 7.56%, retention time = 2.32 min), ES-API [M+H]⁺ = 864.4.

The structure of the other isomeric compound was arbitrarily designated as: (1r,2R,3S)-N-((4S,Z)-1¹-ethyl-(Sₐ)-1²-(2-((S)-1-methoxyethyl)-5-(3-((R)-3-methylmorpholino))prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z813-1, 31.09 mg, yield: 15.14%, retention time = 2.28 min), ES-API [M+H]⁺ = 864.4. ¹H NMR (400 MHz, DMSO-d6) δ 8.83 (s, 1H), 8.54 (s, 1H), 8.41 (d, *J* = 9.0 Hz, 1H), 8.00 (s, 1H), 7.82 (s, 1H), 7.76-7.73(m, 1H), 7.56-7.54 (m, 1H), 5.57-5.52 (m, 1H), 5.06-5.03 (m, 1H), 4.25-4.19 (m, 2H), 3.99-3.93 (m, 2H), 3.88 - 3.74 (m, 3H), 3.68-3.65 (m, 3H), 3.60 - 3.46 (m, 2H), 3.20 - 3.13 (m, 1H), 3.12 - 3.01 (m, 5H), 2.77-2.70 (m, 2H), 2.34-2.31 (m, 1H), 2.15-2.11 (m, 1H), 1.83-1.75 (m, 2H), 1.59 - 1.44 (m, 1H), 1.29 - 1.05 (m, 19H), 0.94 (s, 6H), 0.50 (s, 3H).

### Example 96. Synthesis of Compounds Z778 and Z778-1

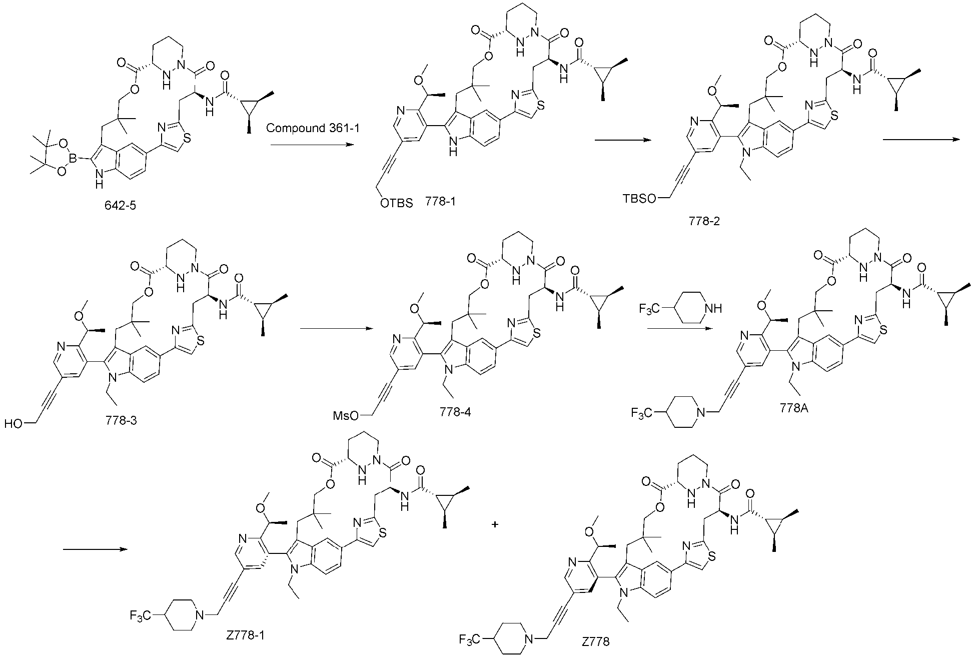

Step 1: Compound 642-5 (200 mg, 0.290 mmol) was dissolved in dioxane (3 mL), toluene (9 mL), and water (3 mL), and compound 361-1 (133 mg, 0.348 mmol), [1,1'-bis(di-tert-butylphosphino)ferrocene]dichloropalladium(II) (18.88 mg, 0.029 mmol), and potassium phosphate (184 mg, 0.870 mmol) were added to the solution described above at room temperature. After the addition was completed, the reaction system was stirred at 70 °C for 1 h under a nitrogen atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 778-1 (180 mg, 0.208 mmol, yield: 71.58%) as a yellow solid. ES-API: [M+H]⁺ = 867.3.

Step 2: Compound 778-1 (180 mg, 0.166 mmol) was dissolved in N,N-dimethylformamide (1 mL), and cesium carbonate (162 mg, 0.498 mmol) and iodoethane (77 mg, 0.498 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, filtered, and concentrated under reduced pressure to give compound 778-2 (130 mg, 0.145 mmol, yield: 87.45%) as a yellow solid. ES-API: [M+H]⁺ = 895.3.

Step 3: Compound 778-2 (130 mg, 0.145 mmol) was dissolved in tetrahydrofuran (1 mL), and tetrabutylammonium fluoride (0.19 mL, 0.19 mmol) was added. The mixture was reacted at room temperature for 2 h. The mixture was concentrated under reduced pressure, and the residue was purified by a flash silica gel column (methanol/dichloromethane: 0%-3%) to give compound 778-3 (110 mg, 0.141 mmol, yield: 96.99%) as a yellow solid. ES-API: [M+H]⁺ = 781.3.

Step 4: Compound 778-3 (110 mg, 0.141 mmol) was dissolved in dichloromethane (5 mL), and methanesulfonic anhydride (29.44 mg, 0.169 mmol) and N,N-diisopropylethylamine (0.070 mL, 0.423 mmol) were added. The mixture was reacted at room temperature for 3 h. The mixture was concentrated under reduced pressure, and the residue was purified by a flash silica gel column (methanol/dichloromethane: 0%-3%) to give compound 778-4 (115 mg, 0.134 mmol, yield: 95.04%) as a yellow solid. ES-API: [M+H]⁺ = 859.2.

Step 5: Compound 778-7 (120 mg, 0.140 mmol) was dissolved in dichloromethane (5 mL), and 4-(trifluoromethyl)piperidine (64 mg, 0.420 mmol) and N,N-diisopropylethylamine (90 mg, 0.700 mmol) were added. The mixture was reacted at room temperature for 18 h. After the reaction was completed, water (15 mL) was added, and the mixture was extracted with dichloromethane (10 mL × 3). The organic phase was washed with saturated brine (15 mL), dried over anhydrous sodium sulfate, filtered, and concentrated under reduced pressure to give crude compound 778A. The crude product was purified by prep-HPLC (ammonium bicarbonate method) to give 2 isomeric compounds. The structure of one of the isomeric compounds was arbitrarily designated as (1r,2R,3S)-*N*-((6³*S*,4*S*,*Z*)-1¹-ethyl-(*Sₐ*)-1²-(2-((S)-1-methoxyethyl-5-(3-(4-(trifluoromethyl)piperidin-1-yl)prop-1-yn-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z778-1, 25 mg, 0.027 mmol, yield: 19.54%, retention time = 2.47 min) as a white solid. ES-API: [M+H]⁺ = 916.3.

The structure of the other isomeric compound was arbitrarily designated as (1r,2R,3S)-*N*-((6³*S*,4*S*,*Z*)-1¹-ethyl-(Rₐ)-1²-(2-((S)-1-methoxyethyl-5-(3-(4-(trifluoromethyl)piperidin-1-yl)prop-1-yn-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z778, 20 mg, 0.022 mmol, yield: 15.59%, retention time = 2.52 min) as a white solid. ES-API: [M+H]⁺ = 916.3.

### Example 97. Synthesis of Compound Z793

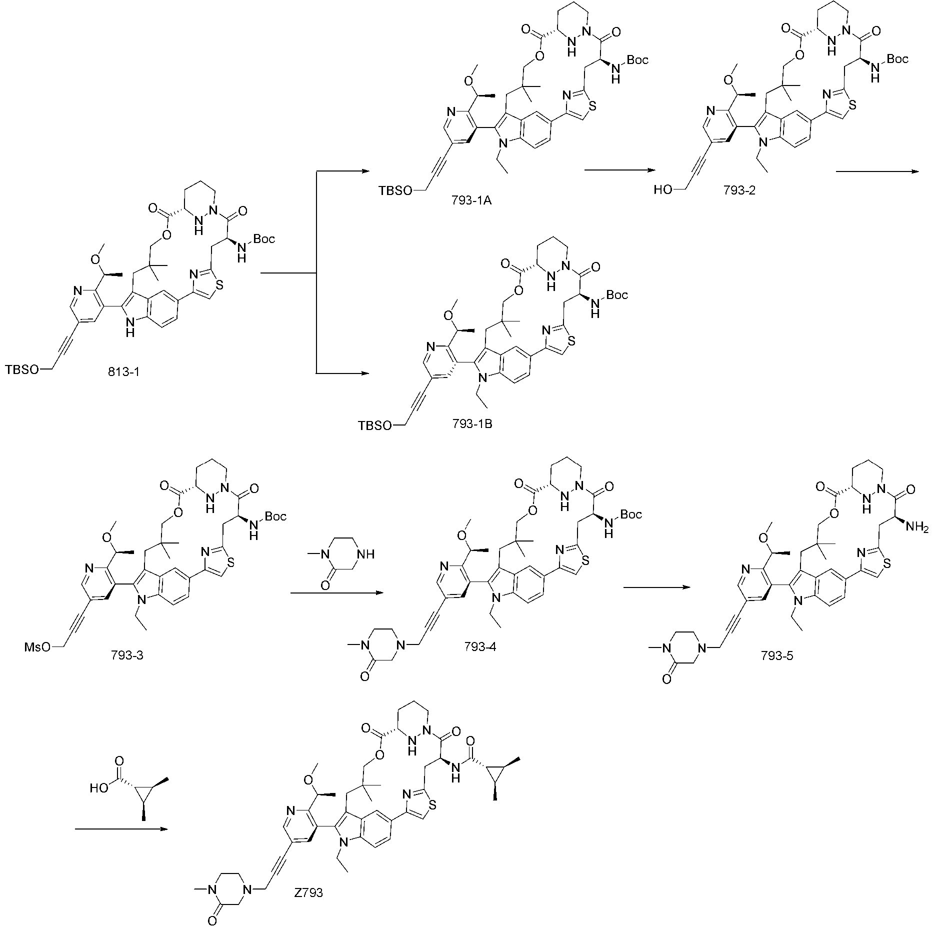

Step 1: Cesium carbonate (1481.03 mg, 4.546 mmol) and iodoethane (0.606 mL, 7.576 mmol) were sequentially added to a solution of compound 813-1 (1320 mg, 1.515 mmol) in N,N-dimethylformamide (10 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness to give a crude product. The crude product was then purified by silica gel column chromatography (petroleum ether/ethyl acetate = 1/1) to give two isomeric compounds. The structure of one of the isomeric compounds was arbitrarily designated as: compound 793-1A (430 mg, yield: 31.56%, retention time = 3.491 min). ES-API: [M+H]⁺ = 899.3.

The structure of the other isomeric compound was arbitrarily designated as compound 793-1B (800 mg, yield: 58.72%, retention time = 3.257 min). ES-API: [M+H]⁺ = 899.3.

Step 2: A tetrabutylammonium fluoride solution (4 mL, a 1.0 M solution in tetrahydrofuran) was added to compound 793-1A (430 mg, 0.478 mmol) under an ice-water bath. The reaction mixture was stirred at room temperature for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to dryness to give crude compound 793-2 (350 mg, 0.446 mmol, yield: 93.24%). ES-API: [M+H]⁺ = 785.3. Step 3: N,N-Diisopropylethylamine (172.89 mg, 1.338 mmol) and methanesulfonic anhydride (116.50 mg, 0.669 mmol) were sequentially added to a solution of compound 793-2 (350 mg, 0.446 mmol) in dichloromethane (10 mL) under an ice-water bath. The reaction mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 793-3 (340 mg, 0.394 mmol, yield: 88.35%). ES-API: [M+H]⁺ = 863.2.

Step 4: 1-Methylpiperazin-2-one (19.84 mg, 0.174 mmol) was added to a solution of compound 793-3 (50 mg, 0.058 mmol) in dichloromethane (5 mL). The reaction mixture was stirred at room temperature for 1.5 h. When the reaction was completed as detected by LCMS, the mixture was concentrated. The crude product was purified by automated flash chromatography on silica gel (methanol/dichloromethane = 0-6%) to give compound 793-4 (50 mg, 0.057 mmol, yield: 97.94%). ES-API: [M+H]⁺ = 881.4.

Step 5: Hydrochloric acid (3 mL, a 4 M solution in dioxane) was added to a solution of compound 793-4 (50 mg, 0.057 mmol) in methanol (1 mL) under an ice-water bath, and the mixture was reacted at room temperature for 1 h. The reaction mixture was concentrated to give crude compound 793-5 (50 mg, hydrochloride), which was directly used in the next step.

Step 6: (1r,2R,3S)-2,3-Dimethylcyclopropane-1-carboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of the hydrochloride (50 mg) of crude compound 793-5 described above in dichloromethane (5 mL) under an ice-water bath. The reaction mixture was warmed to room temperature and stirred for 1 h. When the reaction was completed as detected by LCMS, the reaction mixture was diluted with dichloromethane (20 mL), and then the mixture was washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give: (1r,2R,3S)-N-((6³S,4S,Z)-(Rₐ)-1²-(5-(3-(4-methyl-3-oxopiperazin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z793, 24.7 mg, 0.028 mmol, yield: 43.99%).

### Example 98. Synthesis of Compounds Z703 and Z703-1

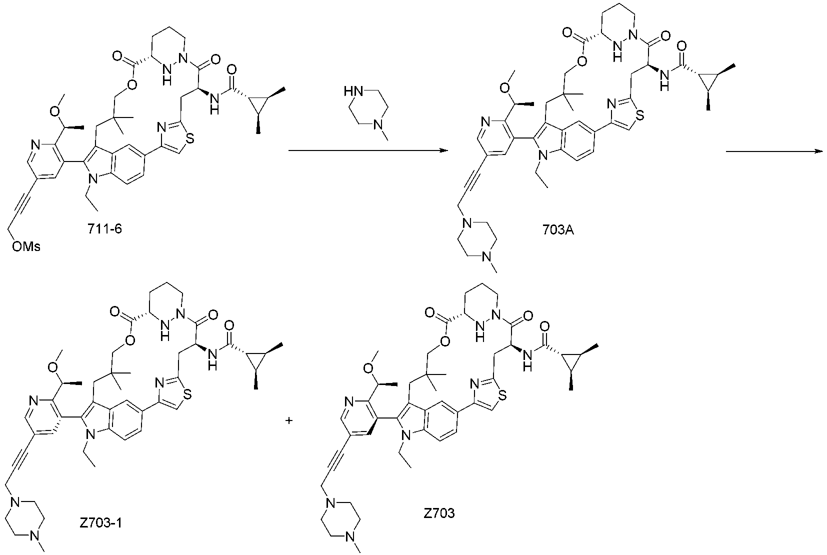

Step 1: N,N-Diisopropylethylamine (0.13 mL, 0.728 mmol) and N-methylpiperazine (29 mg, 0.291 mmol) were sequentially added to a solution of compound 711-6 (125 mg, 0.146 mmol) in dichloromethane (6 mL). The reaction mixture was stirred at room temperature overnight. When the reaction was completed as detected by LCMS, the mixture was concentrated under reduced pressure to remove the solvent to give crude compound 703A. The crude product was purified by prep-HPLC (chromatographic column: CHIRALPAK^{®} IG, 10 µm, 30 × 250 mm; mobile phase A: HEX + 0.2% DEA, mobile phase B: ETOH + 0.2% DEA; flow rate: 25 mL/min; isocratic elution program: mobile phase A:mobile phase B = 60:40 (V/V); column temperature: room temperature) to give 2 isomeric compounds. One of the isomeric compounds was arbitrarily designated as: (1r,2R,3S)-N-((6³S,4S,Z)-1¹-ethyl-(Sₐ)-1²-2-((S)-1-methoxyethyl)-5-(3-(4-methylpiperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z703-1, 16.47 mg, yield: 13%, retention time = 4.642 min), ES-API [M+H]⁺ = 863.3. ¹H NMR (400 MHz, DMSO-d₆) δ 8.82 (d,J= 2.4 Hz, 1 H), 8.53 (s, 1 H), 8.41 (d, J= 8.8 Hz, 1H), 7.99 (d, J= 2.0 Hz, 1 H), 7.82 (s, 1 H), 7.74 (dd, J1= 1.6 Hz, J2= 8.8 Hz, 1 H), 7.55 (d, J= 8.8 Hz, 1 H), 5.54 (t, J= 9.2 Hz, 1 H), 5.04 (d, J= 12.0 Hz, 1 H), 4.25- 4.18 (m, 2 H), 3.98- 3.80 (m, 3 H), 3.67 (d, J= 10.8 Hz, 1 H), 3.57 (s, 3 H), 3.19- 3.03 (m, 6 H), 2.80- 2.74 (m, 1 H), 2.56- 2.31 (m, 7 H), 2.22- 2.11 (m, 5 H), 1.82- 1.76 (m, 2 H), 1.54- 1.50 (m, 1 H), 1.26- 1.06 (m, 16 H), 0.93 (s, 3 H), 0.50 (s, 3 H).

The structure of the other isomeric compound was arbitrarily designated as: (1r,2R,3S)-N-((6³S,4S,Z)-1¹-ethyl-(Rₐ)-1²-2-((S)-1-methoxyethyl)-5-(3-(4-methylpiperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z703, 11.42 mg, yield: 9%, retention time = 6.326 min), ES-API [M+H]⁺ = 863.3.

### Example 99. Synthesis of Compounds Z811-1 and Z811-2

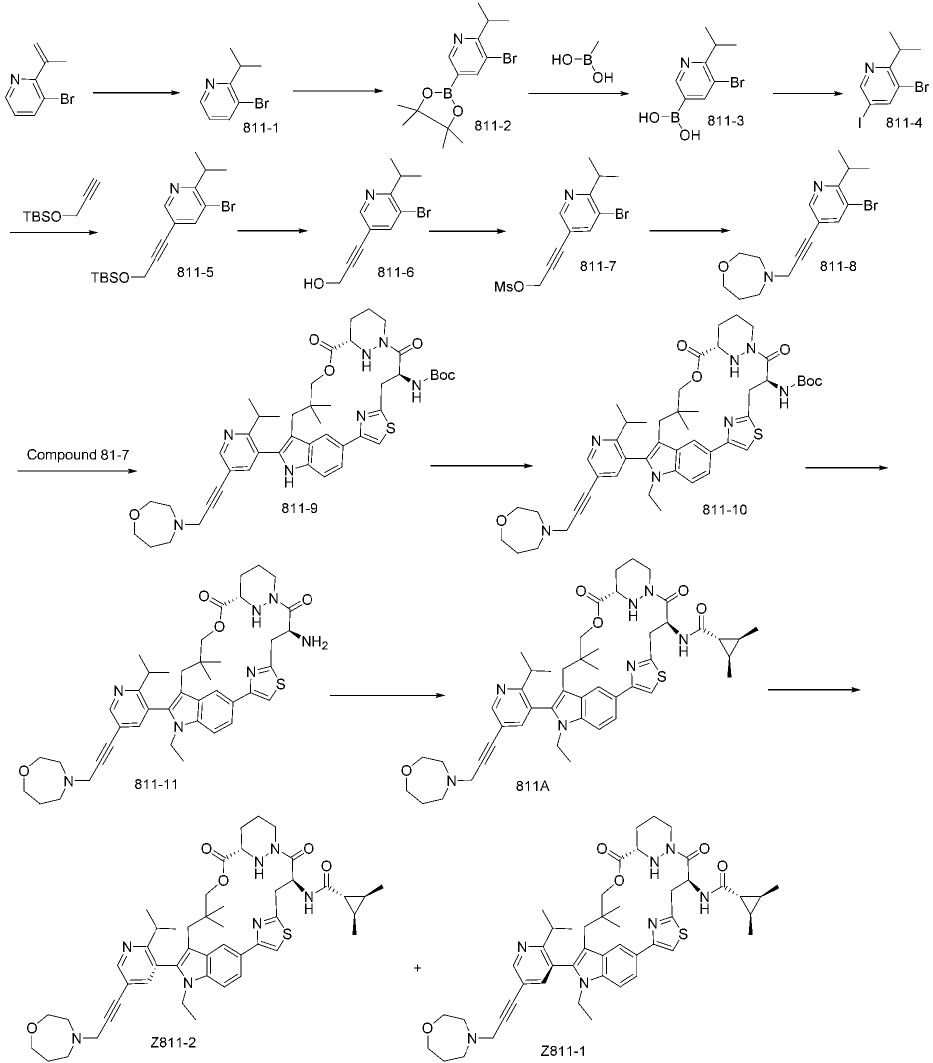

Step 1: 3-Bromo-2-(prop-1-en-2-yl)pyridine (2.7 g, 13.632 mmol) was dissolved in ethyl acetate (30 mL), and platinum(IV) oxide (0.93 g, 4.090 mmol) was added. The mixture was reacted at room temperature for 48 h under a hydrogen atmosphere. The mixture was filtered and concentrated, and the residue was purified by silica gel column chromatography (petroleum ether/ethyl acetate = 10/1) to give compound 811-1 (1.6 g, 7.997 mmol, yield: 58.66%). ES-API: [M+H]⁺ = 200.3/202.3.

Step 2: Compound 811-1 (1.6 g, 7.997 mmol) and bis(pinacolato)diboron (3050.00 mg, 11.995 mmol) were dissolved in tetrahydrofuran (30 mL), and chloro(1,5-cyclooctadiene)iridium(I) dimer (270.00 mg, 0.400 mmol) and 4,4'-di-tert-butyl-2,2'-bipyridine (260.00 mg, 0.960 mmol) were added. The mixture was heated to 75 °C and maintained for 16 h under a nitrogen atmosphere. The mixture was concentrated under reduced pressure, and the residue was dissolved in ethyl acetate (50 mL). The resulting mixture was washed with water (30 mL) and saturated brine (30 mL × 3). The organic phase was dried over anhydrous sodium sulfate, filtered, and concentrated to dryness to give a crude product. The crude product was purified by silica gel column chromatography (petroleum ether/ethyl acetate = 10/1) to give compound 811-2 (1.5 g, 6.150 mmol, yield: 76.91%) as a white solid. ES-API: [M-82+H]⁺ = 244.0/246.0.

Step 3: Compound 811-2 (1.1 g, 3.374 mmol) was dissolved in dichloromethane (20 mL), and methylboronic acid (0.61 g, 10.121 mmol) and trifluoroacetic acid (1 mL, 13.059 mmol) were sequentially added. The mixture was reacted at room temperature for 3 h. The mixture was concentrated under reduced pressure, and the residue was dissolved in ethyl acetate (50 mL). The resulting mixture was washed with sodium bicarbonate (30 mL × 1). The organic phase was dried over anhydrous sodium sulfate, filtered, and concentrated, and the residue was purified by silica gel column chromatography (petroleum ether/ethyl acetate = 1/1) to give compound 811-3 (822.87 mg, 3.374 mmol, yield: 100%) as a white solid. ES-API: [M+H]⁺ = 244.0/246.0.

Step 4: Compound 811-3 (500 mg, 2.050 mmol) was dissolved in acetonitrile (10 mL), and N-iodosuccinimide (1383.70 mg, 6.150 mmol) was added. The reaction mixture was stirred at 80 °C for 17 h under a nitrogen atmosphere. The mixture was concentrated under reduced pressure, and the residue was dissolved in ethyl acetate (50 mL). The resulting mixture was washed with saturated brine (30 mL × 1). The organic phase was dried over anhydrous sodium sulfate, filtered, and concentrated, and the residue was purified by silica gel column chromatography (petroleum ether/ethyl acetate = 10/1) to give compound 811-4 (300 mg, 0.920 mmol, yield: 44.89%) as a white solid. ES-API: [M+H]⁺ = 325.9/327.9.

Step 5: Compound 811-4 (250 mg, 0.767 mmol) and tert-butyldimethyl(2-propynyloxy)silane (156.76 mg, 0.920 mmol) were dissolved in acetonitrile (5 mL), and N,N-diisopropylethylamine (197.86 mg, 1.534 mmol), bis(triphenylphosphine)palladium(II) dichloride (53.83 mg, 0.077 mmol), and copper(I) iodide (29.21 mg, 0.153 mmol) were sequentially added. The reaction mixture was stirred at room temperature for 2 h under a nitrogen atmosphere. After the reaction was completed, the mixture was concentrated under reduced pressure, and the residue was dissolved in ethyl acetate (50 mL). The resulting mixture was washed with saturated brine (30 mL × 1). The organic phase was dried over anhydrous sodium sulfate, filtered, and concentrated, and the residue was purified by silica gel column chromatography (petroleum ether/ethyl acetate = 10/1) to give compound 811-5 (250 mg, 0.679 mmol, yield: 88.49%) as a white solid. ES-API: [M+H]⁺ = 368.1/370.1.

Step 6: A tetrabutylammonium fluoride solution (4 mL, a 1.0 M solution in tetrahydrofuran) was added to compound 811-5 (115 mg, 0.312 mmol) under an ice-water bath. The reaction mixture was stirred at room temperature for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated, and the residue was purified by silica gel column chromatography (petroleum ether/ethyl acetate = 4/1) to give compound 811-6 (50 mg, 0.197 mmol, yield: 63.34%). ES-API: [M+H]⁺ = 254.0/256.0.

Step 7: N,N-Diisopropylethylamine (127.31 mg, 0.985 mmol) and methanesulfonic anhydride (68.56 mg, 0.394 mmol) were added to a solution of compound 811-6 (50 mg, 0.197 mmol) in dichloromethane (5 mL). The mixture was stirred at 25 °C for 1 h. When the reaction was completed as detected by LCMS, the reaction mixture was diluted with dichloromethane (20 mL), and then the mixture was washed with saturated brine (15 mL × 3). The organic phase was dried over anhydrous sodium sulfate, filtered, and concentrated, and the residue was purified by silica gel column chromatography (petroleum ether/ethyl acetate = 3/1) to give compound 811-7 (60 mg, 0.181 mmol, yield: 91.80%). ES-API: [M+H]⁺ = 332.0/334.0.

Step 8: 1,4-Oxazepane (54.92 mg, 0.543 mmol) was added to a solution of compound 811-7 (60 mg, 0.181 mmol) in dichloromethane (5 mL), and the mixture was stirred at 40 °C for 1 h. When the reaction was completed as detected by LCMS, the mixture was concentrated, and the residue was purified by silica gel column chromatography (petroleum ether/ethyl acetate = 3/1) to give product compound 811-8 (50 mg, 0.148 mmol, yield: 82.09%). ES-API: [M+H]⁺ = 337.1/339.1.

Step 9: [1,1'-Bis(di-tert-butylphosphino)ferrocene]dichloropalladium(II) (9.36 mg, 0.0144 mmol) and potassium carbonate (59.77 mg, 0.432 mmol) were added to a solution of compound 81-7 (100 mg, 0.144 mmol) and compound 811-8 (50 mg, 0.148 mmol) in water (1 mL) and tetrahydrofuran (4 mL). The mixture was degassed with nitrogen and stirred at 70 °C for 1 h. The mixture was cooled to room temperature, poured into water (30 mL), and extracted with ethyl acetate (30 mL × 3). The ethyl acetate layers were combined, washed with a saturated sodium chloride solution (30 mL × 1), dried over anhydrous sodium sulfate, filtered, and concentrated. The residue was purified by silica gel column chromatography (methanol/dichloromethane = 1/30) to give compound 811-9 (60 mg, 0.073 mmol, yield: 50.56%), ES-API: [M+H]⁺ = 824.3. Step 10: Cesium carbonate (118.62 mg, 0.364 mmol) and iodoethane (0.058 mL, 0.728 mmol) were sequentially added to a solution of compound 811-9 (60 mg, 0.073 mmol) in N,N-dimethylformamide (3 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated, and the residue was purified by silica gel column chromatography (methanol/dichloromethane = 1/30) to give compound 811-10 (50 mg, 0.059 mmol, yield: 80.59%), ES-API: [M+H]⁺ = 852.4.

Step 11: Hydrochloric acid (3 mL, a 4 M solution in dioxane) was added to a solution of compound 811-10 (50 mg, 0.059 mmol) in methanol (1 mL), and the mixture was reacted at room temperature for 1 h. The reaction mixture was concentrated to give a hydrochloride of crude compound 811-11 (50 mg), which was directly used in the next step. ES-API: [M+H]⁺ = 752.4.

Step 12: (1r,2R,3S)-2,3-Dimethylcyclopropane-1-carboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of the hydrochloride (50 mg) of compound 811-11 described above in dichloromethane (5 mL) under an ice-water bath. The reaction mixture was warmed to room temperature and stirred for 1 h. When the reaction was completed as detected by LCMS, the reaction mixture was diluted with dichloromethane (20 mL), and then the mixture was washed with water (10 mL) and saturated brine (10 mL × 3). The organic phase was dried over anhydrous sodium sulfate, filtered, and concentrated to dryness to give crude compound 811A. The crude product was purified by prep-HPLC (ammonium bicarbonate method) to give 2 isomers. The structure of one of the isomeric compounds was arbitrarily designated as (1r,2R,3S)-N-((6³S,4S,Z)-(Sₐ)-1²-(5-(3-(1,4-oxazepan-4-yl)prop-1-yn-1-yl)-2-isopropylpyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z811-2, 4.5 mg, yield: 7.57%, retention time: = 2.487 min in LCMS 3 min 0.1% NH₄HCO₃), ES-API [M+H]⁺ = 848.4. ¹H NMR (400 MHz, DMSO-*d*₆) δ 8.78 (d, *J* = 2.2 Hz, 1H), 8.52 (d, *J =* 1.6 Hz, 1H), 8.37 (d, *J* = 8.9 Hz, 1H), 7.82 (s, 1H), 7.79 - 7.69 (m, 2H), 7.56 (d, *J =* 8.7 Hz, 1H), 5.63 (t, *J=* 8.9 Hz, 1H), 5.12 (d, *J* = 12.2 Hz, 1H), 4.30-4.20 (m, 2H), 4.13 (t, *J* = 12.0 Hz, 1H), 3.79 - 3.56 (m, 9H), 3.32-3.26 (m, 1H), 3.15 (dd, *J =* 14.7, 9.0 Hz, 1H), 3.02 - 2.82 (m, 2H), 2.80-2.66 (m, 5H), 2.57-252 (m, 1H), 2.04 (d, *J =* 12.1 Hz, 1H), 1.88 - 1.66 (m, 4H), 1.58-1.46 (m, 1H), 1.31 (d, *J =* 6.5 Hz, 3H), 1.21 - 1.10 (m, 6H), 1.1-1.05 (m, 6H), 0.98 (t, *J =* 7.0 Hz, 3H), 0.88 (s, 3H), 0.53 (s, 3H).

The structure of the other isomeric compound was arbitrarily designated as (1r,2R,3S)-N-((6³S,4S,Z)-(Rₐ)-1²-(5-(3-(1,4-oxazepan-4-yl)prop-1-yn-1-yl)-2-isopropylpyridin-3-yl)-1¹-ethyl-10,10-dimethyl-5,7-dioxo-6¹,6²6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z811-1, 7.5 mg, 13.3%, retention time = 2.606 min in LCMS 3 min 0.1% NH₄HCO₃), ES-API [M+H]⁺ = 848.4. ¹H NMR (400 MHz, DMSO-*d*₆) δ 8.78 (d, *J* = 2.1 Hz, 1H), 8.51 (d, *J* = 1.6 Hz, 1H), 8.42 (d, *J* = 9.0 Hz, 1H), 7.90 (d, *J* = 2.2 Hz, 1H), 7.82 (s, 1H), 7.74 (dd, *J* = 8.7, 1.7 Hz, 1H), 7.55 (d, *J* = 8.7 Hz, 1H), 5.55 (t, *J =* 9.1 Hz, 1H), 5.05 (d, *J =* 12.1 Hz, 1H), 4.33 - 4.12 (m, 2H), 4.00 (dd, *J* = 14.6, 7.2 Hz, 1H), 3.92 - 3.77 (m, 1H), 3.74 - 3.60 (m, 7H), 3.56 (d, *J* = 10.9 Hz, 1H), 3.29 (s, 1H), 3.17 (dd, *J =* 14.7, 9.2 Hz, 1H), 3.06 (d, *J* = 14.3 Hz, 1H), 2.80-2.73 (m, 5H), 2.74-2.63 (m, 1H), 2.15-2.04 (m, 2H), 1.89-1.75 (m, 4H), 1.55-1.50 (m, 1H), 1.25 - 1.16 (m, 9H), 1.09 (dd, *J* = 11.7, 5.6 Hz, 6H), 1.00 (d, *J* = 6.7 Hz, 3H), 0.95 (s, 3H), 0.49 (s, 3H).

### Example 100. Synthesis of Compounds Z836-1 and Z836-2

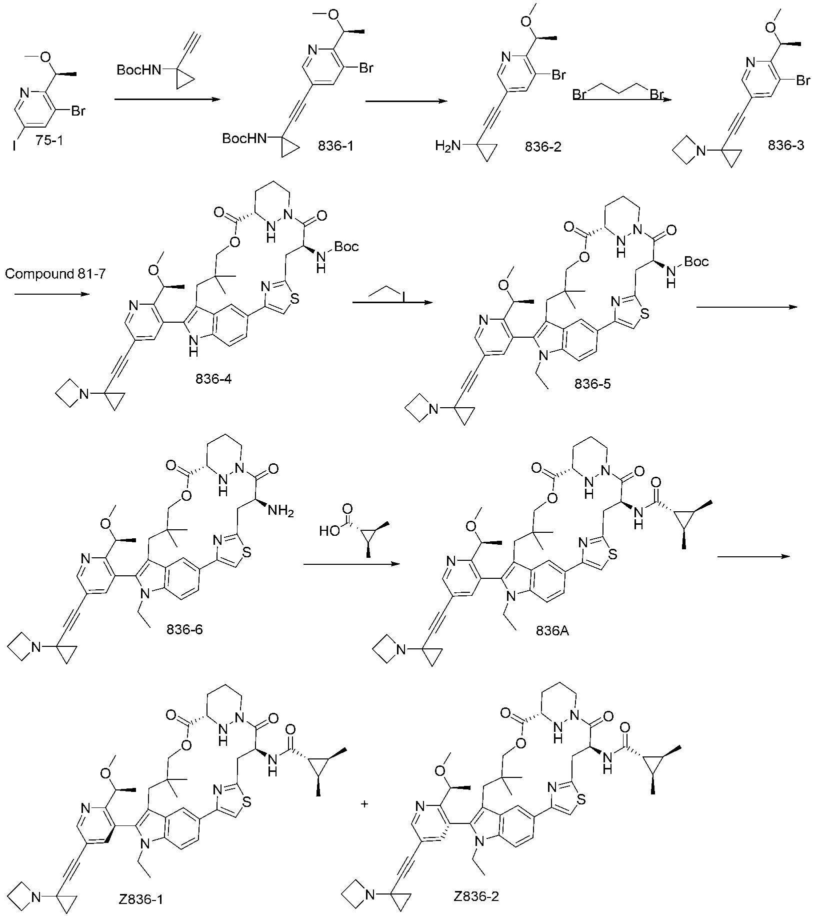

Step 1: Compound 75-1 (4.0 g, 11.697 mmol), tert-butyl (1-ethynylcyclopropyl)carbamate (2.12 g, 11.697 mmol), bis(triphenylphosphine)palladium(II) dichloride (0.41 g, 0.585 mmol), copper(I) iodide (0.22 g, 1.170 mmol), and N,N-diisopropylethylamine (4.0 mL, 23.394 mmol) were dissolved in anhydrous acetonitrile (20 mL) under a nitrogen atmosphere. The reaction mixture was stirred at room temperature overnight. When the reaction was completed as detected by LCMS, the mixture was concentrated under reduced pressure to remove the solvent. The residue was purified by automated flash chromatography on silica gel (ethyl acetate/petroleum ether = 0-10%) to give compound 836-1 (3.2 g, yield: 69.21%). ES-API: [M+H]⁺ = 395.0/397.0.

Step 2: Trifluoroacetic acid (5.0 mL) was slowly added dropwise to a solution of compound 836-1 (1.2 g, 3.036 mmol) in dichloromethane (20.0 mL) under an ice-water bath. The reaction mixture was stirred at room temperature for 1.5 h. When the reaction was completed as detected by LCMS, the mixture was concentrated under reduced pressure at a low temperature (30 °C) to remove the solvent. A saturated sodium bicarbonate solution (5 mL) was added to the residue under an ice-water bath, and the mixture was extracted with dichloromethane (6 mL × 3). The organic phases were combined, washed with saturated brine (6 mL × 2), dried over anhydrous sodium sulfate, filtered, and concentrated to give compound 836-2 (0.8 g, yield: 89.2%). ES-API: [M+H]⁺ = 295.0/297.0.

Step 3: Compound 836-2 (250.0 mg, 0.847 mmol), 1,3-dibromopropane (0.86 mL, 8.469 mmol), and N,N-diisopropylethylamine (1.1 g, 8.469 mmol) were dissolved in anhydrous N,N-dimethylacetamide (10.0 mL) in a sealed tube, and the reaction mixture was heated to 120 °C and stirred for 3 h. When the reaction was completed as detected by LCMS, ethyl acetate (25 mL) was added to the reaction mixture, and the mixture was washed with saturated brine (15 mL × 3), dried over anhydrous sodium sulfate, filtered, and concentrated. The residue was purified by automated flash chromatography on silica gel (methanol/dichloromethane = 0-10%) to give compound 836-3 (190.0 mg, yield: 66.92%). ES-API: [M+H]⁺ = 335.1/337.1.

Step 4: Compound 836-3 (200.0 mg, 0.597 mmol), compound 81-7 (190 mg, 0.274 mmol), potassium phosphate (175.0 mg, 0.822 mmol), and [1,1'-bis(di-tert-butylphosphino)ferrocene]dichloropalladium(II) (35.0 mg, 0.055 mmol) were dissolved in dioxane (4.0 mL), toluene (12.0 mL), and water (4.0 mL) under a nitrogen atmosphere. The reaction mixture was heated to 70 °C under an oil bath and stirred for 5 h. When the reaction was completed as detected by LCMS, the mixture was cooled to room temperature. Ethyl acetate (20 mL) was added to the reaction mixture, and the mixture was washed with water (15 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-80%) to give compound 836-4 (260.0 mg, crude product). ES-API: [M+H]⁺ = 822.3.

Step 5: Cesium carbonate (900.0 mg, 2.740 mmol) and iodoethane (2.740 mg, 2.740 mmol) were sequentially added to a solution of compound 836-4 (260.0 mg, crude product) in N,N-dimethylformamide (10.0 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (methanol/dichloromethane = 0-2%) to give compound 836-5 (34.35 mg, yield: 34.35%). ES-API: [M+H]⁺ = 850.3.

Step 6: A solution of hydrochloric acid in methanol (2.0 mL, 4 M, 8.0 mmol) was slowly added dropwise to a solution of compound 836-5 (80.0 mg, 0.094 mmol) in methanol (2 mL) under an ice-water bath. The reaction mixture was stirred at room temperature for 1.5 h. When the reaction was completed as detected by LCMS, the mixture was concentrated under reduced pressure at a low temperature (30 °C) to remove the solvent. A saturated sodium bicarbonate solution (5 mL) was added to the residue under an ice-water bath, and the mixture was extracted with dichloromethane (6 mL × 3). The organic phases were combined, washed with saturated brine (6 mL × 2), dried over anhydrous sodium sulfate, filtered, and concentrated to give crude compound 836-6 (70.0 mg, yield: 99.18%). ES-API: [M+H]⁺ = 750.3.

Step 7: (1r,2R,3S)-2,3-Dimethylcyclopropane-1-carboxylic acid (14 mg, 0.124 mmol), N,N-diisopropylethylamine (37 mg, 0.285 mmol), and O-(7-azabenzotriazol-1-yl)-N,N,N',N'-tetramethyluronium hexafluorophosphate (54 mg, 0.142 mmol) were sequentially added to a solution of compound 836-6 (70 mg, 0.093 mmol) in N,N-dimethylformamide (2 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness to give crude compound 836A. The crude product was purified by prep-HPLC (ammonium bicarbonate method) to give 2 isomeric compounds. The structure of one of the isomeric compounds was arbitrarily designated as: (1r,2R,3S)-N-((4S,Z)-(Rₐ)-1²-(5-((1-(azetidin-1-yl)cyclopropyl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z836-1, 0.66 mg, yield: 0.84%, retention time = 2.70 min), ES-API [M+H]⁺ = 846.4.

The structure of the other isomeric compound was arbitrarily designated as: (1r,2R,3S)-N-((4S,Z)-(Sₐ)-1²-(5-((1-(azetidin-1-yl)cyclopropyl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z836-2, 1.65 mg, yield: 2.10%, retention time = 2.60 min), ES-API [M+H]⁺ = 846.4.

### Example 101. Synthesis of Compounds Z645 and Z645-1

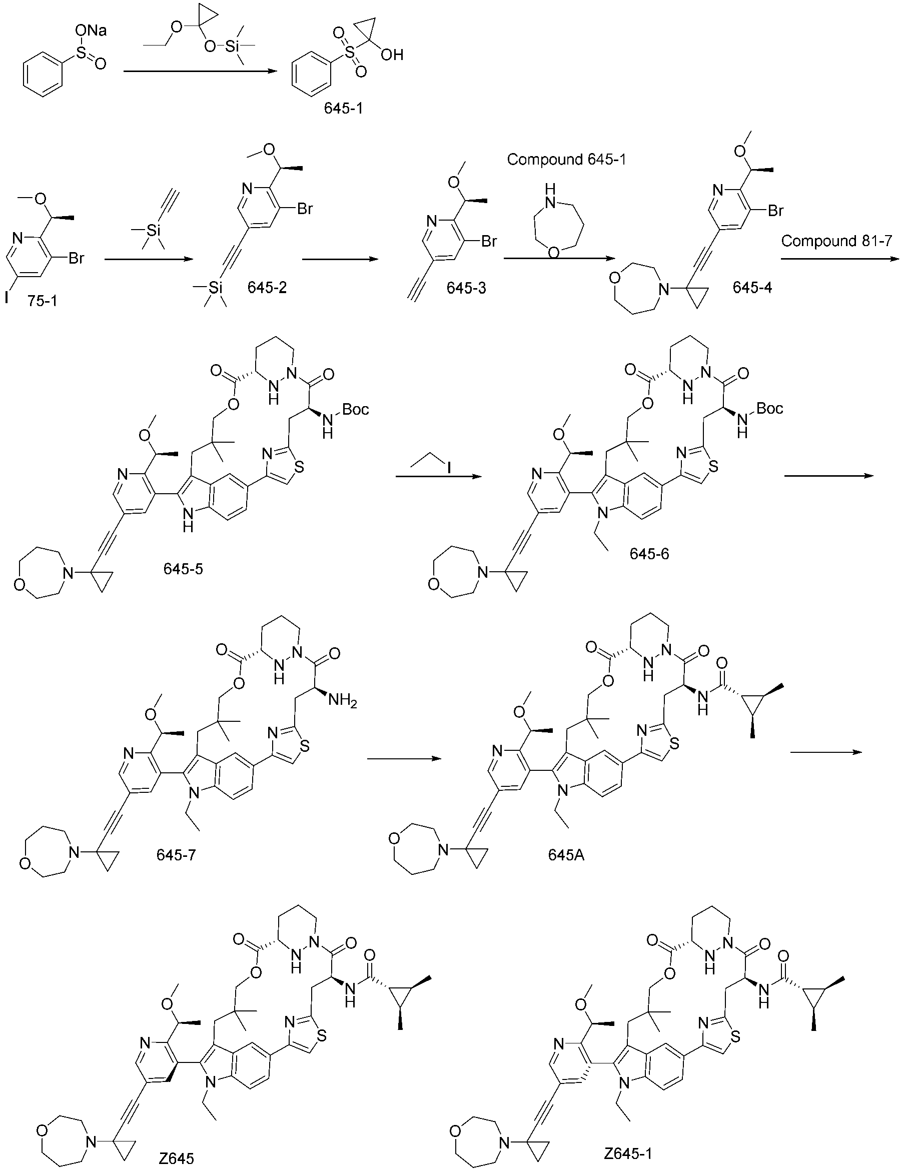

Step 1: (1-Ethoxycyclopropoxy)trimethylsilane (2.0 g, 11.474 mmol) and absolute methanol (6.0 mL) were added to a sealed tube, and 6 drops of a solution (4 M) of hydrochloric acid in methanol were added dropwise. The reaction system was reacted with stirring at room temperature for 10 min, and then the reaction was monitored by TLC (a solution of 10% ethyl acetate in petroleum ether, Rf = 0.3) for completion. Water (12.0 mL), sodium benzenesulfinate (3.798 g, 23.16 mmol), and formic acid (4.32 mL, 114.8 mmol) were sequentially added to the system described above. The mixture was stirred at room temperature for 48 h. After the reaction was completed, water (30 mL) was added, and the mixture was extracted with dichloromethane (60 mL × 3). The organic phases were combined, dried over anhydrous magnesium sulfate, and filtered, and the filtrate was concentrated to dryness by rotary evaporation under reduced pressure to give 645-1 (2.5 g, yield: 57.45%) as a white solid compound, which was directly used in the next step.

Step 2: Compound 75-1 (4.0 g, 11.697 mmol), ethynyltrimethylsilane (1.15 g, 11.697 mmol), bis(triphenylphosphine)palladium(II) dichloride (0.41 g, 0.585 mmol), copper(I) iodide (0.22 g, 1.170 mmol), and N,N-diisopropylethylamine (4.075 mL, 23.394 mmol) were dissolved in anhydrous acetonitrile (20.0 mL) under a nitrogen atmosphere. The reaction mixture was stirred at room temperature overnight. When the reaction was completed as detected by LCMS, the mixture was concentrated under reduced pressure to remove the solvent. The residue was purified by automated flash chromatography on silica gel (ethyl acetate/petroleum ether = 0-10%) to give compound 645-2 (3.3 g, yield: 90.34%). ES-API: [M+H]⁺ = 312.2/314.2.

Step 3: A solution of compound 645-2 (3.30 g, 10.567 mmol) in methanol (6.0 mL) was added to a sodium hydroxide solution (7.0 mmol, 1 M, 7.0 mL) at 15 °C. The mixture was reacted with stirring at room temperature for 2 h and concentrated by rotary evaporation under reduced pressure to remove the solvent. Dichloromethane (80 mL) and a saturated sodium bicarbonate solution (30 mL) were then added, and the mixture was stirred for 0.5 h. The organic phase was dried over anhydrous magnesium sulfate and filtered, and the filtrate was concentrated to dryness by rotary evaporation under reduced pressure to give compound 645-3 (2.50 g, yield: 98.53%). ES-API [M+H]⁺ = 240.1/242.1.

Step 4: Compound 645-3 (1.70 g, 7.080 mmol), compound 645-1 (0.70 g, 3.531 mmol), 1,4-oxazepane (0.54 g, 5.297 mmol), and gold(III) chloride (0.160 g, 0.530 mmol) were added to distilled water (5.0 mL). The mixture was reacted at room temperature for 12 h. After the reaction was completed, the system was extracted with ethyl acetate (100 mL × 2) and saturated sodium chloride (100 mL). The ethyl acetate phase was dried over anhydrous sodium sulfate and filtered, and the filtrate was concentrated to dryness by rotary evaporation under reduced pressure. The residue was purified by automated flash chromatography on silica gel [petroleum ether:ethyl acetate = 100:0 to 30:70, (v/v)] to give compound 645-4 (0.65 g, yield: 48.53%). ES-API [M+H]⁺ = 379.1/381.1.

Step 5: Compound 645-4 (300.0 mg, 0.791 mmol), compound 81-7 (107 mg, 0.155 mmol), potassium phosphate (99 mg, 0.465 mmol), and [1,1'-bis(di-tert-butylphosphino)ferrocene]dichloropalladium(II) (250.0 mg, 0.360 mmol) were dissolved in dioxane (4.0 mL), toluene (12.0 mL), and water (4.0 mL) under a nitrogen atmosphere. The reaction mixture was heated to 70 °C under an oil bath and stirred for 5 h. When the reaction was completed as detected by LCMS, the mixture was cooled to room temperature. Ethyl acetate (60.0 mL) was added to the reaction mixture, and the mixture was washed with water (30.0 mL) and saturated brine (50.0 mL × 3). The organic phase was dried over anhydrous sodium sulfate, filtered, and concentrated to dryness. The residue was purified by automated flash chromatography on silica gel (tetrahydrofuran/petroleum ether = 0-80%) to give compound 645-5 (300.0 mg, yield: 96.11%). ES-API: [M+H]⁺ = 866.3.

Step 6: Cesium carbonate (1.12 g, 3.464 mmol) and iodoethane (540.26 mg, 3.464 mmol) were sequentially added to a solution of compound 645-5 (300.0 mg, 0.346 mmol) in N,N-dimethylformamide (10.0 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (methanol/dichloromethane = 0-2%) to give compound 645-6 (290.0 mg, yield: 93.63%). ES-API: [M+H]⁺ = 894.3.

Step 7: A solution of hydrochloric acid in methanol (5.0 mL, 4 M, 20.0 mmol) was slowly added dropwise to a solution of compound 645-6 (290.0 mg, 0.324 mmol) in methanol (2 mL) under an ice-water bath, and the reaction mixture was stirred at room temperature for 1.5 h. When the reaction was completed as detected by LCMS, the mixture was concentrated under reduced pressure at a low temperature (30 °C) to remove the solvent. A saturated sodium bicarbonate solution (5 mL) was added to the residue under an ice-water bath, and the mixture was extracted with dichloromethane (6 mL × 3). The organic phases were combined, washed with saturated brine (6 mL × 2), dried over anhydrous sodium sulfate, filtered, and concentrated to give crude compound 645-7 (300.0 mg, crude product). ES-API: [M+H]⁺ = 794.3.

Step 8: (1r,2R,3S)-2,3-Dimethylcyclopropane-1-carboxylic acid (55.47 mg, 0.486 mmol), N,N-diisopropylethylamine (209.0 mg, 1.620 mmol), and O-(7-azabenzotriazol-1-yl)-N,N,N',N'-tetramethyluronium hexafluorophosphate (185.0 mg, 0.486 mmol) were sequentially added to a solution of compound 645-7 (300.0 mg, crude product) in N,N-dimethylformamide (10.0 mL) under an ice-water bath.The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness to give crude compound 645A. The crude product was purified by prep-HPLC (ammonium bicarbonate method) to give 2 isomeric compounds.

The structure of one of the isomeric compounds was arbitrarily designated as: (1r,2R,3S)-N-((4S,Z)-(Rₐ)-1²-(5-((1-(1,4-oxazepan-4-yl)cyclopropyl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z645, 45.0 mg, yield: 15.16%, retention time = 9.335 min), ES-API [M+H]⁺ = 890.3. ¹H NMR (400 MHz, DMSO-d6) δ 8.77-8.76 (m, 1H), 8.51 (s, 1H), 8.40-8.38 (m, 1H), 7.87 - 7.70 (m, 3H), 7.59-7.57(m, 1H), 5.59-5.54(m, 1H), 5.08-5.05 (m, 1H), 4.40 - 4.14 (m, 4H), 4.09-4.07(m, 1H), 3.67-3.64 (m, 2H), 3.60-3.58 (m, 4H), 3.25 (s, 3H), 3.16-3.12 (m, 1H), 2.98-2.95(m, 1H), 2.91~2.88 (m, 4H), 2.76-2.71 (m, 1H), 2.40-2.37 (m, 1H), 2.10-2.07(m, 1H), 1.83-1.77 (m, 4H), 1.60 - 1.45 (m, 1H), 1.36-1.30 (m, 7H), 1.11 - 1.04 (m, 8H), 1.00-0.98 (m, 2H), 0.92~0.88 (m, 6H), 0.36 (s, 3H).

The structure of the other isomeric compound was arbitrarily designated as: (1r,2R,3S)-N-((4S,Z)-(Sₐ)-1²-(5-((1-(1,4-oxazepan-4-yl)cyclopropyl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z645-1, 70.0 mg, yield: 24.27%, retention time = 9.084 min), ES-API [M+H]⁺ = 890.3. ¹H NMR (400 MHz, DMSO-d6) δ 8.79-8.77 (m, 1H), 8.54-8.52 (m, 1H), 8.42-8.39(m, 1H), 7.95-7.93 (m, 1H), 7.82 (s, 1H), 7.74-7.72 (m, 1H), 7.56-7.53 (m, 1H), 5.56~5.52 (m, 1H), 5.06~5.02 (m, 1H), 4.25-4.18 (m, 2H), 4.06 - 3.91 (m, 2H), 3.84-3.79 (m, 1H), 3.70 - 3.50 (m, 6H), 3.19~3.13 (m, 1H), 3.12 - 3.02 (m, 4H), 2.91-2.88 (m, 4H), 2.77-2.73 (m, 1H), 2.34-2.31 (m, 1H), 2.14-2.08(m, 1H), 1.83-1.77 (m, 4H), 1.58-1.50(m, 1H), 1.29 - 1.03 (m, 18H), 1.01-0.99 (m, 2H), 0.94 (s, 3H), 0.51 (s, 3H).

### Example 102. Synthesis of Compound Z755

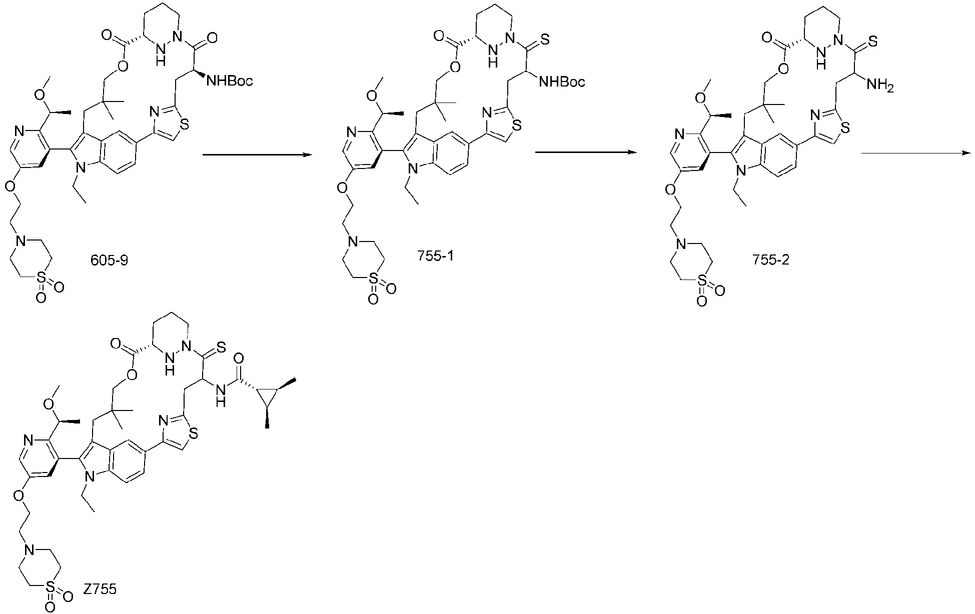

Step 1: Compound 605-9 (65 mg, 0.072 mmol) was dissolved in toluene (5 mL) and 1,4-dioxane (1 mL), and Lawesson's reagent (29 mg, 0.072 mmol) was added. The reaction system was stirred at 110 °C for 3 h. The reaction mixture was cooled to room temperature and concentrated. The crude product was subjected to preparative silica gel thin-layer chromatography (dichloromethane/methanol = 25:1) to give compound 755-1 (20 mg, yield: 30.1%) as a white solid. ES-API: [M+H]⁺ = 924.3.

Step 2: Compound 755-1 (20 mg, 0.022 mmol) was dissolved in absolute methanol (0.25 mL), and a 4.0 M hydrogen chloride/dioxane solution (1.0 mL) was added at 0 °C. The reaction system was stirred for 1 h under an ice bath. The reaction mixture was concentrated to give compound 755-2 (18 mg, yield: 100%) as a white solid. ES-API: [M+H]⁺ = 824.2.

Step 3: Compound 755-2 (18 mg, 0.022 mmol) was dissolved in dichloromethane (5 mL), and (1r,2R,3S)-2,3-dimethylcyclopropane-1-carboxylic acid (4 mg, 0.033 mmol), O-(7-azabenzotriazol-1-yl)-N,N,N',N'-tetramethyluronium hexafluorophosphate (17 mg, 0.044 mmol), and N,N'-diisopropylethylamine (17 mg, 0.131 mmol) were added. The reaction system was stirred at room temperature for 3 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was purified by prep-HPLC (ammonium bicarbonate method) to give the desired product (1r,2R,3S)-N-((6³S,4S,Z)-(Rₐ)-1²-(5-(2-(1,1-dioxidothiomorpholino)ethoxy)-2-((S)-1-methoxyethyl)pyridin-3-yl)-1¹-ethyl-10,10-dimethyl-7-oxo-5-thioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z755, 10 mg, yield: 50.0%) as a white solid. ES-API: [M+H]⁺ = 920.2.

### Example 103. Synthesis of Compounds Z678 and Z678-1

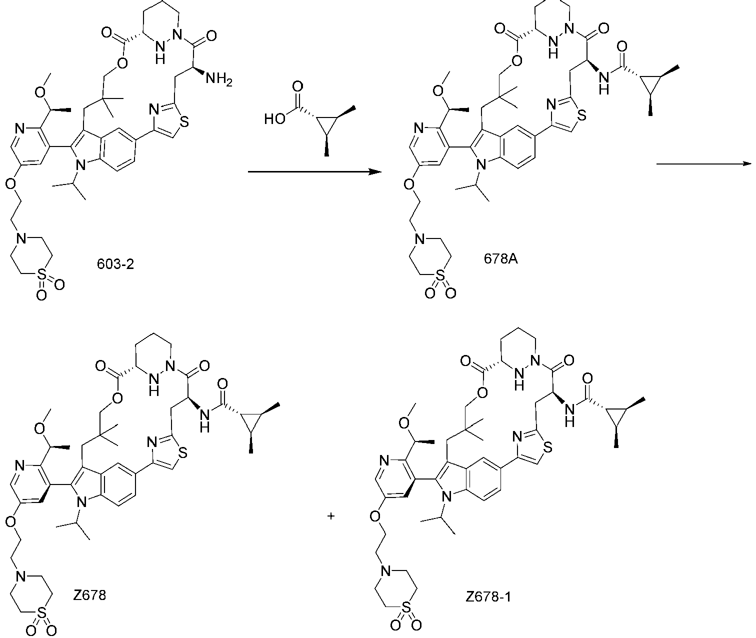

Step 1: Compound 603-2 (90 mg, crude product) was dissolved in dichloromethane (5 mL). (1r,2R,3S)-2,3-Dimethylcyclopropane-1-carboxylic acid (20.83 mg, 0.182 mmol), O-(7-azabenzotriazol-1-yl)-N,N,N',N'-tetramethyluronium hexafluorophosphate (92.51 mg, 0.243 mmol), and N,N'-diisopropylethylamine (48 mg, 0.372 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-5%) to give (1*r*,2*R*,3*S*)-N-((6³*S*,4*S*,*Z*)-1²-(5-(2-(1,1-dioxidothiomorpholino)ethoxy)-2-((*S*)-1-methoxyethyl)pyridin-3-yl)-1¹-isopropyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (compound 678A, 75 mg, purity: 80%). Compound 678A was subjected to prep-HPLC (column type: Waters XBridge C18, 190 × 250 mm, 5 µm; mobile phase system: A: 0.1% aqueous ammonium bicarbonate solution; B: preparative grade acetonitrile; flow rate: 15 mL/min; B% = 20%-100%; column temperature: room temperature) to give 2 isomeric compounds. The structure of one of the isomeric compounds was arbitrarily designated as (1*r*,2*R*,3*S*)-*N*-((63*S*,4*S,Z*)-(*Rₐ*)-1²-(5-(2-(1,1-dioxidothiomorpholino)ethoxy)-2-((*S*)-1-methoxyethyl)pyridin-3-yl)-1¹-isopropyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z678, 13 mg, 0.014 mmol, yield: 12.96%, retention time = 6.88 min). ES-API: [M+H]⁺ = 918.3. ¹H NMR (400 MHz, DMSO-d₆) δ 8.51 - 8.46 (m, 2H), 8.37 (d, J = 8.8 Hz, 1H), 7.79 (s, 1H), 7.74 - 7.64 (m, 2H), 7.28 (d, J = 2.8 Hz, 1H), 5.62 (t, J = 8.8 Hz, 1H), 5.09 (d, J = 12.4 Hz, 1H), 4.5 - 4.4 (m, 1H), 4.35 - 4.10 (m, 5H), 3.67 - 3.57 (m, 2H), 3.32 - 3.25 (m, 1H), 3.20 (s, 3H), 3.18 - 3.11 (m, 1H), 3.11 - 3.03 (m, 8H), 2.94 (t, J = 5.6 Hz, 2H), 2.89 - 2.72 (m, 2H), 2.43 (d, J = 14.0 Hz, 1H), 2.07 -2.00 (m, 1H), 1.83 - 1.70 (m, 5H), 1.60 - 1.45 (m, 1H), 1.40 (d, J = 6.0 Hz, 3H), 1.25 - 1.20 (m, 4H), 1.13 - 1.01 (m, 7H), 0.86 (s, 3H), 0.47 (s, 3H).

The structure of the other isomeric compound was arbitrarily designated as (1*r*,2*R*,3*S*)-N-((6³*S*,4*S,Z*)-(*Sₐ*)-1²-(5-(2-(1,1-dioxidothiomorpholino)ethoxy)-2-((S)-1-methoxyethyl)pyridin-3-yl)-1¹-isopropyl-10,10-dimethyl-5,7-dioxo-6¹,6²6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z678-1, 15 mg, 0.016 mmol, yield: 14.81%, retention time = 7.92 min) as a white solid. ES-API: [M+H]⁺ = 918.3. ¹H NMR (400 MHz, DMSO-d₆) δ 8.53 (s, 1H), 8.49 (d, *J* = 2.8 Hz, 1H), 8.40 (d, *J* = 9.2 Hz, 1H), 7.81 (s, 1H), 7.72 - 7.66 (m, 2H), 7.47 (d, *J* = 2.8 Hz, 1H), 5.55 (t, *J* = 9.2 Hz, 1H), 5.03 (d, *J =* 12.0 Hz, 1H), 4.30 - 4.15 (m, 4H), 4.08 - 3.99 (m, 1H), 3.98 - 3.91 (m, 1H), 3.70 (d, *J* = 10.8 Hz, 1H), 3.59 (d, *J =* 10.8 Hz, 1H), 3.36 - 3.34 (m, 1H), 3.20 - 3.12 (m, 1H), 3.12 - 3.07 (m, 7H), 3.07 - 2.99 (m, 5H), 2.95 (t, *J* = 5.6 Hz, 2H), 2.85 - 2.73 (m, 1H), 2.36 (d, *J* = 14.4 Hz, 1H), 2.16 - 2.07 (m, 1H), 1.88 - 1.72 (m, 2H), 1.59 (d, *J* = 6.4 Hz, 3H), 1.56 - 1.49 (m, 1H), 1.42 (d, *J* = 6.8 Hz, 3H), 1.27 - 1.18 (m, 5H), 1.13 - 1.06 (m, 6H), 0.92 (s, 3H), 0.53 (s, 3H).

### Example 104. Synthesis of Compounds Z788 and Z788-1

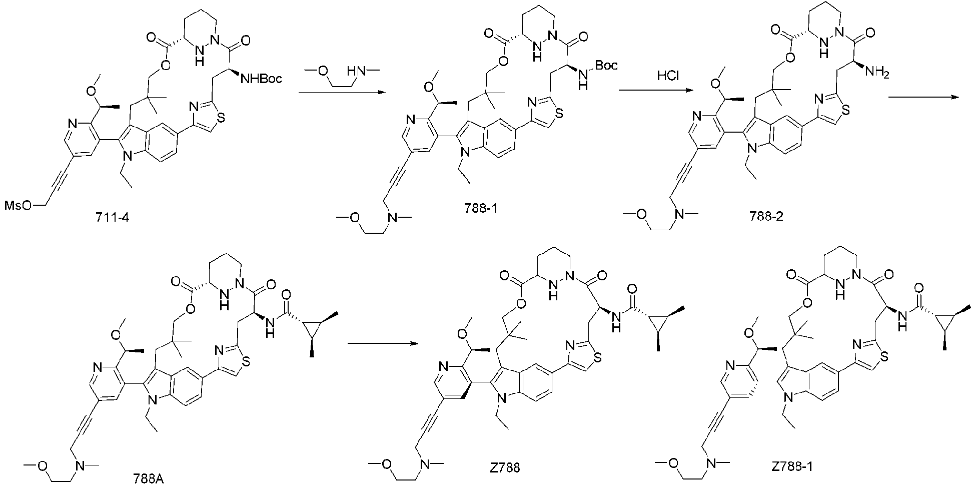

Step 1: N,N-Diisopropylethylamine (788.0 mg, 5.33 mmol) and 2-methoxy-N-methylethan-1-amine (240.0 mg, 2.665 mmol) were sequentially added to a solution of compound 711-4 (230.0 mg, 0.266 mmol) in acetonitrile (10.0 mL). The reaction mixture was stirred at room temperature for 1.5 h. When the reaction was completed as detected by LCMS, the mixture was concentrated under reduced pressure to remove the solvent. The residue was purified by automated flash chromatography on silica gel (tetrahydrofuran/petroleum ether = 0-50%) to give compound 788-1 (210.0 mg, yield: 92.05%). ES-API: [M+H]⁺ = 856.3.

Step 2: A solution of hydrochloric acid in methanol (5 mL, 4 M, 20.0 mmol) was added to a solution of compound 788-1 (210 mg, 0.245 mmol) in methanol (5 mL), and the reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined, dried over anhydrous sodium sulfate, filtered, and concentrated. The crude product was purified by automated flash chromatography on silica gel (methanol/dichloromethane = 0-6%) to give compound 788-2 (240.0 mg, crude product). ES-API: [M+H]⁺ = 756.3.

Step 3: (1r,2R,3S)-2,3-Dimethylcyclopropane-1-carboxylic acid (42.0 mg, 0.367 mmol), N,N-diisopropylethylamine (0.21 mL, 1.225 mmol), and O-(7-azabenzotriazol-1-yl)-N,N,N',N'-tetramethyluronium hexafluorophosphate (140.0 mg, 0.367 mmol) were sequentially added to a solution of compound 788-2 (240.0 mg, crude product) in N,N-dimethylformamide (10 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give (1r,2R,3S)-N-((4S,Z)-1¹-ethyl-1²-(2-((S)-1-methoxyethyl)-5-(3-((2-methoxyethyl)(methyl)amino)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (compound 788A, 150.0 mg, yield: 72.1%). ES-API: [M+H]⁺ = 852.4. Compound 788A was purified by prep-HPLC (chromatographic column: Welch Xtimate, 21.2 × 150 mm, 5 µm; mobile phase A: 0.1% aqueous NH₄HCO₃ solution, mobile phase B: ACN; flow rate: 15 mL/min; chromatographic conditions: 15 mL-60-70-13 min; column temperature: room temperature) to give 2 isomeric compounds. The structure of one of the isomeric compounds was arbitrarily designated as (1r,2R,3S)-N-((4S,Z)-1¹-ethyl-(Sₐ)-1²-(2-((S)-1-methoxyethyl)-5-(3-((2-methoxyethyl)(methyl))amino)prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z788-1, 52.0 mg, yield: 34.6%, retention time = 2.40 min), ES-API [M+H]⁺ = 852.4. ¹H NMR (400 MHz, DMSO-d6) δ 8.75-8.73 (m, 1H), 8.46-8.44 (m, 1H), 8.34-8.32 (m, 1H), 7.92-7.90 (m, 1H), 7.75 (s, 1H), 7.68-7.66(m, 1H), 7.48-7.46(m, 1H), 5.49-5.44 (m, 1H), 4.98-4.95 (m, 1H), 4.18~4.11 (m, 2H), 3.95 - 3.83 (m, 2H), 3.75-3.72(m, 1H), 3.64 - 3.46 (m, 4H), 3.39-3.36 (m, 2H), 3.17 (s, 3H), 3.09-3.05 (m, 1H), 3.04 - 2.95 (m, 4H), 2.74 - 2.64 (m, 1H), 2.55-2.53 (m, 2H), 2.27-2.24 (m, 4H), 2.06-2.03 (m, 1H), 1.75-1.70 (m, 2H), 1.50-1.43 (m, 1H), 1.20 - 0.97 (m, 16H), 0.86 (s, 3H), 0.43 (s, 3H).

The structure of the other isomeric compound was arbitrarily designated as: (1r,2R,3S)-N-((4S,Z)-1¹-ethyl-(Rₐ)-1²-(2-((S)-1-methoxyethyl)-5-(3-((2-methoxyethyl)(methyl))amino)prop-1-yn-1-yl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z788, 38.0 mg, yield: 25.33%, retention time = 2.44 min), ES-API [M+H]⁺ = 852.4. ¹H NMR (400 MHz, DMSO-d₆) δ 8.87~8.85 (m, 1H), 8.56~8.54 (m, 1H), 8.45~8.43(m, 1H), 7.93 - 7.84 (m, 2H), 7.82~7.79 (m, 1H), 7.65-7.62 (m, 1H), 5.65~5.58 (m, 1H), 5.13-5.10(m, 1H), 4.45 - 4.04 (m, 5H), 3.67-3.64 (m, 4H), 3.52-3.49(m, 2H), 3.31~3.29(m, 6H), 3.25 - 3.14 (m, 1H), 3.03~3.00 (m, 1H), 2.89 - 2.76 (m, 1H), 2.69-2.66 (m, 2H), 2.47-2.43(m, 1H), 2.37 (s, 3H), 2.15-2.12 (m, 1H), 1.91 - 1.77 (m, 2H), 1.66 - 1.51 (m, 1H), 1.48 - 1.39 (m, 3H), 1.31 - 1.07 (m, 10H), 1.01 - 0.86 (m, 6H), 0.41 (s, 3H).

### Example 105. Synthesis of Compounds Z796 and Z796-1

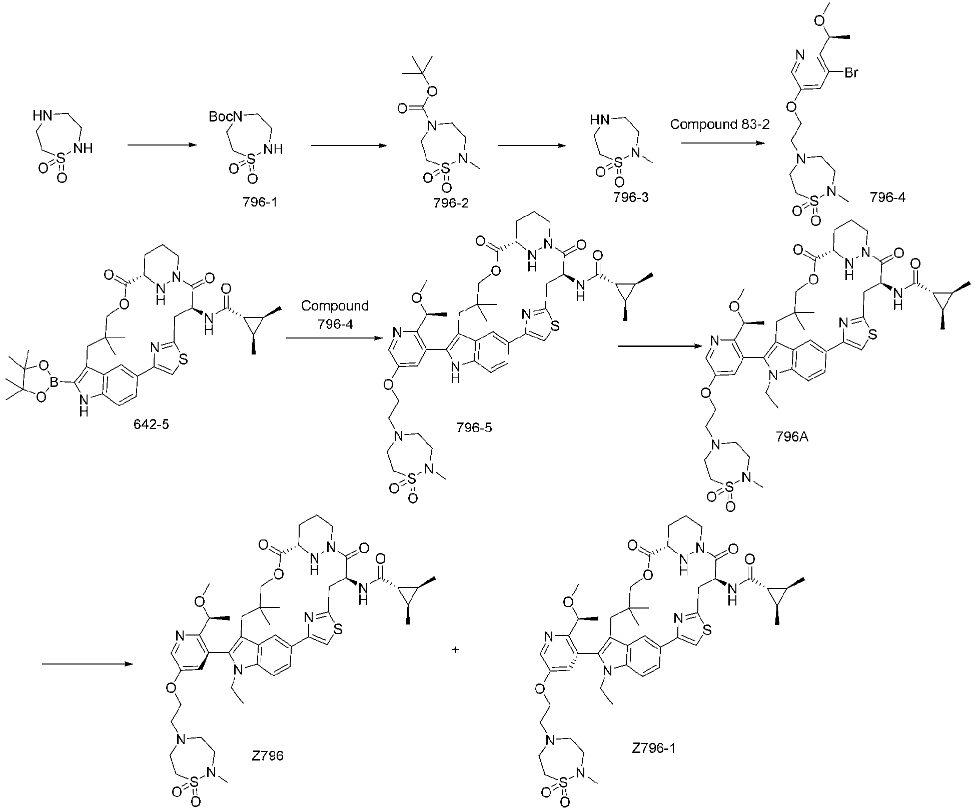

Step 1: Hexahydro-1,2,5-thiadiazepane 1,1-dioxide (400 mg, 2.663 mmol) was dissolved in pyridine (1 mL), and di-tert-butyl carbonate (696 mg, 3.2 mmol) was added. The reaction system was stirred at room temperature for 18 h. Water (20 mL) was added, and the mixture was extracted with dichloromethane (15 mL × 2). The organic phase was washed with saturated brine (10 mL × 3), dried over anhydrous sodium sulfate, filtered, and concentrated to give compound 796-1 (500 mg, 1.998 mmol, yield: 75.01%) as a white solid. ES-API: [M-Boc+H]⁺ = 151.1.

Step 2: Compound 796-1 (400 mg, 1.598 mmol) was dissolved in N,N-dimethylformamide (2 mL), and iodomethane (340 mg, 2.397 mmol) and sodium hydride (128 mg, 3.2 mmol, 60%) were added under an ice-water bath. The reaction system was stirred at room temperature for 18 h. Water (20 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-50%) to give compound 796-2 (380 mg, 1.438 mmol, yield: 89.96%) as a white solid. ES-API: [M-56+H]⁺ = 209.1. Step 3: Compound 796-2 (400 mg, 1.513 mmol) was dissolved in methanol (1 mL) under an ice-water bath, and a solution of hydrochloric acid in dioxane (3 mL) was added. The reaction system was stirred at room temperature for 3 h. The reaction mixture was concentrated under reduced pressure to give compound 796-3 (200 mg, 1.218 mmol, yield: 80.48%) as a white solid. ES-API: [M+H]⁺ = 165.2.

Step 4: Compound 796-3 (120 mg, 0.339 mmol) and sodium iodide (10 mg, 0.061 mmol) were dissolved in N,N-dimethylformamide (2 mL), and N,N'-diisopropylethylamine (220 mg, 1.7 mmol) was added. The reaction system was stirred at 80 °C for 48 h. Water (20 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-100%) to give compound 796-4 (80 mg, 0.189 mmol, yield: 55.91%) as a yellow oily liquid. ES-API: [M+H]⁺ = 422.1.

Step 5: Compound 642-5 (110 mg, 0.159 mmol) was dissolved in dioxane (3 mL), toluene (9 mL), and water (3 mL), and compound 796-4 (92.08 mg, 0.217 mmol), [1,1'-bis(di-tert-butylphosphino)ferrocene]dichloropalladium(II) (18.88 mg, 0.029 mmol), and potassium phosphate (184 mg, 0.870 mmol) were added to the solution described above at room temperature. After the addition was completed, the reaction system was stirred at 70 °C for 1 h under a nitrogen atmosphere. After the reaction was completed, water (30 mL) was added to the solution described above, and the mixture was extracted with ethyl acetate (20 mL × 2). The organic phase was washed with saturated brine (50 mL), dried, and concentrated to give a crude product. The crude product was purified by a flash silica gel column (methanol/dichloromethane: 0%-3%) to give compound 796-5 (90 mg, 0.1 mmol, yield: 62%) as a yellow solid. ES-API: [M+H]⁺ = 905.3.

Step 6: Compound 796-5 (60 mg, 0.066 mmol) was dissolved in N,N-dimethylformamide (1 mL), and cesium carbonate (107.99 mg, 0.331 mmol) and iodoethane (77 mg, 0.498 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give crude compound 796A. The crude product was purified by thin-layer chromatography (methanol/dichloromethane = 1/20) to give 2 isomeric compounds. The structure of one of the isomeric compounds was arbitrarily designated as (1r,2R,3S)*N*-((6³*S*,4*S*,*Z*)-1¹-ethyl-(Rₐ)-1²-2-((S)-1-methoxyethyl-5-(2-(2-methyl-1,1-dioxido-1,2,5-thiadiazepan-5-yl)ethoxy)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z796, 7 mg, 0.007 mmol, yield: 10.18%, Rf = 0.35) as a white solid. ES-API: [M+H]⁺ = 933.3.

The structure of the other isomeric compound was arbitrarily designated as (1r,2R,3S)-N-((6³S,4S,Z)-1¹-ethyl-(Sₐ)-1²-2-((S)-1-methoxyethyl-5-(2-(2-methyl-1,1-dioxido-1,2,5-thiadiazepan-5-yl)ethoxy)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z796-1, 15 mg, 0.015 mmol, yield: 22%, Rf = 0.3) as a white solid. ES-API: [M+H]⁺ = 933.3.

### Example 106. Synthesis of Compound Z794

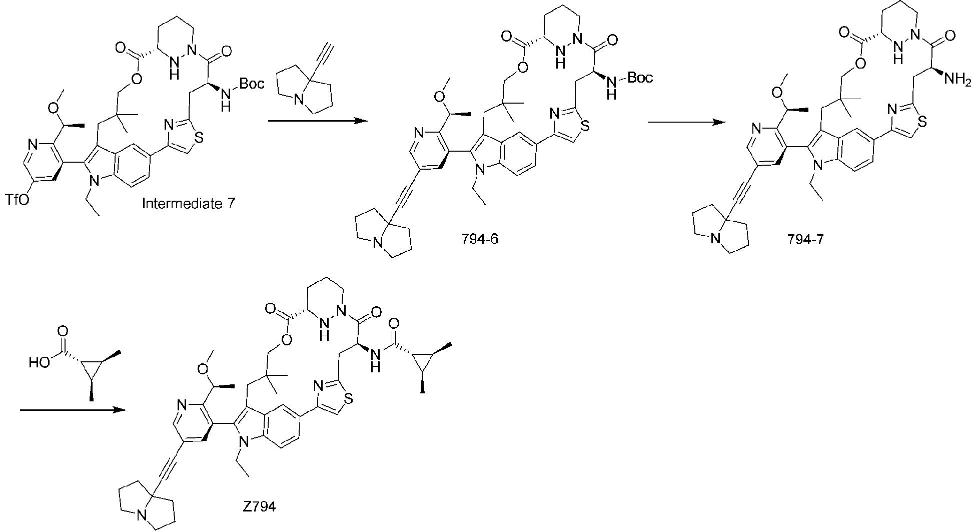

Step 1: Intermediate 7 (30 mg, 0.034 mmol) was dissolved in dioxane (2 mL). 7a-Ethynylhexahydro-1H-pyrrolizine (13.8 mg, 0.102 mmol), bis(triphenylphosphine)palladium(II) dichloride (4.8 mg, 0.007 mmol), copper(I) iodide (2.6 mg, 0.014 mmol), N,N'-diisopropylethylamine (13.2 mg, 0.102 mmol), and lithium chloride (5.8 mg, 0.137 mmol) were added to the solution described above at room temperature. After the addition was completed, the reaction system was stirred at 80 °C for 1 h under an argon atmosphere. After the reaction was completed, water (20 mL) was added to the reaction mixture, and the mixture was extracted with ethyl acetate (20 mL × 2). The organic phase was washed with saturated brine (30 mL × 2), dried over anhydrous sodium sulfate, filtered, and concentrated to give a crude product. The crude product was purified by a flash silica gel column (methanol/dichloromethane: 0%-5%) to give compound 794-6 (25 mg, 0.029 mmol, yield: 85.09%) as a white solid. ES-API: [M+H]⁺ = 864.1.

Step 2: Compound 794-6 (25 mg, 0.029 mmol) was dissolved in dichloromethane (2 mL), and trifluoroacetic acid (1 mL) was added to the solution described above. The mixture was stirred at room temperature for 2 h. The reaction mixture was concentrated, and a saturated aqueous sodium bicarbonate solution (10 mL) and ethyl acetate (20 mL) were added to the residue. The organic phase was separated, washed with saturated brine (10 mL × 2), dried over anhydrous sodium sulfate, filtered, and concentrated to give compound 794-7 (20 mg, crude product) as a white solid. ES-API: [M+H]⁺ = 764.1.

Step 3: Compound 794-7 (20 mg, crude product) was dissolved in dichloromethane (5 mL). (1r,2R,3S)-2,3-Dimethylcyclopropane-1-carboxylic acid (4.5 mg, 0.039 mmol), O-(7-azabenzotriazol-1-yl)-N,N,N',N'-tetramethyluronium hexafluorophosphate (15 mg, 0.04 mmol), and N,N'-diisopropylethylamine (10 mg, 0.08 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-5%) to give (1*r*,2*R*,3*S*)-N-((6³*S*,4*S,Z*)-(*R*a)*-*1¹-ethyl-1²-(2-((*S*)-1-methoxyethyl)-5-((tetrahydro-1H-pyrrolizin-7*a*(5*H*)-yl)ethynyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z794, 16 mg, 0.019 mmol, two-step yield: 65.52%) as a white solid. ES-API: [M+H]⁺ = 860.3. ¹H NMR (400 MHz, CD₃OD) δ 8.75 (d, J = 2.0 Hz, 1H), 8.56 (s, 1H), 7.86 (d, J = 2.0 Hz, 1H), (dd, J = 8.4, 1.2 Hz, 1H), 7.56 (s, 1H), 7.49 (d, J = 8.8 Hz, 1H), 5.74 (d, J = 7.2 Hz, 1H), 4.43 (d, J = 12.4 Hz, 1H), 4.38 - 4.27 (m, 2H), 4.25 - 4.07 (m, 2H), 3.76 - 3.67 (m, 2H), 3.49 - 3.39 (m, 1H), 3.34 (s, 3H), 3.28 - 3.25 (m, 3H), 3.12 - 3.06 (m, 2H), 2.85 - 2.75 (m, 3H), 2.53 (d, J = 14.4 Hz, 1H), 2.38 - 2.30 (m, 2H), 2.25 - 2.10 (m, 1H), 2.12 - 1.98 (m, 5H), 1.97 - 1.92 (m, 1H), 1.83 - 1.72 (m, 1H), 1.65 - 1.53 (m, 2H), 1.43 - 1.35 (m, 6H), 1.17 - 1.12 (m, 7H), 0.90 - 0.82 (m, 4H), 0.45 (s, 3H).

### Example 107. Synthesis of Compound Z810

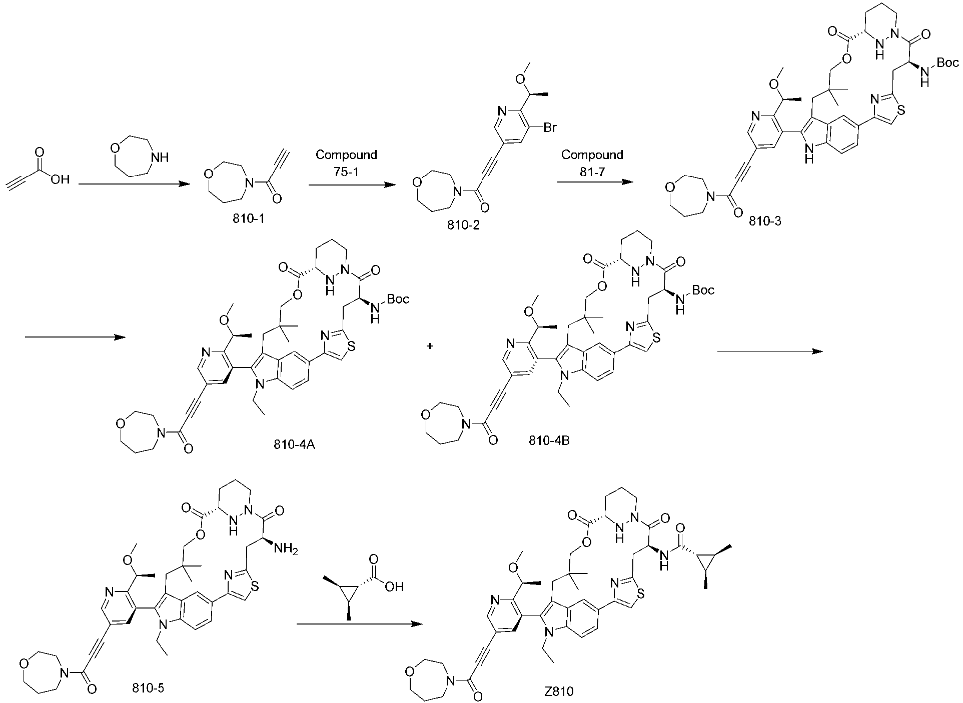

Step 1: Propynoic acid (1.25 g, 17.79 mmol) and triethylamine (4.5 g, 44.48 mmol) were added to a solution of 1,4-oxazepane (1.5 g, 14.83 mmol) in N,N-dimethylformamide (10 mL), and O-(7-azabenzotriazol-1-yl)-N,N,N',N'-tetramethyluronium hexafluorophosphate (8.46 g, 22.24 mmol) was added under an ice bath. The mixture was stirred at room temperature for 1 h. After the reaction was completed, the mixture was extracted with ethyl acetate (20 mL × 3). The combined extract was washed with brine (30 mL), dried over sodium sulfate, filtered, and concentrated, and the residue was subjected to flash silica gel column chromatography (0-40% petroleum ether/ethyl acetate) to give compound 810-1 (1.82 g, yield: 80%). ES-API [M+H]⁺ = 153.5.

Step 2: Compound 810-1 (455 mg, 2.97 mmol), compound 75-1 (1000 mg, 2.924 mmol), bis(triphenylphosphine)palladium(II) dichloride (104.11 mg, 0.149 mmol), copper(I) iodide (56.44 mg, 0.297 mmol), and N,N-diisopropylethylenediamine (766.35 mg, 5.941 mmol) were mixed in acetonitrile (10 mL) under a nitrogen atmosphere, and the mixture was stirred at room temperature for 1 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 810-2 (0.9 g, yield: 82.5%). ES-API [M+H]⁺ = 367/369.

Step 3: Compound 81-7 (340 mg, 0.49 mmol) and compound 810-2 (150 mg, 0.41 mmol) were in dioxane (6 mL)/toluene (2 mL)/water (2 mL) under a nitrogen atmosphere, and potassium phosphate (260.09 mg, 1.225 mmol) and bis(triphenylphosphine)palladium(II) dichloride (260.09 mg, 1.225 mmol) were added. The mixture was stirred at 65 °C for 16 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-80% petroleum ether/ethyl acetate) to give compound 810-3 (155 mg, yield: 44%). ES-API [M+H]⁺ = 854.3.

Step 4: Compound 810-3 (155 mg, 0.18 mmol) was dissolved in N,N-dimethylformamide (5 mL), and cesium carbonate (177.40 mg, 0.544 mmol) and iodoethane (566.14 mg, 3.630 mmol) were sequentially added. The mixture was stirred at room temperature for 16 h. After the reaction was completed, ethyl acetate (20 mL) was added, and the mixture was washed sequentially with saturated brine (10 mL) and diluted brine (10 mL). The organic phase was dried over anhydrous sodium sulfate, filtered, and concentrated, and the residue was purified by preparative thin-layer chromatography (dichloromethane/methanol = 20/1) to give 2 isomeric compounds. The structure of one of the isomeric compounds was arbitrarily designated as compound 810-4A (60 mg, yield: 31%, Rf = 0.5). ES-API [M+H]⁺ = 882.4. The structure of the other isomeric compound was arbitrarily designated as compound 810-4B (50 mg, yield: 37%, Rf = 0.4). ES-API [M+H]⁺ = 882.4. Step 5: Trifluoroacetic acid (3 mL) was added to a solution of compound 810-4A (60 mg, 0.07 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted with ethyl acetate (20 mL × 3), and concentrated to give compound 810-45 (50 mg, yield: 94%). ES-API [M+H]⁺ = 782.1.

Step 6: (1r,2R,3S)-2,3-Dimethylcyclopropane-1-carboxylic acid (8.8 mg, 0.08 mmol) and N,N-diisopropylethylenediamine (50 mg, 0.384 mmol) were added to a solution of compound 810-5 (50 mg, 0.06 mmol) in N,N-dimethylformamide (5 mL), and O-(7-azabenzotriazol-1-yl)-N,N,N',N'-tetramethyluronium hexafluorophosphate (48.6 mg, 0.128 mmol) was added under an ice bath. The mixture was stirred at room temperature for 1 h. After the reaction was completed, the mixture was extracted with ethyl acetate (20 mL × 3), and the combined extract was washed with brine (30 mL). The organic phase was dried over anhydrous sodium sulfate, filtered, and concentrated, and then the residue was purified by preparative thin-layer chromatography (dichloromethane/methanol = 20/1) to give (1r,2R,3S)-N-((6³S,4S,Z)-1¹-ethyl-(Rₐ)-1²-(5-(3-(1,4-oxazepan-4-yl)-3-oxoprop-1-yn-1-yl)-2-((S)-1-methoxyethyl)pyridin-3-yl)-10,10-dimethyl-5,7-dioxo-6¹,6²,6³,6⁴,6⁵,6⁶hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z810, 35 mg, yield: 62%, purity: 98%). ES-API [M+H]⁺ = 878.3.

### Example 108. Synthesis of Compounds Z803 and Z803-1

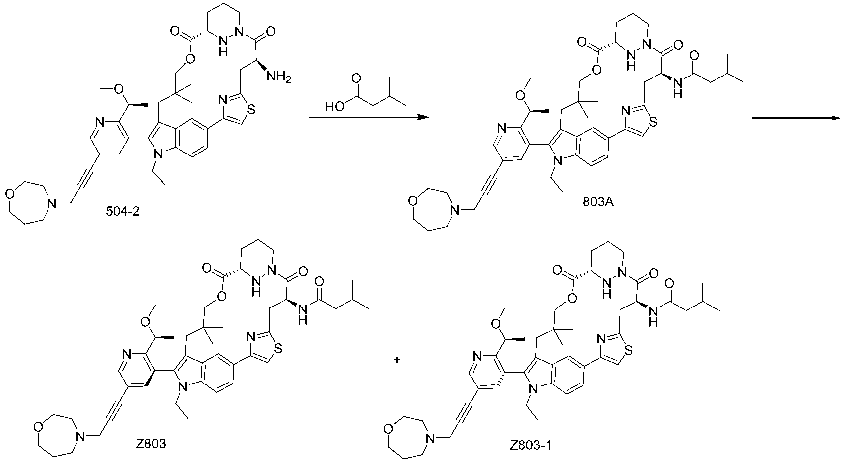

Step 1: Compound 504-2 (90 mg, 0.117 mmol) and isovaleric acid (15 mg, 0.152 mmol) were dissolved in dichloromethane (3 mL), and O-(7-azabenzotriazol-1-yl)-N,N,N',N'-tetramethyluronium hexafluorophosphate (66.84 mg, 0.176 mmol) and N,N'-diisopropylethylamine (0.5 mL, 0.5 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give crude compound 803A. The crude product was purified by prep-HPLC (ammonium bicarbonate) to give 2 isomeric compounds. The structure of one of the isomeric compounds was arbitrarily designated as *N*-((6³*S*,4*S,Z*)-(Rₐ)-1²-(5-(3-(1,4-oxazepan-4-yl)propyn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-methylbutanamide (Z803, 16 mg, 0.019 mmol, yield: 16.02%, retention time = 2.26 min), ES-API: [M+H]⁺ = 852.4. ¹H NMR (400 MHz, DMSO-d₆) δ 8.81 (d, *J* = 1.6 Hz, 1H), 8.50 (s, 1H), 8.29 (d, *J* = 8.8 Hz, 1H), 7.84 (d, *J* = 9.6 Hz, 2H), 7.76 (d, *J* = 7.6 Hz, 1H), 7.58 (d, *J* = 8.8 Hz, 1H), 5.52 (t, *J =* 8.8 Hz, 1H), 5.11 - 5.08 (m, 1H), 4.32 - 4.21 (m, 4H), 4.11 - 4.08 (m, 1H), 3.7-3.68 (m, 6H), 3.57 (s, 2H), 3.26 (s, 3H), 3.21 - 3.13 (m, 1H), 2.99 - 2.96 (m, 1H), 2.85-2.73 (m, 5H), 2.40-2.33 (m, 1H), 2.17 - 1.93 (m, 4H), 1.85-1.80 (m, 4H), 1.54-1.51 (m, 1H), 1.35 (d, *J =* 6.0 Hz, 3H), 0.93-0.85 (m, 13H), 0.33 (s, 3H).

The structure of the other isomeric compound was arbitrarily designated as *N*-((6³*S*,4*S,Z*)-(*Sₐ*)-1²-(5-(3-(1,4-oxazepan-4-yl)propyn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-methylbutanamide (Z803-1, 20 mg, 0.023 mmol, yield: 20.03%, retention time = 2.21 min), ES-API: [M+H]⁺ = 852.4. ¹H NMR (400 MHz, DMSO-d6) δ 8.75 (d, *J =* 2.0 Hz, 1H), 8.46 (s, 1H), 8.24 (d, *J* = 9.2 Hz, 1H), 7.92 (d, *J* = 2.0 Hz, 1H), 7.76 (s, 1H), 7.69 (d, *J* = 9.2 Hz, 1H), 7.47 (d, *J* = 9.2 Hz, 1H), 5.45 (t, *J* = 9.2 Hz, 1H), 5.01 (d, *J* = 12.4 Hz, 1H), 4.17-4.16 (m, 2H), 3.98 - 3.72 (m, 3H), 3.69 - 3.56 (m, 7H), 3.49 (d, *J =* 11.2 Hz, 1H), 3.13-3.07 (m, 1H), 3.05 - 2.92 (m, 4H), 2.81 - 2.59 (m, 5H), 2.25-2.23 (m, 1H), 2.14 - 1.88 (m, 4H), 1.81 - 1.73(m, 4H), 1.47 - 1.43 (m, 1H), 1.15 -1.14 (m, 4H), 1.04 (t, *J* = 7.2 Hz, 3H), 0.88-0.84 (m, 9H), 0.43 (s, 3H).

### Example 109. Synthesis of Compounds Z798 and Z798-1

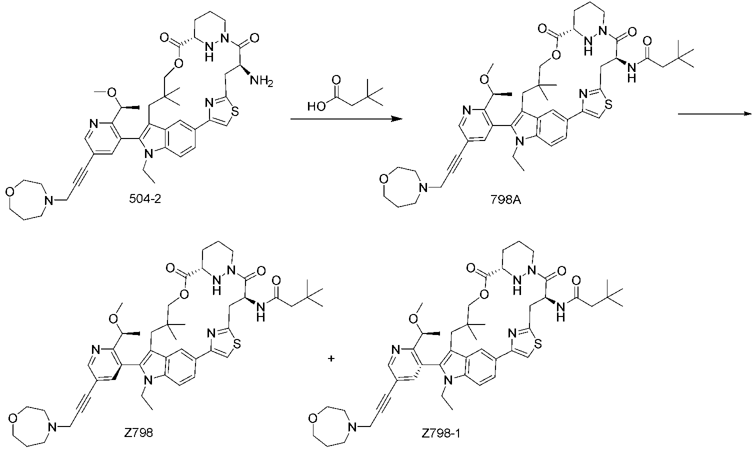

Step 1: Compound 504-2 (90 mg, 0.117 mmol) and 3,3-dimethyl-1-butyric acid (19 mg, 0.169 mmol) were dissolved in dichloromethane (3 mL), and O-(7-azabenzotriazol-1-yl)-N,N,N',N'-tetramethyluronium hexafluorophosphate (66.84 mg, 0.176 mmol) and N,N'-diisopropylethylamine (0.5 mL, 0.5 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give crude compound 798A. The crude product was purified by prep-HPLC (ammonium bicarbonate) to give 2 isomeric compounds. The structure of one of the isomeric compounds was arbitrarily designated as *N*-((6³*S*,4*S,Z*)-(*Rₐ*)-1²-(5-(3-(1,4-oxazepan-4-yl)propyn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3,3-dimethylbutanamide (Z798, 16 mg, 0.019 mmol, yield: 16.02%, retention time = 2.33 min) as a white solid. ES-API: [M+H]⁺ = 866.4. ¹H NMR (400 MHz, DMSO-d6) δ 8.81 (d, *J* = 2.0 Hz, 1H), 8.50 (s, 1H), 8.22 (d, *J* = 8.8 Hz, 1H), 7.88 - 7.82 (m, 2H), 7.76 (dd, *J* = 8.8, 1.6 Hz, 1H), 7.59 (d, *J = 8.8* Hz, 1H), 5.52 (t, *J* = 9.2 Hz, 1H), 5.10 (d, *J* = 12.0 Hz, 1H), 4.46 - 3.95 (m, 5H), 3.82 - 3.54 (m, 8H), 3.31-3.30 (m, 1H),3.27 (s, 3H), 3.21-3.15 (m, 1H), 3.00 - 2.97 (m, 1H), 2.81 - 2.70 (m, 5H), 2.39-2.36 (m, 1H), 2.15 - 2.01 (m, 3H), 1.88 - 1.80 (m, 4H), 1.55-1.52 (m, 1H), 1.36 (d, *J* = 6.4 Hz, 3H), 1.01 (s, 9H), 0.93 (s, 3H), 0.88 (t, *J* = 6.8 Hz, 3H), 0.34 (s, 3H).

The structure of the other isomeric compound was arbitrarily designated as *N*-((6³*S*,4*S*,*Z*)-(*Sₐ*)-1²-(5-(3-(1,4-oxazepan-4-yl)propyn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-methylbutanamide (Z798-1, 20 mg, 0.023 mmol, yield: 20.03%, retention time = 2.29 min) as a white solid. ES-API: [M+H]⁺ = 866.4. ¹H NMR (400 MHz, DMSO-d6) δ 8.82 (d, *J* = 2.0 Hz, 1H), 8.53 (s, 1H), 8.24 (d, *J* = 8.8 Hz, 1H), 7.99 (d, *J* = 2.0 Hz, 1H), 7.84 (s, 1H), 7.75 (dd, *J* = 8.8, 1.6 Hz, 1H), 7.55 (d, *J* = 8.8 Hz, 1H), 5.51 (t, *J* = 9.2 Hz, 1H), 5.08 (d, *J* = 8.4 Hz, 1H), 4.31- 4.14 (m, 2H), 3.97 - 3.92 (m, 1H),3.86 - 3.82 (m, 1H), 3.68 - 3.55 (m, 8H), 3.31-3.30 (m, 1H), 3.21-3.19 (m, 1H), 3.14 - 2.97 (m, 4H), 2.83 - 2.69 (m, 5H), 2.34-2.32 (m, 1H), 2.20 - 2.01 (m, 3H), 1.88 - 1.80 (m, 4H), 1.54-1.52 (m, 1H), 1.23 -1.22 (m, 4H), 1.12 (t, *J* = 7.2 Hz, 3H), 1.02 (s, 9H), 0.96 (s, 3H), 0.51 (s, 3H).

### Example 110. Synthesis of Compounds Z626 and Z837

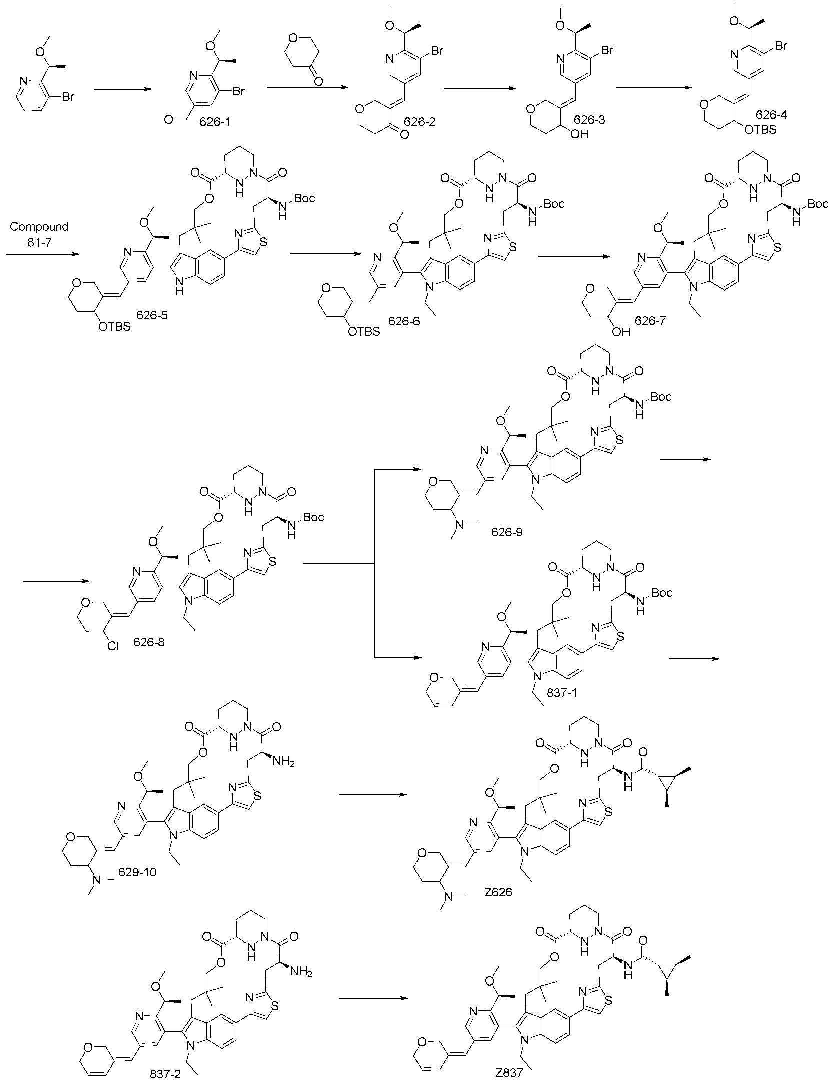

Step 1: Isopropylmagnesium chloride (12.372 mL, 16.083 mmol, a 1.3 M solution in tetrahydrofuran) was added dropwise to a solution of 3-bromo-5-iodo-2-((1S)-1-methoxyethyl)pyridine (5 g, 14.621 mmol) in tetrahydrofuran (2 mL) at -45 °C, and the mixture was stirred at that temperature for 1 h. Dry N,N-dimethylformamide (3.382 mL, 43.863 mmol) was then added dropwise to the mixture, and the resulting mixture was stirred for another 10 min. The reaction was completed as shown by LCMS. The mixture was quenched with a 1 M aqueous hydrochloric acid solution, and the resulting mixture was extracted with ethyl acetate and washed with water (30 mL) and saturated brine (30 mL × 3). The organic phase was dried over anhydrous sodium sulfate, filtered, and concentrated. The residue was purified by silica gel column chromatography (petroleum ether/ethyl acetate = 3/1) to give compound 626-1 (2800 mg, 11.471 mmol, yield: 78.46%). ES-API: [M-82+H]⁺ = 244.1, 246.1.

Step 2: Tetrahydropyran-4-one (102.54 mg, 1.024 mmol) was dissolved in dichloroethane (10 mL), and tetrahydropyrrole (0.042 mL, 0.512 mmol) was added under an ice bath. The mixture was reacted at room temperature for 2 h, and compound 626-1 (250 mg, 1.024 mmol) was added under an ice-water bath. The reaction mixture was reacted at 40 °C for 18 h. After the reaction was completed as shown by TLC, the reaction mixture was concentrated to dryness by rotary evaporation, and the residue was purified by silica gel column chromatography (eluent: petroleum ether/ethyl acetate = 10:1) to give compound 626-2 (230 mg, 0.705 mmol, yield: 68.84%). ES-API [M+H⁺] = 326, 328.

Step 3: Cerium(III) chloride heptahydrate (108.27 mg, 0.331 mmol) and sodium borohydride (12.53 mg, 0.331 mmol) were sequentially added to a solution of compound 626-2 (90 mg, 0.276 mmol) in methanol (5 mL) at -10 °C under an ice brine bath. After the reaction was performed at -10 °C for 10 min, the mixture was concentrated under reduced pressure to remove the solvent, and the residue was diluted with ethyl acetate. Then the mixture was washed with water (30 mL) and saturated brine (30 mL × 3). The organic phase was dried over anhydrous sodium sulfate, filtered, and concentrated, and the residue was purified by silica gel column chromatography (petroleum ether/ethyl acetate = 3/1) to give compound 626-3 (50 mg, 0.152 mmol, yield: 55.21%). ES-API: [M-82+H]⁺ = 328.0, 330.0.

Step 4: Compound 626-3 (50 mg, 0.152 mmol) was dissolved in N,N-dimethylformamide (2 mL), and imidazole (20.74 mg, 0.305 mmol) and tert-butyldimethylsilyl chloride (34.44 mg, 0.229 mmol) were added under an ice-water bath. The mixture was reacted at room temperature for 1 h. The mixture was diluted with ethyl acetate (30 mL) and washed with water (30 mL) and saturated brine (30 mL × 3). The organic phase was dried over anhydrous sodium sulfate, filtered, and concentrated, and the residue was purified by silica gel column chromatography (petroleum ether/ethyl acetate = 3/1) to give compound 626-4 (50 mg, 0.113 mmol, yield: 74.17%). ES-API: [M+H]⁺ = 442.1,443.1.

Step 5: [1,1'-Bis(di-tert-butylphosphino)ferrocene]dichloropalladium(II) (7.35 mg, 0.0113 mmol) and potassium carbonate (46.78 mg, 0.339 mmol) were added to a solution of compound 81-7 (78 mg, 0.113 mmol) and compound 626-4 (50 mg, 0.113 mmol) in water (1 mL) and tetrahydrofuran (4 mL). The mixture was degassed with nitrogen and stirred at 70 °C for 1 h. The mixture was cooled to room temperature, poured into water (30 mL), and extracted with ethyl acetate (30 mL × 3). The organic layer was washed with a saturated sodium chloride solution (30 mL × 1), dried over anhydrous sodium sulfate, filtered, and concentrated. The residue was purified by silica gel column chromatography (methanol/dichloromethane = 1/30) to give compound 626-5 (60 mg, 0.0646 mmol, yield: 57.18%), ES-API: [M+H]⁺ = 929.5.

Step 6: Cesium carbonate (118.62 mg, 0.364 mmol) and iodoethane (0.058 mL, 0.728 mmol) were sequentially added to a solution of compound 625-5 (60 mg, 0.0646 mmol) in N,N-dimethylformamide (3 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated, and the residue was purified by silica gel column chromatography (methanol/dichloromethane = 1/30) to give compound 626-6 (50 mg, 0.059 mmol, yield: 80.59%), ES-API: [M+H]⁺ = 957.4.

Step 7: Compound 626-6 (50 mg, 0.059 mmol) was dissolved in tetrabutylammonium fluoride (3 mL, 3.000 mmol, a 1 M solution in tetrahydrofuran). The mixture was stirred at 25 °C for 1 h. The mixture was concentrated, and the residue was poured into water (100 mL). The mixture was extracted with ethyl acetate (100 mL × 3). The organic phases were combined, washed with a saturated sodium chloride solution (100 mL × 1), dried over anhydrous sodium sulfate, filtered, and concentrated. The residue was purified by silica gel column chromatography (methanol/dichloromethane = 1/30) to give compound 626-7 (50 mg, 0.059 mmol, yield: 100%). ES-API: [M+H]⁺ = 843.4.

Step 8: N,N-Diisopropylethylamine (38 mg, 0.295 mmol) and methanesulfonyl chloride (20.38 mg, 0.178 mmol) were added to a solution of compound 626-7 (50 mg, 0.059 mmol) in dichloromethane (5 mL) under an ice-water bath, and the mixture was stirred at room temperature for 2 h. The mixture was poured into water (30 mL) and extracted with ethyl acetate (30 mL × 3). The organic layer was washed with a saturated sodium chloride solution (30 mL × 1), dried over anhydrous sodium sulfate, filtered, and concentrated. The residue was purified by silica gel column chromatography (methanol/dichloromethane = 1/30) to give compound 626-8 (30.66 mg, 0.035 mmol, yield: 60.4%), ES-API: [M+H]⁺ = 861.3.

Step 9: Dimethylamine (38 mg, 0.295 mmol, a 2 M solution in tetrahydrofuran), sodium iodide (5.22 mg, 0.035 mmol), and potassium methyl carbonate (14.44 mg, 0.104 mmol) were added to compound 626-8 (30 mg, 0.035 mmol) under an ice-water bath. The mixture was stirred at 80 °C for 2 h. The mixture was poured into water (30 mL) and extracted with ethyl acetate (30 mL × 3). The organic layer was washed with a saturated sodium chloride solution (30 mL × 1), dried over anhydrous sodium sulfate, filtered, and concentrated. The residue was purified by silica gel column chromatography (methanol/dichloromethane = 1/10) to give a mixture of compound 629-9 and compound 837-1 (mixture mass: 20.20 mg, yield: 66.67%). ES-API: [M+H]⁺ = 825.4.

Step 10: Hydrochloric acid (5 mL, a 4 M solution in dioxane) was added to a solution of the mixture described above (20.20 mg) in methanol (1 mL) under an ice-water bath, and the mixture was reacted at room temperature for 1 h. The mixture was concentrated to give a crude mixture of hydrochlorides of compound 629-10 and compound 837-2 (30 mg), which was directly used in the next step. ES-API: [M+H]⁺ = 770.4 and ES-API: [M+H]⁺ = 725.3.

Step 11: (1r,2R,3S)-2,3-Dimethylcyclopropane-1-carboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of the hydrochloride (30 mg) of the mixture described above in dichloromethane (5 mL) under an ice-water bath. The reaction mixture was warmed to room temperature and stirred for 1 h. When the reaction was completed as detected by LCMS, the reaction mixture was diluted with dichloromethane (20 mL), and then the mixture was washed with water (10 mL) and saturated brine (10 mL × 3). The organic phase was dried over anhydrous sodium sulfate, filtered, and concentrated to dryness. The residue was purified by prep-HPLC (trifluoroacetic acid method) to give 2 compounds. One of the compounds was (1r,2R,3S)-N-((6³S,4S,Z)-1²-(5-((Z)-(4-(dimethylamino)dihydro-2H-pyran-3(4H)-ylidene)methyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z626, 16 mg, yield: 30%, retention time = 1.433 min in 3 min LCMS (0.1% TFA)). ES-API: [M+H]⁺ = 866.3.

The other compound was (1r,2R,3S)-N-((6³S,4S,Z)-1²-(5-((Z)-(2H-pyran-3(6H)-ylidene)methyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z837, 3.4 mg, yield: 6.4%, retention time = 1.648 min in 3 min LCMS (0.1% TFA)). ES-API: [M+H]⁺ = 821.3.

### Example 111. Synthesis of Compounds Z784-1 and Z784-2

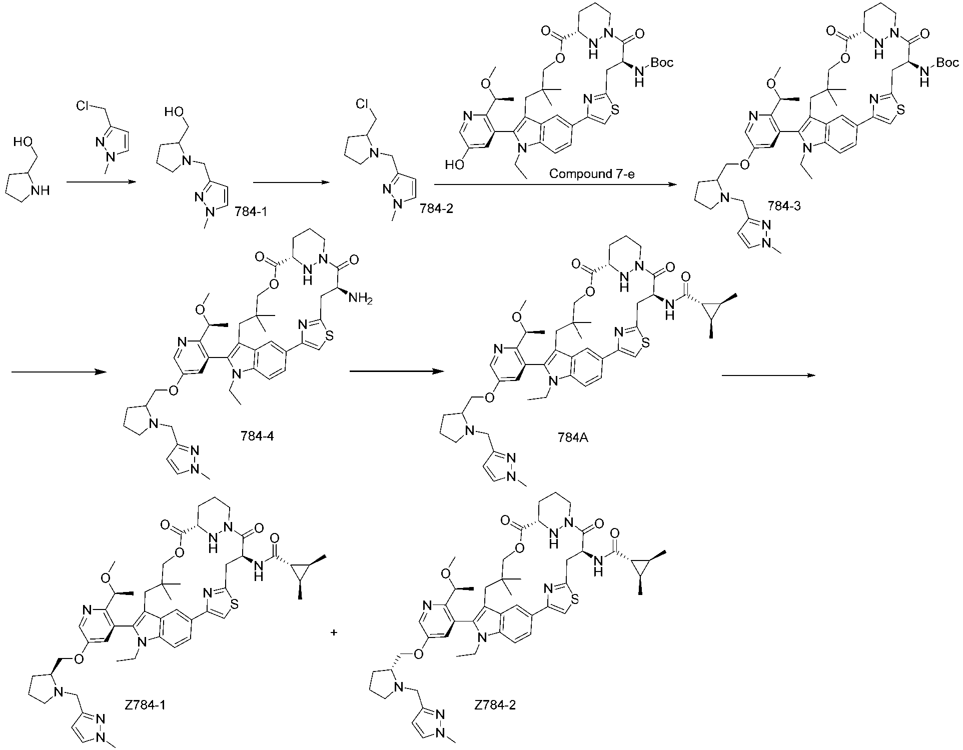

Step 1: Pyrrolidine-2-methanol (300 mg, 2.966 mmol) was dissolved in acetonitrile (10 mL), and 3-(chloromethyl)-1-methylpyrazole (367.89 mg, 2.818 mmol) and N,N-diisopropylethylenediamine (0.981 mL, 5.932 mmol) were added. The reaction mixture was stirred at 80 °C for 2 h. The mixture was concentrated under reduced pressure, and the residue was dissolved in ethyl acetate (50 mL). The resulting mixture was washed with water (30 mL) and saturated brine (30 mL × 3). The organic phase was dried over anhydrous sodium sulfate, filtered, and concentrated to dryness to give a crude product. The crude product was purified by silica gel column chromatography (dichloromethane/methanol = 10/1) to give compound 784-1 (500 mg, 2.561 mmol, yield: 86.33%). ES-API: [M+H]⁺ = 196.1.

Step 2: N,N-Diisopropylethylamine (1.697 mL, 10.242 mmol) and methanesulfonic anhydride (712.70 mg, 4.096 mmol) were added to a solution of compound 784-1 (400 mg, 2.048 mmol) in dichloromethane (5 mL). The mixture was stirred at 25 °C for 1 h. When the reaction was completed as detected by LCMS, the reaction mixture was diluted with dichloromethane (20 mL), and then the mixture was washed with saturated brine (15 mL × 3). The organic phase was dried over anhydrous sodium sulfate, filtered, and concentrated, and the residue was purified by silica gel column chromatography (petroleum ether/ethyl acetate = 3/1) to give compound 784-2 (350.22 mg, 1.639 mmol, yield: 80%). ES-API: [M+H]⁺ = 214.1.

Step 3: Compound 784-2 (21.48 mg, 0.101 mmol) and compound 7-e (50 mg, 0.067 mmol) were dissolved in N,N-dimethylformamide (3 mL), and sodium iodide (10.03 mg, 0.067 mmol) and potassium carbonate (27.75 mg, 0.201 mmol) were sequentially added. The mixture was heated at reflux for 3 h. The mixture was concentrated under reduced pressure, and the residue was dissolved in ethyl acetate (50 mL). The resulting mixture was washed with sodium bicarbonate (30 mL × 1). The organic phase was dried over anhydrous sodium sulfate, filtered, and concentrated, and the residue was purified by silica gel column chromatography (dichloromethane/methanol = 10/1) to give compound 784-3 (49.50 mg, 0.054 mmol, yield: 80%) as a white solid. ES-API: [M+H]⁺ = 924.4.

Step 4: Hydrochloric acid (3 mL, a 4 M solution in dioxane) was added to a solution of compound 784-3 (49.50 mg, 0.054 mmol) in methanol (1 mL). The mixture was reacted at room temperature for 1 h and concentrated to give a hydrochloride of crude compound 784-4 (50 mg), which was directly used in the next step. ES-API: [M+H]⁺ = 824.4.

Step 5: (1r,2R,3S)-2,3-Dimethylcyclopropane-1-carboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of the hydrochloride (50 mg) of compound 784-4 in dichloromethane (5 mL) under an ice-water bath. The reaction mixture was warmed to room temperature and stirred for 1 h. When the reaction was completed as detected by LCMS, the reaction mixture was diluted with dichloromethane (20 mL), and then the mixture was washed with water (10 mL) and saturated brine (10 mL × 3). The organic phase was dried over anhydrous sodium sulfate, filtered, and concentrated to dryness to give crude compound 784A. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-(*R*ₐ)-1²-5-(((S)-1-((1-methyl-1H-pyrazol-3-yl)methyl)pyrrolidin-2-yl)methoxy)-(2-((S)-1-methoxyethyl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z784-1, 6.9 mg, yield: 12.36%, 8.060 min in LCMS 15 min 0.1% NH₄HCO₃). ES-API [M+H]⁺ = 920.3.

The structure of the other isomeric compound was arbitrarily designated as (1r*,*2R*,*3S)-N-((6³S,4S,Z)-(*Rₐ*)-1²-5-(((*R*)-1-((1-methyl-1H-pyrazol-3-yl)methyl)pyrrolidin-2-yl)methoxy)-(2-((S)-1-methoxyethyl)-pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z784-2, 9.4 mg, yield: 16.84%, 8.211 min in LCMS 15 min 0.1% NH₄HCO₃). ES-API [M+H]⁺ = 920.3.

### Example 112.Synthesis of Compounds Z834 and Z834-1

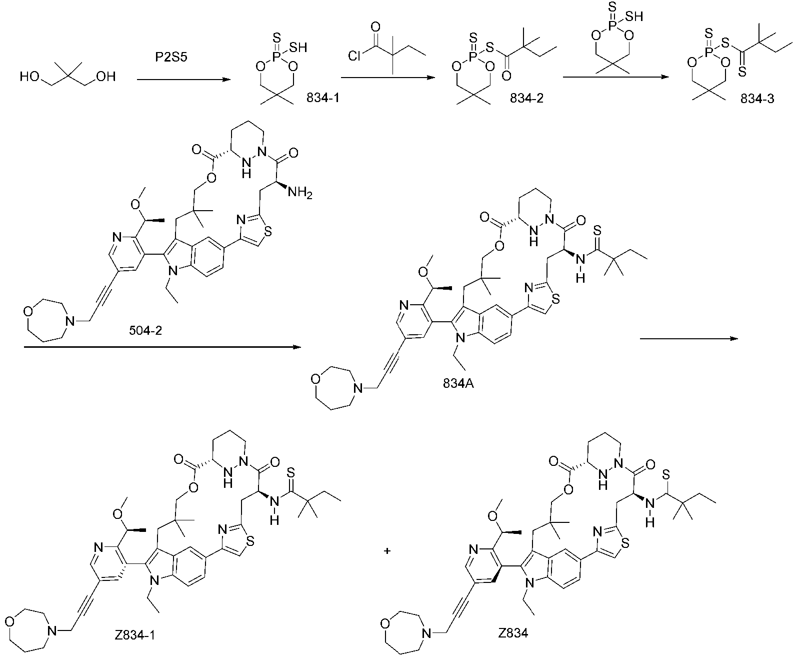

Step 1: Phosphorus pentasulfide (5.60 g, 25.20 mmol) was suspended in toluene (35 mL), and neopentyl glycol (5.25 g, 50.40 mmol) was added. The reaction system was stirred at 80 °C for 16 h. The reaction mixture was cooled to room temperature and concentrated. The crude product was recrystallized from carbon tetrachloride (12 mL), left to stand at -18 °C for 6 h, and filtered, and the filter cake was dried under reduced pressure to give compound 834-1 (5.1 g, yield: 51.1%) as a white solid. ¹H NMR (400 MHz, CDCl₃) δ 4.10 (s, 2H), 4.06 (s, 2H), 2.57 (s, 1H), 1.11 (s, 6H). ES-API: [M+H]⁺ = 199.1.

Step 2: Compound 834-1 (500 mg, 2.52 mmol) was dissolved in dichloromethane (10 mL), and 2,2-dimethylbutyrylchloride (340 mg, 2.52 mmol) was added. The reaction mixture was cooled to 0 °C, and then a solution of N,N-diisopropylethylamine (489 mg, 3.78 mmol) in dichloromethane (0.5 mL) was added dropwise. The reaction system was stirred for 15 min under an ice bath and then stirred at room temperature for 2 h. The reaction mixture was filtered through silica gel and washed with a small amount of dichloromethane. The filtrate was concentrated under reduced pressure to give compound 834-2 (610 mg, yield: 81.6%) as a white solid. ES-API: [M+Na]⁺ = 319.0.

Step 3: Compound 834-2 (610 mg, 2.06 mmol) was dissolved in toluene (15 mL), and 2-mercapto-5,5-dimethyl-1,3,2-diphosphinane-2-sulfide (1.05 g, 2.13 mmol) was added. The reaction system was stirred at 90 °C for 8 h. The reaction mixture was concentrated, and dichloromethane (30 mL) was added. The mixture was washed sequentially with saturated sodium bicarbonate (15 mL × 2) and saturated brine (15 mL). The organic phase was dried over anhydrous sodium sulfate and filtered, and the filtrate was concentrated under reduced pressure to give compound 834-3 (610 mg, yield: 94.8%) as a pink solid. ES-API: [M+Na]⁺ = 335.0.

Step 2: Compound 504-2 (90 mg, 0.117 mmol) was dissolved in dichloromethane (5 mL), and N,N-diisopropylethylamine (45 mg, 0.35 mmol) and compound 834-3 (55 mg, 0.176 mmol) were added at room temperature. The reaction system was stirred at room temperature for 40 h. Ethyl acetate (30 mL) was added to the reaction mixture, and the mixture was washed sequentially with water (10 mL), saturated sodium bicarbonate (10 mL), and saturated brine (10 mL). The organic phase was dried over anhydrous sodium sulfate and filtered, and the filtrate was concentrated under reduced pressure to give crude compound 834A. The crude product was subjected to prep-HPLC (ammonium bicarbonate method) to give 2 isomeric compounds. The structure of one of the isomeric compounds was arbitrarily designated as N-((6³S,4S,Z)-(Sₐ)-1²-(5-(3-(1,4-oxazepan-4-yl)prop-1-yn-1-yl)-2-((S)-1-methoxyethyl)pyridin-3-yl)-1¹ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,2-dimethylbutanethioamide (Z834-1, 30 mg, yield: 29.0%, retention time = 8.65) as a white solid. ¹H NMR (400 MHz, DMSO-d₆) δ 9.34 (d, *J =* 7.2 Hz, 1H), 8.82 (d, *J* = 2.0 Hz, 1H), 8.53 (d, *J =* 1.2 Hz, 1H), 7.99 (d, *J* = 2.0 Hz, 1H), 7.84 (s, 1H), 7.74 (dd, *J* = 8.8, 1.6 Hz, 1H), 7.54 (d, *J* = 8.8 Hz, 1H), 6.17 - 6.04 (m, 1H), 4.96 (d, *J* = 12.0 Hz, 1H), 4.35 - 4.17 (m, 2H), 4.03 - 3.77 (m, 4H), 3.74 - 3.61 (m, 7H), 3.56 (d, *J* = 11.2 Hz, 1H), 3.38 (d, *J* = 14.8 Hz, 1H), 3.13 - 2.99 (m, 4H), 2.88 - 2.70 (m, 5H), 2.32 (d, *J* = 14.0 Hz, 1H), 2.15 - 2.07 (m, 1H), 1.92 - 1.67 (m, 6H), 1.63 - 1.45 (m, 1H), 1.37 - 1.28 (m, 6H), 1.22 (d, *J* = 6.4 Hz, 3H), 1.11 (t, *J* = 7.2 Hz, 3H), 0.94 (s, 3H), 0.82 (t, *J* = 7.2 Hz, 3H), 0.50 (s, 3H). ES-API: [M+H]⁺= 882.3.

The structure of the other isomeric compound was arbitrarily designated as N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,2-dimethylbutanethioamide (Z834, 28 mg, yield: 27.1%, retention time = 8.79) as a white solid. ¹H NMR (400 MHz, DMSO-d₆) δ 9.33 (d, *J* = 7.2 Hz, 1H), 8.82 (d, *J* = 2.0 Hz, 1H), 8.51 (d, *J* = 1.2 Hz, 1H), 7.90 - 7.81 (m, 2H), 7.75 (dd, *J* = 8.8, 1.6 Hz, 1H), 7.58 (d, *J* = 8.8 Hz, 1H), 6.17 - 6.02 (m, 1H), 4.99 (d, *J =* 12.0 Hz, 1H), 4.44 - 4.18 (m, 4H), 4.17 - 4.02 (m, 1H), 3.91 (dd, *J =* 15.2, 9.2 Hz, 1H), 3.85 - 3.47 (m, 8H), 3.38 (d, *J* = 14.8 Hz, 1H), 3.28 (s, 3H), 3.08 - 2.65 (m, 6H), 2.35 (d, *J* = 14.4 Hz, 1H), 2.17 - 2.06 (m, 1H), 1.95 - 1.69 (m, 6H), 1.63 - 1.49 (m, 1H), 1.40 - 1.26 (m, 9H), 0.95 (s, 3H), 0.90 - 0.77 (m, 6H), 0.33 (s, 3H). ES-API: [M+H]⁺ = 882.4.

### Example 113. Synthesis of Compound Z838

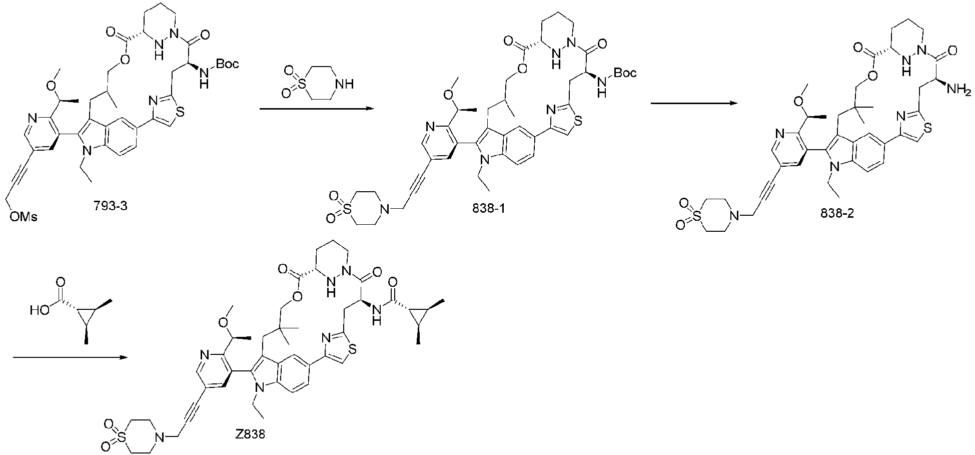

Step 1: N,N-Diisopropylethylamine (0.08 mL, 0.465 mmol) and 1,1-dioxothiomorpholine (25 mg, 0.186 mmol) were sequentially added to a solution of compound 793-3 (80 mg, 0.093 mmol) in dichloromethane (6 mL). The reaction mixture was stirred at room temperature overnight. When the reaction was completed as detected by LCMS, the mixture was concentrated under reduced pressure to give crude compound 838-1 (83 mg, yield: 99%). ES-API: [M+H]⁺ = 902.1.

Step 2: Compound 838-1 (83 mg, 0.092 mmol) was dissolved in a solution of hydrochloric acid in dioxane (3 mL) and a solution of hydrochloric acid in methanol (0.5 mL). The reaction mixture was stirred at room temperature for 1 h. When the reaction was completed as detected by LCMS, the reaction mixture was concentrated under reduced pressure to give crude compound 838-2 (73 mg, yield: 99%). ES-API: [M+H]⁺ = 802.1.

Step 3: (1r,2R,3S)-2,3-Dimethylcyclopropane-1-carboxylic acid (16 mg, 0.137 mmol), N,N-diisopropylethylamine (71 mg, 0.546 mmol), and O-(7-azabenzotriazol-1-yl)-N,N,N',N'-tetramethyluronium hexafluorophosphate (52 mg, 0.137 mmol) were sequentially added to a solution of compound 838-2 (73 mg, 0.0918 mmol) in N,N-dimethylformamide (2 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give compound (1r,2R,3S)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z838, 28.47 mg, yield: 35%, retention time = 1.92 min), ES-API [M+H]⁺ = 898.4.

### Example 114. Synthesis of Compound Z839

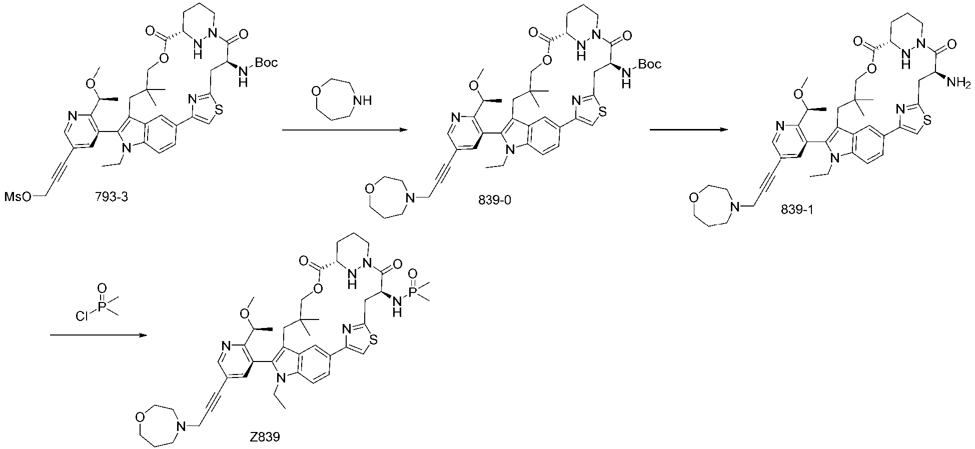

Step 1: 1,4-Oxazepane (1.68 g, 16.650 mmol) was added to a solution of 793-3 (4.79 g, 5.550 mmol) in dichloromethane (50 mL). The reaction mixture was stirred at room temperature for 2 h. When the reaction was completed as detected by LCMS, the mixture was concentrated, and the crude product was purified by automated flash chromatography on silica gel (methanol/dichloromethane = 0-6%) to give compound 839-0 (4.29 g, 4.942 mmol, yield: 89.04%). ES-API: [M+H]⁺ = 868.4. Step 2: Hydrochloric acid (20 mL, a 4 M solution in dioxane) was added to a solution of compound 839-0 (4.29 g, 4.942 mmol) in methanol (5 mL) under an ice-water bath. The mixture was reacted at room temperature for 1 h and concentrated to give a hydrochloride of crude compound 839-1 (3.80 g), which was directly used in the next step. ES-API: [M+H]⁺ = 768.4. Step 3: Compound 839-1 (85 mg, 0.111 mmol) and N-methylmorpholine (112 mg, 1.11 mmol) were dissolved in dichloromethane (5 mL), and dimethylphosphinic chloride (37 mg, 0.332 mmol) was added under an ice bath. The reaction system was stirred at room temperature for 3 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was purified by prep-HPLC (ammonium bicarbonate method) to give the desired product N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-P,P-dimethylphosphinic amide (Z839, 7 mg, yield: 7.5%) as a white solid. ES-API: [M+H]⁺ = 844.3.

### Example 115. Synthesis of Compound Z802

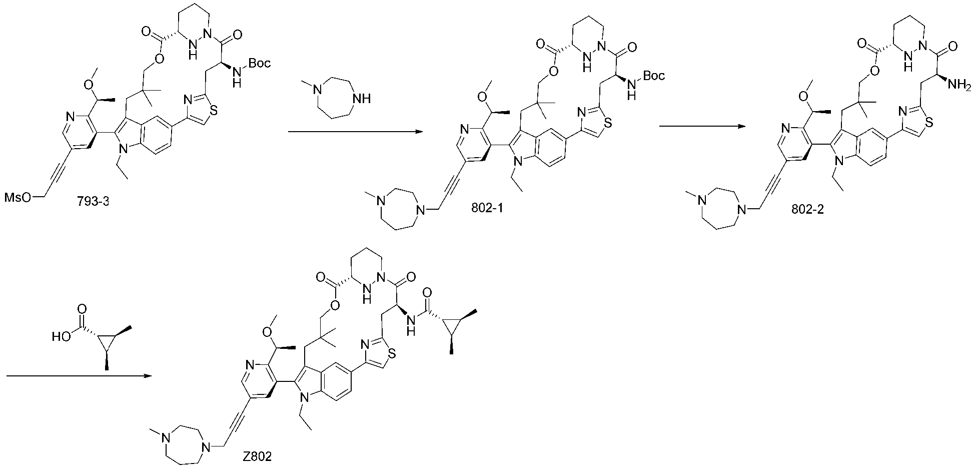

Step 1: N-Methylhomopiperazine (27.75 mg, 0.243 mmol) was added to a solution of compound 793-3 (70 mg, 0.081 mmol) in dichloromethane (5 mL). The reaction mixture was stirred at room temperature for 1.5 h. When the reaction was completed as detected by LCMS, the reaction mixture was concentrated, and the crude product was purified by automated flash chromatography on silica gel (methanol/dichloromethane = 0-6%) to give compound 802-1 (50 mg, 0.057 mmol, yield: 70.3%). ES-API: [M+H]⁺ = 881.3.

Step 2: Hydrochloric acid (3 mL, a 4 M solution in dioxane) was added to a solution of compound 802-1 (50 mg, 0.057 mmol) in methanol (1 mL) under an ice-water bath, and the mixture was reacted at room temperature for 1 h. The reaction mixture was concentrated to give a hydrochloride of crude compound 802-2 (50 mg), which was directly used in the next step without purification. ES-API: [M+H]⁺ = 781.3.

Step 3: (1r,2R,3S)-2,3-Dimethylcyclopropane-1-carboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of the hydrochloride (50 mg) of compound 802-2 described above in dichloromethane (5 mL) under an ice-water bath. The reaction mixture was warmed to room temperature and stirred for 1 h. When the reaction was completed as detected by LCMS, the reaction mixture was diluted with dichloromethane (20 mL), and then the mixture was washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give: (1r,2R,3S)-N-((6³S,4S,Z)-1²-(5-(3-(4-methyl-1,4-diaza-1-yl)prop-1-yn-1-yl)-(Ra)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z802, 28 mg, yield: 49.86%). ES-API: [M+H]⁺ = 877.4.

### Example 116. Synthesis of Compound Z909

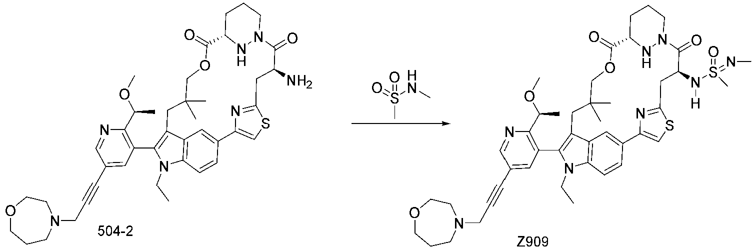

Step 1: Triphenylphosphine dichloride (1.68 g, 5 mmol) was dissolved in dichloromethane (20 mL) under a nitrogen atmosphere, and triethylamine (0.96 mL, 6.88 mmol) was added dropwise. The mixture was stirred at room temperature for 0.5 h. The mixture was then cooled to 0 °C, and N-methylmethanesulfonamide was added to the reaction mixture. The mixture was stirred for 0.5 h. Then the mixture was added to a solution of compound 504-2 (100 mg, 0.130 mmol) and pyridine (50 mg, 0.39 mmol) in dichloromethane (10 mL), and the mixture was stirred at room temperature overnight. The reaction mixture was quenched with a saturated sodium bicarbonate solution (20 mL), and the mixture was extracted three times with dichloromethane (20 mL). The organic phases were combined, dried over anhydrous sodium sulfate, filtered, and concentrated, and the residue was purified by prep-HPLC (ammonium bicarbonate method) to give N-((6³S,4S,Z)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-N'-methylmethanesulfonimidamide (Z909, 8.7 mg, yield: 7.8%) as a white solid. ES-API: [M+H]⁺ = 859.6.

### Example 117. Synthesis of Compound Z855

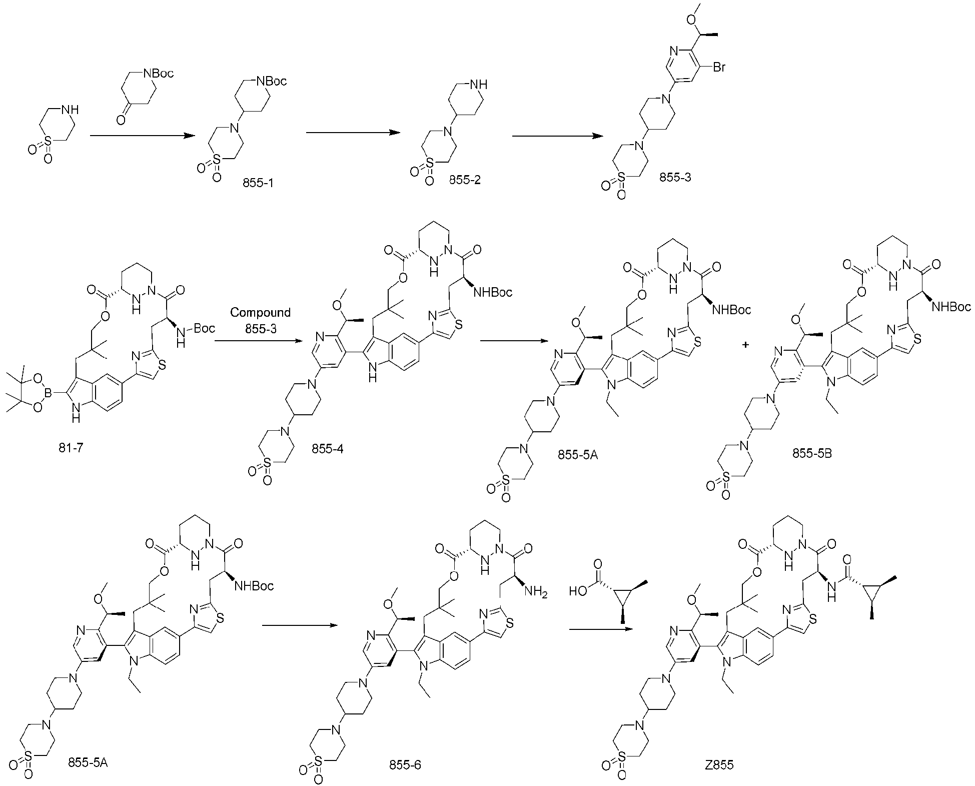

Step 1: N-tert-Butoxycarbonyl-4-piperidone (2.0 g, 10 mmol) was dissolved in methanol (80 mL). Thiomorpholine-1,1-dioxide (1.35 g, 9.987 mmol) and acetic acid (0.572 mL, 10 mmol) were added at room temperature. The mixture was stirred at room temperature for 0.5 h. Sodium cyanoborohydride (0.75 g, 12 mmol) was added, and the mixture was stirred at room temperature for 18 h and then stirred at 60 °C for 3 h. The reaction mixture was concentrated under reduced pressure, and the crude product was purified by a flash silica gel column (ethyl acetate/petroleum ether: 0%-50%) to give compound 855-1 (0.9 g, 2.826 mmol, yield: 28.30%) as a white solid. ES-API: [M-56+H]⁺= 263.2.

Step 2: Compound 855-1 (0.8 g, 2.512 mmol) was dissolved in methanol (1 mL) under an ice-water bath, and a solution of hydrochloric acid in dioxane (3 mL) was added. The reaction system was stirred at room temperature for 3 h. The mixture was concentrated under reduced pressure to give compound 855-2 (0.6 g, 2.355 mmol, yield: 93.74%) as a white solid. ES-API: [M+H]⁺= 219.2.

Step 3: (S)-3-Bromo-5-iodo-2-(1-methoxyethyl)pyridine (500 mg, 1.462 mmol), compound 855-2 (600 mg, 2.355 mmol), 4,5-bis(diphenylphosphino)-9,9-dimethylxanthene (169.20 mg, 0.292 mmol), tris(dibenzylidene-BASE acetone)dipalladium(0) (133.89 mg, 0.146 mmol), and sodium tert-butoxide (421.53 mg, 4.386 mmol) were dissolved in toluene (2 mL), and the mixture was stirred at 60 °C for 3 h under a nitrogen atmosphere. Water (20 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-100%) to give compound 855-3 (0.3 g, 0.694 mmol, yield: 47.45%). ES-API: [M+H]⁺= 432.1, 434.1.

Step 4: Compound 855-3 (383.34 mg, 0.887 mmol), compound 81-7 (410 mg, 0.591 mmol), potassium phosphate (376.37 mg, 1.773 mmol), and [1,1'-bis(di-tert-butylphosphino)ferrocene]dichloropalladium(II) (38.48 mg, 0.059 mmol) were dissolved in dioxane (3 mL), toluene (9 mL), and water (3 mL), and the reaction system was stirred at 70 °C for 1 h under a nitrogen atmosphere. After the reaction was completed, water (30 mL) was added to the solution described above, and the mixture was extracted with ethyl acetate (20 mL × 2). The organic phase was washed with saturated brine (50 mL), dried over anhydrous sodium sulfate, filtered, and concentrated to give a crude product. The crude product was purified by a flash silica gel column (methanol/dichloromethane: 0%-3%) to give compound 855-4 (450 mg, 0.490 mmol, yield: 82.83%) as a yellow solid. ES-API: [M+H]⁺ = 919.3.

Step 5: Compound 855-4 (450 mg, 0.490 mmol) was dissolved in N,N-dimethylformamide (3 mL), and cesium carbonate (478.54 mg, 1.469 mmol) and iodoethane (229.12 mg, 1.469 mmol) were added. The mixture was reacted at room temperature for 15 h. After the reaction was completed, water (5 mL) was added, and the mixture was extracted with ethyl acetate (10 mL × 3). The organic phase was washed with saturated brine (5 mL), dried over anhydrous sodium sulfate, and concentrated under reduced pressure to give a crude product. The crude product was purified by thin-layer chromatography (methanol/dichloromethane = 1/20) to give 2 isomeric compounds. The structure of one of the isomeric compounds was arbitrarily designated as compound 855-5A (80 mg, 0.084 mmol, yield: 17.25%, Rf = 0.35) as a white solid. ES-API: [M+H]⁺ = 947.4.

The structure of the other isomeric compound was arbitrarily designated as compound 855-5B (120 mg, 0.12 mmol, yield: 25.87%, Rf = 0.3) as a white solid. ES-API: [M+H]⁺ = 947.4.

Step 6: Compound 855-5A (80 mg, 0.084 mmol) was dissolved in methanol (1 mL) under an ice-water bath, and a solution of hydrochloric acid in dioxane (3 mL) was added. The reaction system was stirred at room temperature for 3 h. The reaction mixture was concentrated under reduced pressure to give compound 855-6 (70 mg, 0.083 mmol, yield: 97.85%) as a white solid. ES-API: [M+H]⁺= 847.3.

Step 7: Compound 855-6 (70 mg, 0.083 mmol) was dissolved in N,N-dimethylformamide (2 mL). (1r,2S,3R)-2,3-Dimethylcyclopropane-1-carboxylic acid (12 mg, 0.105 mmol), O-(7-azabenzotriazol-1-yl)-N,N,N',N'-tetramethyluronium hexafluorophosphate (48 mg, 0.126 mmol), and N,N'-diisopropylethylamine (54.28 mg, 0.420 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by preparative thin-layer chromatography (methanol/dichloromethane = 1/20, Rf = 0.3) to give (1r,2R,3S)-*N*-((6³*S*,4*S*,*Z*)-(*Rₐ*)-1²-(5-(4-(1,1-dioxidothiomorpholino)piperidin-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z855, 36 mg, 0.038 mmol, yield: 45.44%) as a white solid. ES-API: [M+H]⁺ = 943.3.

### Example 118. Synthesis of Compound Z910

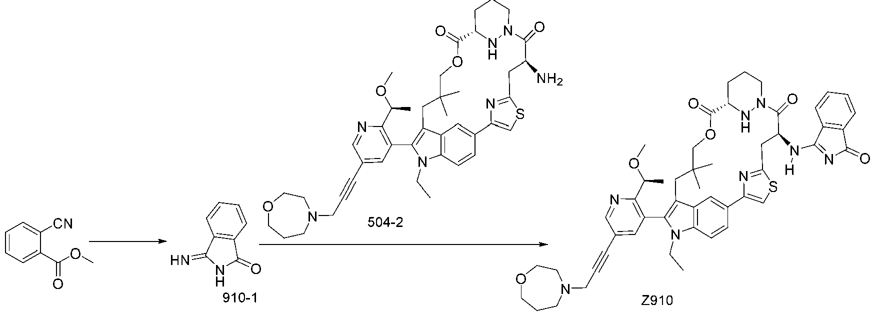

Step 1: Methyl 2-cyanobenzoate (3 g, 18.62 mmol) was added to a 7 M ammonia-methanol solution (50 mL) at 0 °C. The reaction system was stirred at room temperature for 72 h. The precipitated solid was filtered, and the filter cake was dried under reduced pressure to give compound 910-1 (1.1 g, yield: 40.4%) as a white solid. ES-API: [M+H]⁺ = 147.3.

Step 2: Compound 504-2 (50 mg, 0.065 mmol) was dissolved in isopropanol (1.5 mL), and N,N-diisopropylethylamine (42 mg, 0.33 mmol) and compound 910-1 (38 mg, 0.26 mmol) were added at room temperature. The reaction system was stirred at 85 °C for 18 h. The reaction mixture was cooled to room temperature and concentrated. The crude product was subjected to prep-HPLC (ammonium bicarbonate method) to give the desired product (6³S,4S,Z)-1²-(5-(3-(1,4-oxazepan-4-yl)prop-1-yn-1-yl)-2-((S)-1-methoxyethyl)pyridin-3-yl)-1¹-ethyl-10,10-dimethyl-4-((1-oxo-1H-isoindol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (z910, 20 mg, yield: 34.2%) as a white solid. ES-API: [M+H]⁺ = 897.3.

### Example 119. Synthesis of Compound Z911

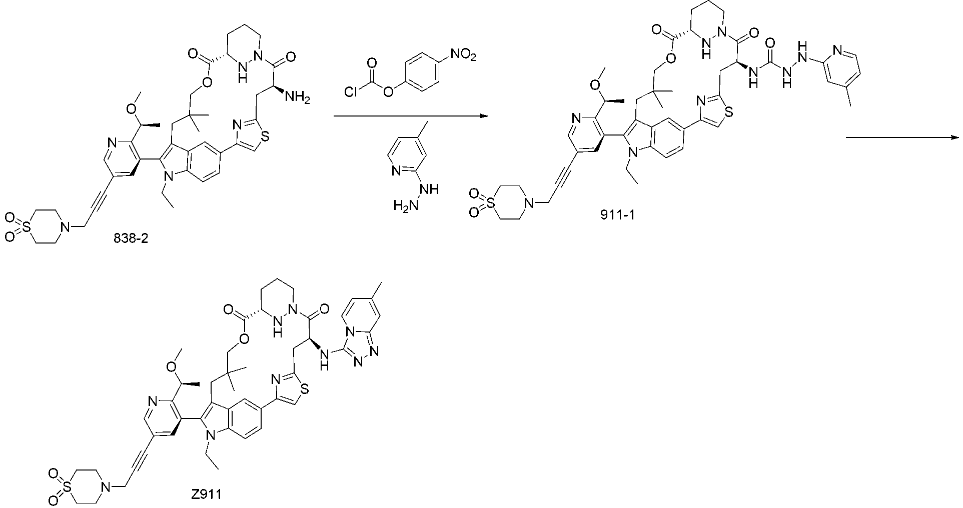

Step 1: N,N-Diisopropylethylamine (38.7 mg, 0.300 mmol) and p-nitrophenyl chloroformate (30.16 mg, 0.150 mmol) were sequentially added to a solution of compound 838-2 (80 mg, 0.100 mmol) in dichloromethane (5 mL) under an ice-water bath. After 5 min of reaction, 2-hydrazineyl-4-methylpyridine (37 mg, 0.300 mmol) was immediately added, and the mixture was reacted for another 0.5 h. When the reaction was completed as detected by LCMS, the reaction mixture was concentrated, and the crude product was purified by automated flash chromatography on silica gel (methanol/dichloromethane = 0-6%) to give compound 911-1 (50 mg, 0.053 mmol, yield: 53%). ES-API: [M+H]⁺ = 951.4.

Step 2: Pyridine (101.9 mg, 0.789 mmol) and phosphorus oxychloride (40.30 mg, 0.263 mmol) were sequentially added to a solution of compound 911-1 (50 mg, 0.053 mmol) in 1,2-dichloroethane (5 mL), and the mixture was reacted at 75 °C for 0.5 h under a nitrogen atmosphere. The reaction mixture was concentrated, and the residue was purified by a silica gel column to give a crude product. The crude product was purified by prep-HPLC (ammonium bicarbonate method) to give (6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z911, 11 mg, yield: 22.2%). ES-API: [M+H]⁺ = 933.4. ¹H NMR (400 MHz, DMSO-*d*₆) δ 8.82 (d, *J* = 2.1 Hz, 1H), 8.53 (d, *J =* 1.7 Hz, 1H), 8.26 (d, *J =* 7.1 Hz, 1H), 7.88 (d, *J =* 2.2 Hz, 1H), 7.79 (s, 1H), 7.75 (dd, *J =* 8.7, 1.6 Hz, 1H), 7.58 (d, *J* = 8.7 Hz, 1H), 7.22 (q, *J* = 1.3 Hz, 1H), 7.16 (d, *J =* 10.6 Hz, 1H), 6.71 (dd, *J* = 7.3, 1.5 Hz, 1H), 5.54 (t, *J* = 9.9 Hz, 1H), 5.20 (d, *J* = 12.1 Hz, 1H), 4.37 - 4.22 (m, 4H), 4.15 - 4.01 (m, 1H), 3.76 (s, 2H), 3.65 - 3.53 (m, 2H), 3.49 (d, *J* = 14.5 Hz, 1H), 3.30 - 3.27 (m, 1H), 3.26 (s, 3H), 3.19 - 3.15 (m, 4H), 3.07 - 3.02 (m, 4H), 2.99 (d, *J* = 14.3 Hz, 1H), 2.87 - 2.78 (m, 1H), 2.39 (d, *J* = 14.6 Hz, 1H), 2.30 (s, 3H), 2.12 (d, *J* = 9.2 Hz, 1H), 1.88 - 1.78 (m, 2H), 1.63 - 1.51 (m, 1H), 1.36 (d, *J* = 6.0 Hz, 3H), 0.94 (s, 3H), 0.88 (t, *J* = 7.1 Hz, 3H), 0.35 (s, 3H).

### Example 120. Synthesis of Compounds Z912, Z912-1, and Z912-2

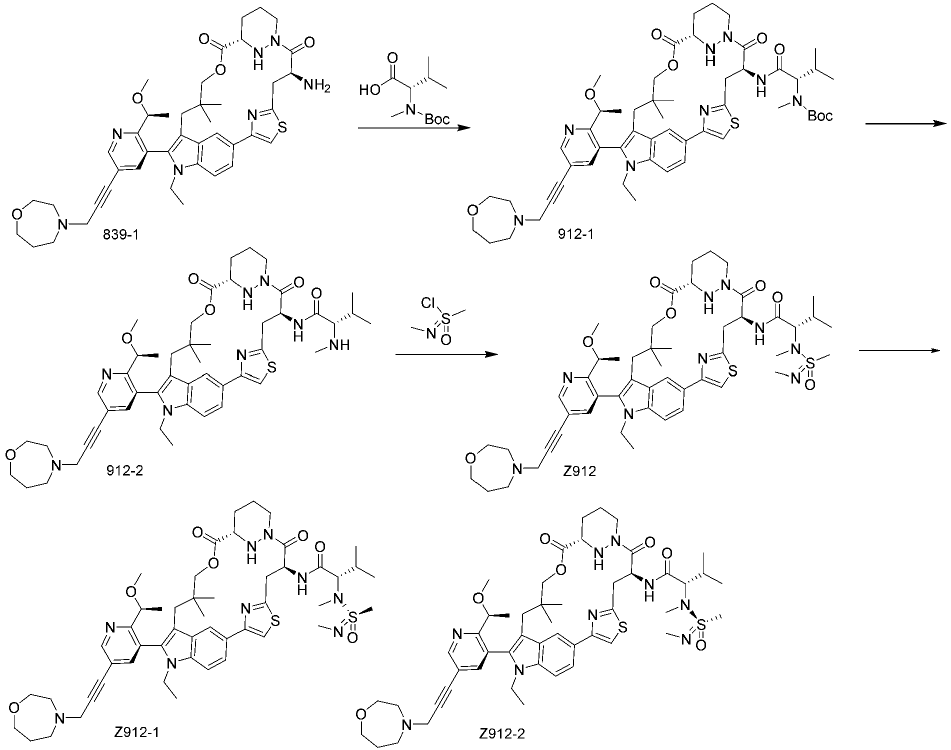

Step 1: Compound 839-1 (55 mg, 0.072 mmol) was dissolved in dichloromethane (3 mL). Boc-*N*-methyl-*L*-valine (24.85 mg, 0.107 mmol), O-(7-azabenzotriazol-1-yl)-*N,N,N',N'*-tetramethyluronium hexafluorophosphate (54.46 mg, 0.143 mmol), and *N*,*N'*-diisopropylethylamine (48 mg, 0.372 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-3%) to give compound 912-1 (55 mg, 0.056 mmol, yield: 78.26%) as a white solid. ES-API: [M+H]⁺ = 981.3.

Step 2: Compound 912-1 (55 mg, 0.056 mmol) was dissolved in methanol (1 mL), and a solution of hydrogen chloride in dioxane (4 M, 3 mL) was added to the solution described above. The mixture was stirred at room temperature for 2 h. After the reaction was completed, the reaction system was concentrated to give a hydrochloride. A saturated sodium bicarbonate solution (10 mL) was added to the hydrochloride described above, and the mixture was extracted with ethyl acetate (15 mL × 2). The organic phase was washed with saturated brine (10 mL × 3), dried over anhydrous sodium sulfate, filtered, and concentrated to give compound 912-2 (45 mg, 0.051 mmol, yield: 91.11 %) as a light white solid. ES-API: [M+H]⁺ = 881.3. Step 3: Compound 912-2 (45 mg, 0.051 mmol) and triethylamine (15 mg, 0.153 mmol) were dissolved in dichloromethane (5 mL), and *N*-methylmethanesulfonimidoyl chloride (65 mg, 0.511 mmol) was added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 2), dried over anhydrous sodium sulfate, filtered, and concentrated. The residue was purified by a flash silica gel column (methanol/dichloromethane: 0%-3%) to give a crude product (10 mg). The crude product was purified by prep-HPLC (ammonium bicarbonate method) to give (2*S*)-*N*-((6³*S*,4*S*,*Z*)-1²-(5-(3-(1,4-oxazepan-4-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-(*N*,*N*'-dimethylmethylsulfonimidoyl)-3-methylbutanamide (Z912, 3 mg, 0.003 mmol, yield: 6.05%) as a white solid. ES-API: [M+H]⁺ = 972.4.

Step 4: Compound Z912 (120 mg, 0.123 mmol) was subjected to chiral resolution (column type: IG 250 mm, 10 mm, 5 µm; mobile phase system: (A: acetonitrile; B: isopropanol; C: diethylamine); flow rate: 1 mL/min; A/B/C = 80/15/2; column temperature: 30 °C) to give 2 isomeric compounds. The structure of one of the isomeric compounds was arbitrarily designated as (2*S*)-*N*-((6³*S*,4*S*,*Z*)-1²-(5-(3-(1,4-oxazepan-4-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2((*R*)-*N*,*N*'-dimethylmethylsulfonimidoyl)-3-methylbutanamide (Z912-1, 35 mg, 0.036 mmol, yield: 29.3%, retention time: 8.842). ¹H NMR (400 MHz, CD₃OD) δ 8.77 (d, J = 2.0 Hz, 1H), 8.59 (s, 1H), 7.88 (d, J = 2.0 Hz, 1H), 7.72 (dd, J = 1.2 Hz, 8.4 Hz, 1H), 7.57 (s, 1H), 7.49 (d, J = 8.8 Hz, 1H), 5.80 (s, 1H), 4.46 - 7.37 (m, 1H), 4.35 - 4.24 (m, 2H), 4.23 - 4.15 (m, 1H), 4.05 - 4.13 (m, 1H), 3.94 (d, J = 11.2 Hz, 1H), 3.85 - 3.67 (m, 9H), 3.49 - 3.33 (m, 3H), 3.01 - 2.76 (m, 10H), 2.70 - 2.61 (m, 4H), 2.57 (s, 3H), 2.25 - 2.10 (m, 2H), 2.03 - 1.91 (m, 3H), 1.85 - 1.73 (m, 1H), 1.70 - 1.55 (m, 1H), 1.44 (d, J = 6.0 Hz, 3H), 1.05 (t, J = 6.8 Hz, 6H), 0.99 (t, J = 7.2 Hz, 3H), 0.90 (s, 3H), 0.54 (s, 3H).

The structure of the other isomeric compound was arbitrarily designated as (2*S)*-*N*-((6³*S*,4*S*,*Z*)-1²-(5-(3-(1,4-oxazepan-4-yl)prop-1-yn-1-yl)-2-((*S*)-1-methoxyethyl)pyridin-3-yl)-1¹-ethyl-10,10-dimethyl-5,7-dioxo-6¹,6²,6^{3,}6⁴,6⁵,6⁶-hexahydro-1¹*H-*8-oxa-2(4,2)-thiazola-1(5,3)-indola-6(1,3)-pyridazinacycloundecaphane-4-yl)-2((*S*)-*N*,*N'*-dimethylmethylsulfonimidoyl)-3-methylbutanamide (Z912-2, 20 mg, 0.021 mmol, yield: 17.1%, retention time: 11.813). Both were white solids. ES-API: [M+H]⁺ = 972.3. ¹H NMR (400 MHz, CD₃OD) δ 8.76 (d, *J =* 2.0 Hz, 1H), 8.57 (s, 1H), 7.88 (d, *J =* 2.0 Hz, 1H), 7.71 (dd, *J* = 1.2 Hz, 8.4 Hz, 1H), 7.55 (s, 1H), 7.49 (d, *J =* 8.8 Hz, 1H), 5.80 (d, *J =* 7.8 Hz, 1H), 4.41 (d, *J =* 13.2 Hz, 1H), 4.36 - 4.24 (m, 2H), 4.20 - 4.14 (m, 1H), 4.14 - 4.06 (m, 1H), 3.91 - 3.66 (m, 10H), 3.49 - 3.32 (m, 3H), 3.02 - 2.85 (m, 9H), 2.84 - 2.75 (m, 4H), 2.70 - 2.60 (m, 1H), 2.50 (s, 3H), 2.22 - 2.08 (m, 2H), 2.02 - 1.89 (m, 3H), 1.85 - 1.70 (m, 1H), 1.68 - 1.55 (m, 1H), 1.44 (d, *J =* 6.0 Hz, 3H), 1.10 - 1.02 (m, 6H), 0.99 (t, *J =* 7.2 Hz, 3H), 0.90 (s, 3H), 0.53 (s, 3H).

### Example 121. Synthesis of Compound Z915

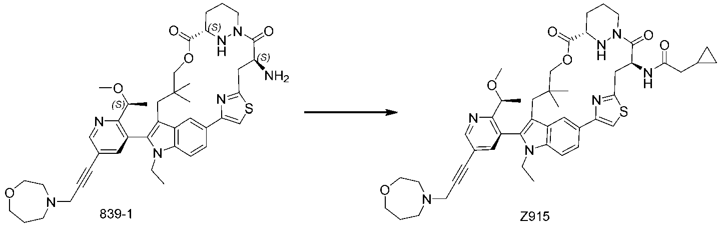

Step 1: Compound 839-1 (88 mg, 0.115 mmol) was dissolved in N,N-dimethylformamide (3 mL), and cyclopropylacetic acid (23 mg, 0.23 mmol), triethylamine (70 mg, 0.688 mmol), and O-(7-azabenzotriazol-1-yl)-N,N,N',N'-tetramethyluronium hexafluorophosphate (130 mg, 0.344 mmol) were sequentially added. The mixture was stirred at room temperature for 0.5 h. After the reaction was completed, ethyl acetate (10 mL) was added, and the mixture was washed with saturated brine (10 mL) and water (10 mL). The organic phase was dried over anhydrous sodium sulfate, filtered, and concentrated, and the residue was purified by a thin-layer chromatography column (dichloromethane/methanol = 20:1) to give N-((6³S,4S,Z)-(Rₐ)-1²-(5-(3-(1,4-oxolan-4-yl)prop-1-yn-1-yl)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-cyclopropylacetamide (Z915, 70 mg, yield: 71%, purity: 98%). ES-API [M+H]⁺ = 850.4. ¹H NMR (400 MHz, DMSO-d₆) δ 8.80 (d, *J =* 2.1 Hz, 1H), 8.51 (d, *J =* 1.2 Hz, 1H), 8.24 (d, *J =* 9.0 Hz, 1H), 7.90 - 7.80 (m, 2H), 7.76 (dd, *J =* 8.7, 1.5 Hz, 1H), 7.58 (d, *J = 8.7* Hz, 1H), 5.56 (t, *J* = 9.2 Hz, 1H), 5.10 (d, *J =* 12.2 Hz, 1H), 4.41 - 4.16 (m, 4H), 4.09 (dd, *J =* 14.8, 7.3 Hz, 1H), 3.72 - 3.56 (m, 8H), 3.34 (d, *J =* 8.7 Hz, 1H), 3.26 (s, 3H), 3.15 (dd, *J =* 14.7, 9.3 Hz, 1H), 2.98 (d, *J =* 14.5 Hz, 1H), 2.76 (dd, *J* = 7.0, 4.5 Hz, 5H), 2.37 (d, *J =* 14.3 Hz, 1H), 2.09 (d, *J =* 6.9 Hz, 3H), 1.90 - 1.75 (m, 4H), 1.53 (d, *J =* 8.1 Hz, 1H), 1.36 (d, *J* = 6.1 Hz, 3H), 0.93 (d, *J =* 5.0 Hz, 3H), 0.87 (t, *J =* 7.6 Hz, 3H), 0.84 - 0.81 (m, 1H), 0.45 (dd, *J =* 8.0, 1.5 Hz, 2H), 0.34 (s, 3H), 0.27 - 0.13 (m, 2H).

### Example 122. Synthesis of Compounds Z914, Z914-1, and Z914-2

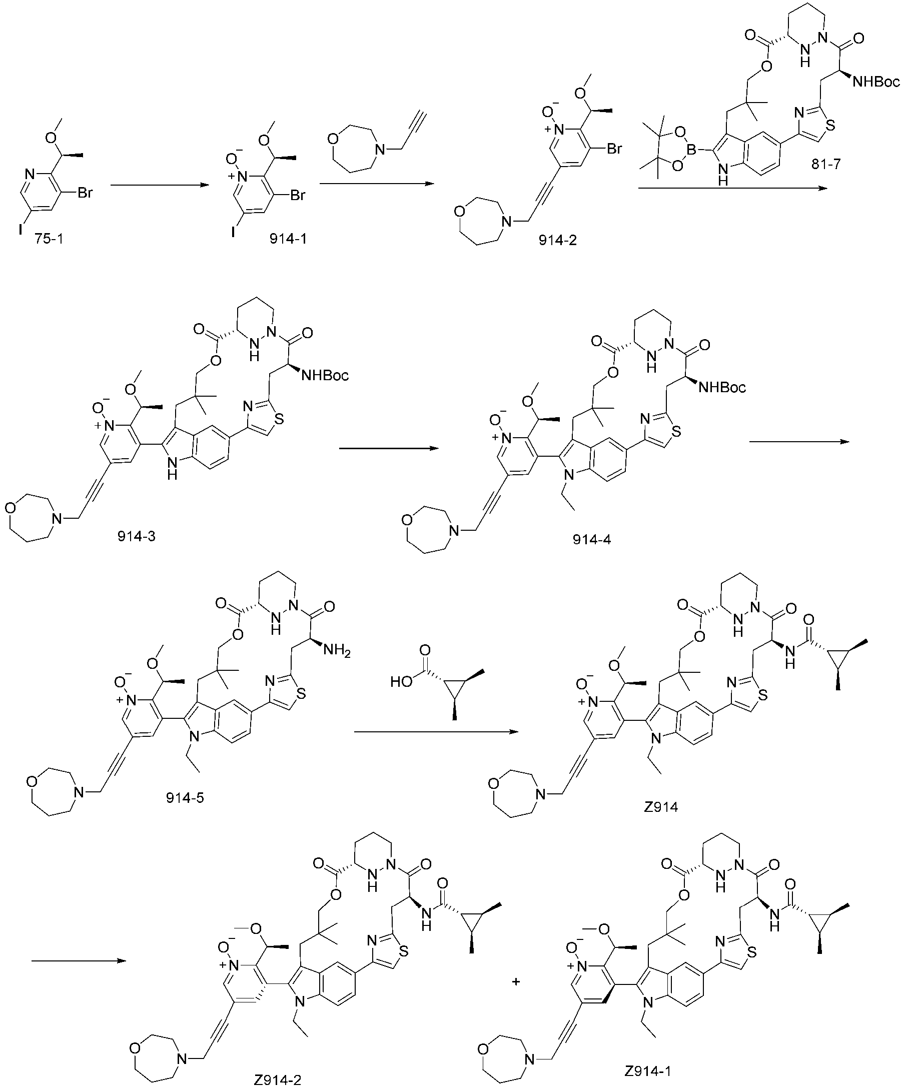

Step 1: Compound 75-1 (1.0 g, 2.92 mmol) was dissolved in dichloromethane (20 mL), and m-chloroperoxybenzoic acid (1.19 g, 5.85 mmol) was added under an ice bath. The reaction system was stirred at room temperature for 18 h. Dichloromethane (50 mL) was added to the reaction mixture, and the mixture was washed sequentially with saturated sodium thiosulfate (20 mL), saturated sodium bicarbonate (30 mL), and saturated brine (30 mL). The organic phase was dried over anhydrous sodium sulfate and filtered, and the filtrate was concentrated under reduced pressure. The crude product was purified by a flash silica gel column (methanol/dichloromethane: 0-2%) to give compound 914-1 (600 mg, yield: 57.3%) as a pale yellow liquid. ES-API: [M+H]⁺ = 358.0, 359.9.

Step 2: Compound 914-1 (500 mg, 1.40 mmol), bis(triphenylphosphine)palladium(II) dichloride (49 mg, 0.07 mmol), and copper(I) iodide (27 mg, 0.14 mmol) were dissolved in acetonitrile (20 mL) under a nitrogen atmosphere. A solution of 4-(prop-2-en-1-yl)-1,4-oxazepane (214 mg, 1.54 mmol) and N,N-diisopropylethylamine (361 mg, 2.79 mmol) in acetonitrile (0.5 mL) was added dropwise under an ice bath. The reaction system was stirred at room temperature for 1 h. Ethyl acetate (100 mL) was added to the reaction mixture, and the mixture was washed with saturated brine (30 mL). The organic phase was dried over anhydrous sodium sulfate and filtered, and the filtrate was concentrated under reduced pressure. The crude product was purified by a flash silica gel column (methanol/dichloromethane: 0-6%) to give compound 914-2 (500 mg, yield: 96.9%) as a yellow liquid. ES-API: [M+H]⁺ = 369.0, 371.0.

Step 3: Compound 81-7 (250 mg, 0.36 mmol) and compound 914-2 (266 mg, 0.72 mmol) were dissolved in dioxane (1 mL), toluene (3 mL), and water (1 mL) under a nitrogen atmosphere, and [1,1'-bis(di-tert-butylphosphino)ferrocene]dichloropalladium(II) (47 mg, 0.072 mmol) and potassium phosphate (153 mg, 0.72 mmol) were added. The reaction system was stirred at 75 °C for 3 h. Ethyl acetate (50 mL) was added to the reaction mixture, and the mixture was washed with saturated brine (15 mL). The organic phase was dried over anhydrous sodium sulfate and filtered, and the filtrate was concentrated under reduced pressure. The crude product was purified by a flash silica gel column (methanol/dichloromethane: 0-8%) to give compound 914-3 (150 mg, yield: 48.6%) as a yellow solid. ¹H NMR (400 MHz, DMSO-d₆) δ 11.32 (s, 1H), 8.53 - 8.41 (m, 2H), 7.76 (s, 1H), 7.66 (dd, *J =* 8.4, 1.2 Hz, 1H), 7.46 (d, *J =* 1.2 Hz, 1H), 7.36 (d, *J =* 8.4 Hz, 1H), 7.30 (d, *J =* 9.2 Hz, 1H), 5.29 - 5.13 (m, 1H), 5.04 (d, *J =* 12.0 Hz, 1H), 4.64 (q, *J =* 6.4 Hz, 1H), 4.30 - 4.11 (m, 2H), 3.74 - 3.59 (m, 7H), 3.55 (d, *J =* 11.6 Hz, 1H), 3.24 - 3.14 (m, 2H), 3.08 - 2.95 (m, 4H), 2.83 - 2.66 (m, 5H), 2.41 - 2.29 (m, 1H), 2.18 - 2.06 (m, 1H), 1.88 - 1.71 (m, 4H), 1.59 - 1.49 (m, 1H), 1.46 - 1.31 (m, 12H), 0.99 (s, 3H), 0.56 (s, 3H). ES-API: [M+H]⁺ = 856.3.

Step 4: Compound 914-3 (125 mg, 0.15 mmol) was dissolved in N,N-dimethylformamide (5 mL), and cesium carbonate (143 mg, 0.44 mmol) was added at room temperature. The reaction system was stirred at room temperature for 1.5 h. Then iodoethane (68 mg, 0.44 mmol) was added, and the reaction system was stirred at room temperature for another 2 h. Ethyl acetate (50 mL) was added to the reaction mixture, and the mixture was washed with saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-6%) to give compound 914-4 (105 mg, yield: 81.3%) as a pale yellow solid. ES-API: [M+H]⁺ = 884.3.

Step 5: Compound 914-4 (90 mg, 0.102 mmol) was dissolved in absolute methanol (1 mL), and a 4.0 M hydrogen chloride/dioxane solution (4 mL) was added at 0 °C. The reaction system was stirred at room temperature for 1 h. The reaction mixture was concentrated to give compound 914-5 (79 mg, yield: 99.0%) as a yellow solid. ES-API: [M+H]⁺ = 784.3.

Step 6: Compound 914-5 (79 mg, 0.10 mmol) was dissolved in dichloromethane (10 mL), and (1r,2R,3S)-2,3-dimethylcyclopropane-1-carboxylic acid (17 mg, 0.15 mmol), O-(7-azabenzotriazol-1-yl)-N,N,N',N'-tetramethyluronium hexafluorophosphate (77 mg, 0.20 mmol), and N,N'-diisopropylethylamine (78 mg, 0.60 mmol) were added. The reaction system was stirred at room temperature for 1 h. Ethyl acetate (50 mL) was added to the reaction mixture, and the mixture was washed with saturated brine (20 mL). The organic phase was dried over anhydrous sodium sulfate and filtered, and the filtrate was concentrated under reduced pressure. The crude product was purified by prep-HPLC (ammonium bicarbonate method) to give the desired product 5-(3-(1,4-oxazepan-4-yl)prop-1-yn-1-yl)-3-((6³S,4S,Z)-4-((1r,2R,3S)-2,3-dimethylcyclopropane-1-carboxamido)-1'-ethyl-10,10-dimethyl-5,7-dioxo-6¹,6²,6³,6⁴,6⁵,6⁶-hexahydro-1'H-8-oxa-2(4,2)-thiazola-1(5,3)-indola-6(1,3)-pyridazinacycloundecaphane-1²-yl)-2-((S)-1-methoxyethyl)pyridine-1-oxide (Z914, 85 mg, yield: 95.8%) as a white solid. ES-API: [M+H]⁺ = 880.3.

Step 7: Compound Z914 (84 mg, 0.095 mmol) was subjected to preparative chiral resolution (separation column: IB, 250 mm × 4.6 mm × 5 µm, mobile phase: n-hexane:ethanol:diethylamine = 40:60:2, flow rate: 1 mL/min, column temperature: 30 °C) to give 2 isomeric compounds. The structure of one of the isomeric compounds was arbitrarily designated as 5-(3-(1,4-oxazepan-4-yl)prop-1-yn-1-yl)-(Sₐ)-3-((6³S,4S,Z)-4-((1r,2R,3S)-2,3-dimethylcyclopropane-1-carboxamido)-1'-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((S)-1-methoxyethyl)pyridine-1-oxide (Z914-2, 20 mg, retention time: 6.527 min, yield: 23.8%) as a white solid. ES-API: [M+H]⁺ = 880.4.

The structure of the other isomeric compound was arbitrarily designated as 5-(3-(1,4-oxazepan-4-yl)prop-1-yn-1-yl)-(Rₐ)-3-((6³S,4S,Z)-4-((1r,2R,3S)-2,3-dimethylcyclopropane-1-carboxamido)-1'-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-2-((S)-1-methoxyethyl)pyridine-1-oxide (Z914-1, 20 mg, retention time: 11.618 min, yield: 23.8%) as a white solid. ES-API: [M+H]⁺ = 880.4.

### Example 123. Synthesis of Compounds Z913, Z913-1, and Z913-2

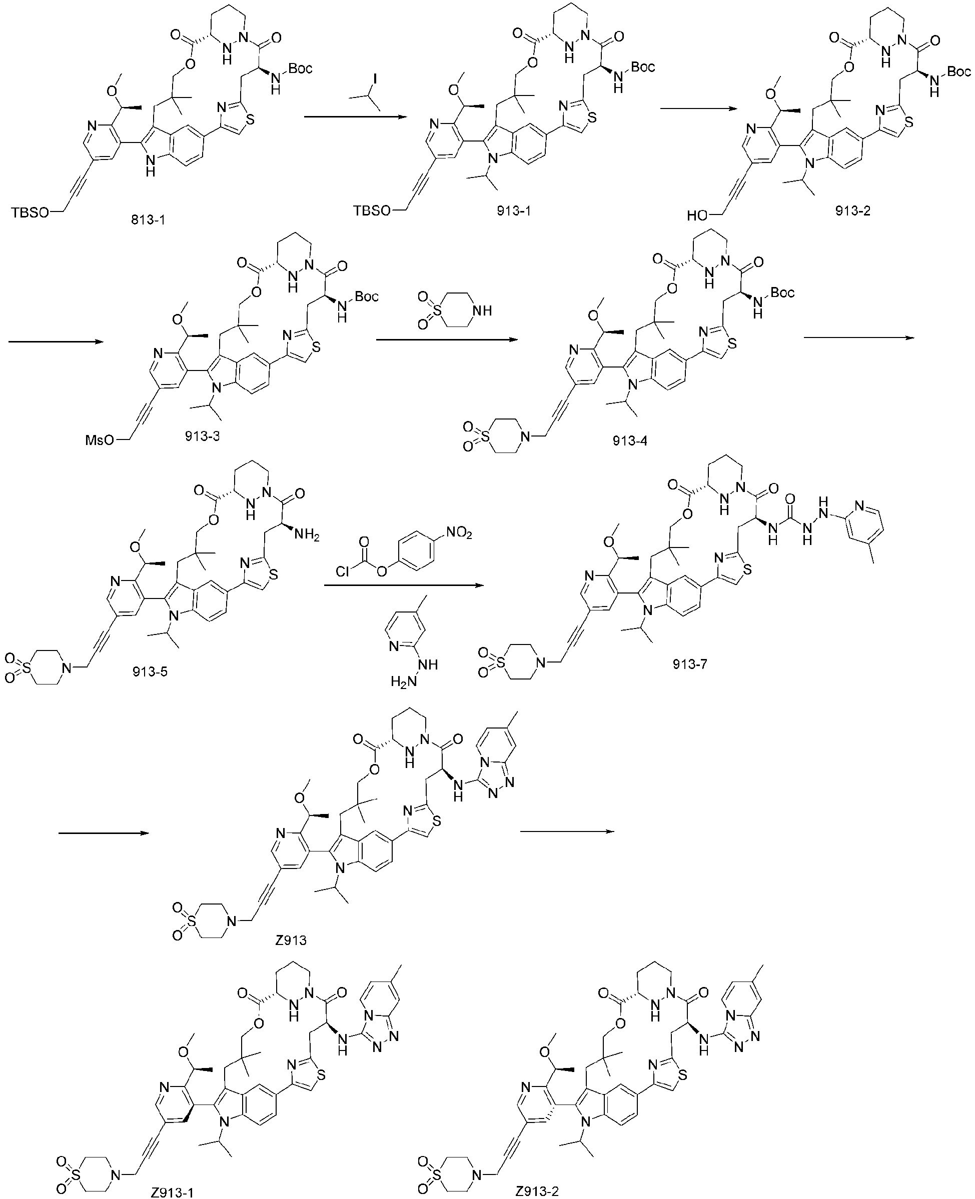

Step 1: Compound 813-1 (1000 mg, 1.148 mmol) was dissolved in N,N-dimethylformamide (10 mL), and cesium carbonate (3731.00 mg, 11.480 mmol) and 2-iodopropane (1.148 mL, 11.479 mmol) were added. The mixture was heated to 50 °C and stirred for 48 h under a nitrogen atmosphere. The mixture was cooled to room temperature, extracted with ethyl acetate (30 mL × 3), washed sequentially with water (50 mL) and saturated brine (50 mL), dried over anhydrous sodium sulfate, filtered, and concentrated, and the residue was purified by silica gel column chromatography (ethyl acetate/dichloromethane = 1/1) to give compound 913-1 (1 g, 1.096 mmol, yield: 95%). ES-API [M+H] ⁺ = 913.4.

Step 2: Compound 913-1 (1 g, 1.096 mmol) was dissolved in tetrabutylammonium fluoride (10 mL, a 1 mmol solution in tetrahydrofuran). The mixture was stirred at room temperature for 1 h. The mixture was diluted with water (20 mL), extracted with ethyl acetate (30 mL × 3), washed sequentially with water (50 mL) and saturated brine (50 mL), dried over anhydrous sodium sulfate, filtered, and concentrated to give compound 913-2 (0.87 g, 1.096 mmol, crude product). ES-API [M+H]⁺ = 799.3.

Step 3: N,N-Diisopropylethylamine (422.21 mg, 3.267 mmol) and methanesulfonic anhydride (284.23 mg, 1.633 mmol) were added to a solution of compound 913-2 (0.87 g, 1.096 mmol) in dichloromethane (15 mL). The mixture was stirred at 25 °C for 1 h. When the reaction was completed as detected by LCMS, the reaction mixture was diluted with 20 mL of dichloromethane, and then the mixture was washed with saturated brine (15 mL × 3). The organic phase was dried over anhydrous sodium sulfate, filtered, and concentrated, and the residue was purified by silica gel column chromatography (ethyl acetate/dichloromethane = 1/1) to give compound 913-3 (880 mg, 1.003 mmol, yield: 92.14%). ES-API: [M+H]⁺ = 877.3. Step 4: Thiomorpholine 1,1-dioxide (678.14 mg, 5.017 mmol) was added to a solution of compound 913-3 (880 mg, 1.003 mmol) in acetonitrile (15 mL). The mixture was stirred at 25 °C for 18 h. When the reaction was completed as detected by LCMS, the reaction mixture was concentrated, and the residue was purified by silica gel column chromatography (methanol/dichloromethane = 1/20) to give compound 913-4 (850 mg, 0.928 mmol, yield: 92.47%). ES-API: [M+H]⁺ = 916.3. Step 5: Compound 913-4 (850 mg, 0.928 mmol) was dissolved in methanol (2 mL), and hydrochloric acid (10 mL, a 4 M solution in dioxane) was slowly added under an ice-water bath. The reaction mixture was reacted for 1 h, concentrated, diluted with dichloromethane (20 mL), washed sequentially with a saturated aqueous sodium bicarbonate solution (10 mL) and saturated brine (10 mL), dried over anhydrous sodium sulfate, filtered, and concentrated to give crude compound 913-5 (605 mg, 0.742 mmol, yield: 80%), which was directly used in the next step. ES-API: [M+H]⁺ = 816.2.

Step 6: N,N-Diisopropylethylamine (316.77 mg, 2.451 mmol) and p-nitrophenyl chloroformate (148.20 mg, 0.735 mmol) were sequentially added to a solution of compound 913-5 (400 mg, 0.490 mmol) in dichloromethane (5 mL) under an ice-water bath. After 10 min of reaction, 2-hydrazineyl-4-methylpyridine (181.11 mg, 1.470 mmol) was immediately added in one portion, and the mixture was reacted for another 0.5 h under an ice-water bath. When the reaction was completed as detected by LCMS, the reaction mixture was concentrated, and the crude product was purified by automated flash chromatography on silica gel (methanol/dichloromethane = 0-6%) to give compound 913-7 (380 mg, 0.393 mmol, yield: 80.3%). ES-API: [M+H]⁺ = 966.2.

Step 7: Pyridine (283 mg, 3.574 mmol) and phosphorus oxychloride (183 mg, 0.191 mmol) were sequentially added to a solution of compound 913-7 (230 mg, 0.238 mmol) in 1,2-dichloroethane (10 mL), and the reaction mixture was reacted at 75 °C for 30 min under a nitrogen atmosphere and concentrated. The residue was purified by a silica gel column to give crude compound Z913. The crude compound was purified by prep-HPLC (ammonium bicarbonate method) to give 2 isomeric compounds. The structure of one of the isomeric compounds was arbitrarily designated as (6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-isopropyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z913-1, 8 mg, yield: 3.54%, retention time: 7.178 min), ES-API: [M+H]⁺ = 947.2. The structure of the other isomeric compound was arbitrarily designated as (6³S,4S,Z)-(Sₐ)-1²-(5-(3-(1,1-dioxidothiomorpholino)prop-1-yn-1-yl)-2-((S)-1-methoxyethyl)pyridin-3-yl)-1¹-isopropyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z913-2, 10 mg, yield: 4.4%, retention time: 7.296 min), ES-API: [M+H]⁺ = 947.2. ¹H NMR (400 MHz, DMSO-*d*₆) δ 8.83 (d, *J* = 2.1 Hz, 1H), 8.53 (s, 1H), 8.32 (d, *J* = 7.2 Hz, 1H), 7.94 (d, *J* = 2.1 Hz, 1H), 7.78 (s, 1H), 7.69 (s, 2H), 7.32 - 7.23 (m, 2H), 6.76 (dd, *J* = 7.2, 1.5 Hz, 1H), 5.50 (t, *J* = 9.9 Hz, 1H), 5.17 (d, *J* = 12.2 Hz, 1H), 4.34 - 4.21 (m, 2H), 4.09-3.97 (m, 2H), 3.77 (s, 2H), 3.62 (q, *J =* 11.0 Hz, 2H), 3.50 (d, *J =* 14.5 Hz, 1H), 3.19-3.16 (m, 4H), 3.11 (s, 3H), 3.05-3.03 (m, 4H), 2.85-2.80 (m, 1H), 2.32 (s, 3H), 2.26 (d, *J* = 14.4 Hz, 1H), 2.16 (t, *J* = 10.2 Hz, 1H), 1.84 (s, 2H), 1.56 (d, *J =* 6.9 Hz, 3H), 1.43 (d, *J =* 6.9 Hz, 3H), 1.29 - 1.17 (m, 6H), 0.94 (s, 3H), 0.50 (s, 3H).

### Example 124. Synthesis of Compound Z940

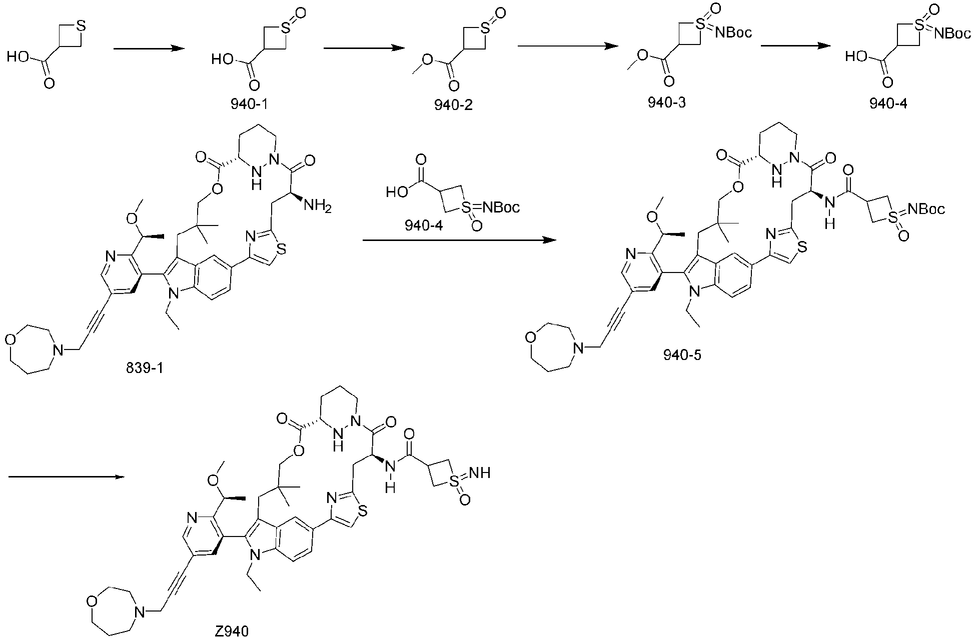

Step 1: 3-Thietanecarboxylic acid (120 mg, 1.016 mmol) was dissolved in dichloromethane (5 mL), and m-chloroperoxybenzoic acid (206.27 mg, 1.016 mmol) was added under an ice-water bath. The mixture was reacted at room temperature for 3 h. The mixture was concentrated under reduced pressure at room temperature, washed with methyl tert-butyl ether, and filtered under vacuum to give compound 940-1 (100 mg, 0.745 mmol, yield: 73.39%) as a white solid, ES-API: [M+H]⁺ = 135.1.

Step 2: Compound 940-1 (110 mg, 0.820 mmol) was dissolved in toluene (1 mL) and methanol (0.3 mL), and trimethylsilyldiazomethane (0.310 mL, 2.460 mmol) and glacial acetic acid (0.094 mL, 1.640 mmol) were added under an ice-water bath. The mixture was reacted at room temperature for 3 h and concentrated under reduced pressure to give crude compound 940-2, which was directly used in the next step. ES-API: [M+H]⁺ = 149.2.

Step 3: Compound 940-2 (50 mg, 0.337 mmol), magnesium oxide (53.99 mg, 1.350 mmol), iodobenzene diacetate (164.05 mg, 0.506 mmol), and dichloromethane (5 mL) were added to rhodium acetate (5.98 mg, 0.013 mmol), and the mixture was reacted at 40 °C for 3 h. The mixture was filtered under vacuum and concentrated under reduced pressure, and the residue was purified by a flash silica gel column (ethyl acetate/petroleum ether: 0%-70%) to give compound 940-3 (70 mg, 0.266 mmol, yield: 78.78%). ES-API: [M-Boc+H]⁺ = 164.2.

Step 4: Compound 940-3 (170 mg, 0.646 mmol) was dissolved in methanol (0.3 mL), tetrahydrofuran (1 mL), and water (0.3 mL), and lithium hydroxide monohydrate (54.18 mg, 1.291 mmol) was added. The mixture was reacted at room temperature for 3 h. The pH was adjusted to 4 with 1 N hydrochloric acid, and the mixture was extracted with ethyl acetate (10 mL), washed with saturated brine (5 mL), dried over anhydrous sodium sulfate, filtered, and concentrated to give compound 940-4 (65 mg, 0.261 mmol, yield: 40.39%) as an oily liquid, ES-API: [M-56+H]⁺ = 194.1.

Step 5: Compound 839-1 (50 mg, 0.065 mmol) was dissolved in N,N-dimethylformamide (1 mL), and compound 940-4 (65 mg, 0.261 mmol), O-(7-azabenzotriazol-1-yl)-*N,N,N',N'*-tetramethyluronium hexafluorophosphate (37.11 mg, 0.098 mmol), and *N*,*N*'-diisopropylethylamine (0.1 mL, 0.326 mmol) were added. The reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0-5%) to give compound 940-5 (30 mg, 0.030 mmol, yield: 46.20%), ES-API: [M+H]⁺ = 999.3.

Step 6: Compound 940-5 (10 mg, 0.010 mmol) was dissolved in dichloromethane (2 mL), and trifluoroacetic acid (1 mL) was added. The reaction system was stirred at room temperature for 2 h. The mixture was concentrated under reduced pressure, and the crude product was purified by prep-HPLC (ammonium bicarbonate method) to give *N*-((6³*S*,4*S*,*Z*)-(Rₐ)-1²-(5-(3-(1,4-oxazepan-4-yl)propyn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-1-imino-1λ⁶-thietane-3-carboxamide-1-oxide (Z940, 4 mg, 0.004 mmol, yield: 44.40%) as a white solid. ES-API: [M+H]⁺ = 899.2.

### Example 125. Synthesis of Compound Z927

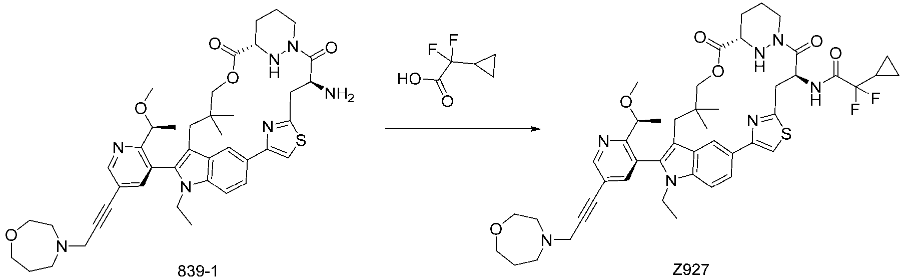

Step 1: N,N-Diisopropylethylamine (35 mg, 0.27 mmol) and O-(7-azabenzotriazol-1-yl)-N,N,N',N'-tetramethyluronium hexafluorophosphate (52 mg, 0.14 mmol) were sequentially added to a solution of 2-cyclopropyl-2,2-difluoroacetic acid (15 mg, 0.11 mmol) and compound 839-1 (70 mg, 0.09 mmol) in dichloromethane (2 mL). The mixture was stirred at room temperature for 1 h. After the reaction was completed, the mixture was washed with water (2 mL) and concentrated, and the residue was purified by prep-HPLC (ammonium bicarbonate method) to give N-((6³S,4S,Z)-(Rₐ)-12-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-cyclopropyl-2,2-difluoroacetamide (Z927, 14.91 mg, yield: 18.5%, purity: 98%). ES-API: [M+H]⁺ = 886.5. ¹H NMR (400 MHz, DMSO-d6) δ 9.03 (dd, J = 8.8, 1.2 Hz, 1H), 8.80 (d, J = 2.0 Hz, 1H), 8.51 (d, J = 1.2 Hz, 1H), 7.85 - 7.82 (m, 2H), 7.76 (dd, J = 8.7 Hz, 1H), 7.59 (d, J = 8.8 Hz, 1H), 5.54 (t, J = 8.8 Hz, 1H), 5.20 (d, J = 12.4 Hz, 1H), 4.40 - 4.18 (m, 4H), 4.08 (q, J = 7.6 Hz, 1H), 3.71 - 3.66 (m, 4H), 3.66 - 3.62 (m, 2H), 3.60 - 3.52 (m, 3H), 3.40 - 3.34 (m, 1H), 3.26 (s, 3H), 2.97 (d, J = 14.0 Hz, 1H), 2.81 - 2.72 (m, 5H), 2.44 - 2.34 (m, 1H), 2.16 - 2.06 (m, 1H), 1.90 - 1.76 (m, 4H), 1.69 - 1.46 (m, 2H), 1.36 (d, J = 6.0 Hz, 3H), 0.93 (s, 3H), 0.87 (t, J = 7.2 Hz, 3H), 0.81 - 0.73 (m, 1H), 0.74 - 0.66 (m, 3H), 0.34 (s, 3H).

### Example 126. Synthesis of Compound Z920

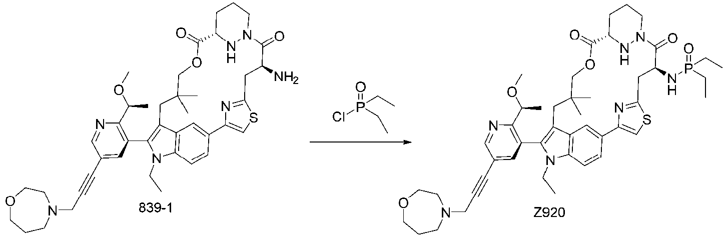

Step 1: Compound 839-1 (85 mg, 0.111 mmol) and N,N-diisopropylethylamine (286 mg, 2.21 mmol) were dissolved in N,N-dimethylformamide (2.5 mL), and diethylphosphinic chloride (37 mg, 0.332 mmol) was added under an ice bath. The reaction system was stirred at room temperature for 2 h. Water (20 mL) was added to the reaction mixture, and the mixture was extracted with ethyl acetate (40 mL). The organic phase was washed with saturated brine (10 mL), dried over anhydrous sodium sulfate, and filtered, and the filtrate was concentrated under reduced pressure. The crude product was purified by prep-HPLC (formic acid method 1) to give the desired product N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵ ,6⁶-hexahydro-1¹H-8-oxa-2(4,2)-thiazola-1(5,3)-indola-6(1,3)-pyridazinacycloundecaphane-4-yl)-P,P-diethylphosphoramidite formate (Z920, 10 mg, yield: 9.8%) as a white solid. ES-API: [M+H]⁺ = 872.3.

### Example 127. Synthesis of Compound Z939

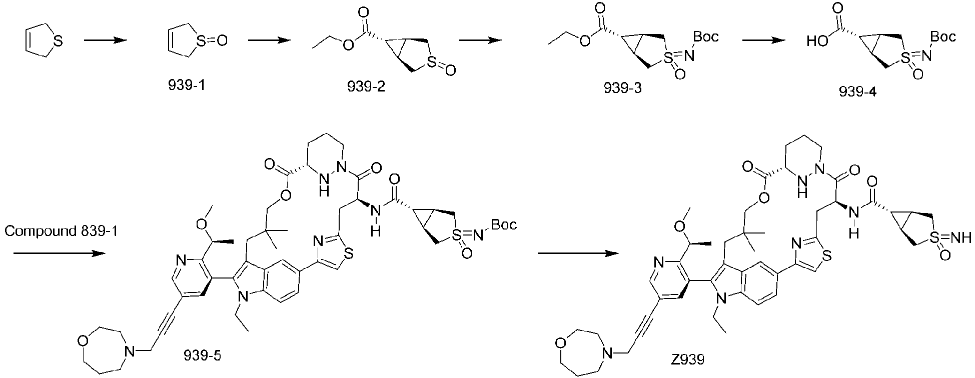

Step 1: 2,5-Dihydrothiophene (200 mg, 2.322 mmol) was dissolved in methanol (150 mL), and the mixture was cooled to 0 °C. An aqueous solution of sodium periodate (1986.21 mg, 9.286 mmol, 150 mL) was added dropwise to the solution described above. After the dropwise addition was completed, the reaction system was stirred at room temperature for 17 h. After the reaction was completed, the mixture was concentrated under reduced pressure to remove methanol. The residual aqueous solution was extracted with (chloroform/methanol = 10:1, 100 mL × 2). The organic phases were combined, dried over anhydrous sodium sulfate, filtered, and concentrated to give crude compound 939-1 (600 mg, 5.874 mmol, yield: 63.25%) as a colorless oily liquid.

Step 2: Compound 939-1 (1000 mg, 9.790 mmol) and rhodium(II) acetate dimer (216.34 mg, 0.489 mmol) were dissolved in dichloromethane (20 mL). A solution of ethyl diazoacetate in tetrahydrofuran (1 M, 3.01 mL, 29.369 mmol) was slowly added dropwise to the solution described above under a nitrogen atmosphere. After the addition was completed, the reaction system was stirred at room temperature for 5 h. After the reaction was completed, the reaction mixture was filtered using a sand core funnel, and the filtrate was concentrated under reduced pressure to give a crude product. The crude product was purified by a flash silica gel column (ethyl acetate/petroleum ether: 0%-25%) to give compound 939-2 (1000 mg, 5.312 mmol, yield: 54.27%) as a white solid. ES-API: [M+H]⁺ = 189.1.

Step 3: Compound 939-2 (1000 mg, 5.312 mmol) was dissolved in dichloromethane (30 mL), and tert-butyl carbamate (933.52 mg, 7.969 mmol), rhodium(II) acetate dimer (234.80 mg, 0.531 mmol), iodobenzene diacetate (3443.58 mg, 10.625 mmol), and magnesium oxide (856.35 mg, 21.249 mmol) were added to the solution described above. After the addition was completed, the reaction system was stirred at room temperature for 3 h. After the reaction was completed, the reaction mixture was filtered using a sand core funnel, and the filtrate was concentrated under reduced pressure to give a crude product. The crude product was purified by a flash silica gel column (tetrahydrofuran/petroleum ether: 0%-30%) to give compound 939-3 (450 mg, 1.483 mmol, yield: 27.92%) as a white solid. ES-API: [M+H]⁺ = 304.1.

Step 4: Compound 939-3 (80 mg, 0.264 mmol) was dissolved in acetonitrile (3 mL) and methanol (0.2 mL), and lithium chloride (55.89 mg, 1.319 mmol), triethylamine (0.183 mL, 1.319 mmol), and water (5 mg, 0.264 mmol) were added to the solution described above. After the addition was completed, the reaction system was stirred at room temperature for 17 h. After the reaction was completed, the reaction mixture was filtered using a sand core funnel, and the filtrate was concentrated to give compound 939-4 (80 mg, crude product) as a yellow oily liquid. ES-API: [M-56+H]⁺ = 223.1.

Step 5: Compound 839-1 (40 mg, 0.052 mmol) was dissolved in dichloromethane (3 mL). Compound 939-4 (80 mg, crude product), O-(7-azabenzotriazol-1-yl)-*N,N,N',N'*-tetramethyluronium hexafluorophosphate (39.61 mg, 0.104 mmol), and *N,N'-*diisopropylethylamine (20 mg, 0.156 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-3%) to give compound 939-5 (20 mg, 0.020 mmol, yield: 37.45%) as a light yellow solid.

ES-API: $\frac{1}{2} {\left[M+2H\right]}^{+} = 513.1$.

Step 6: Compound 939-5 (20 mg, 0.020 mmol) was dissolved in dichloromethane (1 mL), and trifluoroacetic acid (1 mL) was added to the solution described above. After the addition was completed, the reaction system was stirred at room temperature for 2 h. After the reaction was completed, the reaction system was concentrated to give a residue. A saturated sodium bicarbonate solution (10 mL) was added to the residue described above, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by prep-HPLC (ammonium bicarbonate method) to give (1R,5S,6s)-N-((6³S,4S,Z)-(R.)-1²-(5-(3-(1,4-oxazepan-4-yl)prop-1-yn-1-y1)-2-((S)-1-methoxyethyl)pyridin-3-yl)-1¹-ethyl-10,10-dimethyl-5,7-dioxo-6¹,6²,6³,6⁴,6⁵ 6⁶-hexahydro-1¹H-8-oxa-2(4,2)-thiazola-1(5,3)-indola-6(1,3)-pyridazinacycloundecaphane-4-yl)-3-imino-3λ⁶ -thiabicyclo[3.1.0]hexane-6-carboxamide 3-oxide (Z939, 5 mg, 0.005 mmol, yield: 27.70%) as a white solid. ES-API: [M+H]⁺ = 925.3.

### Example 128. Synthesis of Compound Z955

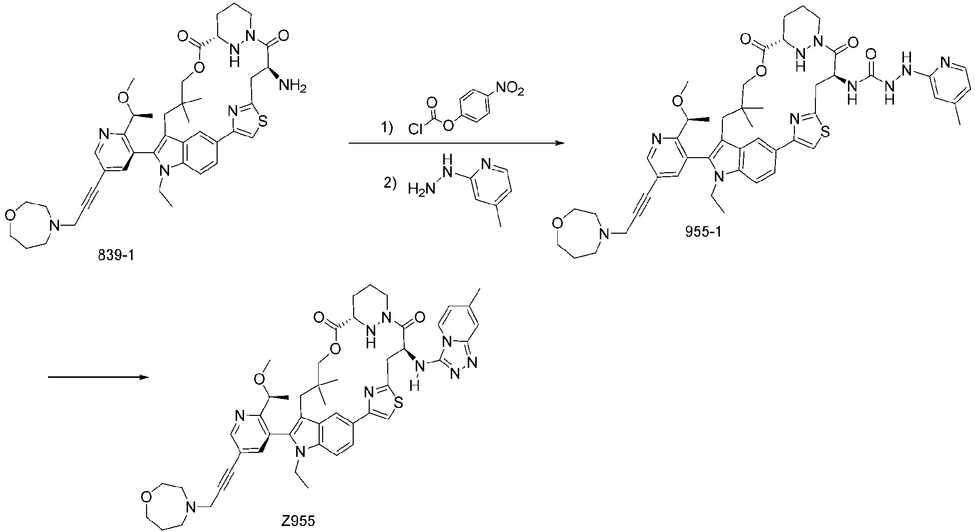

Step 1: Compound 839-1 (160 mg, 0.208 mmol) and N,N-diisopropylethylamine (135.0 mg, 1.042 mmol) were dissolved in dichloromethane (10.0 mL), and nitrophenyl chloroformate (50.4 mg, 0.250 mmol) was added at 0 °C. The reaction system was stirred for 1 h under an ice bath, and then 2-hydrazineyl-4-methylpyridine (102.0 mg, 0.833 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (7 M ammonia-methanol solution/dichloromethane: 0-5%) to give compound 955-1 (180 mg, 0.196 mmol, yield: 94.20%) as a yellow solid, ES-API: [M+H]⁺ = 917.1.

Step 2: Compound 955-1 (180 mg, 0.196 mmol) and pyridine (0.237 mL, 2.944 mmol) were dissolved in dichloromethane (5 mL) and phosphorus oxychloride (150.45 mg, 0.981 mmol). The reaction system was stirred at 45 °C for 5 min. The reaction mixture was cooled to room temperature and concentrated. The residue was purified by a flash silica gel column (7 M ammonia-methanol solution/dichloromethane: 0-5%) to give the desired product (6³S,4S,Z)-1²-(5-(3-(1,4-oxazepan-4-yl)prop-1-yn-1-yl)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z955, 15.0 mg, 0.0166 mmol, yield: 8.5%) as a yellow solid. ES-API: [M+H]⁺ = 899.3.

### Example 129. Synthesis of Compound Z956

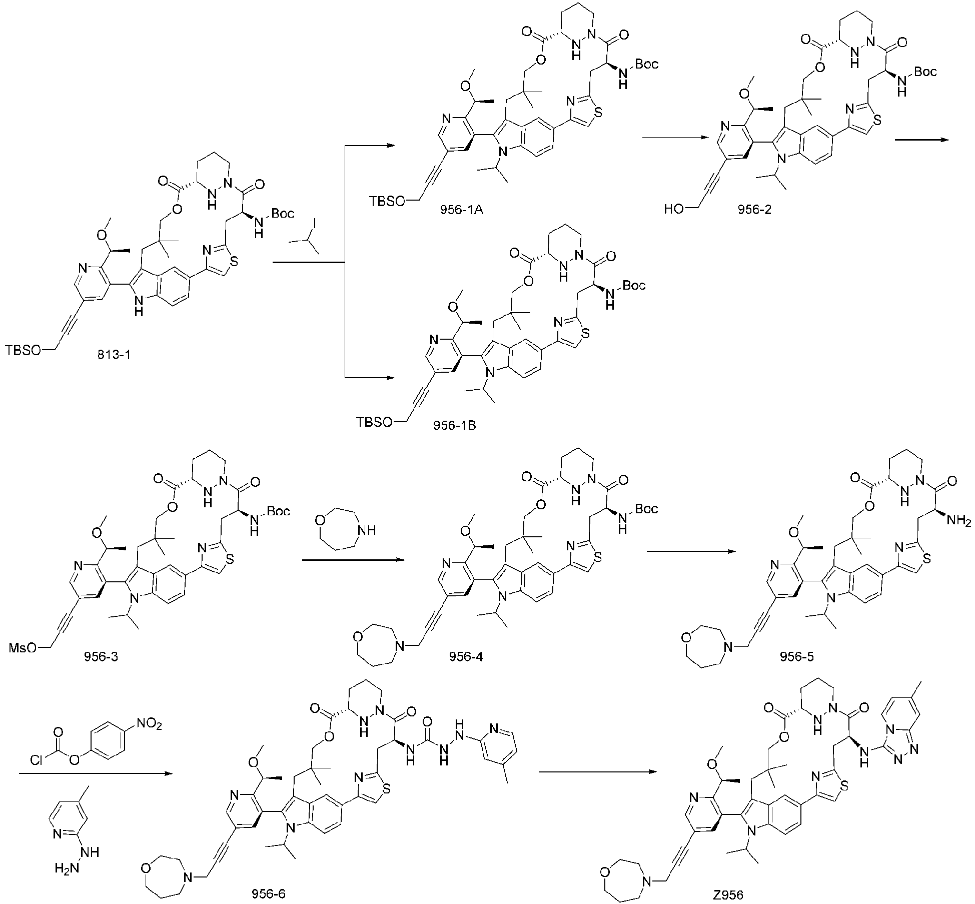

Step 1: Compound 813-1 (1000 mg, 1.148 mmol) was dissolved in N,N-diisopropylethylenediamine (10 mL), and cesium carbonate (3731.00 mg, 11.480 mmol) and 2-iodopropane (1.148 mL, 11.479 mmol) were added. The reaction system was heated to 50 °C and stirred for 48 h under a nitrogen atmosphere. The mixture was cooled to room temperature, extracted with ethyl acetate (30 mL × 3), washed sequentially with water (50 mL) and saturated brine (50 mL), dried over anhydrous sodium sulfate, filtered, and concentrated, and the residue was purified by silica gel column chromatography (ethyl acetate/dichloromethane = 2/3) to give 2 isomeric compounds. The structure of one of the isomeric compounds was arbitrarily designated as compound 956-1A (262 mg, yield: 25%, retention time: 3.554 min). ES-API [M+H]⁺ = 913.4. The structure of the other isomeric compound was arbitrarily designated as compound 956-1B (510 mg, yield: 48.6%, retention time: 3.342 min). ES-API [M+H]⁺ = 913.4.

Step 2: Compound 956-1A (262 mg, 0.287 mmol) was dissolved in tetrabutylammonium fluoride (5 mL, a 1 M solution in tetrahydrofuran), and the mixture was stirred at room temperature for 1 h. The mixture was diluted with water (20 mL), extracted with ethyl acetate (30 mL × 3), washed sequentially with water (50 mL) and saturated brine (50 mL), dried over anhydrous sodium sulfate, filtered, and concentrated to give compound 956-2 (206 mg, 0.258 mmol, yield: 90%). ES-API [M+H]⁺ = 799.3.

Step 3: N,N-Diisopropylethylamine (166.4 mg, 1.29 mmol) and methanesulfonic anhydride (67.3 mg, 0.387 mmol) were added to a solution of compound 956-2 (206 mg, 0.258 mmol) in dichloromethane (15 mL). The mixture was stirred at 25 °C for 1 h. When the reaction was completed as detected by LCMS, the reaction mixture was diluted with dichloromethane (20 mL) and then washed with saturated brine (15 mL × 3). The organic phase was dried over anhydrous sodium sulfate, filtered, and concentrated, and the residue was purified by silica gel column chromatography (ethyl acetate/dichloromethane = 1/1) to give compound 956-3 (181 mg, 0.206 mmol, yield: 85%). ES-API: [M+H]⁺ = 877.3.

Step 4: 1,4-Oxazepane (62.4 mg, 0.618 mmol) was added to a solution of compound 956-3 (181 mg, 0.206 mmol) in acetonitrile (15 mL). The mixture was stirred at 25 °C for 18 h. When the reaction was completed as detected by LCMS, the mixture was concentrated, and the residue was purified by silica gel column chromatography (methanol/dichloromethane = 1/20) to give compound 956-4 (163 mg, 0.185 mmol, yield: 90%). ES-API: [M+H]⁺ = 882.3.

Step 5: Compound 956-4 (163 mg, 0.185 mmol) was dissolved in methanol (1 mL), and hydrochloric acid (5 mL, a 4 M solution in dioxane) was slowly added under an ice-water bath. The mixture was reacted for 1 h and concentrated, and then dichloromethane (20 mL) was added for dilution. The resulting mixture was washed sequentially with a saturated aqueous sodium bicarbonate solution (10 mL) and saturated brine (10 mL), dried over anhydrous sodium sulfate, filtered, and concentrated to give crude compound 956-5 (116 mg, 0.148 mmol, yield: 80%), which was directly used in the next step. ES-API: [M+H]⁺ = 782.2.

Step 6: N,N-Diisopropylethylamine (95.5 mg, 0.74 mmol) and p-nitrophenyl chloroformate (44.7 mg, 0.222 mmol) were sequentially added to a solution of compound 956-5 (116 mg, 0.148 mmol) in dichloromethane (5 mL) under an ice-water bath. After 10 min of reaction, 2-hydrazineyl-4-methylpyridine (55 mg, 0.444 mmol) was immediately added in one portion, and the mixture was reacted for another 0.5 h under an ice-water bath. When the reaction was completed as detected by LCMS, the reaction mixture was concentrated, and the crude product was purified by automated flash chromatography on silica gel (methanol/dichloromethane = 0-6%) to give compound 956-6 (96 mg, 0.104 mmol, yield: 70%). ES-API: [M+H]⁺ = 931.4.

Step 7: Pyridine (123 mg, 1.56 mmol) and phosphorus oxychloride (80 mg, 0.520 mmol) were sequentially added to a solution of compound 956-6 (96 mg, 0.104 mmol) in 1,2-dichloroethane (10 mL). The mixture was reacted at 75 °C for 20 min under a nitrogen atmosphere. The reaction mixture was concentrated, and the residue was purified by a silica gel column to give a crude product, which was purified by prep-HPLC (ammonium bicarbonate method) to give (6³S,4S,Z)-(Rₐ)-1²-(5-(3-(1,4-oxazepan-4-yl)prop-1-yn-1-yl)-2-((S)-1-methoxyethyl)pyridin-3-yl)-1¹-isopropyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z956, 60 mg, yield: 63%), ES-API: [M+H]⁺ = 913.3. ¹H NMR (400 MHz, DMSO-d6) δ 8.81 (d, J = 2.1 Hz, 1H), 8.51 (d, J = 1.6 Hz, 1H), 8.25 (d, J = 7.2 Hz, 1H), 7.76 (d, J = 2.0 Hz, 2H), 7.74 - 7.64 (m, 2H), 7.22 (q, J = 1.4 Hz, 1H), 7.15 (d, J = 10.6 Hz, 1H), 6.70 (dd, J = 7.2, 1.5 Hz, 1H), 5.59 (t, J = 9.8 Hz, 1H), 5.23 (d, J = 12.1 Hz, 1H), 4.52 (q, J = 6.0 Hz, 1H), 4.30-4.18 (m, 3H), 3.74 - 3.57 (m, 8H), 3.48 (d, J = 14.6 Hz, 1H), 3.26 (d, J = 9.3 Hz, 1H), 3.23 (s, 3H), 2.85-2.75 (m, 5H), 2.40 - 2.24 (m, 4H), 2.12-2.05 (m, 1H), 1.90-1.78 (m, 4H), 1.75 (d, J = 6.9 Hz, 3H), 1.60 - 1.52 (m, 1H), 1.40 (d, J = 6.1 Hz, 3H), 1.25 (q, J = 6.6 Hz, 1H), 1.19 (d, J = 6.9 Hz, 3H), 0.88 (s, 3H), 0.47 (s, 3H).

### Example 130. Synthesis of Compounds Z925 and Z925-1

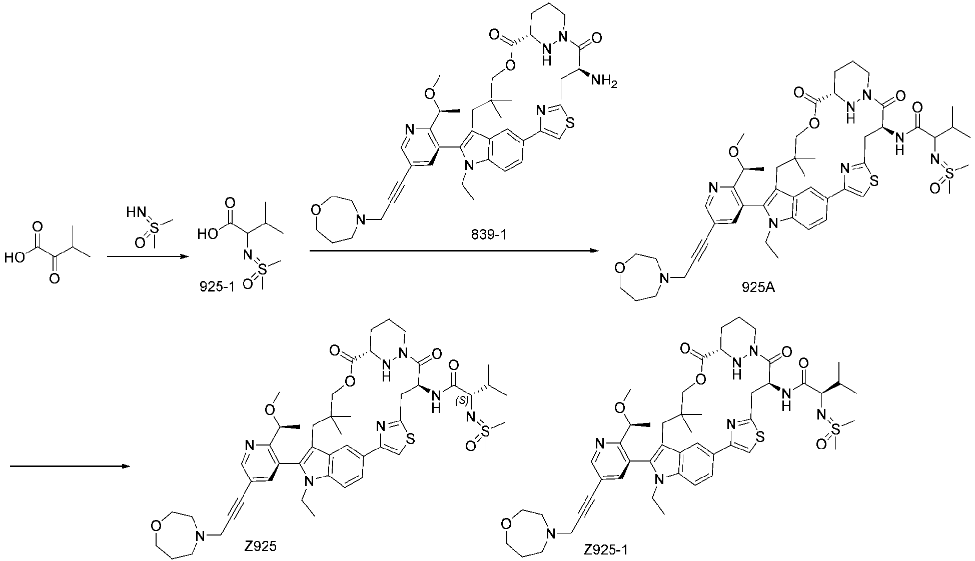

Step 1: Sodium cyanoborohydride (566.41 mg, 8.991 mmol) was added to a solution of 3-methyl-2-oxobutanoic acid (348 mg, 2.997 mmol) and dimethylsulfoximine (307.04 mg, 3.297 mmol) in dichloromethane (10 mL). The mixture was stirred at 25 °C for 3 h. The mixture was then concentrated, and ethyl acetate (10 mL × 3) was added for extraction. The organic phase was washed sequentially with water (10 mL) and saturated brine (10 mL), dried over anhydrous sodium sulfate, filtered, and concentrated. The residue was purified by silica gel column chromatography (methanol/dichloromethane = 1/9) to give compound 925-1 (173.5 mg, 0.899 mmol, yield: 30%). ES-API [M+H⁺] = 194.1.

Step 2: Compound 925-1 (58 mg, 0.3 mmol), N,N-diisopropylethylamine (103 mg, 0.8 mmol), and O-(7-azabenzotriazol-1-yl)-N,N,N',N'-tetramethyluronium hexafluorophosphate (152 mg, 0.4 mmol) were sequentially added to a solution of compound 839-1 (150 mg, 0.2 mmol) in dichloromethane (5 mL) under an ice-water bath. The reaction mixture was warmed to room temperature and stirred for 2 h. When the reaction was completed as detected by LCMS, the reaction mixture was diluted with dichloromethane (20 mL) and then washed with water (10 mL) and saturated brine (10 mL × 3). The organic phase was dried over anhydrous sodium sulfate, filtered, and concentrated to dryness to give crude compound 925A. The crude product was purified by prep-HPLC (ammonium bicarbonate method) to give 2 isomeric compounds. The structure of one of the isomeric compounds was arbitrarily designated as (2S)-N-((6³S,4S,Z)-(Rₐ)-1²-(5-(3-(1,4-diazepan-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-4-yl)-2-((dimethyl(oxo)-1⁶-sulfaneylidene)amino)-3-methylbutanamide (Z925, 17 mg, yield: 9%, retention time: 6.110 min). ¹H NMR (400 MHz, DMSO-d6) δ 8.81 (s, 1H), 8.49 (d, J = 1.5 Hz, 1H), 7.90 (d, J = 9.6 Hz, 1H), 7.85 (s, 1H), 7.81 (s, 1H), 7.76 (d, J = 8.5 Hz, 1H), 7.58 (d, J = 8.7 Hz, 1H), 5.58 (t, J = 9.2 Hz, 1H), 5.13 (d, J = 12.2 Hz, 1H), 4.38 - 4.19 (m, 4H), 4.12 - 4.05 (m, 1H), 3.73 - 3.62 (m, 6H), 3.56 (d, J = 4.0 Hz, 2H), 3.47-3.41 (m, 1H), 3.29 (s, 2H), 3.26 (s, 3H), 3.16 (s, 3H), 3.14 (s, 3H), 2.97 (d, J = 14.4 Hz, 1H), 2.88 - 2.65 (m, 5H), 2.34 (d, J = 13.4 Hz, 1H), 2.12 - 2.01 (m, 2H), 1.83 (d, J = 21.8 Hz, 4H), 1.53 (s, 1H), 1.36 (d, J = 6.0 Hz, 3H), 0.93 (s, 3H), 0.88 (dt, J = 7.1, 3.7 Hz, 6H), 0.75 (d, J = 6.7 Hz, 3H), 0.34 (s, 3H).

The structure of the other isomeric compound was arbitrarily designated as (2R)-N-((6³S,4S,Z)-(Rₐ)-1²-(5-(3-(1,4-diazepan-1-yl)prop-1-yn-1-yl)-2-((S)-1-methoxyethyl)pyridin-3-yl)-1¹-ethyl-10,10-dimethyl-5,7-dioxo-6¹,6^{2,}6³,6^{4,}6⁵,6⁶-hexahydro-1¹H-8-oxa-2(4,2)-thiazola-1(5,3)-indola-6(1,3)-pyridazinacycloundecaphane-4-yl)-2-((dimethyl(oxo)-1⁶-sulfaneylidene)amino)-3-methylbutanamide (Z925-1, 15 mg, yield: 8%, retention time: 6.322 min), ES-API: [M+H]⁺ = 943.4. ¹H NMR (400 MHz, DMSO-d6) δ 8.80 (d, J = 2.1 Hz, 1H), 8.49 (d, J = 1.6 Hz, 1H), 7.93 (d, J = 9.8 Hz, 1H), 7.87 - 7.82 (m, 2H), 7.76 (dd, J = 8.7, 1.7 Hz, 1H), 7.59 (d, J = 8.7 Hz, 1H), 5.57 - 5.50 (m, 1H), 5.14 (d, J = 12.1 Hz, 1H), 4.38 - 4.19 (m, 4H), 4.15 - 4.04 (m, 1H), 3.71 - 3.66 (m, 4H), 3.65 - 3.62 (m, 2H), 3.62 - 3.52 (m, 2H), 3.46 (d, J = 4.3 Hz, 1H), 3.39 - 3.33 (m, 2H), 3.26 (s, 3H), 3.16 (s, 3H), 3.06 (s, 3H), 2.98 (d, J = 14.2 Hz, 1H), 2.76 - 2.65 (m, 5H), 2.37 (d, J = 14.4 Hz, 1H), 2.12 - 2.05 (m, 2H), 1.88- 1.77 (m, 4H), 1.63 - 1.52 (m, 1H), 1.35 (d, J = 6.0 Hz, 3H), 0.96 - 0.90 (m, 9H), 0.87 (t, J = 7.1 Hz, 3H), 0.34 (s, 3H).

### Example 131. Synthesis of Compound Z958

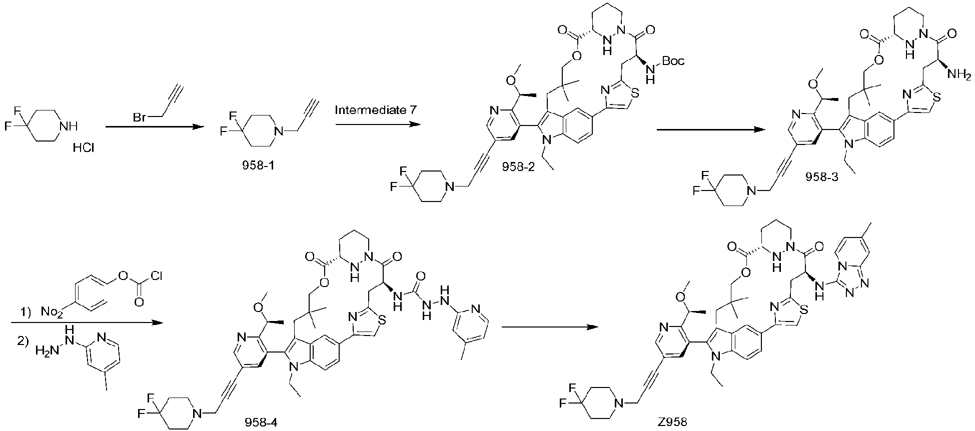

Step 1: Triethylamine (6.58 g, 47.6 mmol) was added to a solution of 4,4-dimethylpiperidine hydrochloride (3 g, 19.04 mmol) in acetonitrile (10 mL), and 3-bromopropyne (2.72 g, 22.84 mmol) was slowly added under an ice bath. The mixture was stirred under the ice bath for 2 h. After the reaction was completed, the reaction mixture was filtered and concentrated, and the residue was purified by a flash silica gel column (0-40% petroleum ether/ethyl acetate) to give compound 958-1 (2 g, yield: 66%). ES-API [M+H]⁺ = 160.1.

Step 2: Compound intermediate 7 (240 mg, 0.273 mmol), compound 958-1 (130.4 mg, 0.82 mmol), bis(triphenylphosphine)palladium(II) dichloride (57.49 mg, 0.082 mmol), copper(I) iodide (31.20 mg, 0.164 mmol), N,N-diisopropylethylenediamine (0.190 mL, 1.092 mmol), and lithium chloride (46.30 mg, 1.092 mmol) were mixed in N,N-dimethylformamide (10 mL) under a nitrogen atmosphere, and the mixture was stirred at 80 °C for 5 h. After the reaction was completed, the mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 958-2 (180 mg, yield: 75%). ES-API [M+H]⁺ = 888.4.

Step 3: Trifluoroacetic acid (3 mL) was added to a solution of compound 958-2 (160 mg, 0.07 mmol) in dichloromethane (5 mL), and the mixture was stirred at room temperature for 2 h. After the reaction was completed, the mixture was concentrated, and the residue was basified with a saturated sodium bicarbonate solution to pH 8, extracted with ethyl acetate (20 mL × 3), and concentrated to give compound 958-3 (100 mg, yield: 70.44%). ES-API [M+H]⁺ = 788.4.

Step 4: N,N-Diisopropylethylenediamine (123 mg, 0.95 mmol) was added to a solution of compound 958-3 (150 mg, 0.19 mmol) in dichloromethane (10 mL), and 4-nitrophenyl chloroformate (57.5 mg, 0.57 mmol) was slowly added under an ice bath. The mixture was stirred under the ice bath for 10 min. After the reaction was completed as monitored by LCMS, 2-hydrazineyl-4-methylpyridine (70 mg, 0.29 mmol) was rapidly added, and the mixture was stirred under the ice bath for 10 min. The reaction mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-60% petroleum ether/ethyl acetate) to give compound 958-4 (133 mg, yield: 75%). ES-API [M+H]⁺ = 937.4.

Step 5: Pyridine (328.77 mg, 4.162 mmol) and phosphorus oxychloride (216.41 mg, 1.387 mmol) were added to a solution of compound 958-4 (130 mg, 0.14 mmol) in dichloroethane (10 mL), and the mixture was stirred at 50 °C for 10 min. After the reaction was completed as detected by LCMS, the reaction mixture was poured into ethyl acetate (30 mL) and washed sequentially with water (15 mL) and saturated brine (15 mL). The organic phase was dried over anhydrous sodium sulfate, filtered, and concentrated, and the residue was purified by a flash silica gel column (0-80% petroleum ether/ethyl acetate) to give (6³S,4S,Z)-(Rₐ)-1²-(5-(3-(4,4-difluoropiperidin-1-yl)prop-1-yn-1-yl)-2-((S)-1-methoxyethyl)pyridin-3-yl)-1¹-ethyl-10,10-dimethyl-4-((7-methyl-[1,2,4]triazolo[4,3-alpyridin-3-yl)amino)-6¹,6 ²,6³,6⁴,6⁵,6⁶-hexahydro-1¹H-8-oxa-2(4,2)-thiazola-1(5,3)-indola-6(1,3)-pyridazinacycloundecaphane-5,7-dione (Z958, 40 mg, yield: 31%). ES-API [M+H]⁺ = 919.4.

### Example 132. Synthesis of Compounds Z932, Z932-1, and Z932-2

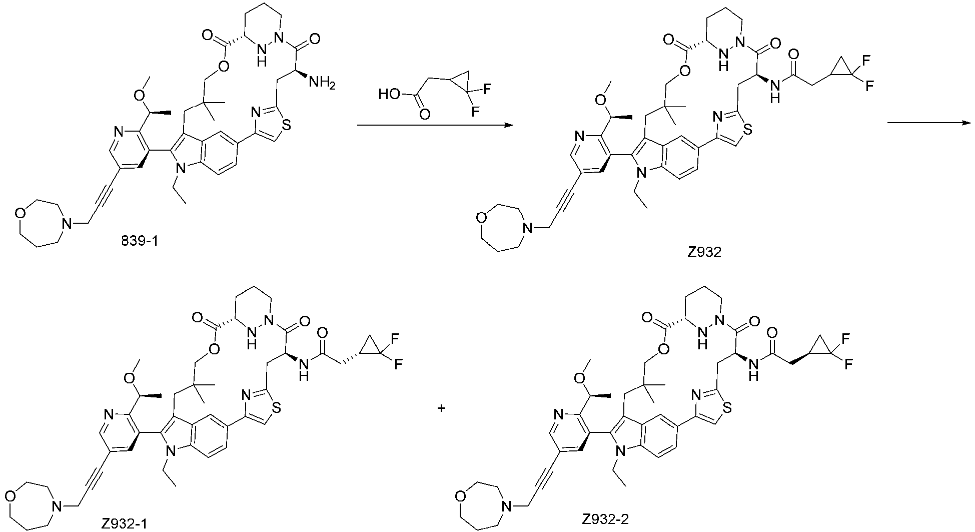

Step 1: N,N-Diisopropylethylamine (40 mg, 0.31 mmol) and O-(7-azabenzotriazol-1-yl)-N,N,N',N'-tetramethyluronium hexafluorophosphate (59 mg, 0.16 mmol) were sequentially added to a solution of 2-(2,2-difluorocyclopropyl)acetic acid (17 mg, 0.12 mmol) and compound 839-1 (80 mg, 0.10 mmol) in dichloromethane (2 mL). The mixture was stirred at room temperature for 1 h. After the reaction was completed, the mixture was washed with water (2 mL) and concentrated, and the residue was purified by prep-HPLC (ammonium bicarbonate method) to give N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1'*H-*8-oxa-2(4,2)-thiazola-1(5,3)-indola-6(1,3)-pyridazinacycloundecaphane-4-yl)-2-(2,2-difluorocyclopropyl)acetamide (Z932, 57.77 mg, yield: 62.6%, purity: 98%). ES-API: [M+H]⁺ = 886.5.

Step 2: Compound Z932 (56 mg) was subjected to chiral resolution (column type: Chiralpak ID: 10 µm, 20 × 250 mm, mobile phase: acetonitrile: isopropanol: diethylamine = 90:10:2, flow rate: 15 mL/min, column temperature: room temperature) to give 2 isomeric compounds. The structure of one of the isomeric compounds was arbitrarily designated as N-((6³S,4S,Z)-(Rₐ)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro- 1*¹H*-8-oxa-2(4,2)-thiazola-1(5,3)-indola-6(1,3)-pyridazinacycloundecaphane-4-yl)-2-((R)-2,2-difluorocyclopropyl)acetamide (Z932-1, 17.1 mg, yield: 30.5%, retention time: 9.02 min, purity: 100%, de value: 100%). ES-API: [M+H]⁺ = 886.5.

The structure of the other isomeric compound was arbitrarily designated as N-((6³S,4S,Z)-(R ₐ)-1²-(5-(3-(1,4-oxazepan-4-yl)prop-1-yn-1-yl)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹*H-*8-oxa-2(4,2)-thiazola-1(5,3)-indola-6(1,3)-pyridazinacycloundecaphane-4-yl)-2-((S)-2,2-difluorocyclopropyl)acetamide (Z932-2, 17.7 mg, yield: 31.6%, retention time: 10.2 min, purity: 100%, de value: 100%). ES-API: [M+H]⁺ = 886.5.

### Example 133. Synthesis of Compound Z957

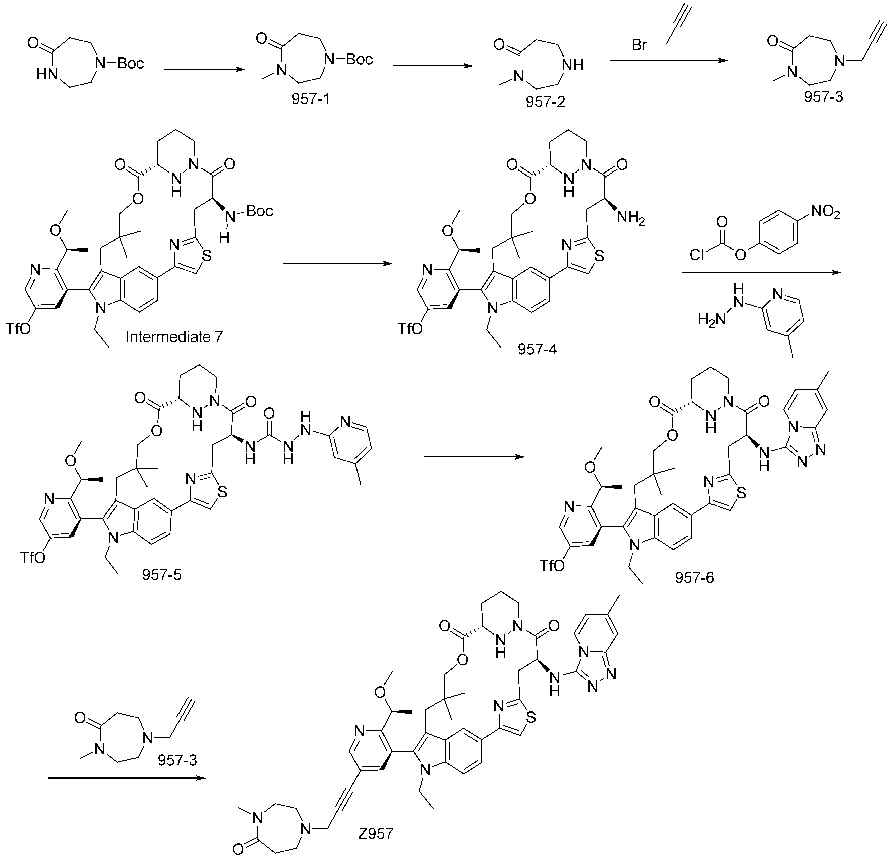

Step 1: tert-Butyl 5-oxo-1,4-diazepane-1-carboxylate (1 g, 4.667 mmol) was dissolved in N,N-dimethylformamide (15 mL) under an ice-water bath, and sodium hydride (0.37 g, 9.334 mmol) was added. The mixture was reacted under the ice-water bath for 0.5 h under a nitrogen atmosphere, and iodomethane (1.506 mL, 23.336 mmol) was added. The mixture was reacted at room temperature for 1 h. After the reaction was completed, an aqueous ammonium chloride solution (5 mL) was added, and the mixture was extracted with ethyl acetate (30 mL × 3). The organic phases were combined, washed with saturated brine (30 mL × 1), dried over anhydrous sodium sulfate, filtered, and concentrated, and the residue was purified by silica gel column chromatography (petroleum ether/ethyl acetate = 1/1) to give compound 957-1 (500 mg, 2.190 mmol, yield: 46.93%). ES-API: [M+H-56]⁺ = 173.1.

Step 2: Hydrochloric acid (10 mL, a 4 M solution in dioxane) was slowly added to a solution of compound 957-1 (500 mg, 2.190 mmol) in methanol (2 mL), and the mixture was reacted for 1 h and concentrated to give crude compound 957-2 (280 mg, 2.184 mmol, yield: 99.74%), which was directly used in the next step. ES-API: [M+H]⁺ = 129.1.

Step 3: Potassium carbonate (905.66 mg, 6.553 mmol) and 3-bromoprop-1-yne (259.86 mg, 2.184 mmol) were added to a solution of compound 957-2 (280 mg, 2.184 mmol) in dichloroethane (10 mL), and the reaction mixture was stirred at room temperature for 18 h. The reaction mixture was then diluted with dichloromethane (30 mL) and water (30 mL). The organic layer was separated, washed with a saturated sodium chloride solution (100 mL × 1), dried over anhydrous sodium sulfate, filtered, and concentrated. The residue was purified by silica gel column chromatography (petroleum ether/tetrahydrofuran = 10/90) to give compound 957-3 (150 mg, 0.902 mmol, yield: 41.31%). ES-API: [M+H-56]⁺ = 167.1.

Step 4: Intermediate 7 (600 mg, 0.683 mmol) was dissolved in methanol (2 mL) under an ice-water bath, and hydrochloric acid (5 mL, a 4 M solution in dioxane) was slowly added. The mixture was reacted for 1 h and concentrated, and then dichloromethane (20 mL) was added for dilution. The resulting mixture was washed sequentially with a saturated aqueous sodium bicarbonate solution (10 mL) and saturated brine (10 mL), dried over anhydrous sodium sulfate, filtered, and concentrated to give crude compound 957-4 (530 mg, 0.680 mmol, yield: 99%), which was directly used in the next step. ES-API: [M+H]⁺ = 779.2.

Step 5: N,N-Diisopropylethylamine (438.91 mg, 3.402 mmol) and p-nitrophenyl chloroformate (205.74 mg, 1.021 mmol) were sequentially added to a solution of compound 957-4 (530 mg, 0.680 mmol) in dichloromethane (5 mL) under an ice-water bath. The mixture was reacted for 5 min, and then 2-hydrazineyl-4-methylpyridine (251.42 mg, 2.041 mmol) was immediately added. The resulting mixture was reacted for another 0.5 h. When the reaction was completed as detected by LCMS, the mixture was concentrated, and the crude product was purified by automated flash chromatography on silica gel (methanol/dichloromethane = 0-6%) to give compound 957-5 (610 mg, 0.657 mmol, yield: 96.60%). ES-API: [M+H]⁺ = 928.3.

Step 6: Pyridine (766.15 mg, 9.698 mmol) and phosphorus oxychloride (504.30 mg, 3.233 mmol) were sequentially added to a solution of compound 957-5 (600 mg, 0.647 mmol) in 1,2-dichloroethane (15 mL). The mixture was reacted at 75 °C for 0.5 h under a nitrogen atmosphere and concentrated, and the residue was purified by silica gel column chromatography (methanol/dichloromethane = 1/20) to give compound 957-6 (290 mg, 0.319 mmol, yield: 49.29%). ES-API: [M+H]⁺ = 910.3. Step 7: Compound 957-6 (50 mg, 0.055 mmol) and compound 957-3 (27.40 mg, 0.165 mmol) were dissolved in N,N-dimethylformamide (3 mL), and bis(triphenylphosphine)palladium(II) dichloride (11.57 mg, 0.016 mmol), copper(I) iodide (6.28 mg, 0.033 mmol), N,N-diisopropylethylenediamine (0.038 mL, 0.220 mmol), and lithium chloride (11.65 mg, 0.275 mmol) were sequentially added. The mixture was heated to 80 °C and reacted for 3 h under a nitrogen atmosphere, then diluted with ethyl acetate (20 mL), washed sequentially with water (20 mL) and saturated brine (20 mL), dried over anhydrous sodium sulfate, filtered, and concentrated. The residue was purified by prep-HPLC (ammonium bicarbonate method) to give (6³S,4S,7)-1²-(5-(3-(4-methyl-1,4-diazepan-5-one-1-yl)prop- 1-yn-1-yl)-(Rₐ)-2-((S)-1-methoxyethyl)pyridin-3-yl)-1'-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z957, 8.5 mg, yield: 16.70%). ES-API: [M+H]⁺ = 926.4. ¹H NMR (400 MHz, DMSO-d6) δ 8.81 (d, J = 2.1 Hz, 1H), 8.53 (d, J = 1.5 Hz, 1H), 8.33 (s, 1H), 7.86 (d, J = 2.1 Hz, 1H), 7.79 (s, 1H), 7.78 - 7.72 (m, 1H), 7.57 (d, J = 8.7 Hz, 1H), 7.26 (s, 2H), 6.75 (d, J = 7.0 Hz, 1H), 5.54 (s, 1H), 5.21 (d, J = 12.0 Hz, 1H), 4.36 - 4.22 (m, 4H), 4.12 - 4.03 (m, 1H), 3.70 (s, 2H), 3.59 (q, J = 11.0 Hz, 2H), 3.52 - 3.44 (m, 3H), 3.29 - 3.27 (m, 1H), 3.25 (s, 3H), 2.98 (d, J = 14.2 Hz, 1H), 2.85 (s, 4H), 2.76 - 2.65 (m, 4H), 2.61 - 2.54 (m, 2H), 2.39 (d, J = 15.0 Hz, 1H), 2.34 - 2.28 (m, 3H), 2.12 (d, J = 12.4 Hz, 1H), 1.83 (s, 2H), 1.63 - 1.52 (m, 1H), 1.35 (d, J = 6.0 Hz, 3H), 0.94 (s, 3H), 0.87 (t, J = 7.1 Hz, 3H), 0.35 (s, 3H).

### Example 134. Synthesis of Compound Z960

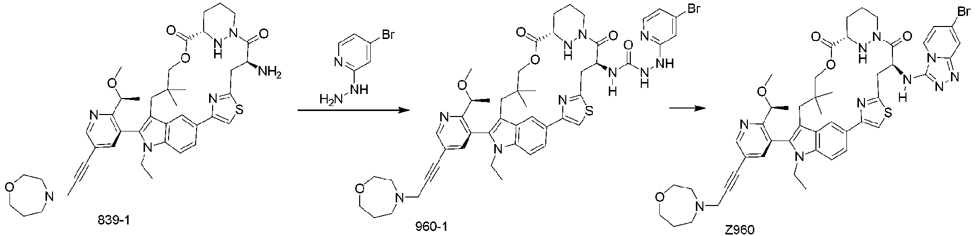

Step 1: Compound 839-1 (170 mg, 0.221 mmol) and N,N-diisopropylethylamine (143.05 mg, 1.107 mmol) were dissolved in dichloromethane (10 mL). Nitrophenyl chloroformate (89.23 mg, 0.443 mmol) was added at 0 °C. The reaction system was stirred under an ice bath for 0.5 h, and then N,N-diisopropylethylamine (83.02 mg, 0.642 mmol) and 4-bromo-2-hydrazineylpyridine (161.04 mg, 0.856 mmol) were added. The reaction system was stirred at 0 °C for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-7%) to give compound 960-1 (150 mg, 0.153 mmol, yield: 71.34%) as a light yellow solid. ES-API: [M+H]⁺ = 981.2.

Step 2: Compound 960-1 (150 mg, 0.153 mmol) was dissolved in 1,2-dichloroethane (5 mL) and pyridine (0.184 mL, 2.291 mmol). Phosphorus oxychloride (0.043 mL, 0.458 mmol) was added to the solution described above. After the addition was completed, the reaction system was stirred at 45 °C for 5 min. The reaction mixture was cooled to room temperature and concentrated. The crude product was purified by a flash silica gel column (methanol/dichloromethane: 0-9%) to give the desired product (6³S,4S,Z)-(*Rₐ*)-1²-(5-(3-(1,4-oxazepan-4-yl)prop-1-yn-1-yl)-2-((S)-1-methoxyethyl)pyridin-3-yl)-4-((7-bromo-[1,2,4]triazolo[4,3-a]pyridin-3-yl)amino)-1¹-ethyl-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z960, 25 mg, 0.026 mmol, yield: 16.98%) as a light yellow solid. ES-API: [M+H]⁺ = 963.3, 965.3.

### Example 135. Synthesis of Compound Z959

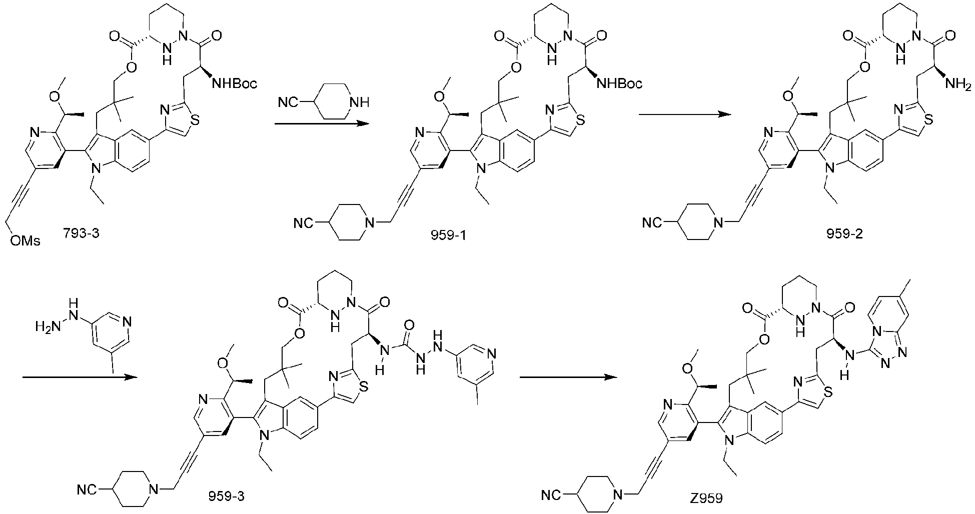

Step 1: Compound 793-3 (0.3 g, 0.348 mmol) and anhydrous N,N'-diisopropylethylamine (0.22 g, 1.738 mmol) were dissolved in anhydrous dichloromethane (10 mL), and 4-cyanopiperidine (0.15 g, 1.390 mmol) was added. After the addition was completed, the reaction system was stirred at room temperature for 4 h. After the reaction was completed, water (10 mL) was added to the solution described above, and the mixture was extracted with dichloromethane (10 mL × 2). The organic phase was washed with saturated brine (10 mL), dried over anhydrous sodium sulfate, filtered, and concentrated to give a crude product. The crude product was purified by a flash silica gel column (methanol/dichloromethane: 0%-3%) to give compound 959-1 (250 mg, 0.285 mmol, yield: 82.00%) as a white solid, ES-API: [M+H]⁺ = 877.3.

Step 2: Compound 959-1 (0.2 g, 0.228 mmol) was dissolved in methanol (0.5 mL), and a 4 M solution of hydrochloric acid in dioxane (2 mL) was added under an ice-water bath. The reaction system was stirred at room temperature for 2 h under a nitrogen atmosphere. After the reaction was completed, the mixture was concentrated under reduced pressure, and water (5 mL) was added. The pH was adjusted to 8 with a saturated sodium bicarbonate solution, and the mixture was extracted with ethyl acetate (10 mL × 2). The organic phase was washed with saturated brine (10 mL), dried over anhydrous sodium sulfate, filtered, and concentrated to give compound 959-2 (170 mg, 0.219 mmol, yield: 95.95%) as a white solid, ES-API: [M+H]⁺ = 777.3.

Step 3: Compound 959-2 (150 mg, 0.193 mmol) and N,N-diisopropylethylamine (75 mg, 0.580 mmol) were dissolved in dichloromethane (8 mL), and nitrophenyl chloroformate (100 mg, 0.49 mmol) was added at 0 °C. The reaction system was stirred for 1 h under an ice bath, and then 3-hydrazineyl-5-methylpyridine (60 mg, 0.298 mmol) was added. The reaction system was stirred at room temperature for 3 h. Ethyl acetate (40 mL) was added to the reaction mixture, and the mixture was washed sequentially with water (20 mL) and saturated brine (20 mL). The organic phase was dried over anhydrous sodium sulfate and filtered, and the filtrate was concentrated under reduced pressure. The crude product was purified by a flash silica gel column (7 M ammonia-methanol solution/dichloromethane: 0-5%) to give compound 959-3 (110 mg, 0.121 mmol, yield: 64.49%) as a yellow solid, ES-API: [M+H]⁺ = 926.3.

Step 4: Compound 959-3 (80 mg, 0.085 mmol) and pyridine (0.157 mL, 1.944 mmol) were dissolved in dichloromethane (5 mL) and phosphorus oxychloride (216.41 mg, 1.387 mmol). The reaction system was stirred at 45 °C for 5 min. The reaction mixture was cooled to room temperature and concentrated. The resulting crude product was purified by a flash silica gel column (7 M ammonia-methanol solution/dichloromethane: 0-5%) to give the desired product 1-(3-((*Rₐ*)-5-((6³S,4S,Z)-1¹-ethyl-10,10-dimethyl-4-(7-ethyl-[1,2,4]triazolo[4,3-a]pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-((S)-1-methoxyethyl)pyridin-3-yl)prop-2-yn-1-yl)piperidine-4-carbonitrile (Z959, 30 mg, 0.033 mmol, yield: 50.99%) as a yellow solid. ES-API: [M+H]⁺ = 908.3.

### Example 136. Synthesis of Compounds Z950 and Z950-1

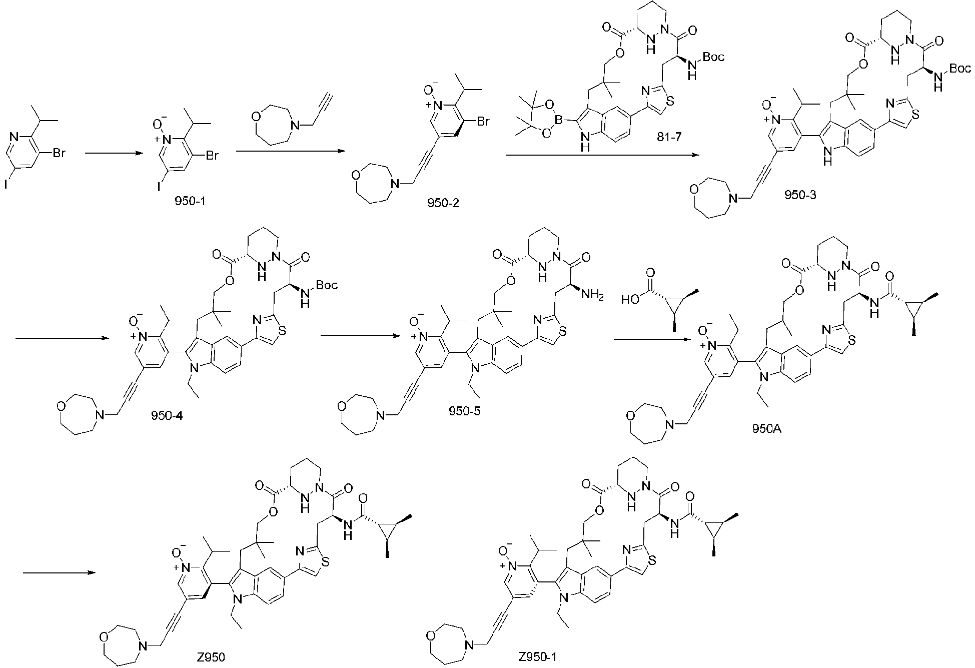

Step 1: m-Chloroperoxybenzoic acid (356.21 mg, 1.755 mmol) was added to a solution of 3-bromo-5-iodo-2-(isopropyl)pyridine (300 mg, 0.877 mmol) in dichloromethane (10 mL) under an ice-water bath. The reaction system was stirred at room temperature for 18 h under a nitrogen atmosphere. The reaction mixture was diluted with dichloromethane (50 mL), washed sequentially with a saturated aqueous sodium thiosulfate solution (20 mL), saturated sodium bicarbonate (30 mL × 2) and brine (30 mL), dried over anhydrous sodium sulfate, filtered, and concentrated in vacuum. The residue was purified by silica gel column chromatography (tetrahydrofuran/petroleum ether = 1/50) to give compound 950-1 (31.40 mg, 0.088 mmol, yield: 10%) as a light yellow oil. ES-API: [M+H-56]⁺ = 341.9, 343.9.

Step 2: Bis(triphenylphosphine)palladium(II) dichloride (6.44 mg, 0.009 mmol) and copper(I) iodide (5.25 mg, 0.028 mmol) were sequentially added to a solution of compound 950-1 (31.40 mg, 0.088 mmol), 4-(prop-2-ynyl)-1,4-oxazepane (14.06 mg, 0.101 mmol), and N,N-diisopropylethylenediamine (47.47 mg, 0.367 mmol) in acetonitrile (5 mL). The reaction system was reacted with stirring at room temperature for 2 h. The reaction mixture was diluted with ethyl acetate (30 mL) and water (30 mL). The organic layer was separated, washed with a saturated sodium chloride solution (100 mL × 1), dried over anhydrous sodium sulfate, filtered, and concentrated. The residue was purified by column chromatography (tetrahydrofuran/petroleum ether = 1/1) to give compound 950-2 (30 mg, 0.086 mmol, yield: 90%). ES-API: [M+H-56]⁺ = 353.1, 355.1.

Step 3: Bis(triphenylphosphine)palladium(II) dichloride (9.36 mg, 0.0144 mmol) and potassium carbonate (59.77 mg, 0.432 mmol) were added to a solution of compound 81-7 (60 mg, 0.086 mmol) and compound 950-2 (30 mg, 0.086 mmol) in water (1 mL) and tetrahydrofuran (4 mL). The mixture was degassed with nitrogen and stirred at 70 °C for 2 h. The mixture was cooled to room temperature, poured into water (30 mL), and extracted with ethyl acetate (30 mL × 3). The ethyl acetate layers were combined, washed with a saturated sodium chloride solution (30 mL × 1), dried over anhydrous sodium sulfate, filtered, and concentrated. The residue was purified by silica gel column chromatography (methanol/dichloromethane = 1/30) to give compound 950-3 (60 mg, 0.071 mmol, yield: 84%), ES-API: [M+H]⁺ = 840.3.

Step 4: Cesium carbonate (118.62 mg, 0.364 mmol) and iodoethane (0.058 mL, 0.728 mmol) were sequentially added to a solution of compound 950-3 (60 mg, 0.073 mmol) in N,N-dimethylformamide (3 mL). The reaction mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated, and the residue was purified by silica gel column chromatography (methanol/dichloromethane = 1/30) to give compound 950-4 (50 mg, 0.058 mmol, yield: 80.64%), ES-API: [M+H]⁺ = 868.4.

Step 5: Hydrochloric acid (3 mL, a 4 M solution in dioxane) was added to a solution of compound 950-4 (50 mg, 0.058 mmol) in methanol (1 mL), and the mixture was reacted at room temperature for 1 h. The reaction mixture was then concentrated to give crude compound 950-5 (hydrochloride, 50 mg), which was directly used in the next step. ES-API: [M+H]⁺ = 768.4.

Step 6: (1r,2R,3S)-2,3-Dimethylcyclopropanecarboxylic acid (7.31 mg, 0.064 mmol), N,N-diisopropylethylamine (24.82 mg, 0.192 mmol), and O-(7-azabenzotriazol-1-yl)-N,N,N',N'-tetramethyluronium hexafluorophosphate (48.67 mg, 0.128 mmol) were sequentially added to a solution of compound 950-5 (50 mg) in dichloromethane (5 mL) under an ice-water bath. The reaction mixture was warmed to room temperature and stirred for 1 h. When the reaction was completed as detected by LCMS, the reaction mixture was diluted with dichloromethane (20 mL) and then washed with water (10 mL) and saturated brine (10 mL × 3). The organic phase was dried over anhydrous sodium sulfate, filtered, and concentrated to dryness to give crude compound 950A. The crude product was purified by prep-HPLC (ammonium bicarbonate method) to give 2 isomeric compounds. The structure of one of the isomeric compounds was arbitrarily designated as (1r,2R,3S)-N-((6³S,4S,Z)-(Rₐ)-1²-(5-(3-(1,4-oxazepan-4-yl)prop-1-yn-1-yl)-2-isopropylpyridin-1-oxide-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z950, 4.2 mg, yield: 8.4%, retention time: 6.587 min), ES-API [M+H]⁺ = 864.4. The structure of the other isomeric compound was arbitrarily designated as (1r,2R,3S)-N-((6³S,4S,Z)-(Sₐ)-1²-(5-(3-(1,4-oxazepan-4-yl)prop-1-yn-1-yl)-2-isopropylpyridin-1-oxide-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z950-1, 4.7 mg, yield: 9.4%, retention time: 6.730 min), ES-API [M+H]⁺ = 864.4.

### Example 137. Synthesis of Compound Z961

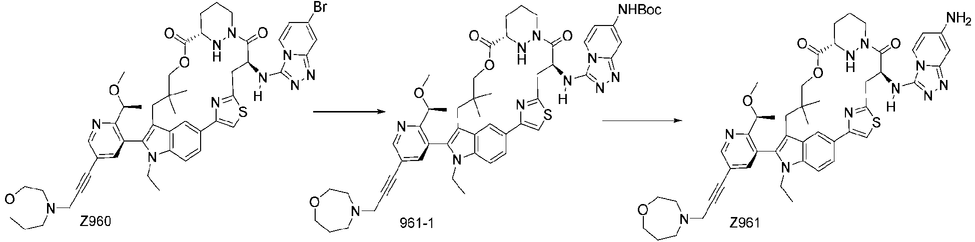

Step 1: tert-Butyl carbamate (40.0 mg, 0.341 mmol), palladium acetate (3.0 mg, 0.013 mmol), cesium carbonate (85.0 mg, 0.261 mmol), and 2-dicyclohexylphosphino-2',4',6'-triisopropylbiphenyl (10.0 mg, 0.021 mmol) were added to a solution of compound Z960 (110.0 mg, 0.114 mmol) in anhydrous dioxane (4.0 mL). The mixture was then stirred at 100 °C for 2 h under a nitrogen atmosphere. After the reaction was completed, the cooled solution was extracted with ethyl acetate (100 mL × 2) and saturated brine (100 mL). The ethyl acetate phase was dried over anhydrous sodium sulfate and filtered. The filtrate was concentrated, and then the residue was purified by silica gel column chromatography [petroleum ether:ethyl acetate = 100:0 to 30:70, (v/v)] to give compound 961-1 (80.0 mg, 0.080 mmol, yield: 70.10%). ES-API: [M+H]⁺ = 1000.3.

Step 2: Compound 961-1 (80.0 mg, 0.080 mmol) was dissolved in methanol (1.0 mL) under an ice-water bath, and a solution of hydrochloric acid in dioxane (1.0 mL, 4 M) was added. The mixture was reacted at room temperature for 1.5 h. After the reaction was completed, the mixture was concentrated by rotary evaporation under reduced pressure to remove the solvent. The resulting crude product was purified by prep-HPLC (formic acid method 1) to give (6³S,4S,Z)-(Rₐ)-1²-(5-(3-(1,4-oxazepan-4-yl)prop-1-yn-1-yl)-2-((S)-1-methoxyethyl)pyridin-3-yl)-4-((7-amino-[1,2,4]triazolo[4,3-a]pyridin-3-yl)amino)-1¹-ethyl-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione formate (Z961, 1.80 mg, 0.002 mmol, yield: 2.5%). ES-API: [M+H]⁺ = 900.3.

### Example 138. Synthesis of Compound Z962

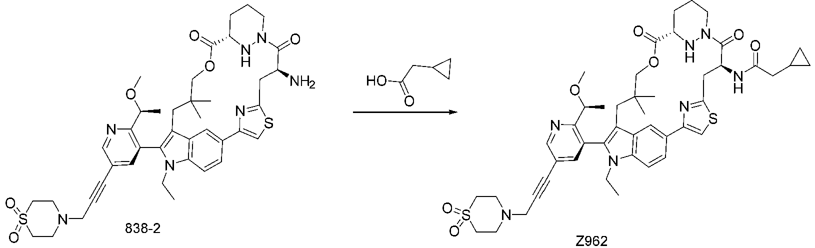

Step 1: N,N-Diisopropylethylamine (44 mg, 0.34 mmol) and O-(7-azabenzotriazol-1-yl)-N,N,N',N'-tetramethyluronium hexafluorophosphate (39 mg, 0.11 mmol) were sequentially added to a solution of 2-cyclopropylacetic acid (8 mg, 0.08 mmol) and compound 838-2 (60 mg, 0.07 mmol) in dichloromethane (2 mL). The mixture was stirred at room temperature for 1 h. After the reaction was completed, the mixture was washed with water (2 mL) and concentrated, and the residue was purified by prep-HPLC (ammonium bicarbonate method) to give 2-cyclopropyl-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1'-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)acetamide (Z962, 18.42 mg, yield: 30.2%, purity: 98%). ES-API: [M+H]⁺ = 884.3.

### Example 139. Synthesis of Compound Z963

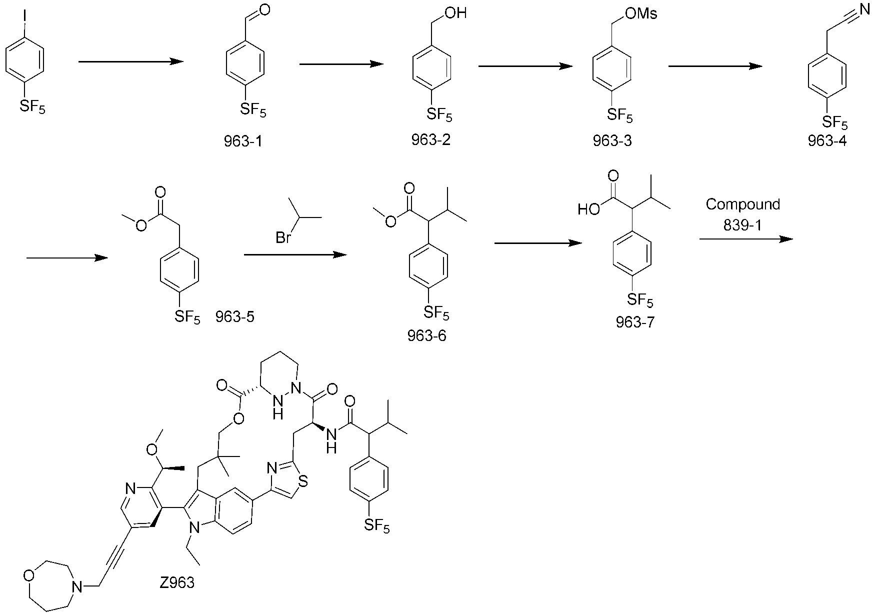

Step 1: Pentafluoro(4-iodophenyl)-λ⁶-sulfane (2500 mg, 8.832 mmol) was dissolved in tetrahydrofuran (30 mL), and the mixture was cooled to -78 °C. Isopropylmagnesium chloride (2 M, 5.681 mL, 11.362 mmol) was slowly added to the solution described above under a nitrogen atmosphere. After the addition was completed, the reaction system was stirred at -78 °C for 1 h. N,N-Dimethylformamide (1107 mg, 15.149 mmol) was then slowly added dropwise to the reaction system, and the reaction system was stirred at -78 °C for another 2 h. After the reaction was completed, the reaction was quenched with saturated ammonium chloride (20 mL), and the system was extracted with ethyl acetate (20 mL × 2). The organic phase was washed with saturated brine (30 mL × 2), dried over anhydrous sodium sulfate, filtered, and concentrated to give a crude product. The crude product was purified by a flash silica gel column (tetrahydrofuran/petroleum ether: 0%-12%) to give compound 963-1 (1300 mg, 5.599 mmol, yield: 73.92%) as a transparent oily liquid. ¹H NMR (400 MHz, CDCl₃) δ 10.03 (s, 1H), 7.96 - 7.84 (m, 4H).

Step 2: Compound 963-1 (1300 mg, 5.599 mmol) was dissolved in tetrahydrofuran (20 mL) and methanol (5 mL), and the mixture was cooled to 0 °C. Sodium borohydride (423.65 mg, 11.199 mmol) was added to the solution described above under a nitrogen atmosphere. After the addition was completed, the reaction system was stirred at 0 °C for 1 h. After the reaction was completed, the reaction was quenched with saturated ammonium chloride (20 mL), and the system was extracted with ethyl acetate (20 mL × 2). The organic phase was washed with saturated brine (20 mL × 2), dried over anhydrous sodium sulfate, filtered, and concentrated to give a crude product. The crude product was purified by a flash silica gel column (ethyl acetate/petroleum ether: 0%-20%) to give compound 963-2 (1100 mg, 4.697 mmol, yield: 83.89%) as a transparent oily liquid. Step 3: Compound 963-2 (1100 mg, 4.697 mmol) was dissolved in dichloromethane (25 mL) and triethylamine (0.890 mL, 6.405 mmol), and the mixture was cooled to 0 °C. Methanesulfonic anhydride (1636.42 mg, 9.394 mmol) was added to the solution described above under a nitrogen atmosphere. After the addition was completed, the reaction system was stirred at 0 °C for 1 h. After the reaction was completed, the reaction was quenched with water (20 mL), and the system was extracted with dichloromethane (20 mL × 2). The organic phase was washed with saturated brine (20 mL × 2), dried over anhydrous sodium sulfate, filtered, and concentrated to give a crude product. The crude product was purified by a flash silica gel column (ethyl acetate/petroleum ether: 0%-15%) to give compound 963-3 (1000 mg, 3.202 mmol, yield: 68.18%) as a white solid.

Step 4: Compound 963-3 (1000 mg, 3.202 mmol) was dissolved in tetrahydrofuran (20 mL) and a solution of tetrabutylammonium fluoride in tetrahydrofuran (1 M, 6.405 mL, 6.405 mmol). Trimethylsilyl cyanide (635.41 mg, 6.405 mmol) was added to the solution described above. After the addition was completed, the reaction system was stirred at room temperature for 5 h. After the reaction was completed, the reaction was quenched with water (20 mL), and the system was extracted with ethyl acetate (20 mL). The organic phase was washed with saturated brine (20 mL × 5), dried over anhydrous sodium sulfate, filtered, and concentrated to give a crude product. The crude product was purified by a flash silica gel column (ethyl acetate/petroleum ether: 0%-25%) to give compound 963-4 (600 mg, 2.467 mmol, yield: 77.04%) as a white solid. ¹H NMR (400 MHz, CDCl₃) δ 7.85 - 7.79 (m, 2H), 7.49 (d, *J* = 8.4 Hz, 2H), 3.85 (s, 2H).

Step 5: Compound 963-4 (400 mg, 1.645 mmol), a solution of hydrogen chloride in dioxane (4 M, 3 mL), and methanol (3 mL) were added to a sealed tube reactor. After the reaction was sealed, the mixture was stirred at 80 °C for 17 h. After the reaction was completed, the reaction mixture was concentrated. The residue was basified with a saturated aqueous sodium bicarbonate solution (20 mL) and extracted with dichloromethane (30 mL). The organic phase was washed with saturated brine (20 mL), dried over anhydrous sodium sulfate, filtered, and concentrated to give compound 963-5 (400 mg, 1.448 mmol, yield: 88.05%) as a white solid.

Step 6: Compound 963-5 (100 mg, 0.362 mmol) was dissolved in N,N-dimethylformamide (3 mL), and the mixture was cooled to 0 °C. A solution of potassium tert-butoxide in tetrahydrofuran (1 M, 0.471 mmol, 0.471 mL) was added to the solution described above, and the mixture was stirred for 30 min. 2-Bromopropane (45 mg, 0.362 mmol) was then dissolved in N,N-dimethylformamide (0.5 mL), and the resulting solution was slowly added dropwise to the reaction mixture. After the addition was completed, the reaction system was warmed to room temperature and stirred for 2 h. After the reaction was completed, the reaction was quenched with water (20 mL), and the system was extracted with ethyl acetate (20 mL × 2). The organic phase was washed with saturated brine (20 mL × 2), dried over anhydrous sodium sulfate, filtered, and concentrated to give a crude product. The crude product was purified by a flash silica gel column (ethyl acetate/petroleum ether: 0%-20%) to give compound 963-6 (40 mg, 0.126 mmol, yield: 34.71%) as a colorless oily liquid. ¹H NMR (400 MHz, CDCl₃) δ 7.66 - 7.60 (m, 2H), 7.37 (d, *J* = 8.4 Hz, 2H), 3.60 (s, 3H), 3.16 (d, *J* = 10.4 Hz, 1H), 2.33 - 2.21 (m, 1H), 0.97 (d, *J =* 6.4 Hz, 3H), 0.65 (d, *J* = 6.4 Hz, 3H).

Step 7: Compound 963-6 (40 mg, 0.126 mmol) was dissolved in tetrahydrofuran (0.5 mL), methanol (0.5 mL), and water (0.5 mL). Lithium hydroxide (21 mg, 0.503 mmol) was added to the solution described above, and the mixture was stirred for 17 h. The reaction mixture was acidified with diluted hydrochloric acid (0.5 M) and extracted with ethyl acetate (20 mL × 2). The organic phase was washed with saturated brine (20 mL × 2), dried over anhydrous sodium sulfate, filtered, and concentrated to give compound 963-7 (30 mg, 0.099 mmol, yield: 78.46%) as a white solid.

Step 8: Compound 839-1 (40 mg, 0.052 mmol) was dissolved in dichloromethane (3 mL). Compound 963-7 (23.73 mg, 0.078 mmol), O-(7-azabenzotriazol-1-yl)-*N,N,N',N'*-tetramethyluronium hexafluorophosphate (39.61 mg, 0.104 mmol), and *N,N'-*diisopropylethylamine (20 mg, 0.156 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried over anhydrous sodium sulfate, filtered, and concentrated to give a crude product. The crude product was subjected to prep-HPLC (ammonium bicarbonate method) to give N-((6³S,4S,Z)-(*Rₐ*)-1²-(5-(3-(1,4-oxazepan-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1 (5,3)-indola-6(1 ,3)-pyridazinacycloundecaphane-4-yl)-3-methyl-2-(4-(pentafluoro-λ⁶-sulfaneyl)phenyl)butanamide (Z963, 30 mg, 0.028 mmol, yield: 54.72%) as a white solid. ES-API: [M+H]⁺ = 1054.3.

### Example 140. Synthesis of Compound Z966

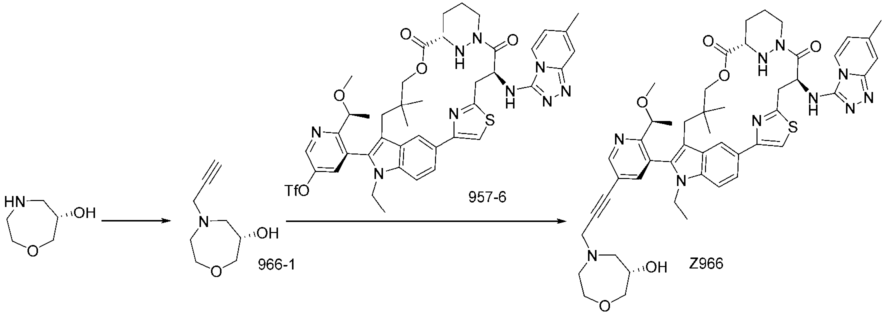

Step 1: (S)-[1,4]Oxazepane-6-hydroxy (150 mg, 1.280 mmol) was dissolved in acetonitrile (5 mL), and potassium carbonate (527.45 mg, 3.817 mmol) and 3-bromopropyne (152.32 mg, 1.280 mmol) were added. The mixture was reacted at room temperature for 4 h under a nitrogen atmosphere. After the reaction was completed, the mixture was filtered under vacuum and concentrated to dryness under reduced pressure, and the residue was purified by a flash silica gel column (ethyl acetate/petroleum ether: 0%-50%) to give compound 966-1 (120 mg, 0.773 mmol, yield: 60.39%) as a colorless oily liquid. Step 2: Compound 957-6 (30 mg, 0.034 mmol) and compound 966-1 (12 mg, 0.0773 mmol) were dissolved in acetonitrile (3 mL), and bis(triphenylphosphine)palladium(II) dichloride (16.02 mg, 0.023 mmol), copper(I) iodide (6.58 mg, 0.034 mmol), N,N-diisopropylethylamine (29.49 mg, 0.229 mmol), and anhydrous lithium chloride (9.60 mg, 0.229 mmol) were added. The mixture was reacted at 80 °C for 5 h under a nitrogen atmosphere. After the reaction was completed, the mixture was filtered under vacuum, dried, and concentrated to give a crude product, which was purified by a flash silica gel column (methanol/dichloromethane: 0%-10%) and further purified by prep-HPLC (ammonium bicarbonate method) to give (6³S,4S,Z)-1¹-ethyl-(*Rₐ*)-1²-(5-(3-((S)-6-hydroxy-1,4-oxazepan-4-yl)prop-1-yn-1-yl)-2-((S)-1-methoxyethyl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹*H*-8-oxa-2(4,2)-thiazola-1(5,3)-indola-6(1,3)-pyridazinacycloundecaphane-5,7-dione (Z966, 2 mg, 0.002 mmol, yield: 6.43%) as a white solid, ES-API: [M+H]⁺ = 915.3.

### Example 141. Synthesis of Compound Z969

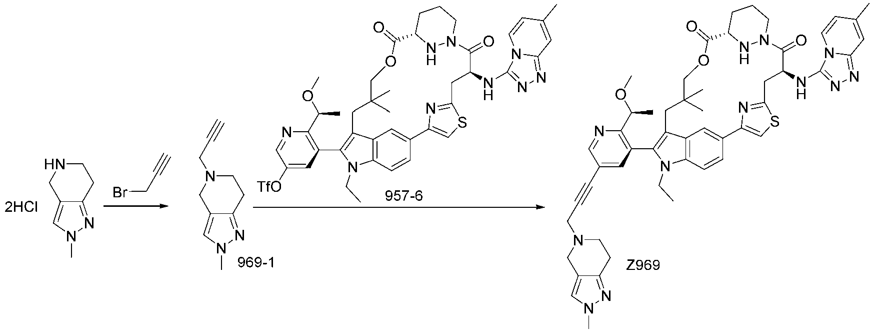

Step 1: 3-Bromoprop-1-yne (0.171 g, 1.437 mmol) and potassium carbonate (0.60 g, 4.326 mmol) were added to a solution of 2-methyl-4,5,6,7-tetrahydro-2H-pyrazolo[4,3-c]pyridine dihydrochloride (0.30 g, 1.442 mmol) in N,N-dimethylformamide (15.0 mL), and the mixture was stirred at 25 °C for 18 h. After the reaction was completed, the cooled solution was extracted with ethyl acetate (2 × 100 mL) and saturated brine (100 mL). The ethyl acetate phase was dried over anhydrous sodium sulfate and filtered. The filtrate was concentrated, and then the residue was purified by automated flash chromatography on silica gel [dichloromethane:methanol = 100:0 to 90:10, (v/v)] to give compound 969-1 (0.160 g, yield: 63.32%). ES-API: [M+H]⁺ = 176.2.

Step 2: Compound 957-6 (30.0 mg, 0.033 mmol) was added to a solution of compound 969-1 (40.0 mg, 0.228 mmol) in N,N-dimethylformamide (3.0 mL), and bis(triphenylphosphine)palladium(II) dichloride (7.0 mg, 0.010 mmol), copper(I) iodide (3.70 mg, 0.021 mmol), N,N-diisopropylethylamine (17.0 mg, 0.132 mmol), and lithium chloride (7.0 mg, 0.165 mmol) were added. After the addition, the reaction mixture was stirred at 80 °C for 1.5 h under a nitrogen atmosphere. The reaction mixture was then diluted with water (80 mL) and extracted with ethyl acetate (30 mL × 3). The organic layers were combined, washed with brine, dried over anhydrous sodium sulfate, filtered, and concentrated. The residue was purified by prep-HPLC (ammonium bicarbonate method) to give (6³S,4S,Z)-1¹-ethyl-1²-(2-((S)-1-methoxyethyl)-5-(3-(2-methyl-2,4,6,7-tetrahydro-5H-pyrazolo[4,3-c]pyridin-5-yl)prop-1-yn-1-yl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione hydrochloride (Z969, 11.5 mg, yield: 36.0%). ES-API: [M+H]⁺ = 935.3.

### Example 142. Synthesis of Compound Z847

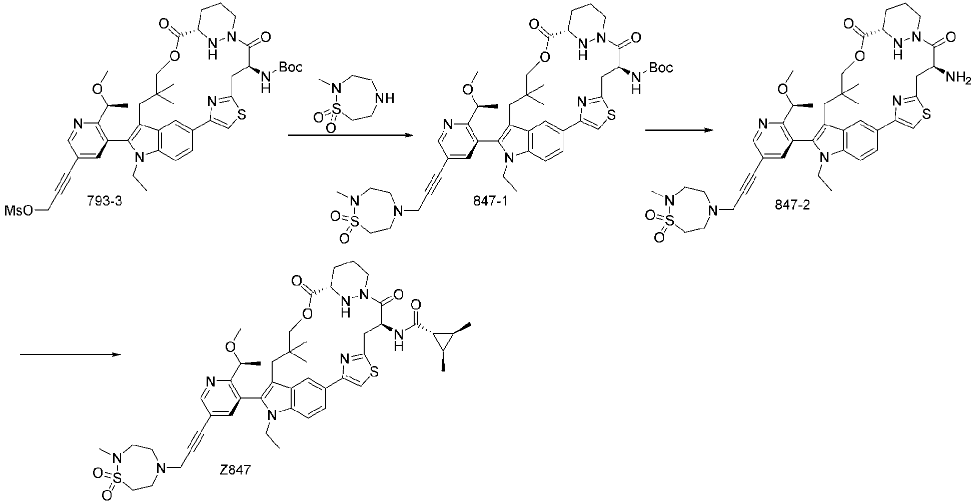

Step 1: N,N-Diisopropylethylamine (389.0 mg, 3.00 mmol), sodium iodide (11.3 mg, 0.075 mmol), and 2-methyl-1,2,5-thiadiazepane 1,1-dioxide (150.0 mg, 0.913 mmol) were sequentially added to a solution of compound 793-3 (130.0 mg, 0.151 mmol) in acetonitrile (10.0 mL). The reaction mixture was stirred at 80 °C for 1.5 h. When the reaction was completed as detected by LCMS, the mixture was concentrated under reduced pressure to remove the solvent. The residue was purified by automated flash chromatography on silica gel (tetrahydrofuran/petroleum ether = 0-50%) to give compound 847-1 (45.0 mg, yield: 32.08%). ES-API: [M+H]⁺ = 931.3.

Step 2: A solution of hydrochloric acid in methanol (5 mL, 4 M, 20.0 mmol) was added to a solution of compound 847-1 (45.0 mg, 0.048 mmol) in methanol (5 mL). The reaction mixture was stirred at room temperature for 1.5 h. When the reaction was completed as detected by LCMS, the mixture was concentrated under reduced pressure to remove most of the solvent, and 10 mL of a saturated sodium bicarbonate solution was added to the residue under an ice-water bath. The mixture was extracted with dichloromethane (15 mL × 3). The organic phases were combined, dried over anhydrous sodium sulfate, and concentrated. The crude product was purified by automated flash chromatography on silica gel (methanol/dichloromethane = 0-6%) to give compound 847-2 (50.0 mg, crude product). ES-API: [M+H]⁺ = 831.3.

Step 3: (1r,2R,3S)-2,3-Dimethylcyclopropane-1-carboxylic acid (15.0 mg, 0.131 mmol), N,N-diisopropylethylamine (0.21 mL, 1.225 mmol), and O-(7-azabenzotriazol-1-yl)-N,N,N',N'-tetramethyluronium hexafluorophosphate (55.0 mg, 0.144 mmol) were sequentially added to a solution of compound 847-2 (50.0 mg, crude product) in N,N-dimethylformamide (10 mL) under an ice-water bath. The reaction mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by prep-HPLC (ammonium bicarbonate method) to give (1r,2R,3S)-N-((6³S,4S,Z)-1¹-ethyl-1²-(2-((S)-1-methoxyethyl)-5-(3-(2-methyl-1,1-dioxido- 1,2,5-thiadiazepan-5-yl)prop-1-yn-1-yl)pyridin-3-yl)-10,10-dimethyl-5,7-dioxo-6¹,6²,6³,6⁴6⁵6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z847, 2.15 mg, yield: 4.83%). ES-API: [M+H]⁺ = 927.3.

### Example 143. Synthesis of Compound Z1032

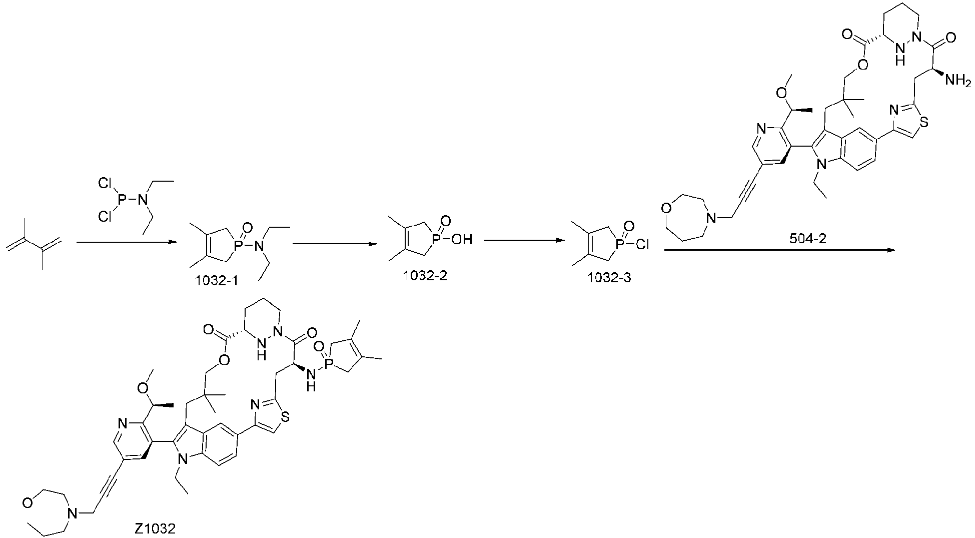

Step 1: Aluminum trichloride (7.14 g, 53.56 mmol) was suspended in dichloromethane (80 mL), and dichloro(diethylamino)phosphine (9.32 g, 53.56 mmol) was slowly added dropwise at 0 °C under a nitrogen atmosphere. The reaction system was stirred at 0 °C for 40 min until the solid was completely dissolved. A solution of 2,3-dimethylbuta-1,3-diene (4.0 g, 48.69 mmol) in dichloromethane (50 mL) was then slowly added dropwise. The reaction mixture was stirred at 0 °C for 8 h and then stirred at room temperature overnight. The reaction mixture was cooled to 0 °C and slowly added to a mixed solution of ethylenediaminetetraacetic acid (17.08 g, 58.43 mmol) in water (150 mL) and a saturated sodium bicarbonate solution (150 mL). The reaction system was stirred at 0 °C for another 4 h. The reaction mixture was filtered and washed with dichloromethane, and the filtrate was extracted with dichloromethane (200 mL × 2). The organic phase was washed sequentially with a saturated sodium bicarbonate solution (150 mL), 1 M diluted hydrochloric acid (150 mL), and saturated brine (150 mL), dried over anhydrous sodium sulfate, and filtered, and the filtrate was concentrated under reduced pressure. The crude product was purified by a flash silica gel column (methanol/dichloromethane: 0-5%) to give compound 1032-1 (1.0 g, yield: 10.2%) as a pale yellow solid. ES-API: [M+H]⁺ = 202.1. ¹H NMR (400 MHz, CDCl₃) δ 3.07 - 2.93 (m, 4H), 2.46 - 2.36 (m, 4H), 1.75 - 1.63 (m, 6H), 1.11 (t, *J =* 7.2 Hz, 6H).

Step 2: Compound 1032-1 (1.0 g, 4.97 mmol) was dissolved in ethanol (8 mL), and 6 M hydrochloric acid (10 mL) was added at 0 °C. The reaction system was stirred at room temperature for 18 h. The reaction mixture was concentrated, adjusted to pH 12 with a 4 M aqueous sodium hydroxide solution, and then concentrated. The residue was adjusted to pH 1 with 4 M hydrochloric acid and extracted with dichloromethane (25 mL × 2). The organic phase was dried over anhydrous sodium sulfate and filtered. The filtrate was concentrated under reduced pressure to give compound 1032-2 (700 mg, yield: 95.9%) as a white solid. ES-API: [M+H]⁺ = 147.1. ¹H NMR (400 MHz, DMSO-d6) δ 2.32 - 2.19 (m, 4H), 1.75 - 1.59 (m, 6H). Step 3: Compound 1032-2 (100 mg, 0.68 mmol) was dissolved in dichloromethane (2.5 mL), and thionyl chloride (0.25 mL, 3.43 mmol) was added. The reaction mixture was stirred at 40 °C for 3 h. The reaction mixture was then concentrated under reduced pressure to give compound 1032-3 (112 mg, yield: 99.4%) as a pale brown liquid, which was directly used in the next step without further purification.

Step 4: Compound 504-2 (80 mg, 0.110 mmol) and N,N-diisopropylethylamine (269 mg, 2.08 mmol) were dissolved in dichloromethane (5 mL), and compound 1032-3 (86 mg, 0.52 mmol) was added under an ice bath. The reaction system was stirred at room temperature for 1 h. Dichloromethane (20 mL) was added to the reaction mixture. The resulting mixture was washed sequentially with water (10 mL) and saturated brine (10 mL), dried over anhydrous sodium sulfate, and filtered. The filtrate was concentrated under reduced pressure. The crude product was purified by preparative thin-layer chromatography (petroleum ether/tetrahydrofuran = 1:4) to give the desired product (6³S,4S,Z)-(Rₐ)-1²-(5-(3-(1,4-oxazepan-4-yl)prop-1-yn-1-yl)-2-((S)-1-methoxyethyl)pyridin-3-yl)-4-((3,4-dimethyl-1-oxido-2,5-dihydrophosphol-1-yl)amino)-1¹-ethyl-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z1032, 30 mg, yield: 32.1%) as a white solid. ¹H NMR (400 MHz, DMSO-d6) δ 8.80 (d, *J* = 2.0 Hz, 1H), 8.51 (d, *J =* 1.2 Hz, 1H), 7.86 (d, *J* = 2.0 Hz, 1H), 7.82 (s, 1H), 7.76 (dd, *J* = 8.8, 1.2 Hz, 1H), 7.58 (d, *J* = 8.8 Hz, 1H), 5.10 - 4.91 (m, 3H), 4.40 - 4.21 (m, 3H), 4.19 - 3.98 (m, 2H), 3.74 - 3.55 (m, 8H), 3.27 (s, 1H), 3.21 (s, 3H), 3.02 (dd, *J =* 14.4, 8.0 Hz, 1H), 2.90 (d, *J* = 14.8 Hz, 1H), 2.84 -2.71 (m, 5H), 2.46 - 2.01 (m, 6H), 1.88 - 1.47 (m, 11H), 1.36 (d, *J* = 6.0 Hz, 3H), 0.97 - 0.81 (m, 6H),, 0.39 (s, 3H). ES-API: [M+H]⁺ = 896.3.

### Example 144. Synthesis of Compound Z974

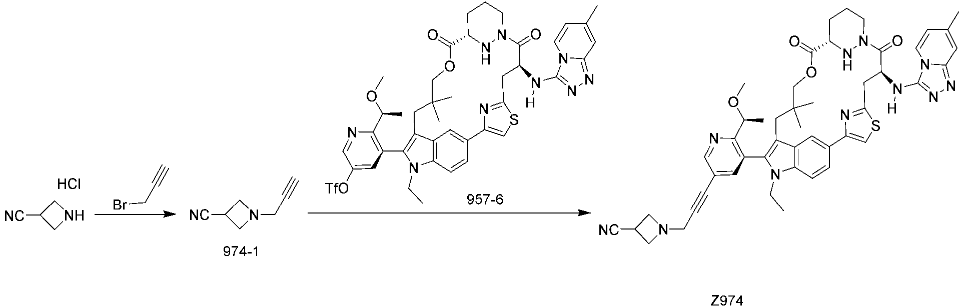

Step 1: Azetidine-3-carbonitrile hydrochloride (1000 mg, 8.435 mmol) was dissolved in acetonitrile (30 mL). 3-Bromopropyne (1003.37 mg, 8.435 mmol) and potassium carbonate (3497.15 mg, 25.305 mmol) were slowly added to the solution described above. After the addition was completed, the reaction system was stirred at 0 °C for 2 h. After the reaction was completed, water (30 mL) was added to the reaction mixture, and the mixture was extracted with ethyl acetate (30 mL × 2). The organic phase was washed with saturated brine (30 mL × 2), dried, and concentrated to give a crude product. The crude product was purified by a flash silica gel column (methanol/dichloromethane: 0%-3%) to give compound 974-1 (700 mg, 5.826 mmol, yield: 69.07%) as a colorless oily liquid. ES-API: [M+H]⁺ = 121.1.

Step 2: Compound 957-6 (30 mg, 0.033 mmol) was dissolved in acetonitrile (2 mL). 974-1 (11.88 mg, 0.099 mmol), bis(triphenylphosphine)palladium(II) dichloride (4.63 mg, 0.007 mmol), copper(I) iodide (2.51 mg, 0.013 mmol), N,N'-diisopropylethylamine (12.78 mg, 0.099 mmol), and lithium chloride (5.59 mg, 0.132 mmol) were added to the solution described above at room temperature. After the addition was completed, the reaction system was stirred at 80 °C for 4 h under an argon atmosphere. After the reaction was completed, water (10 mL) was added to the reaction mixture, and the mixture was extracted with ethyl acetate (10 mL × 2). The organic phase was washed with saturated brine (10 mL × 2), dried, and concentrated to give a crude product. The crude product was purified by prep-HPLC (ammonium bicarbonate method) to give 1-(3-(5-((6³,S,4S,Z)-1¹-ethyl-10,10-dimethyl-4-((7-methyl-[1,2,4]triazolo[4,3-a])pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-(Rₐ)-((S)-1-methoxyethyl)pyridin-3-yl)prop-2-yn-1-yl)azetidine-3-carbonitrile (Z974, 5 mg, 0.006 mmol, yield: 17.24%) as a light yellow solid. ES-API: [M+H]⁺ = 880.3. ¹H NMR (300 MHz, CD₃OD) δ 8.84 (d, *J =* 2.1 Hz, 1H), 8.62 (s, 1H), 8.14 (d, *J =* 7.2 Hz, 1H), 7.94 (d, *J =* 2.1 Hz, 1H), 7.73 (dd, *J =* 8.4 Hz, 1.2 Hz, 1H), 7.57 - 7.48 (m, 2H), 7.21 (s, 1H), 6.73 (d, *J =* 7.2 Hz, 1H), 5.62 (d, *J =* 8.4 Hz, 1H), 4.55 - 4.30 (m, 4H), 4.25 - 4.10 (m, 1H), 3.80 - 3.72 (m, 4H), 3.67 - 3.58 (m, 5H), 3.52 - 3.42 (m, 2H), 3.38 (s, 3H), 3.15 (t, *J =* 14.4 Hz, 1H), 2.83 (t, *J =* 11.1 Hz, 1H), 2.57 (d, *J =* 14.4 Hz, 1H), 2.40 (s, 3H), 2.27 (d, *J* = 10.5 Hz, 1H), 2.06 - 1.80 (m, 2H), 1.75 - 1.55 (m, 1H), 1.47 (d, *J* = 6.0 Hz, 3H), 1.02 (s, 3H), 0.97 (t, *J =* 6.9 Hz, 3H), 0.49 (s, 3H).

### Example 145. Synthesis of Compounds Z970 and Z967

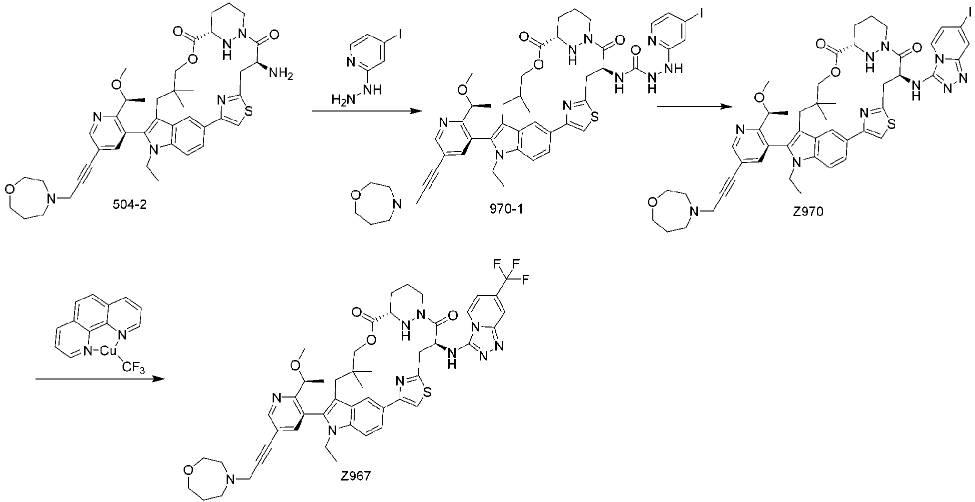

Step 1: Compound 504-2 (400 mg, 0.521 mmol) and N,N-diisopropylethylamine (336.59 mg, 2.604 mmol) were dissolved in dichloromethane (10 mL). Nitrophenyl chloroformate (136.47 mg, 0.677 mmol) was added at 0 °C. The reaction system was stirred under an ice bath for 0.5 h, and then N,N-diisopropylethylamine (195.34 mg, 1.511 mmol) and 4-iodo-2-hydrazineylpyridine (355.37 mg, 1.512 mmol) were added. The reaction system was stirred at 0 °C for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-7%) to give compound 970-1 (450 mg, 0.437 mmol, yield: 83.9%) as a light yellow solid. ES-API: [M+H]⁺ = 1029.3.

Step 2: Compound 970-1 (450 mg, 0.437 mmol) was dissolved in 1,2-dichloroethane (10 mL) and pyridine (0.528 mL, 6.560 mmol). Phosphorus oxychloride (0.122 mL, 1.312 mmol) was added to the solution described above. After the addition was completed, the reaction system was stirred at 45 °C for 5 min. The reaction mixture was cooled to room temperature and concentrated. The crude product was purified by a flash silica gel column (methanol/dichloromethane: 0-7%) to give (6³S,4S,Z)-1²-(*Rₐ*)-(5-(3-(1,4-oxazepan-4-yl)prop-1-yn-1-yl)-2-((S)-1-methoxyethyl)pyridin-3-yl)-1¹-ethyl-4-((7-iodo-[1,2,4]triazolo[4,3-a]pyridin-3-yl)amino)-10,10-dimethyl-6¹,6₂,6₃,6₄,6₅,6⁶-hexahydro-1¹H-8-oxa-2(4,2)-thiazola-1(5,3)-indola-6(1,3)-pyridazinacycloundecaphane-5,7-dione (Z970, 300 mg, 0.072 mmol, yield: 67.85%) as a light yellow solid. ES-API: [M+H]⁺ = 1011.3.

Step 3: Compound Z970 (30 mg, 0.029 mmol) was dissolved in N,N-dimethylformamide (1 mL), and (1,10-phenanthroline)(trifluoromethyl)copper(I) (10.03 mg, 0.032 mmol) was added to the solution described above. After the addition was completed, the reaction system was stirred at 50 °C for 30 min under a nitrogen atmosphere. After the reaction was completed, water (10 mL) was added to the reaction mixture, and the mixture was extracted with ethyl acetate (15 mL × 2). The organic phase was washed with saturated brine (10 mL × 3), dried, and concentrated. The residue was purified by a flash silica gel column (methanol/dichloromethane: 0%-7%) to give a crude product. The crude product was purified by prep-HPLC (ammonium bicarbonate method) to give (6³S,4S,Z)-1²-(5-(3-(1,4-oxazepan-4-yl)prop-1-yn-1-yl)-2-((S)-1-methoxyethyl)pyridin-3-yl)-1¹-ethyl-10,10-dimethyl-4-((7-(trifluoromethyl)-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z967, 5 mg, 0.005 mmol, yield: 17.99%) as a white solid. ES-API: [M+H]⁺ = 953.3.

### Example 146. Synthesis of Compound Z965

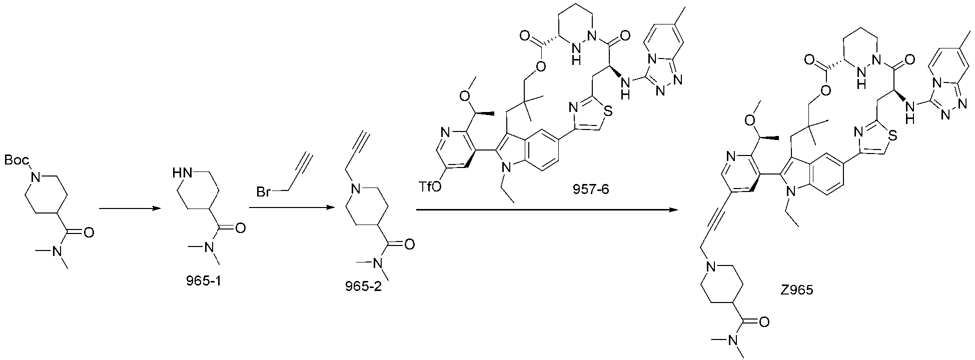

Step 1: tert-Butyl 4-(dimethylcarbamoyl)piperidine-1-carboxylate (711 mg, 2.774 mmol) was dissolved in a solution of hydrogen chloride in methanol (20 mL, 4 M). The reaction mixture was stirred at room temperature for 0.5 h. When the reaction was completed as detected by LCMS, the mixture was concentrated under reduced pressure to remove most of the solvent to give crude compound 965-1 (433 mg, yield: 99%). ES-API: [M+H]⁺ = 157.3.

Step 2: 3-Bromopropyne (330 mg, 2.772 mmol) was slowly added dropwise to a solution of compound 965-1 (433 mg, 2.772 mmol) and potassium carbonate (1149 mg, 8.315 mmol) in acetonitrile (15 mL) under an ice-water bath. The reaction mixture was stirred at 0 °C for 1 h. The reaction was completed as detected by LCMS. The mixture was filtered to remove insoluble substances, and the filtrate was concentrated to dryness under reduced pressure. The residue was purified by automated flash chromatography on silica gel (tetrahydrofuran/petroleum ether = 0-50%) to give compound 965-2 (50 mg, yield: 9%). ES-API: [M+H]⁺ = 195.3.

Step 3: Bis(triphenylphosphine)palladium(II) dichloride (9.2 mg, 0.013 mmol), copper(I) iodide (5 mg, 0.026 mmol), N,N-diisopropylethylamine (0.04 mL, 0.220 mmol), and lithium chloride (9.3 mg, 0.220 mmol) were added to a solution of compound 957-6 (40 mg, 0.044 mmol) and compound 965-2 (26 mg, 0.132 mmol) in N,N-dimethylformamide (2 mL) under a nitrogen atmosphere. The reaction mixture was purged 3 times with nitrogen, heated to 80 °C, and stirred for 2 h. The reaction was completed as detected by LCMS. The reaction mixture was cooled to room temperature, and 20 mL of water was added to the reaction mixture. The resulting mixture was then extracted with ethyl acetate (10 mL × 3). The organic phases were combined, washed with saturated brine (10 mL × 3), dried over anhydrous sodium sulfate, filtered, and concentrated to dryness. The residue was purified by prep-HPLC (hydrochloric acid method) to give the desired compound 1-(3-(5-((6³S,4S,Z)-(Rₐ)-1¹-ethyl-10,10-dimethyl-4-((7-methyl-[1,2,4]triazolo[4,3-a]pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-((S)-1-methoxyethyl)pyridin-3-yl)prop-2-yn-1-yl)-*N*,*N*-dimethylpiperidine-4-carboxamide (Z965, 2.27 mg, yield: 5.4%, retention time: 2.04 min), ES-API [M+H]⁺ = 954.3.

### Example 147. Synthesis of Compounds Z976-1 and Z976

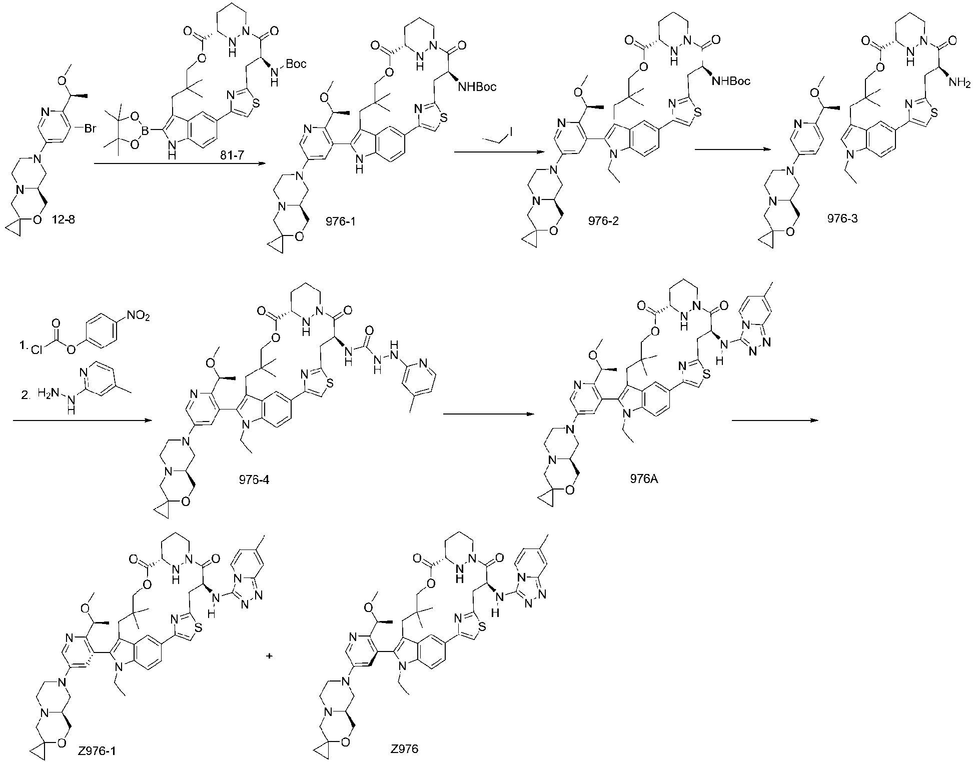

Step 1: Compound 12-8 (130 mg, 0.340 mmol), compound 81-7 (236 mg, 0.340 mmol), potassium phosphate (216.5 mg, 1.020 mmol) and [1,1'-bis(di-tert-butylphosphino)ferrocene]dichloropalladium(II) (44 mg, 0.068 mmol) were dissolved in dioxane (1 mL), toluene (3 mL), and water (1 mL) under a nitrogen atmosphere. The reaction mixture was heated to 65 °C under an oil bath and stirred for 2 h. When the reaction was completed as detected by LCMS, the mixture was cooled to room temperature. 15 mL of ethyl acetate was added to the reaction mixture, and the mixture was washed with water (10 mL) and saturated brine (10 mL × 3). The organic phase was dried over anhydrous sodium sulfate, filtered, and concentrated to dryness. The residue was purified by automated flash chromatography on silica gel (methanol:dichloromethane = 0-10%) to give compound 976-1 (220 mg, yield: 74%). ES-API: [M+H]⁺ = 869.3.

Step 2: Cesium carbonate (824 mg, 2.531 mmol) and iodoethane (194 mg, 1.266 mmol) were sequentially added to a solution of compound 976-1 (220 mg, 0.253 mmol) in N,N-dimethylformamide (10 mL). The reaction mixture was stirred at room temperature for 2 h. When the reaction was completed as detected by LCMS, the reaction mixture was diluted with 20 mL of ethyl acetate and then washed with water (10 mL) and saturated brine (15 mL × 3). The organic phase was dried over anhydrous sodium sulfate, filtered, and concentrated under reduced pressure to give crude compound 976-2 (180 mg, yield: 79%). ES-API: [M+H]⁺ = 897.3.

Step 3: Compound 976-2 (180 mg, 0.201 mmol) was dissolved in a solution of hydrochloric acid in dioxane (10 mL, 4 M) and a solution of hydrochloric acid in methanol (2 mL, 4 M). The reaction mixture was stirred at room temperature for 1 h. When the reaction was completed as detected by LCMS, the mixture was concentrated under reduced pressure to remove most of the solvent. The residue was adjusted to pH 7.0-8.0 with a saturated sodium bicarbonate solution (5 mL) and extracted with dichloromethane (10 mL × 3). The organic phases were combined, washed with saturated brine (10 mL × 2), dried over anhydrous sodium sulfate, filtered, and concentrated under reduced pressure to give crude compound 976-3 (130 mg, yield: 81%). ES-API: [M+H]⁺ = 797.3.

Step 4: N,N-Diisopropylethylamine (0.13 mL, 0.753 mmol) and p-nitrophenyl chloroformate (36 mg, 0.181 mmol) were sequentially added to a solution of compound 976-3 (120 mg, 0.151 mmol) in dichloromethane (10 mL) under an ice-water bath. The reaction mixture was stirred at 0 °C for 5 min. 2-Hydrazineyl-4-methylpyridine (74 mg, 0.602 mmol) was then rapidly added to the reaction mixture described above, and the reaction mixture was stirred at 0 °C for another 10 min. The reaction was completed as detected by LCMS. The reaction mixture was diluted with dichloromethane (20 mL) and then washed with a saturated sodium bicarbonate solution (15 mL × 3) and saturated brine (15 mL × 3). The organic phase was dried over anhydrous sodium sulfate, filtered, and concentrated to dryness. The residue was purified by automated flash chromatography on silica gel (methanol:dichloromethane = 0-10%) to give compound 976-4 (122 mg, yield: 86%). ES-API: [M+H]⁺ = 946.2.

Step 5: Pyridine (0.16 mL, 1.934 mmol) and phosphorus oxychloride (0.06 mL, 0.645 mmol) were sequentially added to a solution of compound 976-4 (122 mg, 0.129 mmol) in dichloromethane (10 mL). The reaction mixture was heated to 50 °C and stirred for 30 min. The reaction was completed as detected by LCMS. The mixture was concentrated under reduced pressure to remove the solvent, and the residue was purified by automated flash chromatography on silica gel (ammonia-methanol:dichloromethane = 0-5%) to give compound (6³S,4S,Z)-1¹-ethyl-1²-(2-((S)-1-methoxyethyl)-5-((S)-tetrahydro-4'-pyrazolo[cyclopropane-1,3'-pyrazino[2,1-c][1,4]oxazin]-8'(1'H)-yl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1'H-8-oxa-2(4,2)-thiazola-1(5,3)-indola-6(1,3)-pyridazinacycloundecaphane-5,7-dione (976A, 78 mg, yield: 65%). ES-API: [M+H]⁺ = 928.3.

Step 6: Compound 976A (78 mg) was resolved by a chiral column (CHIRALPAK^{®} IB 30 × 250 mm, 10 µm ; mobile phase A: CO₂, mobile phase B: MEOH + 0.2% DEA; detection wavelength: 214 nm/254 nm; flow rate: 25 mL/min; injection volume: 5 µL; column temperature: 30 °C; run time: 15 min; isocratic elution program: mobile phase A:mobile phase B = 55:45 (V/V)) to give 2 isomeric compounds. The structure of one of the isomeric compounds was arbitrarily designated as (6³S,4S,Z)-1¹-ethyl-1²-(*Sₐ*)-(2-((S)-1-methoxyethyl)-5-((S)-tetrahydro-4'-pyrazolo[cyclopropane-1,3'-pyrazino[2,1-c][1,4]oxazin]-8'(1'H)-yl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z976-1, 31.85 mg, retention time: 7.74 min). ES-API [M+H]⁺ = 928.3. ¹H NMR (400 MHz, DMSO-d6) δ 8.54 (s, 1 H), 8.47 (d, J= 2.8 Hz, 1 H), 8.28 (d, J= 7.2 Hz, 1 H), 7.77 (s, 1 H), 7.71 (dd, J1= 1.6 Hz, J2= 8.4 Hz, 1 H), 7.51 (d, J= 8.4 Hz, 1 H), 7.39 (d, J= 2.4 Hz, 1 H), 7.22- 7.18 (m, 2 H), 6.71 (dd, J1= 1.2 Hz, J2= 7.2 Hz, 1 H), 5.51 (t, J= 10.0 Hz, 1 H), 5.17 (d, J= 12.4 Hz, 1 H), 4.34- 4.24 (m, 2 H), 3.96- 3.79 (m, 4 H), 3.72- 3.64 (m, 3 H), 3.59 (d, J= 11.2 Hz, 1 H), 3.50 (d, J= 14.4 Hz, 1 H), 3.30-3.22 (m, 2 H), 3.08 (s, 3 H), 3.03 (d, J= 14.0 Hz, 1 H), 2.90- 2.84 (m, 2 H), 2.79- 2.76 (m, 1 H), 2.67- 2.64 (m, 1 H), 2.45-2.43 (m, 2 H), 2.32- 2.28 (m, 5 H), 2.21 (d, J= 10.8 Hz, 1 H), 2.17- 2.14 (m, 1 H), 1.86- 1.80 (m, 2 H), 1.61- 1.51 (m, 1 H), 1.22 (d, J= 6.4 Hz, 4 H), 1.09 (t, J= 7.2 Hz, 3 H), 1.00- 0.91 (m, 3 H), 0.73- 0.68 (m, 1 H), 0.63- 0.57 (m, 1 H), 0.54- 0.50 (m, 4 H).

The structure of the other isomeric compound was arbitrarily designated as: (6³S,4S,Z)-1¹-ethyl-1²-(*Rₐ*)-(2-((S)-1-methoxyethyl)-5-((S)-tetrahydro-4'-pyrazolo[cyclopropane-1,3'-pyrazino[2,1-c][1,4]oxazin]-8'(1'H)-yl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z976, 22.39 mg, retention time: 9.98 min). ES-API [M+H]⁺ = 928.3. ¹H NMR (400 MHz, DMSO-d6) δ 8.51 (s, 1 H), 8.46 (d, J= 2.4 Hz, 1 H), 8.26 (d, J= 7.2 Hz, 1 H), 7.77 (s, 1 H), 7.72 (d, J= 7.6 Hz, 1 H), 7.54 (d, J= 8.4 Hz, 1 H), 7.24- 7.16 (m, 3 H), 6.71 (d, J= 7.2 Hz, 1 H), 5.53 (t, J= 20.0 Hz, 1 H), 5.20 (d, J= 11.6 Hz, 1 H), 4.30- 4.12 (m, 5 H), 3.82- 3.80 (m, 1 H), 3.75- 3.67 (m, 2 H), 3.62- 3.54 (m, 2 H), 3.51- 3.47 (m, 1 H), 3.21-3.13 (m, 3 H), 2.98- 2.77 (m, 4 H), 2.67- 2.58 (m, 3 H), 2.45- 2.42 (m, 2 H), 2.34- 2.26 (m, 5 H), 2.24- 2.21 (m, 1 H), 2.15-2.08 (m, 1 H), 1.85- 1.79 (m, 2 H), 1.60- 1.49 (m, 1 H), 1.35- 1.33 (m, 4 H), 1.09- 0.97 (m, 3 H), 0.94- 0.83 (m, 4 H), 0.72-0.48 (m, 5 H).

### Example 148. Synthesis of Compound Z964

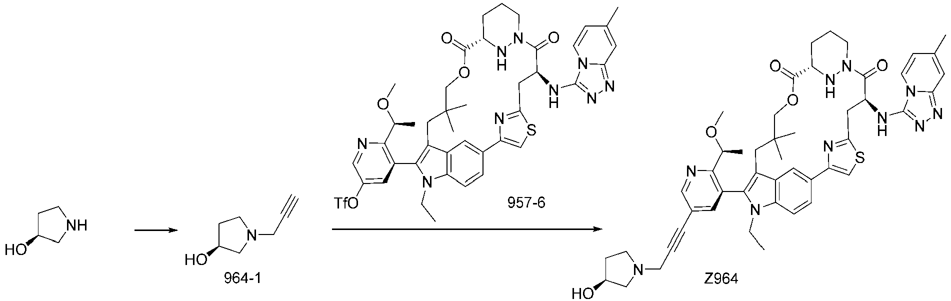

Step 1: Potassium carbonate (793 mg, 5.74 mmol) and 3-bromoprop-1-yne (410 mg, 3.44 mmol) were added to a solution of (3S)-tetrahydropyrrol-3-ol (250 mg, 2.87 mmol) in acetonitrile (10 mL) under an ice bath. The mixture was stirred for 2 h. The reaction mixture was filtered and concentrated, and the residue was purified by column chromatography (0-60% petroleum ether/tetrahydrofuran) to give compound 964-1 (80 mg) as a yellow oil. ES-API: [M+H]⁺ = 126.2.

Step 2: Compound 957-6 (60 mg, 0.07 mmol) and compound 964-1 (41 mg, 0.33 mmol) were dissolved in N,N-dimethylformamide (2 mL), and bis(triphenylphosphine)palladium(II) dichloride (9 mg, 0.01 mmol), copper(I) iodide (5 mg, 0.03 mmol), N,N-diisopropylethylamine (0.034 mL, 0.2 mmol), and lithium chloride (11 mg, 0.26 mmol) were sequentially added. The mixture was heated to 80 °C and reacted for 3 h under a nitrogen atmosphere, then diluted with ethyl acetate (20 mL), washed sequentially with water (20 mL) and saturated brine (20 mL), dried over anhydrous sodium sulfate, filtered, and concentrated. The residue was purified by prep-HPLC (ammonium bicarbonate method) to give (6³S,4S,Z)-1¹-ethyl-1²-(5-(3-((S)-3-hydroxypyrrolidin-1-yl)prop-1-yn-1-yl)-(Rₐ)-2-((S)-1-methoxyethyl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1'H-8-oxa-2(4,2)-thiazola-1(5,3)-indola-6(1,3)-pyridazinacycloundecaphane-5,7-dione (Z964, 5.4 mg, yield: 9.2%). ES-API: [M+H]⁺ = 885.3.

### Example 149. Synthesis of Compound Z1033

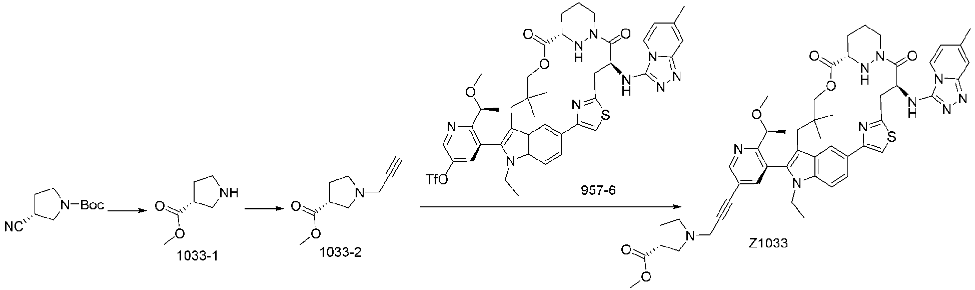

Step 1: 2-Methylpropan-2-yl (3R)-3-cyanotetrahydropyrrole-1-carboxylate (300 mg, 1.53 mmol) was dissolved in methanol (2 mL), and a 4 M solution of hydrochloric acid in dioxane (2 mL) was added. The mixture was stirred at room temperature for 2 h. The reaction mixture was concentrated to give compound 1033-1 (150 mg) as a white solid.

Step 2: Potassium carbonate (482 mg, 3.48 mmol) and 3-bromoprop-1-yne (131 mg, 1.10 mmol) were added to a solution of compound 1033-1 (150 mg, 1.16 mmol) in acetonitrile (10 mL) under an ice bath. The mixture was stirred for 2 h. The reaction mixture was filtered and concentrated, and the residue was purified by column chromatography (0-60% petroleum ether/tetrahydrofuran) to give compound 1033-2 (120 mg) as a yellow oil. ES-API: [M+H]⁺ = 168.2.

Step 3: Compound 957-6 (60 mg, 0.07 mmol) and compound 1033-2 (33 mg, 0.20 mmol) were dissolved in a solution of N,N-dimethylformamide (2 mL), and bis(triphenylphosphine)palladium(II) dichloride (9 mg, 0.01 mmol), copper(I) iodide (5 mg, 0.03 mmol), N,N-diisopropylethylamine (0.034 mL, 0.2 mmol), and lithium chloride (11 mg, 0.26 mmol) were sequentially added. The mixture was heated to 80 °C and reacted for 3 h under a nitrogen atmosphere, then diluted with ethyl acetate (20 mL), washed sequentially with water (20 mL) and saturated brine (20 mL), dried over anhydrous sodium sulfate, filtered, and concentrated. The residue was purified by prep-HPLC (ammonium bicarbonate method) to give methyl (3R)-1-(3-(5-((6³S,4S,Z)-1¹-ethyl-10,10-dimethyl-4-((7-methyl-[1,2,4]triazolo[4,3-a]pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-(Rₐ)-6-((S)-1-methoxyethyl)pyridin-3-yl)prop-2-yn-1-yl)pyrrolidine-3-carboxylate (Z1033, 14.38 mg, yield: 23.5%). ES-API: [M+H]⁺ = 927.2.

### Example 150. Synthesis of Compound Z1010

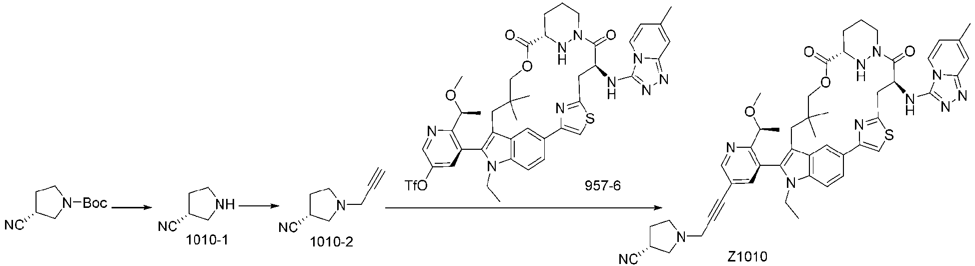

Step 1: 2-Methylpropan-2-yl (3R)-3-cyanotetrahydropyrrole-1-carboxylate (300 mg, 1.53 mmol) was dissolved in dichloromethane (2 mL), and trifluoroacetic acid (2 mL) was added. The mixture was stirred at room temperature for 2 h. The reaction mixture was concentrated to give compound 1010-1 (150 mg) as a yellow oil.

Step 2: Potassium carbonate (647 mg, 4.68 mmol) and 3-bromoprop-1-yne (176 mg, 1.48 mmol) were added to a solution of compound 1010-1 (150 mg, 1.56 mmol) in acetonitrile (10 mL) under an ice bath. The mixture was stirred for 2 h. The reaction mixture was filtered and concentrated, and the residue was purified by column chromatography (0-60% petroleum ether/tetrahydrofuran) to give compound 1010-2 (120 mg) as a yellow oil. ES-API: [M+H]⁺ = 135.2.

Step 3: Compound 957-6 (60 mg, 0.07 mmol) and compound 1010-2 (44 mg, 0.33 mmol) were dissolved in N,N-dimethylformamide (2 mL), and bis(triphenylphosphine)palladium(II) dichloride (9 mg, 0.01 mmol), copper(I) iodide (5 mg, 0.03 mmol), N,N-diisopropylethylamine (0.034 mL, 0.2 mmol), and lithium chloride (11 mg, 0.26 mmol) were sequentially added. The mixture was heated to 80 °C and reacted for 3 h under a nitrogen atmosphere, then diluted with ethyl acetate (20 mL), washed sequentially with water (20 mL) and saturated brine (20 mL), dried over anhydrous sodium sulfate, filtered, and concentrated. The residue was purified by prep-HPLC (ammonium bicarbonate method) to give (3R)-1-(3-(5-((6³S,4S,Z)-1¹-ethyl-10,10-dimethyl-4-((7-methyl-[1,2,4]triazolo[4,3-a]pyridin-3-yl)amino)-5,7-dioxo-6¹,6²,6³,6⁴,6⁵ 6⁶-hexahydro-1¹H-8-oxa-2(4,2)-thiazola-1(5,3)-indola-6(1,3)-pyridazinacycloundecaphane-1²-yl)-(Rₐ)-6-((S)-1-methoxyethyl)pyridin-3-yl)prop-2-yn-1-yl)pyrrolidine-3-carbonitrile (Z1010, 9.12 mg, yield: 15.5%). ES-API: [M+H]⁺ = 894.3.

### Example 151. Synthesis of Compound Z978

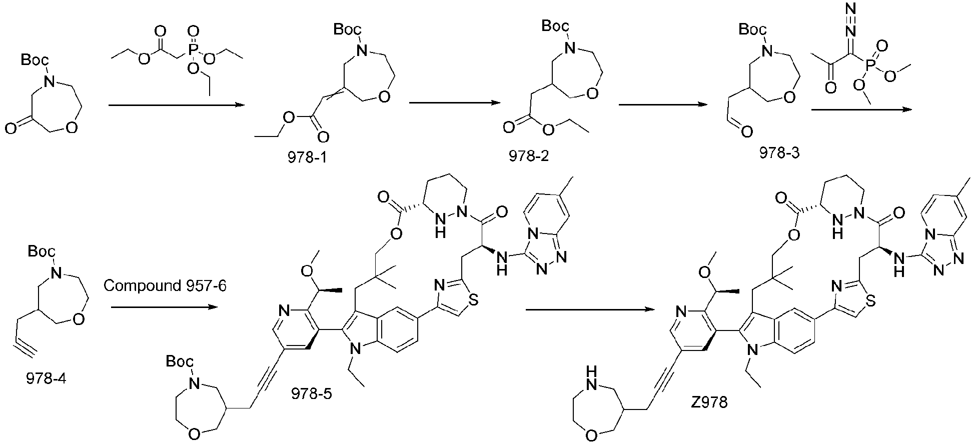

Step 1: Sodium hydride (1.11 g, 27.87 mmol) was dissolved in 30.0 mL of anhydrous tetrahydrofuran. A solution of triethyl phosphonoacetate (6.25 g, 27.87 mmol) in tetrahydrofuran (10 mL) was added at 0 °C. The mixture was reacted for 1 h, and then a solution of tert-butyl 6-oxo-1,4-oxazepane-4-carboxylate (5.0 g, 23.23 mmol) in tetrahydrofuran (30 mL) was added. After the dropwise addition was completed, the reaction mixture was warmed to 20 °C and reacted for 3 h. After the reaction was completed, the reaction was quenched with saturated ammonium (300 mL), and the mixture was extracted with ethyl acetate (300 mL), dried over anhydrous sodium sulfate, and concentrated by distillation under reduced pressure to give crude compound 978-1 (6.0 g, yield: 90.52%), which was directly used in the next step. ES-API: [M-56+H]⁺ = 230.1.

Step 2: Palladium on carbon (0.6 g) was added to a solution of compound 978-1 (6.0 g, 21.028 mmol) in ethanol (10.0 mL), and the mixture was stirred at 25 °C for 3-12 h under a hydrogen atmosphere. After the reaction was completed, the solution was filtered, and the filtrate was dried under reduced pressure to give compound 978-2 (5.0 g, yield: 82.75%). ES-API: [M-56+H]⁺ = 232.1.

Step 3: A solution of compound 978-2 (1.96 g, 6.821 mmol) in anhydrous dichloromethane (50 mL) was cooled to -78 °C under a nitrogen atmosphere. A solution of DIBALH in cyclohexane (1.01 M, 20.5 mL, 20.5 mmol) was added dropwise, and the solution was stirred at -78 °C until the starting material was completely reacted (the reaction was completed as monitored by TLC, reaction time: about 0.75 h). Methanol (20.0 mL) was added at -78 °C, and then the solution was warmed to room temperature and poured into a saturated aqueous potassium sodium tartrate solution. The aqueous layer was separated and extracted with dichloromethane (3 × 100 mL). The organic layers were combined, dried (over anhydrous magnesium sulfate), filtered, and concentrated by evaporation. The residue was purified by flash silica gel column chromatography [petroleum ether:ethyl acetate = 100:0 to 50:50, (v/v)] to give compound 978-3 (1400 mg, yield: 84.36%). ES-API: [M-56+H]⁺ = 188.2. Step 4: Compound 978-3 (890 mg, 3.658 mmol) was added to a solution of dimethyl (1-diazo-2-oxopropyl)phosphonate (843.29 mg, 4.390 mmol) and potassium carbonate (1011.08 mg, 7.316 mmol) in methanol (30 mL), and the mixture was stirred at room temperature for 3 h. The mixture was then diluted with ethyl acetate (300 mL) and washed with water (80 mL). The organic phase was dried over anhydrous sodium sulfate, filtered, and concentrated to give compound 978-4 (633.0 mg, 2.645 mmol, yield: 72.31%). ES-API: [M-56+H]⁺ = 184.1.

Step 5: Compound 957-6 (120.0 mg, 0.132 mmol) was added to a solution of tert-butyl 6-(prop-2-yn-1-yl)-1,4-oxazepane-4-carboxylate (120.0 mg, 0.50 mmol) in N,N-dimethylformamide (3.0 mL), and bis(triphenylphosphine)palladium(II) dichloride (59.89 mg, 0.085 mmol), copper(I) iodide (150.00 mg, 0.866 mmol), N,N-diisopropylethylamine (0.092 mL, 0.527 mmol), and lithium chloride (28.0 mg, 0.660 mmol) were added. After the addition, the reaction mixture was stirred at 80 °C for 1.5 h under a nitrogen atmosphere. The reaction mixture was then diluted with water (80 mL) and extracted with ethyl acetate (30 mL × 3). The organic layers were combined, washed with brine, dried over anhydrous sodium sulfate, filtered, and concentrated. The residue was purified by automated flash chromatography on silica gel [petroleum ether:tetrahydrofuran = 100:0 to 50:50, (v/v)] to give compound 978-5 (128.0 mg, yield: 97.14%). ES-API: [M+H]⁺ = 999.5.

Step 6: A solution of hydrochloric acid in dioxane (4.0 mL, 4 M, 16.0 mmol) was added to a solution of compound 978-5 (128.0 mg, 0.128 mmol) in methanol (4.0 mL) at 0 °C. The mixture was stirred at room temperature for 2 h and concentrated by evaporation under reduced pressure to remove the solvent to give a crude product, which was then purified by prep-HPLC (trifluoroacetic acid method) to give (6³S,4S,Z)-1²-(5-(3-(1,4-oxazepan-6-yl)prop-1-yn-1-yl)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione trifluoroacetate (Z978, 11.0 mg, yield: 8.5%).

### Example 152. Synthesis of Compound Z1011

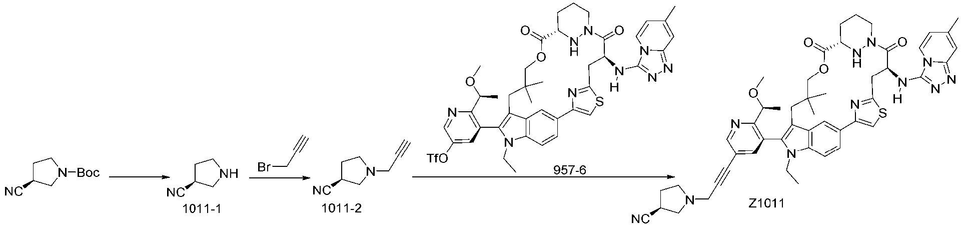

Step 1: tert-Butyl (S)-3-cyanopyrrolidine-1-carboxylate (680 mg, 3.465 mmol) was dissolved in trifluoroacetic acid (3 mL) and dichloromethane (3 mL). The reaction mixture was stirred at room temperature for 1 h. When the reaction was completed as detected by LCMS, the mixture was concentrated under reduced pressure to remove the solvent to give crude compound 1011-1 (68 mg, yield: 20%).

Step 2: 3-Bromopropyne (57 mg, 0.482 mmol) was slowly added dropwise to a solution of compound 1011-1 (58 mg, 0.603 mmol) and potassium carbonate (416 mg, 3.012 mmol) in acetonitrile (5 mL) under an ice-water bath. The reaction mixture was stirred at 0 °C for 1 h. The reaction was completed as detected by LCMS. The mixture was filtered to remove insoluble substances, and the filtrate was concentrated to dryness under reduced pressure. The residue was purified by automated flash chromatography on silica gel (ethyl acetate/petroleum ether = 0-80%) to give compound 1011-2 (72 mg, yield: 89%). ES-API: [M+H]⁺ = 135.3.

Step 3: Bis(triphenylphosphine)palladium(II) dichloride (9.2 mg, 0.013 mmol), copper(I) iodide (5 mg, 0.026 mmol), N,N-diisopropylethylamine (0.04 mL, 0.220 mmol), and lithium chloride (9.3 mg, 0.220 mmol) were added to a solution of compound 957-6 (40 mg, 0.044 mmol) and (S)-1-(prop-2-yn-1-yl)pyrrolidine-3-carbonitrile (18 mg, 0.132 mmol) in N,N-dimethylformamide (2 mL) under a nitrogen atmosphere. The reaction mixture was purged 3 times with nitrogen, heated to 80 °C, and stirred for 2 h. The reaction was completed as detected by LCMS. The reaction mixture was cooled to room temperature, and 20 mL of water was added to the reaction mixture. The resulting mixture was then extracted with ethyl acetate (10 mL × 3). The organic phases were combined, washed with saturated brine (10 mL × 3), dried over anhydrous sodium sulfate, filtered, and concentrated to dryness. The residue was purified by prep-HPLC (ammonium bicarbonate method) to give the desired compound (3S)-1-(3-(5-((6³S,4S,Z)-(Rₐ)-1¹-ethyl-10,10-dimethyl-4-((7-methyl-[1,2,4]triazolo[4,3-alpyridin-3-yl)amino)-5,7-dioxo-6¹,6²,6³,6⁴,6⁵,6⁶-hexahydro-1'H-8-oxa-2(4,2)-thiazola-1(5,3)-indola-6(1,3)-pyridazinacycloundecaphane-1²-yl)-6-((S)-1-methoxyethyl)pyridin-3-yl)prop-2-yn-1-yl)pyrrolidine-3-carbonitrile (Z1011, 10.17 mg, yield: 26%). ES-API [M+H]⁺ = 894.3. ^{I}H NMR (400 MHz, DMSO-d6) δ 8.81 (d, J= 2.0 Hz, 1 H), 8.53 (s, 1 H), 8.28- 8.26 (m, 1 H), 7.88 (d, J= 2.0 Hz, 1 H), 7.79 (s, 1 H), 7.75 (dd, J1= 1.6 Hz, J2= 8.8 Hz, 1 H), 7.57 (d, J= 8.8 Hz, 1 H), 7.22- 7.17 (m, 2 H), 6.71 (d, J= 7.2 Hz, 1 H), 5.53 (t, J= 9.6 Hz, 1 H), 5.21 (d, J= 12.4 Hz, 1 H), 4.36- 4.24 (m, 4 H), 4.12- 4.04 (m, 1 H), 3.83- 3.73 (m, 2 H), 3.62- 3.55 (m, 2 H), 3.51- 3.47 (m, 1 H), 3.25 (s, 3 H), 3.00- 2.80 (m, 5 H), 2.68-2.60 (m, 1 H), 2.41- 2.37 (m, 1 H), 2.30 (s, 3 H), 2.25- 2.18 (m, 1 H), 2.13- 2.11 (m, 1 H), 2.03- 1.93 (m, 1 H), 1.84- 1.80 (m, 2 H), 1.61- 1.51 (m, 1 H), 1.35 (d, J= 6.0 Hz, 3 H), 1.23 (s, 2 H), 0.94 (s, 3 H), 0.87 (t, J= 6.8 Hz, 3 H), 0.35 (s, 3 H).

### Example 153. Synthesis of Compound Z982

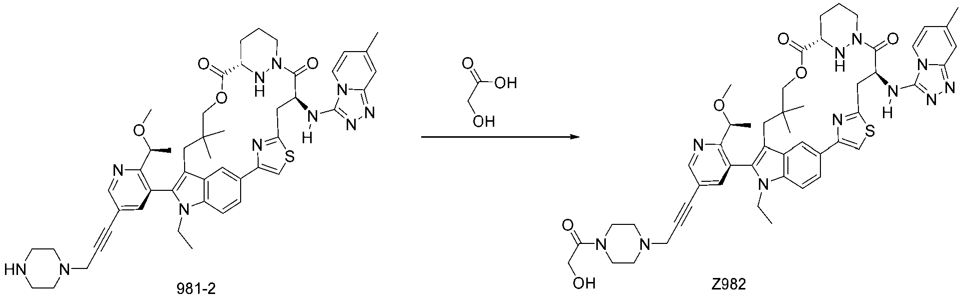

Step 1: N,N-Diisopropylethylenediamine (51.17 mg, 0.396 mmol), hydroxyacetic acid (12.04 mg, 0.158 mmol), and O-(7-azabenzotriazol-1-yl)-N,N,N',N'-tetramethyluronium hexafluorophosphate (30.11 mg, 0.079 mmol) were sequentially added to a solution of compound 981-2 (70 mg, 0.079 mmol) in dichloromethane (5 mL). The mixture was reacted at room temperature for 1 h. The mixture was then concentrated, and dichloromethane (20 mL) was added for dilution. The resulting mixture was washed sequentially with a saturated aqueous sodium bicarbonate solution (20 mL) and saturated brine (20 mL), dried over anhydrous sodium sulfate, filtered, and concentrated. The residue was purified by prep-HPLC (ammonium bicarbonate method) to give (6³S,4S,Z)-1¹-ethyl-1²-(5-(3-(4-(2-hydroxyacetyl)piperazin-1-yl)prop-1-yn-1-yl)-2-((S)-1-methoxyethyl)pyridin-3-yl)-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z982, 14 mg, yield: 18.77%), ES-API: [M+H]⁺ = 942.3.

### Example 154. Synthesis of Compound Z1016

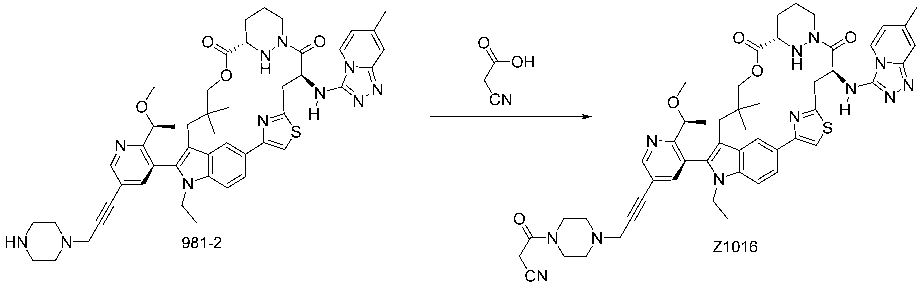

Step 1: N,N-Diisopropylethylenediamine (51.17 mg, 0.396 mmol), cyanoacetic acid (13.43 mg, 0.158 mmol), and O-(7-azabenzotriazol-1-yl)-N,N,N',N'-tetramethyluronium hexafluorophosphate (30.11 mg, 0.079 mmol) were sequentially added to a solution of compound 981-2 (70 mg, 0.079 mmol) in dichloromethane (5 mL). The mixture was reacted at room temperature for 1 h. The mixture was then concentrated, and dichloromethane (20 mL) was added for dilution. The resulting mixture was washed sequentially with a saturated aqueous sodium bicarbonate solution (20 mL) and saturated brine (20 mL), dried over anhydrous sodium sulfate, filtered, and concentrated. The crude product was purified by prep-HPLC (ammonium bicarbonate method) to give 3-(4-(3-(5-((6³S,4S,Z)-1¹-ethyl-10,10-dimethyl-4-((7-methyl-[1,2,4]triazolo[4,3-a]pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-((S)-1-methoxyethyl)pyridin-3-yl)prop-2-yn-1-yl)piperazin-1-yl)-3-oxopropanenitrile (Z1016, 18 mg, yield: 24%), ES-API: [M+H]⁺ = 951.3.

### Example 155. Synthesis of Compound Z1031

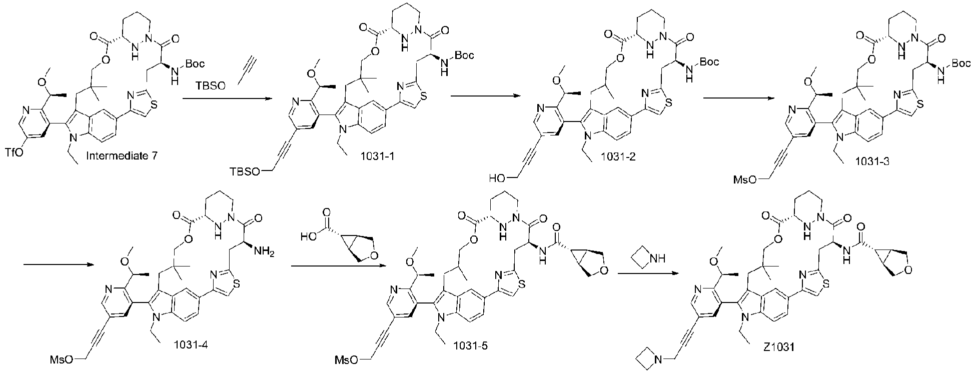

Step 1: Bis(triphenylphosphine)palladium(II) dichloride (1006 mg, 1.433 mmol), copper(I) iodide (546 mg, 2.867 mmol), N,N-diisopropylethylamine (3.3 mL, 19.113 mmol), and lithium chloride (1013 mg, 23.891 mmol) were added to a solution of compound intermediate 7 (4200 mg, 4.778 mmol) and tert-butyldimethyl(2-propynyloxy)silane (2442 mg, 14.335 mmol) in N,N-dimethylformamide (150 mL) under a nitrogen atmosphere. The reaction mixture was purged 3 times with nitrogen, heated to 80 °C, and stirred for 4 h. The reaction was completed as detected by LCMS. The reaction mixture was cooled to room temperature, and 500 mL of water was added to the reaction mixture. The resulting mixture was then extracted with ethyl acetate (100 mL × 3). The organic phases were combined, washed with saturated brine (100 mL × 3), dried over anhydrous sodium sulfate, filtered, and concentrated to dryness. The residue was purified by automated flash chromatography on silica gel (methanol/dichloromethane = 0-1.0%) to give compound 1031-1 (2600 mg, yield: 61%) as an off-white solid. ES-API: [M+H]⁺ = 898.4.

Step 2: A tetrabutylammonium fluoride solution (8.7 mL, 8.674 mmol) was added to a solution of compound 1031-1 (2600 mg, 2.891 mmol) in tetrahydrofuran (30 mL) under an ice-water bath. The reaction mixture was stirred at 0 °C for 1 h. When the reaction was completed as detected by LCMS, the reaction mixture was diluted with 100 mL of ethyl acetate and then washed with saturated brine (50 mL × 3). The organic phase was dried over anhydrous sodium sulfate, filtered, and concentrated to dryness to give crude compound 1031-2 (2269 mg, yield: 99%). ES-API: [M+H]⁺ = 785.3.

Step 3: N,N-Diisopropylethylamine (2.5 mL, 14.453 mmol) and methanesulfonic anhydride (755 mg, 4.336 mmol) were sequentially added to a solution of compound 1031-2 (2269 mg, 2.891 mmol) in dichloromethane (30 mL) under an ice-water bath. The reaction mixture was stirred at room temperature for 1 h. When the reaction was completed as detected by LCMS, the reaction mixture was concentrated to dryness under reduced pressure. The residue was purified by automated flash chromatography on silica gel (tetrahydrofuran/petroleum ether = 0-45%) to give compound 1031-3 (550 mg, purity: 50%). ES-API: [M+H]⁺ = 863.2.

Step 4: A hydrogen chloride/dioxane solution (10 mL, 4 M) was added to a solution of compound 1031-3 (550 mg, 0.319 mmol) in methanol (2 mL). The reaction mixture was stirred at room temperature for 1 h. When the reaction was completed as detected by LCMS, the mixture was concentrated under reduced pressure to remove most of the solvent to give crude compound 1031-4 (243 mg, yield: 99%). ES-API: [M+H]⁺ = 763.2.

Step 5: (1R,5S,6r)-3-Oxabicyclo[3.1.0]hexane-6-carboxylic acid (49 mg, 0.382 mmol), N,N-diisopropylethylamine (0.34 mL, 1.911 mmol), and O-(7-azabenzotriazol-1-yl)-N,N,N',N'-tetramethyluronium hexafluorophosphate (182 mg, 0.479 mmol) were sequentially added to a solution of compound 1031-4 (243 mg, 0.319 mmol) in N,N-dimethylformamide (5 mL) under an ice-water bath. The reaction mixture was warmed to room temperature and stirred for 10 min. When the reaction was completed as detected by LCMS, the reaction mixture was diluted with 30 mL of ethyl acetate and then washed with saturated brine (15 mL × 5). The organic phase was dried over anhydrous sodium sulfate, filtered, and concentrated under reduced pressure to give crude compound 1031-5 (278 mg, yield: 99%). ES-API: [M+H]⁺ = 873.3.

Step 6: Potassium carbonate (220 mg, 1.592 mmol) and azetidine (73 mg, 1.274 mmol) were sequentially added to a solution of compound 1031-5 (278 mg, 0.318 mmol) in acetonitrile (10 mL). The reaction mixture was heated to 50 °C and stirred for 1 h. When the reaction was completed as detected by LCMS, the mixture was filtered to remove insoluble substances, and the filtrate was concentrated to dryness. The residue was purified by automated flash chromatography on silica gel (methanol/dichloromethane = 0-5%) and then purified by prep-HPLC (ammonium bicarbonate method) to give (1R,5S,6r)-N-((6³S,4S,Z)-1²-(5-(3-(azetidin-1-yl)prop-1-yn-1-yl)-(Ra)-2-((S)-1-methoxyethyl)pyridin-3-yl)-1¹-ethyl-10,10-dimethyl-5,7-dioxo-6¹,6² 6³,6⁴6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z1031, 57 mg, yield: 21%) as a white solid. ES-API: [M+H]⁺ = 834.3. ¹H NMR (400 MHz, DMSO-D6) δ 8.81 (d, J= 2.0 Hz, 1 H), 8.57 (d, J= 8.8 Hz, 1 H), 8.50 (s, 1 H), 7.85 (d, J= 2.0 Hz, 1 H), 7.82 (s, 1 H), 7.76 (dd, J1= 1.6 Hz, J2= 8.8 Hz, 1 H), 7.58 (d, J= 8.4 Hz, 1 H), 5.56 (t, J= 9.2 Hz, 1 H), 5.09 (d, J= 12.4 Hz, 1 H), 4.36-4.05 (m, 5 H), 3.85- 3.81 (m, 2 H), 3.66- 3.62 (m, 2 H), 3.57 (s, 2 H), 3.47- 3.45 (m, 2 H), 3.25- 3.22 (m, 7 H), 3.15- 3.09 (m, 1 H), 2.97- 2.93 (m, 1 H), 2.79- 2.72 (m, 1 H), 2.41- 2.37 (m, 1 H), 2.10- 2.06 (m, 1 H), 2.00- 1.88 (m, 4 H), 1.83- 1.75 (m, 2 H), 1.66- 1.64 (m, 1 H), 1.56- 1.46 (m, 1 H), 1.37- 1.34 (m, 3 H), 1.25-1.21 (m, 1 H), 0.91- 0.86 (m, 6 H), 0.34 (s, 3 H).

### Example 156. Synthesis of Compound Z1030

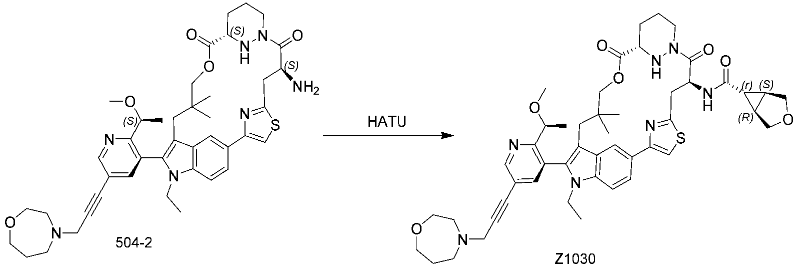

Step 1: Compound 504-2 (420 mg, 0.55 mmol) was dissolved in N,N-dimethylformamide (3 mL), and rac-(1R,5S,6r)-3-oxabicyclo[3.1.0]hexane-6-carboxylic acid (140 mg, 1.1 mmol), triethylamine (330 mg, 3.3 mmol), and O-(7-azabenzotriazol-1-yl)-N,N,N',N'-tetramethyluronium hexafluorophosphate (420 mg, 1.1 mmol) were sequentially added. The mixture was stirred at room temperature for 0.5 h. After the reaction was completed, 10 mL of ethyl acetate was added, and the mixture was washed with 10 mL of saturated brine and 10 mL of water. The organic phase was dried and concentrated, and then the residue was purified by a thin-layer chromatography column (dichloromethane/methanol = 20:1) to give compound (1R,5S,6r)-N-((6³S,4S,Z)-1²-(5-(3-(1,4-oxolan-4-yl)prop-1-yn-1-yl)-(Rₐ)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexanecarboxamide (Z1030, 300 mg, yield: 62%, purity: 98%). ES-API [M+H]⁺ = 878.3.

### Example 157. Synthesis of Compound Z979

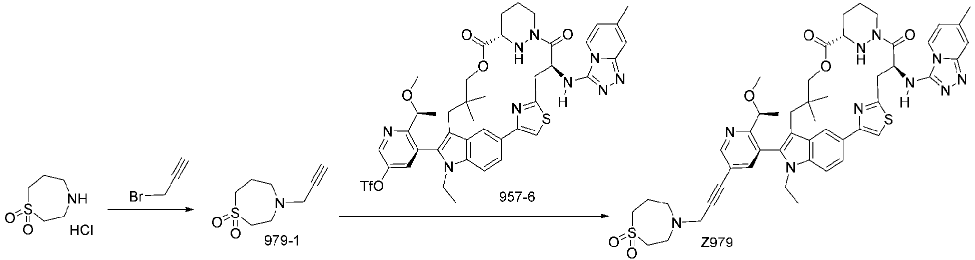

Step 1: 1,4-Thiazepane 1,1-dioxide hydrochloride (900 mg, 4.85 mmol) and potassium carbonate (2.0 g, 14.54 mmol) were suspended in acetonitrile (5 mL), and 3-bromopropyne (692 mg, 5.82 mmol) was added at 0 °C. The reaction system was stirred at room temperature for 18 h. The reaction mixture was filtered, and the filtrate was concentrated. The crude product was purified by a flash silica gel column (methanol/dichloromethane: 0-5%) to give compound 979-1 (480 mg, yield: 52.9%) as a pale yellow liquid. ES-API: [M+H]⁺ = 188.2.

Step 2: Compound 957-6 (50 mg, 0.055 mmol) was dissolved in N,N-dimethylformamide (3 mL), and 4-(prop-2-ynyl)-1¹6-1,4-thiazine-1,1-dione (42 mg, 0.22 mmol), bis(triphenylphosphine)palladium(II) dichloride (12 mg, 0.016 mmol), copper(I) iodide (6 mg, 0.031 mmol), N,N'-diisopropylethylamine (28 mg, 0.22 mmol), and lithium chloride (12 mg, 0.275 mmol) were added. The reaction system was stirred at 85 °C for 5 h under a nitrogen atmosphere. Water (20 mL) was added to the reaction mixture, and the mixture was extracted with ethyl acetate (30 mL × 2). The organic phase was washed with saturated brine (20 mL × 2), dried, and concentrated to give a crude product. The crude product was purified by prep-HPLC (ammonium bicarbonate method) to give the desired product ((6³S,4S,Z)-(Rₐ)-1²-(5-(3-(1,1-dioxido-1,4-thiazepan-4-yl)prop-1-yn-1-yl)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z979, 10 mg, yield: 19.2%) as a white solid. ¹H NMR (400 MHz, DMSO-d6) δ 8.82 (d, *J* = 2.0 Hz, 1H), 8.53 (d, *J =* 1.2 Hz, 1H), 8.26 (d, *J=* 7.2 Hz, 1H), 7.88 (d, *J* = 2.0 Hz, 1H), 7.79 (s, 1H), 7.75 (dd, *J =* 8.8, 1.6 Hz, 1H), 7.58 (d, *J =* 8.8 Hz, 1H), 7.22 (d, *J=* 1.2 Hz, 1H), 7.17 (d, *J* = 10.8 Hz, 1H), 6.71 (dd, *J =* 7.2, 12 Hz, 1H), 5.53 (t, *J =* 10.0 Hz, 1H), 5.21 (d, *J* = 12.0 Hz, 1H), 4.41 - 4.20 (m, 4H), 4.17 - 4.01 (m, 1H), 3.75 (s, 2H), 3.66 - 3.54 (m, 2H), 3.49 (d, *J =* 14.4 Hz, 1H), 3.31 - 3.20 (m, 8H), 3.05 - 2.94 (m, 3H), 2.91 (t, *J =* 6.0 Hz, 2H), 2.86 - 2.77 (m, 1H), 2.39 (d, *J =* 14.8 Hz, 1H), 2.30 (d, *J =* 0.8 Hz, 3H), 2.12 (d, *J* = 11.6 Hz, 1H), 2.03 - 1.94 (m, 2H), 1.88 - 1.75 (m, 2H), 1.64 - 1.50 (m, 1H), 1.36 (d, *J =* 6.0 Hz, 3H), 0.98 - 0.79 (m, 6H), 0.35 (s, 3H). ES-API: [M+H]⁺ = 947.3.

### Example 158. Synthesis of Compound Z1015

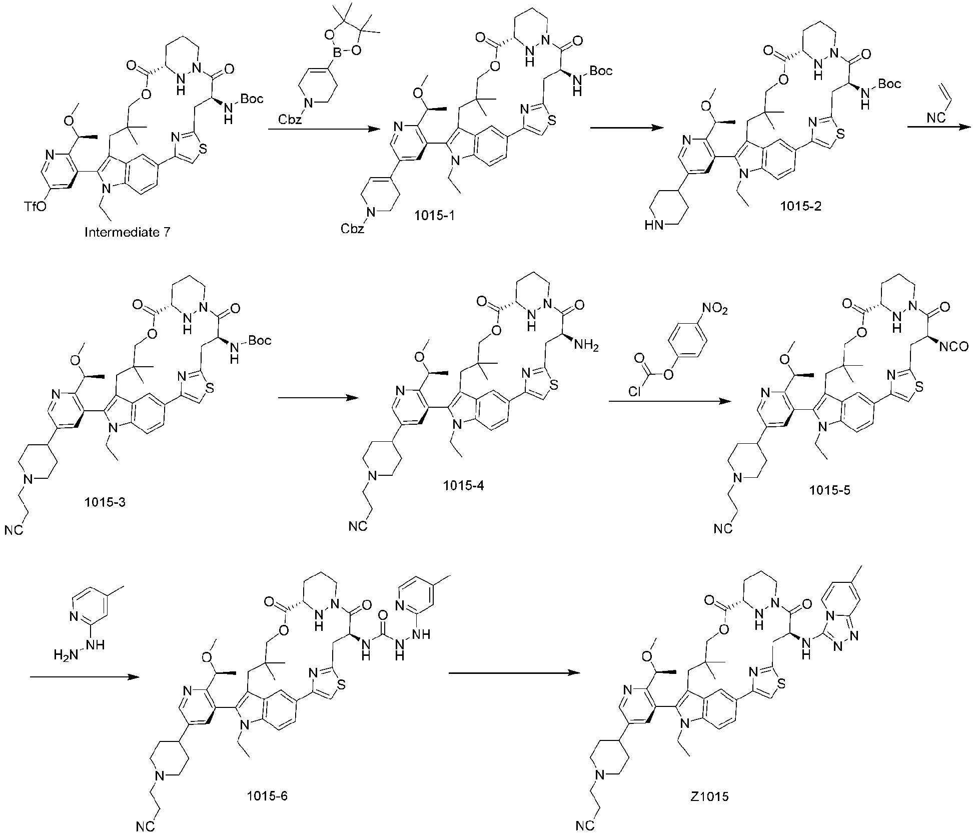

Step 1: Intermediate 7 (300 mg, 0.341 mmol) was dissolved in dioxane and water (1 mL). Benzyl 4-(4,4,5,5-tetramethyl-1,3,2-dioxaborolan-2-yl)-3,6-dihydropyridine-1(2H)-carboxylate (140.45 mg, 0.409 mmol), [1,1'-bis(diphenylphosphino)ferrocene]palladium(II) dichloride (24.97 mg, 0.034 mmol), and sodium carbonate (72.35 mg, 0.683 mmol) were added to the solution described above. After the addition was completed, the reaction system was stirred at 100 °C for 2 h under a nitrogen atmosphere. After the reaction was completed, water (20 mL) was added to the reaction mixture, and the mixture was extracted with ethyl acetate (20 mL × 2). The organic phase was washed with saturated brine (20 mL × 2), dried, and concentrated to give a crude product. The crude product was purified by a flash silica gel column (ethyl acetate/petroleum ether: 5%-70%) to give compound 1015-1 (300 mg, 0.317 mmol, yield: 92.90%) as a white solid. ES-API: [M+H]⁺ = 946.3.

Step 2: Compound 1015-1 (300 mg, 0.317 mmol) was dissolved in methanol (10 mL). Wet palladium on carbon (10% wt, 101.23 mg, 0.095 mmol) was added to the solution described above, and the mixture was purged three times with hydrogen. The reaction system was stirred at 40 °C for 17 h under a hydrogen atmosphere. After the reaction was completed, the reaction mixture was filtered through celite, and the filter cake was washed with methanol (100 mL). The mother liquor was concentrated to give compound 1015-2 (250 mg, 0.307 mmol, yield: 96.86%) as a white solid. ES-API: [M+H]⁺ = 814.4.

Step 3: Compound 1015-2 (200 mg, 0.246 mmol) was dissolved in ethanol (8 mL), and triethylamine (0.102 mL, 0.737 mmol) and acrylonitrile (26.07 mg, 0.491 mmol) were added to the solution described above. After the addition was completed, the reaction system was stirred at room temperature for 2 h. After the reaction was completed, the reaction mixture was concentrated to give a crude product. The crude product was purified by a flash silica gel column (methanol/dichloromethane: 0%-5%) to give compound 1015-3 (180 mg, 0.208 mmol, yield: 84.49%) as a white solid. ES-API: [M+H]⁺ = 867.4.

Step 4: Compound 1015-3 (180 mg, 0.208 mmol) was dissolved in dichloromethane (1 mL), and trifluoroacetic acid (1 mL) was added to the solution described above. After the addition was completed, the reaction system was stirred at room temperature for 1 h. After the reaction was completed, the reaction mixture was concentrated to give a trifluoroacetate. The trifluoroacetate described above was basified with a saturated sodium bicarbonate solution (10 mL) and extracted with ethyl acetate (10 mL × 3). The organic phase was washed with saturated brine (10 mL × 2), dried, and concentrated to give compound 1015-4 (130 mg, 0.169 mmol, yield: 81.65%) as a white solid. ES-API: [M+H]⁺ = 767.3.

Step 5: Compound 1015-4 (130 mg, 0.169 mmol) and N,N-diisopropylethylamine (109.53 mg, 0.847 mmol) were dissolved in dichloromethane (4 mL). Nitrophenyl chloroformate (44.41 mg, 0.220 mmol) was added at 0 °C, and the reaction system was stirred under an ice bath for 0.5 h. After the reaction was completed, compound 1015-5 was obtained. The reaction mixture of the crude product was directly used in the next step.

Step 6: N,N-diisopropylethylamine (63.57 mg, 0.492 mmol) and 2-hydrazineyl-4-methylpyridine (60.57 mg, 0.492 mmol) were added to the reaction mixture of the crude product of compound 1015-5 at 0 °C. After the addition was completed, the reaction system was stirred at 0 °C for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-6%) to give compound 1015-6 (100 mg, 0.109 mmol, yield: 66.58%) as a light yellow solid. ES-API: [M+H]⁺ = 916.3.

Step 7: Compound 1015-6 (100 mg, 0.109 mmol) was dissolved in 1,2-dichloroethane (5 mL) and pyridine (0.132 mL, 1.637 mmol). Phosphorus oxychloride (0.030 mL, 0.327 mmol) was added to the solution described above. After the addition was completed, the reaction system was stirred at 45 °C for 5 min. The reaction mixture was cooled to room temperature and concentrated. The crude product was purified by a flash silica gel column (methanol/dichloromethane: 0-7%) to give a crude product. The crude product was purified by prep-HPLC (ammonium bicarbonate method) to give 3-(4-(5-((6³S,4S,Z)-1¹-ethyl-10,10-dimethyl-4-((7-methyl-[1,2,4]triazolo[4,3-a])pyridin-3-yl)amino)-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-(Rₐ)-((S)-1-methoxyethyl)pyridin-3-yl)piperidin-1-yl)propanenitrile (Z1015, 29 mg, 0.032 mmol, yield: 29.58%) as a light yellow solid. ES-API: [M+H]⁺ = 898.3. ¹H NMR (300 MHz, CD₃OD) δ 8.69 - 8.62 (m, 2H), 8.14 (d, *J* = 7.2 Hz, 1H), 7.80 - 7.69 (m, 2H), 7.58 - 7.48 (m, 2H), 7.22 (d, *J =* 1.2 Hz, 1H), 6.74 (dd, *J* = 7.2, 1.2 Hz, 1H), 5.66 (d, *J =* 8.4 Hz, 1H), 4.56 - 4.25 (m, 4H), 4.25 - 4.09 (m, 1H), 3.78 (s, 2H), 3.68 - 3.58 (m, 1H), 3.51 - 3.39 (m, 1H), 3.38 (s, 3H), 3.21 - 3.07 (m, 3H), 2.91 - 2.74 (m, 4H), 2.74 - 2.67 (m, 2H), 2.67 - 2.57 (m, 1H), 2.41 (s, 3H), 2.38 - 2.23 (m, 3H), 2.07 - 1.80 (m, 6H), 1.75 - 1.60 (m, 1H), 1.47 (d, *J* = 6.3 Hz, 3H), 1.03 - 0.91 (m, 6H), 0.50 (s, 3H).

### Example 159. Synthesis of Compound Z1029

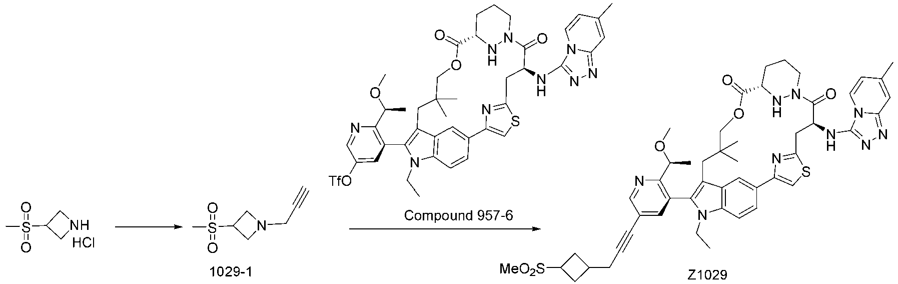

Step 1: Sodium hydride (0.59 g, 14.796 mmol) was added to a solution of 3-(methylsulfonyl)azetidine hydrochloride (0.50 g, 3.699 mmol) in N,N-dimethylformamide (10.0 mL) at 0 °C. The mixture was stirred for 0.5 h. 3-Bromoprop-1-yne (0.88 g, 7.394 mmol) was then added to the solution, and the mixture was stirred at 25 °C for 18 h. After the reaction was completed, the cooled solution was extracted with ethyl acetate (2 × 100 mL) and saturated brine (100 mL). The ethyl acetate phase was dried over anhydrous sodium sulfate and filtered. The filtrate was concentrated, and then the residue was purified by flash silica gel column chromatography [dichloromethane: methanol = 100:0 to 90:10, (v/v)] to give compound 1029-1 (345 mg, yield: 53.84%). ES-API: [M+H]⁺ = 174.1.

Step 2: Compound 957-6 (120.0 mg, 0.132 mmol) was added to compound 1029-1 (120.0 mg, 0.862 mmol) in N,N-dimethylformamide (3.0 mL), and bis(triphenylphosphine)palladium(II) dichloride (59.89 mg, 0.085 mmol), copper(I) iodide (150.00 mg, 0.866 mmol), N,N-diisopropylethylamine (0.092 mL, 0.527 mmol), and lithium chloride (28.0 mg, 0.660 mmol) were added. After the addition, the reaction mixture was stirred at 80 °C for 1.5 h under a nitrogen atmosphere. The reaction mixture was then diluted with water (80 mL) and extracted with ethyl acetate (30 mL × 3). The organic layers were combined, washed with brine, dried over anhydrous sodium sulfate, filtered, and concentrated. The residue was purified by prep-HPLC (ammonium bicarbonate method) to give (6³S,4S,Z)-1¹-ethyl-1²-(2-((S)-1-methoxyethyl)-5-(3-(3-(methylsulfonyl)cyclobutyl)prop-1-yn-1-yl)pyridin-3-yl)-10,10-dimethyl-4-((7-methyl-[1,2,4]triazolo[4,3-a]pyridin-3-yl)amino)-6¹,6²,6³,6⁴,6⁵ ,6⁶-hexahydro-1¹H-8-oxa-2(4,2)-thiazola-1(5,3)-indola-6(1,3)-pyridazinacycloundecaphane-5,7-dione (Z1029, 20.0 mg, yield: 16.24%). ES-API: [M+H]⁺ = 933.2. ¹H NMR (400 MHz, DMSO-d6) δ 8.88-8.86 (m, 1H), 8.53 (s, 1H), 8.28-8.26 (m, 1H), 7.93-7.91(m, 1H), 7.81 - 7.70 (m, 2H), 7.59-7.57(m, 1H), 7.26 - 7.14 (m, 2H), 6.70-6.72 (m, 1H), 5.56-5.51 (m, 1H), 5.22~5.18 (m, 1H), 4.37-4.06 (m, 7H), 3.69 - 3.45 (m, 10H), 3.28-3.25(m, 4H), 2.96∼2.94 (m, 5H), 2.46∼2.42 (m, 1H), 2.30 (s, 3H), 2.14-2.12(m, 1H), 1.84∼1.82 (m, 2H), 1.65 - 1.50 (m, 1H), 1.37∼1.34 (m, 3H), 0.95∼0.86 (m, 6H), 0.35 (s, 3H).

### Example 160. Synthesis of Compound Z981

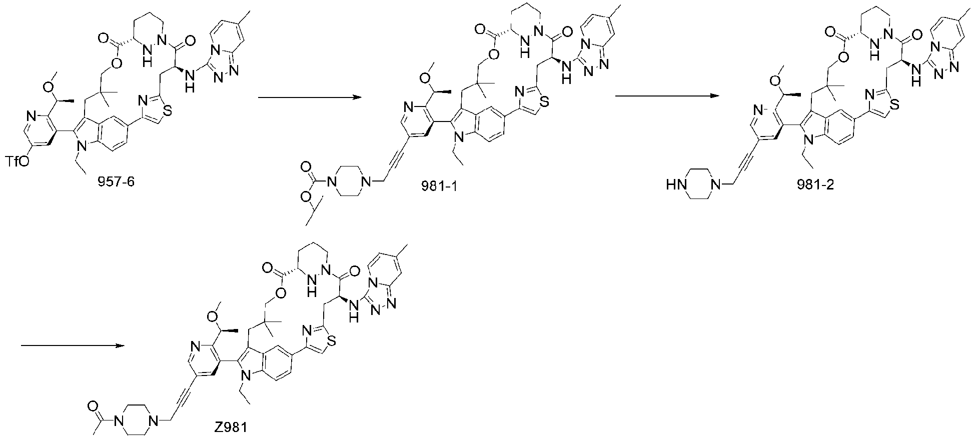

Step 1: Compound 957-6 (300 mg, 0.33 mmol), 2-methylprop-2-yl-4-(prop-2-ynyl)piperazine-1-carboxylate (147 mg, 0.65 mmol), bis(triphenylphosphine)palladium(II) dichloride (46 mg, 0.06 mmol), copper(I) iodide (20 mg, 0.1 mmol), and N,N-diisopropylethylenediamine (170 mg, 1.3 mmol) were mixed in N,N-dimethylformamide (10 mL) under a nitrogen atmosphere. The mixture was stirred at 80 °C for 10 h. After the reaction was completed, the mixture was poured into 30 mL of ethyl acetate and washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 981-1 (280 mg, yield: 86%). ES-API [M+H]⁺ = 984.3.

Step 2: Trifluoroacetic acid (3 mL) was added to a solution of compound 981-1 (210 mg, 0.21 mmol) in dichloromethane (5 mL). The mixture was stirred at room temperature for 2 h. After the reaction was completed, the mixture was basified with a saturated sodium bicarbonate solution to pH 8, extracted three times with 20 mL of ethyl acetate, and concentrated to give compound 981-2 (280 mg, yield: 67%). ES-API [M+H]⁺ = 884.3.

Step 3: Hydroxyacetic acid (12 mg, 0.2 mmol) and N,N-diisopropylethylenediamine (71 mg, 0.5 mmol) were added to a solution of compound 981-2 (60 mg, 0.07 mmol) in N,N-dimethylformamide (5 mL), and O-(7-azabenzotriazol-1-yl)-N,N,N',N'-tetramethyluronium hexafluorophosphate (51 mg, 0.14 mmol) was added under an ice bath. The mixture was stirred at room temperature for 1 h. After the reaction was completed, the mixture was extracted with ethyl acetate (3 × 20 mL), and the combined extract was washed with brine (30 mL). The organic phase was dried and concentrated, and then the residue was purified by preparative thin-layer chromatography (dichloromethane/methanol = 20/1) to give (6³S,4S,Z)-1²-(5-(3-(4-acetylpiperazin-1-yl)prop-1-yn-1-yl)-(Rₐ)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z981, 20 mg, yield: 31%, purity: 98%). ES-API [M+H]⁺ = 926.3.

### Example 161. Synthesis of Compound Z999

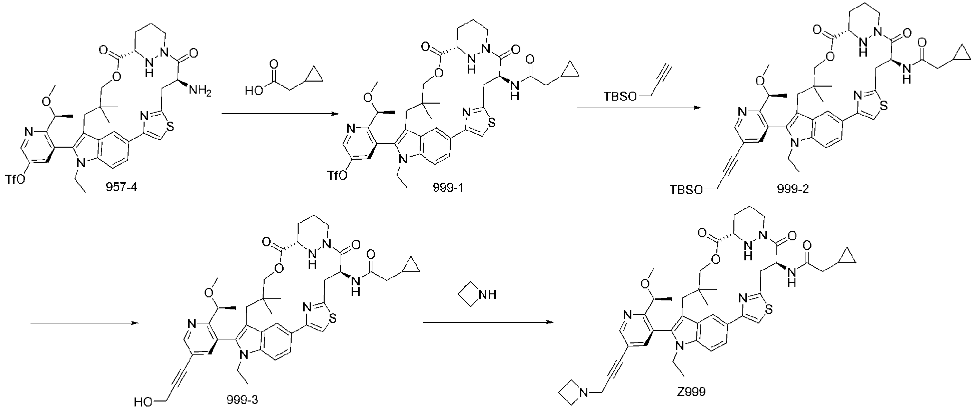

Step 1: N,N-Diisopropylethylamine (735.23 mg, 5.688 mmol), cyclopropylacetic acid (170.86 mg, 1.707 mmol), and O-(7-azabenzotriazol-1-yl)-N,N,N',N'-tetramethyluronium hexafluorophosphate (865.19 mg, 2.275 mmol) were sequentially added to a solution of compound 957-4 (886.10 mg, 1.138 mmol) in dichloromethane (15 mL) under an ice-water bath. The mixture was reacted at room temperature for 1 h. The mixture was then concentrated, and dichloromethane (20 mL) was added for dilution. The resulting mixture was washed sequentially with a saturated aqueous sodium bicarbonate solution (20 mL) and saturated brine (20 mL), dried over anhydrous sodium sulfate, filtered, and concentrated. The crude product was purified by automated flash chromatography on silica gel (methanol/dichloromethane = 0-6%) to give compound 999-1 (0.93 g, 1.081 mmol, yield: 95%). ES-API: [M+H]⁺ = 861.3.

Step 2: Compound 999-1 (0.93 g, 1.081 mmol) and tert-butyldimethyl(2-propynyloxy)silane (735.95 mg, 4.321 mmol) were dissolved in N,N-dimethylformamide (10 mL), and bis(triphenylphosphine)palladium(II) dichloride (227.45 mg, 0.324 mmol), copper(I) iodide (123.43 mg, 0.648 mmol), N,N-diisopropylethylenediamine (1.129 mL, 6.481 mmol), and lithium chloride (183.15 mg, 4.321 mmol) were sequentially added. The mixture was heated to 80 °C and reacted for 3 h under a nitrogen atmosphere, then diluted with ethyl acetate (20 mL), washed sequentially with water (20 mL) and saturated brine (20 mL), dried over anhydrous sodium sulfate, filtered, and concentrated. The residue was purified by silica gel column chromatography (methanol/dichloromethane = 0-6%) to give compound 999-2 (730 mg, 0.828 mmol, yield: 76.69%). ES-API: [M+H]⁺ = 881.4.

Step 3: Compound 999-2 (730 mg, 0.828 mmol) was dissolved in tetrabutylammonium fluoride (10 mL, 0.828 mmol, a 1 M solution in tetrahydrofuran). The mixture was reacted for 1 h, diluted with ethyl acetate (20 mL), washed sequentially with water (20 mL × 3) and saturated brine (20 mL), dried over anhydrous sodium sulfate, filtered, and concentrated to give crude compound 999-3 (570 mg, 0.743 mmol, yield: 89.71%). ES-API: [M+H]⁺ = 767.4.

Step 4: N,N-Diisopropylethylenediamine (480.16 mg, 3.715 mmol) and methanesulfonic anhydride (258.56 mg, 1.486 mmol) were added to a solution of compound 999-3 (570 mg, 0.743 mmol) in dichloromethane (10 mL) under an ice-water bath, and the reaction system was stirred at room temperature for 1 h. After the starting materials were consumed, azetidine (202.71 mg, 3.550 mmol) was added, and the mixture was reacted at room temperature for another 3 h. After the reaction was completed, dichloromethane (30 mL) was added, and the mixture was washed sequentially with water (50 mL × 3) and saturated brine (50 mL), dried over anhydrous sodium sulfate, filtered, and concentrated. The residue was purified by silica gel column chromatography (methanol/dichloromethane = 0-5%) to give the product N-((6³S,4S,Z)-1²-(5-(3-(azetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-cyclopropylacetamide (Z999, 330 mg, yield: 57.66%), ES-API: [M+H]⁺ = 806.4. ¹H NMR (400 MHz, DMSO-d6) δ 8.82 (d, J = 2.1 Hz, 1H), 8.51 (d, J = 1.6 Hz, 1H), 8.25 (d, J = 9.0 Hz, 1H),7.85 (d, J = 2.2 Hz, 1H), 7.83 (s, 1H), 7.76 (dd, J = 8.6, 1.6 Hz, 1H), 7.59 (d, J = 8.7 Hz, 1H),5.56 (t, J = 9.1 Hz, 1H), 5.11 (d, J = 12.2 Hz, 1H), 4.38 - 4.19 (m, 4H), 4.15 - 4.06 (m, 1H), 3.58 (s, 2H),3.46 (s, 2H), 3.36 - 3.30 (m, 3H), 3.24 (d, J = 7.0 Hz, 3H), 3.19 - 3.12 (m, 1H), 2.99 (d, J = 14.3 Hz, 1H),2.81 - 2.72 (m, 1H), 2.38 (d, J = 14.4 Hz, 1H), 2.10 (d, J = 7.1 Hz, 3H), 1.99 - 1.95 (m, 2H), 1.80 (s, 2H),1.53 (q, J = 10.5, 8.2 Hz, 1H), 1.36 (d, J = 6.0 Hz, 3H), 1.24 (d, J = 6.1 Hz, 1H), 1.01 (td, J = 8.0, 3.6 Hz, 1H),0.93 (d, J = 4.3 Hz, 3H), 0.88 (t, J = 7.1 Hz, 4H), 0.46 (dq, J = 8.3, 1.5 Hz, 2H), 0.34 (s, 3H), 0.27 - 0.16 (m, 2H).

### Example 162. Synthesis of Compound Z1027

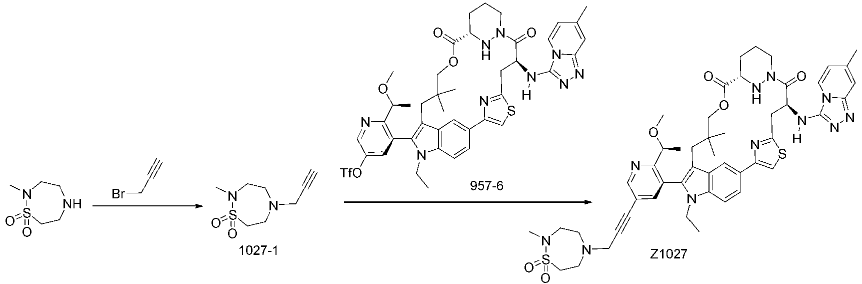

Step 1: 3-Bromopropyne (189 mg, 1.588 mmol) was slowly added dropwise to a solution of 2-methyl-1,2,5-thiadiazepane 1,1-dioxide (326 mg, 1.985 mmol) and potassium carbonate (1372 mg, 9.926 mmol) in acetonitrile (10 mL) under an ice-water bath. The reaction mixture was stirred at 0 °C for 1 h. The reaction was completed as detected by LCMS. The mixture was filtered to remove insoluble substances, and the filtrate was concentrated to dryness under reduced pressure. The residue was purified by automated flash chromatography on silica gel (ethyl acetate/petroleum ether = 0-80%) to give compound 1027-1 (72 mg, yield: 18%). ES-API: [M+H]⁺ = 203.2.

Step 2: Bis(triphenylphosphine)palladium(II) dichloride (11.6 mg, 0.016 mmol), copper(I) iodide (6.3 mg, 0.033 mmol), N,N-diisopropylethylamine (0.04 mL, 0.220 mmol), and lithium chloride (11.7 mg, 0.275 mmol) were added to a solution of compound 957-6 (50 mg, 0.044 mmol) and compound 1027-1 (33 mg, 0.165 mmol) in N,N-dimethylformamide (2 mL) under a nitrogen atmosphere. The reaction mixture was purged 3 times with nitrogen, heated to 80 °C, and stirred for 2 h. The reaction was completed as detected by LCMS. The reaction mixture was cooled to room temperature, and 20 mL of water was added to the reaction mixture. The resulting mixture was then extracted with ethyl acetate (10 mL × 3). The organic phases were combined, washed with saturated brine (10 mL × 3), dried over anhydrous sodium sulfate, filtered, and concentrated to dryness. The residue was purified by prep-HPLC (ammonium bicarbonate method) to give the desired compound (6³S,4S,7)-1¹-ethyl-1²-(Ra)-(2-((S)-1-methoxyethyl)-5-(3-(2-methyl-1,1-dioxido-1,2,5-thiadiazepan-5-yl)prop-1-yn-1-yl)pyridin-3-yl)-10,10-dimethyl-4-((7-methyl-[1,2,4]triazolo[4,3-a]pyridin-3-yl)amino)-6¹,6²,6³,6⁴,6⁵ 6⁶-hexahydro-1¹H-8-oxa-2(4,2)-thiazola-1(5,3)-indola-6(1,3)-pyridazinacycloundecaphane-5,7-dione (Z1027, 14 mg, yield: 26%). ES-API [M+H]⁺ = 962.3. ¹H NMR (400 MHz, DMSO-d6) δ 8.81 (d, J= 2.0 Hz, 1 H), 8.53 (s, 1 H), 8.41- 8.35 (m, 1 H), 7.87 (d, J= 2.0 Hz, 1 H), 7.80 (s, 1 H), 7.75 (dd, J1= 1.2 Hz, J2= 8.8 Hz, 1 H), 7.58 (d, J= 8.8 Hz, 1 H), 7.44- 7.26 (m, 3 H), 6.81 (d, J= 7.2 Hz, 1 H), 5.53 (t, J= 10.0 Hz, 1 H), 5.22 (d, J= 11.2 Hz, 1 H), 4.36- 4.24 (m, 4 H), 4.11- 4.05 (m, 1 H), 3.83- 3.73 (m, 2 H), 3.63- 3.48 (m, 4 H), 3.25 (s, 3 H), 3.08- 2.92 (m, 6 H), 2.85- 2.76 (m, 2 H), 2.41- 2.33 (m, 1 H), 2.33 (s, 3 H), 2.13- 2.11 (m, 1 H), 1.84- 1.82 (m, 2 H), 1.59- 1.55 (m, 1 H), 1.36- 1.35 (m, 3 H), 1.23- 1.21 (m, 4 H), 0.94 (s, 3 H), 0.88 (t, J= 6.8 Hz, 3 H), 0.35 (s, 3 H).

### Example 163. Synthesis of Compound Z1014

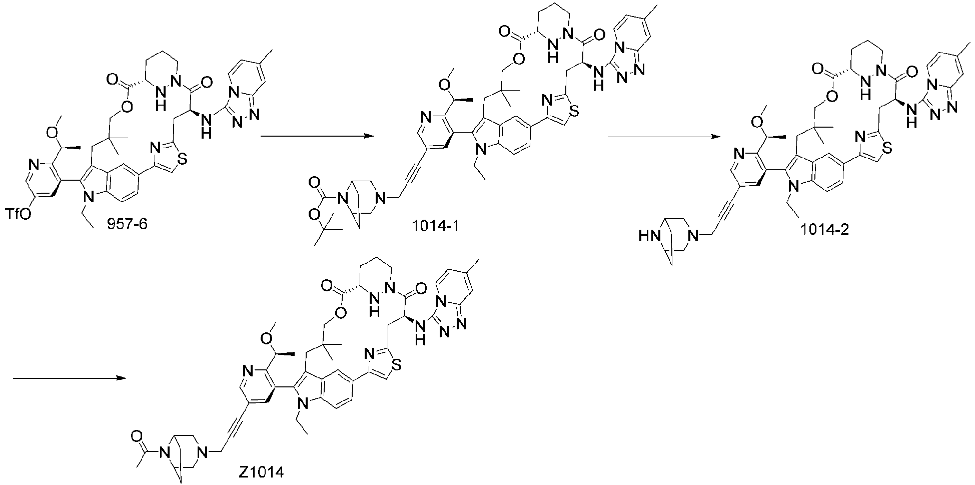

Step 1: Compound 957-6 (50 mg, 0.2 mmol), bis(triphenylphosphine)palladium(II) dichloride (14 mg, 0.02 mmol), copper(I) iodide (8 mg, 0.04 mol), and N,N-diisopropylethylenediamine (50 mg, 0.4 mol) were mixed in N,N-dimethylformamide (10 mL) under a nitrogen atmosphere. The mixture was stirred at 80 °C for 10 h. After the reaction was completed, the mixture was poured into 30 mL of ethyl acetate and washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 1014-1 (70 mg, yield: 70%), ES-API [M+H]⁺ = 1010.5.

Step 2: Trifluoroacetic acid (3 mL) was added to a solution of compound 1014-1 (70 mg, 0.07 mmol) in dichloromethane (5 mL), and the mixture was stirred at room temperature for 2 h. After the reaction was completed, the mixture was concentrated, basified with a saturated sodium bicarbonate solution to pH 8, extracted three times with 20 mL of ethyl acetate, and concentrated to give compound 1014-2 (62 mg, yield: 99%), ES-API [M+H]⁺ = 884.3.

Step 3: Hydroxyacetic acid (7 mg, 0.12 mmol) and N,N-diisopropylethylenediamine (46 mg, 0.12 mol) were added to a solution of compound 1014-2 (54 mg, 0.06 mmol) in N,N-dimethylformamide (5 mL), and O-(7-azabenzotriazol-1-yl)-N,N,N',N'-tetramethyluronium hexafluorophosphate (45 mg, 0.12 mmol) was added under an ice bath. The mixture was stirred at room temperature for 1 h. After the reaction was completed, the mixture was extracted with ethyl acetate (20 mL × 3), and the combined extract was washed with brine (30 mL). The organic phase was dried and concentrated, and then the residue was purified by preparative thin-layer chromatography (dichloromethane/methanol = 20/1) to give (6³S,4S,Z)-1²-(5-(3-(8-acetyl-3,8-diazabicyclo[3.2.1]octan-3-yl)prop-1-yn-1-yl)-(Rₐ)-2-((S)-1-methoxyethyl)pyridin-3-yl)-1¹-ethyl-10,10-dimethyl-4-((7-methyl-[1,2,4]triazolo[4,3-a]pyridin-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z1014, 20 mg, yield: 31%, purity: 98%). ES-API [M+H]⁺ = 926.3.

### Example 164. Synthesis of Compound Z951

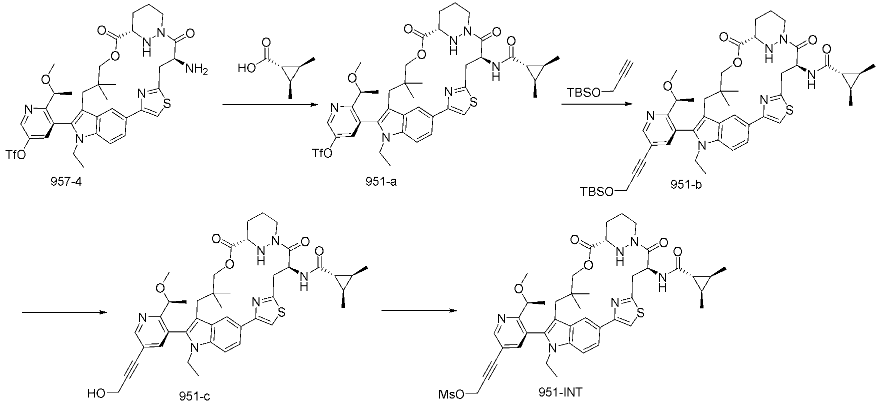

Step 1: Compound 957-4 (5700 mg, 7.318 mmol) was dissolved in N,N-dimethylformamide (10 mL). (1R,2R,3S)-2,3-Dimethylcyclopropane-1-carboxylic acid (1085.92 mg, 9.514 mmol), O-(7-azabenzotriazol-1-yl)-N,N,N',N'-tetramethyluronium hexafluorophosphate (1416.11 mg, 10.978 mmol), and N,N'-diisopropylethylamine (945.90 mg, 7.318 mmol) were added to the solution described above.After the addition was completed, the reaction system was stirred at room temperature for 2 h. After the reaction was completed, water (50 mL) was added, and the mixture was extracted with ethyl acetate (35 mL × 2). The organic phase was washed with saturated brine (20 mL × 3), dried, and concentrated to give a crude product. The crude product was purified by a flash silica gel column (methanol/dichloromethane: 0%-10%) to give compound 951-a (5650 mg, 6.457 mmol, yield: 88.23%) as a white solid, ES-API: [M+H]⁺ = 875.2.

Step 2: Compound 951-a (5650 mg, 6.457 mmol) and tert-butyldimethyl(2-propynyloxy)silane (2199.72 mg, 12.914 mmol) were dissolved in acetonitrile (50 mL), and bis(triphenylphosphine)palladium(II) dichloride (1810.60 mg, 2.583 mmol), copper(I) iodide (743.87 mg, 3.874 mmol), N,N'-diisopropylethylamine (3331.92 mg, 25.829 mmol), and anhydrous lithium chloride (1084.81 mg, 25.829 mmol) were added. The mixture was reacted at 80 °C for 5 h under a nitrogen atmosphere. After the reaction was completed, the mixture was filtered under vacuum and concentrated under reduced pressure to give a crude product. The crude product was purified by a flash silica gel column (40 g, methanol/dichloromethane: 0%-10%) to give compound 951-b (4550 mg, 5.082 mmol, 78.71%) as a brown solid, ES-API: [M+H]⁺ = 895.3.

Step 3: Compound 951-b (4550 mg, 5.082 mmol) was dissolved in tetrahydrofuran (50 mL), and a 1 M tetrabutylammonium fluoride solution (12 mL) was added. The mixture was reacted at room temperature for 2 h under a nitrogen atmosphere. After the reaction was completed as monitored by LC-MS, water (50 mL) was added, and the mixture was extracted with ethyl acetate (35 mL × 2). The organic phase was washed with saturated brine (20 mL × 3), dried, and concentrated to give a crude product. The crude product was purified by a flash silica gel column (methanol/dichloromethane: 0%-10%) to give compound 951-c (3900 mg, 4.994 mmol, 98.25%, purity: 85%) as a brown solid, ES-API: [M+H]⁺ = 781.3.

Step 4: Compound 951-c (3900 mg, 4.994 mmol, purity: 85%) was dissolved in dichloromethane (50 mL), and methanesulfonic anhydride (1042.68 mg, 5.992 mmol) was added. N,N'-Diisopropylethylamine (2581.72 mg, 19.975 mmol) was then added under an ice-water bath. The mixture was reacted at room temperature for 1 h under a nitrogen atmosphere. After the starting materials were consumed as monitored by LC-MS, the mixture was concentrated to dryness under reduced pressure to give compound 951-INT (3600 mg, 4.191 mmol, yield: 98.73%), ES-API: [M+H]⁺ = 859.3.

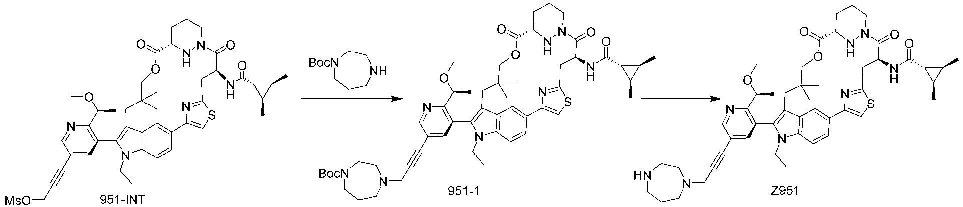

Step 1: Compound 951-INT (0.15 g, 0.175 mmol) and N,N'-diisopropylethylamine (0.11 g, 0.875 mmol) were dissolved in dichloromethane (20 mL), and 2-methylpropan-2-yl 1,4-diazepine-1-carboxylate (0.19 g, 0.931 mmol) was added. The reaction system was stirred at room temperature for 5 h. The mixture was concentrated under reduced pressure, and the crude product was purified by a flash silica gel column (methanol/dichloromethane: 0-5%) to give compound 951-1 (150 mg, 0.156 mmol, yield: 89.18%). ES-API: [M+H]⁺ = 963.2.

Step 2: Compound 951-1 (120 mg, 0.125 mmol) was dissolved in methanol (0.5 mL), and a solution of hydrogen chloride in dioxane (4 M, 1 mL) was added to the solution described above. The mixture was stirred at room temperature for 2 h. After the reaction was completed, the reaction system was concentrated to give a crude product, which was purified by prep-HPLC (formic acid method 1) to give (1r,2R,3S)-N-((6³S,4S,Z)-(Rₐ)-12-(5-(3-(1,4-oxazepan-4-yl)propyn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z951, 60 mg, 0.063 mmol, yield: 50.42%) as a white solid, ES-API: [M+H]⁺ =863.4.

### Example 165. Synthesis of Compound Z952

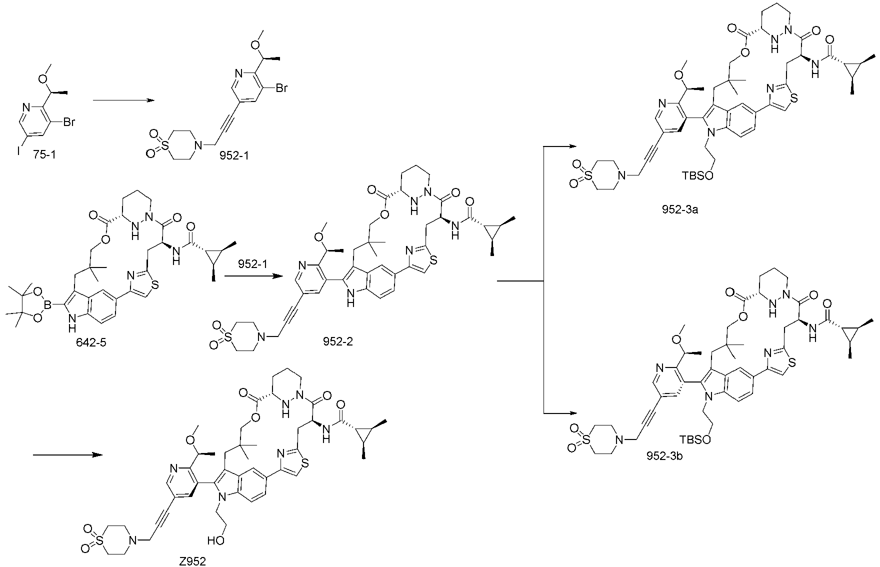

Step 1: compound 75-1 (340 mg, 1 mmol) and 4-(prop-2-ynyl)-1^{λ}6-1,4-thiazine-1,1-dione (345 mg, 2 mmol) were dissolved in acetonitrile (5 mL), and bis(triphenylphosphine)palladium(II) dichloride (140 mg, 0.2 mmol), copper(I) iodide (75 mg, 0.394 mmol), N,N-diisopropylethylamine (0.5 mL, 2.98 mmol), and anhydrous lithium chloride (170 mg, 4.010 mmol) were added. The mixture was stirred at 80 °C for 2 h under a nitrogen atmosphere, then filtered under vacuum, and concentrated to dryness under reduced pressure. The crude product was purified by a flash silica gel column (ethyl acetate/petroleum ether: 0%-50%) to give compound 952-1 (250 mg, 0.646 mmol, yield: 64.93%) as a yellow solid. ES-API: [M+H]⁺ = 387.0/389.0. Step 2: Compound 642-5 (230 mg, 0.333 mmol), compound 952-1 (200 mg, 0.516 mmol), [1,1'-bis(di-tert-butylphosphino)ferrocene]dichloropalladium(II) (25 mg, 0.038 mmol), and potassium phosphate (220 mg, 1.036 mmol) were dissolved in toluene (3 mL), 1,4-dioxane (1 mL), and water (1 mL). The reaction system was stirred at 70 °C for 1 h under a nitrogen atmosphere. After the reaction was completed, water (30 mL) was added to the solution described above, and the mixture was extracted with ethyl acetate (20 mL × 2). The organic phase was washed with saturated brine (50 mL), dried, and concentrated to give a crude product. The crude product was purified by a flash silica gel column (methanol/dichloromethane: 0%-3%) to give compound 952-2 (200 mg, 0.230 mmol, yield: 68.93%) as a yellow solid. ES-API: [M+H]⁺ = 870.3.

Step 3: Compound 952-2 (100 mg, 0.115 mmol) was dissolved in N,N-dimethylformamide (3 mL), and anhydrous cesium carbonate (112.34 mg, 0.345 mmol) was added. The mixture was stirred at room temperature for 1 h, and then tert-butyl(2-iodoethoxy)dimethylsilane (98.69 mg, 0.345 mmol) was added. The reaction system was stirred at 50 °C for 4 h under a nitrogen atmosphere. After the reaction was completed, water (10 mL) was added to the reaction mixture, and the mixture was extracted with ethyl acetate (10 mL × 2). The organic phase was washed with saturated brine (10 mL × 2), dried, and concentrated to give a crude product. The crude product was purified by a thin-layer chromatography column (methanol/dichloromethane = 1:15) to give 2 isomeric compounds. The structure of one of the isomeric compounds was arbitrarily designated as compound 952-3a (40 mg, 0.039 mmol, yield: 33.84%, Rf = 0.45) as a yellow solid. ES-API: [M+H]⁺ = 1028.1.

The structure of the other isomeric compound was arbitrarily designated as compound 952-3b (60 mg, 0.06 mmol, yield: 50.7%, Rf = 0.4) as a yellow solid. ES-API: [M+H]⁺ = 1028.1.

Step 4: Compound 952-3a (19 mg, 0.018 mmol) was dissolved in tetrahydrofuran (3 mL), and a 4 M solution of hydrochloric acid in dioxane (1 mL, 0.018 mmol) was added under an ice-water bath. The mixture was stirred at room temperature for 1 h under a nitrogen atmosphere. After the reaction was completed, the mixture was concentrated under reduced pressure, and the residue was redissolved in dichloromethane, adjusted to pH 8 with a 7 M solution of ammonia water/methanol, and concentrated under reduced pressure. The residue was purified by a flash silica gel column (methanol/dichloromethane: 0%-3%) to give (1r,2R,3S)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-(2-hydroxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z952, 10 mg, 0.011 mmol, yield: 59.21%) as a white solid, ES-API: [M+H]⁺ = 914.3.

### Example 166. Synthesis of Compound Z850

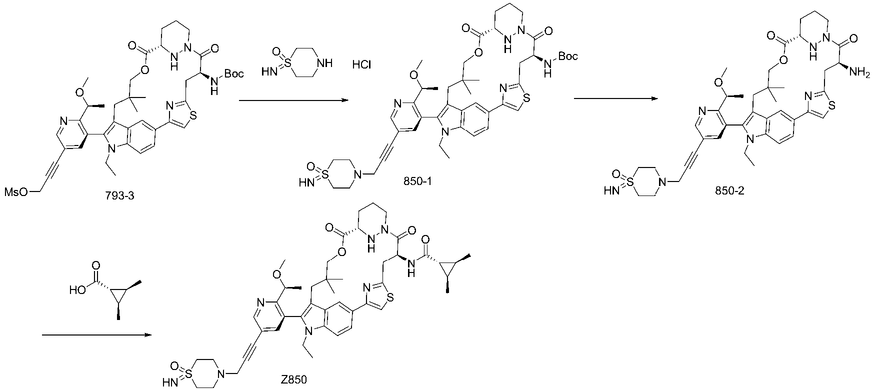

Step 1: Compound 793-3 (45 mg, 0.052 mmol) was dissolved in acetonitrile (10 mL), and 1-imino-1^{λ}6-1,4-thiazin-1-one (13.99 mg, 0.104 mmol), N,N'-diisopropylethylamine (26.96 mg, 0.209 mmol), and sodium iodide (7.82 mg, 0.052 mmol) were added. The mixture was reacted at 60 °C for 2 h under a nitrogen atmosphere. After the reaction was completed, the mixture was filtered under vacuum and concentrated to dryness under reduced pressure, and the residue was purified by a flash silica gel column (methanol/dichloromethane: 0%-10%) to give compound 850-1 (38 mg, 0.042 mmol, yield: 80.87%) as a white solid, ES-API: [M+H]⁺ = 901.3.

Step 2: Compound 850-1 (30 mg, 0.033 mmol) was dissolved in methanol (0.5 mL), and a 4 M solution of hydrochloric acid in dioxane (1.5 mL) was added. The mixture was reacted at room temperature for 2 h under a nitrogen atmosphere. After the reaction was completed, the mixture was concentrated to dryness under reduced pressure to give compound 850-2 (26 mg, 0.032 mmol, yield: 97.49%) as a white solid, ES-API: [M+H]⁺ = 801.3.

Step 3: Compound 850-2 (0.25 g, 0.421 mmol) was dissolved in dichloromethane (2 mL). (1r,2R,3S)-2,3-Dimethylcyclopropane-1-carboxylic acid (3.42 mg, 0.030 mmol), O-(7-azabenzotriazol-1-yl)-N,N,N',N'-tetramethyluronium hexafluorophosphate (14.22 mg, 0.037 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give (1*r*,2*R*,3*S*)-*N*-((6³*S*,4*S,Z*)-1¹-ethyl-(*Rₐ*)-1²-(5-(3-(1-imino-1-oxo-11⁶-thiomorpholino)prop-1-yn-1-yl)-2-((*S*)-1-methoxyethyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z850, 2.4 mg, 0.003 mmol, yield: 10.71%) as a white solid, ES-API: [M+H]⁺ = 897.3.

### Example 167. Synthesis of Compounds Z954-1 and Z954

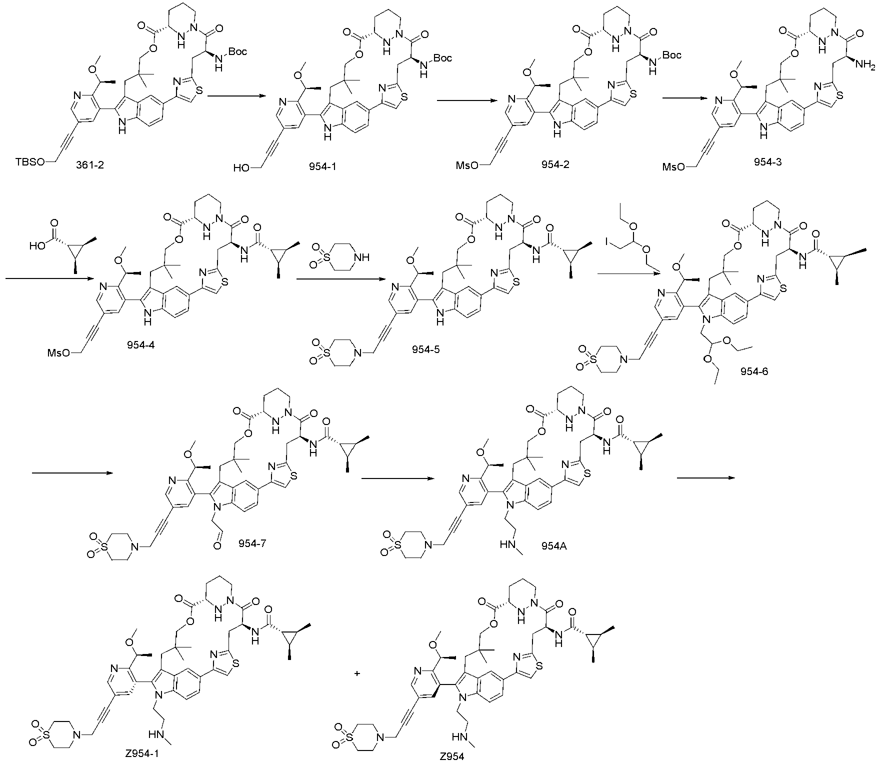

Step 1: A tetrabutylammonium fluoride solution (3.5 mL, 3.444 mmol) was added to a solution of compound 361-2 (1000 mg, 1.148 mmol) in tetrahydrofuran (15 mL) under an ice-water bath. The reaction mixture was stirred at 0 °C for 1 h. When the reaction was completed as detected by LCMS, the reaction mixture was diluted with 50 mL of ethyl acetate and then washed with saturated brine (15 mL × 3). The organic phase was dried over anhydrous sodium sulfate, filtered, and concentrated to dryness to give crude compound 954-1 (868 mg, yield: 99%). ES-API: [M+H]⁺ = 757.3.

Step 2: N,N-Diisopropylethylamine (1.0 mL, 5.734 mmol) and methanesulfonic anhydride (300 mg, 1.720 mmol) were sequentially added to a solution of compound 954-1 (868 mg, 1.147 mmol) in dichloromethane (15 mL) under an ice-water bath. The reaction mixture was stirred at room temperature for 1 h. When the reaction was completed as detected by LCMS, the reaction mixture was diluted with 20 mL of dichloromethane and then washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 954-2 (957 mg, yield: 99%). ES-API: [M+H]⁺ = 835.2.

Step 3: A hydrogen chloride/dioxane solution (10 mL, 4 M) was added to a solution of compound 954-2 (957 mg, 1.146 mmol) in methanol (2 mL). The reaction mixture was stirred at room temperature for 1 h. When the reaction was completed as detected by LCMS, the mixture was concentrated under reduced pressure to remove most of the solvent to give compound 954-3 (842 mg, yield: 99%). ES-API: [M+H]⁺ = 735.2.

Step 4: (1r,2R,3S)-2,3-Dimethylcyclopropyl-1-carboxylic acid (170 mg, 1.489 mmol), N,N-diisopropylethylamine (1.2 mL, 6.874 mmol), and O-(7-azabenzotriazol-1-yl)-N,N,N',N'-tetramethyluronium hexafluorophosphate (653 mg, 1.719 mmol) were sequentially added to a solution of compound 954-3 (842 mg, 1.146 mmol) in N,N-dimethylformamide (10 mL) under an ice-water bath. The reaction mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by automated flash chromatography on silica gel (methanol/dichloromethane = 0-2%) to give compound 954-4 (579 mg, yield: 61%). ES-API: [M+H]⁺ = 831.3.

Step 5: Potassium carbonate (315 mg, 2.280 mmol) and thiomorpholine 1,1-dioxide (308 mg, 2.280 mmol) were sequentially added to a solution of compound 954-4 (379 mg, 0.456 mmol) in N,N-dimethylformamide (10 mL). The reaction mixture was heated to 60 °C and stirred overnight. When the reaction was completed as detected by LCMS, the mixture was filtered to remove insoluble substances, and the filtrate was concentrated to dryness. The residue was purified by automated flash chromatography on silica gel (methanol/dichloromethane = 0-5%) to give compound 954-5 (255 mg, yield: 64%). ES-API: [M+H]⁺ = 870.1.

Step 6: Cesium carbonate (382 mg, 1.172 mmol) and 1,1-diethoxy-2-iodoethane (286 mg, 1.172 mmol) were sequentially added to a solution of compound 954-5 (255 mg, 0.293 mmol) in N,N-dimethylformamide (10 mL). The reaction mixture was heated to 50 °C and stirred for 2 h. When the reaction was completed as detected by LCMS, the reaction mixture was cooled to room temperature, diluted with 20 mL of ethyl acetate, and then washed with water (10 mL) and saturated brine (15 mL × 3). The organic phase was dried over anhydrous sodium sulfate, filtered, and concentrated to dryness to give crude compound 954-6 (118 mg, yield: 41%). ES-API: [M+H]⁺ = 986.3.

Step 7: Concentrated hydrochloric acid (5.0 mL) was added to a solution of compound 954-6 (118 mg, 0.120 mmol) in acetonitrile (5 mL), and the reaction mixture was stirred at room temperature for 24 h. When the reaction was completed as detected by LCMS, the mixture was concentrated under reduced pressure to remove most of the solvent. The aqueous phase was extracted with ethyl acetate (10 mL × 3), and the organic phase was dried over anhydrous sodium sulfate, filtered, and concentrated to give crude compound 954-7 (102 mg, yield: 93%). ES-API: [M+H]⁺ = 912.1.

Step 8: Triethylamine (0.03 mL, 0.224 mmol) was added to a solution of compound 954-7 (102 mg, 0.112 mmol) and methylamine hydrochloride (15.10 mg, 0.224 mmol) in tetrahydrofuran (2 mL) and dichloromethane (6 mL), and the reaction mixture was heated to 50 °C and stirred for 0.5 h. Sodium triacetoxyborohydride (15 mg, 0.224 mmol) was then added to the reaction mixture, and the reaction mixture was stirred at 50 °C for another 24 h. As detected by LCMS, the desired compound was generated, and part of the starting materials remained. The reaction mixture was cooled to room temperature, and 20 mL of a saturated sodium bicarbonate solution was added to quench the reaction. The mixture was then extracted with ethyl acetate (15 mL × 3). The organic phase was washed with saturated brine (15 mL × 3), dried over anhydrous sodium sulfate, filtered, and concentrated to dryness. The residue was purified by automated flash chromatography on silica gel (methanol/dichloromethane = 0-5%) to give a mixture 954A. The mixture 954A was subjected to chiral resolution (chromatographic column: CHIRALPAK^{®} IB, 10 µm, 30 × 250 mm; mobile phase A: ACN + 0.2% DEA, mobile phase B: IPA + 0.2% DEA; flow rate: 25 mL/min; isocratic elution program: mobile phase A:mobile phase B = 95:5 (V/V); column temperature: room temperature) to give 2 isomeric compounds. The structure of one of the isomeric compounds was arbitrarily designated as (1r,2R,3S)-N-((6³S,4S,Z)-1²-(5-(3-(1,1-dioxidodimorpholino)prop-1-yn-1-yl)-(Sₐ)-2-((S)-1-methoxyethyl)pyridin-3-yl)-10,10-dimethyl-1¹-(2-(methylamino)ethyl)-5,7-dioxo-6¹,6²,6³,6⁴,6⁵,6⁶-hexahydro-1¹H-8-oxa-2-(4,2)-thiazola-1(5,3)-indola-6(1,3)-pyridazinacycloundecaphane-4-yl)-2,3-dimethylcyclopropane-1-carboxamide (Z954-1, 21.67 mg, yield: 21%, retention time: 5.056 min). ES-API [M+H]⁺ = 927.2. ¹H NMR (400 MHz, DMSO-d6) δ 8.81 (d, J= 2.0 Hz, 1 H), 8.52 (s, 1 H), 8.41 (d, J= 9.2 Hz, 1 H), 8.04 (d, J= 2.0 Hz, 1 H), 7.82 (s, 1 H), 7.74 (dd, J1= 1.6 Hz, J2= 8.8 Hz, 1 H), 7.56 (d, J= 8.8 Hz, 1 H), 5.53 (t, J= 9.2 Hz, 1 H), 5.06- 5.03 (m, 1 H), 4.24- 4.18 (m, 2 H), 4.03- 3.91 (m, 2 H), 3.84- 3.80 (m, 1 H), 3.77 (s, 2 H), 3.66 (d, J= 10.8 Hz, 1 H), 3.54 (d, J= 11.2 Hz, 1 H), 3.17- 3.16 (m, 5 H), 3.08 (s, 3 H), 3.05- 3.02 (m, 5 H), 2.80- 2.71 (m, 2 H), 2.62- 2.56 (m, 1 H), 2.34- 2.30 (m, 1 H), 2.14- 2.12 (m, 4 H), 1.79- 1.75 (m, 2 H), 1.53- 1.48 (m, 1 H), 1.23- 1.16 (m, 8 H), 1.10- 1.06 (m, 6 H), 0.93 (s, 3 H), 0.50 (s, 3 H).

The structure of the other isomeric compound was arbitrarily designated as (1r,2R,3S)-N-((6³S,4S,Z)-1²-(5-(3-(1,1-dioxidodimorpholino)prop-1-yn-1-yl)-(Rₐ)-2-((S)-1-methoxyethyl)pyridin-3-yl)-10,10-dimethyl-1¹-(2-(methylamino)ethyl)-5,7-dioxo-6¹,6²,6³,6⁴,6⁵,6⁶-hexahydro-1¹H-8-oxa-2-(4,2)-thiazola-1(5,3)-indola-6(1,3)-pyridazinacycloundecaphane-4-yl)-2,3-dimethylcyclopropane-1-carboxamide (Z954, 9.04 mg, yield: 9%, retention time: 5.709 min). ES-API [M+H]⁺ = 927.2. ¹H NMR (400 MHz, DMSO-d6) δ 8.80 (d, J= 2.0 Hz, 1 H), 8.49 (s, 1 H), 8.39 (d, J= 9.2 Hz, 1 H), 8.24- 7.86 (m, 1 H), 7.82 (s, 1 H), 7.75 (d, J= 8.4 Hz, 1 H), 7.59 (d, J= 8.4 Hz, 1 H), 5.58- 5.53 (m, 1 H), 5.09- 5.06 (m, 1 H), 4.32- 4.15 (m, 5 H), 3.76 (s, 2 H), 3.57 (s, 2 H), 3.25 (s, 3 H), 3.16- 3.15 (m, 5 H), 3.05- 2.99 (m, 4 H), 2.96- 2.93 (m, 1 H), 2.79- 2.72 (m, 1 H), 2.44-2.28 (m, 2 H), 2.11- 2.04 (m, 1 H), 1.95 (s, 2 H), 1.80- 1.75 (m, 2 H), 1.55- 1.48 (m, 1 H), 1.35 (d, J=6.0 Hz, 3 H), 1.23- 1.05 (m, 13 H), 0.90 (s, 3 H), 0.34 (s, 3 H).

### Example 168. Synthesis of Compound Z945

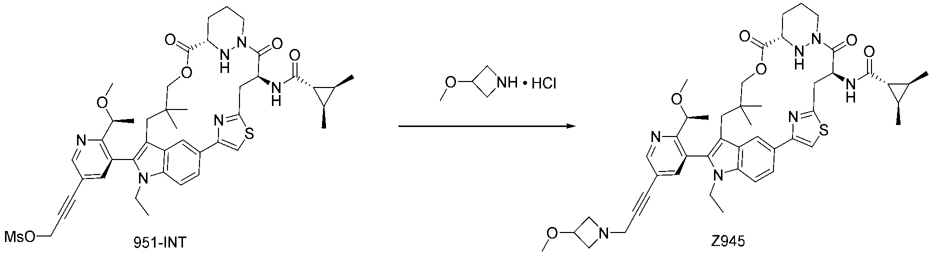

Step 1: Potassium carbonate (64 mg, 0.466 mmol) and 3-methoxy-azetidine hydrochloride (22 mg, 0.175 mmol) were sequentially added to a solution of compound 951-INT (100 mg, 0.116 mmol) in acetonitrile (10 mL). The reaction mixture was heated to 40 °C and stirred for 2 h. When the reaction was completed as detected by LCMS, the mixture was filtered to remove insoluble substances, and the filtrate was concentrated to dryness. The residue was purified by automated flash chromatography on silica gel (methanol/dichloromethane = 0-5%), followed by secondary purification by prep-HPLC (ammonium bicarbonate method) to give (1r,2R,3S)-N-((6³S,4S,Z)-1¹-ethyl-1²-(5-(3-(3-methoxyazetidin-1-yl)prop-1-yn-1-yl)-(Rₐ)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z945, 26.15 mg, yield: 26%) as a white solid. ES-API: [M+H]⁺ = 850.2. ¹H NMR (400 MHz, DMSO-d6) δ 8.81 (s, 1 H), 8.50 (s, 1 H), 8.38 (d, J= 8.8 Hz, 1 H), 7.85 (s, 1 H), 7.81 (s, 1 H), 7.76- 7.74 (m, 1 H), 7.58 (d, J= 8.4 Hz, 1 H), 5.56 (t, J= 9.2 Hz, 1 H), 5.06 (d, J= 11.6 Hz, 1 H), 4.36- 4.05 (m, 5 H), 4.00- 3.94 (m, 1 H), 3.58- 3.49 (m, 6 H), 3.25 (s, 3 H), 3.17- 3.12 (m, 4 H), 3.10- 3.06 (m, 2 H), 2.97- 2.93 (m, 1 H), 2.78- 2.72 (m, 1 H), 2.42- 2.38 (m, 1 H), 2.09- 2.06 (m, 1 H), 1.81- 1.74 (m, 2 H), 1.56- 1.49 (m, 1 H), 1.36 (d, J= 6.0 Hz, 3 H), 1.20- 1.04 (m, 10 H), 0.91- 0.87 (m, 6 H), 0.35 (s, 3 H).

### Example 169. Synthesis of Compound Z1034

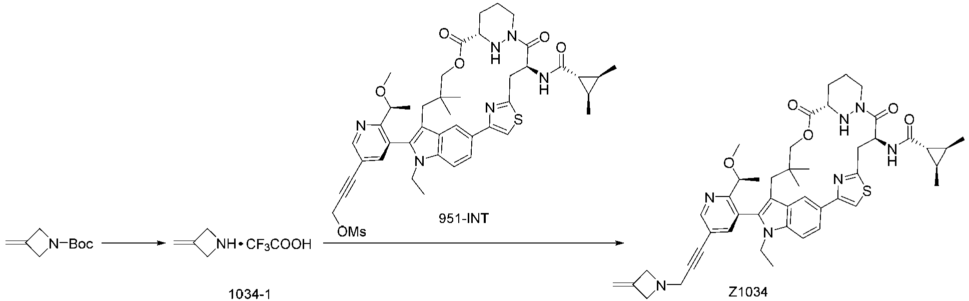

Step 1: tert-Butyl 3-methyleneazetidine-1-carboxylate (500 mg, 2.96 mmol), dichloromethane (6 mL), and trifluoroacetic acid (2.5 mL) were added to a round-bottom flask. The system was stirred at room temperature for 2 h. The reaction mixture was concentrated to give compound 1034-1 (540 mg, trifluoroacetate) as a colorless oil, and the crude product was directly used in the next step.

Step 2: Compound 1034-1 (40 mg, crude product), compound 951-INT (100 mg, 0.12 mmol), potassium carbonate (113 mg, 0.81 mmol), and acetonitrile (4 mL) were added to a round-bottom flask. The reaction system was stirred at 50 °C for 1 h. Water (50 mL) was added to the reaction mixture, and the mixture was extracted with ethyl acetate (50 mL × 3). The organic phase was dried and concentrated. The crude product was purified by prep-HPLC (ammonium bicarbonate method) to give (1r,2R,3S)-N-((6³S,4S,Z)-1'-ethyl-1²-(2-((S)-1-methoxyethyl)-5-(3-(3-methyleneazetidin-1-yl)prop-1-yn-1-yl)pyridin-3-yl)-10,10-dimethyl-5,7-dioxo-6¹,6²,6³,6⁴,6⁵ 6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z1034, 14 mg, yield: 14%) as a white solid. ES-API: [M+H]⁺ = 832.2. ¹HNMR (400MHz, DMSO-*d*₆): 8.80 (s, 1H), 8.50 (s, 1H), 8.38 (d, *J*=8.8Hz, 1H), 7.85 (s, 1H), 7.81 (s, 1H), 7.75 (d, *J*=8.4Hz, 1H), 7.58 (d, *J*=8.4Hz, 1H), 5.56 (t, *J*=9.2Hz, 1H), 5.06 (d, *J*=12.4Hz, 1H), 4.85 (brs, 2H), 4.35-4.04 (m, 5H), 3.95-3.90 (m, 4H), 3.61 (s, 2H), 3.57 (s, 2H), 3.31-3.11 (m, 5H), 2.96-2.93 (m, 1H), 2.80-2.65 (m, 1H), 2.41-2.37 (m, 1H), 2.08-2.06 (m, 1H), 1.80-1.70 (m, 2H), 1.60-1.45 (m, 1H), 1.36 (d, *J*=6.0Hz, 3H),1.25-1.15 (m, 3H), 1.09-1.05 (m, 6H), 0.90-0.86 (m, 6H), 0.34 (s, 3H).

### Example 170. Synthesis of Compound Z1045

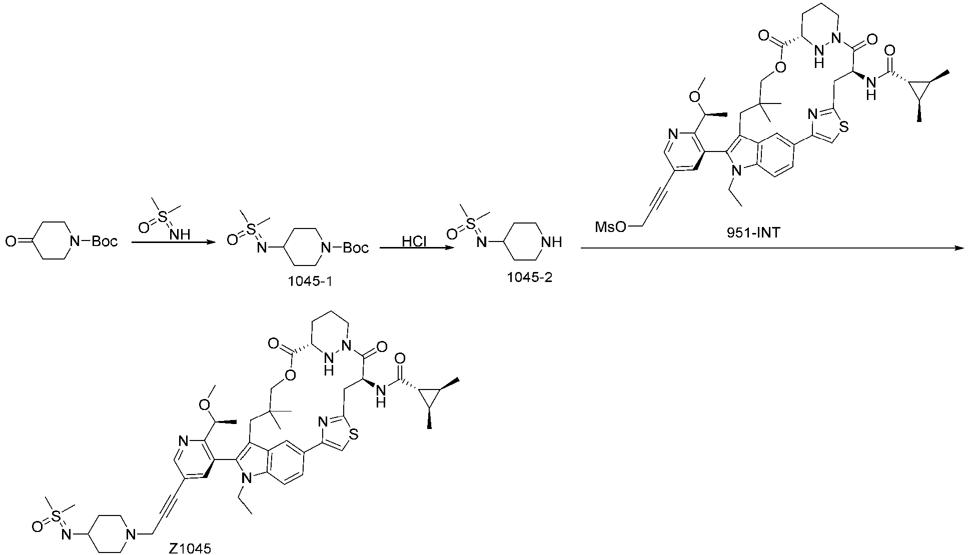

Step 1: Sodium triacetoxyborohydride (10.63 g, 46.728 mmol) was added to a solution of tert-butyl 3-formylpiperidine-1-carboxylate (4.0 g, 23.364 mmol) and (methanesulfonylimino)methane (1.09 g, 11.682 mmol) in 1,2-dichloroethane (120.0 mL), and the reaction system was stirred at 35 °C for 15 h. After the reaction was completed, the reaction mixture was cooled to ambient temperature and filtered, and the filtrate was concentrated in vacuum. The residue was then dissolved in water, and the mixture was adjusted to about pH 1 with an aqueous hydrochloric acid solution (2 M) and extracted with dichloromethane (200 mL × 3). The aqueous layer was adjusted to about pH 9 with an aqueous sodium hydroxide solution (2 M) and extracted with dichloromethane (200 mL × 3). The organic extracts were combined, dried (over anhydrous magnesium sulfate), filtered, and concentrated in vacuum to give compound 1045-1 (700 mg, yield: 10.88%) as a colorless oil, which was directly used in the next step without further purification. ES-API: [M+H]⁺ = 277.2

Step 2: A solution of hydrochloric acid in dioxane (5.0 mL, 4 M, 20.0 mmol) was added to a solution of compound 1045-1 (300 mg, 1.085 mmol) in methanol (10.0 mL). The mixture was reacted at room temperature for 1 h and then concentrated by rotary evaporation under reduced pressure to remove the solvent to give crude compound 1045-2 (257 mg, crude product). ES-API: [M+H]⁺ = 177.3.

Step 3: Potassium carbonate (64 mg, 0.466 mmol) and compound 1045-2 (257 mg, crude product) were sequentially added to a solution of compound 951-INT (100 mg, 0.116 mmol) in acetonitrile (10 mL). The reaction mixture was heated to 50 °C and stirred for 1 h. When the reaction was completed as detected by LCMS, the mixture was filtered to remove insoluble substances, and the filtrate was concentrated to dryness. The residue was purified by automated flash chromatography on silica gel (methanol/dichloromethane = 0-5%), followed by secondary purification by prep-HPLC (formic acid method 1) to give (2R,3S)-N-((6³S,4S,Z)-1²-(5-(3-(4-((dimethyl(oxo)-1⁶-sulfaneylidene)amino)piperidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide formate (Z1045, 30.0 mg, yield: 18.29%) as a white solid. ES-API: [M+H]⁺ = 939.4.

### Example 171. Synthesis of Compound Z1046

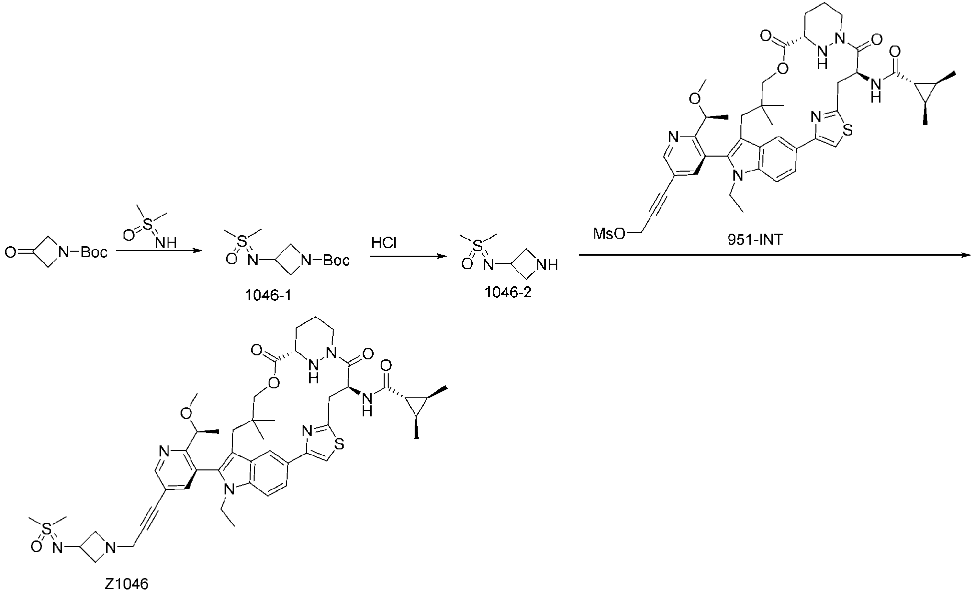

Step 1: Sodium triacetoxyborohydride (10.63 g, 46.728 mmol) was added to a solution of tert-butyl 3-formylpiperidine-1-carboxylate (4.0 g, 23.364 mmol) and (methanesulfonylimino)methane (1.09 g, 11.682 mmol) in 1,2-dichloroethane (120.0 mL), and the reaction system was stirred at 35 °C for 15 h. After the reaction was completed, the reaction mixture was cooled to ambient temperature and filtered, and the filtrate was concentrated in vacuum. The residue was then dissolved in water, and the mixture was adjusted to about pH 1 with an aqueous hydrochloric acid solution (2 M) and extracted with dichloromethane (200 mL × 3). The aqueous layer was adjusted to about pH 9 with an aqueous sodium hydroxide solution (2 M) and extracted with dichloromethane (200 mL × 3). The organic extracts were combined, dried (over anhydrous magnesium sulfate), filtered, and concentrated in vacuum to give compound 1046-1 (160.0 mg, yield: 2.76%) as a colorless oil, which was directly used in the next step without further purification. ES-API: [M-56+H]⁺ = 193.1 .

Step 2: A solution of hydrochloric acid in dioxane (5.0 mL, 4 M, 20.0 mmol) was added to a solution of compound 1046-1 (160.0 mg, 0.644 mmol) in methanol (10.0 mL). The mixture was reacted at room temperature for 1 h and then concentrated by rotary evaporation under reduced pressure to remove the solvent to give crude compound 1046-2 (190.0 mg, crude product). ES-API: [M+H]⁺ = 149.2.

Step 3: Potassium carbonate (64 mg, 0.466 mmol) and compound 1046-2 (190.0 mg, crude product) were sequentially added to a solution of compound 951-INT (100 mg, 0.116 mmol) in acetonitrile (10 mL). The reaction mixture was heated to 50 °C and stirred for 1 h. When the reaction was completed as detected by LCMS, the mixture was filtered to remove insoluble substances, and the filtrate was concentrated to dryness. The residue was purified by automated flash chromatography on silica gel (methanol/dichloromethane = 0-5%), followed by secondary purification by prep-HPLC (ammonium bicarbonate method) to give the desired compound (2R,3S)-N-((6³S,4S,Z)-1²-(5-(3-(3-((dimethyl(oxo)-1⁶-sulfaneylidene)amino)azetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵ 6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z1046, 5.0 mg, yield: 4.70%) as a white solid. ES-API: [M+H]⁺ = 911.4.

### Example 172. Synthesis of Compound Z369-1

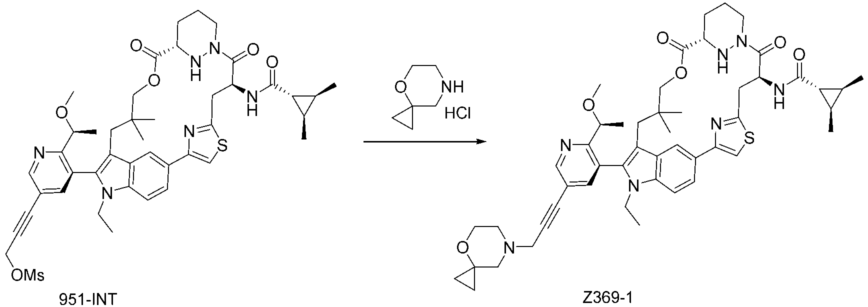

Step 1: 4-Oxa-7-aza-spiro[2.5]octane hydrochloride (53 mg, 0.35 mmol), compound 951-INT (150 mg, 0.17 mmol), potassium carbonate (73 mg, 0.53 mmol), sodium iodide (26 mg, 0.17 mmol), and acetonitrile (4 mL) were added to a round-bottom flask. The reaction system was stirred at 70 °C for 1 h. Water (50 mL) was added to the reaction mixture, and the mixture was extracted with ethyl acetate (50 mL × 3). The organic phase was dried over anhydrous sodium sulfate and concentrated. The crude product was purified by prep-HPLC (formic acid method 1) to give (1r,2R,3S)-N-((6³S,4S,Z)-12-(5-(3-(4-oxa-7-azaspiro[2.5]octan-7-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide formate (Z369-1, 85 mg, yield: 52.8%) as a white solid. ES-API: [M+H]⁺ = 876.3. ¹H NMR (400MHz, MeOD-*d*₄): 8.79 (d, *J*=2.0Hz, 1H), 8.58(d, *J*=1.2Hz, 1H), 7.89 (d, *J*=2.0Hz, 1H), 7.74-7.72 (m, 1H), 7.57 (s, 1H), 7.51 (d, *J*=8.8Hz, 1H), 5.78 (d, *J*=6.8Hz, 1H), 4.36-4.11 (m, 5H), 3.83-3.80 (m, 2H), 3.77-3.71 (m, 2H), 3.64 (s, 2H), 3.46-3.42 (m, 1H), 3.35 (s, 3H), 3.30-3.26 (m, 1H), 3.15-3.07 (m, 1H), 2.82-2.76 (m, 3H), 2.67 (s, 2H), 2.59-2.55 (m, 1H), 2.23-2.18 (m, 1H), 2.01-1.92 (m, 1H), 1.82-1.72 (m, 1H), 1.65-1.57 (m, 1H), 1.46 (d, *J*=6.0Hz, 3H), 1.42-1.34 (m, 2H), 1.18-1.13 (m, 7H), 1.02-0.92 (m, 6H), 0.80-0.77 (m, 2H), 0.64-0.61 (m, 2H), 0.49 (s, 3H).

### Example 173. Synthesis of Compound Z1047

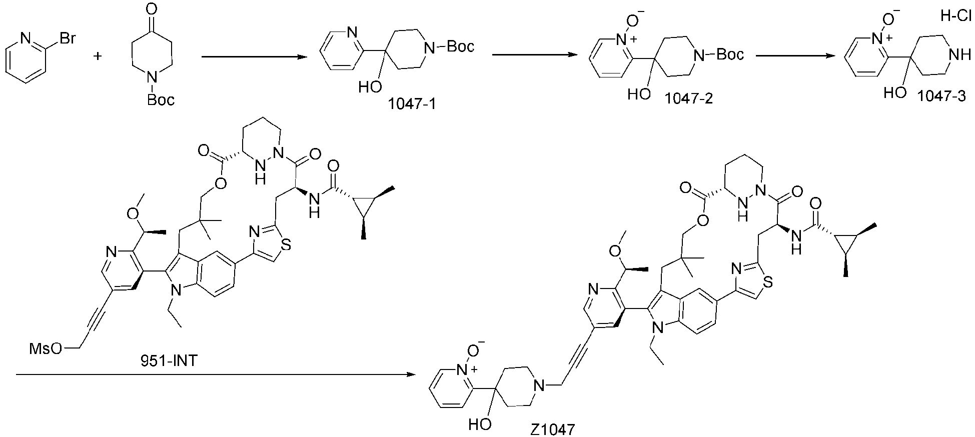

Step 1: n-Butyllithium (40 mL, 100 mmol) was slowly added dropwise to a solution of 2-bromopyridine (5 g, 31.646 mmol) in anhydrous tetrahydrofuran (50 mL) at -78 °C. The reaction mixture was stirred for 30 min under a nitrogen atmosphere. A solution of tert-butyl 4-oxopiperidine-1-carboxylate (6.936 g, 34.811 mmol) in tetrahydrofuran (10 mL) was then slowly added dropwise to the solution described above. The reaction mixture was slowly warmed to room temperature and stirred for 2 h. When the reaction was completed as detected by LC-MS, saturated ammonium chloride (50 mL) was slowly added to the reaction mixture to quench the reaction. The mixture was then extracted with ethyl acetate (30 mL × 3). The organic phases were combined, washed with saturated brine (30 mL × 3), dried over anhydrous sodium sulfate, filtered, and concentrated to dryness. The residue was purified by automated flash chromatography on silica gel (ethyl acetate:petroleum ether = 0-30%) to give compound 1047-1 (1.9 g, yield: 22%). ES-API: [M+H]⁺ = 279.3.

Step 2: Compound 1047-1 (472 mg, 1.696 mmol) was dissolved in dichloromethane (15 mL) at room temperature, and m-chloroperoxybenzoic acid (861 mg, 4.239 mmol) was added to the reaction mixture. The mixture was then stirred at room temperature for 36 h. The reaction mixture was diluted with dichloromethane (20 mL) and then washed with a sodium sulfite solution (20 mL × 2) and a saturated sodium bicarbonate solution (20 mL × 3). The organic phase was dried over anhydrous sodium sulfate, filtered, and concentrated to dryness. The residue was purified by automated flash chromatography on silica gel (methanol:dichloromethane = 0-2%) to give compound 1047-2 (1.9 g, yield: 22%). ES-API: [M+H]⁺ = 295.1.

Step 3: A solution of hydrochloric acid in dioxane (3 mL, 4 M) was added to a solution of compound 1047-2 (60 mg, 0.204 mmol) in methanol (1 mL). The reaction mixture was stirred at room temperature for 1.5 h. When the reaction was completed as detected by LCMS, the mixture was concentrated under reduced pressure to give crude compound 1047-3 (39 mg). ES-API: [M+H]⁺ = 195.2.

Step 4: Potassium carbonate (37 mg, 0.265 mmol) and compound 1047-3 (31 mg, 0.157 mmol) were sequentially added to a solution of compound 951-INT (76 mg, 0.088 mmol) in acetonitrile (10 mL). The reaction mixture was heated to 40 °C and stirred for 3 h. When the reaction was completed as detected by LC-MS, the mixture was filtered to remove insoluble substances, and the filtrate was concentrated to dryness. The residue was purified by HPLC (formic acid method 1) to give the desired compound 2-(1-(3-(5-((6³S,4S,Z)-4-((lr,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-(Rₐ)-6-((S)-1-methoxyethyl)pyridin-3-yl)prop-2-yn-1-yl)-4-hydroxypiperidin-4-yl)pyridine-1-oxide formate (Z1047, 35.63 mg, yield: 42%) as a white solid. ES-API: [M+H]⁺ = 957.3. ¹H NMR (400 MHz, DMSO-d₆) δ 8.81 (d, J= 2.0 Hz, 1 H), 8.50 (s, 1 H), 8.38 (d, J= 8.8 Hz, 1 H), 8.32 (d, J= 6.4 Hz, 1 H), 7.85 (d, J= 2.0 Hz, 1 H), 7.81 (s, 1 H), 7.75 (dd, J1= 1.2 Hz, J2= 8.8 Hz, 1 H), 7.64 (dd, J1= 1.6 Hz, J2= 8.0 Hz, 1 H), 7.58 (d, J= 8.4 Hz, 1 H), 7.53- 7.49 (m, 1 H), 7.44- 7.40 (m, 1 H), 7.26 (s, 1 H), 5.56 (t, J= 8.8 Hz, 1 H), 5.06 (d, J= 12.0 Hz, 1 H), 4.36- 4.15 (m, 4 H), 4.11- 4.06 (m, 1 H), 3.58 (s, 4 H), 3.25 (s, 3 H), 3.17- 3.11 (m, 1 H), 2.98- 2.94 (m, 1 H), 2.78- 2.67 (m, 5 H), 2.45- 2.33 (m, 2 H), 2.23- 2.17 (m, 2 H), 2.09- 2.06 (m, 1 H), 1.96- 1.93 (m, 2 H), 1.81- 1.74 (m, 2 H), 1.56- 1.48 (m, 1 H), 1.36 (d, J= 6.0 Hz, 3 H), 1.20- 1.41 (m, 3 H), 1.09- 1.05 (m, 6 H), 0.91- 0.87 (m, 6 H), 0.35 (s, 3 H).

### Example 174. Synthesis of Compound Z390-1

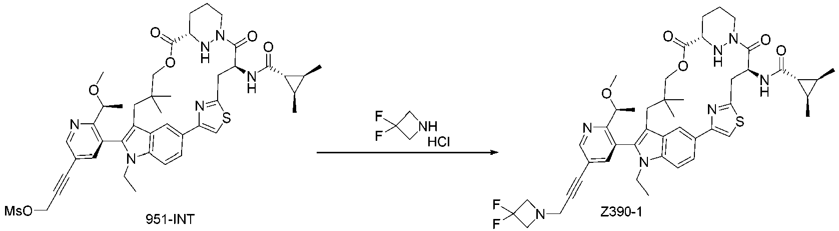

Step 1: Sodium iodide (50.0 mg, 0.3 mmol), potassium carbonate (145.0 mg, 1.048 mmol), and 3,3-difluoroazetidine hydrochloride (113.0 mg, 0.875 mmol) were sequentially added to a solution of compound 951-INT (150 mg, 0.175 mmol) in acetonitrile (10 mL). The reaction mixture was heated to 80 °C and stirred for 1 h. When the reaction was completed as detected by LC-MS, the mixture was filtered to remove insoluble substances, and the filtrate was concentrated to dryness.

The residue was purified by automated flash chromatography on silica gel (methanol/dichloromethane = 0-5%), followed by secondary purification by prep-HPLC (ammonium bicarbonate method) to give the desired compound (2R,3S)-N-((6³S,4S,Z)-1²-(5-(3-(3,3-difluoroazetidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z390-1, 16.0 mg, yield: 10.68%) as a white solid. ES-API: [M+H]⁺ = 856.3.

### Example 175. Synthesis of Compounds Z362-1 and Z362-2

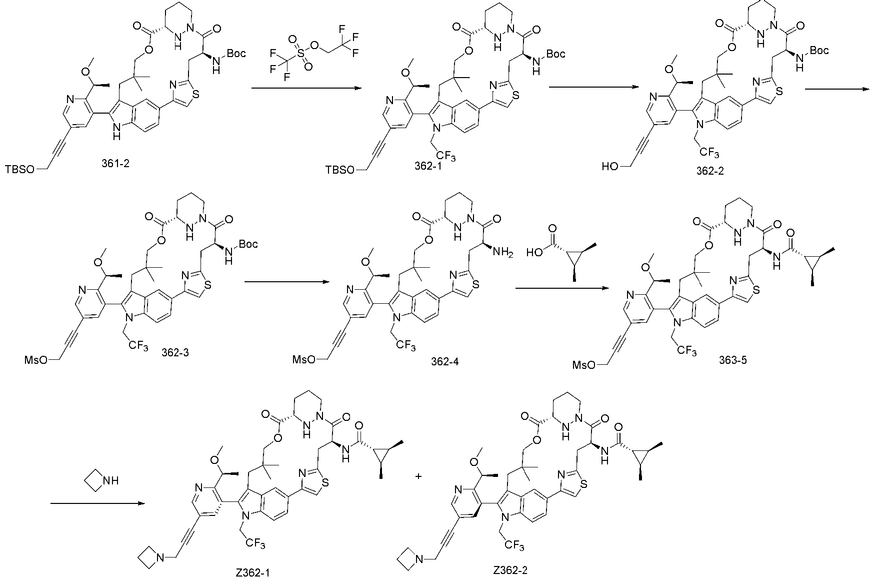

Step 1: Cesium carbonate (673 mg, 2.067 mmol) and 2,2,2-trifluoroethyl trifluoromethanesulfonate (800 mg, 3.447 mmol) were sequentially added to a solution of compound 361-2 (600 mg, 0.689 mmol) in N,N-dimethylformamide (10 mL). The reaction mixture was stirred at 25 °C overnight. When the reaction was completed as detected by LC-MS, the reaction mixture was diluted with 50 mL of ethyl acetate and then washed with saturated brine (15 mL × 3). The organic phase was dried over anhydrous sodium sulfate, filtered, and concentrated to dryness to give crude compound 362-1 (656 mg, yield: 99%). ES-API: [M+H]⁺ = 953.2.

Step 2: A tetrabutylammonium fluoride solution (2.1 mL, 2.065 mmol) was added to a solution of compound 362-1 (656 mg, 0.688 mmol) in tetrahydrofuran (15 mL) under an ice-water bath. The reaction mixture was stirred at 0 °C for 1 h. When the reaction was completed as detected by LCMS, the reaction mixture was diluted with 50 mL of ethyl acetate and then washed with saturated brine (15 mL × 3). The organic phase was dried over anhydrous sodium sulfate, filtered, and concentrated to dryness to give crude compound 362-2 (577 mg, yield: 99%). ES-API: [M+H]⁺ = 839.2.

Step 3: N,N-Diisopropylethylamine (0.6 mL, 3.439 mmol) and methanesulfonic anhydride (179.7 mg, 1.032 mmol) were sequentially added to a solution of compound 362-2 (577 mg, 0.688 mmol) in dichloromethane (20 mL) under an ice-water bath. The reaction mixture was stirred at room temperature for 1 h. When the reaction was completed as detected by LCMS, the reaction mixture was diluted with 20 mL of dichloromethane and then washed with water (10 mL) and saturated brine (15 mL × 3). The organic phase was dried over anhydrous sodium sulfate, filtered, and concentrated to give crude compound 362-3 (630 mg, yield: 99%). ES-API: [M+H]⁺ = 917.2.

Step 4: A hydrogen chloride/dioxane solution (10 mL, 4 M) was added to a solution of compound 362-3 (630 mg, 0.687 mmol) in methanol (2 mL). The reaction mixture was stirred at room temperature for 1 h. When the reaction was completed as detected by LC-MS, the mixture was concentrated under reduced pressure to give crude compound 362-4 (561 mg, yield: 99%). ES-API: [M+H]⁺ = 817.2.

Step 5: (1r,2R,3S)-2,3-Dimethylcyclopropyl-1-carboxylic acid (102 mg, 0.893 mmol), N,N-diisopropylethylamine (0.72 mL, 4.120 mmol), and O-(7-azabenzotriazol-1-yl)-N,N,N',N'-tetramethyluronium hexafluorophosphate (392 mg, 1.030 mmol) were sequentially added to a solution of compound 362-4 (561 mg, 0.687 mmol) in N,N-dimethylformamide (10 mL) under an ice-water bath. The reaction mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by automated flash chromatography on silica gel (methanol/dichloromethane = 0-2%) to give compound 362-5 (430 mg, yield: 69%). ES-API: [M+H]⁺ = 913.2.

Step 6: Potassium carbonate (197 mg, 1.424 mmol) and azetidine (49 mg, 0.854 mmol) were sequentially added to a solution of compound 362-5 (260 mg, 0.285 mmol) in acetonitrile (10 mL). The reaction mixture was heated to 50 °C and stirred for 0.5 h. When the reaction was completed as detected by LC-MS, the mixture was filtered to remove insoluble substances, and the filtrate was concentrated to dryness. The residue was primarily purified by automated flash chromatography on silica gel (methanol/dichloromethane = 0-2%), followed by secondary purification by prep-HPLC (ammonium bicarbonate method) to give 2 isomeric compounds. The structure of one of the isomeric compounds was arbitrarily designated as (1r,2R,3S)-N-((6³S,4S,Z)-1²-(5-(3-(azetidin-1-yl)prop-1-yn-1-yl)-(Sₐ)-2-((S)-1-methoxyethyl)pyridin-3-yl)-10,10-dimethyl-5,7-dioxo-1¹-(2,2,2-trifluoroethyl)-6¹,6²,6³,6^{4,}6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z362-1, 33.31 mg, yield: 13%, retention time: 7.39 min). ES-API [M+H]⁺ = 874.3. ¹H NMR (400 MHz, DMSO-d₆) δ 8.83 (d, J= 2.0 Hz, 1 H), 8.54 (s, 1 H), 8.40 (d, J= 8.8 Hz, 1 H), 7.95 (d, J= 2.0 Hz, 1 H), 7.88 (s, 1 H), 7.83 (dd, J1= 1.2 Hz, J2= 8.8 Hz, 1 H), 7.69 (d, J= 8.8 Hz, 1 H), 5.53 (t, J= 9.2 Hz, 1 H), 5.04 (d, J= 12.4 Hz, 1 H), 4.95- 4.81 (m, 2 H), 4.25-4.19 (m, 2 H), 3.92- 3.87 (m, 1 H), 3.67 (d, J= 10.8 Hz, 1 H), 3.51 (d, J= 10.8 Hz, 1 H), 3.46 (s, 2 H), 3.24 (t, J= 6.8 Hz, 4 H), 3.19- 3.13 (m, 1 H), 3.08 (s, 3 H), 3.06- 3.03 (m, 1 H), 2.80- 2.73 (m, 1 H), 2.43 (d, J= 14.4 Hz, 1 H), 2.13- 2.10 (m, 1 H), 2.00- 1.93 (m, 2 H), 1.82- 1.76 (m, 2 H), 1.57- 1.47 (m, 1 H), 1.25- 1.23 (m, 4 H), 1.20- 1.15 (m, 3 H), 1.10- 1.06 (m, 6 H), 0.93 (s, 3 H), 0.47 (s, 3 H).

The structure of the other isomeric compound was arbitrarily designated as (1r,2R,3S)-N-((6³S,4S,Z)-1²-(5-(3-(azetidin-1-yl)prop-1-yn-1-yl)-(Rₐ)-2-((S)-1-methoxyethyl)pyridin-3-yl)-10,10-dimethyl-5,7-dioxo-1¹-(2,2,2-trifluoroethyl)-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z362-2, 1.66 mg, yield: 0.7%, retention time: 8.34 min). ES-API [M+H]⁺ = 874.3. ¹H NMR (400 MHz, DMSO-d₆) δ 8.84 (d, J= 2.0 Hz, 1 H), 8.51 (d, J= 0.8 Hz, 1 H), 8.40 (d, J= 8.8 Hz, 1 H), 7.88 (s, 1 H), 7.85-7.83 (m, 1 H), 7.80 (d, J= 1.2 Hz, 1 H), 7.80- 7.76 (m, 1 H), 5.63- 5.51 (m, 2 H), 5.05 (d, J= 12.4 Hz, 1 H), 4.88- 4.81 (m, 1 H), 4.26- 4.21 (m, 3 H), 3.61- 3.54 (m, 2 H), 3.46 (s, 2 H), 3.34- 3.29 (m, 4 H), 3.25- 3.22 (m, 4 H), 3.18- 3.12 (m, 1 H), 3.05-3.01 (m, 1 H), 2.79- 2.72 (m, 1 H), 2.41- 2.37 (m, 1 H), 2.12- 2.08 (m, 1 H), 2.00- 1.93 (m, 2 H), 1.79- 1.77 (m, 2 H), 1.55-1.46 (m, 1 H), 1.36 (d, J= 6.0 Hz, 3 H), 1.21- 1.14 (m, 3 H), 1.10- 1.06 (m, 6 H), 0.94 (s, 3 H), 0.30 (s, 3 H).

### Example 176. Synthesis of Compound Z1062

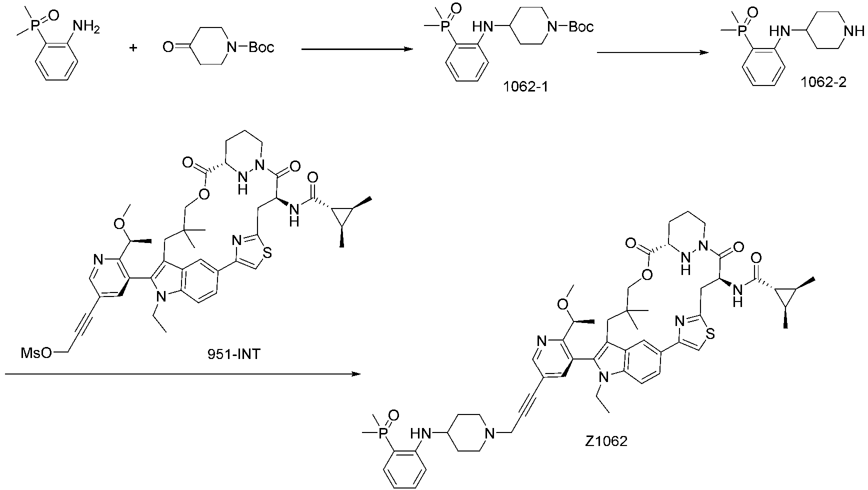

Step 1: 2-(Dimethylphosphinyl)aniline (100 mg, 0.591 mmol) and N-tert-butoxycarbonyl-4-piperidone (121.5 mg, 0.710 mmol) were dissolved in a solution of tetrahydrofuran (3 mL) and methanol (3 mL) at 0 °C, and acetic acid (1 mL) was added to the reaction mixture. The reaction mixture was stirred at 0 °C for 1 h. Sodium cyanoborohydride (76.5 mg, 1.183 mmol) was then added to the solution described above, and the reaction mixture was slowly warmed to room temperature and stirred overnight. When the reaction was completed as detected by LCMS, the reaction mixture was concentrated to dryness under reduced pressure. The residue was purified by automated flash chromatography on silica gel (methanol:dichloromethane = 0-6%) to give compound 1062-1 (64 mg, yield: 33%). ES-API: [M+H]⁺ = 353.2.

Step 2: Trifluoroacetic acid (1 mL) was slowly added dropwise to a solution of compound 1062-1 (64 mg, 0.182 mmol) in dichloromethane (5 mL) at room temperature. The reaction mixture was stirred at room temperature for 1 h. When the reaction was completed as detected by LCMS, the mixture was concentrated under reduced pressure to give crude compound 1062-2 (45 mg, yield: 99%). ES-API: [M+H]⁺ = 253.2.

Step 3: Potassium carbonate (48 mg, 0.349 mmol), sodium iodide (17 mg, 0.116 mmol), and compound 1062-2 (31 mg, 0.157 mmol) were sequentially added to a solution of compound 951-INT (100 mg, 0.116 mmol) in acetonitrile (6 mL). The reaction mixture was heated to 70 °C and stirred for 3 h. When the reaction was completed as detected by LCMS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The residue was purified by HPLC (formic acid method 1) to give the desired compound (1r,2R,3S)-N-((6³S,4S,Z)-1²-(5-(3-(4-((2-(dimethylphosphoryl)phenyl)amino)piperidin-1-yl)prop-1-yn-1-yl)-(Ra)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z1062, 36.81 mg, yield: 31%, formate) as a white solid. ES-API: [M+H]⁺ = 1015.3. ¹H NMR (400 MHz, DMSO-d₆) δ 8.86 (d, J= 2.0 Hz, 1 H), 8.50 (s, 1 H), 8.38 (d, J= 9.2 Hz, 1 H), 7.91 (d, J= 2.0 Hz, 1 H), 7.81 (s, 1 H), 7.77- 7.74 (m, 1 H), 7.59- 7.55 (m, 2 H), 7.26- 7.18 (m, 2 H), 6.68- 6.65 (m, 1 H), 6.57- 6.53 (m, 1 H), 5.56 (t, J= 9.2 Hz, 1 H), 5.06 (d, J= 11.6 Hz, 1 H), 4.36- 4.06 (m, 5 H), 3.59(d, J= 9.2 Hz, 4 H), 3.25 (s, 3 H), 3.18- 3.12 (m, 1 H), 2.98- 2.94 (m, 1 H), 2.83- 2.72 (m, 3 H), 2.49- 2.32 (m, 5 H), 2.08- 2.05 (m, 1 H), 1.95-1.92 (m, 2 H), 1.80- 1.74 (m, 2 H), 1.61- 1.60 (m, 3 H), 1.58- 1.57 (m, 3 H), 1.53- 1.43 (m, 3 H), 1.37- 1.35 (m, 3 H), 1.24-1.13 (m, 3 H), 1.09- 1.04 (m, 6 H), 0.91- 0.88 (m, 6 H), 0.35 (s, 3 H).

### Example 177. Synthesis of Compound Z1054

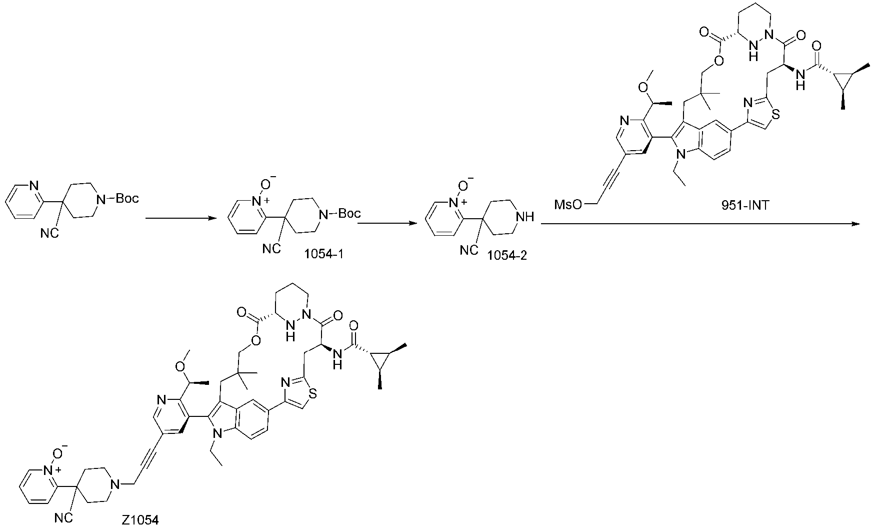

Step 1: m-Chloroperoxybenzoic acid (353.26 mg, 1.740 mmol) was added to a solution of tert-butyl 4-cyano-4-(pyridin-2-yl)piperidine-1-carboxylate (250.0 mg, 0.87 mmol) in dichloromethane (20 mL) at 0 °C. The reaction system was stirred at room temperature for 18 h under a nitrogen atmosphere. The reaction mixture was diluted with dichloromethane (50 mL), washed with a saturated aqueous sodium sulfite solution (20 mL), saturated sodium bicarbonate (30 mL × 2) and brine (30 mL), dried over anhydrous sodium sulfate, filtered, and concentrated in vacuum. The residue was purified by silica gel column chromatography (20 g, eluted with a 0-2% solution of methanol in dioxane) to give compound 1054-1 (160.0 mg, 0.527 mmol, yield: 60.62%) as a light yellow oil. ES-API: [M+H]⁺ = 304.1.

Step 2: Trifluoroacetic acid (1.0 mL) was added to a solution of compound 1054-2 (160.0 mg, 0.527 mmol, crude product) in dichloromethane (4.0 mL) at 0 °C, and the mixture was stirred at room temperature for 1 h. The mixture was then concentrated to dryness by rotary evaporation under reduced pressure to give crude compound 1054-2 (180.0 mg, crude product). ES-API: [M+H]⁺ = 204.3.

Step 3: Potassium carbonate (64 mg, 0.466 mmol) and compound 1054-2 (180.0 mg, crude product) were sequentially added to a solution of compound 951-INT (150 mg, 0.175 mmol) in acetonitrile (10 mL). The reaction mixture was heated to 70 °C and stirred for 1 h. When the reaction was completed as detected by LCMS, the mixture was filtered to remove insoluble substances, and the filtrate was concentrated to dryness. The residue was purified by automated flash chromatography on silica gel (methanol/dichloromethane = 0-5%), followed by secondary purification by prep-HPLC (formic acid method 2) to give the desired compound 2-(4-cyano-1-(3-(5-((6³S,4S,Z)-4-((2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-6-((S)-1-methoxyethyl)pyridin-3-yl)prop-2-yn-1-yl)piperidin-4-yl)pyridine 1-oxide formate (Z1054, 31.0 mg, yield: 18.33%) as a white solid. ES-API: [M+H]⁺ = 966.4. ¹H NMR (400 MHz, DMSO-d₆) δ 8.83 (d, J= 2.4 Hz, 1 H), 8.50 (s, 1 H), 8.39- 8.36 (m, 2 H), 7.85 (d, J= 2.0 Hz, 1 H), 7.81 (s, 1 H), 7.76- 7.73 (m, 1 H), 7.60- 7.57 (m, 2 H), 7.51- 7.42 (m, 2 H), 5.56 (t, J= 9.2 Hz, 1 H), 5.05 (d, J= 12.0 Hz, 1 H), 4.35- 4.04 (m, 5 H), 3.70 (s, 2 H), 3.57 (s, 2 H), 3.25 (s, 3 H), 3.17- 3.11 (m, 1 H), 3.02- 2.93 (m, 3 H), 2.80- 2.74 (m, 3 H), 2.63- 2.60 (m, 2 H), 2.42- 2.38 (m, 1 H), 2.09-2.06 (m, 1 H), 1.97- 1.93 (m, 2 H), 1.83- 1.74 (m, 2 H), 1.56- 1.49 (m, 1 H), 1.37- 1.35 (m, 3 H), 1.24- 1.19 (m, 4 H), 1.16-1.04 (m, 6 H), 0.90- 0.86 (m, 6 H), 0.35 (s, 3 H).

### Example 178. Synthesis of Compounds Z1039 and Z1039-1

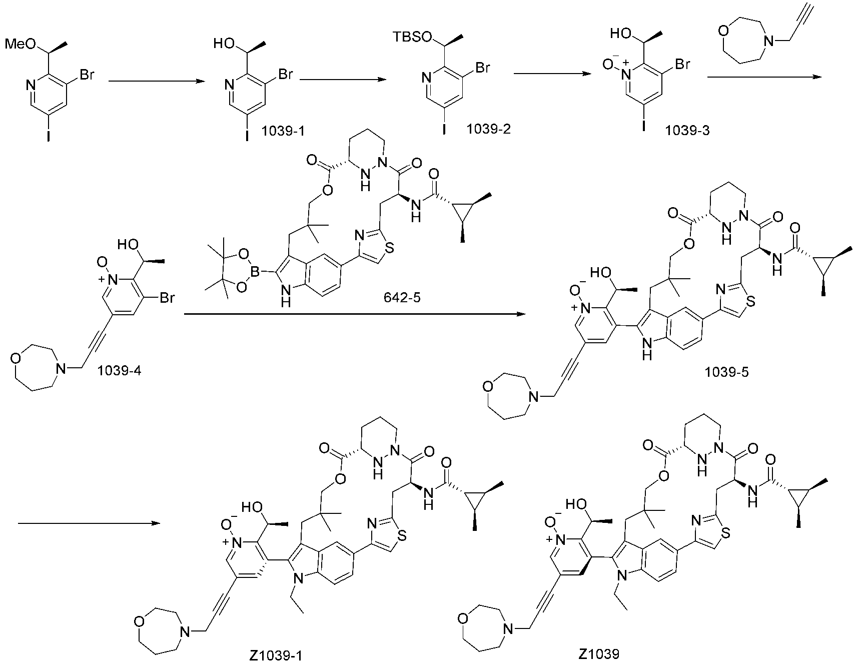

Step 1: (S)-3-bromo-5-iodo-2-(1-methoxyethyl)pyridine (2500 mg, 7.31 mmol) and dichloromethane (20 mL) were added to a round-bottom flask, and a 1 M solution of boron tribromide in dichloromethane (22 mL, 22 mmol) was added dropwise thereto. The reaction system was stirred at room temperature for 2 h. A saturated aqueous sodium bicarbonate solution was added to the reaction mixture, and the mixture was extracted with dichloromethane (50 mL × 3). The organic phase was washed with saturated brine, dried over sodium sulfate, and filtered. The filtrate was concentrated, and the crude product was purified by a flash silica gel column (ethyl acetate/petroleum ether: 0-10%) to give compound 1039-1 (1700 mg, yield: 70.9%). ES-API: [M+H]⁺ = 328.0.

Step 2: Compound 1039-1 (1600 mg, 4.88 mmol), imidazole (531 mg, 7.80 mmol), and N,N-dimethylformamide (15 mL) were added to a round-bottom flask, and tert-butyldimethylsilyl chloride (882 mg, 5.85 mmol) was added thereto. The reaction system was stirred at room temperature for 16 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (ethyl acetate/petroleum ether: 0-10%) to give compound 1039-2 (2000 mg, yield: 92.7%) as a colorless oil. ES-API: [M+H]⁺ = 442.0.

Step 3: Compound 1039-2 (1900 mg, 4.30 mmol) and dichloromethane (20 mL) were added to a round-bottom flask, and m-chloroperoxybenzoic acid (1500 mg, 8.69 mmol) was added thereto. The reaction system was stirred at room temperature for 16 h. A saturated aqueous sodium bicarbonate solution was added to the reaction mixture, and the mixture was extracted with dichloromethane (50 mL × 3). The organic phase was washed with saturated brine, dried over sodium sulfate, and filtered. The filtrate was concentrated, and the crude product was purified by a flash silica gel column (ethyl acetate/petroleum ether: 0-20%) to give compound 1039-3 (900 mg, yield: 60.9%) as a yellow solid. ES-API: [M+H]⁺ = 343.9.

Step 4: Bis(triphenylphosphine)palladium(II) dichloride (122.44 mg, 0.17 mmol), copper(I) iodide (88 mg, 0.46 mmol), and N,N-diisopropylethylamine (0.6 mL, 3.43 mmol) were sequentially added to a solution of 4-(prop-2-yn-1-yl)-1,4-oxazepane (200 mg, 1.437 mmol) and compound 1039-3 (400 mg, 1.163 mmol) in acetonitrile (12 mL) at 0 °C. The reaction mixture was purged 3 times with nitrogen and stirred at room temperature for 5 h. The reaction mixture was then filtered through celite. The filtrate was concentrated. The crude product was purified by a flash silica gel column (methanol/dichloromethane: 0-10%) to give compound 1039-4 (400 mg, yield: 96.8%) as a yellow solid. ES-API: [M+H]⁺ = 355.2.

Step 5: Compound 642-5 (220 mg, 0.319 mmol), compound 1039-4 (226 mg, 0.636 mmol), [1,1'-bis(di-tert-butylphosphino)ferrocene]dichloropalladium(II) (31.15 mg, 0.048 mmol), potassium carbonate (132.25 mg, 0.957 mmol), 1,4-dioxane (3 mL), toluene (3 mL), and water (1 mL) were added to a round-bottom flask. The reaction mixture was purged 3 times with nitrogen and stirred at 70 °C for 2 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (tetrahydrofuran/petroleum ether: 0-100%) to give compound 1039-5 (100 mg, yield: 37.4%) as a yellow solid. ES-API: [M+H]⁺ = 838.2.

Step 6: Compound 1039-5 (90 mg, 0.107 mmol), cesium carbonate (70 mg, 0.215 mmol), acetonitrile (3 mL), and N,N-dimethylformamide (1 mL) were added to a round-bottom flask. The reaction system was stirred at 25 °C for 16 h. Water was added to the reaction mixture, and the mixture was extracted with ethyl acetate (50 mL × 3). The organic phase was washed with saturated brine, dried over sodium sulfate, and filtered. The filtrate was concentrated, and the residue was subjected to prep-HPLC (ammonium bicarbonate method) to give 2 isomeric compounds. The structure of one of the isomeric compounds was arbitrarily designated as 5-(3-(1,4-oxazepan-4-yl)prop-1-yn-1-yl)-3-((6³S,4S,Z)-4-((1r,2R,3S)-2,3-dimethylcyclopropane-1-carboxamido)-11-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-2-((S)-1-hydroxyethyl)pyridine-1-oxide (Z1039-1, 22 mg, yield: 23.6%, retention time: 8.84 min) as a white solid. ES-API: [M+H]⁺ = 866.3. ¹H NMR (400MHz, MeOD-*d*₄): 8.59 (s, 1H), 8.55 (d, *J*=1.2Hz, 1H), 7.80-7.72 (m, 2H), 7.59 (s, 1H), 7.50 (d, *J*=8.8Hz, 1H), 5.68 (d, *J*=8.8Hz, 1H), 4.87-4.71 (m, 2H), 4.50-4.40 (m, 1H), 4.33-4.25 (m, 1H), 4.18-4.12 (m, 1H), 4.02-3.95 (m, 1H), 3.85-3.70 (m, 7H), 3.68-3.65 (m, 1H), 3.48-3.44 (m, 1H), 3.33-3.21 (m, 2H), 2.91-2.88 (m, 4H), 2.82-2.76 (m, 1H), 2.46 (d, *J*=14.4Hz, 1H), 2.31-2.24 (m, 1H), 2.01-1.95 (m, 3H), 1.90-1.84 (m, 1H), 1.68-1.58 (m, 1H), 1.50-1.30 (m, 8H), 1.20-1.18 (m, 4H), 1.15 (d, *J*=6.0Hz, 3H), 1.07 (s, 3H), 0.55 (s, 3H).

The structure of the other isomeric compound was arbitrarily designated as 5-(3-(1,4-oxazepan-4-yl)prop-1-yn-1-yl)-3-((6³S,4S,Z)-4-((1r,2R,3S)-2,3-dimethylcyclopropane-1-carboxamido)-11-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-2-((S)-1-hydroxyethyl)pyridine-1-oxide (Z1039, 13 mg, yield: 14.0%, retention time: 9.32 min) as a white solid. ES-API: [M+H]⁺ = 866.3. ¹H NMR (400MHz, MeOD-*d*₄): 8.57 (d, *J*=1.0Hz, 1H), 8.50 (d, *J*=1.2Hz, 1H), 7.74 (dd, *J=*8.4 Hz, 1.6Hz, 1H), 7.64 (d, *J*=1.6Hz, 1H), 7.56 (s, 1H), 7.49 (d, *J*=8.4Hz, 1H), 5.78-5.72 (m, 1H), 4.80-4.76 (m, 1H), 4.46-4.38 (m, 1H), 4.34-4.27 (m, 1H), 4.22-4.16 (m, 2H), 3.82-3.67 (m, 9H), 3.44-3.40 (m, 1H), 3.27-3.24 (m, 1H), 3.16-3.12 (m, 1H), 2.90-2.85 (m, 4H), 2.80-2.74 (m, 1H), 2.71-2.67 (m, 1H), 2.22-2.14 (m, 1H), 2.00-1.92 (m, 3H), 1.82-1.70 (m, 1H), 1.62-1.31 (m, 8H), 1.15-1.11 (m, 8H), 1.01 (s, 3H), 0.61 (s, 3H).

### Example 179. Synthesis of Compound Z1065

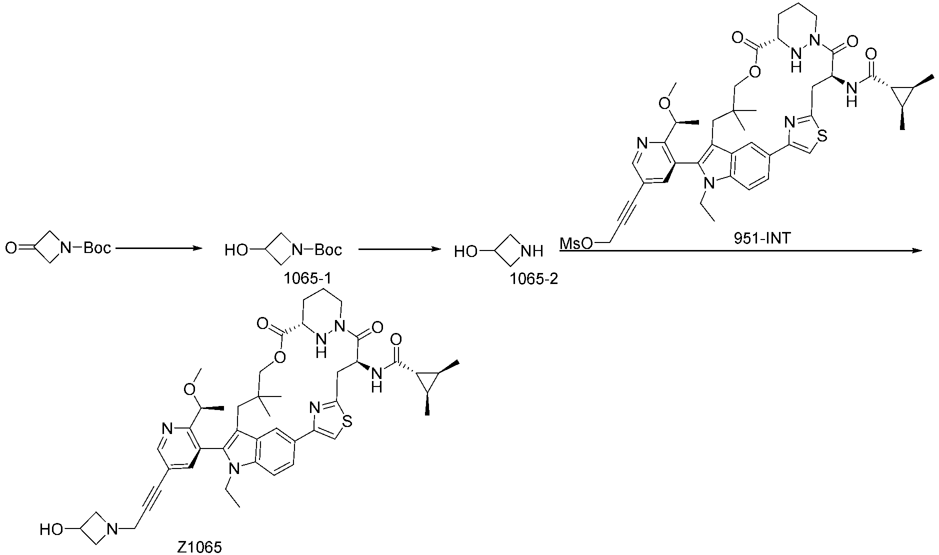

Step 1: tert-Butyl 3-oxoazetidine-1-carboxylate (200 mg, 1.169 mmol) was dissolved in a solution of tetrahydrofuran (3 mL) and methanol (3 mL) at 0 °C, and acetic acid (1 mL) was added to the reaction mixture. The reaction mixture was then stirred at 0 °C for 1 h. Sodium cyanoborohydride (76.5 mg, 1.183 mmol) was then added to the solution described above, and the reaction mixture was slowly warmed to room temperature and stirred overnight. When the reaction was completed as detected by LCMS, the reaction mixture was concentrated to dryness under reduced pressure. The residue was purified by automated flash chromatography on silica gel (methanol:dichloromethane = 0-6%) to give compound 1065-1 (100 mg, yield: 49.5%). ES-API: [M-56+H]⁺ = 118.2.

Step 2: Trifluoroacetic acid (1 mL) was slowly added dropwise to a solution of compound 1065-1 (100.0 mg, 0.578 mmol) in dichloromethane (5 mL) at room temperature. The reaction mixture was stirred at room temperature for 1 h. When the reaction was completed as detected by LCMS, the mixture was concentrated under reduced pressure to give crude compound 1065-2 (120 mg, crude product). ES-API: [M+H]⁺ = 74.1.

Step 3: Potassium carbonate (48 mg, 0.349 mmol), sodium iodide (17 mg, 0.116 mmol), and compound 1065-2 (120 mg, crude product) were sequentially added to a solution of compound 951-INT (150 mg, 0.175 mmol) in acetonitrile (6 mL). The reaction mixture was heated to 70 °C and stirred for 3 h. When the reaction was completed as detected by LCMS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The residue was purified by HPLC (formic acid method 1) to give the desired compound (2R,3S)-N-((6³S,4S,Z)-1¹-ethyl-1²-(5-(3-(3-hydroxyazetidin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxo-2(4,2)-thiazola-1(5,3)-indola-6(1,3)-pyridazinacycloundecaphane-4-yl)-2,3-dimethylcyclopropane-1-carboxamide formate (Z1065, 57.0 mg, yield: 31%) as a white solid. ES-API: [M+H]⁺ = 863.2. ¹H NMR (400 MHz, DMSO-d₆) δ 8.84 (d, J= 2.0 Hz, 1 H), 8.50 (s, 1 H), 8.38 (d, J= 9.2 Hz, 1 H), 7.89 (d, J= 2.0 Hz, 1 H), 7.82 (s, 1 H), 7.77- 7.74 (m, 1 H), 7.58 (d, J= 8.8 Hz, 1 H), 5.58- 5.49 (m, 2 H), 5.06 (d, J= 12.4 Hz, 1 H), 4.36- 4.05 (m, 6 H), 3.71- 3.68 (m, 4 H), 3.58 (s, 2 H), 3.25 (s, 3 H), 3.22- 3.12 (m, 4 H), 2.98- 2.94 (m, 1 H), 2.79- 2.72 (m, 1 H), 2.41- 2.32 (m, 1 H), 2.09- 2.06 (m, 1 H), 1.78- 1.72 (m, 2 H), 1.55- 1.48 (m, 1 H), 1.36 (d, J= 6.0 Hz, 3 H), 1.24- 1.14 (m, 3 H), 1.09- 1.06 (m, 6 H), 0.91- 0.87 (m, 6 H), 0.35 (s, 3 H).

### Example 180. Synthesis of Compound Z1091

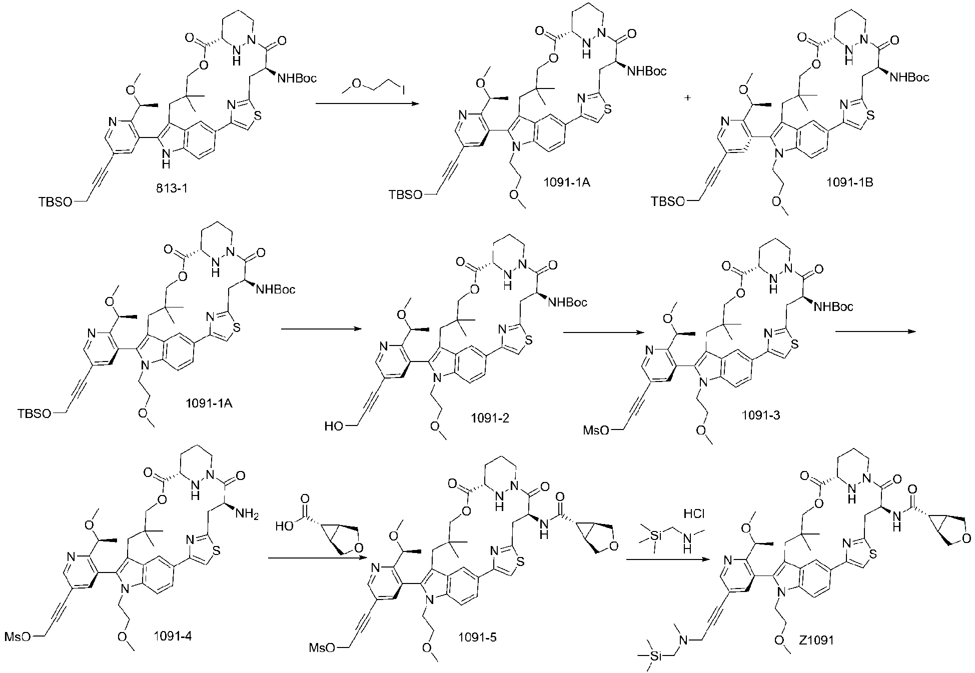

Step 1: Cesium carbonate (1346 mg, 4.130 mmol) and 1-iodo-2-methoxyethane (461 mg, 2.479 mmol) were sequentially added to a solution of compound 813-1 (720 mg, 0.826 mmol) in N,N-dimethylformamide (10 mL). The reaction mixture was stirred at 25 °C overnight. When the reaction was completed as detected by LCMS, the reaction mixture was diluted with 50 mL of ethyl acetate and then washed with saturated brine (15 mL × 3). The organic phase was dried over anhydrous sodium sulfate, filtered, and concentrated to dryness. The crude product was purified by column chromatography (ethyl acetate/petroleum ether = 1:1) to give 2 isomeric compounds. The structure of one of the isomeric compounds was isomer 1: tert-butyl ((6³S,4S,Z)-12-(5-(3-((tert-butyldimethylsilyl)oxy)prop-1-yn-1-yl)-(Rₐ)-2-((S)-1-methoxyethyl)pyridin-3-yl)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)carbamate (1091-1A, 232 mg, yield: 30%, Rf = 0.5), ES-API: [M+H]⁺ = 929.3; the structure of the other isomeric compound was isomer 2: tert-butyl ((6³S,4S,Z)-12-(5-(3-((tert-butyldimethylsilyl)oxy)prop-1-yn-1-yl)-(Sₐ)-2-((S)-1-methoxyethyl)pyridin-3-yl)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)carbamate (1091-1B, 480 mg, yield: 62%, Rf = 0.4), ES-API: [M+H]⁺ = 929.3.

Step 2: A tetrabutylammonium fluoride solution (0.75 mL, 0.750 mmol) was added to a solution of isomer 1 (1091-1A, 656 mg, 0.688 mmol) in tetrahydrofuran (15 mL) under an ice-water bath. The reaction mixture was stirred at 0 °C for 1 h. When the reaction was completed as detected by LCMS, the reaction mixture was diluted with 30 mL of ethyl acetate and then washed with saturated brine (15 mL × 3). The organic phase was dried over anhydrous sodium sulfate, filtered, and concentrated to dryness to give crude compound 1091-2 (203 mg, yield: 99%). ES-API: [M+H]+ = 815.3

Step 3: N,N-Diisopropylethylamine (0.22 mL, 1.245 mmol) and methanesulfonic anhydride (59 mg, 0.336 mmol) were sequentially added to a solution of compound 1091-2 (203 mg, 0.249 mmol) in dichloromethane (20 mL) under an ice-water bath. The reaction mixture was stirred at room temperature for 1 h. When the reaction was completed as detected by LCMS, the reaction mixture was diluted with 20 mL of dichloromethane and then washed with water (10 mL) and saturated brine (15 mL × 3). The organic phase was dried over anhydrous sodium sulfate, filtered, and concentrated to give crude compound 1091-3 (222 mg, yield: 99%). ES-API: [M+H]⁺ = 893.3.

Step 4: A hydrogen chloride/dioxane solution (5 mL, 4 M) was added to a solution of compound 1091-3 (222 mg, 0.249 mmol) in methanol (1 mL). The reaction mixture was stirred at room temperature for 1 h. When the reaction was completed as detected by LCMS, the mixture was concentrated under reduced pressure to give crude compound 1091-4 (197 mg, yield: 99%). ES-API: [M+H]⁺ = 793.2.

Step 5: (1R,5S,6r)-3-Oxabicyclo[3.1.0]hexane-6-carboxylic acid (48 mg, 0.373 mmol), N,N-diisopropylethylamine (0.26 mL, 1.488 mmol), and O-(7-azabenzotriazol-1-yl)-N,N,N',N'-tetramethyluronium hexafluorophosphate (141 mg, 0.372 mmol) were sequentially added to a solution of compound 1091-4 (197 mg, 0.248 mmol) in N,N-dimethylformamide (6 mL) under an ice-water bath. The reaction mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by automated flash chromatography on silica gel (methanol/dichloromethane = 0-2%) to give compound 1091-5 (170 mg, yield: 76%). ES-API: [M+H]⁺ = 903.2.

Step 6: Potassium carbonate (46 mg, 0.333 mmol), sodium iodide (17 mg, 0.111 mmol), and methyl[(trimethylsilyl)methyl]amine hydrochloride (20 mg, 0.167 mmol) were sequentially added to a solution of compound 1091-5 (100 mg, 0.111 mmol) in acetonitrile (10 mL). The reaction mixture was heated to 50 °C and stirred for 2 h. When the reaction was completed as detected by LCMS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The residue was purified by HPLC (formic acid method 1) to give the desired compound (1R,5S,6r)-N-((6³S,4S,Z)-1¹-(2-methoxyethyl)-1²-(Rₐ)-(2-((S)-1-methoxyethyl)-5-(3-(methyl((trimethylsilyl)methyl)amino)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z1091, 56.20 mg, Y: 55%) as a white solid. ES-API: [M+H]⁺ = 924.2. ¹H NMR (400 MHz, DMSO-d₆) δ 8.78 (d, J= 2.0 Hz, 1 H), 8.56 (d, J= 8.8 Hz, 1 H), 8.49 (s, 1 H), 7.82 (s, 1 H), 7.76- 7.73 (m, 1 H), 7.72 (d, J= 2.0 Hz, 1 H), 7.60 (d, J= 8.4 Hz, 1 H), 5.56 (t, J= 8.8 Hz, 1 H), 5.08 (d, J= 12.4 Hz, 1 H), 4.48- 4.43 (m, 1 H), 4.29- 4.18 (m, 4 H), 3.83 (t, J= 8.4 Hz, 2 H), 3.66- 3.62 (m, 2 H), 3.58- 3.54 (m, 4 H), 3.35- 3.30 (m, 2 H), 3.29- 3.23 (m, 4 H), 3.16- 3.10 (m, 1 H), 2.97-2.93 (m, 1 H), 2.89 (s, 3 H), 2.79- 2.72 (m, 1 H), 2.40- 2.37 (m, 1 H), 2.28 (s, 3 H), 2.09- 2.06 (m, 1 H), 2.01 (s, 2 H), 1.96-1.88 (m, 2 H), 1.80- 1.75 (m, 2 H), 1.66- 1.65 (m, 1 H), 1.56- 1.48 (m, 1 H), 1.35 (d, J= 6.0 Hz, 3 H), 0.91 (s, 3 H), 0.35 (s, 3 H), 0.04 (s, 9 H).

### Example 181. Synthesis of Compound Z1055

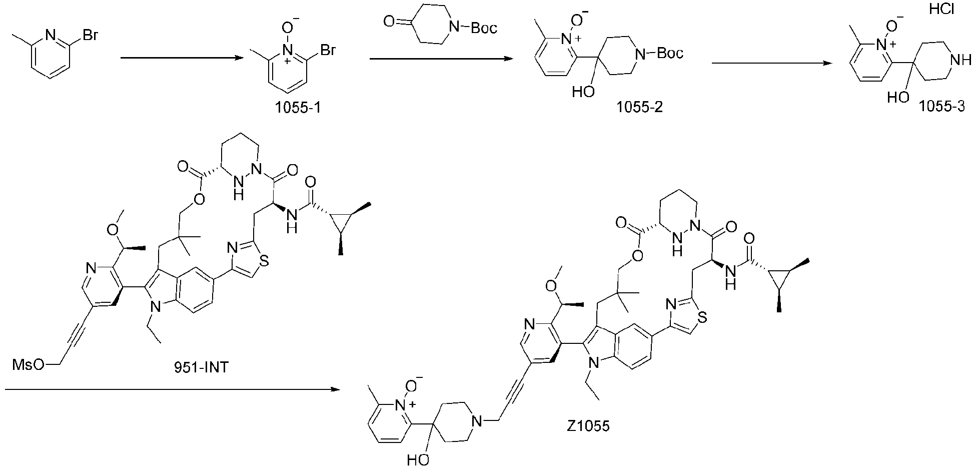

Step 1: 2-Bromo-6-methylpyridine (4.5 g, 26.158 mmol) was dissolved in dichloromethane (40 mL) at room temperature, and m-chloroperoxybenzoic acid (7.967 g, 39.237 mmol) was added to the reaction mixture. The reaction mixture was then heated to 65 °C and refluxed overnight. When the reaction was completed as detected by LCMS, the reaction mixture was cooled to 0 °C, stirred for 3 h, and filtered to remove insoluble substances. The filtrate was concentrated under reduced pressure to remove the solvent. A sodium hydroxide solution (30 mL, 2 M) was added to the residue, and then the mixture was extracted with dichloromethane (20 mL × 3). The organic phases were combined, dried over anhydrous sodium sulfate, filtered, and concentrated to give crude compound 1055-1 (4.6 g, yield: 94%). ES-API: [M+H]⁺ = 188.1.

Step 2: An isopropylmagnesium chloride-lithium chloride complex solution (20.5 mL, 26.593 mmol, 1.3 M) was slowly added dropwise to a solution of compound 1055-1 (2 g, 10.637 mmol) in anhydrous tetrahydrofuran (50 mL) at -40 °C. The reaction mixture was stirred for 30 min under a nitrogen atmosphere. A solution of tert-butyl 4-oxopiperidine-1-carboxylate (2.225 g, 11.169 mmol) in tetrahydrofuran (10 mL) was then slowly added dropwise to the solution described above. The reaction mixture was stirred at -40 °C for another 2 h. When the reaction was completed as detected by LCMS, saturated ammonium chloride (50 mL) was slowly added to the reaction mixture to quench the reaction. The mixture was then extracted with ethyl acetate (30 mL × 3). The organic phases were combined, washed with saturated brine (30 mL × 3), dried over anhydrous sodium sulfate, filtered, and concentrated to dryness. The residue was purified by automated flash chromatography on silica gel (methanol:dichloromethane = 0-6%) to give compound 1055-2 (1.494 g, yield: 46%). ES-API: [M+H]⁺ = 309.3.

Step 3: A solution of hydrochloric acid in dioxane (5 mL, 4 M) was added to a solution of compound 1055-2 (150 mg, 0.486 mmol) in methanol (1 mL). The reaction mixture was stirred at room temperature for 1 h. When the reaction was completed as detected by LCMS, the mixture was concentrated under reduced pressure to give crude compound 1055-3 (101 mg). ES-API: [M+H]⁺ = 209.2.

Step 4: Potassium carbonate (80 mg, 0.582 mmol), sodium iodide (17 mg, 0.116 mmol), and 1055-3 (72 mg, 0.349 mmol) were sequentially added to a solution of compound 951-INT (100 mg, 0.116 mmol) in acetonitrile (10 mL). The reaction mixture was heated to 40 °C and stirred for 3 h. When the reaction was completed as detected by LCMS, the mixture was filtered to remove insoluble substances, and the filtrate was concentrated to dryness. The residue was purified by HPLC (formic acid method 1) to give the desired compound 2-(1-(3-(5-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-12-yl)-(Ra)-6-((S)-1-methoxyethyl)pyridin-3-yl)prop-2-yn-1-yl)-4-hydroxypiperidin-4-yl)-6-methylpyridine 1-oxide (Z1055, 30 mg, yield: 27%) as a white solid. ES-API: [M+H]⁺ = 971.0.

### Example 182. Synthesis of Control Compound 1

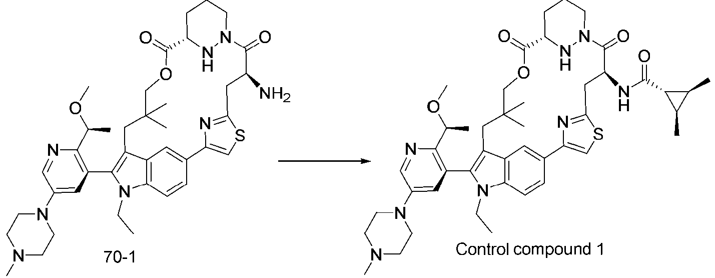

Step 1: Compound 70-1 (230 mg, 0.32 mmol) was dissolved in dichloromethane (15 mL), and (1r,2R,3S)-2,3-dimethylcyclopropane-1-carboxylic acid (36 mg, 0.32 mmol), O-(7-azabenzotriazol-1-yl)-N,N,N',N'-tetramethyluronium hexafluorophosphate (240 mg, 0.63 mmol), and N,N'-diisopropylethylamine (245 mg, 1.89 mmol) were added. The reaction system was stirred at room temperature for 1 h. Water (20 mL) was added to the reaction mixture, and the mixture was extracted with ethyl acetate (60 mL). The organic phase was washed with saturated brine (20 mL), dried over anhydrous sodium sulfate, and filtered, and the filtrate was concentrated under reduced pressure. The crude product was subjected to prep-HPLC (ammonium bicarbonate method) to give the desired product (1r,2R,3S)-N-((6³S,Z)-1¹-ethyl-(Ra)-1²-(2-((S)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (control compound 1, 160 mg, yield: 61.5%) as a white solid. 1H NMR (400 MHz, DMSO-d6) δ 8.48 (d, J = 1.2 Hz, 1H), 8.44 (d, J = 2.8 Hz, 1H), 8.38 (d, J = 8.8 Hz, 1H), 7.79 (s, 1H), 7.72 (dd, J = 8.8, 1.2 Hz, 1H), 7.55 (d, J = 8.8 Hz, 1H), 7.21 (d, J = 2.8 Hz, 1H), 5.56 (t, J = 9.2 Hz, 1H), 5.06 (d, J = 12.0 Hz, 1H), 4.35- 4.02 (m, 5H), 3.57 (s, 2H), 3.34- 3.29 (m, 1H), 3.28- 3.23 (m, 4H), 3.20 (s, 3H), 3.18- 3.10 (m, 1H), 2.92 (d, J = 14.4 Hz, 1H), 2.81- 2.69 (m, 1H), 2.47- 2.41 (m, 4H), 2.22 (s, 3H), 2.13- 2.02 (m, 1H), 1.85- 1.69 (m, 2H), 1.60- 1.43 (m, 1H), 1.33 (d, J = 6.0 Hz, 3H), 1.24- 1.11 (m, 3H), 1.11- 1.01 (m, 6H), 0.95- 0.84 (m, 6H), 0.35 (s, 3H). ES-API: [M+H]+ = 825.3.

According to the methods described in the above examples and by modifying some of the starting materials, the compounds listed in Table A below were synthesized:

**Table A**

| Structure and Number | MS [M+H]⁺ | Structure and Number | MS [M+H]⁺ | Structure and Number | MS [M+H]⁺ |
|---|---|---|---|---|---|
| 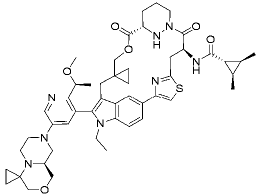 | 891.5 | 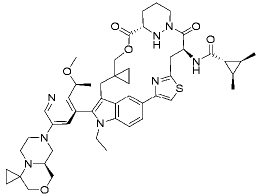 | 891.5 | 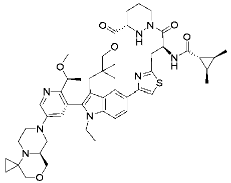 | 891.5 |
| Z6 | | Z7 | | Z8 | |
| 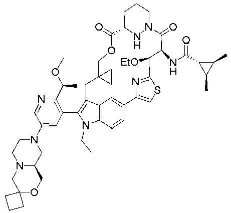 | 949.5 | 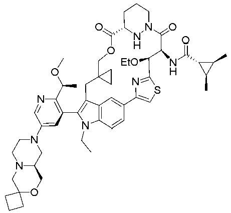 | 949.5 | 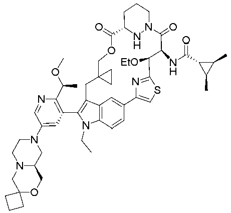 | 949.5 |
| Z9 | | Z10 | | Z11 | |
| 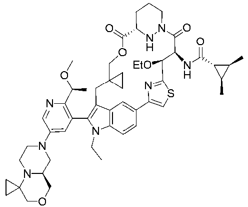 | 935.5 | 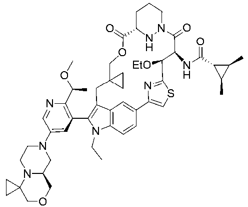 | 935.5 | 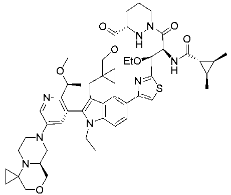 | 935.5 |
| Z15 | | Z16 | | Z17 | |
| 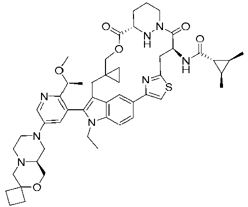 | 905.5 | 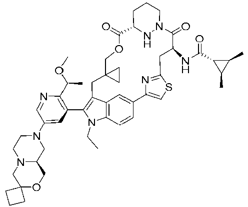 | 905.5 | 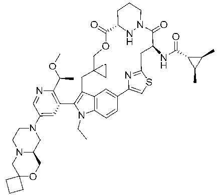 | 905.5 |
| Z18 | | Z19 | | Z20 | |
| 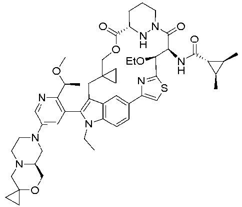 | 935.5 | 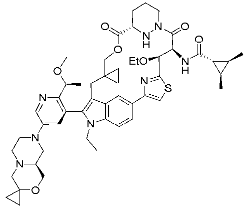 | 935.5 | 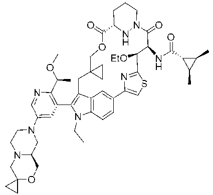 | 935.5 |
| Z21 | | Z22 | | Z23 | |
| 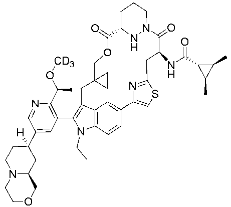 | 867.5 | 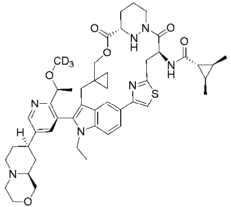 | 867.5 | 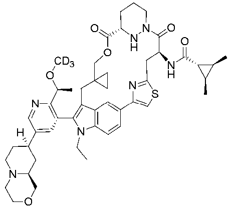 | 867.5 |
| Z24 | | Z25 | | Z26 | |
| 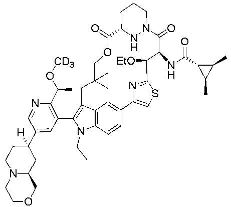 | 911.5 | 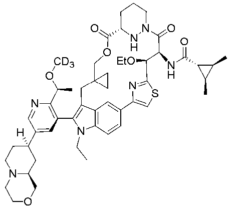 | 911.5 | 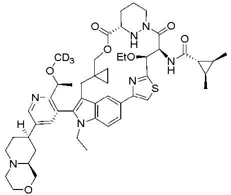 | 911.5 |
| Z27 | | Z28 | | Z29 | |
| 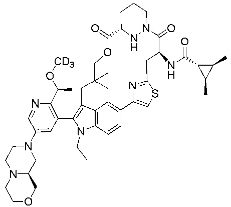 | 868.5 | 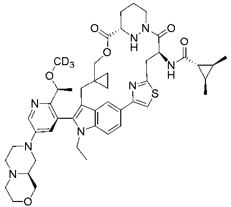 | 868.5 | 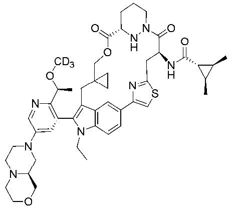 | 868.5 |
| Z30 | | Z31 | | Z32 | |
| 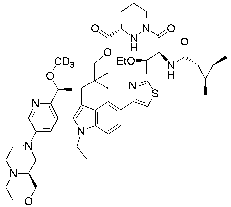 | 912.5 | 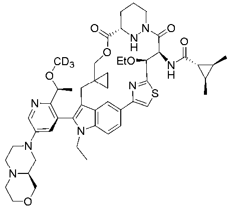 | 912.5 | 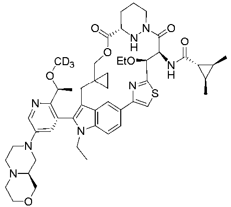 | 912.5 |
| Z33 | | Z34 | | Z35 | |
| 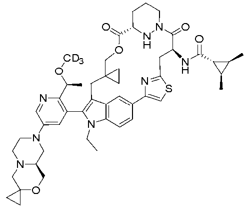 | 894.5 | 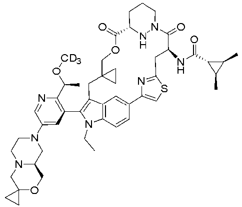 | 894.5 | 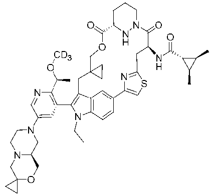 | 894.5 |
| Z36 | | Z37 | | Z38 | |
| 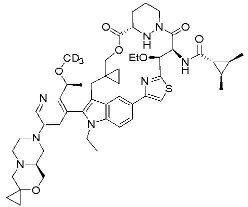 | 938.5 | 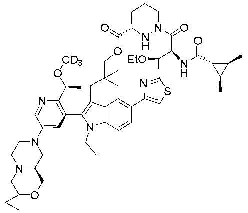 | 938.5 | 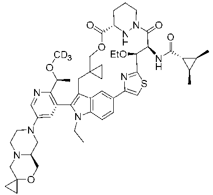 | 938.5 |
| Z39 | | Z40 | | Z41 | |
| 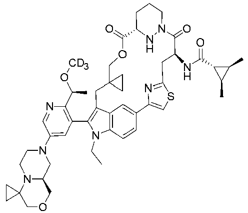 | 894.5 | 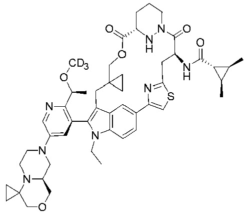 | 894.5 | 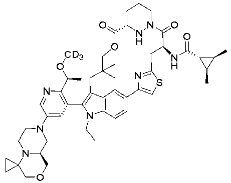 | 894.5 |
| Z42 | | Z43 | | Z44 | |
| 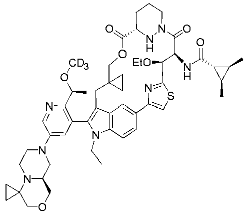 | 938.5 | 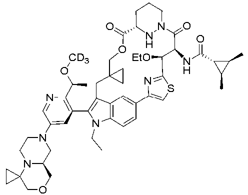 | 938.5 | 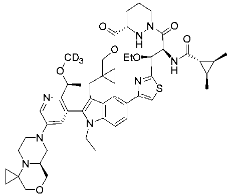 | 938.5 |
| Z45 | | Z46 | | Z47 | |
| 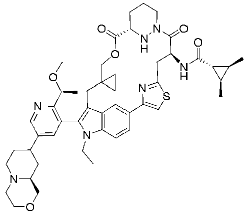 | 864.4 | 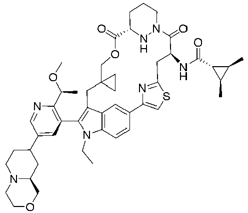 | 864.4 | 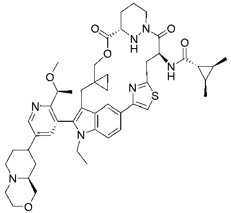 | 864.4 |
| Z48 | | Z49 | | Z50 | |
| 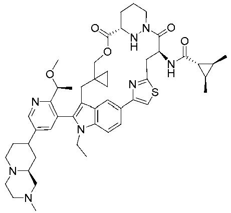 | 877.5 | 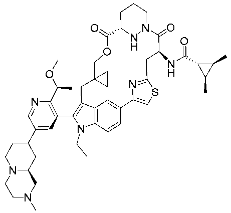 | 877.5 | 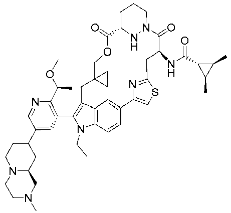 | 877.5 |
| Z51 | | Z52 | | Z53 | |
| 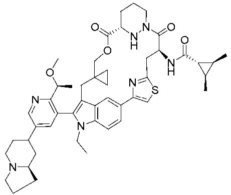 | 848.4 | 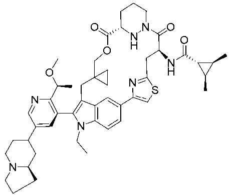 | 848.4 | 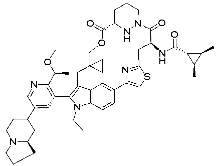 | 848.4 |
| Z54 | | Z55 | | Z56 | |
| 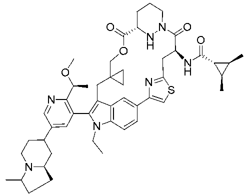 | 848.4 | 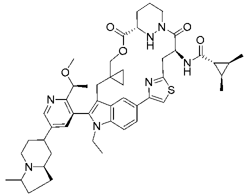 | 848.4 | 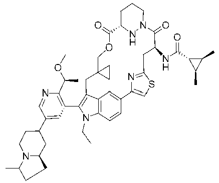 | 848.4 |
| Z57 | | Z58 | | Z59 | |
| 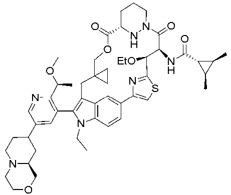 | 908.5 | 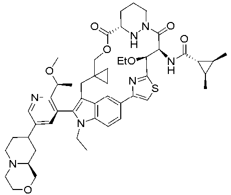 | 908.5 | 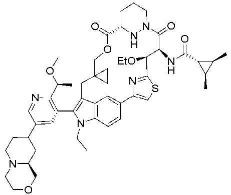 | 908.5 |
| Z60 | | Z61 | | Z62 | |
| 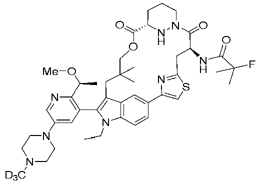 | 820.4 | 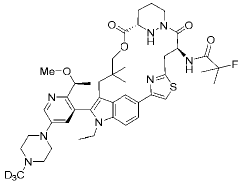 | 820.4 | 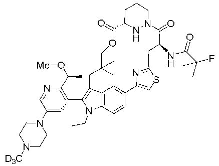 | 820.4 |
| Z66-1 | | Z67 | | Z68 | |
| 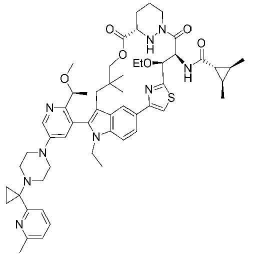 | 986.5 | 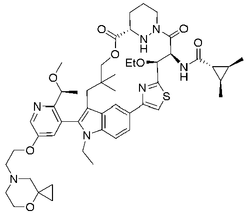 | 926.5 | 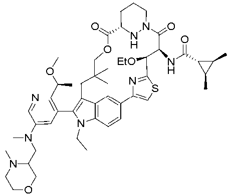 | 913.5 |
| Z82 | | Z84 | | Z85 | |
| 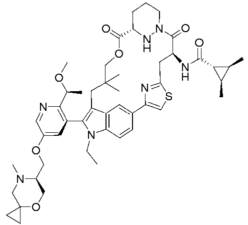 | 882.5 | 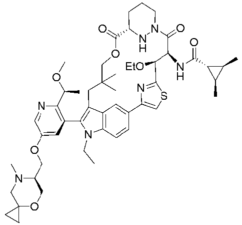 | 926.5 | 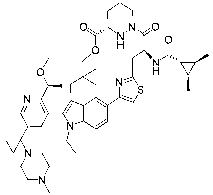 | 865.5 |
| Z86 | | Z87 | | Z88 | |
| 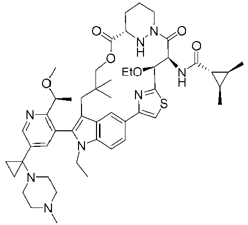 | 909.5 | 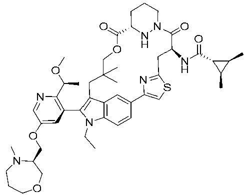 | 870.5 | 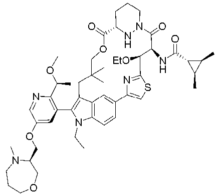 | 914.5 |
| Z89 | | Z90 | | Z91 | |
| 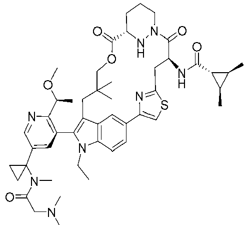 | 881.5 | 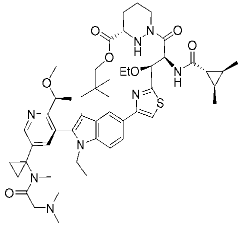 | 925.5 | 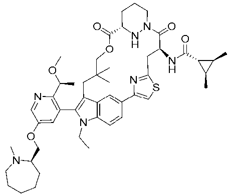 | 868.5 |
| Z92 | | Z93 | | Z94 | |
| 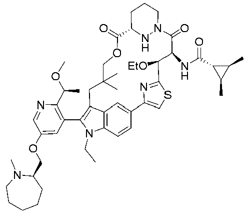 | 912.5 | 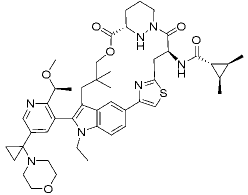 | 852.4 | 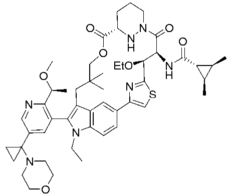 | 896.5 |
| Z95 | | Z96 | | Z97 | |
| 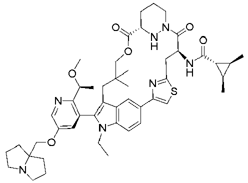 | 866.5 | 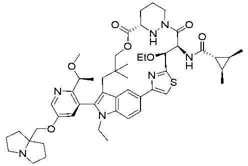 | 910.5 | 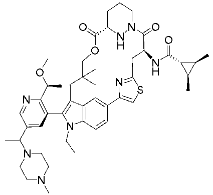 | 853.5 |
| Z98 | | Z99 | | Z100 | |
| 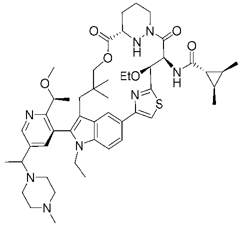 | 897.5 | 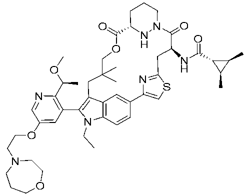 | 870.5 | 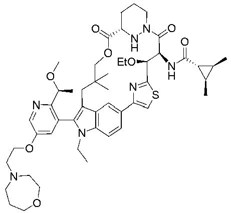 | 914.5 |
| Z101 | | Z102 | | Z103 | |
| 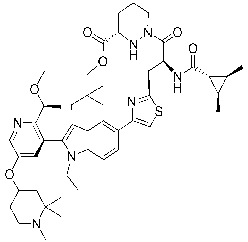 | 866.5 | 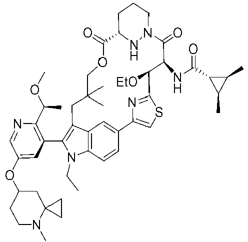 | 910.5 | 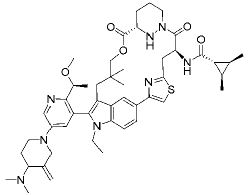 | 865.5 |
| Z104 | | Z105 | | Z106 | |
| 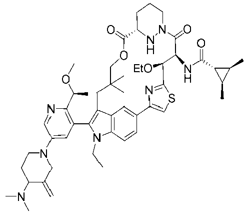 | 909.5 | 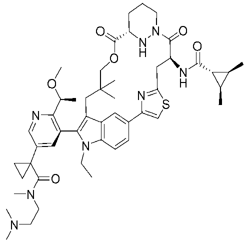 | 895.5 | 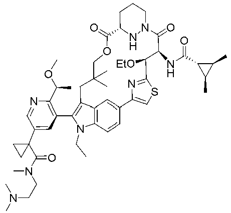 | 939.5 |
| Z107 | | Z108 | | Z109 | |
| 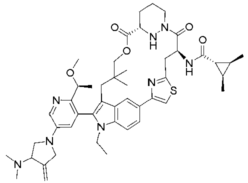 | 851.5 | 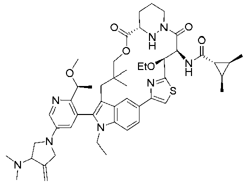 | 895.5 | 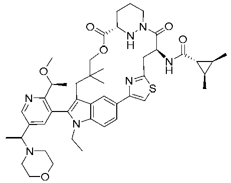 | 840.4 |
| Z110 | | Z111 | | Z112 | |
| 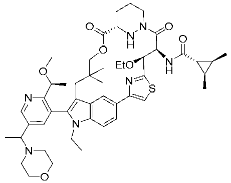 | 884.5 | 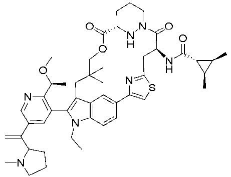 | 836.4 | 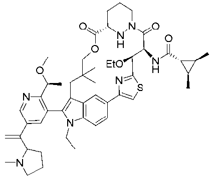 | 880.5 |
| Z113 | | Z114 | | Z115 | |
| 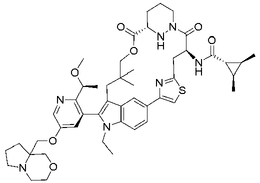 | 882.5 | 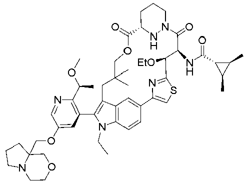 | 926.5 | 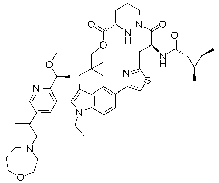 | 866.5 |
| Z116 | | Z117 | | Z118 | |
| 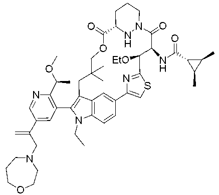 | 910.5 | 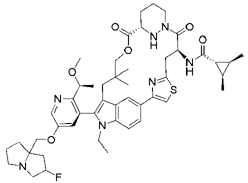 | 884.4 | 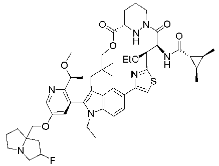 | 928.5 |
| Z119 | | Z120 | | Z121 | |
| 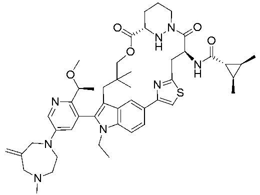 | 851.5 | 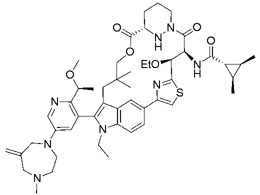 | 895.5 | 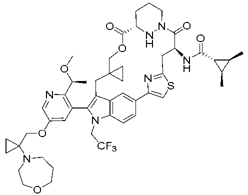 | 948.4 |
| Z122 | | Z123 | | Z330 | |
| 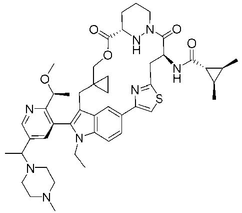 | 851.5 | 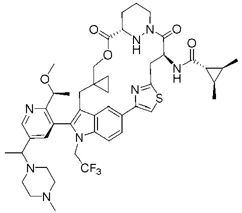 | 905.4 | 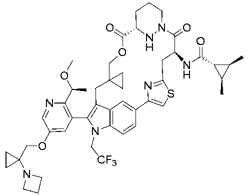 | 904.4 |
| Z331 | | Z332 | | Z334 | |
| 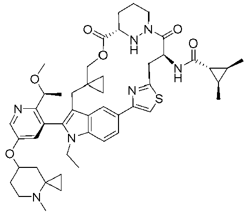 | 864.4 | 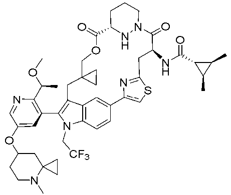 | 918.4 | 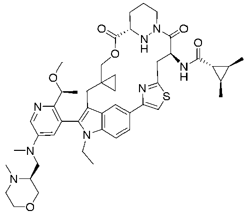 | 867.5 |
| Z335 | | Z336 | | Z337 | |
| 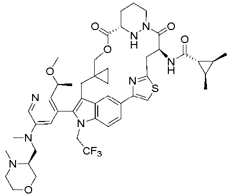 | 921.4 | 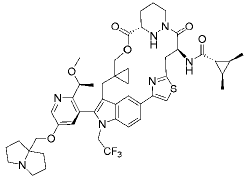 | 918.4 | 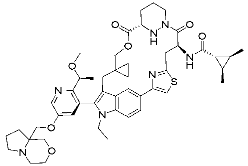 | 800.4 |
| Z338 | | Z340 | | Z341 | |
| 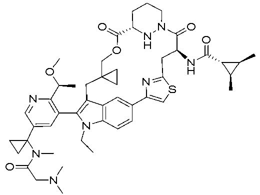 | 879.5 | 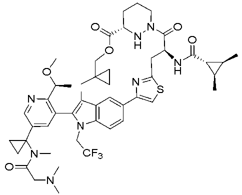 | 933.4 | 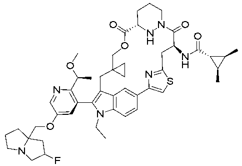 | 882.4 |
| Z343 | | Z344 | | Z345 | |
| 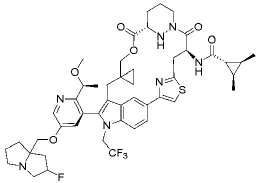 | 936.4 | 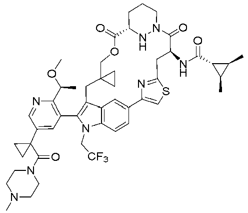 | 945.4 | 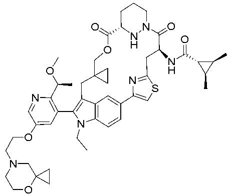 | 880.4 |
| Z346 | | Z348 | | Z349 | |
| 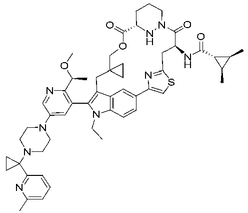 | 940.5 | 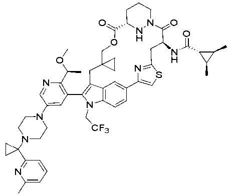 | 994.5 | 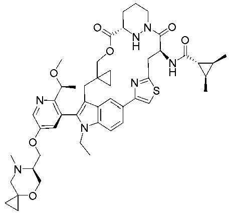 | 880.4 |
| Z351 | | Z352 | | Z353 | |
| 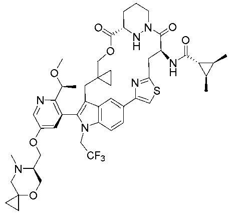 | 934.4 | 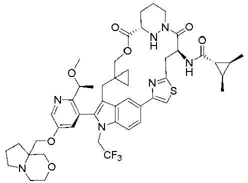 | 934.4 | 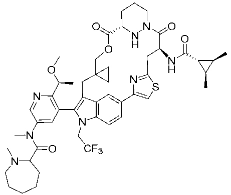 | 947.4 |
| Z354 | | Z342 | | Z356 | |
| 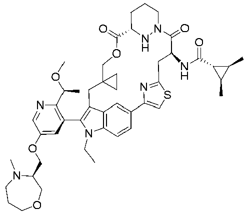 | 868.4 | 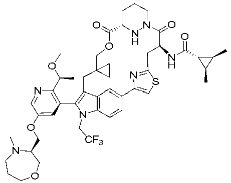 | 922.4 | 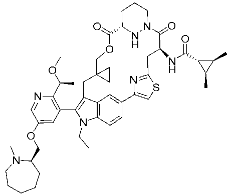 | 866.5 |
| Z357 | | Z358 | | Z359 | |
| 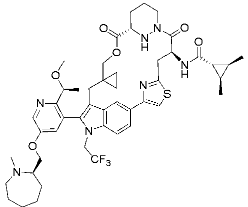 | 920.4 | 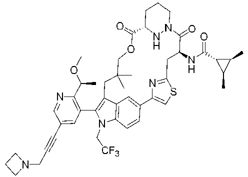 | 874.4 | 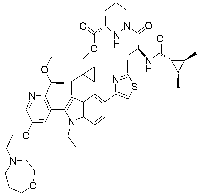 | 868.4 |
| Z360 | | Z362 | | Z363 | |
| 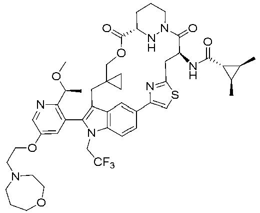 | 922.4 | 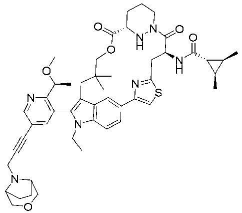 | 876.4 | 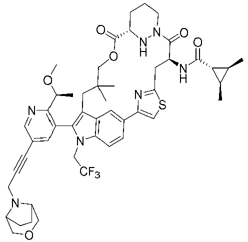 | 930.4 |
| Z364 | | Z365 | | Z366 | |
| 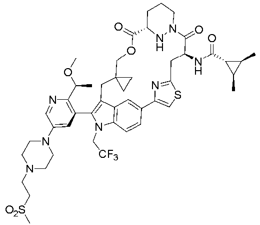 | 969.4 | 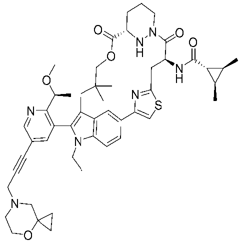 | 876.4 | 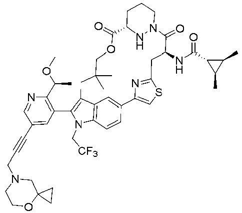 | 930.4 |
| Z368 | | Z369 | | Z370 | |
| 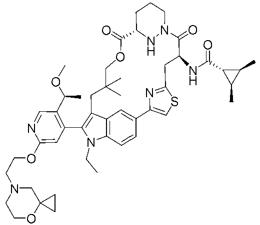 | 882.5 | 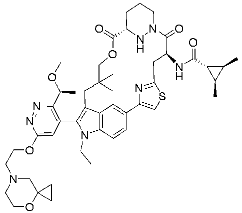 | 883.4 | | |
| Z381 | | Z382 | | | |
| 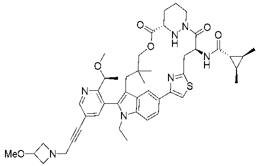 | 850.3 | 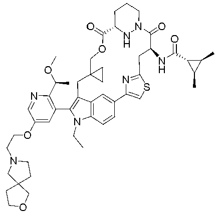 | 894.5 | 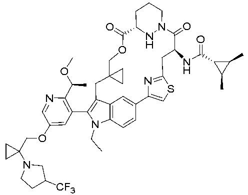 | 932.4 |
| Z384 | | Z386 | | Z387 | |
| 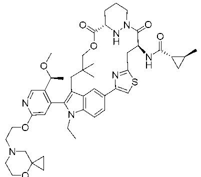 | 868.4 | 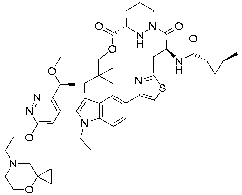 | 869.4 | 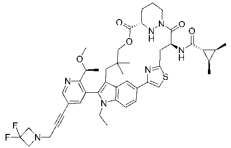 | 856.4 |
| Z388 | | Z389 | | Z390 | |
| 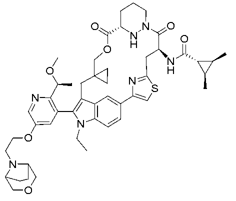 | 880.4 | 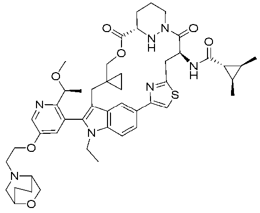 | 880.4 | 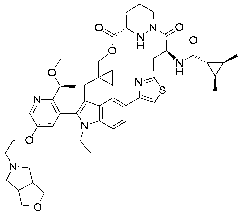 | 880.4 |
| Z391 | | Z392 | | Z393 | |
| 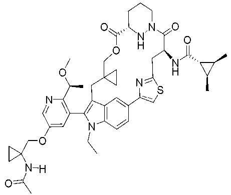 | 852.4 | 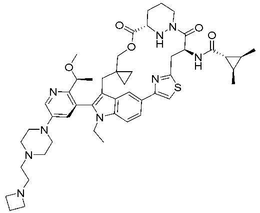 | 892.5 | 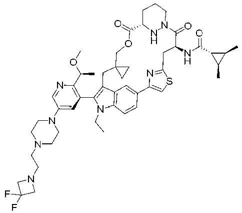 | 928.5 |
| Z394 | | Z395 | | Z396 | |
| 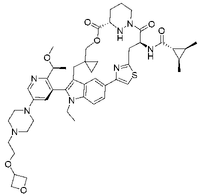 | 909.5 | 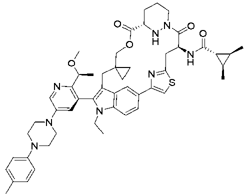 | 899.5 | 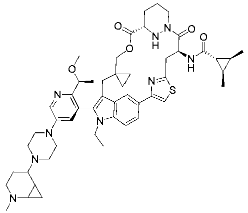 | 918.5 |
| Z397 | | Z398 | | Z399 | |
| 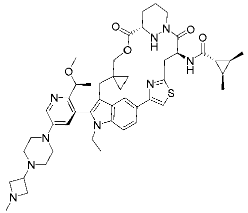 | 878.5 | 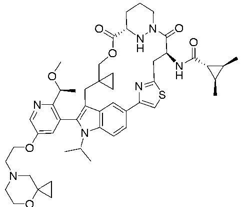 | 894.5 | 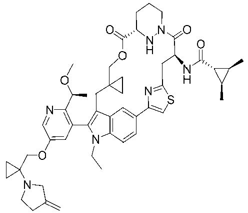 | 876.4 |
| Z400 | | Z401 | | Z403 | |
| 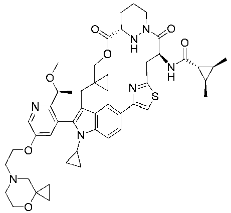 | 892.4 | 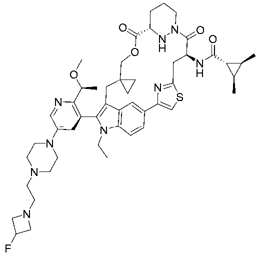 | 910.5 | 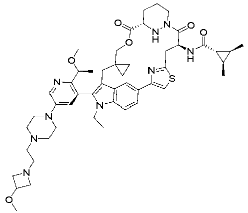 | 922.5 |
| Z402 | | Z404 | | Z405 | |
| 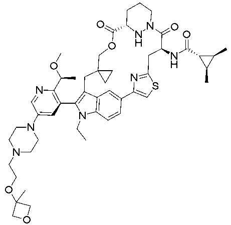 | 923.5 | 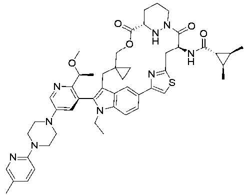 | 900.5 | 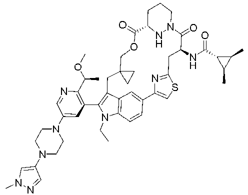 | 889.4 |
| Z406 | | Z407 | | Z408 | |
| 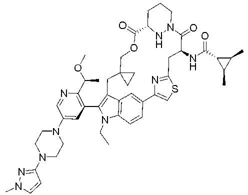 | 889.4 | 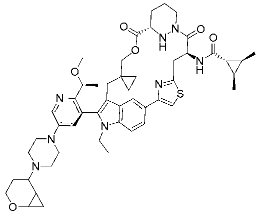 | 905.5 | 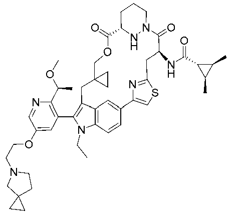 | 864.4 |
| Z409 | | Z410 | | Z411 | |
| 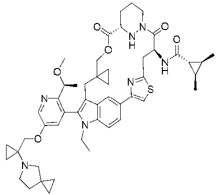 | 890.5 | 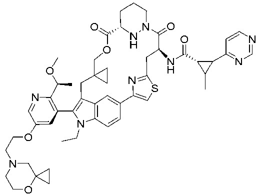 | 944.4 | 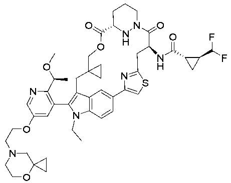 | 902.4 |
| Z412 | | Z413 | | Z414 | |
| 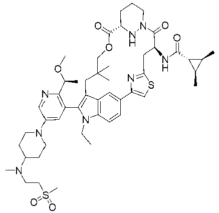 | 945.4 | 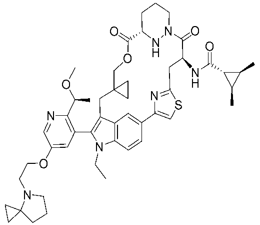 | 864.4 | 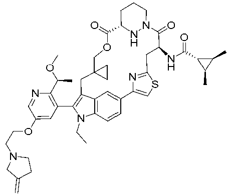 | 850.4 |
| Z416 | | Z419 | | Z420 | |
| 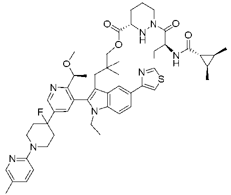 | 919.5 | 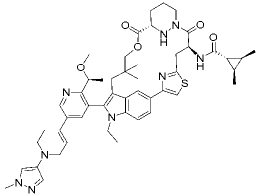 | 888.5 | 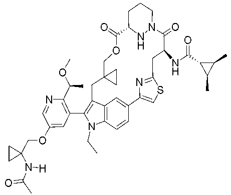 | 852.4 |
| Z438 | | Z439 | | Z440 | |
| 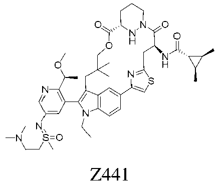 | 875.4 | 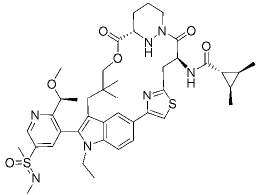 | 818.4 | 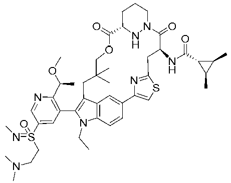 | 875.4 |
| | | Z442 | | Z443 | |
| 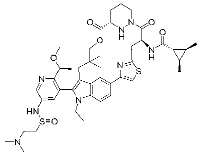 | 861.4 | 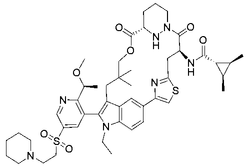 | 902.4 | 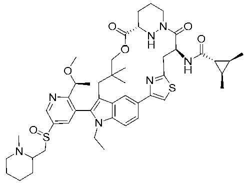 | 886.4 |
| Z444 | | Z445 | | Z446 | |
| 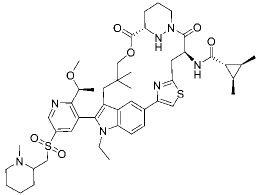 | 902.4 | 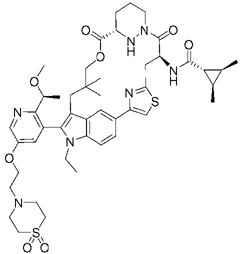 | 904.4 | 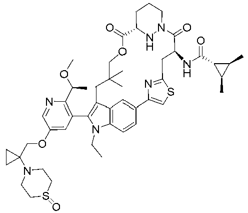 | 914.4 |
| Z447 | | Z448 | | Z450 | |
| 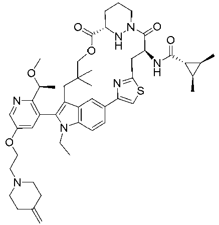 | 866.5 | 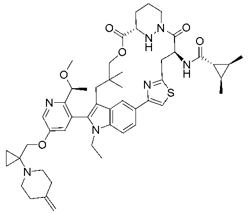 | 892.5 | 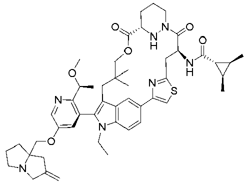 | 878.5 |
| Z451 | | Z452 | | Z453 | |
| 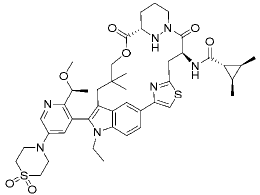 | 860.4 | 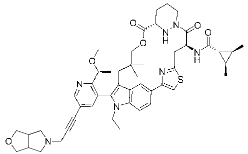 | 876.4 | 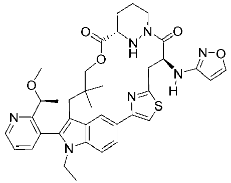 | 698.3 |
| Z454 | | Z455 | | Z124 | |
| 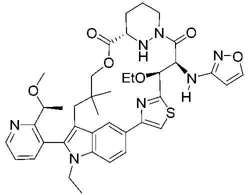 | 742.3 | 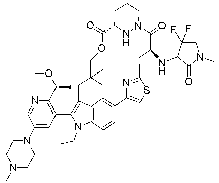 | 862.4 | 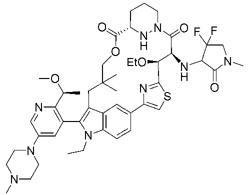 | 906.4 |
| Z125 | | Z126 | | Z127 | |
| 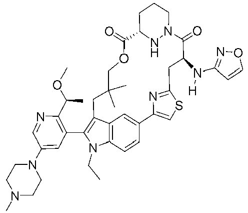 | 796.4 | 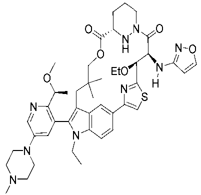 | 840.4 | 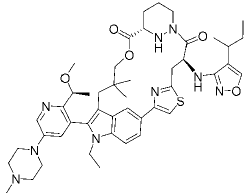 | 852.5 |
| Z128 | | Z129 | | Z130 | |
| 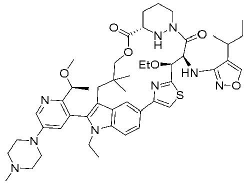 | 896.5 | 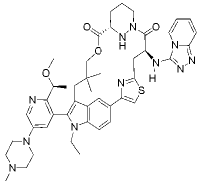 | 846.4 | 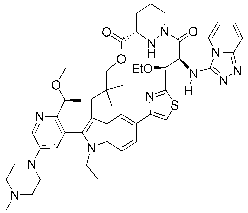 | 890.4 |
| Z131 | | Z132 | | Z133 | |
| 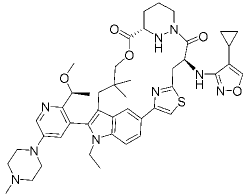 | 836.4 | 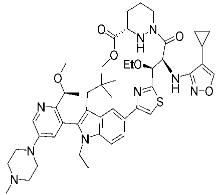 | 880.4 | 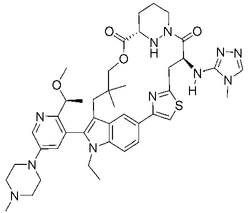 | 810.4 |
| Z134 | | Z135 | | Z136 | |
| 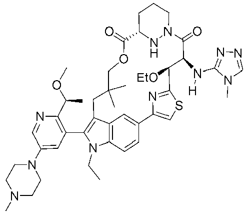 | 854.4 | 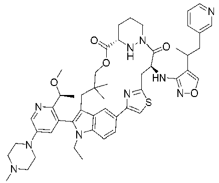 | 915.5 | 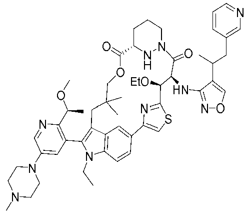 | 959.5 |
| Z137 | | Z138 | | Z139 | |
| 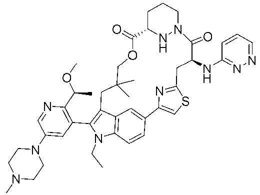 | 807.4 | 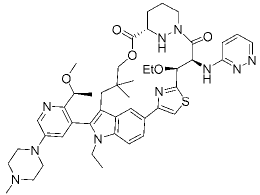 | 851.4 | 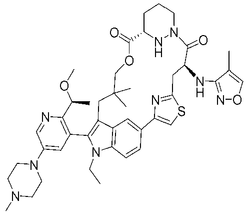 | 810.4 |
| Z140 | | Z141 | | Z142 | |
| 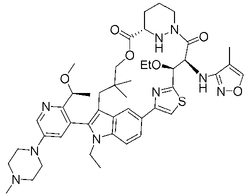 | 854.4 | 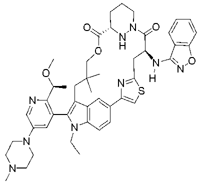 | 846.4 | 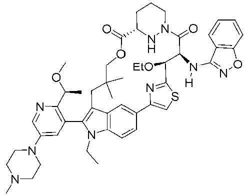 | 890.4 |
| Z143 | | Z144 | | Z145 | |
| 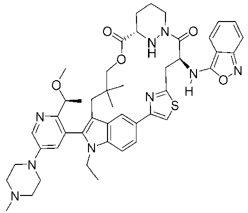 | 846.4 | 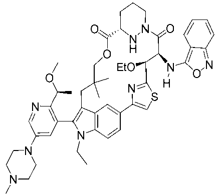 | 890.4 | 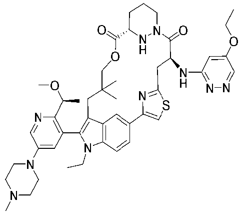 | 851.4 |
| Z146 | | Z147 | | Z148 | |
| 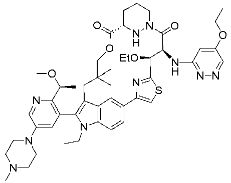 | 895.5 | 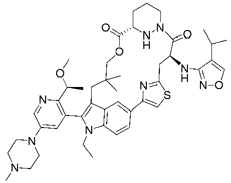 | 838.4 | 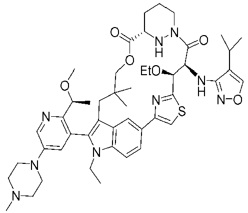 | 882.5 |
| Z149 | | Z150 | | Z151 | |
| 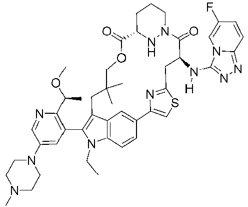 | 864.4 | 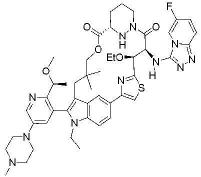 | 908.4 | 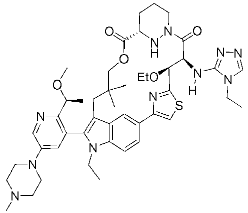 | 868.5 |
| Z152 | | Z153 | | Z154 | |
| 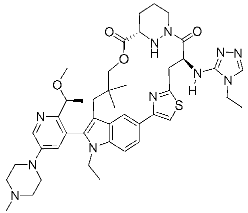 | 824.4 | 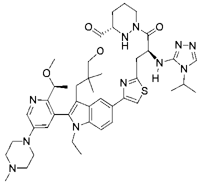 | 838.4 | 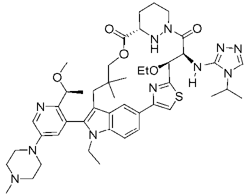 | 882.5 |
| Z155 | | Z156 | | Z157 | |
| 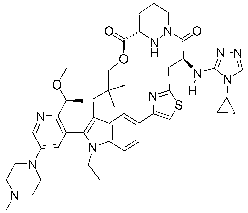 | 836.4 | 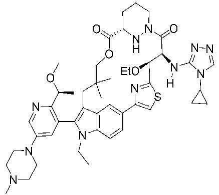 | 880.5 | 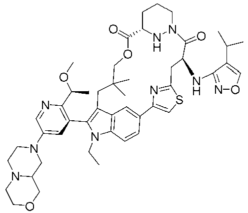 | 880.4 |
| Z158 | | Z159 | | Z160 | |
| 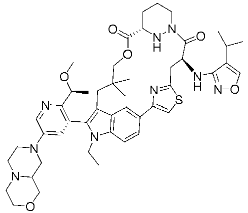 | 880.4 | 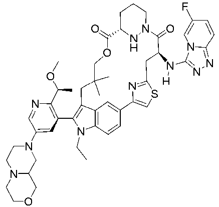 | 906.4 | 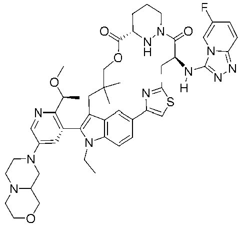 | 906.4 |
| Z161 | | Z162 | | Z163 | |
| 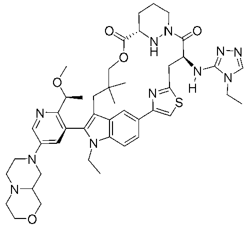 | 866.4 | 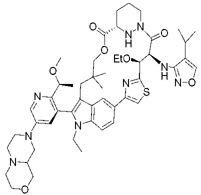 | 924.5 | 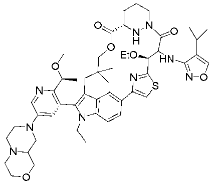 | 924.5 |
| Z164 | | Z165 | | Z166 | |
| 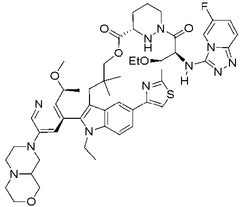 | 950.4 | 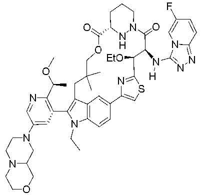 | 950.4 | 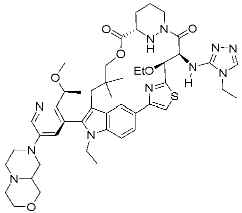 | 910.5 |
| Z167 | | Z168 | | Z169 | |
| 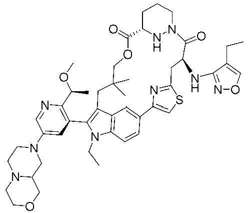 | 866.4 | 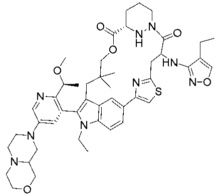 | 866.4 | 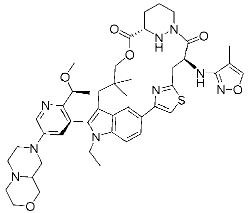 | 852.4 |
| Z170 | | Z171 | | Z172 | |
| 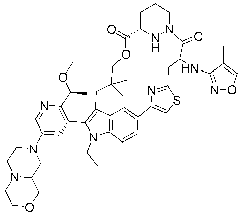 | 852.4 | 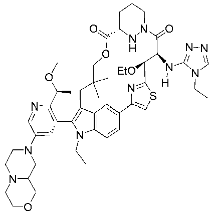 | 910.5 | 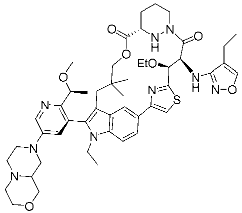 | 910.5 |
| Z173 | | Z174 | | Z175 | |
| 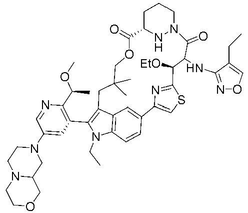 | 910.5 | 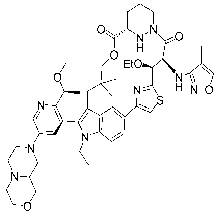 | 896.4 | 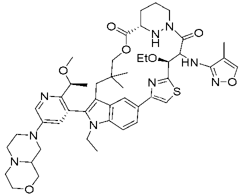 | 896.4 |
| Z176 | | Z177 | | Z178 | |
| 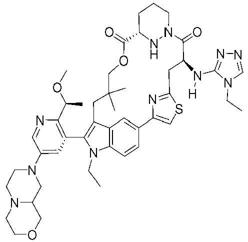 | 866.4 | 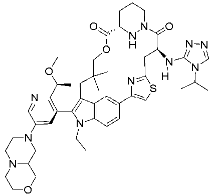 | 880.5 | 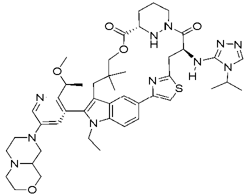 | 880.5 |
| Z179 | | Z180 | | Z181 | |
| 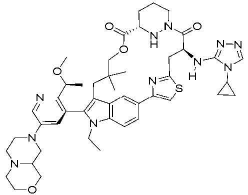 | 878.4 | 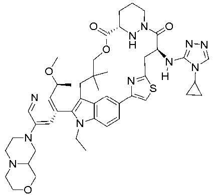 | 878.4 | 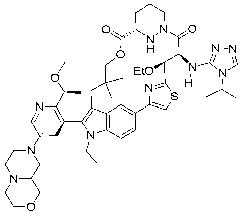 | 924.5 |
| Z182 | | Z183 | | Z184 | |
| 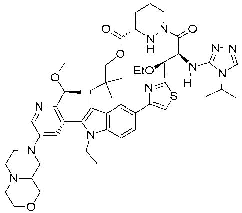 | 924.5 | 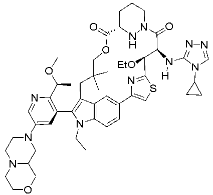 | 922.5 | 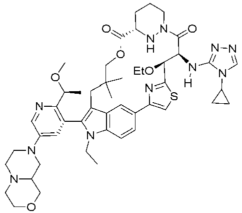 | 922.5 |
| Z185 | | Z186 | | Z187 | |
| 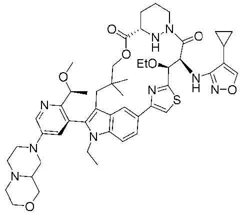 | 922.5 | 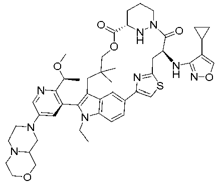 | 878.4 | 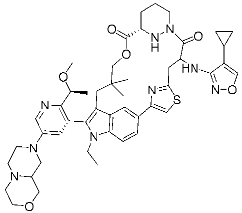 | 878.4 |
| Z188 | | Z189 | | Z190 | |
| 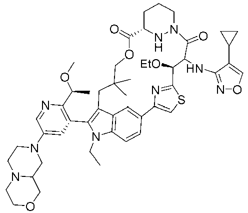 | 922.5 | 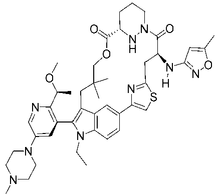 | 810.4 | 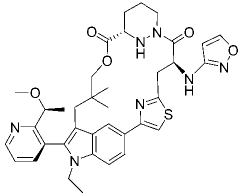 | 698.2 |
| Z191 | | Z421 | | Z371 | |
| 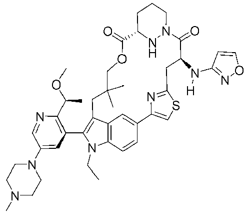 | 796.3 | 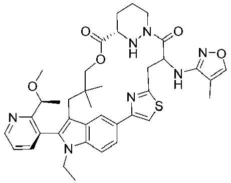 | 712.3 | 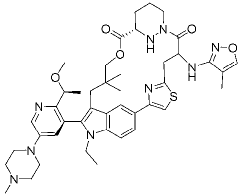 | 810.3 |
| Z459 | | Z466 | | Z142-1 | |
| 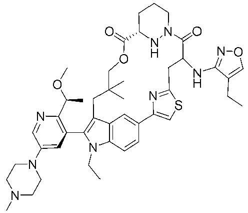 | 824.3 | 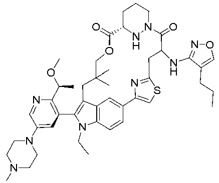 | 838.3 | 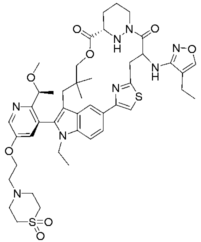 | 807.3 |
| Z471 | | Z657 | | Z580 | |
| 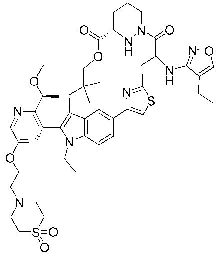 | 807.3 | 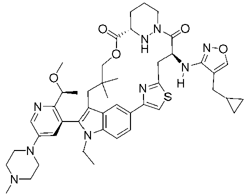 | 850.3 | 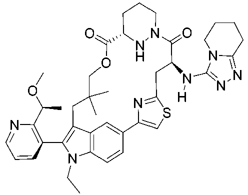 | 752.4 |
| Z580-1 | | Z660 | | Z422 | |
| 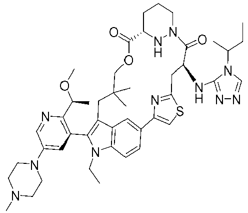 | 852.5 | 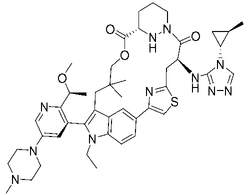 | 850.4 | 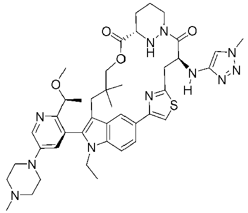 | 810.4 |
| Z424 | | Z425 | | Z426 | |
| 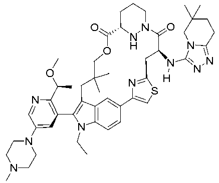 | 878.5 | 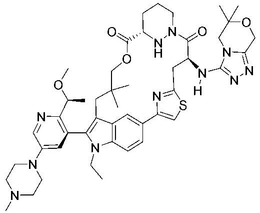 | 880.5 | 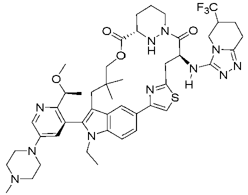 | 918.4 |
| Z427 | | Z428 | | Z429 | |
| 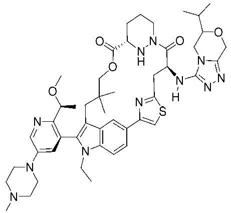 | 894.5 | 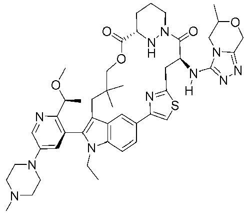 | 866.4 | 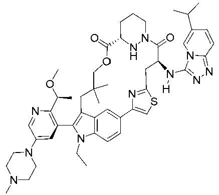 | 888.5 |
| Z430 | | Z432 | | Z433 | |
| 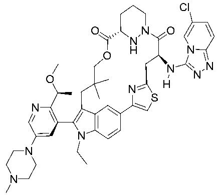 | 880.4 | 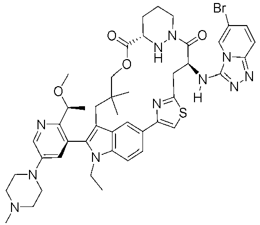 | 924.4 | 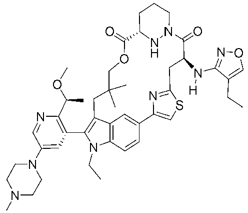 | 824.4 |
| Z436 | | Z437 | | Z472 | |
| 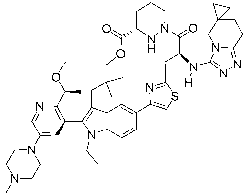 | 876.5 | 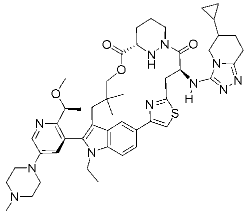 | 890.5 | 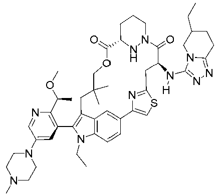 | 878.5 |
| Z473 | | Z474 | | Z475 | |
| 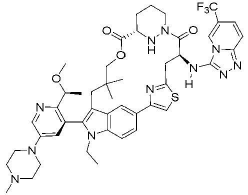 | 914.4 | 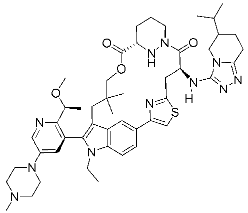 | 892.5 | 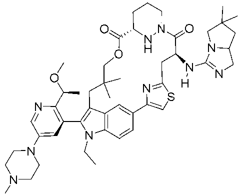 | 865.5 |
| Z476 | | Z477 | | Z560 | |
| 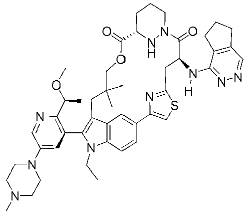 | 847.4 | 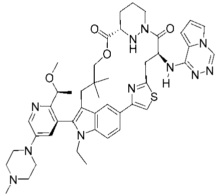 | 846.4 | 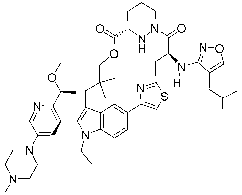 | 852.5 |
| Z561 | | Z562 | | Z563 | |
| 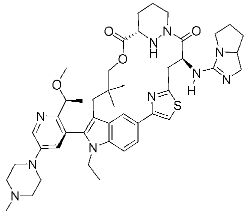 | 837.5 | 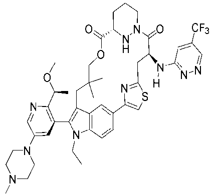 | 875.4 | 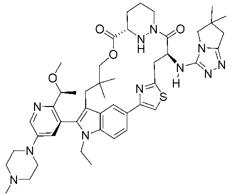 | 864.5 |
| Z564 | | Z565 | | Z566 | |
| 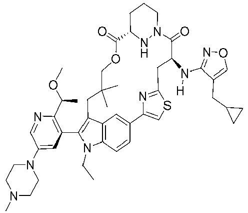 | 850.4 | 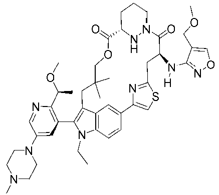 | 840.4 | 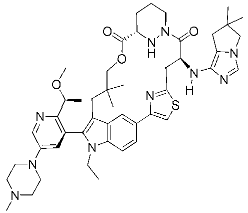 | 863.5 |
| Z567 | | Z568 | | Z570 | |
| 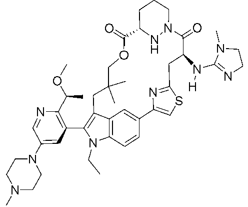 | 811.4 | 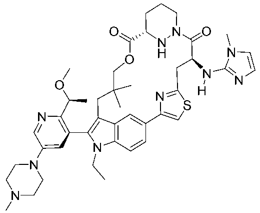 | 809.4 | 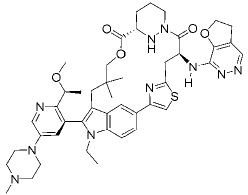 | 849.4 |
| Z571 | | Z572 | | Z573 | |
| 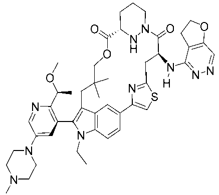 | 849.4 | 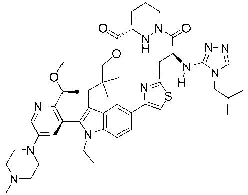 | 851.5 | 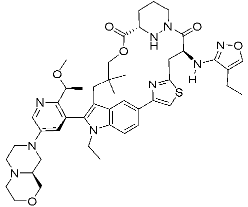 | 866.4 |
| Z574 | | Z575 | | Z576 | |
| 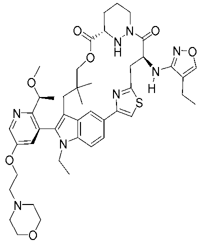 | 855.4 | 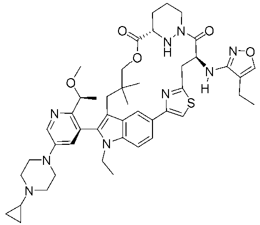 | 850.4 | 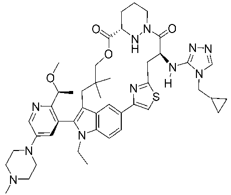 | 849.5 |
| Z577 | | Z578 | | Z579 | |
| 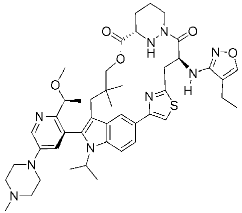 | 838.4 | 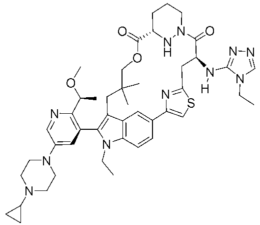 | 850.4 | 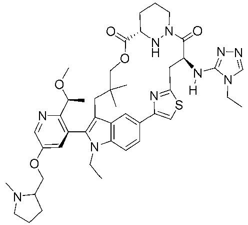 | 839.4 |
| Z581 | | Z582 | | Z583 | |
| 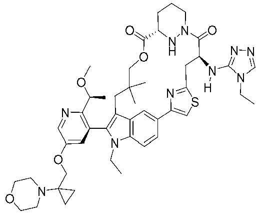 | 881.4 | 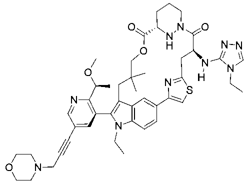 | 849.4 | 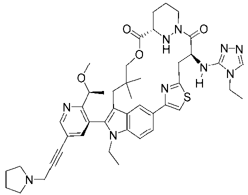 | 833.4 |
| Z584 | | Z585 | | Z586 | |
| 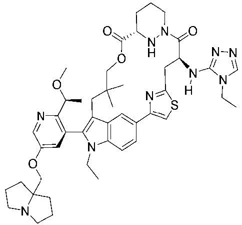 | 865.4 | 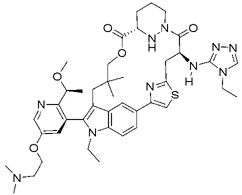 | 813.4 | 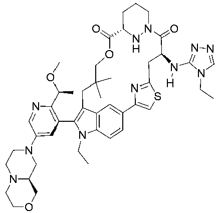 | 866.4 |
| Z587 | | Z588 | | Z589 | |
| 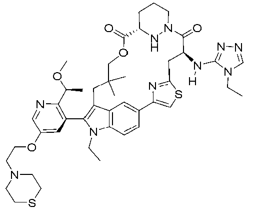 | 871.4 | 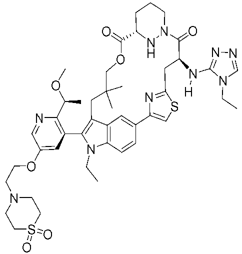 | 903.4 | 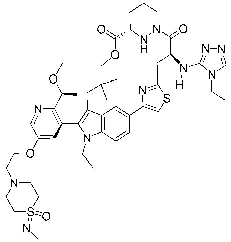 | 916.4 |
| Z590 | | Z591 | | Z592 | |
| 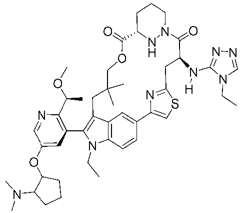 | 853.4 | 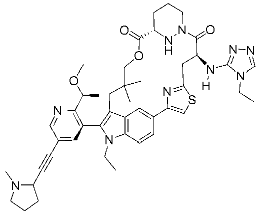 | 833.4 | 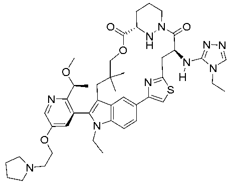 | 839.4 |
| Z593 | | Z594 | | Z595 | |
| 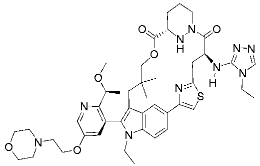 | 855.4 | 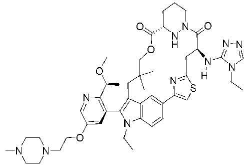 | 868.5 | 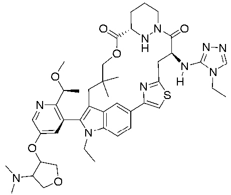 | 855.4 |
| Z596 | | Z597 | | Z617 | |
| 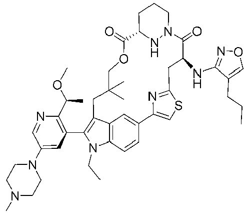 | 838.4 | 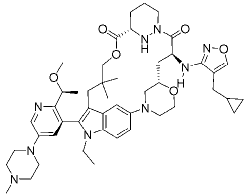 | 852.5 | 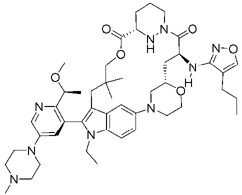 | 840.5 |
| Z654 | | Z655 | | Z656 | |
| 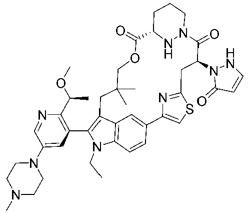 | 796.4 | 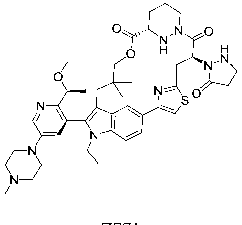 | 798.4 | 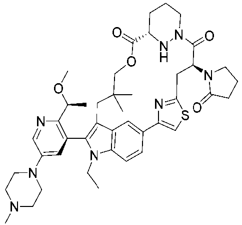 | 797.4 |
| Z770 | | Z771 | | Z772 | |
| 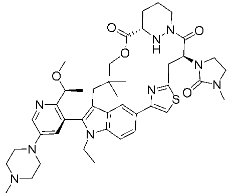 | 812.4 | 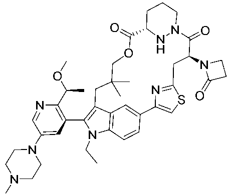 | 783.4 | 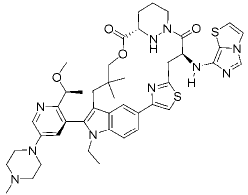 | 851.4 |
| Z773 | | Z774 | | Z817 | |
| 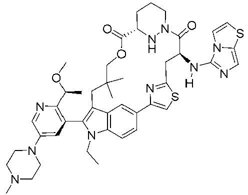 | 851.4 | 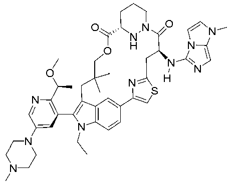 | 848.4 | 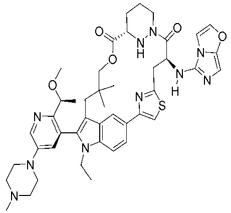 | 835.4 |
| Z818 | | Z819 | | Z820 | |
| 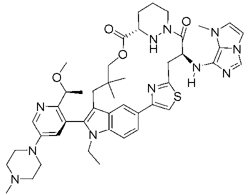 | 848.4 | 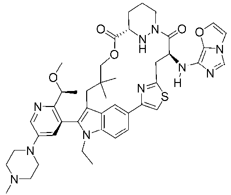 | 835.4 | 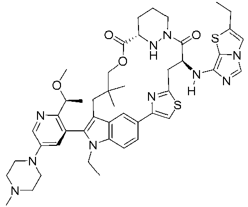 | 879.4 |
| Z821 | | Z822 | | Z823 | |
| 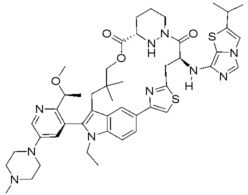 | 893.4 | 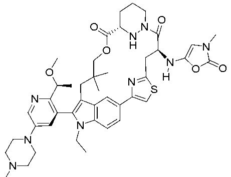 | 826.4 | 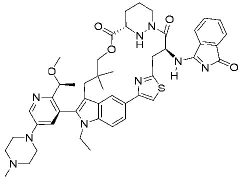 | 858.4 |
| Z824 | | Z825 | | Z826 | |
| 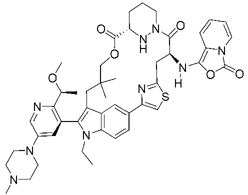 | 862.4 | 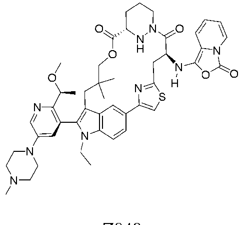 | 862.4 | 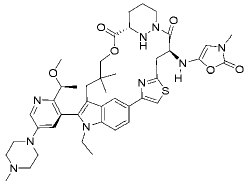 | 826.4 |
| Z827 | | Z840 | | Z841 | |
| 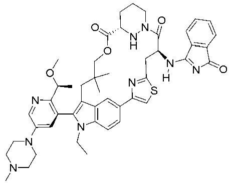 | 858.4 | 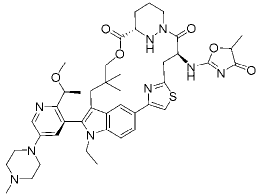 | 826.4 | 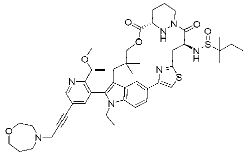 | 886.4 |
| Z842 | | Z843 | | Z844 | |
| 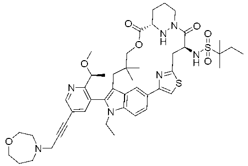 | 902.4 | 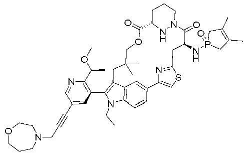 | 896.4 | 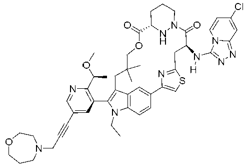 | 919.4 |
| Z845 | | Z968 | | Z971 | |
| 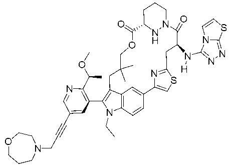 | 891.4 | 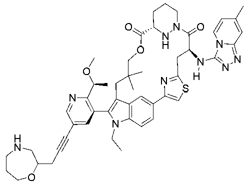 | 899.4 | 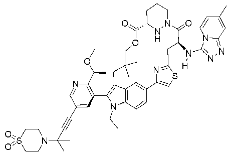 | 961.4 |
| Z972 | | Z973 | | Z975 | |
| 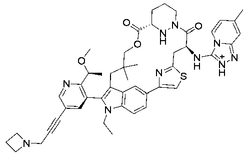 | 856.4 | 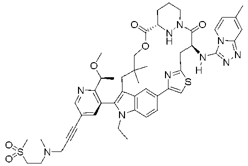 | 935.4 | 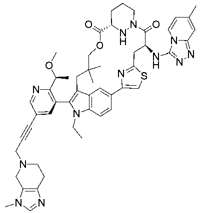 | 935.4 |
| Z977 | | Z980 | | Z983 | |
| 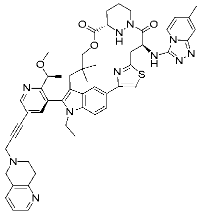 | 932.4 | 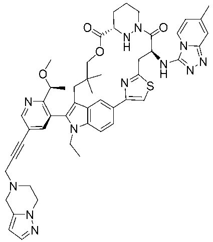 | 921.4 | 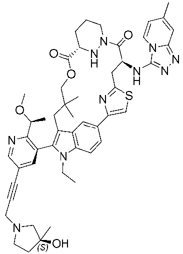 | 899.4 |
| Z984 | | Z985 | | Z986 | |
| 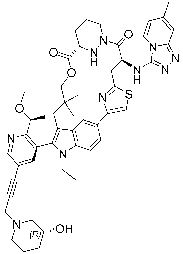 | 899.4 | 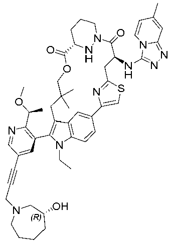 | 913.4 | 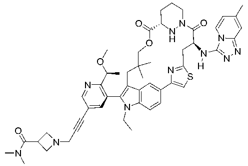 | 926.4 |
| Z987 | | Z988 | | Z989 | |
| 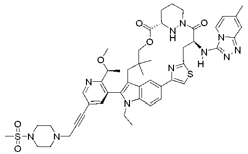 | 962.4 | 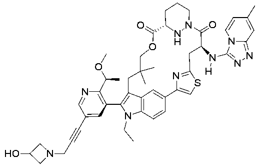 | 871.4 | 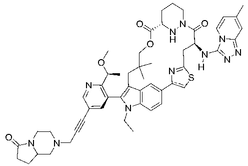 | 938.4 |
| Z990 | | Z991 | | Z992 | |
| 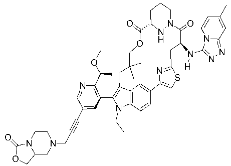 | 940.4 | 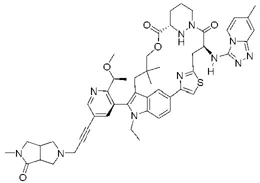 | 938.4 | 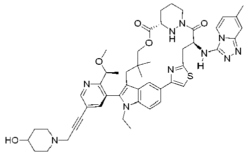 | 899.4 |
| Z993 | | Z994 | | Z995 | |
| 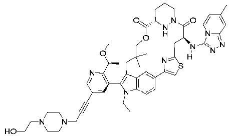 | 928.5 | 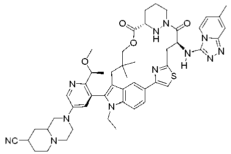 | 925.5 | 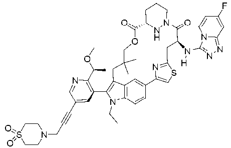 | 937.4 |
| Z996 | | Z1012 | | Z1013 | |
| 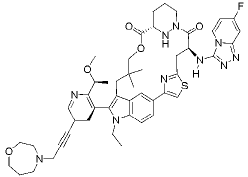 | 903.4 | 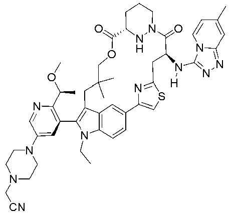 | 885.4 | 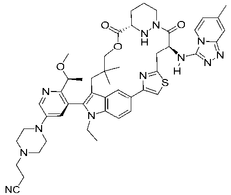 | 899.4 |
| Z1017 | | Z1018 | | Z1019 | |
| 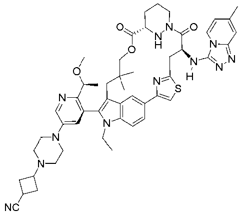 | 925.5 | 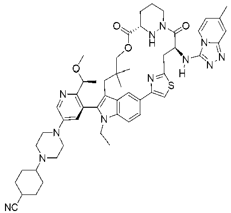 | 953.5 | 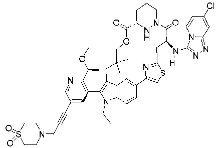 | 953.3 |
| Z1020 | | Z1021 | | Z1022 | |
| 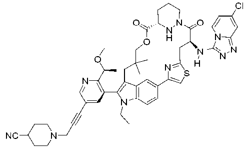 | 928.4 | 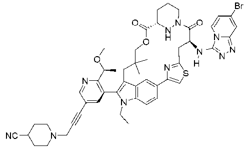 | 972.3 | 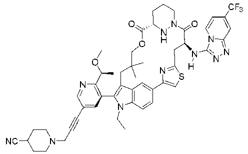 | 962.4 |
| Z1023 | | Z1024 | | Z1025 | |
| 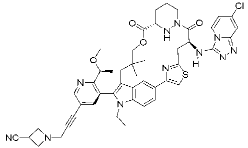 | 900.3 | 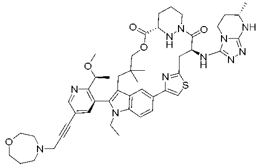 | 904.5 | 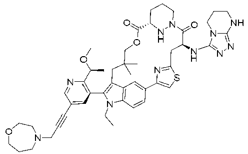 | 890.4 |
| Z1026 | | Z1035 | | Z1036 | |
| 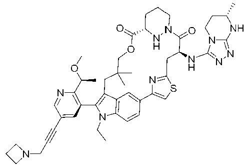 | 860.4 | 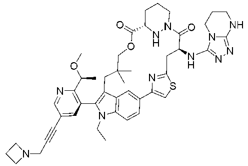 | 846.4 | | |
| Z1037 | | Z1038 | | | |
| 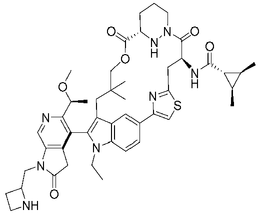 | 851.4 | 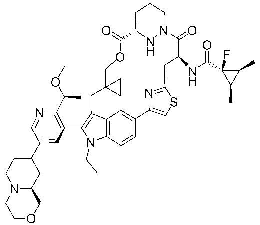 | 882.4 | 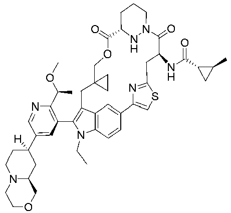 | 850.4 |
| Z828 | | Z192 | | Z193 | |
| 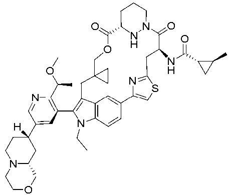 | 850.4 | 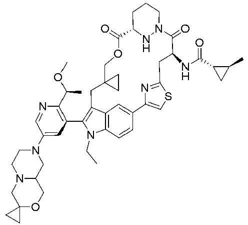 | 877.4 | 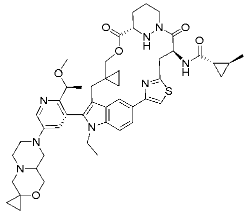 | 877.4 |
| Z194 | | Z195 | | Z196 | |
| 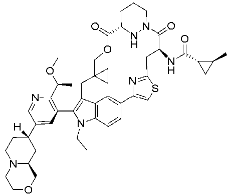 | 850.4 | 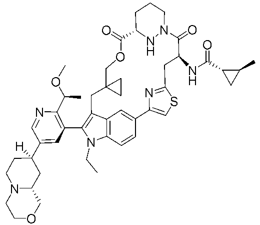 | 850.4 | 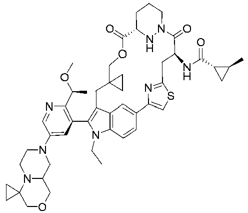 | 877.4 |
| Z197 | | Z198 | | Z199 | |
| 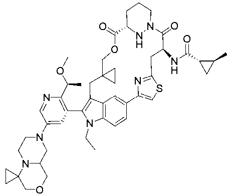 | 877.4 | 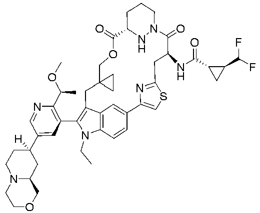 | 886.4 | 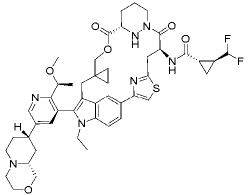 | 886.4 |
| Z200 | | Z201 | | Z202 | |
| 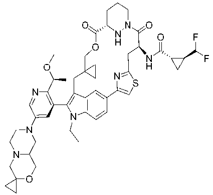 | 913.4 | 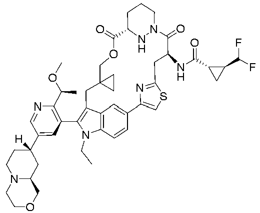 | 886.4 | 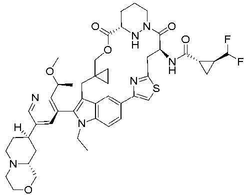 | 886.4 |
| Z203 | | Z204 | | Z205 | |
| 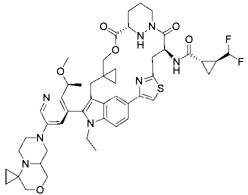 | 913.4 | 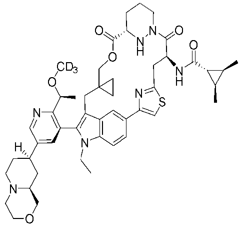 | 867.5 | 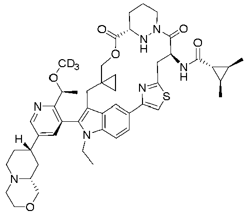 | 867.5 |
| Z206 | | Z207 | | Z208 | |
| 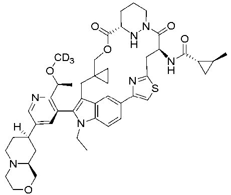 | 853.4 | 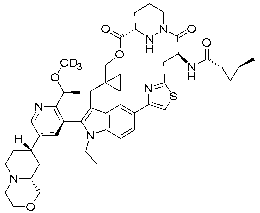 | 853.4 | 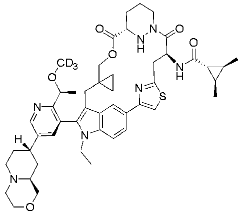 | 867.5 |
| Z209 | | Z210 | | Z211 | |
| 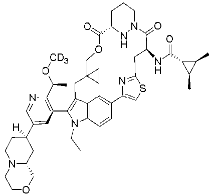 | 867.5 | 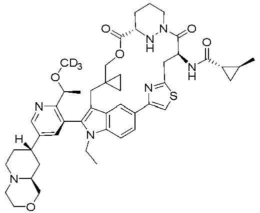 | 853.4 | 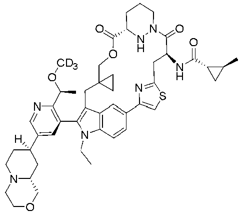 | 853.4 |
| Z212 | | Z213 | | Z214 | |
| 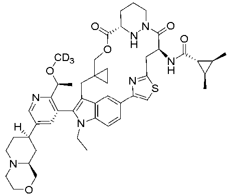 | 867.5 | 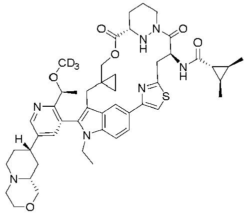 | 867.5 | 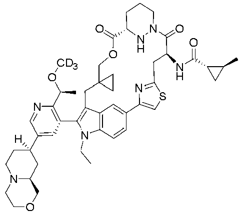 | 853.4 |
| Z215 | | Z216 | | Z217 | |
| 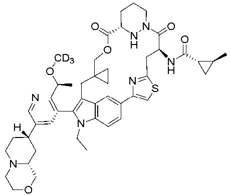 | 853.4 | 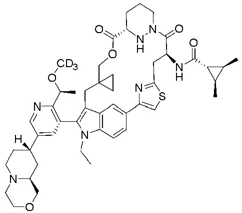 | 867.5 | 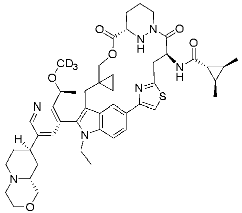 | 867.5 |
| Z218 | | Z219 | | Z220 | |
| 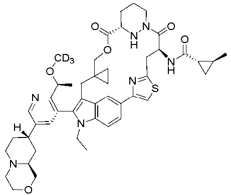 | 853.4 | 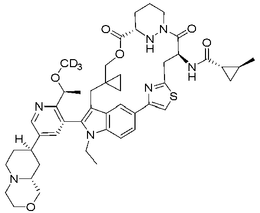 | 853.4 | 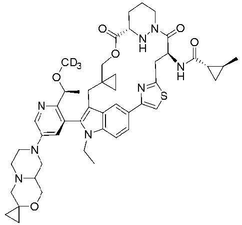 | 880.5 |
| Z221 | | Z222 | | Z223 | |
| 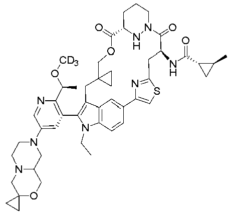 | 880.5 | 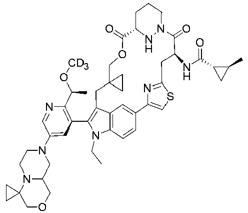 | 880.5 | 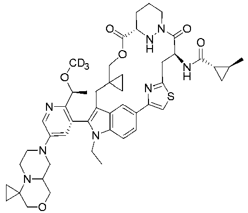 | 880.5 |
| Z224 | | Z225 | | Z226 | |
| 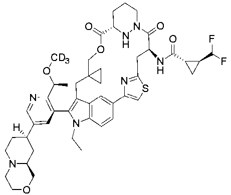 | 889.4 | 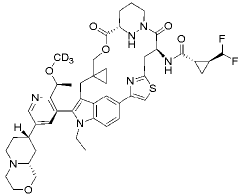 | 889.4 | 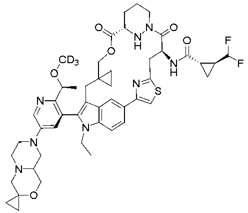 | 916.4 |
| Z227 | | Z228 | | Z229 | |
| 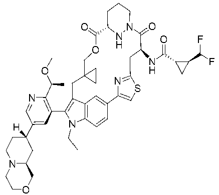 | 886.5 | 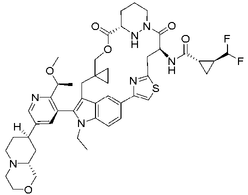 | 886.4 | 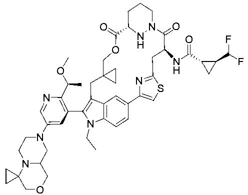 | 913.4 |
| Z230 | | Z231 | | Z232 | |
| 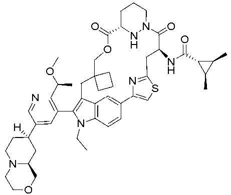 | 878.5 | 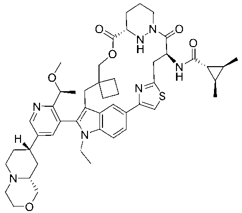 | 878.5 | 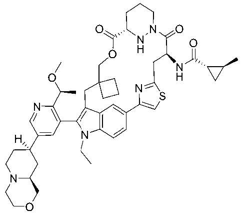 | 864.4 |
| Z233 | | Z234 | | Z235 | |
| 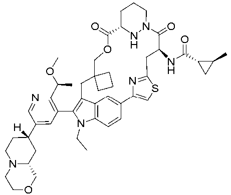 | 864.4 | 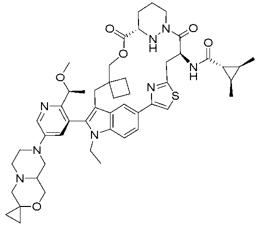 | 905.5 | 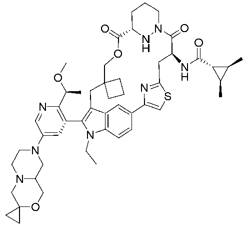 | 905.5 |
| Z236 | | Z237 | | Z238 | |
| 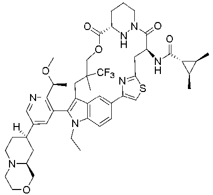 | 920.4 | 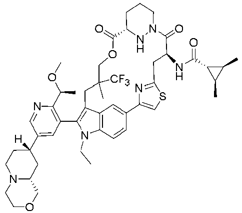 | 920.4 | 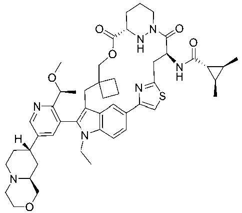 | 878.5 |
| Z239 | | Z240 | | Z241 | |
| 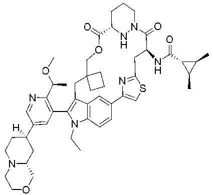 | 878.5 | 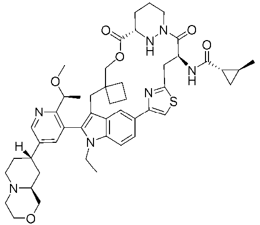 | 864.4 | 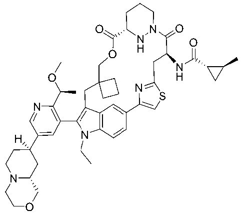 | 864.4 |
| Z242 | | Z243 | | Z244 | |
| 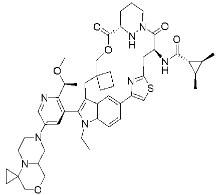 | 905.5 | 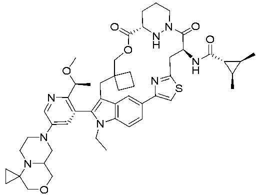 | 905.5 | 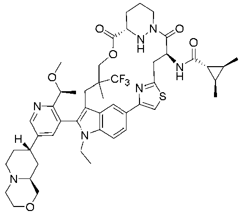 | 920.4 |
| Z245 | | Z246 | | Z247 | |
| 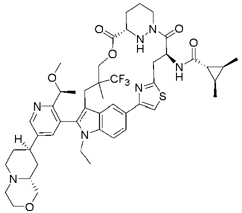 | 920.4 | 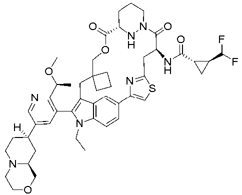 | 900.4 | 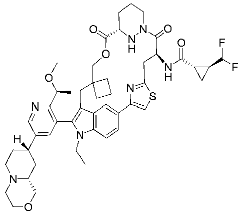 | 900.4 |
| Z248 | | Z249 | | Z250 | |
| 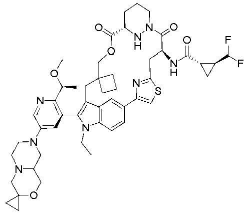 | 927.4 | 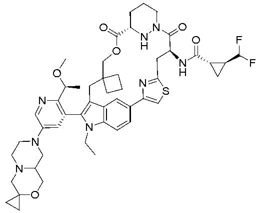 | 927.4 | 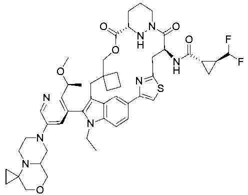 | 927.4 |
| Z251 | | Z252 | | Z253 | |
| 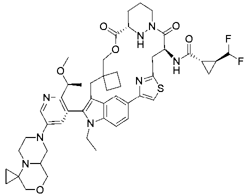 | 927.4 | 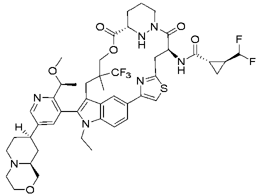 | 942.4 | 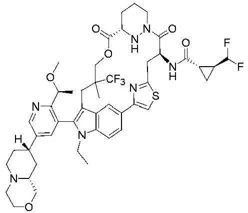 | 942.4 |
| Z254 | | Z255 | | Z256 | |
| 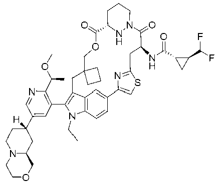 | 900.4 | 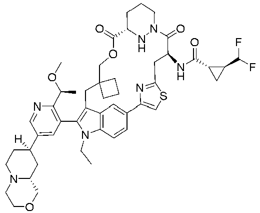 | 900.4 | 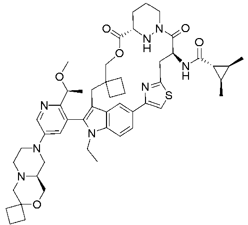 | 919.5 |
| Z257 | | Z258 | | Z259 | |
| 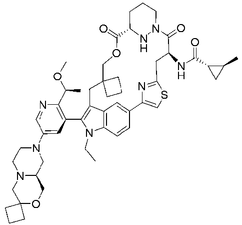 | 905.5 | 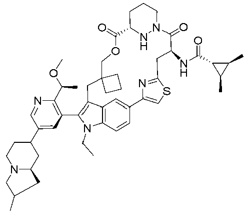 | 876.5 | 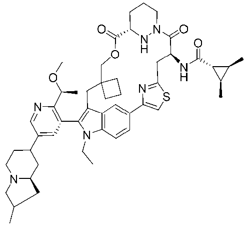 | 876.5 |
| Z260 | | Z261 | | Z262 | |
| 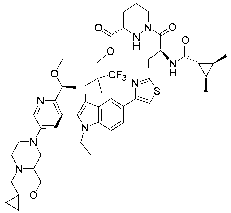 | 947.4 | 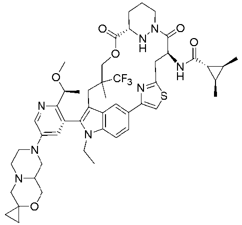 | 947.4 | 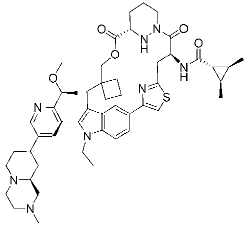 | 891.5 |
| Z263 | | Z264 | | Z265 | |
| 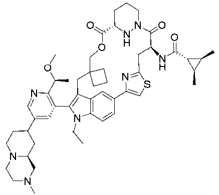 | 891.5 | 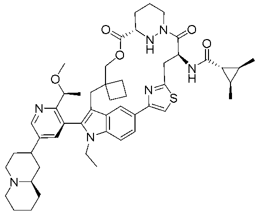 | 876.5 | 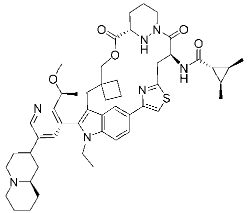 | 876.5 |
| Z266 | | Z267 | | Z268 | |
| 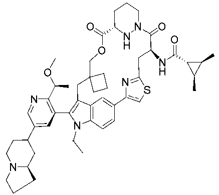 | 862.5 | 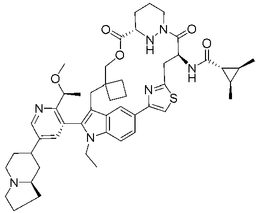 | 862.5 | 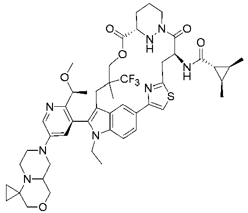 | 947.4 |
| Z269 | | Z270 | | Z271 | |
| 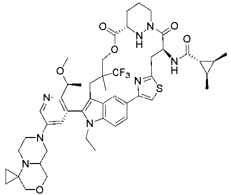 | 947.4 | 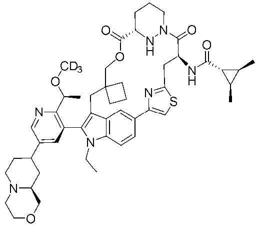 | 881.5 | 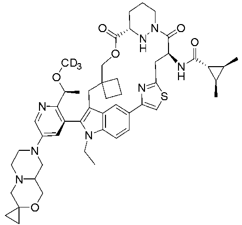 | 908.5 |
| Z272 | | Z273 | | Z274 | |
| 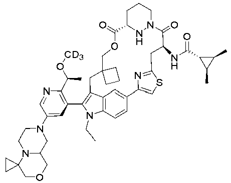 | 908.5 | 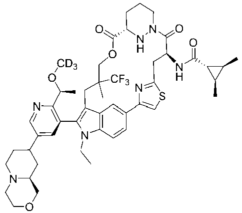 | 923.4 | 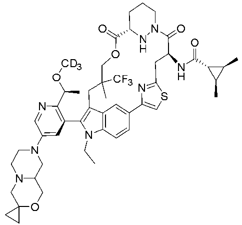 | 950.5 |
| Z275 | | Z276 | | Z277 | |
| 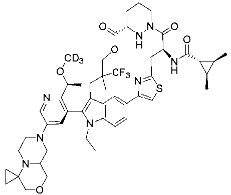 | 950.5 | 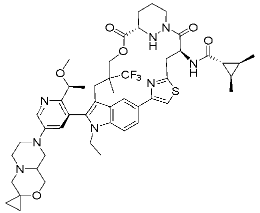 | 947.4 | 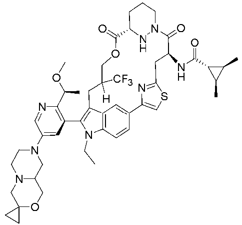 | 933.4 |
| Z278 | | Z279 | | Z280 | |
| 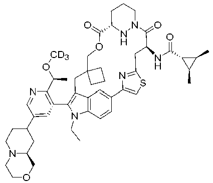 | 881.5 | 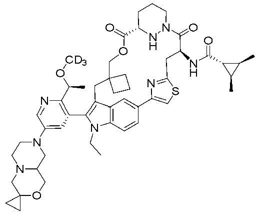 | 908.5 | 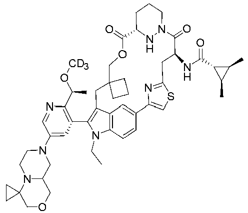 | 908.5 |
| Z281 | | Z282 | | Z283 | |
| 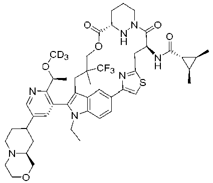 | 923.4 | 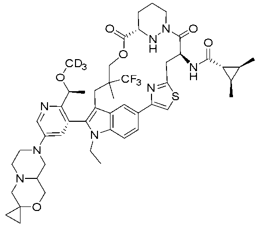 | 950.5 | 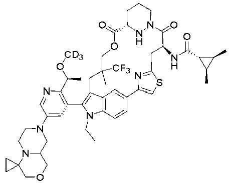 | 950.5 |
| Z284 | | Z285 | | Z286 | |
| 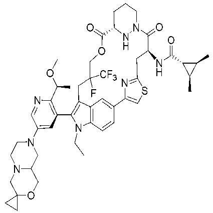 | 951.4 | 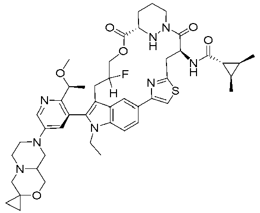 | 883.4 | 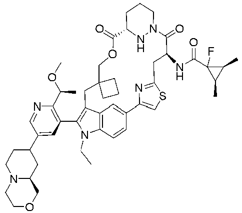 | 896.4 |
| Z287 | | Z288 | | Z289 | |
| 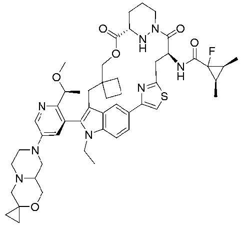 | 923.5 | 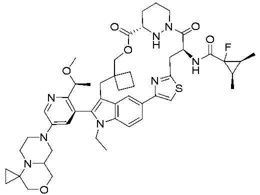 | 923.5 | 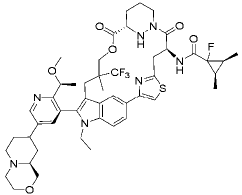 | 938.4 |
| Z290 | | Z291 | | Z292 | |
| 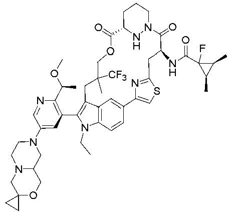 | 965.4 | 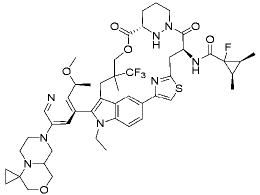 | 965.4 | 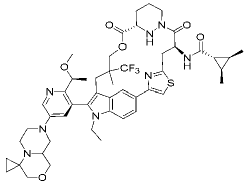 | 947.4 |
| Z293 | | Z294 | | Z295 | |
| 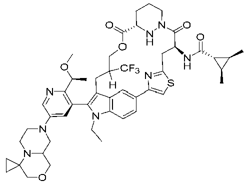 | 933.4 | 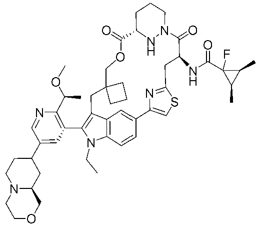 | 896.4 | 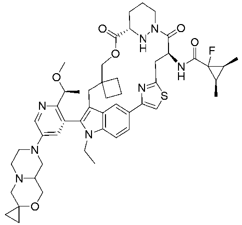 | 923.5 |
| Z296 | | Z297 | | Z298 | |
| 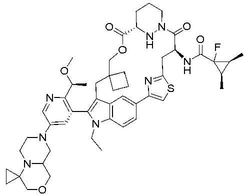 | 923.5 | 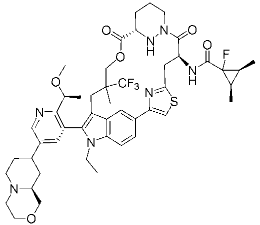 | 938.4 | 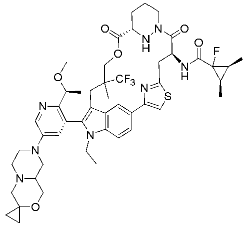 | 965.4 |
| Z299 | | Z300 | | Z301 | |
| 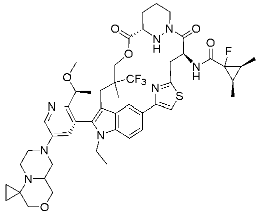 | 965.4 | 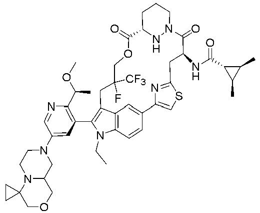 | 951.4 | 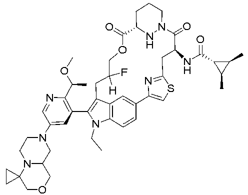 | 883.4 |
| Z302 | | Z303 | | Z304 | |
| 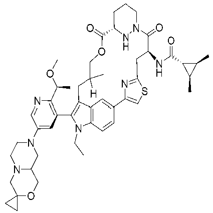 | 879.5 | 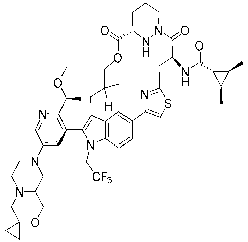 | 933.4 | 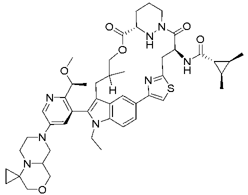 | 879.5 |
| Z305 | | Z306 | | Z307 | |
| 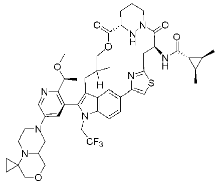 | 933.4 | 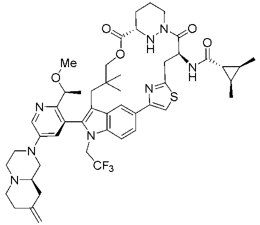 | 931.4 | 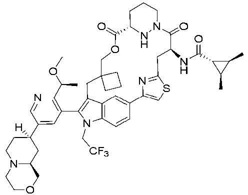 | 932.4 |
| Z308 | | Z310 | | Z311 | |
| 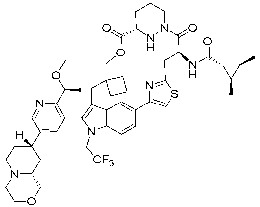 | 932.4 | 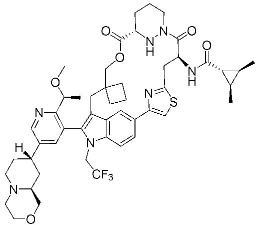 | 932.4 | 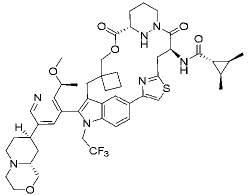 | 932.4 |
| Z312 | | Z313 | | Z314 | |
| 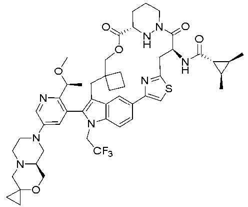 | 959.4 | 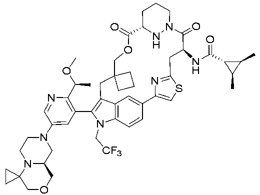 | 959.4 | 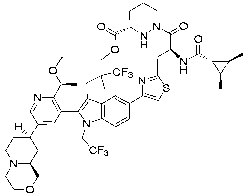 | 974.4 |
| Z315 | | Z316 | | Z317 | |
| 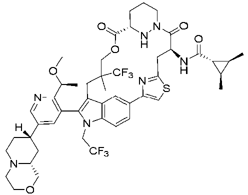 | 974.4 | 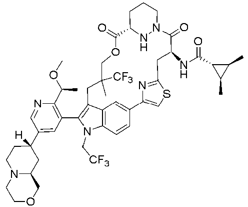 | 974.4 | 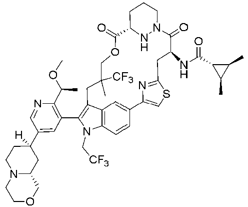 | 974.4 |
| Z318 | | Z319 | | Z320 | |
| 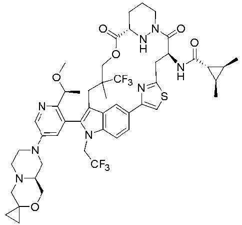 | 1001.4 | 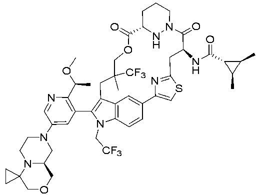 | 1001.4 | 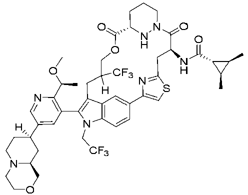 | 960.4 |
| Z321 | | Z322 | | Z323 | |
| 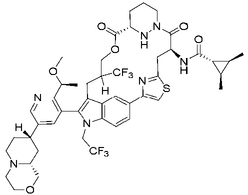 | 960.4 | 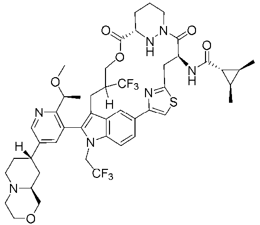 | 960.4 | 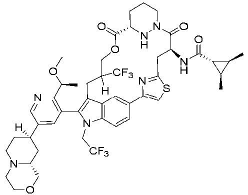 | 960.4 |
| Z324 | | Z325 | | Z326 | |
| 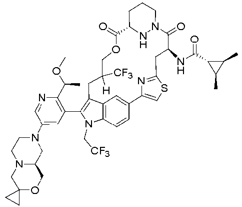 | 987.4 | 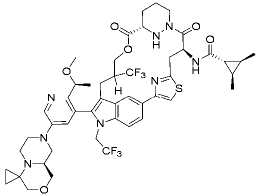 | 987.4 | 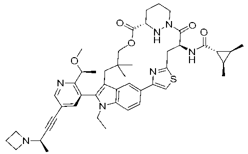 | 834.4 |
| Z327 | | Z328 | | Z478 | |
| 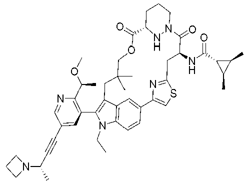 | 834.4 | 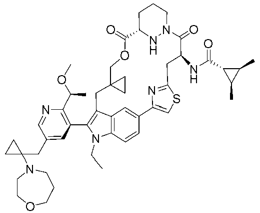 | 878.5 | 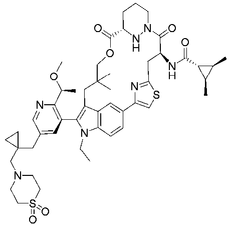 | 928.4 |
| Z479 | | Z480 | | Z481 | |
| 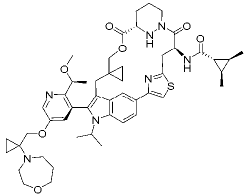 | 908.5 | 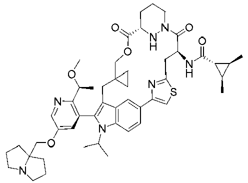 | 878.5 | 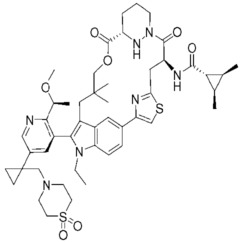 | 914.4 |
| Z482 | | Z483 | | Z486 | |
| 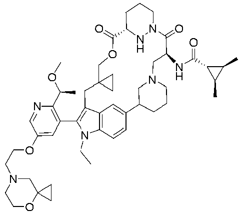 | 880.5 | 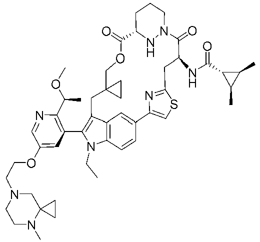 | 893.5 | 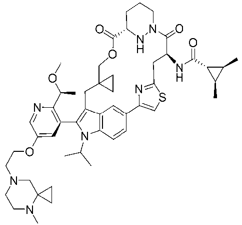 | 907.5 |
| Z487 | | Z488 | | Z489 | |
| 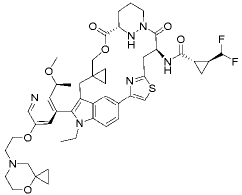 | 902.4 | 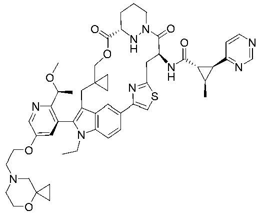 | 944.4 | 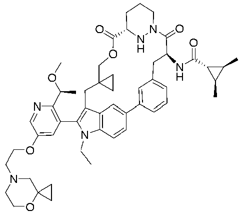 | 873.5 |
| Z490 | | Z491 | | Z492 | |
| 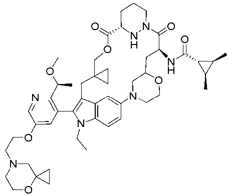 | 882.5 | 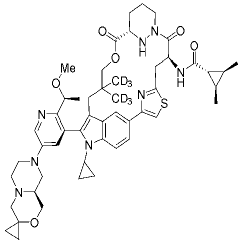 | 911.5 | 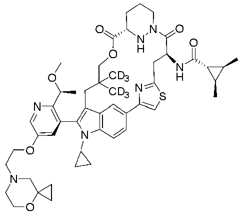 | 900.5 |
| Z493 | | Z494 | | Z495 | |
| 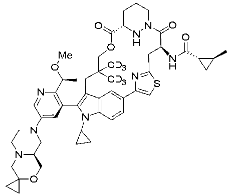 | 897.5 | 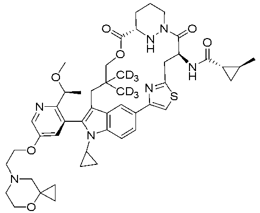 | 886.5 | 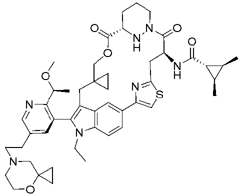 | 864.4 |
| Z496 | | Z497 | | Z499 | |
| 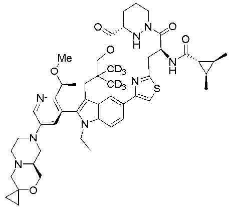 | 899.5 | 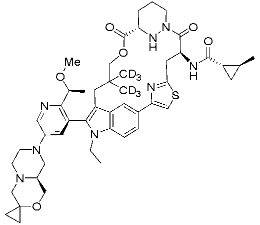 | 885.5 | 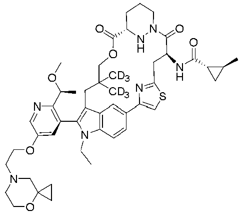 | 874.5 |
| Z500 | | Z502 | | Z503 | |
| 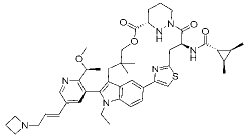 | 822.4 | 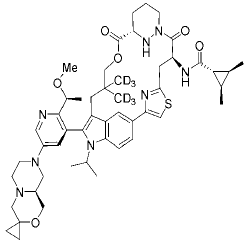 | 913.5 | 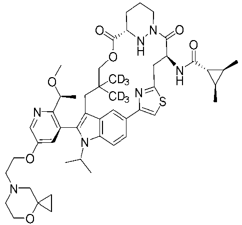 | 902.5 |
| Z505 | | Z506 | | Z507 | |
| 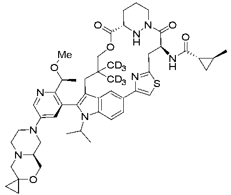 | 899.5 | 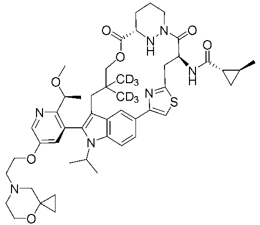 | 888.5 | 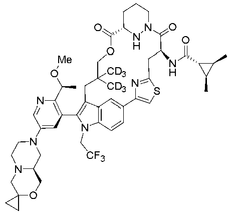 | 953.5 |
| Z508 | | Z509 | | Z510 | |
| 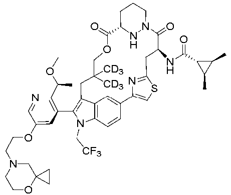 | 942.5 | 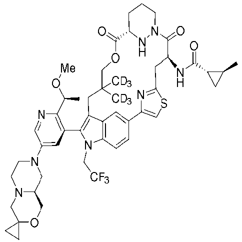 | 939.5 | 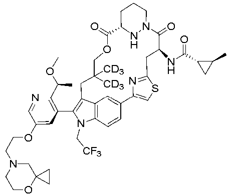 | 928.4 |
| Z511 | | Z512 | | Z513 | |
| 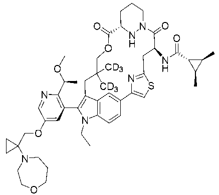 | 902.5 | 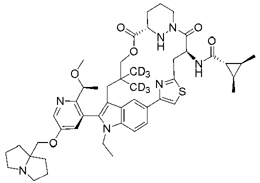 | 872.5 | 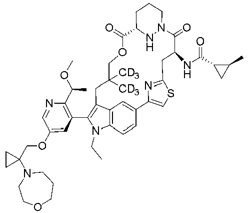 | 888.5 |
| Z514 | | Z515 | | Z516 | |
| 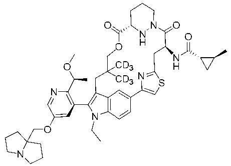 | 858.5 | 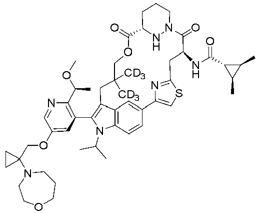 | 916.5 | 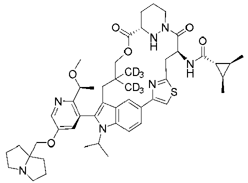 | 886.5 |
| Z517 | | Z518 | | Z519 | |
| 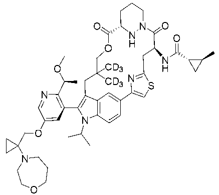 | 902.5 | 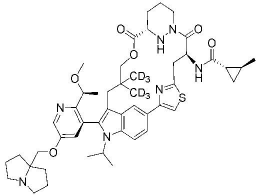 | 872.5 | 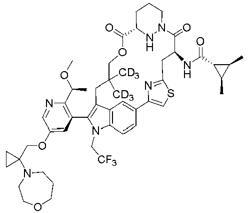 | 956.5 |
| Z520 | | Z521 | | Z522 | |
| 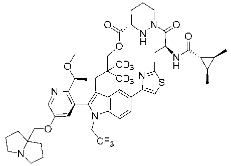 | 926.5 | 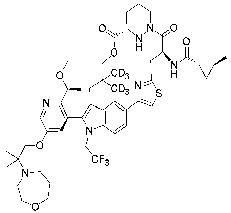 | 942.5 | 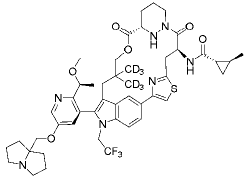 | 912.4 |
| Z523 | | Z524 | | Z525 | |
| 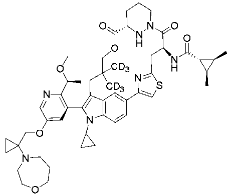 | 914.5 | 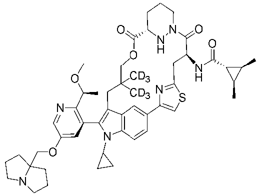 | 884.5 | 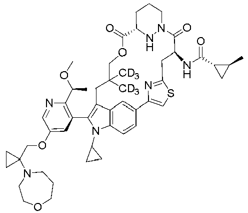 | 900.5 |
| Z526 | | Z527 | | Z528 | |
| 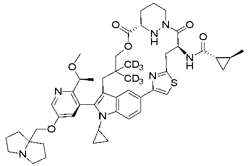 | 870.5 | 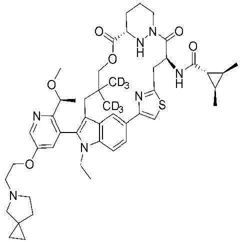 | 872.5 | 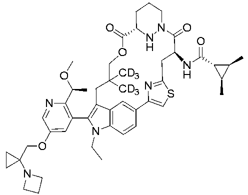 | 858.5 |
| Z529 | | Z530 | | Z531 | |
| 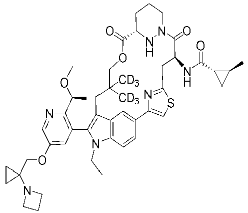 | 844.5 | 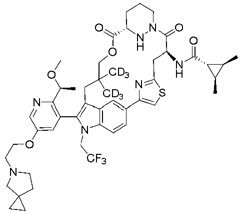 | 926.5 | 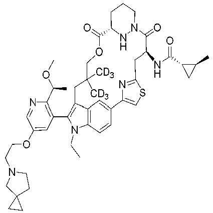 | 858.5 |
| Z533 | | Z534 | | Z532 | |
| 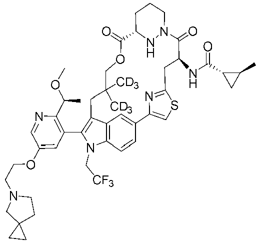 | 912.4 | 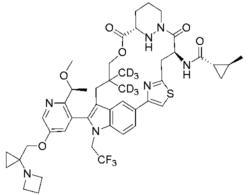 | 898.4 | 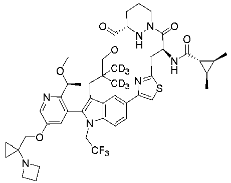 | 912.4 |
| Z536 | | Z537 | | Z535 | |
| 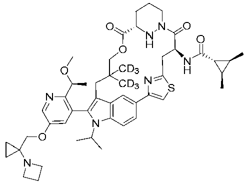 | 872.5 | 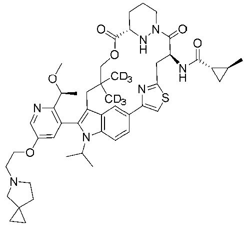 | 872.5 | 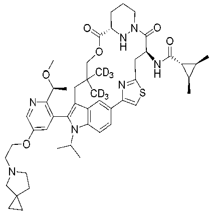 | 886.5 |
| Z539 | | Z540 | | Z538 | |
| 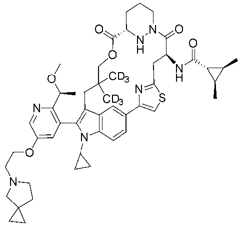 | 884.5 | 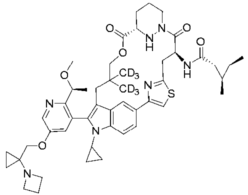 | 870.5 | 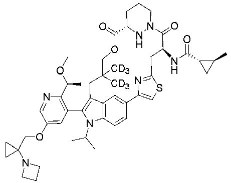 | 858.5 |
| Z542 | | Z543 | | Z541 | |
| 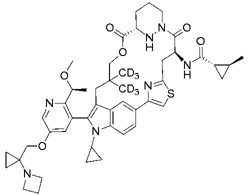 | 856.5 | 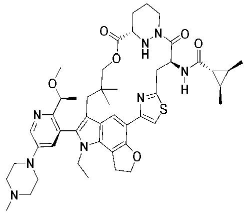 | 867.5 | 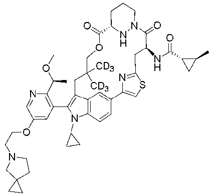 | 870.5 |
| Z545 | | Z546 | | Z544 | |
| 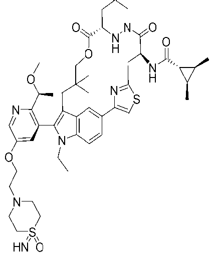 | 903.4 | 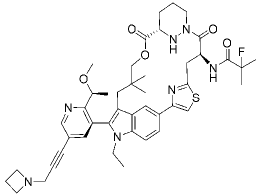 | 812.4 | 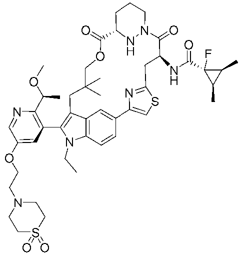 | 922.4 |
| Z602 | | Z606 | | Z608 | |
| 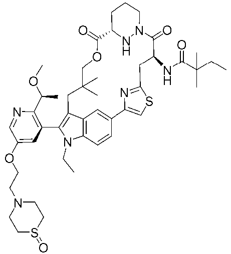 | 890.4 | 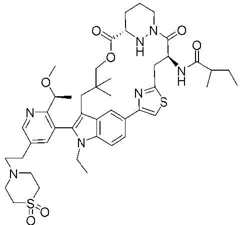 | 876.4 | | |
| Z611 | | Z612 | | | |
| 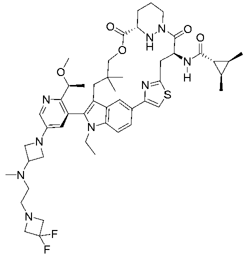 | 930.5 | 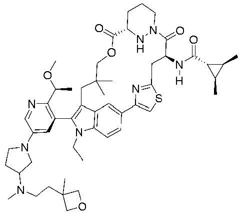 | 923.5 | 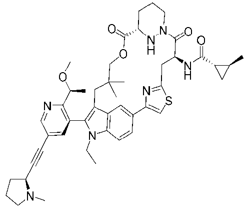 | 820.4 |
| Z615 | | Z616 | | Z618 | |
| 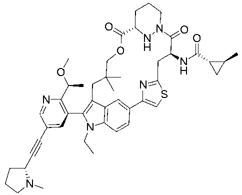 | 820.4 | 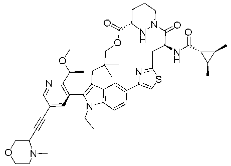 | 850.4 | 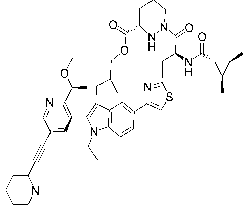 | 848.4 |
| Z619 | | Z620 | | Z621 | |
| 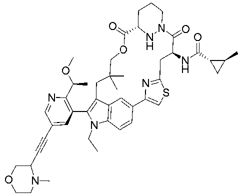 | 836.4 | 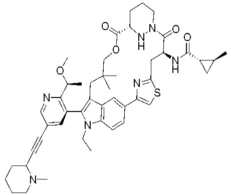 | 834.4 | 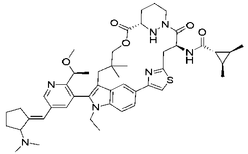 | 850.5 |
| Z622 | | Z623 | | Z627 | |
| 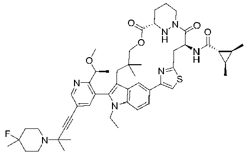 | 908.5 | 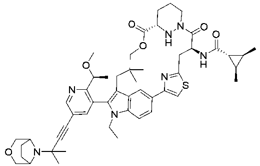 | 904.5 | 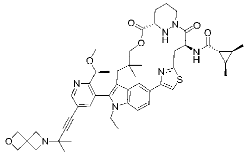 | 900.5 |
| Z629 | | Z630 | | Z631 | |
| 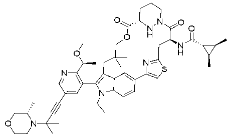 | 892.5 | 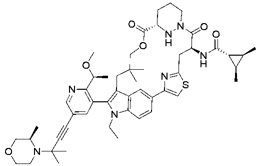 | 892.5 | 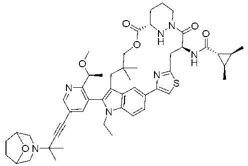 | 904.5 |
| Z632 | | Z633 | | Z634 | |
| 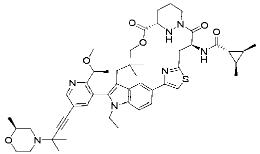 | 892.5 | 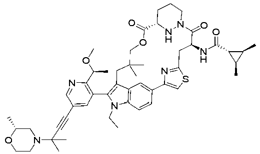 | 892.5 | 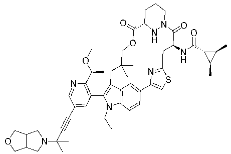 | 904.5 |
| Z636 | | Z637 | | Z635 | |
| 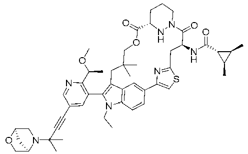 | 900.5 | 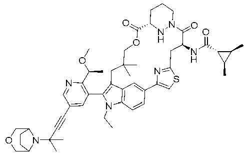 | 904.5 | 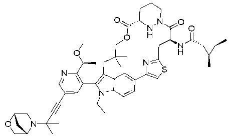 | 900.5 |
| Z639 | | Z640 | | Z638 | |
| 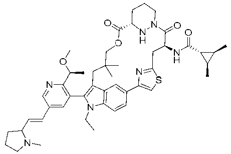 | 836.4 | 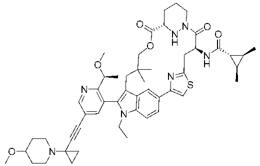 | 904.5 | 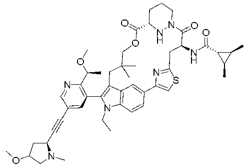 | 864.4 |
| Z641 | | Z644 | | Z646 | |
| 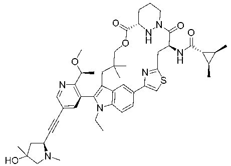 | 864.4 | 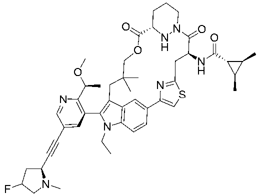 | 852.4 | 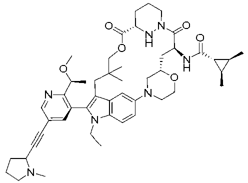 | 836.5 |
| Z647 | | Z648 | | Z649 | |
| 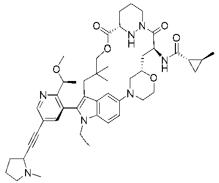 | 822.5 | 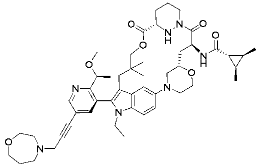 | 866.5 | 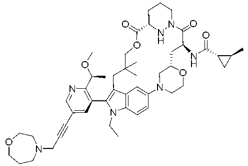 | 852.5 |
| Z650 | | Z651 | | Z652 | |
| 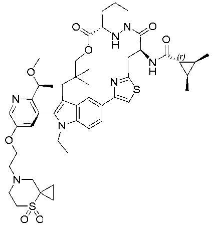 | 930.4 | 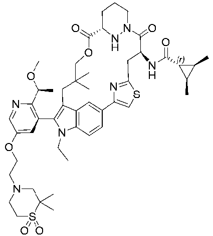 | 932.4 | 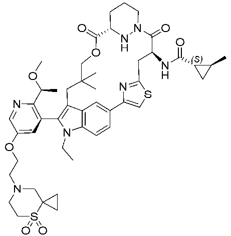 | 916.4 |
| Z663 | | Z664 | | Z666 | |
| 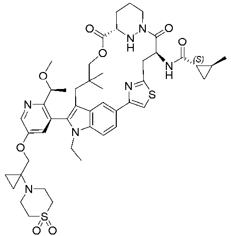 | 916.4 | 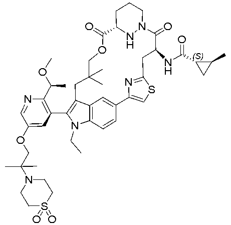 | 918.4 | 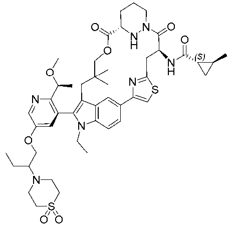 | 918.4 |
| Z668 | | Z669 | | Z670 | |
| 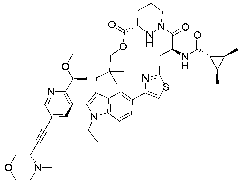 | 850.4 | 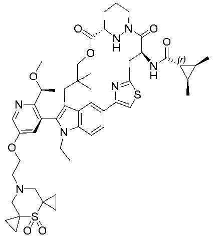 | 956.4 | 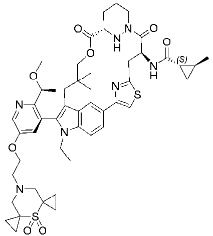 | 942.4 |
| Z671 | | Z675 | | Z676 | |
| 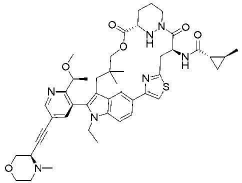 | 836.4 | 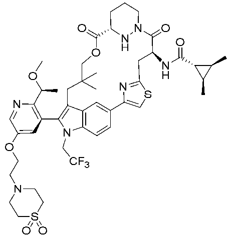 | 958.4 | 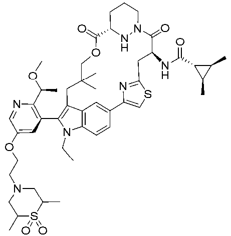 | 932.4 |
| Z674 | | Z679 | | Z680 | |
| 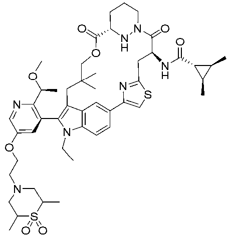 | 932.4 | 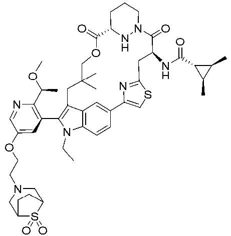 | 930.4 | 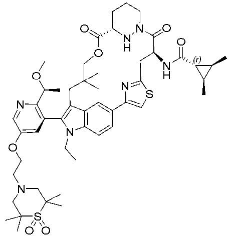 | 960.5 |
| Z681 | | Z682 | | Z683 | |
| 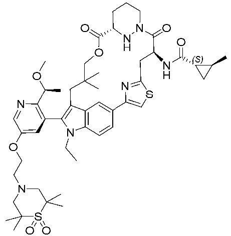 | 946.4 | 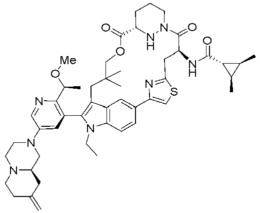 | 877.5 | 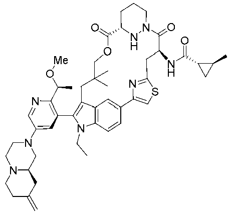 | 863.5 |
| Z684 | | Z685 | | Z686 | |
| 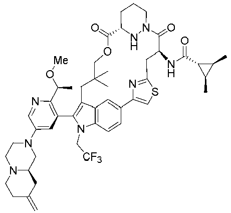 | 931.4 | 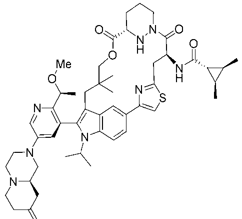 | 891.5 | 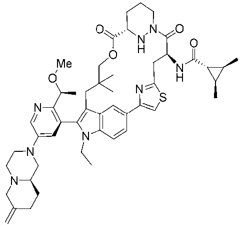 | 877.5 |
| Z687 | | Z688 | | Z689 | |
| 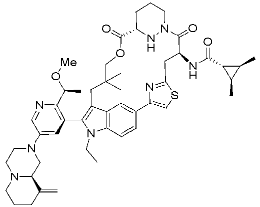 | 877.5 | 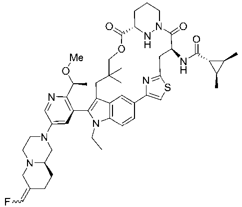 | 895.5 | 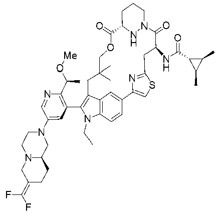 | 913.5 |
| Z690 | | Z691 | | Z692 | |
| 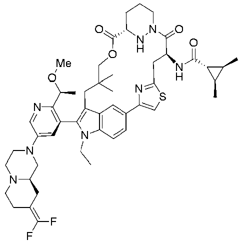 | 913.5 | 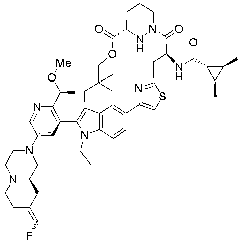 | 895.5 | 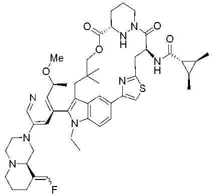 | 895.5 |
| Z693 | | Z694 | | Z695 | |
| 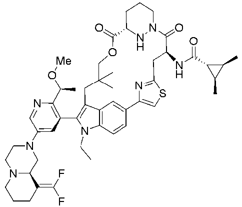 | 913.5 | 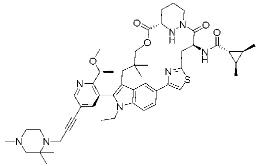 | 891.5 | 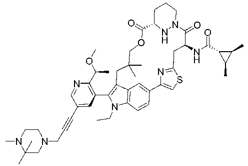 | 891.5 |
| Z696 | | Z697 | | Z698 | |
| 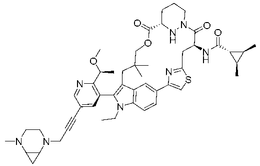 | 875.5 | 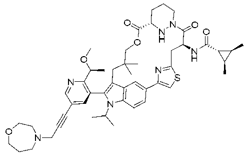 | 878.5 | 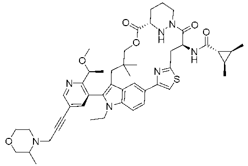 | 864.4 |
| Z699 | | Z701 | | Z702 | |
| 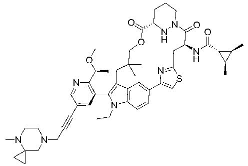 | 889.5 | 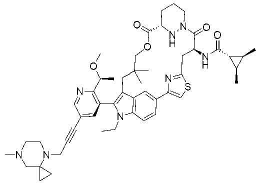 | 889.5 | 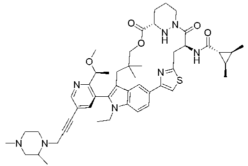 | 877.5 |
| Z704 | | Z705 | | Z706 | |
| 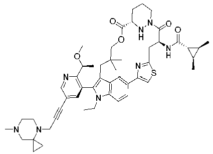 | 889.5 | 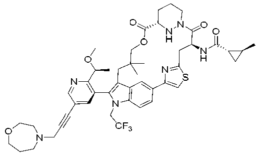 | 904.4 | 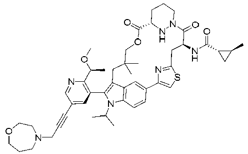 | 864.4 |
| Z707 | | Z708 | | Z709 | |
| 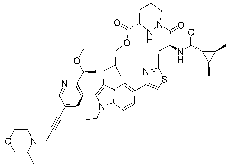 | 878.5 | 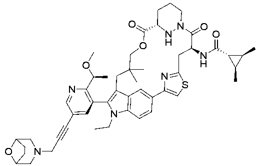 | 876.4 | 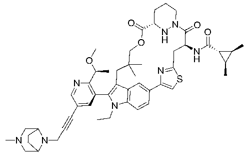 | 889.5 |
| Z710 | | Z712 | | Z713 | |
| 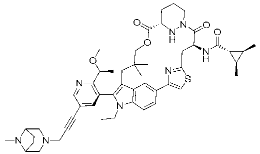 | 889.5 | 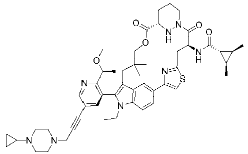 | 889.5 | 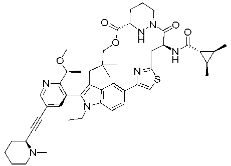 | 848.4 |
| Z714 | | Z715 | | Z716 | |
| 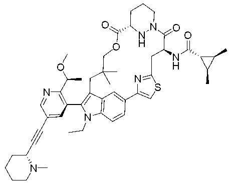 | 848.4 | 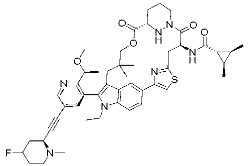 | 866.4 | 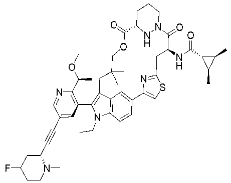 | 866.4 |
| Z717 | | Z718 | | Z719 | |
| 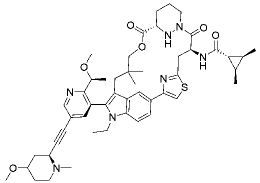 | 878.5 | 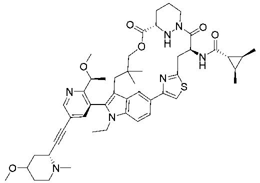 | 878.5 | 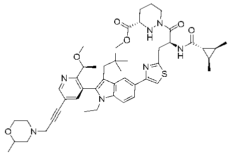 | 864.4 |
| Z720 | | Z721 | | Z722 | |
| 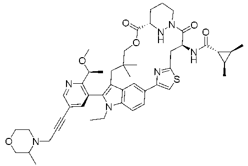 | 864.4 | 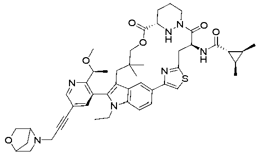 | 862.4 | 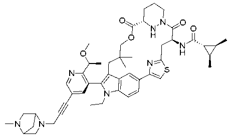 | 875.5 |
| Z723 | | Z724 | | Z725 | |
| 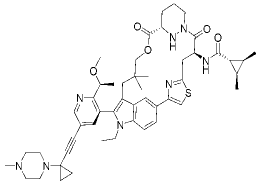 | 889.5 | 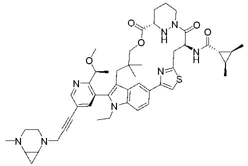 | 875.5 | 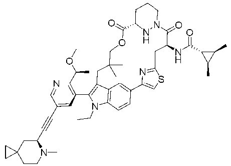 | 874.5 |
| Z726 | | Z727 | | Z728 | |
| 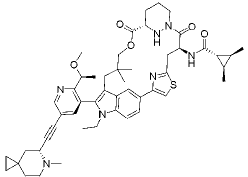 | 874.5 | 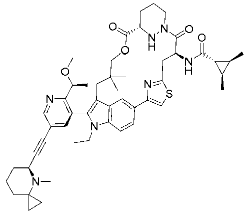 | 874.5 | 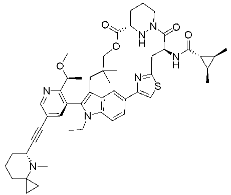 | 874.5 |
| Z729 | | Z730 | | Z731 | |
| 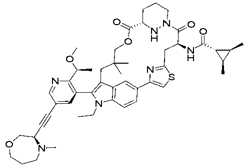 | 864.4 | 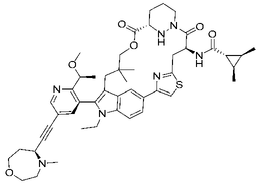 | 864.4 | 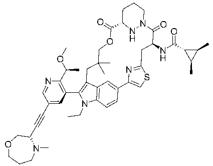 | 864.4 |
| Z732 | | Z733 | | Z734 | |
| 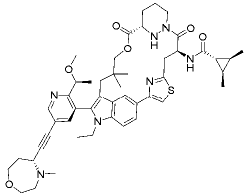 | 864.4 | 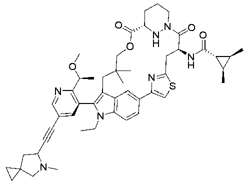 | 860.4 | 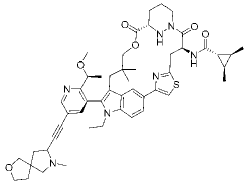 | 890.5 |
| Z735 | | Z736 | | Z737 | |
| 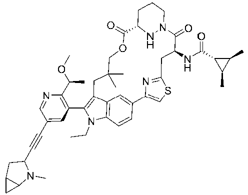 | 846.4 | 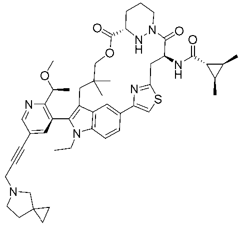 | 860.4 | 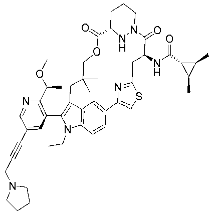 | 834.4 |
| Z738 | | Z739 | | Z740 | |
| 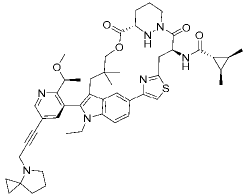 | 860.4 | 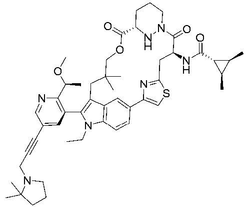 | 862.5 | 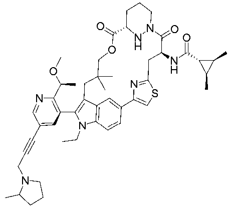 | 848.4 |
| Z741 | | Z742 | | Z743 | |
| 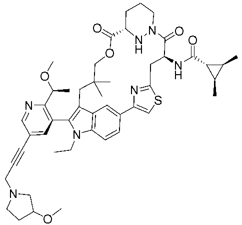 | 864.4 | 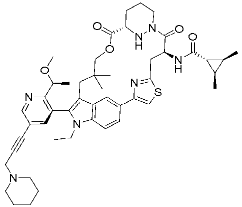 | 848.4 | 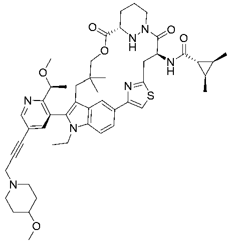 | 878.5 |
| Z744 | | Z745 | | Z746 | |
| 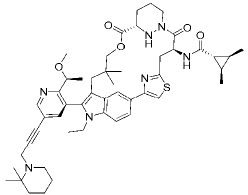 | 876.5 | 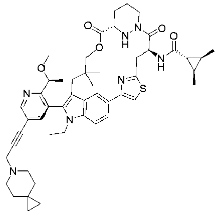 | 874.5 | 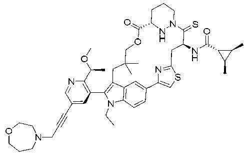 | 880.4 |
| Z747 | | Z748 | | Z749 | |
| 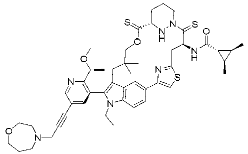 | 896.4 | 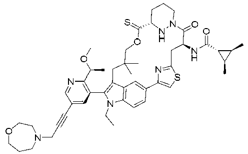 | 880.4 | 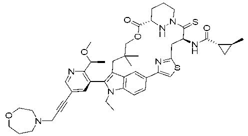 | 866.4 |
| Z750 | | Z751 | | Z752 | |
| 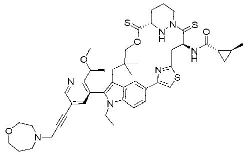 | 882.4 | 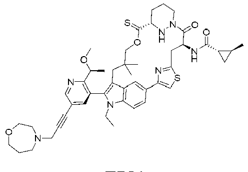 | 866.4 | 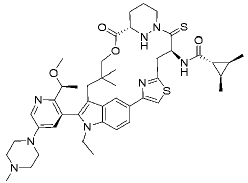 | 841.4 |
| Z753 | | Z754 | | Z756 | |
| 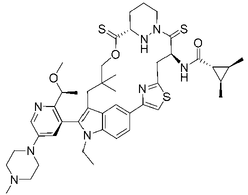 | 857.4 | 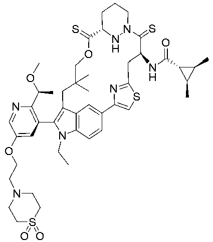 | 936.4 | 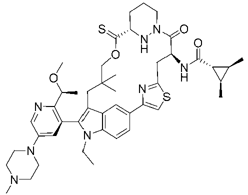 | 841.4 |
| Z757 | | Z758 | | Z759 | |
| 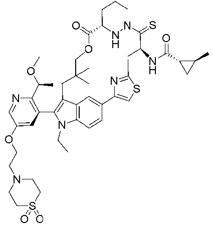 | 906.4 | 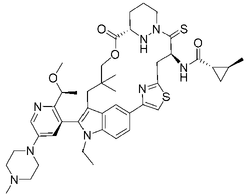 | 827.4 | 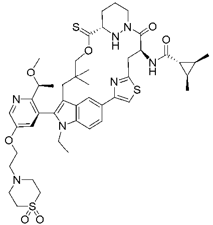 | 920.4 |
| Z761 | | Z762 | | Z760 | |
| 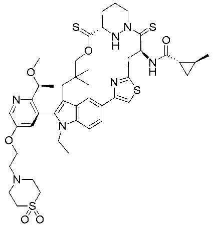 | 922.3 | 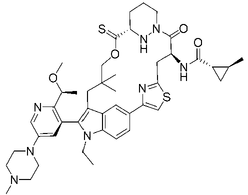 | 827.4 | 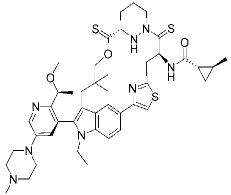 | 843.4 |
| Z764 | | Z765 | | Z763 | |
| 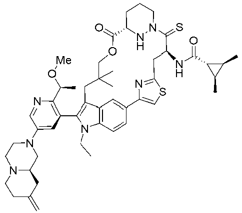 | 893.4 | 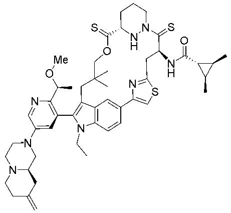 | 909.4 | 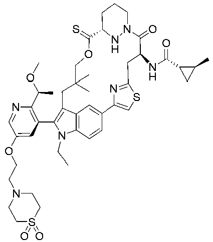 | 906.4 |
| Z767 | | Z768 | | Z766 | |
| 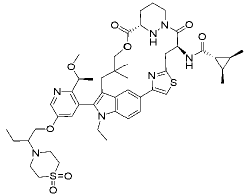 | 932.4 | 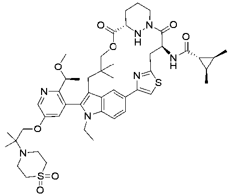 | 932.4 | 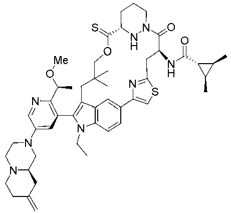 | 893.4 |
| Z658 | | Z776 | | Z769 | |
| 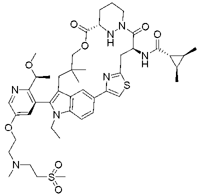 | 906.4 | 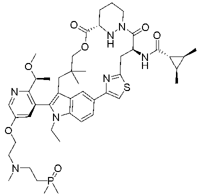 | 904.4 | 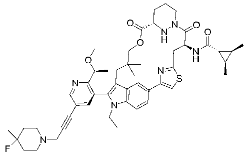 | 880.5 |
| Z781 | | Z782 | | Z779 | |
| 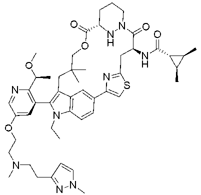 | 908.5 | 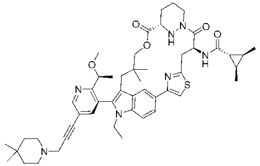 | 876.5 | | |
| Z785 | | Z783 | | | |
| 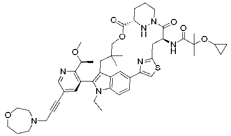 | 894.5 | 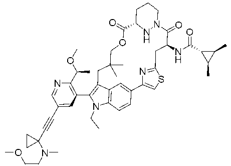 | 878.5 | 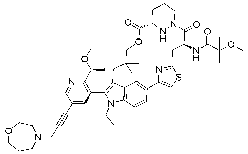 | 868.4 |
| Z787 | | Z789 | | Z786 | |
| 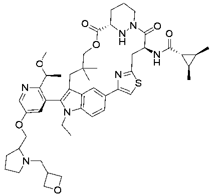 | 896.5 | 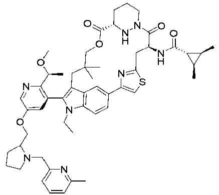 | 931.5 | 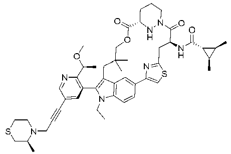 | 880.4 |
| Z790 | | Z791 | | Z792 | |
| 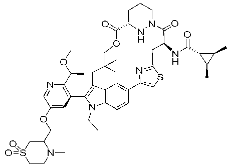 | 904.4 | 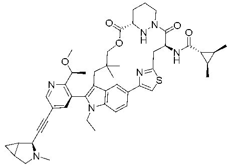 | 846.4 | 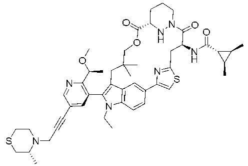 | 880.4 |
| Z795 | | Z797 | | Z799 | |
| 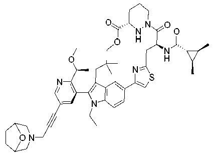 | 876.4 | 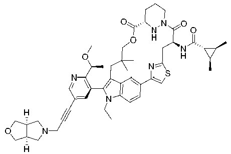 | 876.4 | 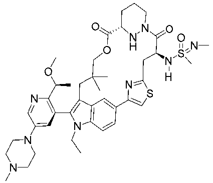 | 820.4 |
| Z800 | | Z801 | | Z804 | |
| 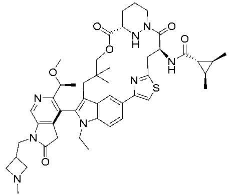 | 865.4 | 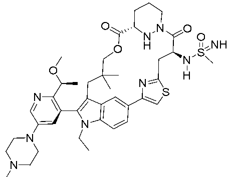 | 806.4 | 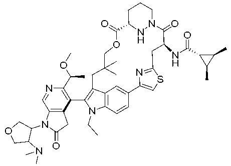 | 895.4 |
| Z807 | | Z805 | | Z806 | |
| 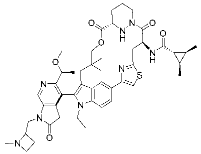 | 865.4 | 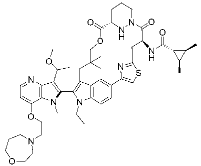 | 923.5 | | |
| Z808 | | Z809 | | | |
| 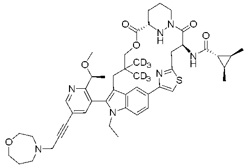 | 870.5 | 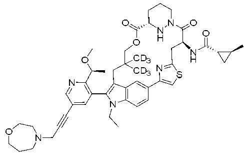 | 856.5 | 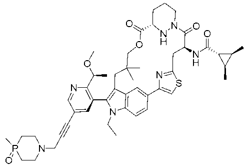 | 896.4 |
| Z815 | | Z816 | | Z829 | |
| 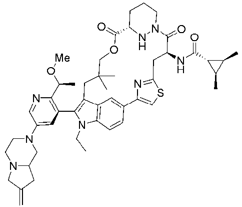 | 863.5 | 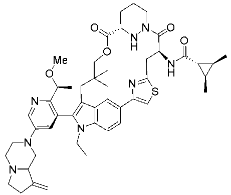 | 863.5 | 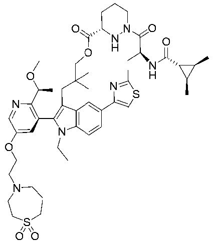 | 918.4 |
| Z830 | | Z831 | | Z832 | |
| 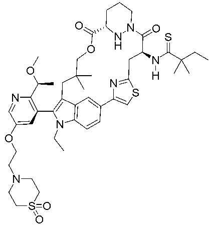 | 922.4 | 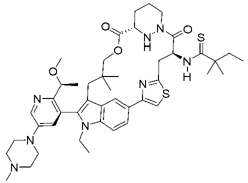 | 843.4 | 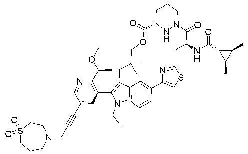 | 912.4 |
| Z833 | | Z835 | | Z846 | |
| 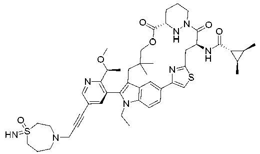 | 911.4 | 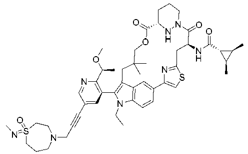 | 925.4 | 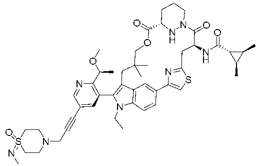 | 911.4 |
| Z848 | | Z849 | | Z851 | |
| 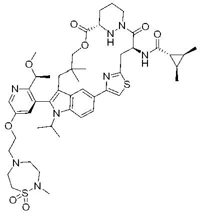 | 947.4 | | | | |
| Z852 | | | | | |
| 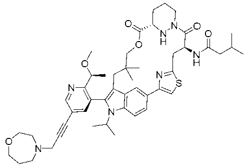 | 866.5 | 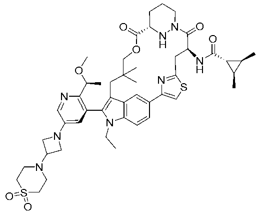 | 915.4 | 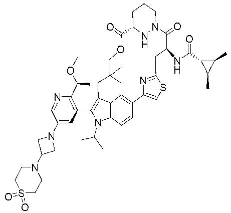 | 929.4 |
| Z853 | | Z854 | | Z856 | |
| 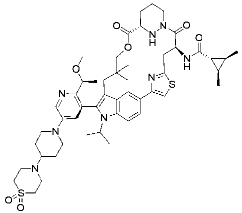 | 957.5 | 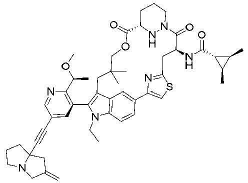 | 872.4 | 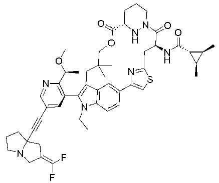 | 908.4 |
| Z857 | | Z858 | | Z859 | |
| 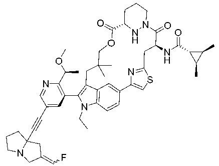 | 890.4 | 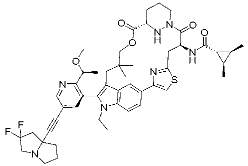 | 896.4 | 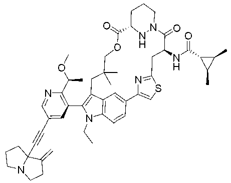 | 872.4 |
| Z860 | | Z861 | | Z862 | |
| 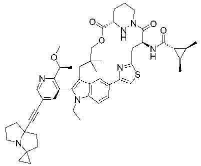 | 886.5 | 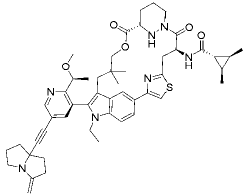 | 872.4 | 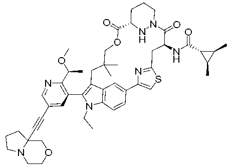 | 876.4 |
| Z863 | | Z864 | | Z865 | |
| 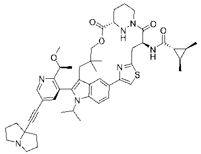 | 874.5 | 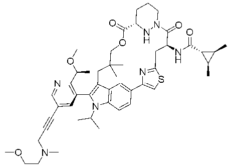 | 866.5 | 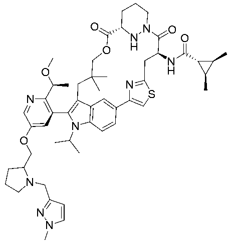 | 934.5 |
| Z866 | | Z867 | | Z868 | |
| 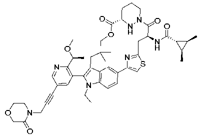 | 864.4 | 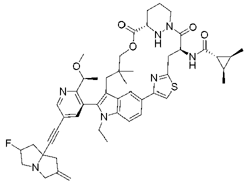 | 890.4 | 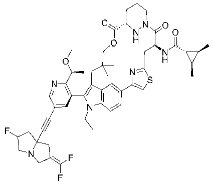 | 926.4 |
| Z869 | | Z870 | | Z871 | |
| 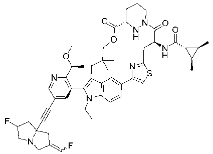 | 908.4 | 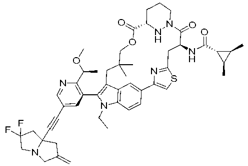 | 908.4 | 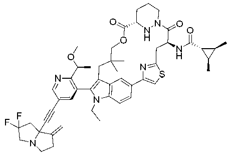 | 908.4 |
| Z872 | | Z873 | | Z874 | |
| 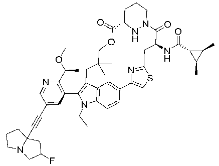 | 878.4 | 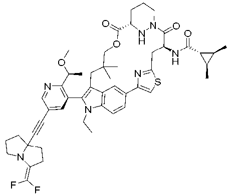 | 908.4 | 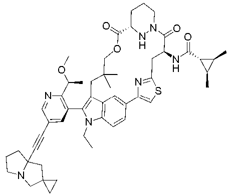 | 886.5 |
| Z875 | | Z876 | | Z877 | |
| 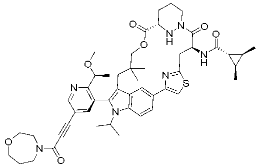 | 892.4 | 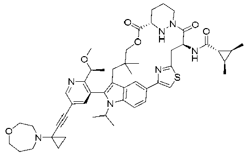 | 904.5 | 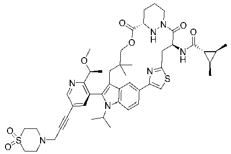 | 912.4 |
| Z878 | | Z879 | | Z880 | |
| 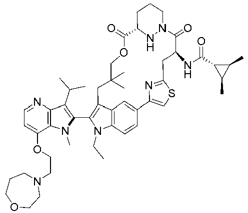 | 907.5 | 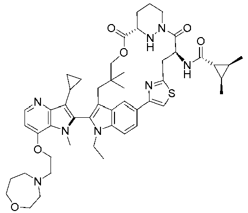 | 905.5 | 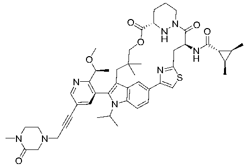 | 891.5 |
| Z881 | | Z882 | | Z883 | |
| 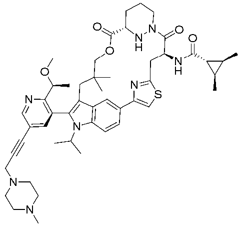 | 877.5 | 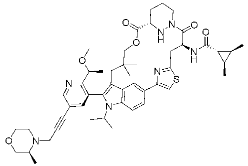 | 878.5 | 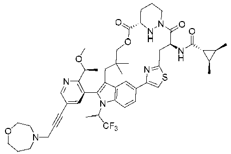 | 932.4 |
| Z884 | | Z885 | | Z886 | |
| 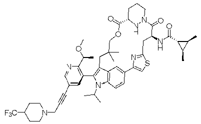 | 930.4 | 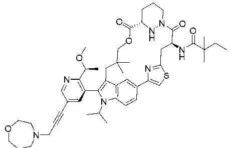 | 880.5 | 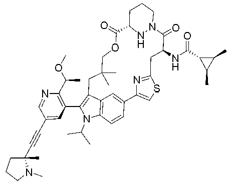 | 862.5 |
| Z887 | | Z888 | | Z889 | |
| 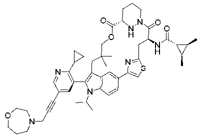 | 860.4 | 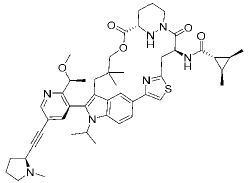 | 848.4 | 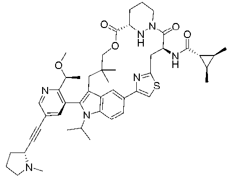 | 848.4 |
| Z890 | | Z891 | | Z892 | |
| 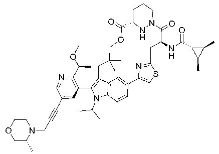 | 878.5 | 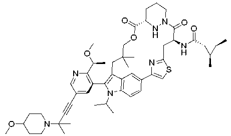 | 920.5 | 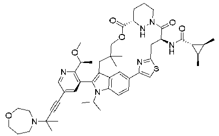 | 906.5 |
| Z893 | | Z894 | | Z895 | |
| 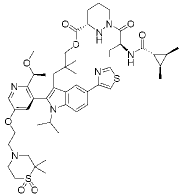 | 946.4 | 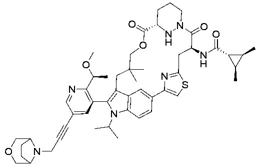 | 890.5 | 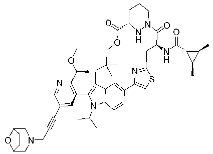 | 890.5 |
| Z896 | | Z897 | | Z898 | |
| 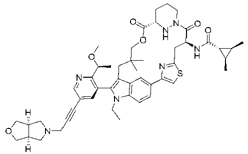 | 876.4 | 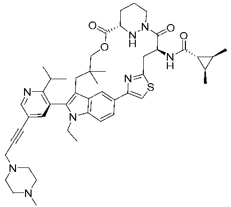 | 847.5 | 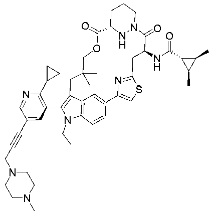 | 845.4 |
| Z899 | | Z900 | | Z901 | |
| 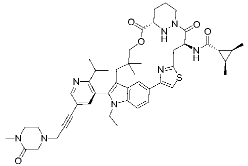 | 861.4 | 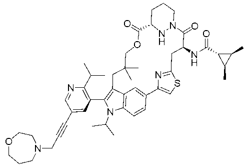 | 862.5 | 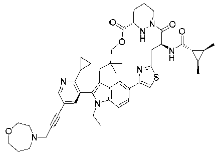 | 846.4 |
| Z902 | | Z903 | | Z904 | |
| 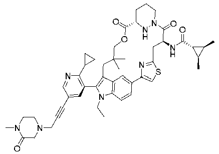 | 859.4 | 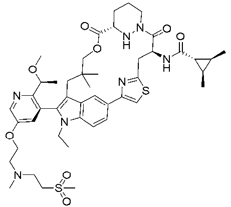 | 906.4 | 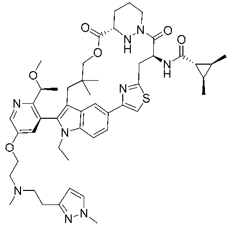 | 908.5 |
| Z905 | | Z906 | | Z907 | |
| 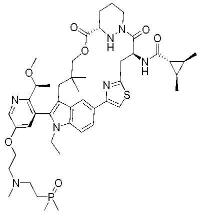 | 904.4 | 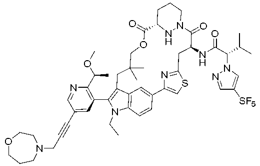 | 1044.4 | 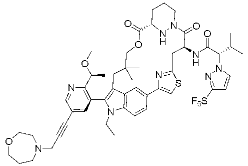 | 1044.4 |
| Z908 | | Z916 | | Z917 | |
| 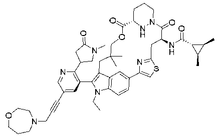 | 903.5 | 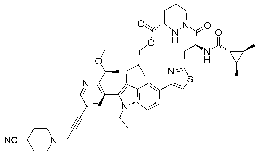 | 873.4 | 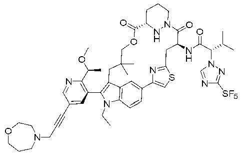 | 1045.4 |
| Z918 | | Z919 | | Z921 | |
| 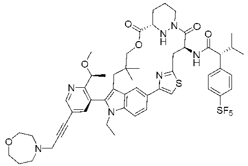 | 1054.4 | 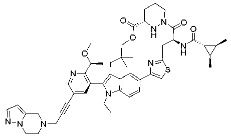 | 886.4 | 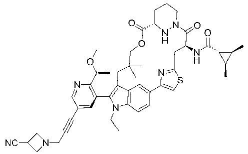 | 845.4 |
| Z922 | | Z923 | | Z924 | |
| 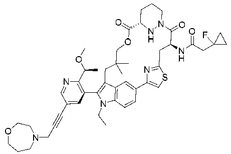 | 868.4 | 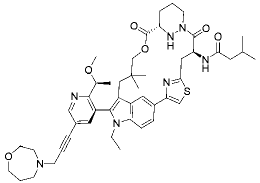 | 852.4 | 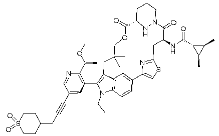 | 897.4 |
| Z926 | | Z928 | | Z929 | |
| 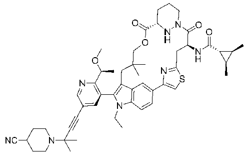 | 901.5 | 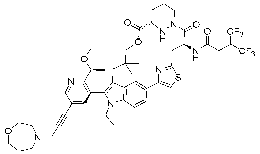 | 960.4 | 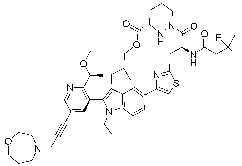 | 870.4 |
| Z930 | | Z931 | | Z933 | |
| 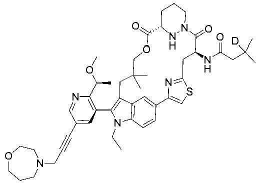 | 853.4 | 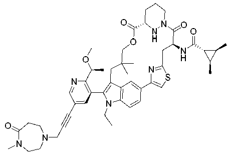 | 891.5 | 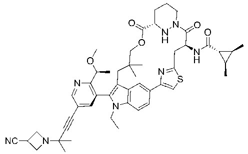 | 873.4 |
| Z934 | | Z935 | | Z936 | |
| 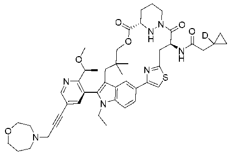 | 851.4 | 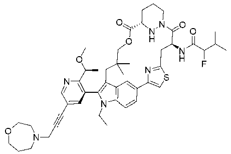 | 870.4 | 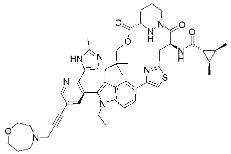 | 886.4 |
| Z937 | | Z938 | | Z941 | |
| 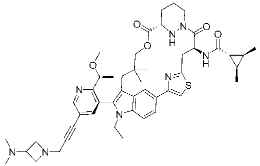 | 863.5 | 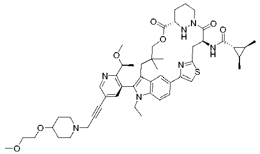 | 922.5 | 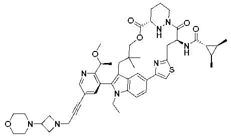 | 905.5 |
| Z942 | | 7943 | | Z944 | |
| 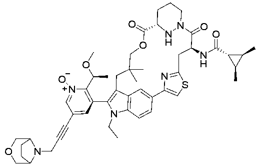 | 892.4 | 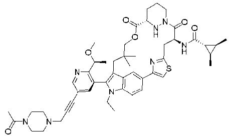 | 891.5 | | |
| Z946 | | Z947 | | | |
| 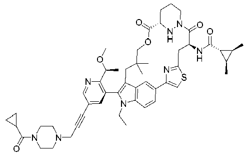 | 917.5 | 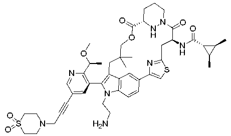 | 913.4 | 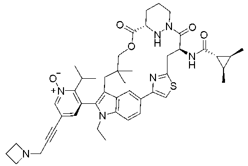 | 820.4 |
| Z948 | | Z953 | | Z997 | |
| 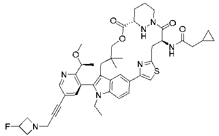 | 824.4 | 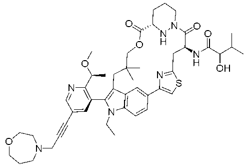 | 868.4 | 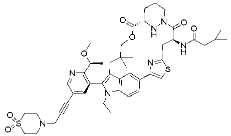 | 886.4 |
| Z998 | | Z1000 | | Z1001 | |
| 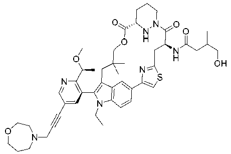 | 868.4 | 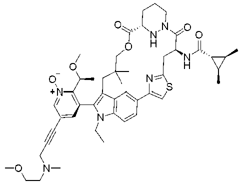 | 868.4 | 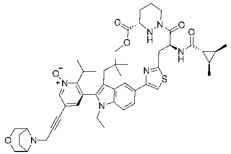 | 876.4 |
| Z1002 | | Z1003 | | Z1004 | |
| 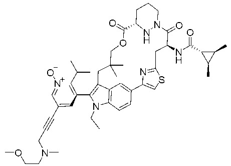 | 852.4 | 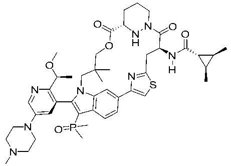 | 873.4 | 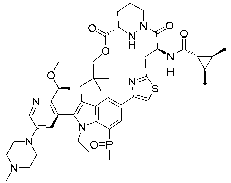 | 901.4 |
| Z1005 | | Z1006 | | Z1007 | |
| 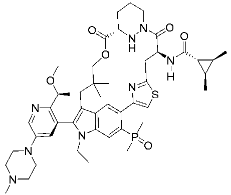 | 901.4 | 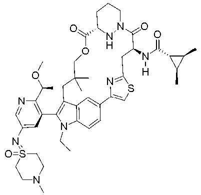 | 873.4 | 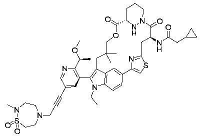 | 913.4 |
| Z1008 | | Z1009 | | Z1028 | |
| 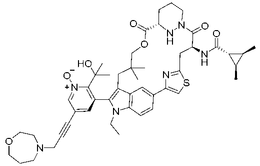 | 880.4 | 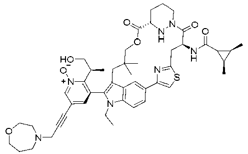 | 880.4 | | |
| **Z1040** | | Z1041 | | | |
| 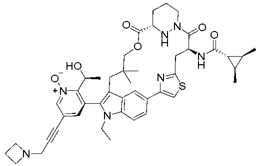 | 822.4 | 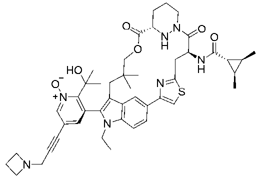 | 836.4 | 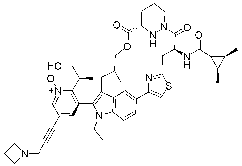 | 836.4 |
| Z1042 | | Z1043 | | Z1044 | |
| 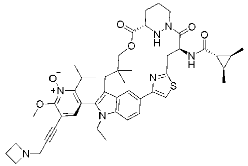 | 850.4 | 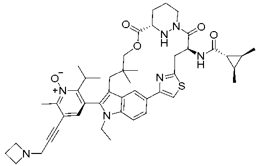 | 834.4 | 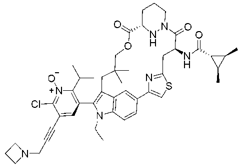 | 854.4 |
| Z1048 | | Z1049 | | Z1050 | |
| 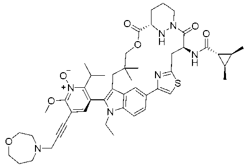 | 894.5 | 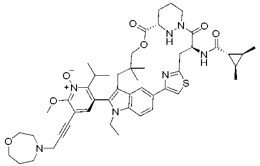 | 894.5 | 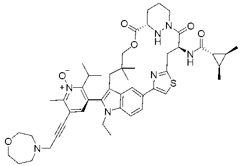 | 878.5 |
| Z1051 | | Z1052 | | Z1053 | |
| 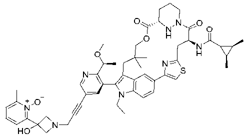 | 943.4 | 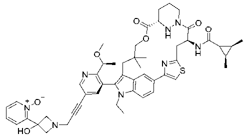 | 929.4 | | |
| Z1056 | | Z1057 | | | |
| 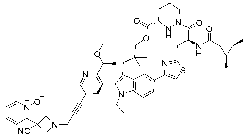 | 938.4 | 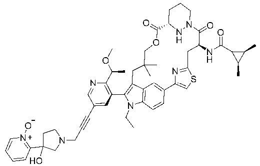 | 929.4 | 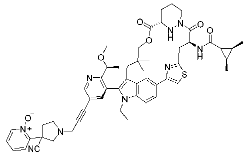 | 952.4 |
| Z1058 | | Z1059 | | Z1060 | |
| 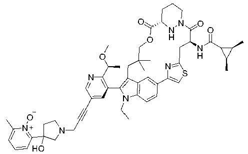 | 957.5 | 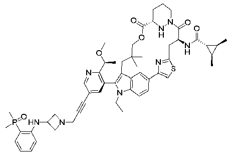 | 987.5 | 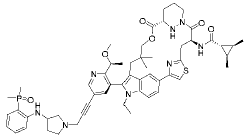 | 1001.5 |
| Z1061 | | Z1063 | | Z1064 | |
| 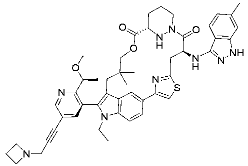 | 854.4 | 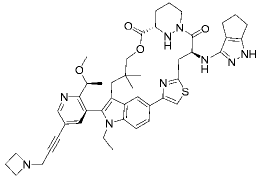 | 830.4 | 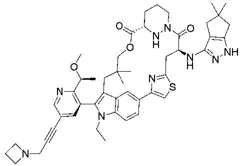 | 858.4 |
| Z1066 | | Z1067 | | Z1068 | |
| 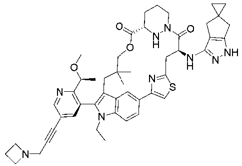 | 856.4 | 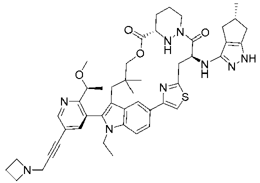 | 844.4 | 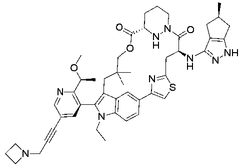 | 844.4 |
| Z1069 | | Z1070 | | Z1071 | |
| 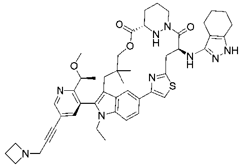 | 844.4 | 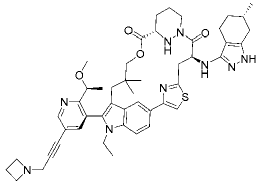 | 858.4 | 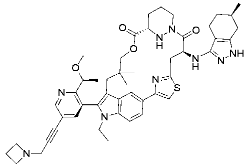 | 858.4 |
| Z1072 | | Z1073 | | Z1074 | |
| 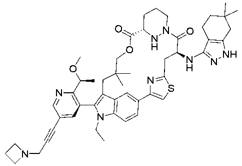 | 872.5 | 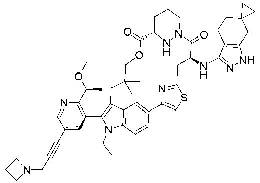 | 870.4 | 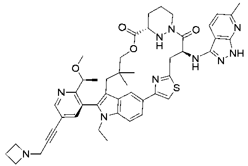 | 855.4 |
| Z1075 | | Z1076 | | Z1077 | |
| 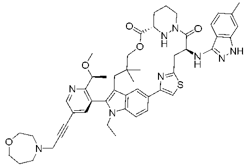 | 898.4 | 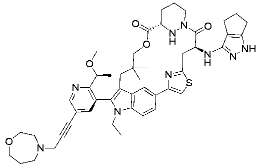 | 874.4 | 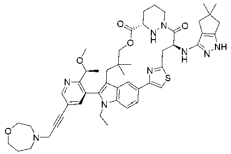 | 902.5 |
| Z1078 | | Z1079 | | Z1080 | |
| 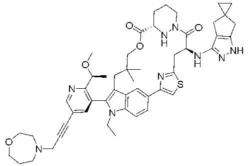 | 900.5 | 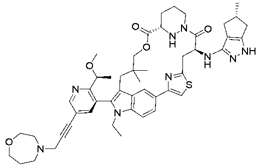 | 888.5 | 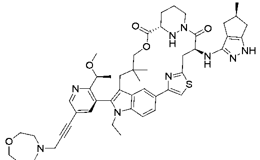 | 888.5 |
| Z1081 | | Z1082 | | Z1083 | |
| 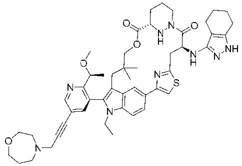 | 888.4 | 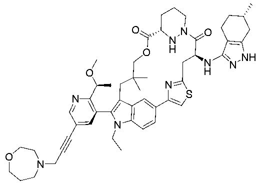 | 902.5 | 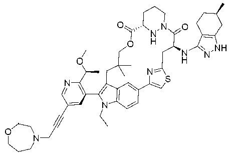 | 902.5 |
| Z1084 | | Z1085 | | Z1086 | |
| 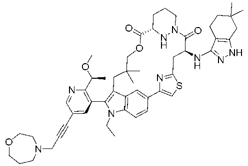 | 916.5 | 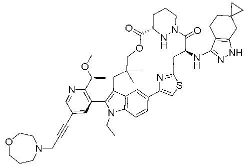 | 914.5 | 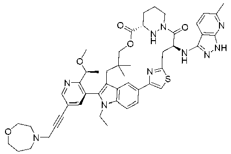 | 899.4 |
| Z1087 | | Z1088 | | Z1089 | |
| 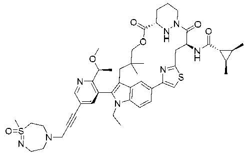 | 911.4 | 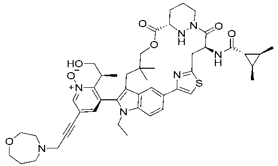 | 880.4 | 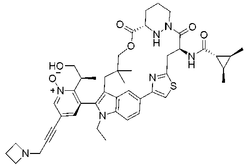 | 836.4 |
| Z1090 | | Z1041-1 | | Z1044-1 | |
| 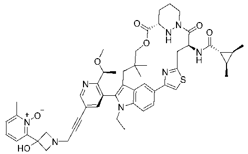 | 943.4 | 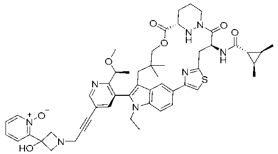 | 929.4 | 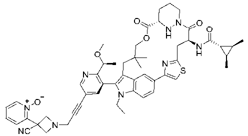 | 938.4 |
| Z1056-1 | | Z1057-1 | | Z1058-1 | |
| 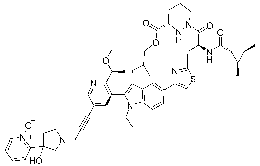 Z1059-1 | 943.4 | 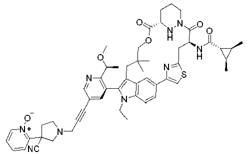 Z1060-1 | 952.4 | 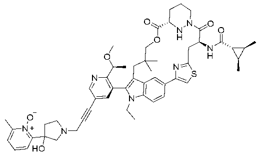 Z1061-1 | 957.5 |

### Test Example 1: Cell p-ERK Assay

AsPC-1 cells were purchased from ATCC, Cat. No. CRL-1682; NCI-H441 cells were purchased from ATCC, Cat. No. HTB-174; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; trypsin (containing EDTA) was purchased from Gibco, Cat. No. 25200-072; DMSO was purchased from VWRAMRESCO, Cat. No. 0231-500ML; 96-well cell culture plates were purchased from Corning, Cat. No. 3599; white-bottomed, opaque 96-well plates were purchased from Cisbio, Cat. No. 66PL96025; Advance ERK phospho-T202/Y204 kit was purchased from Cisbio, Cat. No. 64AERPEH; cell counter was purchased from CHEMOMETEC, model: NC-200; microplate reader was purchased from PerkinElmer, model: EnVision.

AsPC-1, a human pancreatic cancer cell strain endogenously containing a KRAS^{G12D} mutation, was cultured in a complete medium containing RPMI-1640 + 10% FBS + 1% PS. NCI-H441, a human non-small cell lung cancer cell strain endogenously containing a KRAS^{G12V} mutation, was cultured in a complete medium containing RPMI-1640 + 10% FBS + 1% PS.

The cells were digested with trypsin (containing EDTA), collected in their corresponding complete media, and counted. The density of the cell suspensions was adjusted with their corresponding complete media, and 10000 AsPC-1 cells or 20000 NCI-H441 cells per well were seeded into a 96-well cell culture plate, with the volume of the medium in each well being 80 µL. The cell culture plate was incubated in an incubator at 37 °C with 5% CO₂ overnight. A series of 3.16-fold gradient dilutions of a 1000× compound stock solution were prepared with DMSO, and then diluted 200-fold with the complete medium described above to give 5× compound stock solutions. On the day after cell seeding, 20 µL of the 5× compound stock solution was added to each cell culture well. As a result, the final concentration of the compound in the cell culture well became 1×, and the DMSO content in the well was 0.1%. In addition to the compound-treated wells described above, cell-containing DMSO control wells were also set. A complete medium containing 0.5% DMSO was added at 20 µL/well. After the mixture was well mixed, the culture plate was incubated in the incubator at 37 °C with 5% CO₂ for another 3 h. The medium was then removed from the culture wells, and 50 µL of cell lysis buffer (from Advance ERK phospho-T202/Y204 kit) was added to each well. The mixture was well mixed and incubated for 30 min. Then, 16 µL of the mixture was transferred to a white-bottomed, opaque 96-well assay plate, and separately, 16 µL of cell lysis buffer was added to additional wells (serving as blank wells) in the assay plate. After the transfer was completed, 4 µL of p-ERK HTRF antibody mixture (from Advance ERK phospho-T202/Y204 kit) was added to each well of the assay plate, followed by a 4-hour incubation. The HTRF signal values (RLU) were then measured using a microplate reader, and the inhibition rate of the compound against p-ERK levels was calculated as follows: IR (%) = (RLU_{DMSO} - RLU_{compound})/(RLUnuso - RLU _{blank}) × 100%, where RLU_{DMSO} is the average HTRF signal value from the DMSO control well samples, RLU_{blank} is the average HTRF signal value from the blank well samples, and RLU_{compound} is the HTRF signal value from the compound-treated well samples.Using an XLfit software, the IR (%) values were plotted against the logarithm of the compound concentrations, and the dose-response curves were fitted using the four-parameter method to calculate the IC₅₀ values.

The results showed that the compounds of the present disclosure exhibited relatively high inhibitory activity against p-ERK in the cancer cells described above. The IC₅₀ values of some of the compounds were less than 1 µM, and the IC₅₀ values of some of the compounds were less than 500 nM or 100 nM, or even less than 50 nM or 10 nM. The results for some of the exemplary compounds are shown in Table 3 below.

**Table 3**

| Number | H441 p-ERK IC₅₀(nM) | AsPC-1 p-ERK IC₅₀(nM) | Number | H441 p-ERK IC₅₀(nM) | AsPC-1 p-ERK IC₅₀(nM) |
|---|---|---|---|---|---|
| Z66 | 4.26 | 2.72 | Z501 | 0.22 | 0.35 |
| Z3 | 9.58 | 15.02 | Z667A | 1.6 | 2.6 |
| Z5 | 4.24 | 2.81 | Z677 | 0.29 | 0.35 |
| Z71 | 41.7 | 96.44 | Z667 | 0.38 | 0.63 |
| Z72 | 0.31 | 0.31 | Z672 | 0.39 | 0.39 |
| Z73 | 0.24 | 0.21 | Z673 | 0.33 | 0.27 |
| Z64 | 3.32 | 6.24 | Z484 | 0.67 | 1.3 |
| Z75 | 18.57 | 38.67 | Z643 | 0.22 | 0.51 |
| Z13 | 0.88 | 2.24 | Z665 | 0.11 | 0.32 |
| Z76 | 18.98 | 19.17 | Z711 | 0.23 | 0.32 |
| Z77 | 7.48 | 13.91 | Z812 | 1.06 | 1.69 |
| Z78 | 9.86 | 18.71 | Z810A | 0.63 | 1.22 |
| Z79 | 9.7 | 24.12 | Z780 | 1.56 | 3.35 |
| Z80 | 11.73 | 25.32 | Z814 | 0.14 | 0.15 |
| Z372 | 13.98 | 31.42 | Z813 | 0.08 | 0.08 |
| Z81 | 1.7 | 2.01 | Z793 | 0.09 | 0.19 |
| Z83 | 0.75 | 0.43 | Z778 | 0.37 | 1.53 |
| Z347 | 151.65 | - | Z703 | 0.17 | 0.36 |
| Z350 | 9.1 | 16.34 | Z811-2 | 0.21 | 0.58 |
| Z329 | 8.74 | 15.69 | Z836-1 | 0.83 | 0.5 |
| Z375 | 0.91 | 3.92 | Z645 | 0.48 | 0.15 |
| Z367 | 2.1 | 7.29 | Z678 | 0.17 | 0.06 |
| Z377 | 87.78 | - | Z788 | 0.19 | 0.38 |
| Z380 | 3.67 | 9.45 | Z796 | 0.55 | 0.42 |
| Z456 | 61.33 | - | Z794 | 0.49 | - |
| Z361 | 2.61 | 2.66 | Z810 | 1.65 | 1.35 |
| Z457 | 26.06 | 92.04 | Z803 | 1.14 | 0.85 |
| Z378-1 | 4.64 | 10.62 | Z798 | 3.89 | - |
| Z460 | 6.3 | 26.16 | Z784-1 | 0.9 | 0.89 |
| Z339 | 5.48 | 10.81 | Z784-2 | 0.8 | 0.66 |
| Z461 | 93.01 | - | Z838 | 0.15 | 0.08 |
| Z462 | 1.12 | 1.60 | Z802 | 0.4 | 0.2 |
| Z464 | 1.9 | 3.03 | Z855 | 0.17 | 0.29 |
| Z355 | 45.69 | 109.26 | Z911 | 0.74 | 2.23 |
| Z467 | 28.22 | 69.02 | Z912 | 1.83 | - |
| Z415 | 11.66 | 10.08 | Z914 | 0.46 | 0.25 |
| Z469 | 0.35 | 0.11 | Z915 | 0.68 | 1.26 |
| Z470 | 69.38 | 35.3 | Z914-1 | 0.27 | 0.19 |
| Z423 | 33.28 | - | Z927 | 1.77 | 3.89 |
| Z333 | 10.91 | 18.82 | Z932 | 1.22 | 2.14 |
| Z547 | 78.11 | - | Z920 | 9.41 | 33.81 |
| Z504 | 0.55 | 0.16 | Z955 | 2.6 | - |
| Z498 | 4.06 | 10.88 | Z912-1 | 2.61 | - |
| Z549 | 26.73 | 32.57 | Z912-2 | 3.41 | - |
| Z309 | 0.96 | 0.38 | Z958 | 5.78 | - |
| Z551 | 0.27 | 0.29 | Z932-2 | 1.16 | 2.62 |
| Z434 | 17.47 | 69.36 | Z932-1 | 1.02 | 2.28 |
| Z431 | 44.38 | - | Z957 | 2.63 | - |
| Z554 | 0.42 | 0.67 | Z960 | 5.46 | - |
| Z555 | 11.2 | 13.41 | Z959 | 1.01 | 4.15 |
| Z385 | 20.74 | 33.48 | Z950 | 0.16 | 0.27 |
| Z557 | 5.56 | 20.7 | Z962 | 0.15 | 0.26 |
| Z653 | 4.55 | 13.43 | Z847 | 0.13 | 0.11 |
| Z604 | 0.26 | 0.51 | Z1032 | 2.18 | 7.52 |
| Z613-2 | 0.34 | 0.68 | Z974 | 2.12 | - |
| Z485A | 1.14 | 1.45 | Z1011 | 1.22 | 2.89 |
| Z449 | 0.33 | 0.21 | Z1031 | 0.67 | 0.74 |
| Z383-1 | 2.79 | 7.39 | Z1030 | 0.17 | 0.17 |
| Z600-1 | 82.72 | - | Z979 | 1.05 | 2.47 |
| Z600 | 0.51 | 0.51 | Z981 | 2.17 | - |
| Z661-1 | 37.74 | 92.95 | Z999 | 2.16 | 3.57 |
| Z661-2 | 1.58 | 3.99 | Z1027 | 1.54 | - |
| Z598A | 1.7 | 4.13 | Z1014 | 3.35 | - |
| Z662 | 5.4 | 34.39 | Z951 | 0.5 | 0.42 |
| Z601 | 0.62 | 0.49 | Z952 | 0.54 | 0.53 |
| Z614 | 0.23 | 0.09 | Z850 | 0.25 | 0.18 |
| Z598 | 0.58 | 0.8 | Z945 | 0.18 | 0.28 |
| Z485 | 0.35 | 0.73 | Z1034 | 0.29 | 0.33 |
| Z605 | 0.3 | 0.6 | Z1045 | 0.19 | 0.36 |
| Z559 | 14.55 | 45.03 | Z1046 | 0.34 | 0.71 |
| Z628-1 | 0.35 | 0.31 | Z362-2 | 0.71 | 0.48 |
| Z625 | 0.2 | 0.33 | Z369-1 | 0.16 | 0.12 |
| Z777 | 22.17 | 61.87 | Z390-1 | 0.40 | 0.34 |
| Z607 | 0.45 | 0.54 | Z1047 | 0.22 | 0.24 |
| Z610 | 0.57 | 0.71 | Z1054 | 0.25 | 0.16 |
| Z609 | 0.76 | 0.76 | Z1065 | 0.31 | 0.42 |
| Z642 | 0.29 | 0.4 | Z1062 | 0.53 | 0.82 |
| Z603 | 0.22 | 0.6 | Z1039 | 0.51 | 0.95 |
| Z599 | 0.7 | 1.05 | Z1091 | 1.92 | 2.34 |
| Z700 | 0.21 | 0.16 | Z1055 | 0.19 | 0.32 |

| | | | | | |
|---|---|---|---|---|---|
| Note: "-" denotes that no test was performed. | | | | | |

### Test Example 2: 3D Cell Proliferation Assay

MIA PaCa-2 cells were purchased from ATCC, Cat. No. CRL-1420; AsPC-1 cells were purchased from ATCC, Cat. No. CRL-1682; NCI-H441 cells were purchased from ATCC, Cat. No. HTB-174; NCI-H1975 cells were purchased from ATCC, Cat. No. CRL-5908; DMEM medium was purchased from Gibco, Cat. No. 11995-065; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; Horse serum was purchased from Gibco, Cat. No. 16050130; trypsin (containing EDTA) was purchased from Gibco, Cat. No. 25200-072; DMSO was purchased from VWRAMRESCO, Cat. No. 0231-500ML; 384-well cell culture plates were purchased from Corning, Cat. No. 3830; white-bottomed, opaque 384-well assay plates were purchased from Corning, Cat. No. 3570; CellTiter Glo-3D kit was purchased from Promega, Cat. No. G9683; cell counter was purchased from CHEMOMETEC, model: NC-200; microplate reader was purchased from PerkinElmer, model: EnVision.

MIA PaCa-2, a human pancreatic cancer cell strain endogenously containing a KRAS^{G12C} mutation, was cultured in a complete medium containing DMEM + 10% FBS + 2.5% Horse serum + 1% PS. AsPC-1, a human pancreatic cancer cell strain endogenously containing a KRAS^{G12D} mutation, was cultured in a complete medium containing RPMI-1640 + 10% FBS + 1% PS. NCI-H441 cells, a human lung cancer cell strain endogenously containing a KRAS^{G12V} mutation, were cultured in a complete medium containing RPMI-1640 + 10% FBS + 1% PS. NCI-H1975, a KRAS wild-type human lung cancer cell strain whose cell proliferation depends on KRAS activity, was cultured in a complete medium containing RPMI-1640 + 10% FBS + 1% PS.

The cells were digested with trypsin (containing EDTA), collected in their respective low-serum complete media containing 2% FBS (for MIA Paca-2 cell culture: DMEM + 2% FBS + 0.5% Horse serum + 1% PS), and counted. After the cell density was adjusted as required, 200 MIA PaCa-2 cells, 800 AsPC-1 cells, 800 NCI-H441 cells, or 400 NCI-H1975 cells per well were seeded into a 384-well cell culture plate, with the volume of the medium in each well being 45 µL. The cell culture plate was incubated in an incubator at 37 °C with 5% CO₂ overnight. A series of 3.16-fold gradient dilutions of a 1000× compound stock solution were prepared with DMSO, and then diluted 100-fold with the complete medium containing 2% FBS described above to give 10× compound stock solutions. On the day after cell seeding, 5 µL of the 10× compound stock solution was added to each cell culture well. As a result, the final concentration of the compound in the cell culture well became 1×, and the DMSO content in the well was 0.1%. In addition to the compound-treated wells described above, cell-containing DMSO control wells (with 5 µL of the complete medium containing 1% DMSO added to each well) and cell-free medium control wells (containing 50 µL of the complete medium with 2% FBS) were also set. The culture plate was incubated in an incubator at 37 °C with 5% CO₂ for another 5 days, and then 30 µL of CellTiter-Glo working solution was added to each well. The mixture was well mixed, incubated for 30 min, left to stand at room temperature for 30 min, and then transferred to a white-bottomed, opaque 384-well assay plate at 40 µL/well. The chemiluminescence values (RLU) were measured using a microplate reader, and the cell proliferation inhibition rate for each well was calculated: IR (%) = (RLU_{DMSO} - RLU_{compound})/(RLU_{OMSO} - RLU_{blank}) × 100%, where RLU_{DMSO} is the average RLU signal value from the DMSO control well samples, RLU_{blank} is the average RLU signal value from the medium control well samples, and RLU_{compound} is the RLU signal value from the compound-treated well samples. Using an XLfit software, the IR (%) values were plotted against the logarithm of the compound concentrations, and the dose-response curves were fitted using the four-parameter method to calculate the IC₅₀ values.

The results showed that the compounds of the present disclosure exhibited relatively high inhibitory activity against the proliferation of KRAS mutant cancer cells, wherein the IC₅₀ values of some of the compounds were lower than 1000 nM, and the IC₅₀ values of some of the compounds were lower than 500 nM, or even lower than 100 nM, or even lower than 50 nM or 10 nM. The results for some of the exemplary compounds are shown in Table 4 below.

**Table 4**

| Number | H441 AP IC₅₀(nM) | AsPC-1 AP IC₅₀(nM) | Number | H441 AP IC₅₀(nM) | AsPC-1 AP IC₅₀(nM) |
|---|---|---|---|---|---|
| Z1 | 445.52 | 565.23 | Z603 | 0.31 | 0.89 |
| Z69 | 399.37 | 347.02 | Z599 | 1.17 | 2.15 |
| Z3 | 24.52 | 39.30 | Z700 | 0.17 | 0.18 |
| Z5 | 12.33 | 19.53 | Z501 | 0.22 | 0.52 |
| Z66 | 5.69 | 20.94 | Z667A | 1.31 | 2.97 |
| Z70 | 343.77 | 415.70 | Z677 | 0.2 | 0.53 |
| Z71 | 149.04 | 302.47 | Z667 | 0.71 | 1.63 |
| Z72 | 0.51 | 0.88 | Z672 | 0.3 | 0.73 |
| Z73 | 0.35 | 0.65 | Z673 | 0.23 | 0.52 |
| Z74 | 964.61 | - | Z484 | 0.59 | 3.19 |
| Z64 | 6.27 | 12.81 | Z643 | 0.3 | 0.64 |
| Z75 | 19.36 | 56.46 | Z665 | 0.21 | 0.78 |
| Z13 | 1.2 | 5.00 | Z711 | 0.27 | 0.3 |
| Z76 | 29.89 | 30.34 | Z812 | 1.64 | 3.52 |
| Z77 | 4.94 | 17.43 | Z780 | 1.58 | 4.51 |
| Z78 | 7.54 | 28.28 | Z814 | 0.18 | 0.14 |
| Z79 | 6.59 | 19.29 | Z813 | 0.15 | 0.18 |
| Z80 | 7.27 | 18.98 | Z793 | 0.14 | 0.19 |
| Z372 | 6.8 | 15.26 | Z778 | 1.14 | 2.39 |
| Z81 | 2.03 | 3.42 | Z703 | 0.3 | 0.68 |
| Z83 | 0.48 | 0.77 | Z811-2 | 0.63 | 1.41 |
| Z350 | 19.68 | 53.41 | Z836-1 | 0.64 | 1.05 |
| Z329 | 23.1 | 51.94 | Z645 | 0.5 | 0.81 |
| Z375 | 3.7 | 6.90 | Z678 | 0.06 | 0.17 |
| Z367 | 2.14 | 13.52 | Z788 | 0.28 | 0.78 |
| Z380 | 4.75 | 6.28 | Z796 | 0.28 | 1.1 |
| Z456 | 124.81 | 150.88 | Z794 | 0.66 | 2.07 |
| Z361 | 2.25 | 2.38 | Z810 | 1.83 | 3.22 |
| Z457 | 27.65 | - | Z803 | 0.61 | 1.49 |
| Z458 | 168.16 | - | Z784-1 | 0.93 | 1.37 |
| Z378-1 | 3.62 | 14.06 | Z784-2 | 0.53 | 0.83 |
| Z459 | 31.35 | - | Z838 | 0.06 | 0.03 |
| Z460 | 14.29 | 73.43 | Z802 | 0.26 | 0.34 |
| Z339 | 7.33 | 18.27 | Z855 | 0.16 | 0.22 |
| Z462 | 1.37 | 2.72 | Z911 | 1.16 | 2.27 |
| Z464 | 6.32 | 15.03 | Z914 | 0.26 | 0.58 |
| Z355 | 54.69 | - | Z915 | 1.04 | 2.82 |
| Z467 | 58.35 | - | Z913-1 | 4.18 | 8.88 |
| Z415 | 7.54 | 12.58 | Z914-1 | 0.11 | 0.24 |
| Z469 | 0.09 | 0.24 | Z927 | 3.86 | 6.34 |
| Z423 | 74.21 | - | Z932 | 1.68 | 4.14 |
| Z333 | 18.63 | 29.18 | Z920 | 14.97 | 38.33 |
| Z504 | 0.31 | 0.4 | Z955 | 2.96 | 4.82 |
| Z309 | 0.62 | 2.09 | Z912-1 | 5.13 | - |
| Z434 | 18.12 | 104.47 | Z912-2 | 8.59 | - |
| Z431 | 100.98 | - | Z958 | 4.93 | 6.12 |
| Z555 | 18.39 | 30.43 | Z932-2 | 2.32 | 5.52 |
| Z498 | 5.58 | 8.99 | Z932-1 | 2.25 | 3.82 |
| Z551 | 0.37 | 0.32 | Z957 | 2.47 | 6.69 |
| Z554 | 0.83 | 1.18 | Z966 | 4.91 | 9.41 |
| Z557 | 7.66 | 16.31 | Z847 | 0.29 | 0.13 |
| Z653 | 4.06 | 21.15 | Z1032 | 8.14 | 20.95 |
| Z604 | 0.45 | 0.74 | Z974 | 3.46 | 5.6 |
| Z613-2 | 0.7 | 0.79 | Z970 | 7.2 | - |
| Z485A | 1 | 2.4 | Z1011 | 1.54 | 2.07 |
| Z449 | 0.34 | 0.45 | Z982 | 2.57 | - |
| Z383-1 | 6.04 | 5.52 | Z1031 | 0.91 | 1.47 |
| Z600-1 | 96.33 | - | Z1030 | 0.28 | 0.39 |
| Z600 | 0.36 | 0.66 | Z979 | 1.23 | 5.14 |
| Z661-1 | 42.57 | 97.18 | Z951 | 0.43 | 0.36 |
| Z661-2 | 0.83 | 2.68 | Z952 | 0.34 | 0.27 |
| Z598A | 4.73 | 6.52 | Z850 | 0.13 | 0.08 |
| Z662 | 10.97 | 66.97 | Z954 | 2.33 | - |
| Z601 | 0.36 | 0.58 | Z945 | 0.23 | 0.23 |
| Z614 | 0.15 | 0.11 | Z1034 | 0.28 | 0.20 |
| Z598 | 0.65 | 0.62 | Z1045 | 0.17 | 0.21 |
| Z485 | 0.42 | 0.96 | Z1046 | 0.34 | 0.63 |
| Z605 | 0.26 | 0.5 | Z362-2 | 0.67 | 0.82 |
| Z559 | 17.51 | 47.55 | Z369-1 | 0.21 | 0.14 |
| Z628-1 | 0.34 | 0.62 | Z390-1 | 0.48 | 0.54 |
| Z625 | 0.24 | 0.36 | Z1047 | 0.24 | 0.37 |
| Z777 | 18.18 | 87.9 | Z1054 | 0.15 | 0.08 |
| Z607 | 0.36 | 1.12 | Z1065 | 0.4 | 0.43 |
| Z610 | 0.32 | 1.37 | Z1062 | 1.3 | 0.94 |
| Z609 | 0.22 | 0.79 | Z1039 | 1.21 | 1.28 |
| Z642 | 0.36 | 0.65 | Z1091 | 2.01 | 1.67 |

| | | | | | |
|---|---|---|---|---|---|
| Note: "-" denotes that no test was performed. | | | | | |

### Test Example 3: Assay for Stability in Liver Microsomes

This experiment was intended to investigate the metabolic stability of the test compounds in human liver microsomes. The materials used in the experiment included 10 mM solutions of the test compounds and the control sample, human liver microsomes, and reduced nicotinamide adenine dinucleotide phosphate (NADPH) powder. In the experiment, a 100 mM potassium phosphate buffer was used as the buffer system.

The liver microsome working solution was prepared by diluting the liver microsome stock solution to 0.75 mg/mL using the 100 mM potassium phosphate buffer. Then, 5 µL of the 10 mM stock solution of the test compound was taken, and 195 µL of methanol was added. The mixture was well mixed to prepare a working solution A with a concentration of 0.25 mM. Subsequently, the working solution A was diluted to 1.5 µM using the liver microsome working solution to prepare a compound-liver microsome mixed solution. Additionally, NADPH powder was dissolved in a 100 mM potassium phosphate buffer to prepare a 6 mM NADPH solution. The compound-liver microsome mixed solution was dispensed into a 96-well plate at 30 µL/well, with labeled time points of 0 min, 30 min, and 60 min. For 0-min samples, 180 µL of acetonitrile containing 100 ng/mL tolbutamide and 15 µL of the NADPH solution were added directly. The 96-well plate and the NADPH solution were then preheated under a 37 °C water bath for 5 min. After the preheating, 15 µL of the NADPH solution was added to each of the 30-min and 60-min sample wells. The plate was gently shaken for mixing and then incubated under the 37 °C water bath for the corresponding time. After the incubation was completed, 180 µL of acetonitrile containing 100 ng/mL tolbutamide was added to each sample well. The plate was sealed with a sealing film, shaken for 10 min (800 rpm/min), and then centrifuged for 15 min (4000 rpm). After the centrifugation, 50 µL of the supernatant was taken and added to a 96-well plate containing 150 µL of deionized water, and then the plate was sealed and shaken for 10 min (800 rpm) for uniform mixing. Subsequently, sample analysis was performed using liquid chromatography-tandem mass spectrometry (LC-MS/MS). The elimination rate constant and clearance rate of the test compound in liver microsomes were calculated using the following formulas: Remaining rate (%) = (peak area ratio of the compound to internal standard at any time point/peak area ratio of the compound to internal standard at 0 min) × 100%. T_{1/2} = 0.693/kₑ, where kₑ is the elimination rate constant and T_{1/2} is the half-life. Liver microsome intrinsic clearance rate (CL_{int(mic)}) = 0.693/(T_{1/2} × microsome protein concentration (mg/mL)). Liver intrinsic clearance rate (CLᵢₙₜ₍ₗᵢᵥₑᵣ₎) = CL_{int(mic)} × microsome protein-to-liver weight ratio (mg/g) × liver-to-body weight ratio (g/kg). In the formulas, CL_{int(mic)} is the liver microsome intrinsic clearance rate, CLᵢₙₜ₍ₗᵢᵥₑᵣ₎ is the liver intrinsic clearance rate, and CL₍ₗᵢᵥₑᵣ₎ is the liver clearance rate. For humans, the microsome protein-to-liver weight ratio was 45 mg/g, and the liver-to-body weight ratio was 20.7 g/kg.

The experimental results (Table 5) showed that the liver intrinsic clearance rates of the compounds of the present disclosure were generally lower than that of control compound 1, indicating that the compounds of the present disclosure had superior stability in human liver microsomes.

**Table 5. Assay results of stability in human liver microsomes**

| Number | T_{1/2} (min) | CLᵢₙᵢ₍ₗᵢᵥₑᵣ₎ (mL/min/kg) |
|---|---|---|
| Control compound 1 | 11.8 | 135.8 |
| Z802 | 98.7 | 16.2 |
| Z378-1 | 64.2 | 25.0 |
| Z367 | 48.2 | 33.2 |
| Z911 | 36.0 | 44.5 |
| Z13 | 36.7 | 43.7 |
| Z811-2 | 38.9 | 41.2 |
| Z778 | 50.3 | 31.9 |
| Z838 | 25.0 | 64.2 |

### Test Example 4: Pharmacokinetic Study in Rats

A total of 6 healthy adult male Sprague-Dawley (SD) rats were provided by Zhejiang Vital River Laboratory Animal Technology Co., Ltd. For each test compound, 6 healthy adult male SD rats were selected and randomly divided into 2 groups: an intravenous administration (IV) group and an intragastric administration (PO) group. The LC-MS/MS method was used to determine the drug concentrations in the plasma of rats at different time points after intravenous and intragastric administration of the test compounds. This experiment was intended to investigate the pharmacokinetic performance of the test compounds in rats and to evaluate the pharmacokinetic characteristics. Each drug used in the experiment was prepared from the test compound powder: An appropriate amount of test compound powder was weighed out, and appropriate amounts of DMSO, a 10% aqueous Solutol HS 15 solution, and a 6% aqueous HP-β-CD solution were sequentially added according to a volume ratio of 5:10:85. The mixture was vortexed and sonicated until clarification. The final concentration of the drug in the IV group was 0.5 mg/mL, and the final concentration of the drug in the PO group was 1 mg/mL; the concentrations could be adjusted according to actual needs. For the drug in the PO group of Z504, an appropriate amount of test compound powder was weighed out, and an appropriate amount of 0.5% MC + 0.2% Tween 80 aqueous solution was added. The mixture was well mixed by vortexing to give a 1 mg/mL homogeneous suspension; the concentration could be adjusted according to actual needs.

All experimental rats were fasted overnight, and food intake was resumed 4 h after administration. For the rats in the IV group, the administration volume was set at 2 mL/kg, with Z554 and Z915 administered at a dose of 0.5 mg/kg and the other compounds at a dose of 1 mg/kg. For the rats in the PO group, the administration volume was 10 mL/kg and the dose was 10 mg/kg. The administration amount was adjusted according to the body weight of the rats on the day of administration. In the experiment, blood samples were collected from the rats in the IV group at multiple time points (0.0833 h, 0.25 h, 0.5 h, 1 h, 2 h, 4 h, 7 h, and 24 h) after administration, while blood samples were collected from the PO group at 0.25 h, 0.5 h, 1 h, 2 h, 4 h, 7 h, and 24 h after administration, with approximately 100 µL collected each time via the orbital blood collection method. The blood samples were placed in centrifuge tubes containing EDTA-K2 and centrifuged at 8000 rpm for 4 min within 30 min to separate the supernatant plasma. The resulting plasma samples were stored at -20 °C for later use. For sample analysis, 20 µL of each of plasma samples and calibration standards was added to a 96-well plate, and 160 µL of acetonitrile containing 100 ng/mL tolbutamide was added. The samples were vortexed for 5 min for uniform mixing, and then centrifuged at 4000 rpm for 15 min. After the centrifugation was completed, 50 µL of supernatant was added to a 96-well plate containing 150 µL of deionized water. The plate was then sealed and shaken for uniform mixing. Sample analysis was performed by LC-MS/MS. The sampling volume could be adjusted according to actual needs, but the final concentration should be kept unchanged.

The experimental results showed that compared with control compound 1, the compounds of the present disclosure exhibited lower clearance rates (CL) in rats, and the drug exposure (AUC) after intragastric administration and the maximum concentration (Cₘₐₓ) after oral administration were significantly higher than those of control compound 1. These data are detailed in Table 6. These results revealed the superiority of the compounds of the present disclosure in terms of pharmacokinetic properties, particularly in terms of drug absorption and metabolism performance in rats.

**Table 6. Pharmacokinetic parameters in rats**

| Number | IV | | PO | | |
|---|---|---|---|---|---|
| | Dose (mg/kg) | CL (mL/min/kg) | Dose (mg/kg) | Cₘₐₓ (ng/mL) | AUC (hr*ng/mL) |
| Control compound 1 | 1 | 42.8 | 10 | 365 | 1221 |
| Z813 | 1 | 14.9 | 10 | 401 | 1643 |
| Z700 | 1 | 7.66 | 10 | 681 | 3259 |
| Z778 | 1 | 5.31 | 10 | 702 | 4020 |
| Z504 | 1 | 4.00 | 10 | 605 | 5322 |
| Z711 | 1 | 3.82 | 10 | 1867 | 8876 |
| Z788 | 1 | 3.58 | 10 | 2110 | 8529 |
| Z554 | 0.5 | 2.52 | 10 | 2777 | 16626 |
| Z793 | 1 | 3.16 | 10 | 2043 | 5925 |
| Z672 | 1 | 11.8 | 10 | 738 | 2940 |
| Z915 | 0.5 | 6.00 | 10 | 1728 | 6837 |
| Z838 | 1 | 2.51 | 10 | 1397 | 3896 |
| Z604 | 1 | 2.46 | 10 | 3010 | 14611 |
| Z803 | 1 | 1.83 | 10 | 1114 | 8403 |
| Z811-2 | 1 | 1.14 | 10 | 4303 | 29002 |
| Z911 | 1 | 0.41 | 10 | 427 | 2540 |

Although specific embodiments of the present disclosure have been described in detail, various modifications and substitutions can be made to the details of the technical solutions of the present disclosure by those skilled in the art according to all the teachings that have been disclosed, and these changes are all encompassed within the claimed scope of the present disclosure. The entire scope of the present disclosure is given by the appended claims and any equivalents thereof.

## Claims

1. A compound represented by formula (I), or a stable deuterated derivative thereof, or a stereoisomer thereof, or a pharmaceutically acceptable salt thereof, a solvate thereof, or a prodrug thereof: 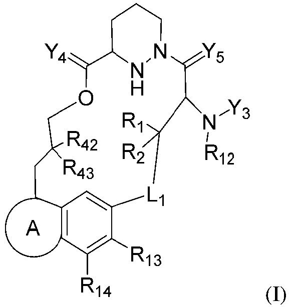 wherein in the formula, 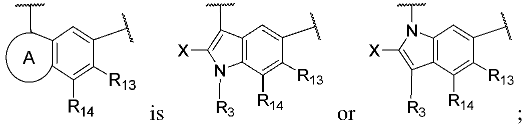
X is 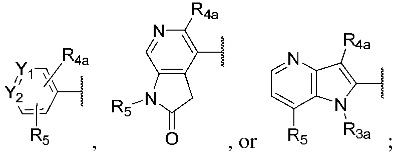
Y₁ is N, CH, CR₄ₐ, CR_{4b}, or CR₅, wherein N may be oxidized (N⁺-O⁻);
Y₂ is N, CH, CR₄ₐ, CR_{4b}, or CR₅, wherein N may be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl or substituted or unsubstituted 8- to 10-membered bicyclic heteroaryl, or -P(=O)-(C₁₋₆ alkyl)₂;
R₄ₐ is substituted or unsubstituted C₁₋₆ alkyl, substituted or unsubstituted C₃₋₆ cycloalkyl, or substituted or unsubstituted 3- to 8-membered heterocyclyl;
R_{4b} is deuterium, halogen, substituted or unsubstituted C₁₋₆ alkyl, or substituted or unsubstituted C₁₋₆ heteroalkyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, - O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), - S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)-C₁₋₄ alkyl-NR₀₁R₀₂), - S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 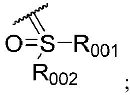 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), -C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form substituted or unsubstituted 3- to 8-membered heterocyclyl;
Y₅ is O or S;
Y₄ is O or S;
R₁₃ is hydrogen, halogen, substituted or unsubstituted C₁₋₆ alkyl, or -P(=O)-(C₁₋₆ alkyl)₂; R₁₄ is hydrogen, halogen, substituted or unsubstituted C₁₋₆ alkyl, or -P(=O)-(C₁₋₆ alkyl)₂; or
R₁₃ and R₁₄, together with the carbon atoms to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, - C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, - C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, - C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkylS(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, - C₁₋₄ alkyl-C(=O)-C₆₋₁₄ aryl, -C(=O)-5 or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, - NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, -N=S(=O) (R^{g1})(R^{h1}), and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, - NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C ₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, - (C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted by -(CH₂)ⱼ- to form cycloalkyl, wherein j is 2, 3, 4, 5, or 6; or 2 hydrogen atoms on the same carbon atom of the C₁₋₄ alkyl are both substituted by =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus sulfur atom is optionally substituted with one or two groups selected from oxo and =NR^{e}; R^{e} is as defined above;
the proviso is that when R₆ is dimethylcyclopropyl, Y₄ is O, Y₅ is O, R₁ and R₂ are hydrogen, L₁ is thiazole, R₄₂ and R₄₃ are methyl, and R₁₃ and R₁₄ are hydrogen, and when R₃ is ethyl, R₅ is not methylpiperazinyl.

2. The compound, or the stable deuterated derivative thereof, or the stereoisomer thereof, or the pharmaceutically acceptable salt thereof, the solvate thereof, or the prodrug thereof according to claim 1, wherein the compound is represented by formula (A) or formula (A'): 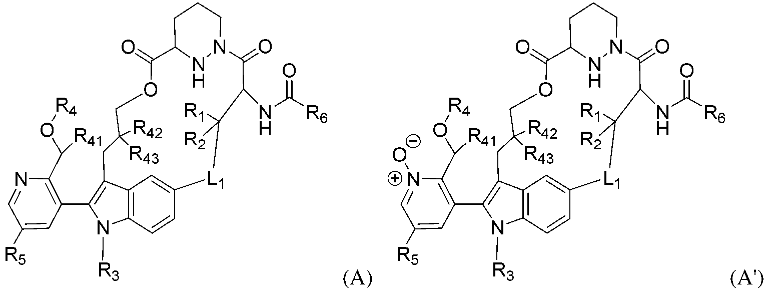 wherein in the formula,
R₄₂ is hydrogen, halogen, or substituted or unsubstituted C₁₋₆ alkyl; R₄₃ is hydrogen, halogen, or substituted or unsubstituted C₁₋₆ alkyl; and R₄₂ and R₄₃ are not both methyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is -NR₀₁R₀₂ or -C(R₀₅)R₀₃R₀₄;
R₀₁ and R₀₂, together with the nitrogen atom to which they are attached, form substituted or unsubstituted 5- to 15-membered tricyclic heterocyclyl; the 5- to 15-membered tricyclic heterocyclyl has, in addition to the existing nitrogen atom, 0 or more (e.g., 0, 1, 2, 3, 4, or 5) ring atoms that are heteroatoms selected from nitrogen, oxygen, and sulfur;
R₀₃ and R₀₄, together with the carbon atom to which they are attached, form substituted or unsubstituted 9- to 11-membered bicyclic heterocyclyl, substituted or unsubstituted 5- to 15-membered tricyclic heterocyclyl, substituted or unsubstituted 5- or 6-membered monocyclic heteroaryl, or substituted or unsubstituted 8- to 10-membered bicyclic heteroaryl; one or more (e.g., 1, 2, 3, 4, or 5) ring atoms of the 5- to 15-membered tricyclic heterocyclyl are heteroatoms selected from nitrogen, oxygen, and sulfur;
R₀₅ is hydrogen, deuterium, or C₁₋₆ alkyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; the substitutions each independently refer to that 1, 2, 3, or 4 hydrogen atoms on the group described above are substituted by a group selected from group S1; the groups of group S1 are each independently selected from the group consisting of: deuterium, oxo (=O), thio (=S), =CR^{e}R^{f}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, - C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, - C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, - C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkylS(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, and -NR^{d1}-S(=O)₂-NR^{a1}R^{b1}, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8-to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the group consisting of: oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, and -(C=O)C₁₋₆ alkyl;
in the groups described above, the -C₁₋₄ alkyl- is unsubstituted; or 1, 2, 3, or 4 hydrogen atoms on the -C₁₋₄ alkyl- are each independently substituted by a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkyl- are both substituted by -(CH₂)ⱼ- to form cycloalkyl, wherein j is 2, 3, 4, 5, or 6;
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 9- to 11-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur.

3. The compound, or the stable deuterated derivative thereof, or the stereoisomer thereof, or the pharmaceutically acceptable salt thereof, the solvate thereof, or the prodrug thereof according to claim 1 or 2, wherein the compound is represented by formula (A-1), formula (A-2), formula (A'-1), or formula (A'-2): 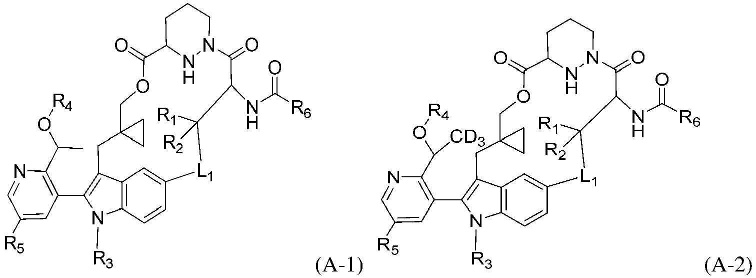 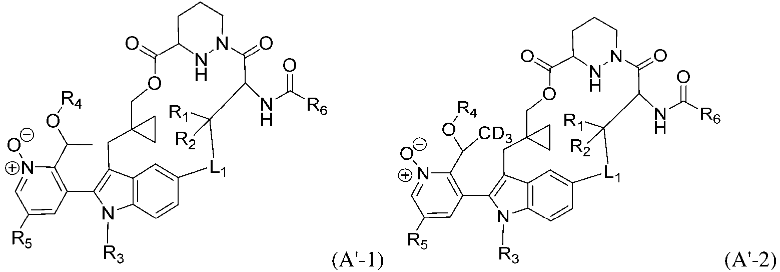 wherein in the formula, R₁, R₂, R₃, R₄, R₅, R₆, and L₁ are each as defined above.

4. The compound, or the stable deuterated derivative thereof, or the stereoisomer thereof, or the pharmaceutically acceptable salt thereof, the solvate thereof, or the prodrug thereof according to claim 1 or 2, wherein the compound is represented by formula (A-3) or formula (A'-3): 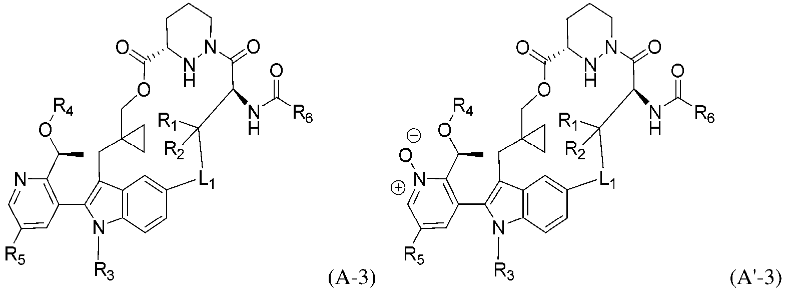 wherein in the formula, R₁, R₂, R₃, R₄, R₅, R₆, and L₁ are each as defined above.

5. The compound, or the stable deuterated derivative thereof, or the stereoisomer thereof, or the pharmaceutically acceptable salt thereof, the solvate thereof, or the prodrug thereof according to claim 1 or 2, wherein R₄ is methyl or deuterated methyl.

6. The compound, or the stable deuterated derivative thereof, or the stereoisomer thereof, or the pharmaceutically acceptable salt thereof, the solvate thereof, or the prodrug thereof according to claim 1 or 2, wherein R₅ is -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, or -C₁₋₄ alkyl-C₂₋₄ alkynyl-C₁₋₄ alkyl-NR₀₁R₀₂.

7. The compound, or the stable deuterated derivative thereof, or the stereoisomer thereof, or the pharmaceutically acceptable salt thereof, the solvate thereof, or the prodrug thereof according to claim 1 or 2, wherein R₅ is 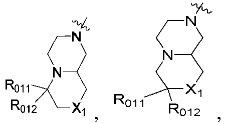 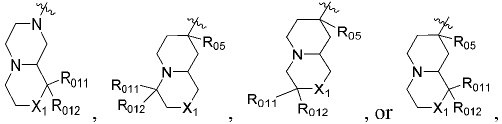 wherein R₀₁₁ and R₀₁₂ are each independently hydrogen, C₁₋₆ alkyl, C₁₋₆ haloalkyl, or deuterated C₁₋₆ alkyl; or R₀₁₁ and R₀₁₂, together with the carbon atom to which they are attached, form substituted or unsubstituted C₃₋₆ cycloalkyl; the substitution is as defined above; X₁ is a bond, S, O, or N(R₀₁₃); R₀₁₃ is hydrogen, C₁₋₆ alkyl, or deuterated C₁₋₆ alkyl; R₀₅ is hydrogen, deuterium, or C₁₋₆ alkyl.

8. The compound, or the stable deuterated derivative thereof, or the stereoisomer thereof, or the pharmaceutically acceptable salt thereof, the solvate thereof, or the prodrug thereof according to claim 1 or 2, wherein R₃ is hydrogen, C₁₋₆ alkyl, C₁₋₆ haloalkyl, or deuterated C₁₋₆ alkyl.

9. The compound, or the stable deuterated derivative thereof, or the stereoisomer thereof, or the pharmaceutically acceptable salt thereof, the solvate thereof, or the prodrug thereof according to claim 1 or 2, wherein R₁ is hydrogen, C₁₋₆ alkoxy, or deuterated C₁₋₆ alkoxy; R₂ is hydrogen.

10. The compound, or the stable deuterated derivative thereof, or the stereoisomer thereof, or the pharmaceutically acceptable salt thereof, the solvate thereof, or the prodrug thereof according to claim 1 or 2, wherein L₁ is 5- or 6-membered monocyclic heteroaryl.

11. The compound, or the stable deuterated derivative thereof, or the stereoisomer thereof, or the pharmaceutically acceptable salt thereof, the solvate thereof, or the prodrug thereof according to claim 1, wherein the compound is represented by formula (C): 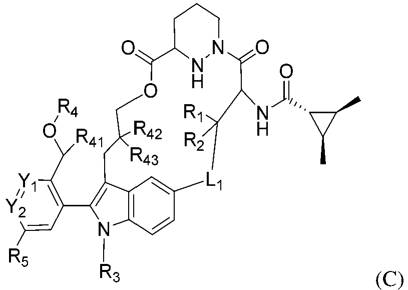 wherein in the formula, Y₁, Y₂, R₁, R₂, R₃, R₄, R₅, R₄₁, R₄₂, R₄₃, and L₁ are each as defined above.

12. The compound, or the stable deuterated derivative thereof, or the stereoisomer thereof, or the pharmaceutically acceptable salt thereof, the solvate thereof, or the prodrug thereof according to claim 1, wherein the compound is represented by formula (D): 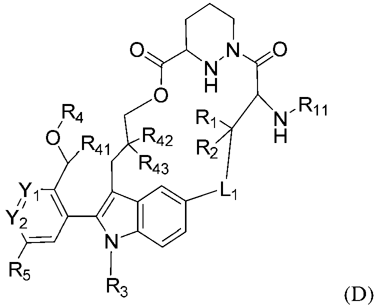 wherein in the formula, Y₁, Y₂, R₁, R₂, R₃, R₄, R₅, R₁₁, R₄₁, R₄₂, R₄₃, and L₁ are each as defined above.

13. The compound, or the stable deuterated derivative thereof, or the stereoisomer thereof, or the pharmaceutically acceptable salt thereof, the solvate thereof, or the prodrug thereof according to claim 12, wherein the compound is represented by formula (D-1), formula (D-2), formula (D-3), formula (D-4), formula (D'-1), formula (D'-2), formula (D'-3), or formula (D'-4): 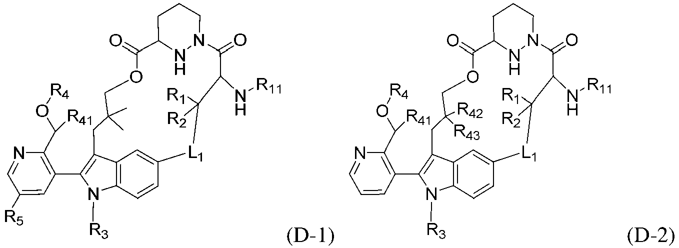 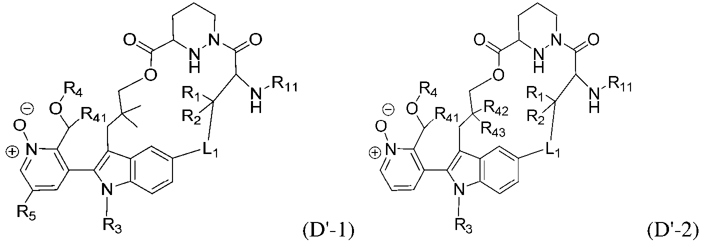 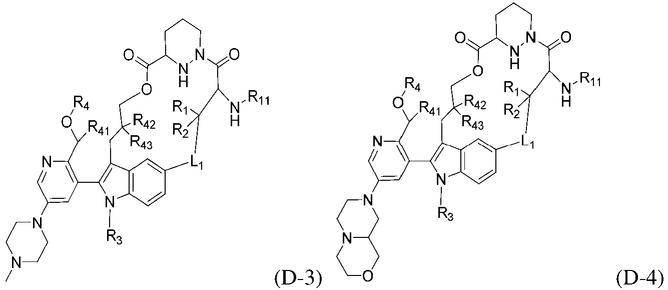 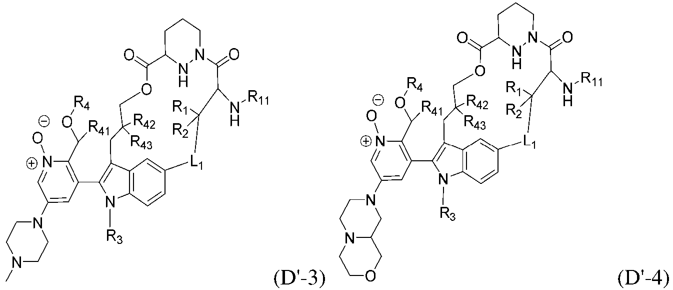 wherein in the formula, R₁, R₂, R₃, R₄, R₅, R₄₁, R₄₂, R₄₃, R₁₁, and L₁ are each as defined above.

14. The compound, or the stable deuterated derivative thereof, or the stereoisomer thereof, or the pharmaceutically acceptable salt thereof, the solvate thereof, or the prodrug thereof according to claim 1, wherein the compound is represented by formula (E): 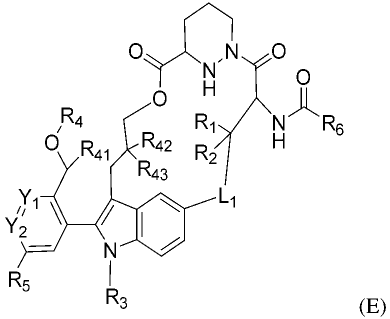 wherein in the formula, R₁, R₂, R₃, R₄, R₅, R₆, R₄₁, R₄₂, R₄₃, Y₁, Y₂, and L₁ are each as defined above.

15. The compound, or the stable deuterated derivative thereof, or the stereoisomer thereof, or the pharmaceutically acceptable salt thereof, the solvate thereof, or the prodrug thereof according to claim 14, wherein the compound is represented by formula (E1) or (E2): 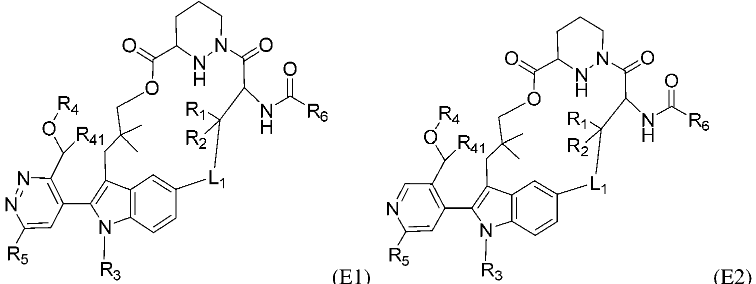 wherein in the formula, R₁, R₂, R₃, R₄, R₅, R₆, R₄₁, and L₁ are each as defined above.

16. The compound, or the stable deuterated derivative thereof, or the stereoisomer thereof, or the pharmaceutically acceptable salt thereof, the solvate thereof, or the prodrug thereof according to claim 1, wherein the compound is represented by formula (F) or formula (G): 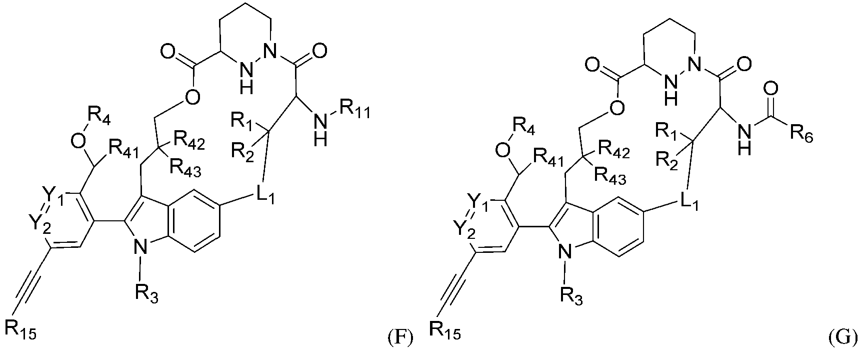
wherein in the formula, R₁, R₂, R₃, R₄, R₆, R₄₁, R₄₂, R₄₃, R₁₁, Y₁, Y₂, and L₁ are each as defined above;
R₁₅ is selected from -NR₀₁R₀₂, -C(=O)-NR₀₁R₀₂, -C(R₀₅)R₀₃R₀₄, -C₁₋₄ alkyl-NR₀₁R₀₂, and -C₁₋₄ alkyl-C(R₀₅)R₀₃R₀₄;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or,
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3-to 8-membered monocyclic heterocyclyl; or
R₀₁ and R₀₂ together form 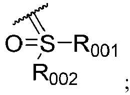 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted by -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
the substitutions each independently refer to that 1, 2, 3, or 4 hydrogen atoms on the group described above are substituted by a group selected from group S1; the groups of group S1 are as defined above.

17. The compound, or the stable deuterated derivative thereof, or the stereoisomer thereof, or the pharmaceutically acceptable salt thereof, the solvate thereof, or the prodrug thereof according to claim 1 or 12, wherein R₅ is hydrogen,

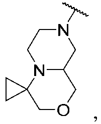

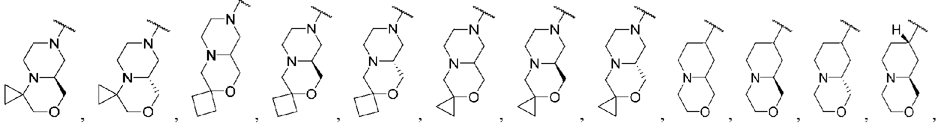

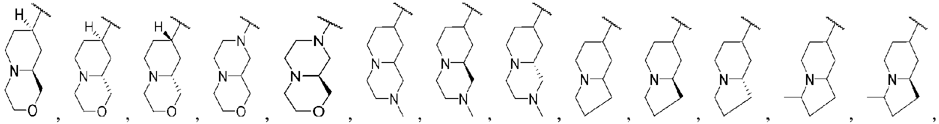

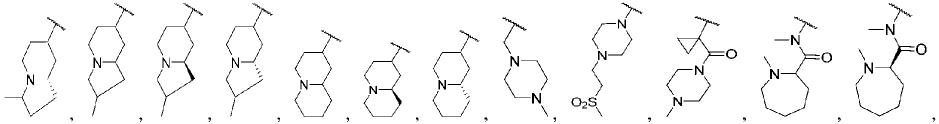

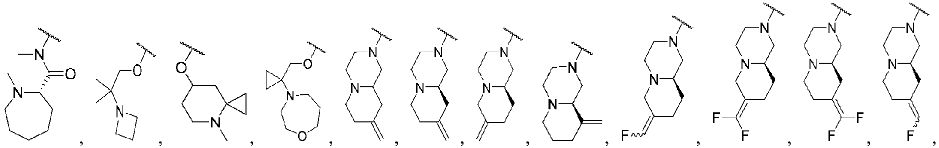

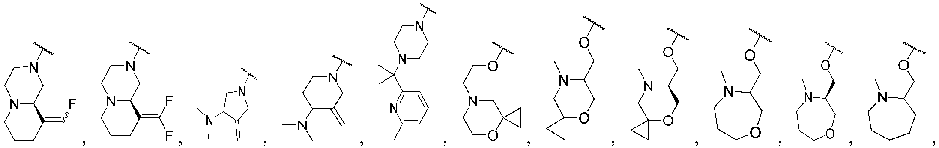

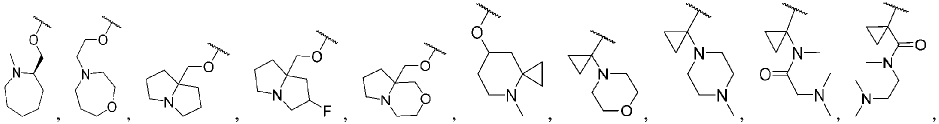

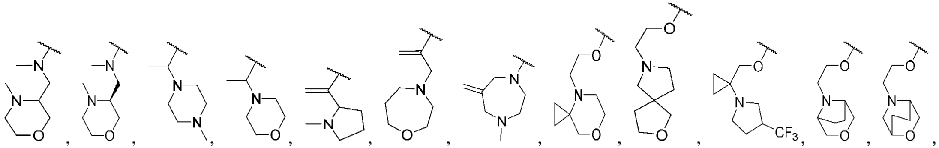

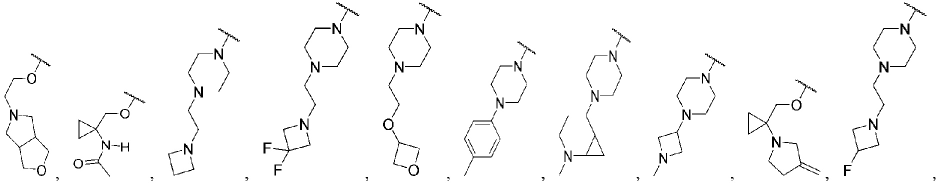

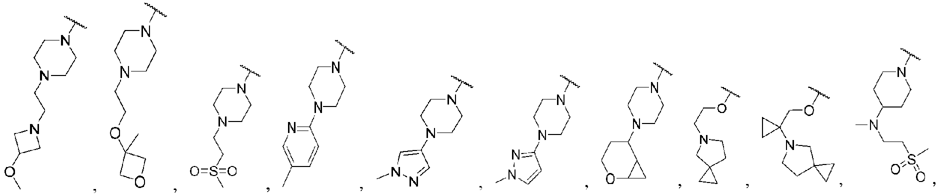

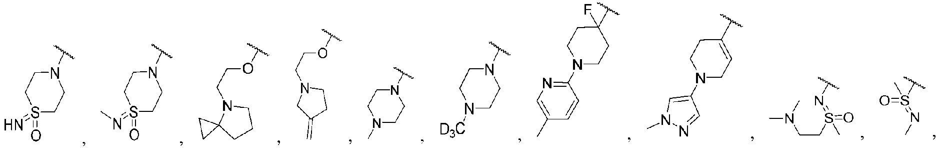

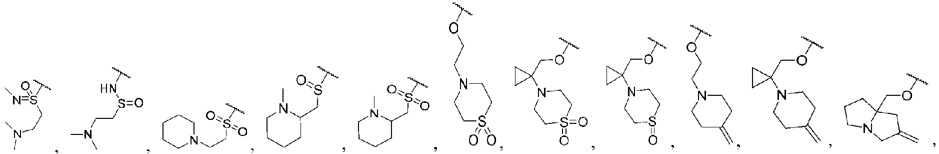

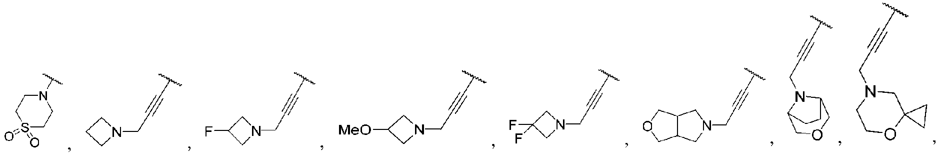

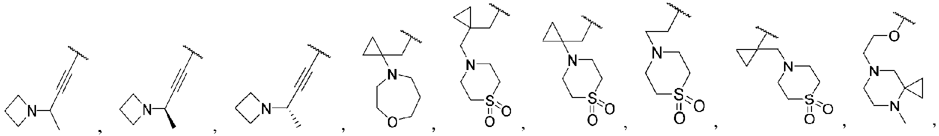

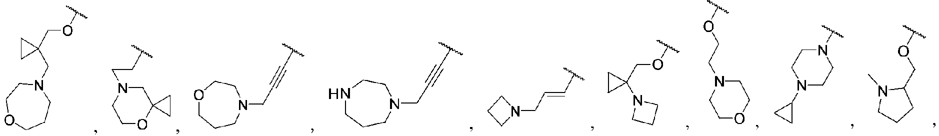

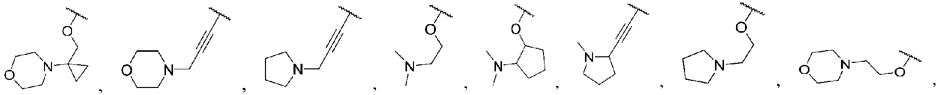

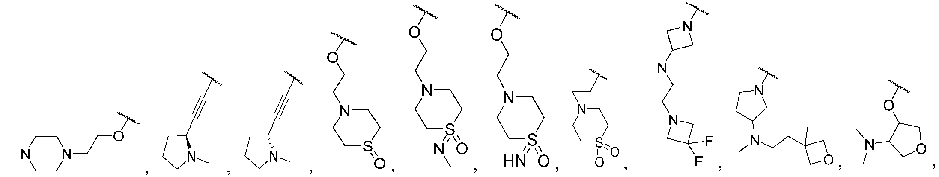

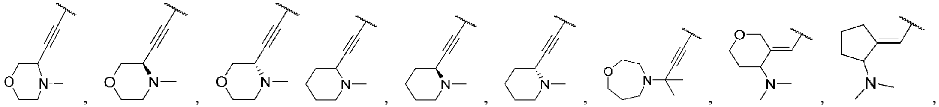

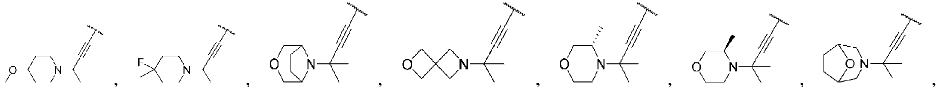

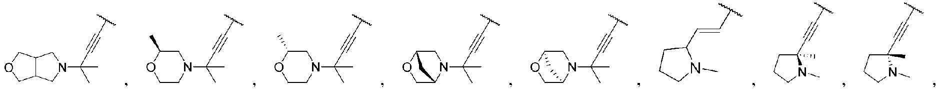

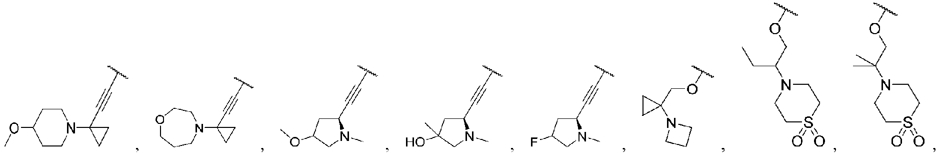

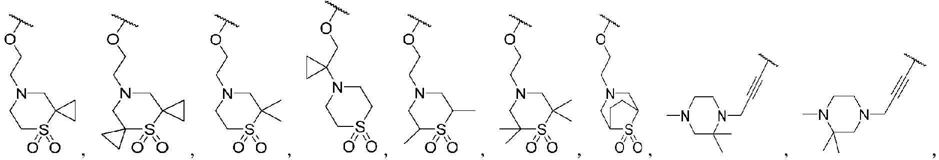

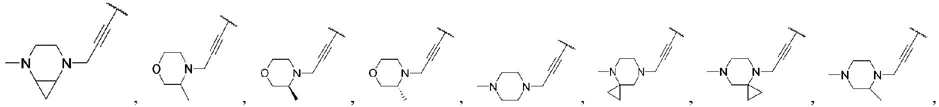

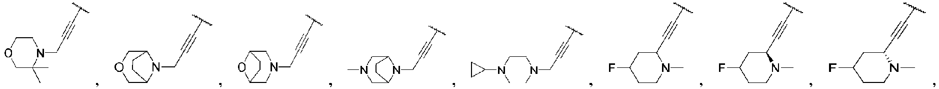

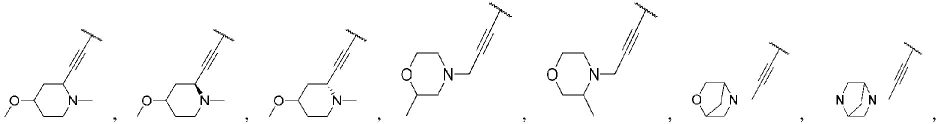

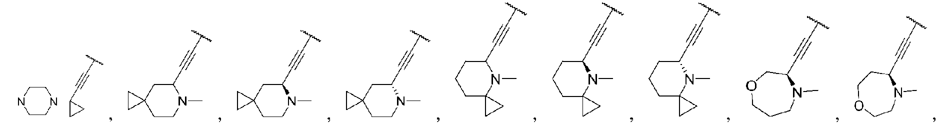

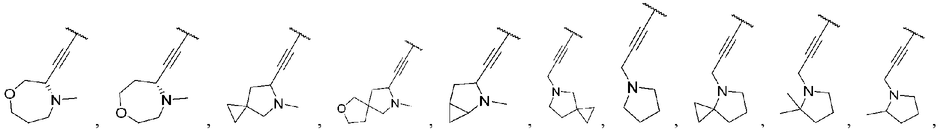

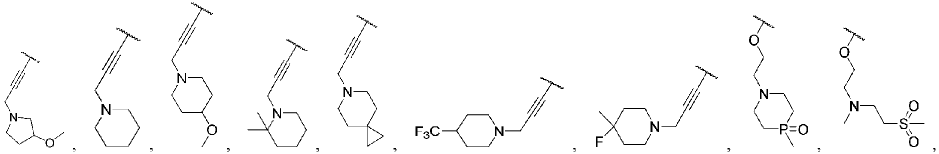

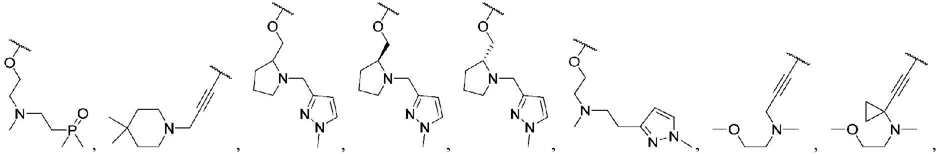

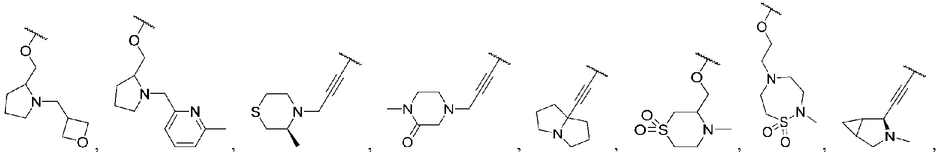

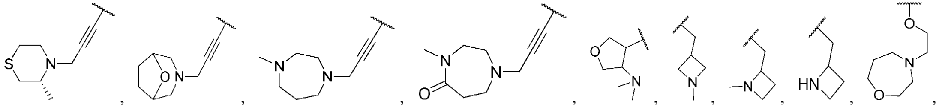

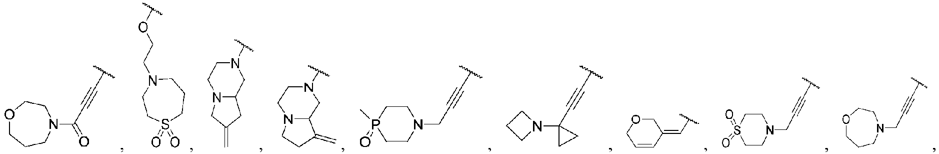

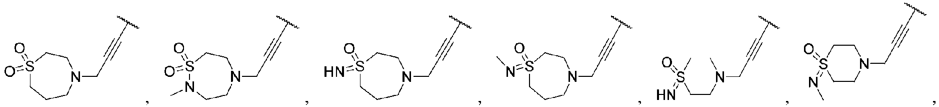

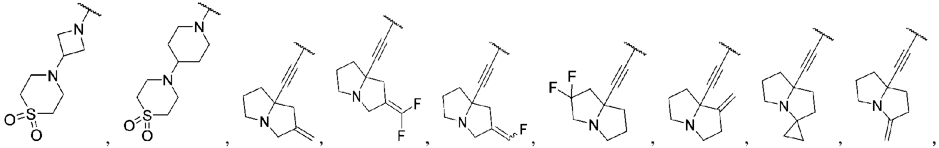

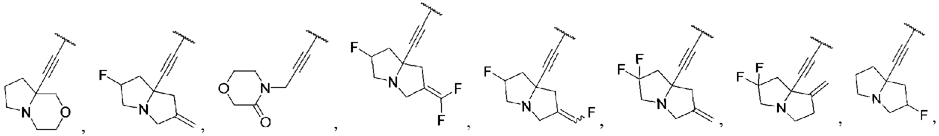

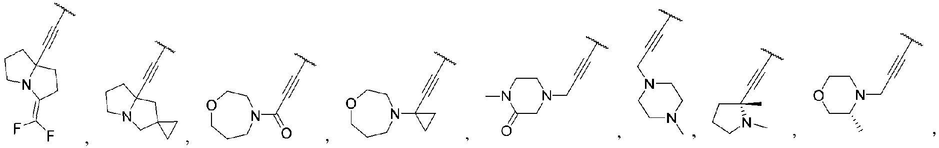

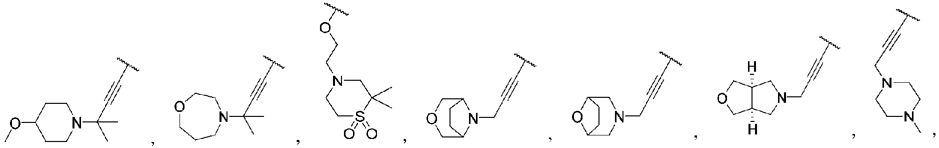

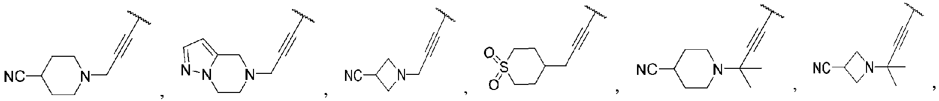

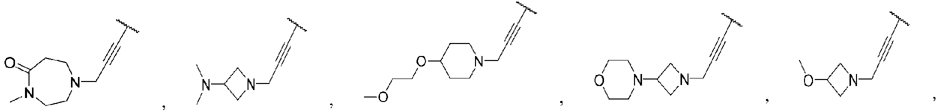

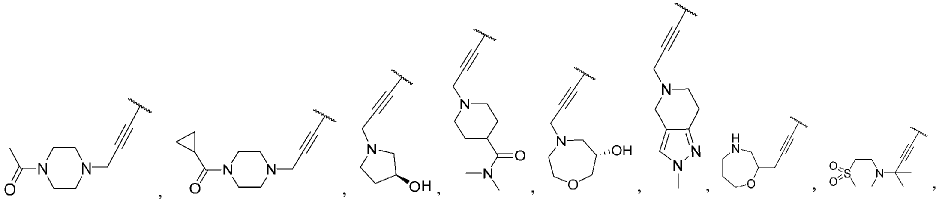

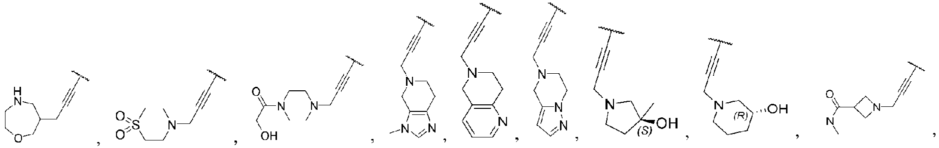

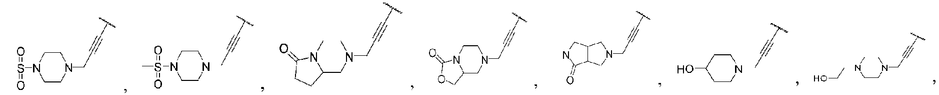

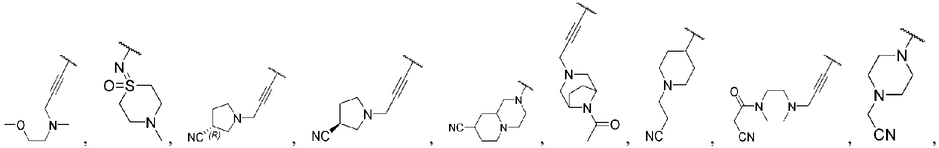

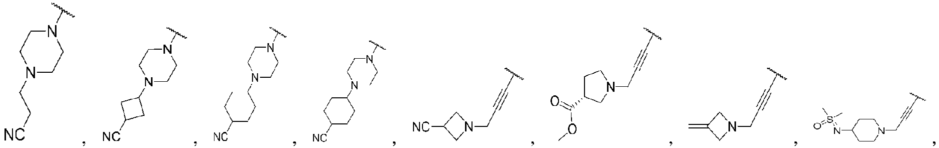

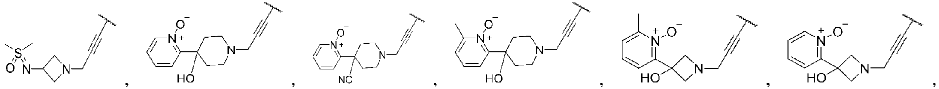

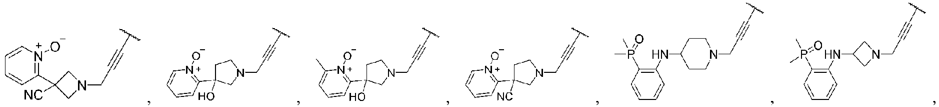

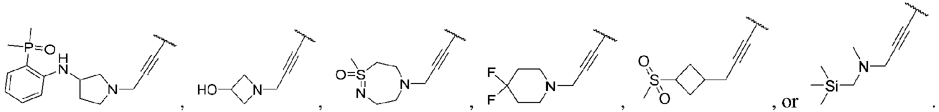

18. The compound, or the stable deuterated derivative thereof, or the stereoisomer thereof, or the pharmaceutically acceptable salt thereof, the solvate thereof, or the prodrug thereof according to claim 1 or 12, wherein R₁₁ is selected from the group consisting of:

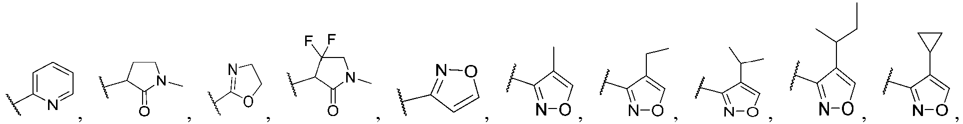

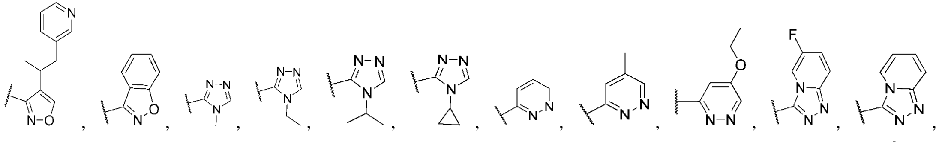

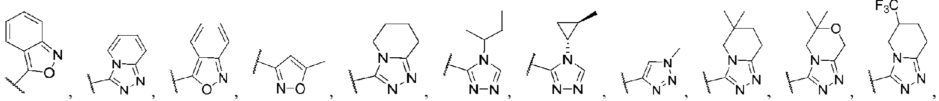

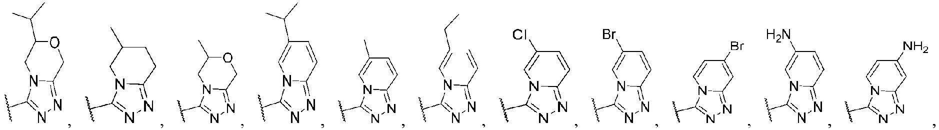

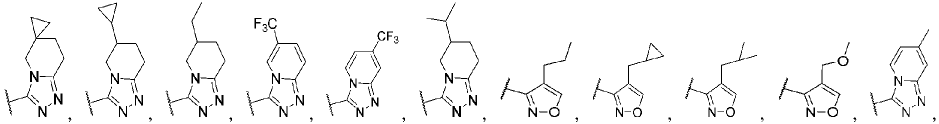

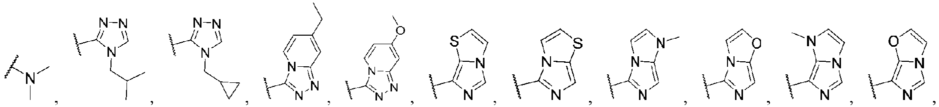

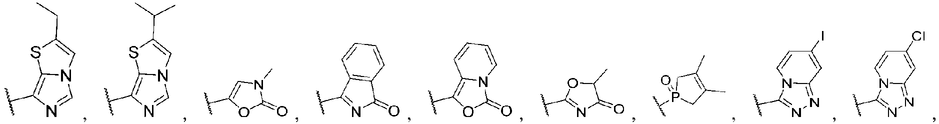

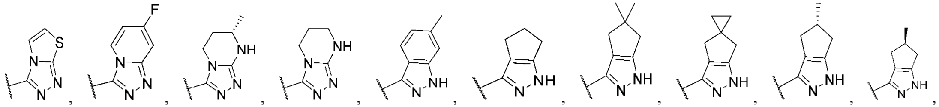

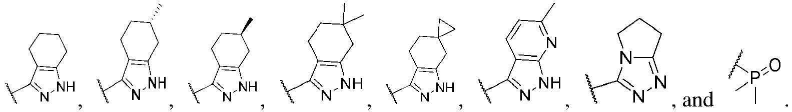

19. The compound, or the stable deuterated derivative thereof, or the stereoisomer thereof, or the pharmaceutically acceptable salt thereof, the solvate thereof, or the prodrug thereof according to claim 1, wherein R₁₁ is pyrrolidinyl, oxazolyl, isoxazolyl, dihydrooxazolyl, triazolyl, pyridazinyl, triazolopyridinyl, tetrahydrotriazolopyridinyl, or triazolomorpholinyl, wherein the pyrrolidinyl, oxazolyl, isoxazolyl, dihydrooxazolyl, triazolyl, pyridazinyl, triazolopyridinyl, tetrahydrotriazolopyridinyl, or triazolomorpholinyl is optionally substituted with a substituent selected from oxo, halogen, C₁₋₆ alkyl, C₁₆ alkoxy, C₃₋₆ cycloalkyl, and -C₁₋₄ alkyl-5- or 6-membered heteroaryl.

20. The compound, or the stable deuterated derivative thereof, or the stereoisomer thereof, or the pharmaceutically acceptable salt thereof, the solvate thereof, or the prodrug thereof according to claim 1, wherein 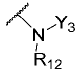 is selected from the group consisting of:

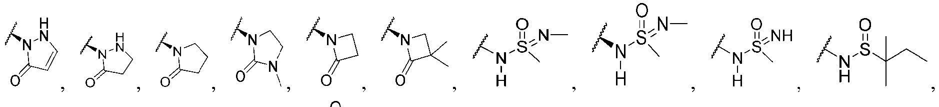

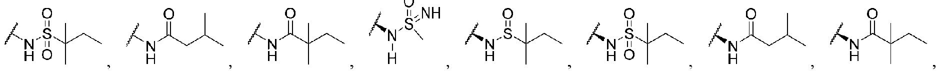

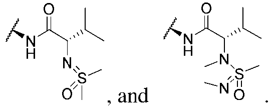

21. The compound, or the stable deuterated derivative thereof, or the stereoisomer thereof, or the pharmaceutically acceptable salt thereof, the solvate thereof, or the prodrug thereof according to claim 1, wherein R₆ is

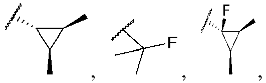

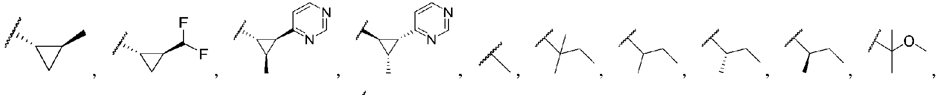

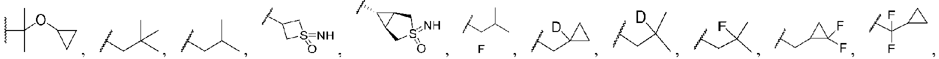

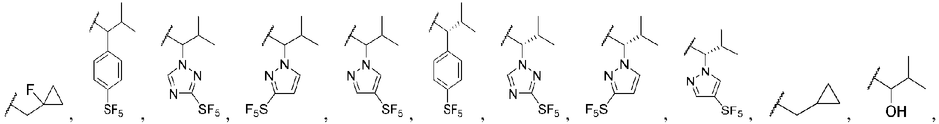

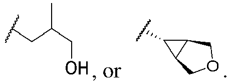

22. The compound, or the stable deuterated derivative thereof, or the stereoisomer thereof, or the pharmaceutically acceptable salt thereof, the solvate thereof, or the prodrug thereof according to claim 1, wherein the compound is selected from the compounds in Table A or the examples.

23. A pharmaceutical composition, comprising the compound, or the stable deuterated derivative thereof, or the stereoisomer thereof, or the pharmaceutically acceptable salt thereof, the solvate thereof, or the prodrug thereof according to any one of claims 1 to 22, and a pharmaceutically acceptable carrier.

24. Use of the compound, or the stable deuterated derivative thereof, or the stereoisomer thereof, or the pharmaceutically acceptable salt thereof, the solvate thereof, or the prodrug thereof according to any one of claims 1 to 22 or the pharmaceutical composition according to claim 23 in the preparation of a medicament for preventing and/or treating a disease or disorder associated with RAS protein activity.

25. The use according to claim 24, wherein the disease or disorder associated with RAS protein activity is a cancer.

26. The use according to claim 25, wherein the cancer is pancreatic cancer, colorectal cancer, non-small cell lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.
